(19) Europäisches Patentamt
European Patent Office
Office européen des brevets

(11) EP 1 940 835 B1

(12) **EUROPEAN PATENT SPECIFICATION**

(45) Date of publication and mention of the grant of the patent:
**30.03.2011 Bulletin 2011/13**

(21) Application number: **06779578.1**

(22) Date of filing: **29.09.2006**

(51) Int Cl.:
***C07D 471/04*** *(2006.01)* ***C07D 519/00*** *(2006.01)*
***A61K 31/437*** *(2006.01)* ***A61P 29/00*** *(2006.01)*

(86) International application number:
**PCT/GB2006/003626**

(87) International publication number:
**WO 2007/036733 (05.04.2007 Gazette 2007/14)**

(54) **PYRAZOLO[3,4-B]PYRIDINE COMPOUNDS, AND THEIR USE AS PDE4 INHIBITORS**

PYRAZOLO[3,4-B]PYRIDINVERBINDUNGEN UND IHRE VERWENDUNG ALS PDE4-INHIBITOREN

COMPOSES DE PYRAZOLO[3,4-B]PYRIDINE ET LEUR UTILISATION COMME INHIBITEURS DE PDE4

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **29.09.2005 US 721597 P**

(43) Date of publication of application:
**09.07.2008 Bulletin 2008/28**

(73) Proprietor: **GLAXO GROUP LIMITED**
**Greenford, Middlesex UB6 0NN (GB)**

(72) Inventors:
- **EDLIN, Christopher, David**
  **Stevenage**
  **Hertfordshire SG1 2NY (GB)**
- **HOLMAN, Stuart**
  **Stevenage**
  **Hertfordshire SG1 2NY (GB)**
- **JONES, Paul, Spencer**
  **Stevenage**
  **Hertfordshire SG1 2NY (GB)**
- **KEELING, Suzanne, Elaine**
  **Stevenage**
  **Hertfordshire SG1 2NY (GB)**
- **LINDVALL, Mika, Kristian**
  **Stevenage**
  **Hertfordshire SG1 2NY (GB)**
- **MITCHELL, Charlotte, Jane**
  **Stevenage**
  **Hertfordshire SG1 2NY (GB)**
- **TRIVEDI, Naimisha**
  **Stevenage**
  **Hertfordshire SG1 2NY (GB)**

(74) Representative: **Gladwin, Amanda Rachel**
**GlaxoSmithKline**
**Corporate Intellectual Property**
**CN925.1**
**980 Great West Road**
**Brentford, Middlesex TW8 9GS (GB)**

(56) References cited:
**WO-A-2004/056823** **WO-A2-2004/024728**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 940 835 B1

Printed by Jouve, 75001 PARIS (FR)

## Description

The present invention relates to pyrazolo[3,4-b]pyridine compounds or salts thereof, processes for their preparation, intermediates usable in these processes, and pharmaceutical compositions containing the compounds or salts. The invention also relates to the use of the pyrazolo[3,4-b]pyridine compounds or salts thereof in therapy, for example as inhibitors of phosphodiesterase type IV (PDE4) and/or for the treatment and/or prophylaxis of inflammatory and/or allergic diseases such as chronic obstructive pulmonary disease (COPD), asthma, rheumatoid arthritis, allergic rhinitis, psoriasis or atopic dermatitis.

## BACKGROUND TO THE INVENTION

WO 2004/024728 A2 (PCT/EP2003/011814, filed on 12 September 2003, published on 25 March 2004, Glaxo Group Limited), and incorporated herein by reference in its entirity as though fully set forth, discloses pyrazolo[3,4-b] pyridine compounds or salts thereof with a 4-$NHR^3$ group and a 5-C(O)-X group (wherein X is $NR^4R^5$ or $OR^{5a}$), according to the following formula (I):

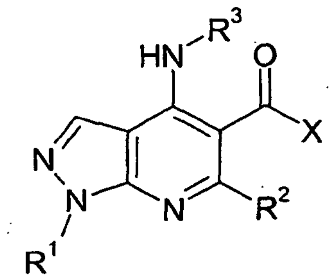

(I)

In WO 2004/024728 A2, the pyrazolo[3,4-b]pyridine compounds of formula (I) and salts thereof disclosed therein are disclosed as being inhibitors of phosphodiesterase type IV (PDE4).

WO 2004/056823 A1 (PCT/EP2003/014867, filed on 19 December 2003, published on 8 July 2004, Glaxo Group Limited), and incorporated herein by reference in its entirity as though fully set forth, discloses and claims further pyrazolo[3,4-b]pyridine compounds or salts thereof. WO 2004/056823 A1 also discloses the use of these compounds as PDE4 inhibitors.

WO 2004/024728 has been reviewed, and WO 2004/056823 mentioned, in Expert Opin. Ther. Patents, 2005 (January edition), 15(1), 111-114.

WO 2005/058892 A1 (PCT/EP2004/014490, filed on 17 December 2004, published on 30 June 2005, Glaxo Group Limited), and incorporated herein by reference in its entirity as though fully set forth, discloses further pyrazolo [3,4-b]pyridine compounds or salts thereof and their use as PDE4 inhibitors.

Further pyrazolo[3,4-b]pyridine compounds or salts thereof, and their use as PDE4 inhibitors, are disclosed in copending patent publications WO 2005/090348 A1 (PCT/GB2005/000983), WO 2005/090354 A1 (PCT/GB2005/000987), WO 2005/090352 A1 (PCT/EP2005/003038), and WO 2005/090353 A1 (PCT/GB2005/000976) (all Glaxo Group Limited, all PCT-filed on 15 March 2005 and all published on 29 September 2005).

## THE INVENTION

We have now found new pyrazolo[3,4-b]pyridine compounds, having a -$C(R^a)(R^b)$-$NR^4R^5$ substituent at the 5-position of the pyrazolo[3,4-b]pyridine ring system, which inhibit phosphodiesterase type IV (PDE4).

The present invention therefore provides a compound of formula (I) or a salt thereof (in particular, a pharmaceutically acceptable salt thereof):

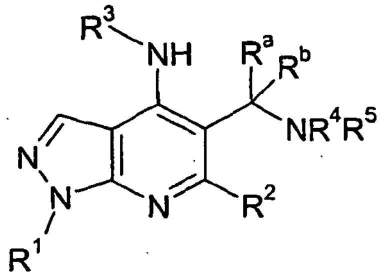

(I)

wherein:

$R^1$ is $C_{1-3}$alkyl, $C_{1-3}$fluoroalkyl, or $-CH_2CH_2OH$;

$R^2$ is a hydrogen atom (H), methyl, ethyl, n-propyl, isopropyl, n-butyl, $C_{1-2}$fluoroalkyl, cyclopropyl, cyclobutyl, (cyclopropyl)methyl-, cyano (-CN), or $-CH_2OH$

$R^3$ is optionally substituted $C_{4-7}$cycloalkyl or optionally substituted mono-unsaturated-$C_{5-7}$cycloalkenyl or an optionally substituted heterocyclic group of sub-formula (aa), (bb) or (cc);

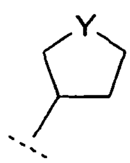 or 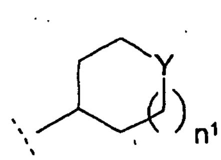 or 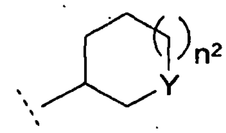

(aa) (bb) (cc)

in which $n^1$ and $n^2$ independently are 1 or 2; and in which Y is O, S, $SO_2$ or $NR^{10}$; where $R^{10}$ is a hydrogen atom (H), methyl, $C(O)NH_2$, C(O)-methy), or C(O)-$C_1$ fluoroalkyl;
or $R^3$ is a bicyclic group of sub-formula (ee):

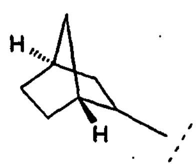

(ee) ;

and wherein, when $R^3$ is optionally substituted $C_{4-7}$cycloalkyl, then $R^3$ is $C_{4-7}$cycloalkyl optionally substituted on a ring carbon with one or two substituents independently being: oxo (=O); OH; methoxy; $C_1$fluoroalkoxy;. $NH_2$; $C_{1-2}$alkyl; $C_1$fluoroalkyl; $-CH_2OH$; -CH(Me)OH; $-CH_2CH_2OH$; $-CH_2NH_2$; -C(O)OH; $-C(O)NHR^{24}$ wherein $R^{24}$ is H or methyl; $-C(O)R^{25}$ wherein $R^{25}$ is methyl; fluoro; hydroxyimino (=N-OH); or ($C_{1-2}$alkoxy)imino (=N-$OR^{26}$ where $R^{26}$ is $C_{1-2}$alkyl); and wherein any OH, methoxy, fluoroalkoxy or $NH_2$ substituent is not substituted at the $R^3$ ring carbon attached (bonded) to the -NH- group of formula (I);
and wherein, when $R^3$ is the optionally substituted heterocyclic group of sub-formula (aa), (bb) or (cc), then $R^3$ is the heterocyclic group of sub-formula (aa), (bb) or (cc) optionally substituted on a ring carbon with one or two substituents independently being oxo (=O), OH or methyl; and wherein any OH substituent is not substituted at the $R^3$ ring carbon attached (bonded) to the -NH- group of formula (I) and is not substituted at either R3 ring carbon bonded to the Y group of the heterocyclic group (aa), (bb) or (cc);
and wherein, when $R^3$ is optionally substituted mono-unsaturated-$C_{5-7}$cycloalkenyl, then the cycloalkenyl is optionally substituted on a ring carbon with one substituent being fluoro or methyl , and the $R^3$ ring carbon bonded to the -NHR- group of formula (I) does not partake in the cycloalkenyl double bond;

provided that:

when $R^3$ is the heterocyclic group of sub-formula (aa) and Y is $NR^{10}$, then $R^{10}$ is not C(O)-methyl, or C(O)-$C_1$fluoroalkyl; and
when $R^3$ is the heterocyclic group of sub-formula (bb), and Y is $NR^{10}$, then $R^{10}$ is not methyl; and
when $R^3$ is the heterocyclic group of sub-formula (cc), then Y is O S, $SO_2$ or $NR^{10}$ wherein $R^{10}$ is H or methyl;

and wherein:

when $R^3$ is optionally substituted $C_{4-7}$cycloalkyl, then any -C(O)NHR$^{24}$ or -C(O)R$^{25}$ substituent on a ring carbon is: at the 3-position of a $R^3$ cyclobutyl ring; or at the 3- or 4-position of a $R^3$ cyclopentyl ring; or at the 4-position of a $R^3$ cyclohexyl ring; or at the 3-, 4-, 5- or 6- position of a $R^3$ cycloheptyl ring (wherein, in this connection, the 1-position of the $R^3$ cycloalkyl ring is deemed to be the connection point to the -NH- in formula (I), that is the ring atom connecting to the -NH- in formula (I));

and wherein:

when $R^3$ is optionally substituted $C_{4-7}$cycloalkyl, then any OH, methoxy, fluoroalkoxy, -$CH_2$OH -CH(Me)OH, -$CH_2CH_2$OH, -$CH_2NH_2$, or -C(O)OH substituent on a ring carbon is: at the 3-position of a $R^3$ cyclobutyl ring; or at the 3- or 4- position of a $R^3$ cyclopentyl ring; or at the 3-, 4- or 5- position of a $R^3$ cyclohexyl ring; or at the 3-, 4-, 5-or 6- position of a $R^3$ cycloheptyl ring; and

and wherein:

when $R^3$ is the heterocyclic group of sub-formula (aa), (bb) or (cc), then any OH substituent on a ring carbon is: at the 5-position of a six-membered $R^3$ heterocyclic group of sub-formula (cc) wherein $n^2$ is 1; or at the 5- or 6- position of a seven-membered $R^3$ heterocyclic group of sub-formula (cc) wherein $n^2$ is 2; or at the 6-position of a seven-membered $R^3$ heterocyclic group of sub-formula (bb) wherein $n^1$ is 2 (wherein, in this connection, the 1-position of the $R^3$ heterocyclic ring is deemed to be the connection point to the -NH- in formula (I), that is the ring atom connecting to the - NH- in formula (I), and the remaining positions of the ring are then numbered so that the ring heteroatom takes the lowest possible number);

and wherein:

$R^a$ is a hydrogen atom (H), methyl or ethyl;

$R^b$ is a hydrogen atom (H) or methyl;

wherein, when $R^b$ is methyl, then $R^a$ is methyl and $R^2$ is a hydrogen atom (H);

$R^4$ is a hydrogen atom (H), methyl, ethyl, n-propyl, -C(O)-Me, or -C(O)-$C_1$fluoroalkyl;
provided that when $R^4$ is -C(O)-Me or -C(O)-$C_1$fluoroalkyl, then $R^5$ is -$CH_2$-Ar;
and

$R^5$ is:

- C(O)-$(CH_2)_n$-Ar, -C(O)-Het, -C(O)-$C_{1-6}$alkyl, -C(O)-$C_1$fluoroalkyl, -C(O)-$(CH_2)_2$-C(O)-NR$^{15b}$NR$^{15b}$, -C(O)-$CH_2$-C(O)-NR$^{15b}$NR$^{15b}$, -C(O)-NR$^{15b}$-$(CH_2)_m$$^1$-Ar, -C(O)-NR$^{15b}$-Het, -C(O)-NR$^{15b}$-$C_{1-6}$alkyl, -C(O)-NR$^{5a}$R$^{5b}$, -S(O)$_2$-$(CH_2)_m$$^2$-Ar,-S(O)$_2$-Het, -S(O)$_2$-$C_{1-6}$alkyl,
or -$CH_2$-Ar;

wherein n and $m^1$ and $m^2$ independently are 0, 1 or 2; and
Ar, independent of other Ar, has the sub-formula (x) or (z), wherein (z) is connected at a ring carbon:

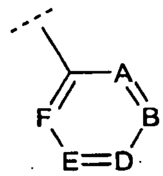

(x)

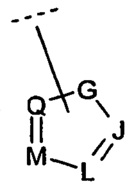

(z)

or $R^4$ and $R^5$ taken together are $-(CH_2)_{p^1}-$ (optionally substituted), or $-(CH_2)_2-X^5-(CH_2)_2-$, or $-C(O)-(CH_2)_{p^2}-$, or $-C(O)-N(R^{15})-(CH_2)_{p^3}-$, in which: $X^5$ is $NR^{17}$ and $p^1$ is 4, 5 or 6, and $p^2$ is 3, 4 or 5, and $p^3$ is 2 or 3;
or $NR^4R^5$ is of sub-formula (y), (y1), (y2) or (y3) wherein $p^4$ is 1 or 2:

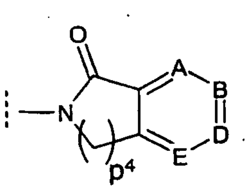

(y)

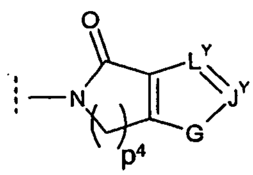

(y1)

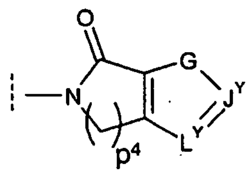

(y2)

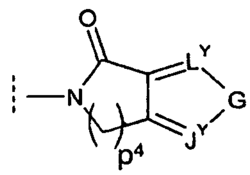

(y3)

and wherein, when $R^4$ and $R^5$ taken together is $-(CH_2)p^1-$, then the $NR^4R^5$ ring is optionally substituted on the 3-position ring-carbon atom and/or on the 4-position ring-carbon atom (wherein the ring-nitrogen is the 1-position) by one or two substituents independently being:
phenyl; phenyl substituted by one or two (e.g. one) substituents independently being methyl or $CF_3$ or fluoro or chloro or methoxy or difluoromethoxy;
phenyl-C(O)-; phenyl-C(O)- whose phenyl ring is substituted by one or two (e.g. one) substituents independently being methyl or fluoro or chloro or methoxy;
benzyloxy; phenyloxy; phenyloxy whose phenyl ring is substituted by one or two (e.g. one) substituents independently being methyl or $CF_3$ or fluoro or chloro or methoxy or difluoromethoxy;
a 5-membered heteroaromatic ring being oxazolyl, isoxazolyl, thiazolyl, 1,2,4-oxadiazolyl, 1,3,4-oxadiazolyl, furanyl, thienyl, pyrrolyl, pyrazolyl, imidazolyl; triazolyl or tetrazolyl, wherein the 5-membered heteroaromatic ring is optionally substituted on a ring carbon by one substituent being methyl or ethyl or isopropyl or t-butyl or phenyl or pyridinyl and/or is optionally substituted on a ring nitrogen by one substituent being methyl or ethyl or isopropyl or t-butyl or phenyl; $-CH_2$-(pyrrol-1-yl);
a 6-membered heteroaromatic ring being pyridinyl, pyrimidinyl, pyrazinyl or pyridazinyl, wherein the 6-membered heteroaromatic ring is optionally substituted on a ring carbon either by one methyl substituent or by one OH substituent which is substituted on a ring carbon bonded to a ring nitrogen (including the keto tautomer thereof);
$C_{1-2}$alkyl (e.g. methyl);
cyano (-CN);
or (4-hydroxyphenyl)-C(O)-$CH_2$-$CH_2$-;
or alternatively when $R^4$ and $R^5$ taken together is $-(CH_2)_{p^1}-$ and $p^1$ is 5, then the $NR^4R^5$ ring is optionally substituted on the 4-position ring-carbon atom by two substituents which when taken together are -O-C(O)-N(benzyl)-$CH_2$-;
or alternatively when $R^4$ and $R^5$ taken together is $-(CH_2)_{p^1}-$ and $p^1$ is 4 or 5, then the $NR^4R^5$ ring is optionally substituted on the 3-position and 4-position ring-carbon atoms by two substituents which when taken together are =CH-CH=CH-CH=;
and wherein, in sub-formula (x), and independently in sub-formula (y):

A is C-$R^{6A}$ or nitrogen (N),
B is C-$R^{6B}$ or nitrogen (N),
D is C-$R^{6D}$ or nitrogen (N),
E is C-$R^{6E}$ or nitrogen (N),
F is C-$R^{6F}$ or nitrogen (N),

wherein, $R^{6A}$, $R^{6B}$, $R^{6D}$, $R^{6E}$ and $R^{6F}$ independently are: a hydrogen atom (H), a halogen atom; $C_{1-6}$alkyl (e.g. $C_{1-4}$alkyl or $C_{1-2}$alkyl); $C_{1-4}$fluoroalkyl (e.g. $C_{1-2}$fluoroalkyl); $C_{3-6}$cycloalkyl; $C_{1-4}$alkoxy (e.g. $C_{1-2}$alkoxy); $C_{1-2}$fluoroalkoxy; $C_{3-6}$cycloalkyloxy; $-C(O)R^{16}$; $-C(O)OR^{30}$; $-S(O)_2-R^{16}$ (e.g. $C_{1-3}$alkyl-$S(O)_2$-); $R^{16}-S(O)_2-NR^{15}$- (e.g. $C_{1-3}$alkyl-$S(O)_2$-NH-); $R^{12}R^{13}N-S(O)_2$-; $C_{1-2}$alkyl-C(O)-$R^{15}N-S(O)_2$-; $C_{1-4}$alkyl-S(O)-, Ph-S(O)-, $R^{12}R^{13}N$-C(O)-; $-NR^{15}-C(O)R^{16}$; $R^7R^8N$; nitro ($-NO_2$); OH (including any tautomer thereof); $C_{1-4}$alkoxymethyl; $C_{1-4}$alkoxyethyl; $C_{1-2}$alkyl-$S(O)_2-CH_2$-; $R^{12}R^{13}N-S(O)_2-CH_2$-;. $C_{1-2}$alkyl-$S(O)_2-NR^{15}-CH_2$-; $-CH_2$-OH; $-CH_2CH_2$-OH; $-CH_2-NR^7R^8$; $-CH_2-CH_2-NR^7R^8$; $-CH_2-C(O)OR^{30}$; -CH2-C(O)-$NR^{12}R^{13}$; $-CH_2-NR^{15}-C(O)C_{1-3}$alkyl; $-(CH_2)n^{14}$-$Het^1$ where $n^{14}$ is 0 or 1; cyano (-CN); $Ar^6$; 1-pyrrolyl; or phenyl, pyridinyl or pyrimidinyl wherein the phenyl, pyridinyl or pyrimidinyl independently are optionally substituted by one or two substituents independently being fluoro, chloro, $C_{1-2}$alkyl, $C_1$fluoroalkyl, $C_{1-2}$alkoxy or $C_1$fluoroalkoxy;
provided that $R^{6A}$, $R^{6B}$, $R^{6D}$, $R^{6E}$ and $R^{6F}$ independently cannot be a chlorine, bromine or iodine atom (and optionally not a fluorine atom) when substituted on a ring-carbon which is bonded to a ring-nitrogen;
and/or two adjacent groups selected from $R^{6A}$, $R^{6B}$, $R^{6D}$, $R^{6E}$ and $R^{6F}$ are taken together and are: -CH=CH-CH=CH-, $-(CH_2)_n{}^{14a}$- where $n^{14a}$ is 3, 4 or 5 (e.g. 3 or 4),$-O-(CH_2)n^{14b}$-O- where $n^{14b}$ is 1 or 2; $-O-(CH_2)n^{14}$- or $-S(O)_2-(CH_2)_n{}^{14c}$-where $n^{14c}$ is 2 or 3 (e.g. 2); $-CH=CH-NR^{15b}$-; $-N=CH-NR^{15b}$-; $-CH=N-NR^{15b}$-; $-N=N-NR^{15b}$-; -NH-NH-C(O)-; -CH=CH-O-; -N=CH-O-; or -CH=CH-S-;
provided that:

two or more of A, B, D, E and F are independently C-H (carbon-hydrogen), C-F (carbon-fluorine), or nitrogen (N); and no more than two of A, B, D, E and F are nitrogen (N),

and wherein, in sub-formula (z), and independently in sub-formulae (y1), (y2) and (y3):

G is O or S or $NR^9$ wherein $R^9$ is a hydrogen atom (H), $C_{1-4}$alkyl, or $C_{1-2}$fluoroalkyl;
J is C-$R^{6J}$, C-[connection point to formula (I)], or nitrogen (N),
L is C-$R^{6L}$, C-[connection point to formula (I)], or nitrogen (N),
M is C-$R^{6M}$, C-[connection point to formula (I)], or nitrogen (N),
Q is C-$R^{6Q}$, C-[connection point to formula (I)], or nitrogen (N),
$J^Y$ is C-$R^{6J}$ or nitrogen (N), and
$L^Y$ is C-$R^{6L}$ or nitrogen (N),

wherein, $R^{6J}$, $R^{6L}$, $R^{6M}$ and $R^{6Q}$ independently are: a hydrogen atom (H), a halogen atom; $C_{1-4}$alkyl (e.g. $C_{1-2}$alkyl); $C_{1-3}$fluoroalkyl (e.g. $C_{1-2}$fluoroalkyl); $C_{3-6}$cycloalkyl; $C_{1-4}$alkoxy (e.g. $C_{1-2}$alkoxy);.$C_{1-2}$fluoroalkoxy; $C_{3-6}$cycloalkyloxy; $-CH_2$-OH; $-CH_2$-OMe; OH (including any tautomer thereof); 2-pyridinyl; 3-pyridinyl; 4-pyridinyl; or phenyl optionally substituted by one or two substituents independently being fluoro, chloro, $C_{1-2}$alkyl, $C_1$fluoroalkyl, $C_{1-2}$alkoxy or $C_1$fluoroalkoxy;
provided that:

two or more of J, L, M and Q are independently C-H, C-F, C-$C_{1-2}$alkyl (e.g. C-Me), C-$C_1$fluoroalkyl (e.g. C-$CF_3$), C-[connection point to formula (I)], or nitrogen (N);
and no more than three of J, L, M and Q are nitrogen (N);
and one or both of $J^Y$ and $L^Y$ are independently C-H, C-F, C-$C_{1-2}$alkyl (e.g. C-Me), C-$C_1$fluoroalkyl (e.g. C-$CF_3$), or nitrogen (N);

and wherein:

$NR^{5a}R^{5b}$ is a 1-pyrrolidinyl, 1-piperidinyl, or N-morpholino (4-morpholinyl) group;

$R^7$ and $R^8$ are independently a hydrogen atom (H); $C_{1-4}$alkyl (e.g. $C_{1-2}$alkyl such as methyl); $C_{3-6}$cycloalkyl; ($C_{3-6}$cycloalkyl)methyl-; or phenyl optionally substituted by one or two substituents independently being: fluoro,-chloro, $C_{1-2}$alkyl, $C_1$fluoroalkyl, $C_{1-2}$alkoxy or $C_1$fluoroalkoxy;

or $R^7$ and $R^8$ together are $-(CH_2)_n{}^6$- or $-C(O)-(CH_2)_n{}^7$- or $-C(O)-(CH_2)_n{}^{10}$-C(O)- or $-(CH_2)_2-X^7-(CH_2)_2$- or-C(O)-$X^7$-$(CH_2)_n{}^{10}$- in which: $n^6$ is 3, 4, 5, 6 or 7, $n^7$ is 3, 4 or $^5$, $n^{10}$ is 2 or 3, and $X^7$ is O or $NR^{14}$;

$R^{12}$ and $R^{13}$ independently are H; $C_{1-4}$alkyl (e.g. $C_{1-2}$alkyl); $C_{3-6}$cycloalkyl; ($C_{3-6}$cycloalkyl)methyl-; or phenyl optionally substituted by one or two substituents independently being: fluoro, chloro, $C_{1-2}$alkyl, $C_1$fluoroalkyl, $C_{1-2}$alkoxy or $C_1$fluoroalkoxy;

or $R^{12}$ and $R^{13}$ together are $-(CH_2)_n{}^{6a}-$ or or $-(CH_2)_2-X^{12}-(CH_2)_2-$ in which: $n^{6a}$ is 3, 4, 5, 6 or 7, and $X^{12}$ is O or $NR^{14}$;

$R^{14}$, independent of other $R^{14}$, is: a hydrogen atom (H); $C_{1-4}$alkyl (e.g. $C_{1-2}$alkyl); $C_{1-2}$fluoroalkyl (e.g. $CF_3$); cyclopropyl; -C(O)-$C_{1-4}$alkyl (e.g. -C(O)Me); -C(O)$NR^{7a}R^{8a}$ (e.g. -C(O)$NH_2$); or -$S(O)_2$-$C_{1-4}$alkyl (e.g. -$S(O)_2$Me);

$R^{7a}$ is a hydrogen atom (H) or $C_{1-4}$alkyl;

$R^{8a}$ is a hydrogen atom (H) or methyl;

$R^{15}$, independent of other $R^{15}$, is a hydrogen atom (H) or $C_{1-4}$alkyl;

$R^{15b}$, independent of other $R^{15b}$, is H or $C_{1-2}$alkyl;

$R^{16}$, independent of other $R^{16}$, is:

$C_{1-6}$alkyl (e.g. $C_{1-4}$alkyl or $C_{1-2}$alkyl);
$C_{3-6}$cycloalkyl (e.g. $C_{5-6}$cycloalkyl);
$C_{3-6}$cycloalkyl-$CH_2$- (e.g. $C_{5-6}$cycloalkyl-$CH_2$-);
pyridinyl (e.g. pyridin-2-yl) optionally substituted on a ring carbon atom by one of: a halogen atom, $C_{1-2}$alkyl, $C_1$fluoroalkyl, $C_{1-2}$alkoxy or $C_1$fluoroalkoxy;
$Ar^{16}$;
phenyl optionally substituted by one or two substituents independently being: a halogen atom, $C_{1-2}$alkyl, $C_1$fluoroalkyl, $C_{1-2}$alkoxy or $C_1$fluoroalkoxy;
benzyl optionally substituted on its ring by one or two substituents independently being: a halogen atom, $C_{1-2}$alkyl, $C_1$fluoroalkyl, $C_{1-2}$alkoxy or $C_1$fluoroalkoxy; or
a 4-, 5-, 6- or 7-membered saturated heterocyclic ring connected at a ring-carbon and containing one or two ring-hetero-atoms independently selected from O, S, and N; wherein any ring-nitrogens which are present are present as $NR^{27}$ where $R^{27}$ is H, $C_{1-2}$alkyl or -C(O)Me; and wherein the ring is optionally substituted at carbon by one $C_{1-2}$alkyl or oxo (=O) substituent, provided that any oxo (=O) substituent is substituted at a ring-carbon atom bonded to a ring-nitrogen;

$R^{17}$, independent of other $R^{17}$, is:

$C_{5-6}$cycloalkyl; -C(O)-$C_{1-4}$alkyl (e.g. -C(O)-$C_{3-4}$alkyl); -C(O)-$C_{3-6}$cycloalkyl (e.g. -C(O)-cyclopropyl); -S(O)$_2$-$C_{1-4}$alkyl (e.g. -$S(O)_2$Me or -$S(O)_2$Et); -C(O)-$Ar^{176}$; -C(O)-$Ar^{175}$; -C(O)-$Het^1$; - $CH_2$-C(O)-$Het^1$; - $CH_2$-C(O)-(pyrrolidin-1-yl); -$S(O)_2$-$Ar^{176}$; -$S(O)_2$-$Ar^{175}$; $Ar^{176}$; $Ar^{175}$; -C(O)-phenyl or -$S(O)_2$-phenyl, wherein, independently, the phenyl ring is unsubstituted or substituted by one or two (e.g. one) substituents independently being methyl or ethyl or $CF_3$ or fluoro or chloro or methoxy or difluoromethoxy or cyano (-CN) or -C(O)$NH_2$ or -C(O)-Me;
phenyl; phenyl substituted by one or two (e.g. one) substituents independently being methyl or ethyl or $CF_3$ or fluoro or chloro or methoxy or difluoromethoxy or cyano (-CN) or -C(O)$NR^{7a}R^{8a}$ (e.g. -C(O)$NH_2$) or -C(O)-Me; or benzyl or -CH(Me)-phenyl, wherein, independently, the phenyl ring is unsubstituted or substituted by one or two (e.g. one) substituents independently being methyl or fluoro or chloro or methoxy;

wherein $Ar^{175}$ is a 5-membered heteroaromatic ring being oxazolyl, isoxazolyl, thiazolyl,' 1,2,4-oxadiazolyl, 1,3,4-oxadiazolyl, furanyl, thienyl, pyrrolyl, pyrazolyl, imidazolyl, triazolyl or tetrazolyl,
wherein the 5-membered heteroaromatic ring $Ar^{175}$ is optionally substituted on a ring carbon by one substituent being methyl or ethyl or isopropyl or t-butyl or phenyl or pyridinyl and/or is optionally substituted on a ring nitrogen by one substituent being methyl or ethyl or isopropyl or t-butyl or phenyl;
or wherein the 5-membered heteroaromatic ring $Ar^{175}$ is optionally fused to a phenyl ring wherein the connection point to the remainder of the molecule is within the 5-membered ring;
and wherein $Ar^{176}$ is a 6-membered heteroaromatic ring being pyridinyl, pyrimidinyl, pyrazinyl or pyridazinyl, wherein the 6-membered heteroaromatic ring is optionally substituted on a ring carbon either by one methyl substituent or by one OH substituent which is substituted on a ring carbon bonded to a ring nitrogen (including the keto tautomer thereof);

$R^{30}$, independent of other $R^{30}$, is a hydrogen atom (H), $C_{1-4}$alkyl or $C_{3-6}$cycloalkyl;

$Ar^6$ and $Ar^{16}$ independently is/are a 5-membered aromatic heterocyclic ring connected at a ring-carbon and containing one O, S or $NR^{15}$ in the 5-membered ring, wherein the 5-membered ring can optionally contain in addition one or two N atoms, and wherein the heterocyclic ring is optionally substituted on a ring carbon atom by one of: a fluorine atom, a chlorine atom, $C_{1-2}$alkyl, $C_1$fluoroalkyl, $-CH_2OH$, $-CH_2$-OMe, or OH (including any keto tautomer thereof);

Het, independent of other Het, is a 4-, 5-, 6- or 7-membered saturated heterocyclic ring connected at a ring-carbon and containing one or two ring-hetero-atoms independently selected from O, S, and N; wherein any ring-nitrogens which are present are present as $NR^{31}$ where $R^{31}$ is H, $C_{1-4}$alkyl, -C(O)-$C_{1-4}$alkyl or -S(O)$_2$-$C_{1-4}$alkyl; and wherein the Het ring is optionally substituted at a ring carbon by one methyl or oxo (=O) substituent, provided that any oxo (=O) substituent is substituted at a ring-carbon atom bonded to a ring-nitrogen wherein $R^{31}$ is H or $C_{1-4}$alkyl; and

$Het^1$, independent of other $Het^1$, is a 4-, 5-, 6- or 7-membered saturated heterocyclic ring connected at a ring-carbon and containing one or two ring-hetero-atoms independently selected from O, S, and N; wherein any ring-nitrogens which are present are present as $NR^{31}$ a where $R^{31}$ a is H, $C_{1-2}$alkyl, -C(O)Me or -S(O)$_2$Me; and wherein the $Het^1$ ring is optionally substituted at a ring carbon by one methyl or oxo (=O) substituent, provided that any oxo (=O) substituent is substituted at a ring-carbon atom bonded to a ring-nitrogen wherein $R^{31}$a is H or $C_{1-2}$alkyl.

In compounds, for example in the compounds of formula (I), an "alkyl" group or moiety may be straight-chain or branched. Alkyl groups, for example $C_{1-8}$alkyl or $C_{1-6}$alkyl or $C_{1-4}$alkyl or $C_{1-3}$alkyl or $C_{1-2}$alkyl, which may be employed include $C_{1-6}$alkyl or $C_{1-4}$alkyl or $C_{1-3}$alkyl or $C_{1-2}$alkyl such as methyl, ethyl, n-propyl, n-butyl, n-pentyl, or n-hexyl or any branched isomers thereof such as isopropyl, t-butyl, sec-butyl, isobutyl, 3-methylbutan-2-yl, 2-ethylbutan-1-yl, or the like.

A corresponding meaning is intended for "alkoxy", "alkylene", and like terms derived from alkyl. For example, "alkoxy" such as $C_{1-6}$alkoxy or $C_{1-4}$alkoxy or $C_{1-2}$alkoxy includes methoxy, ethoxy, propyloxy, and oxy derivatives of the alkyls listed above. "Alkylsulfonyl'' such as $C_{1-4}$alkylsulfonyl includes methylsulfonyl (methanesulfonyl), ethylsulfonyl, and others derived from the alkyls listed above. "Alkylsulfonyloxy" such as $C_{1-4}$alkylsulfonyloxy includes methanesulfonyloxy (methylsulfonyloxy), ethanesulfonyloxy, *et al.*

"Cycloalkyl", for example $C_{3-8}$cycloalkyl (e.g. $C_{4-7}$cycloalkyl), includes cyclopropyl, cyclobutyl, cyclopentyl, cyclohexyl, cycloheptyl, cyclooctyl, and the like. Suitably, a $C_{3-8}$cycloalkyl group can be $C_{3-6}$cycloalkyl or $C_{5-6}$cycloalkyl or $C_{4-7}$cycloalkyl or $C_{6-7}$cycloalkyl, that is contains a 3-6 membered or 5-6 membered or 4-7 membered or 6-7 membered carbocyclic ring.

"Fluoroalkyl" includes alkyl groups with one, two, three, four, five or more fluorine substituents, for example $C_{1-4}$fluoroalkyl or $C_{1-3}$fluoroalkyl or $C_{1-2}$fluoroalkyl such as monofluoromethyl, difluoromethyl, trifluoromethyl, pentafluoroethyl, 2,2,2-trifluoroethyl ($CF_3CH_2$-), 2,2-difluoroethyl ($CHF_2CH_2$-), 2-fluoroethyl ($CH_2FCH_2$-), etc. "Fluoroalkoxy" includes $C_{1-4}$fluoroalkoxy or $C_{1-2}$fluoroalkoxy such as trifluoromethoxy, pentafluoroethoxy, monofluoromethoxy, difluoromethoxy, etc. "Fluoroalkylsulfonyl" such as $C_{1-4}$fluoroalkylsulfonyl includes trifluoromethanesulfonyl, pentafluoroethylsulfonyl, etc.

A halogen atom ("halo") present in compounds, for example in the compounds of formula (I), means a fluorine, chlorine, bromine or iodine atom ("fluoro", "chloro", "bromo" or "iodo"), for example fluoro, chloro or bromo.

When the specification states that atom or moiety A is "bonded" or "attached" to atom or moiety B, it means that atom/moiety A is directly bonded to atom/moiety B usually by means of a covalent bond or a double covalent bond, and excludes A being indirectly attached to B via one or more intermediate atoms/moieties (e.g. excludes A-C-B); unless it is clear from the context that another meaning is intended.

When $R^1$ is $C_{1-3}$alkyl or $C_{1-3}$fluoroalkyl, it can be straight-chained or branched. Where $R^1$ is $C_{1-3}$alkyl then it can be methyl, ethyl, n-propyl, or isopropyl. When $R^1$ is $C_{1-3}$fluoroalkyl, then $R^1$ can for example be $C_1$fluoroalkyl such as monofluoromethyl, - difluoromethyl, trifluoromethyl; or $R^1$ can be $C_2$fluoroalkyl such as pentafluoroethyl or more preferably $C_1$fluoroalkyl-$CH_2$- such as 2,2,2-trifluoroethyl ($CF_3CH_2$-), 2,2-difluoroethyl ($CHF_2CH_2$-), or 2-fluoroethyl ($CH_2FCH_2$-).

$R^1$ is $C_{1-3}$alkyl (e.g. methyl, ethyl or n-propyl), $C_{1-3}$fluoroalkyl or $-CH_2CH_2OH$. $R^1$ is suitably $C_{1-3}$alkyl, $C_{1-2}$fluoroalkyl, or $-CH_2CH_2OH$. Preferably, $R^1$ is $C_{2-3}$alkyl (e.g. ethyl or n-propyl), $C_2$fluoroalkyl (e.g. $C_1$fluoroalkyl-$CH_2$- such as $CF_3$-$CH_2$-) or- $CH_2CH_2OH$; in particular ethyl, n-propyl or $-CH_2CH_2OH$. More preferably, $R^1$ is $C_2$alkyl (ethyl) or $C_2$fluoroalkyl. $R^1$ is most preferably ethyl.

$R^2$ can for example be a hydrogen atom (H), methyl, ethyl, n-propyl, isopropyl, $C_1$fluoroalkyl (such as $CF_3$ or $CHF_2$ or $CH_2F$), $C_2$fluoroalkyl such as $C_2F_5$ or $C_1$fluoroalkyl-$CH_2$- (e.g. 2,2,2-trifluoroethyl ($CF_3CH_2$-), 2,2-difluoroethyl ($CHF_2CH_2$-), or 2-fluoroethyl ($CH_2FCH_2$-)), cyclopropyl, cyano (-CN), or $-CH_2OH$.

$R^2$ can for example be a hydrogen atom (H), methyl, ethyl, $C_1$fluoroalkyl (such as $CF_3$ or $CHF_2$ or $CH_2F$), cyano (-CN), or $-CH_2OH$. Suitably, $R^2$ is a hydrogen atom (H), methyl or ethyl.

$R^2$ can suitably be methyl, ethyl, $C_1$fluoroalkyl (such as $CF_3$ or $CHF_2$ or $CH_2F$), or $C_2$fluoroalkyl such as $C_2F_5$ or $C_1$fluoroalkyl-$CH_2$- (e.g. 2,2,2-trifluoroethyl ($CF_3CH_2$-), 2,2-difluoroethyl ($CHF_2CH_2$-), or 2-fluoroethyl ($CH_2FCH_2$-)).

Suitably, $R^2$ is methyl, ethyl or $C_1$fluoroalkyl (such as $CF_3$ or $CHF_2$ or $CH_2F$). Preferably, $R^2$ is methyl or ethyl. More preferably, $R^2$ is ethyl.

Preferably, in $R^3$ there is one substituent or no substituent on a ring carbon.

In one preferable embodiment, $R^3$ is the optionally substituted $C_{4-7}$cycloalkyl or the optionally substituted heterocyclic group of sub-formula (aa), (bb) or (cc).

In one optional embodiment, when $R^3$ is optionally substituted $C_{4-7}$cycloalkyl, it is not unsubstituted $C_5$cycloalkyl, i.e. not unsubstituted cyclopentyl. In this case, suitably, $R^3$ is optionally substituted $C_{6-7}$cycloalkyl or optionally substituted cyclobutyl.

When $R^3$ is optionally substituted $C_{4-7}$cycloalkyl, it is suitably optionally substituted $C_{6-7}$cycloalkyl or optionally substituted cyclobutyl, preferably optionally substituted $C_6$cycloalkyl (i.e. optionally substituted cyclohexyl).

When $R^3$ is optionally substituted $C_{4-7}$cycloalkyl, then $R^3$ is $C_{4-7}$cycloalkyl (e.g. $C_{6-7}$cycloalkyl or cyclobutyl) optionally substituted on a ring carbon with one or two substituents independently being (e.g. one substituent being): oxo (=O); OH; methoxy; $C_1$fluoroalkoxy (e.g. trifluoromethoxy or difluoromethoxy); $NH_2$; $C_{1-2}$alkyl such as methyl; $C_1$fluoroalkyl such as $-CH_2F$ or $-CHF_2$; $-CH_2OH$; -CH(Me)OH; $-CH_2CH_2OH$; $-CH_2NH_2$; -C(O)OH; $-C(O)NHR^{24}$ wherein $R^{24}$ is H or methyl (preferably H); $-C(O)R^{25}$ wherein $R^{25}$ is methyl; fluoro; hydroxyimino (=N-OH); or ($C_{1-2}$alkoxy)imino ($=N-OR^{26}$ where $R^{26}$ is $C_{1-2}$alkyl); and wherein any OH, methoxy, fluoroalkoxy or $NH_2$ substituent is not substituted at the $R^3$ ring carbon attached (bonded) to the -NH- group of formula (I).

When $R^3$ is optionally substituted $C_{4-7}$cycloalkyl, then $R^3$ can suitably be $C_{4-7}$cycloalkyl (e.g. $C_{6-7}$cycloalkyl or cyclobutyl) optionally substituted on a ring carbon with one or two substituents independently being (e.g. one substituent being): oxo (=O); OH; $NH_2$; $C_{1-2}$alkyl such as methyl; $C_1$fluoroalkyl such as $-CH_2F$ or $-CHF_2$; $-CH_2OH$; -CH(Me)OH; ; $-C(O)NHR^{24}$ wherein $R^{24}$ is H or methyl (preferably H); $-C(O)R^{25}$ wherein $R^{25}$ is methyl; fluoro; hydroxyimino (=N-OH); or ($C_{1-2}$alkoxy)imino ($=N-OR^{26}$ where $R^{26}$ is $C_{1-2}$alkyl).

Preferably, when $R^3$ is optionally substituted $C_{4-7}$cycloalkyl, then $R^3$ is $C_{4-7}$cycloalkyl (e.g. $C_{6-7}$cycloalkyl or cyclobutyl) optionally substituted on a ring carbon with one or two substituents independently being (e.g. one substituent being): oxo (=O); OH; methyl; $-CH_2F$; $-CHF_2$; $-CH_2OH$; $-C(O)NHR^{24}$ wherein $R^{24}$ is H; fluoro; hydroxyimino (=N-OH); or methoxyimino ($=N-OR^{26}$ where $R^{26}$ is methyl).

More preferably, when $R^3$ is optionally substituted $C_{4-7}$cycloalkyl, then $R^3$ is $C_{4-7}$cycloalkyl (e.g. $C_{6-7}$cycloalkyl-or cyclobutyl) optionally substituted on a ring carbon with one or two substituents independently being (e.g. one substituent being): oxo (=O); OH; methyl; $-C(O)NHR^{24}$ wherein $R^{24}$ is H; fluoro; or hydroxyimino (=N-OH).

Still more preferably, when $R^3$ is optionally substituted $C_{4-7}$cycloalkyl, then $R^3$ is $C_{4-7}$cycloalkyl (e.g. $C_{6-7}$cycloalkyl or cyclobutyl) optionally substituted on a ring carbon with one or two substituents independently being (e.g. one substituent being): oxo (=O); OH; $-C(O)NHR^{24}$ wherein $R^{24}$ is H; or hydroxyimino (=N-OH).

In one optional embodiment, in $R^3$, the $C_{4-7}$cycloalkyl can be unsubstituted.

When $R^3$ is optionally substituted $C_{4-7}$cycloalkyl or optionally substituted $C_{5-7}$cycloalkenyl, e.g. optionally, substituted ($C_{6-7}$cycloalkyl or cyclobutyl or $C_{5-7}$cycloalkenyl), such as optionally substituted $C_6$cycloalkyl (optionally substituted cyclohexyl) or optionally substituted cyclohexenyl, the one or two optional substituents on a ring carbon if present suitably can comprise a substituent (for example is or are substituent(s)) at the 3-, 4- and/or 5- position(s), e.g. at the 3- and/or 4- position(s), of the $R^3$ cycloalkyl or cycloalkenyl ring.

(In this connection and generally herein, the 1-position of the $R^3$ ring, e.g. of the $R^3$ cycloalkyl or cycloalkenyl ring, is deemed to be the connection point to the -NH- in formula (I) = the ring atom connecting to the -NH- in formula (I)).

Suitably, for $R^3$, and in particular when $R^3$ is optionally substituted $C_{4-7}$cycloalkyl or optionally substituted $C_{5-7}$cycloalkenyl, $R^3$ is not substituted (other than optionally by alkyl or fluoroalkyl) at the ring atom connecting to the -NH- in formula (I), and $R^3$. is not substituted (other than optionally by alkyl, fluoroalkyl or $NHR^{21}$) at the two ring atoms either side of (bonded to) the connecting atom. For example, suitably, for $R^3$, and in particular when $R^3$ is optionally substituted $C_{4-7}$cycloalkyl or optionally substituted $C_{5-7}$cycloalkenyl, $R^3$ is not substituted at the ring atom connecting to the -NH- in formula (I), and $R^3$ is not substituted at the two ring atoms either side of (bonded to) the connecting atom.

Suitably, for $R^3$, and in particular when $R^3$ is optionally substituted $C_{4-7}$cycloalkyl or optionally substituted $C_{5-7}$cycloalkenyl, the one or two optional $R^3$ ring-carbon substituents if present can comprise a substituent (for example is or are substituent(s)):

(a) at the 3-position of a $R^3$ cyclobutyl ring, or
(b) at the 3- and/or 4- position(s) of a $R^3$ cyclopentyl or cyclopentenyl ring, or
(c) at the 3-, 4- and/or 5- position(s) of a $R^3$ cyclohexyl or cyclohexenyl ring, or
(d) at the 3-, 4-, 5- and/or 6- position(s) of a $R^3$ cycloheptyl or cycloheptenyl ring, and/or
(f) at the 1-, 2- and/or highest-numbered- position(s) of a $R^3$ cycloalkyl or cycloalkenyl ring, for alkyl or fluoroalkyl substituent(s), and/or
(g) at the 2- and/or highest-numbered- position(s) of a $R^3$ cycloalkyl or cycloalkenyl ring, for $NH_2$ or fluoro substituent (s).

When $R^3$ is optionally substituted $C_{4-7}$cycloalkyl, then any OH, methoxy, fluoroalkoxy, $-CH_2OH$, -CH(Me)OH, $-CH_2CH_2OH$, $-CH_2NH_2$, or -C(O)OH substituent on a ring carbon is: at the 3-position of a $R^3$ cyclobutyl ring; or at the 3- or 4- position of a $R^3$ cyclopentyl ring; or at the 3-, 4- or 5- position of a $R^3$ cyclohexyl ring (such as at the 3- or 5- position of a $R^3$ cyclohexyl ring especially for any OH substituent); or at the 3-, 4-, 5-or 6- position (e.g. 4- or 5- position) of a $R^3$ cycloheptyl ring. Suitably, when $R^3$ is optionally substituted $C_{4-7}$cycloalkyl, then any OH, methoxy, fluoroalkoxy, $-CH_2OH$, -CH(Me)OH, $-CH_2CH_2OH$ or $-CH_2NH_2$, or -C(O)OH substituent (or any OH substituent) on a ring carbon is at the 3- or 4- position of a $R^3$ cyclopentyl ring; or more suitably at the 3-, 4- or 5- position, such as at the 3- or 5-position, of a $R^3$ cyclohexyl ring.

When $R^3$ is optionally substituted $C_{4-7}$cycloalkyl, then any $-C(O)NHR^{24}$ or $-C(O)R^{25}$ substituent on a ring carbon is: at the 3-position of a $R^3$ cyclobutyl ring; or at the 3- or 4-position of a $R^3$ cyclopentyl ring; or at the 4-position of a $R^3$ cyclohexyl ring; or at the 3-, 4-, 5- or 6- position (e.g. 4- or 5- position) of a $R^3$ cycloheptyl ring. When $R^3$ is optionally substituted $C_{4-7}$cycloalkyl, then any $-C(O)NHR^{24}$ or $-C(O)R^{25}$ substituent, or any-$C(O)NHR^{24}$ substituent, on a ring carbon is suitably at the 3-position of a $R^3$ cyclobutyl ring or at the 4-position of a $R^3$ cyclohexyl ring. When $R^3$ is optionally substituted $C_{4-7}$cycloalkyl, it is preferable for any $-C(O)NHR^{24}$ substituent to be at the 4-position of a $R^3$ cyclohexyl ring.

When $R^3$ is optionally substituted $C_{4-7}$cycloalkyl, any $NH_2$ substituent on a ring carbon is at any position other than the 1-position (the ring atom connecting to the -NH- in formula (I)), e.g. at the 2-, 3-, 4-, 5-, 6- or 7- position. Suitably, any $NH_2$ substituent is at the 2-, 3-, 4-, 5- or 6- position, for example at the 3-, 4- or 5- position or at the 3- or 5-position, of a $R^3$ cyclohexyl ring.

When $R^3$ is optionally substituted $C_{4-7}$cydoalkyl or optionally substituted $C_{5-7}$cycloalkenyl, any alkyl or fluoroalkyl substituent on a ring carbon can for example be at the 1-, 2-, 3-, 4-, 5-, 6- or 7- position, for example at the 1-, 2-, 3-, 5- or 6- position, e.g. the 1-position, of the $R^3$ ring. Preferably, any such alkyl or fluoroalkyl substituent on a ring carbon is at the 1-, 2-, 3-, 5- or 6- position, or more preferably at the 1-, 3- or 5-position, of a $R^3$ cyclohexyl or cyclohexenyl ring.

When $R^3$ is optionally substituted $C_{4-7}$cycloalkyl or optionally substituted $C_{5-7}$cycloalkenyl, any fluoro substituent on a ring carbon can for example be at the 1-, 2-, 3-, 4-, 5-, 6- or 7- position, for example at the 2-, 3-, 4-, 5- or 6- position, such as at the 3- or 4- position, of the $R^3$ ring. Suitably, any fluoro substituent on a ring carbon is at the 3-, 4- or 5- position, in particular at the 4- position, of a $R^3$ cyclohexyl or cyclohexenyl ring.

When $R^3$ is optionally substituted $C_{4-7}$cycloalkyl, any oxo (=O), hydroxyimino (=N-OH); or ($C_{1-2}$alkoxy)imino ($=N-OR^{26}$) substituent on a ring carbon can for example be at the 3-, 4- or 5- position, e.g. at the 4-position, of the $R^3$ cycloalkyl (e.g. $C_{6-7}$cycloalkyl e.g. cyclohexyl, or cyclobutyl) ring. Any such substituent can for example be at the 3-position of a $R^3$ cyclobutyl ring or at the 4-position of a $R^3$ cyclohexyl ring. Preferably, any such substituent is at the 4-position of a $R^3$ cyclohexyl ring.

When $R^3$ is optionally substituted $C_{4-7}$cycloalkyl (e.g. $C_{6-7}$cycloalkyl or cyclobutyl, optionally substituted), then $R^3$ is suitably cyclohexyl (i.e. unsubstituted); or cycloheptyl (i.e. unsubstituted); or cyclohexyl substituted on a ring carbon by one substituent being oxo (=O), OH, $NH_2$, $C_{1-2}$alkyl, $C_1$fluoroalkyl such as $-CH_2F$ or-$CHF_2$, $-CH_2OH$, -CH(Me)OH, $-C(O)NHR^{24}$ wherein $R^{24}$ is H or methyl (preferably H), $-C(O)R^{25}$, fluoro, hydroxyimino (=N-OH), or ($C_{1-2}$alkoxy)imino ($=N-OR^{26}$ wherein $R^{26}$ is $C_{1-2}$alkyl); or cyclohexyl substituted by two fluoro substituents; or cyclobutyl (i.e. unsubstituted); or cyclobutyl substituted on a ring carbon with one substituent being oxo (=O), OH, methyl, $-CH_2F$, $-CHF_2$, $-CH_2OH$, -C (O)$NHR^{24}$ wherein $R^{24}$ is H or methyl (preferably H), fluoro, hydroxyimino (=N-OH), or methoxyimino ($=N-OR^{26}$ where $R^{26}$ is methyl). Preferably, when $R^3$ is optionally substituted $C_{4-7}$cycloalkyl (e.g. $C_{6-7}$cycloalkyl or cyclobutyl, optionally substituted), then $R^3$ is cyclohexyl (i.e. unsubstituted); or cycloheptyl (i.e. unsubstituted); or cyclohexyl substituted on a ring carbon by one substituent being oxo (=O), OH, $NH_2$, $C_{1-2}$alkyl, $C_1$fluoroalkyl such as $-CH_2F$ or-$CHF_2$, $-CH_2OH$, -C (O)$NHR^{24}$ wherein $R^{24}$ is H, fluoro, hydroxyimino (=N-OH), or (methoxy)imino ($=N-OR^{26}$, wherein $R^{26}$ is methyl); or cyclohexyl substituted by two fluoro substituents; or cyclobutyl (i.e. unsubstituted); or cyclobutyl substituted on a ring carbon with one substituent being oxo (=O), OH, methyl, $-CH_2F$, $-CHF_2$, $-CH_2OH$, $-C(O)NHR^{24}$ wherein $R^{24}$ is H, fluoro, hydroxyimino (=N-OH), or methoxyimino ($=N-OR^{26}$ where $R^{26}$ is methyl). More preferably, when $R^3$ is optionally substituted $C_{4-7}$cycloalkyl. (e.g. $C_{6-7}$cycloalkyl or cyclobutyl, optionally substituted), then $R^3$ is cyclohexyl (i.e. unsubstituted); or cyclohexyl substituted on a ring carbon by one oxo (=O), hydroxyimino (=N-OH), $-C(O)NH_2$, methyl or OH substituent; or cyclobutyl substituted on a ring carbon by one $-C(O)NHR^{24}$ substituent wherein $R^{24}$ is H. The optional substituent can for example be at the 3- or 4- position of the $R^3$ cyclohexyl ring. Preferably, when $R^3$ is optionally substituted $C_{4-7}$cycloalkyl (e.g. $C_{6-7}$cycloalkyl or cyclobutyl, optionally substituted), then any OH substituent on a ring carbon is preferably at the 3-position of a $R^3$ cyclohexyl ring, and/or any oxo (=O), hydroxyimino (=N-OH), or ($C_{1-2}$alkoxy)imino (=N-OR26) substituent on a ring carbon is preferably at the 4-position of a $R^3$ cyclohexyl ring or at the 3-positions of a $R^3$ cyclobutyl ring, and/or any alkyl or fluoroalkyl substituent is preferably at the 1-, 3- or 5- position of a $R^3$ cyclohexyl ring.

When $R^3$ is optionally substituted cyclobutyl, then $R^3$ can preferably be cyclobutyl (i.e. unsubstituted) or more preferably 3-(aminocarbonyl)cyclobutyl (i.e. 3-(aminocarbonyl)cyclobutan-1-yl) (e.g. in a *cis* or *trans* configuration, preferably *cis*).

When $R^3$ is optionally substituted cyclopentyl, $R^3$ can for examples be cyclopentyl (i.e. unsubstituted) or more suitably 3-hydroxy-cyclopentyl.

When $R^3$ is optionally substituted $C_{4-7}$cycloalkyl (e.g. optionally substituted $C_{6-7}$cycloalkyl or optionally substituted cyclobutyl), $R^3$ can for example be cyclobutyl (i.e. unsubstituted), 4-hydroxy-cyclohexyl (i.e. 4-hydroxycyclohexan-1-yl) (e.g. racemic or in a *cis* or *trans* configuration), 4-methylcyclohexyl (e.g. racemic), 2-aminocyclohexyl (e.g. racemic or in a *cis* or *trans* configuration, preferably *trans*), 4-aminocyclohexyl (e.g. racemic or in a *cis* or *trans* configuration, preferably racemic or *cis*), 3-oxocyclohexyl, 4-acetylcyclohexyl (e.g. racemic or in a *cis* or *trans* configuration, preferably racemic or *cis*), 4-(1-hydroxyethyl)cyclohexyl (e.g. racemic or in a *cis* or *trans* configuration with respect to the ring, preferably racemic or *cis*), or 3-(hydroxymethyl)cyclohexyl (e.g. racemic or in a *cis* or *trans* configuration).

However, when $R^3$ is optionally substituted $C_{4-7}$cycloalkyl (e.g. optionally substituted $C_{6-7}$cycloalkyl or optionally substituted cyclobutyl), $R^3$ is more preferably cyclohexyl (i.e. unsubstituted), cycloheptyl (i.e. unsubstituted), 3-hydroxy-cyclohexyl (i.e. 3-hydroxycyclohexan-1-yl) (e.g. racemic or in a *cis* or *trans* configuration, preferably racemic or *cis*), 4-oxo-cyclohexyl (i.e. 4-oxocyclohexan-1-yl), 4-(hydroxyimino)cyclohexyl (i.e. 4-(hydroxyimino)cyclohexan-1-yl), 4-($C_{1-2}$alkoxyimino)cyclohexyl, 4-(aminocarbonyl)cyclohexyl (i.e. 4-(aminocarbonyl)cyclohexan-1-yl) (e.g. racemic or in a *cis* or *trans* configuration, preferably racemic or *cis*); 1-methylcyclohexyl (e.g. racemic), 3-methylcyclohexyl (e.g. racemic), 4,4-(difluoro)cyclohexyl, 3-aminocyclohexyl (e.g. racemic or in a *cis* or *trans* configuration), , 4-(hydroxymethyl) cyclohexyl (e.g. racemic or in a *cis* or *trans* configuration), or 3-(aminocarbonyl)cyclobutyl (i.e. 3-(aminocarbonyl)cyclobutan-1-yl) (e.g. racemic or in a *cis* or *trans* configuration, preferably *cis*).

A "*cis* configuration" in general includes mixtures of configurations wherein the *cis* configuration is the major component.

When $R^3$ is optionally substituted $C_{4-7}$cycloalkyl (e.g. optionally substituted $C_{6-7}$cycloalkyl or optionally substituted cyclobutyl), $R^3$ is still more preferably cyclohexyl (i.e. unsubstituted), 3-hydroxy-cyclohexyl (i.e. 3-hydroxycyclohexan-1-yl) (preferably racemic or in a *cis* configuration), 4-oxo-cyclohexyl (i.e. 4-oxocyclohexan-1-yl), 4-(hydroxyimino) cyclohexyl (i.e. 4-(hydroxyimino)cyclohexan-1-yl), 4-(aminocarbonyl)cyclohexyl (i.e. 4-(aminocarbonyl)cyclohexan-1-yl) (preferably racemic or in a *cis* configuration), or 3-(aminocarbonyl)cyclobutyl (i.e. 3-(aminocarbonyl)cyclobutan-1-yl) (preferably racemic or in a *cis* configuration).

When $R^3$ is optionally substituted mono-unsaturated-$C_{5-7}$cycloalkenyl, suitably it is optionally substituted mono-unsaturated-$C_{5-6}$cycloalkenyl, preferably optionally substituted mono-unsaturated-$C_6$cycloalkenyl (i.e. optionally substituted mono-unsaturated-cyclohexenyl = optionally substituted cyclohexenyl). For example, the $R^3$ cyclohexenyl can be optionally substituted cyclohex-3-en-1-yl.

When $R^3$ is optionally substituted mono-unsaturated-$C_{5-7}$cycloalkenyl, suitably the $R^3$ cycloalkenyl (e.g. cyclohexenyl) is substituted on a ring carbon with one fluoro substituent or is unsubstituted. For example, the $R^3$ optionally substituted cycloalkenyl can be cyclohex-3-en-1-yl (i.e. unsubstituted) or 4-fluoro-cyclohex-3-en-1-yl.

For $R^3$ cycloalkenyl, the optional substituent(s) on a ring carbon can for example be at the 1-, 2-, 3-, 4-, 5- or 6- position(s) of the cycloalkenyl ring.

When $R^3$ is the heterocyclic group of sub-formula (aa), (bb) or (cc), then Y is suitably O or $NR^{10}$. When $R^3$ is the heterocyclic group of sub-formula (aa) or (bb), then Y is preferably O or N-C(O)-$NH_2$.

$R^{10}$ can for example be a hydrogen atom (H), methyl, ethyl, C(O)$NH_2$, C(O)-methyl or C(O)-$C_1$fluoroalkyl.

Suitably, $R^{10}$ is not methyl.

Suitably, $R^{10}$ is a hydrogen atom (H), C(O)$NH_2$, C(O)-methyl or C(O)-$C_1$fluoroalkyl (e.g. C(O)-$CF_3$). More suitably, $R^{10}$ is H, C(O)$NH_2$ or C(O)-methyl; in particular C(O)$NH_2$.

When $R^3$ is the heterocyclic group of sub-formula (aa), (bb) or (cc), then it is preferable that $R^3$ is the heterocyclic group of sub-formula (aa) or (bb), more preferably of sub-formula (bb).

In sub-formula (bb), $n^1$ is preferably 1. In sub-formula (cc), $n^2$ is preferably 1. That is, six-membered rings are preferred in the $R^3$ heterocyclic group.

When $R^3$ is the optionally substituted heterocyclic group of sub-formula (aa), (bb) or (cc), then $R^3$ is the heterocyclic group of sub-formula (aa), (bb) or (cc) optionally substituted on a ring carbon with one or two substituents independently being (e.g. one substituent being) oxo (=O), OH or methyl; and wherein any OH substituent is not substituted at the $R^3$ ring carbon attached (bonded) to the -NH- group of formula (I) and is not substituted at either $R^3$ ring carbon bonded to the Y group of the heterocyclic group (aa), (bb) or (cc).

Preferably, the $R^3$ heterocyclic group of sub-formula (aa), (bb) or (cc) is optionally substituted on a ring carbon with one or two substituents independently being (e.g. one substituent being) oxo (=O) or methyl. More preferably, the $R^3$ heterocyclic group of sub-formula (aa), (bb) or (cc) is optionally substituted on a ring carbon with one or two (e.g. one) substituents being oxo (=O).

It is generally preferable that, in $R^3$, the heterocyclic group of sub-formula (aa), (bb) or (cc) is not substituted on a ring carbon. (In this connection, where Y is $NR^{10}$, $R^{10}$ is not a substituent on a ring carbon).

In the $R^3$ heterocyclic group of sub-formula (aa), (bb) or (cc), any oxo (=O) substituent on a ring carbon is suitably on a carbon atom bonded (adjacent) to Y. In one embodiment, any oxo (=O) substituent on a ring carbon can be on a carbon atom bonded (adjacent) to Y only when Y is O or $NR^{10}$.

In the $R^3$ heterocyclic group of sub-formula (aa), (bb) or (cc), any oxo (=O) substituent on a ring carbon can

suitably be at the 2-, 3-, 4-, 5- or 6- position of the $R^3$ heterocyclic ring. For example any ring-carbon oxo (=O) substituent (s) can be: at the 2-, 4- or 5-position(s) (e.g. 2-position or 4- position, or two oxo substituents at 2- and 4- positions) of a $R^3$ heterocyclic group of sub-formula (aa), at the 2-, 4-, 5- or 6- position(s) (e.g. 4-position) of a six-membered $R^3$ heterocyclic group of sub-formula (cc) wherein $n^2$ is.1, at the 2-, 3-, 5-, 6- or 7- position(s) (e.g. 5-position) of a seven-membered $R^3$ Heterocyclic group of sub-formula (bb) wherein $n^1$ is 2, or at the 2-, 4-, 5-, 6- or 7- position(s) (e.g. 2-position) of a seven-membered $R^3$ heterocyclic group of sub-formula (cc) wherein $n^2$ is 2.

(In this connection and generally herein, the 1-position of the $R^3$ heterocyclic ring is deemed to be the connection point to the -NH- in formula (I) = the ring atom connecting to the -NH- in formula (I), and the remaining positions of the ring are then numbered so that the ring heteroatom takes the lowest possible number).

In the $R^3$ heterocyclic group of sub-formula (aa), (bb) or (cc), any methyl substituent on a ring carbon can for example be at the 1-, 2-, 3-, 4-, 5- or 6- position, e.g. the 1-position, of the $R^3$ heterocyclic ring, in particular at the 1-, 3- or 5- position of a six-membered $R^3$ heterocyclic ring which is of sub-formula (bb) wherein $n^1$ is 1 or which is of sub-formula (cc) wherein $n^2$ is 1.

In the $R^3$ heterocyclic group of sub-formula (aa), (bb) or (cc), then any OH substituent on a ring carbon is: at the 5-position of a six-membered $R^3$ heterocyclic group of sub-formula (cc) wherein $n^2$ is 1; at the 5- or 6- position of a seven-membered $R^3$ heterocyclic group of sub-formula (cc) wherein $n^2$ is 2; or at the 6- position of a seven-membered $R^3$ heterocyclic group of sub-formula (bb) wherein $n^1$ is 2.

Therefore, in the $R^3$ heterocyclic group of sub-formuta (aa), (bb) or (cc), only methyl or oxo (=O) substitution or no substitution is allowed on a ring carbon, independently at each of the 2- and highest-numbered- positions of the $R^3$ heterocyclic ring (e.g. at each of the 2- and 6- positions of a six-membered $R^3$ heterocyclic ring); and only methyl substitution or no substitution is allowed at the 1-position ring-carbon of the $R^3$ heterocyclic ring.

However, it is generally preferable that, in $R^3$, the heterocyclic group of sub-formula (aa) (bb) or (cc) is not substituted on a ring carbon. (In this connection, where Y is $NR^{10}$, $R^{10}$ is not a substituent on a ring carbon).

When $R^3$ is the heterocyclic group of sub-formula (aa) and Y is $NR^{10}$, then $R^{10}$ is not C(O)-methyl, or C (O)-$C_1$fluoroalkyl.

In one preferable embodiment, when $R^3$ is the heterocyclic group of sub-formula (aa), then Y is O, S, $SO_2$, NH or $NC(O)NH_2$ (in particular Y can be O, S, NH or $NC(O)NH_2$, such as $NC(O)NH_2$).

When $R^3$ is the heterocyclic group of sub-formula (bb); and Y is $NR^{10}$ (e.g. when $NHR^3$ is

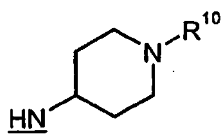

),

then $R^{10}$ is not methyl.

Therefore, when $R^3$ is the heterocyclic group of sub-formula (bb), then Y is O, S, $SO_2$ or $NR^{10}$ wherein $R^{10}$ is H, $C(O)NH_2$, C(O)-methyl or C(O)-$C_1$fluoroalkyl (e.g. C(O)-$CF_3$). When $R^3$ is the heterocyclic group of sub-formula (bb), then $R^{10}$ is preferably H, $C(O)NH_2$ or C(O)-methyl, for example $C(O)NH_2$ or C(O)-methyl, more preferably $C(O)NH_2$.

When $R^3$ is the heterocyclic group of sub-formula (cc), then Y is O, S, $SO_2$ or $NR^{10}$ wherein $R^{10}$ is H or methyl.

Suitably, when $R^3$ is the heterocyclic group of sub-formula (cc), then:

either Y is O, S, $SO_2$ or $NR^{10}$ wherein $R^{10}$ is H,

or $NHR^3$ is of sub-formula (m4):

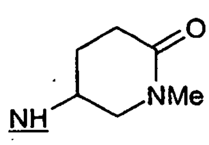

(m4) ,

wherein the -NH- connection point of the $NHR^3$ group to the 4-position of the pyrazolopyridine of formula (I) is underlined.

Suitably, when $R^3$ is the heterocyclic group of sub-formula (cc), then Y is O, S, $SO_2$ or $NR^{10}$ wherein $R^{10}$ is H, or Y is O or $NR^{10}$ wherein $R^{10}$ is H.

Optionally, for sub-formula (bb) and/or for sub-formula (cc), Y is O or $NR^{10}$.

When $R^3$ is optionally substituted $C_{4-7}$cycloalkyl (e.g. optionally substituted $C_{6-7}$cycloalkyl or optionally substituted cyclobutyl) or optionally substituted mono-unsaturated-$C_{5-7}$cycloalkenyl or an optionally substituted heterocyclic group of sub-formula (aa), (bb) or (cc), then a substituent on a ring carbon can be racemic or in the *cis* or *trans* configuration with respect to the -NH- group of formula (I) to which $R^3$ is attached (bonded). A *cis* or *trans* configuration includes mixtures of configurations wherein the stated configuration is the major component. In this context, "racemic" refers to a mixture of isomers containing substantially equal amounts of the *cis* and *trans* configurations with respect to a substituent and the -NH- group on the $R^3$ ring, and in this context "racemic" does not refer to isomerism at atoms other than $R^3$ ring carbon atoms. For example, an OH or $-C(O)NHR^{24}$ substituent on $C_{6-7}$cycloalkyl or cyclobutyl can for example be in the *cis* configuration and/or a $NH_2$ substituent on $C_{6-7}$cycloalkyl can for example be racemic or in the *cis* or *trans* configuration, with respect to the -NH-group of formula (I) to which $R^3$ is attached (bonded), including mixtures of configurations wherein the stated configuration is the major component.

When $R^3$ is a bicyclic group of sub-formula (ee), then $NHR^3$ can be of sub-formula (c6) or (c7):

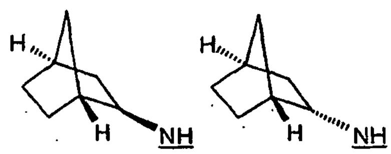

(c 6) (c 7) ;

wherein the -NH- connection point of the $NHR^3$ group to the 4-position of the pyrazolopyridine of formula (I) is underlined.

Preferably, $NHR^3$ is of sub-formula (a1), (b), (c), (c 1), (c 2), (c 3), (c 4), (c 5), (c 6), (c 7), (d), (e), (f), (g), (g2), (g4), (h), (i). G), (k), (k1), (k2), (k3), (L), (m), (m1), (m3), (m4), (n), (o), (o1), (o2), (o3). (p), (p1), (p2), (p3), (p5), (p6), (p9), (p10), (p12), (p13), (p14), (p15), or (q):

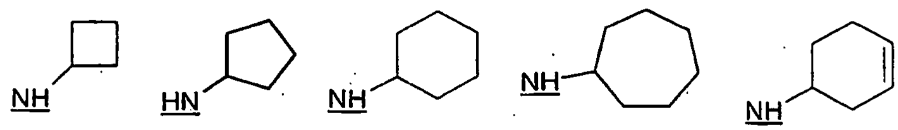

(a1) (b) (c) (c 1) (c 2)

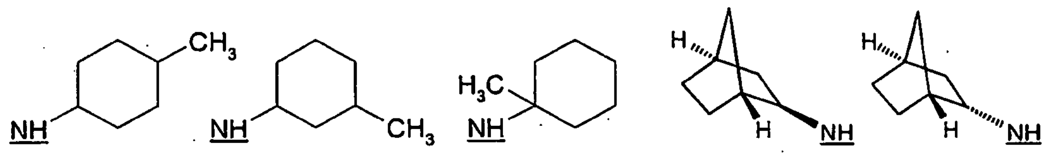

(c 3) (c 4) (c 5) (c 6) (c 7)

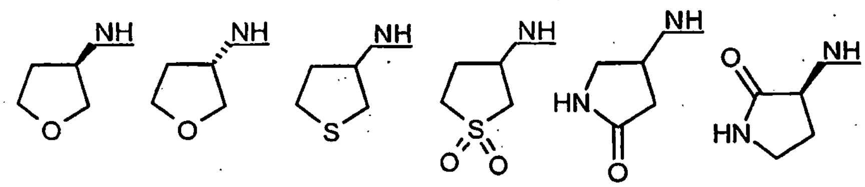

(d) (e) (f) (g) (g2) (g4)

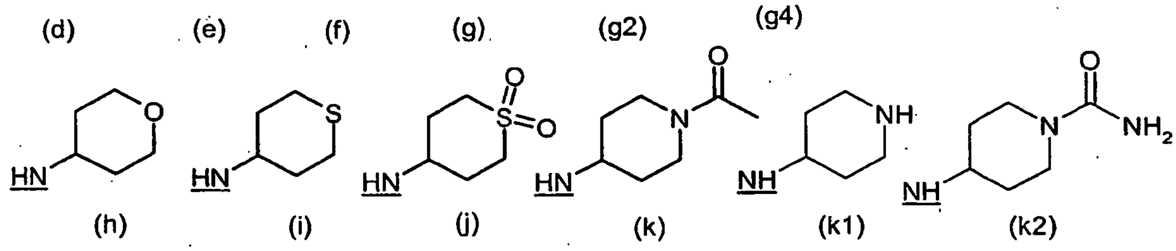

(h) (i) (j) (k) (k1) (k2)

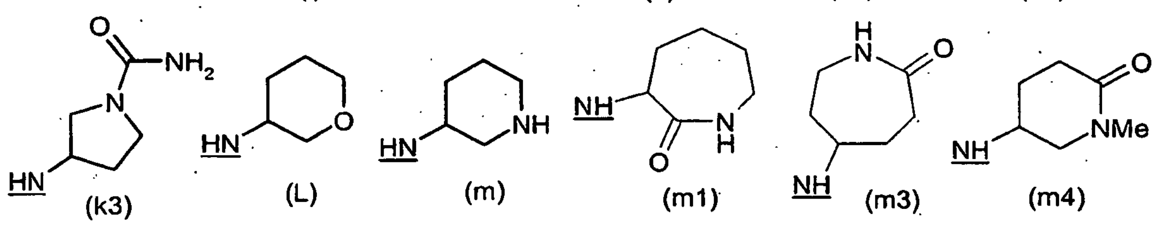

(k3) (L) (m) (m1) (m3) (m4)

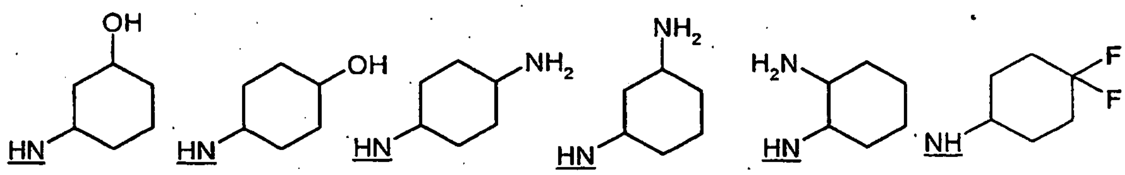

(n) (p) (p1) (p2) (p3) (p5)

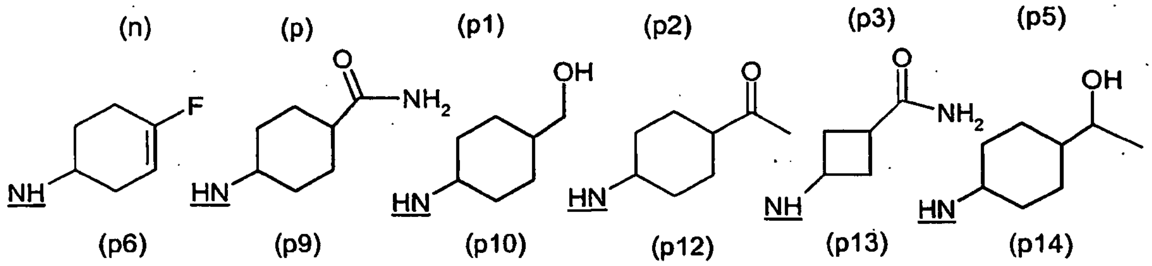

(p6) (p9) (p10) (p12) (p13) (p14)

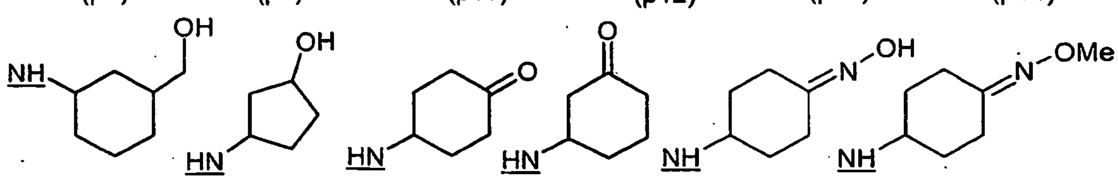

(p15) (q) (o) (o1) (o2) (o3)

In the sub-formulae (a1) to (q) etc above, the -NH- connection point of the $NHR^3$ group to the 4-position of the pyrazolopyridine of formula (I) is underlined.

Preferably, $NHR^3$ is of sub-formula (c), (c1), (c 2), (c 3), (c 4), (c 5), (c 6), (c 7), (d), (e), (f), (g4), (h), (i), (j), (k), (k1), (k2), (k3), (L), (m), (m1), (m3), (m4), (n), (o), (o1), (o2), (o3), (p), (p2), (p5), (p6), (p9), (p10), (p12), (p13), (p14), (p15) or (q); or preferably $NHR^3$ is of sub-formula (a1), (c), (c1), (c 2), (c 3), (c 4), (c 5), (c 6), (c 7), (d), (e), (f), (g4), (h), (i), (j), (k), (k1), (k2), (k3), (L), (m), (m1), (m3), (m4), (n), (o), (o1), (o2), (o3), (p), (p1), (p2), (p5), (p6), (p9), (p10), (p12), (p13), (p14), (p15) or (q).

More preferably, $NHR^3$ is of sub-formula (c), (c1), (c 4), (c 5), (h), (i), (k), (k2), (k3), (m1), (n), (o), (o2), (o3), (p2), (p5), (p6), (p9), (p10), (p13) or (p15).

$NHR^3$ is more preferably of sub-formula (c), (h), (k), (k2), (k3), (n), (o), (o2), (p9) or (p13); still more preferably $NHR^3$ is (c), (h), (k2), (k3), (n), (o), (o2), (p9) or (p13).

Most preferably, $R^3$ is tetrahydro-2H-pyran-4-yl or 1-(aminocarbonyl)-4-piperidinyl; that is $NHR^3$ is most preferably of sub-formula (h) or (k2), as shown above, in particular of sub-formula (h).

When $NHR^3$ is of sub-formula (n), then it can be in the *trans* configuration. But preferably it is in the *cis* configuration, i.e. preferably it is a *cis*-(3-hydroxycyclohexan-1-yl)amino group (including mixtures of configurations wherein the *cis* configuration is the major component), or it is racemic.

When $NHR^3$ isof sub-formula (p9), then it can be in the *trans* configuration. But preferably it is in the *cis* configuration, i.e. preferably it is a *cis*-[4-(aminocarbonyl)cyclohexan-1-yl]amino group (including mixtures of configurations wherein the *cis* configuration is the major component), or it is racemic.

When $NHR^3$ is of sub-formula (p12), then it can be in the *trans* configuration. But, preferably, it is in the *cis* configuration, i.e. preferably $NHR^3$ is a *cis*-[4-acetylcyclohexan-1-yl]amino group (including mixtures of configurations wherein the *cis* configuration is the major component), or it is racemic.

When $NHR^3$ is of sub-formula (p13), then it can be in the *trans* configuration. But, preferably, it is in the *cis* configuration, i.e. preferably $NHR^3$ is a *cis*-[3-(aminocarbonyl)cyclobutan-1-yl]amino group (including mixtures of configurations wherein the *cis* configuration is the major component), or it is racemic.

The $NHR^3$ group of sub-formula (p10), (p14) or (p15), independently, can for example be racemic; or it can be in the *cis* configuration with respect to the ring (including mixtures of configurations wherein the *cis* configuration is the major component).

Preferably, $R^b$ is a hydrogen atom (H).

More preferably, $R^a$ is a hydrogen atom (H) and $R^b$ is a hydrogen atom (H).

$R^4$ can suitably be a hydrogen atom (H), methyl (Me) or ethyl.

Preferably, $R^4$ is a hydrogen atom (H).

When $R^4$ is -C(O)-$C_1$fluoroalkyl then it can for example be -C(OFC$F_3$.

The invention involving $R^5$, and optionally $R^4$, can have two separable embodiments.

In a first separable embodiment, $R^5$ is:

- C(O)-$(CH_2)_n$-Ar, -C(O)-Het, -C(O)-$C_{1-6}$alkyl; -C(O)-$C_1$fluoroalky),
- C(O)-$(CH_2)_2$-C(O)-$NR^{15b}NR^{15b}$, -C(O)-$CH_2$-$C_{(O)}$-$NR^{15b}NR^{15b}$,
- C(O)-$NR^{15b}$-$(CH_2)_m{}^1$-Ar, -C(O)-$NR^{15b}$-Het, -C(O)-$NR^{15b}$-$C_{1-6}$alkyl, -C(O)-$NR^{5a}R^{5b}$,
- $S(O)_2$-$(CH_2)_m{}^2$-Ar, -$S(O)_2$-Het, or -$S(O)_2$-$C_{1-6}$alkyl;

or $R^4$ and $R^5$ taken together are -C(O)-$(CH_2)p^2$- or -C(O)-N($R^{15}$)-$(CH_2)p^3$-, in which:

$p^2$ is 3, 4 or 5 (e.g. 3 or 4), and $p^3$ is 2 or 3 (e.g. 2);
or $NR^4R^5$ is of sub-formula (y), (y1), (y2) or (y3) as herein described, wherein $p^4$ is 1 or 2 (e.g.1).

In a second separable embodiment, $R^5$ is -$CH_2$-Ar;
or $R^4$ and $R^5$ taken together are -$(CH_2)p^1$- (optionally substituted), or -$(CH_2)_2$-$X^5$-$(CH_2)_2$-, in which: $X^5$ is $NR^{17}$ and $p^1$ is 4, 5 or 6 (e.g. 4 or 5).

In one embodiment of the invention, $R^5$ is:

- C(O)-$(CH_2)_n$-Ar, -C(O)-Het, -C(O)-$C_{1-6}$alkyl, -C(O)-$C_1$fluoroalkyl,
- C(O)-$(CH_2)_2$-C(O)-$NR^{15b}NR^{15b}$, -C(O)-$NR^{15b}$-$(CH_2)_m{}^1$-Ar, -C(O)-$NR^{15b}$-Het,
- C(O)-$NR^{5a}R^{5b}$, -$S(O)_2$-$(CH_2)_m{}^2$-Ar, -$S(O)_2$-$C_{1-6}$alkyl, or -$CH_2$-Ar;

and optionally $R^4$ is a hydrogen atom (H);
or $R^4$ and $R^5$ taken together are -$(CH_2)_p{}^{1-}$ (optionally substituted), or -$(CH_2)_2$-$X^5$-$(CH_2)_2$-, or -C(O)-$(CH_2)_p{}^2$-, or -C(O)-N($R^{15}$)-$(CH_2)_p{}^3$-;

or $NR^4R^5$ is of sub-formula (y), (y1), (y2) or (y3).

In one particular embodiment, $R^5$ is:

$-C(O)-(CH_2)_n-Ar$, -C(O)-Het, $-C(O)-C_{1-6}alkyl$, $-C(O)-C_1fluoroalkyl$,
$-C(O)-NR^{15b}-(CH_2)_m{}^1-Ar$, $-C(O)-NR^{5a}R^{5b}$,
or $-S(O)_2-(CH_2)_m{}^2-Ar$;

and optionally $R^4$ is a hydrogen atom (H);
or $R^4$ and $R^5$ taken together are $-(CH_2)p^1-$ (optionally substituted), or $-(CH_2)_2-X^5-(CH_2)_2-$, or $-C(OP)-(CH_2)_p{}^2-$, or $-C(O)-N(R^{15})-(CH_2)_p{}^3-$;
or $NR^4R^5$ is of sub-formula (y), (y1), (y2) or (y3).

Suitably, $R^5$ is:

- $C(O)-(CH_2)_n-Ar$, -C(O)-Het, $-C(O)-C_{1-6}alkyl$; $-C(O)-C_1fluoroalkyl$,
- $C(O)-NR^{15b}-(CH_2)_m{}^1-Ar$, or $-C(O)-NR^{5a}R^{5b}$;

(in one preferable embodiment of which, $R^4$ is a hydrogen atom (H));
or $R^4$ and $R^5$ taken together are $-C(O)-N(R^{15})-(CH_2)p^3-$;
or $NR^4R^5$ is of sub-formula (y), (y1), (y2) or (y3).

Preferably, $R^5$ is:

$-C(O)-(CH_2)_n-Ar$, -C(O)-Het, $-C(O)-NR^{15b}-(CH_2)_m{}^1-Ar$, or $-C(O)-NR^{5a}R^{5b}$;

(in one preferable embodiment of which, $R^4$ is a hydrogen atom (H));
or $R^4$ and $R^5$ taken together are $-C(O)-N(R^{15})-(CH_2)_p{}^3-$;
or $NR^4R^5$, is of sub-formula (y), (y1), (y2) or (y3).

More preferably, $R^5$ is $-C(O)-(CH_2)_n-Ar$ (in one preferable embodiment of which, $R^4$ is a hydrogen atom (H)); or $NR^4R^5$ is of sub-formula (y), (y1) or (y2).

In particular, $R^5$ can preferably be $-C(O)-(CH_2)_n-Ar$. In one preferable embodiment of this, $R^4$ is a hydrogen atom (H).

n can for example be 0 or 1. n is preferably 0.

$m^1$ can for example be 0 or 1. $m^1$ is preferably 0.

$m^2$ can for example be 0 or 1, such as $m^2$ being 0.

When $R^5$ is $-C(O)-C_1fluoroalkyl$, then it can for example be $-C(O)-CF_3$.

$p^1$ can for example be 4 or 5. Suitably, $p^1$ is 5.

$p^2$ can for example be 3 or 4.

When $R^4$ and $R^5$ taken together are $-C(O)-N(R^{15})-(CH_2)p^3-$, then $p^3$ can for example be 2. In this case, $NR^4R^5$ can for example be

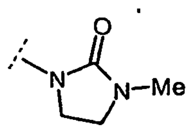

$p^4$ can for example be 1.

When $R^4$ and $R^5$ taken together are $-(CH_2)p^1-$ (optionally substituted), then the $NR^4R5$ ring is preferably optionally substituted on the 4-position ring-carbon atom (wherein the ring-nitrogen of $NR^4R^5$ is the 1-position) by the one or two independent substituents (e.g. one substituent) as herein defined.

Preferably, when $R^4$ and $R^5$ taken together are $-(CH_2)p^1-$ (optionally substituted), then the $NR^4R^5$ ring is optionally substituted on the 3-position ring-carbon atom and/or (preferably) on the 4-position ring-carbon atom (wherein the ring-nitrogen of $NR^4R^5$ is the 1-position) by one substituent being:

phenyl; phenyl substituted by one or two substituents independently being (e.g. one substituent being) methyl or fluoro or chloro or methoxy; phenyl-C(O)-;
benzyloxy; phenyloxy; phenyloxy whose phenyl ring is substituted by one or two substituents independently being (e.g. one substituent being)methyl or fluoro or chloro or methoxy;
a 5-membered heteroaromatic ring being oxazolyl, isoxazolyl, thiazolyl, 1,2,4-oxadiazolyl, 1;3,4-oxadiazolyl, furanyl,

thienyl, pyrrolyl, pyrazolyl, imidazolyl, triazolyl or tetrazolyl, wherein the 5-membered heteroaromatic ring is optionally substituted on a ring carbon by one substituent being methyl or ethyl or isopropyl or phenyl or pyridinyl and/or is optional substituted on a ring nitrogen by one substituent being methyl or t-butyl; or

a 6-membered heteroaromatic ring being pyridinyl, pyrimidinyl, pyrazinyl or pyridazinyl, wherein the 6-membered heteroaromatic ring is not substituted on a ring carbon;

or alternatively when $R^4$ and $R^5$ taken together is $-(CH_2)_p{}^1-$, then the $NR^4R^5$ ring is optionally substituted on the 3-position and 4-position ring-carbon atoms by two substituents which when taken together are =CH-CH=CH-CH=;

When $R^4$ and $R^5$ taken together are $-(CH_2)_2-X^5-(CH_2)_2-$, then $X^5$ is $NR^{17}$.

Suitably, $R^{17}$, independent of other $R^{17}$, is:

-C(O)-cyclopropyl; -S(O)2-$C_{1-4}$alkyl (e.g. $-S(O)_2Me$ or $-S(O)_2Et$);

-C(O)-$Ar^{176}$; -C(O)-$Ar^{175}$;-$S(O)_2$-$Ar^{176}$; -$S(O)_2$-$Ar^{175}$; $Ar^{176}$; $Ar^{175}$;

-C(O)-phenyl or -$S(O)_2$-phenyl, wherein, independently, the phenyl ring is unsubstituted or substituted by one or two (e.g. one) substituents independently being methyl or ethyl or $CF_3$ or fluoro or chloro or methoxy or difluoromethoxy or cyano (-CN) or $-C(O)NH_2$ or -C(O)-Me;

phenyl; phenyl substituted by one or two (e.g. one) substituents independently being methyl or ethyl or $CF_3$ or fluoro or chloro or methoxy or difluoromethoxy or cyano (-CN) or $-C(O)NR^{7a}R^{8a}$ (e.g. $-C(O)NH_2$) or -C(O)-Me;

or benzyl.

Suitably, $Ar^{175}$ is a 5-membered heteroaromatic ring being oxazolyl, isoxazolyl, thiazolyl, 1,2,4-oxadiazolyl, 1,3,4-oxadiazolyl, furanyl, thienyl, pyrrolyl, pyrazolyl, imidazolyl, triazolyl or tetrazolyl,
wherein the 5-membered heteroaromatic ring $Ar^{175}$ is optionally substituted on a ring carbon by one substituent being methyl and/or is optionally substituted on a ring nitrogen by one substituent being methyl;
or wherein the 5-membered heteroaromatic ring $Ar^{175}$ is optionally fused to a phenyl ring wherein the connection point to the remainder of the molecule is within the 5-membered ring.

Suitably, $Ar^{176}$ is a 6-membered heteroaromatic ring being 2-pyridinyl, 3-pyridinyl, 4-pyridinyl, 2-pyrimidinyl, 5-pyrimidinyl, pyrazinyl or 5-pyridazinyl, wherein the 6-membered heteroaromatic ring is optionally substituted on a ring carbon either by one methyl substituent or by one OH substituent which is substituted on a ring carbon bonded to a ring nitrogen (including the keto tautomer thereof).

When $NR^4R^5$ is of sub-formula (y), (y1), (y2) or (y3), then $p^4$ can for example be 1.

Suitably, when $NR^4R^5$ is of sub-formula (y), then it can for example be:

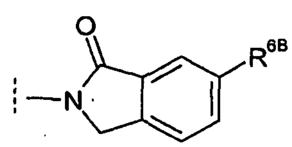

,

such as

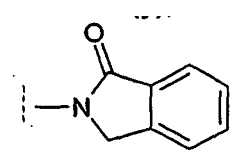

or

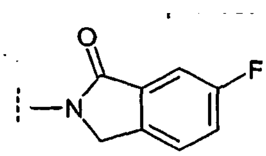

or

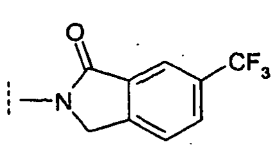

; or

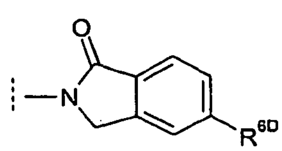

.

Suitably, when $NR^4R^5$ is of sub-formula (y1), (y2) or (y3), then it can for example be:

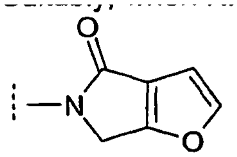

or

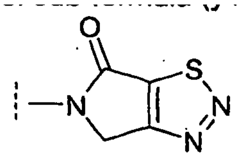

(examples of (y1) and (y2) respectively).

In one embodiment, Ar has the sub-formula (x).

In sub-formula (x), two or more (suitably three or more) of A, B, D, E and F can be independently C-H (carbon-hydrogen), C-F (carbon-fluorine) or nitrogen (N).

Suitably, in sub-formula (x), three or more of A, B, D, E and F are independently C-H (carbon-hydrogen), C-F (carbon-fluorine), or nitrogen (N).

Preferably, in sub-formula (x), two or more (e.g. three or more) of A, B, D, E and F are independently C-H (carbon-hydrogen), C-F (carbon-fluorine), or nitrogen (N); and one or more (e.g. two or more) others of A, B, D, E and F are independently C-H (carbonhydrogen), C-F (carbon-fluorine), C-Cl (carbon-chlorine), C-Me, C-OMe, or nitrogen (N). More preferably, in sub-formula (x), two or more (e.g. three or more) of A, B, D, E and F are C-H (carbon-hydrogen); and one or more (e.g. two or more) others of A, B, D, E and F are independently C-H (carbon-hydrogen), C-F (carbon-fluorine), C-Cl (carbon-chlorine), C-Me, C-OMe, or nitrogen (N).

Preferably, in sub-formula (x), two or more (e.g. three or more, e.g. four or more) of A, B, D, E and F are C-H.

In one embodiment, in sub-formula (x), no more than one (e.g. none) of A, B, D, E and F are nitrogen.

Suitably, sub-formula (x) is sub-formula (x1), (x2), (x3), (x3a), (x4), (x5), (x6), (x7), (x8), (x9), (x10), (x11), (x12), (x12a), (x12b), (x12c), (x12d), (x13), (x14), (x15) or (x16):

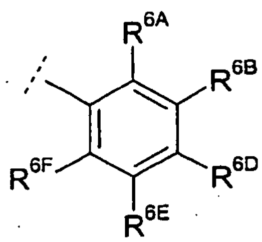

(x1)

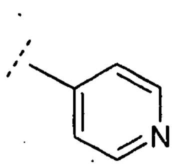

(x2)

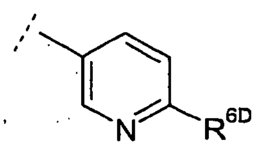

(x3)

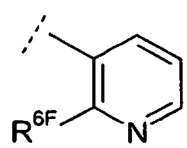

(x3a)

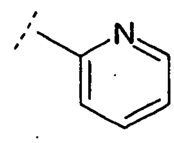

(x4)

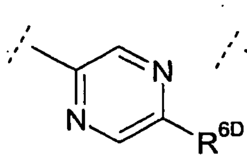

(x5)

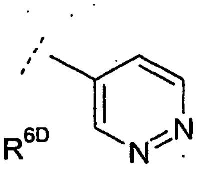

(x6)

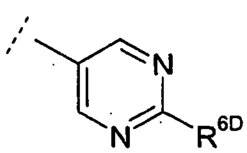

(x7)

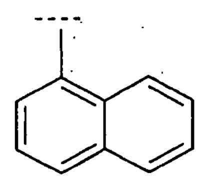

(x8)

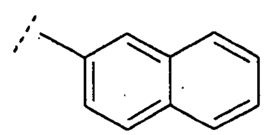

(x9)

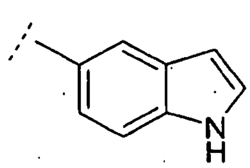

(x10)

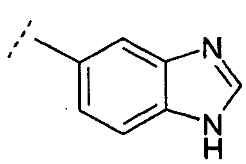

(x11)

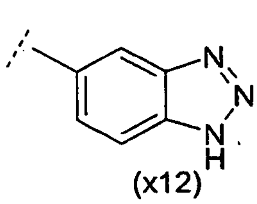

(x12)

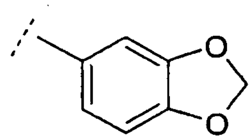

(x12a)

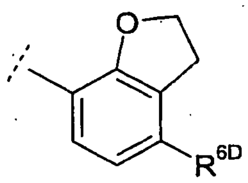

(x12b)

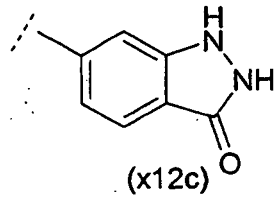

(x12c)

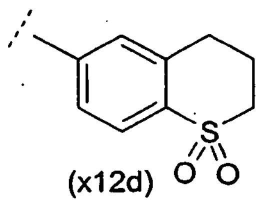

(x12d)

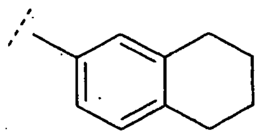

(x13)

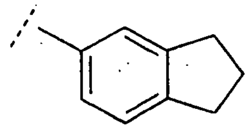

(x14)

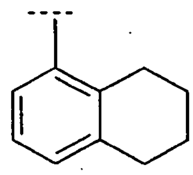

(x15)

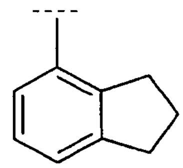

(x16)

In one suitable embodiment, sub-formula (x) is sub-formula (x1), (x3), (x3a), (x5), (x6), (x7), (x8), (x9), (x12b), (x12c), (x12d), (x13) or (x14).

Preferably, sub-formula (x) is sub-formula (X1), (x3), (x3a), (x5), (x6), (x7), (x12b) or (x12c). More preferably, sub-formula (x) is sub-formula (x1), (x3), (x5), (x7) or (x12b). Still more preferably, sub-formula (x) is sub-formula (x1), (x3), (x5) or (x7), such as (x3), (x5) or (x7).

In sub-formula (x), [and/or independently in sub-formulae (y)], preferably, $R^{6A}$, $R^{6B}$, $R^{6D}$, $R^{6E}$ and/or $R^{6F}$, independently of each other, (in particular $R^{6B}$, $R^{6D}$ and/or $R^{6E}$, independently of each other, e.g. $R^{6D}$) is or are: a hydrogen atom (H), a fluorine, chlorine, bromine or iodine atom, methyl, ethyl, n-propyl, isopropyl, $C_4$alkyl such as t-butyl, $C_1$fluoroalkyl such as trifluoromethyl, cyclohexyl, cyclopentyl, cyclopropyl, $-CH_2OH$, methoxy, ethoxy, n-propoxy, isopropoxy, $C_1$fluoroalkoxy (e.g. trifluoromethoxy or difluoromethoxy), cyclohexyloxy; cyclopentyloxy; nitro ($-NO_2$), OH, $C_{1-3}$alkylS(O)$_2$-such as MeS(O)$_2$-, Pus(O)2-, $C_{1-3}$alkylS(O)$_2$-NH- such as Me-S(O)$_2$-NH-, ((cyclopropyl)methyl)NH-S(O)$_2$-, $Et_2$N-S(O)$_2$-, $Me_2$N-S(O)$_2$-, $H_2$N-S(O)$_2$-, -C(O)$NH_2$, -C(O)NHMe, -C(O)NHEt, -C(O)$Me_2$, -C(O)$NEt_2$, -NHC(O)$C_{1-3}$alkyl (such as -NHC(O)Me, -NHC(O)Et or -NHC(O)-$^i$Pr), -NHC(O)$CF_3$, -C(O)OH, cyano (-CN), $NMe_2$, 1-piperidinyl, 1-pyrrolidinyl, 2-oxo-1-pyrrolidinyl, N-morpholino (4-morpholinyl), or $C_{1-2}$alkyl-S(O)$_2$-$CH_2$- such as Me-S(O)$_2$-$CH_2$-;
provided that $R^{6A}$, $R^{6B}$, $R^{6D}$, $R^{6E}$ and $R^{6F}$ independently cannot be a chlorine, bromine or iodine atom (and optionally not a fluorine atom) when substituted on a ring-carbon which is bonded to a ring-nitrogen.

More preferably, $R^{6A}$, $R^{6B}$, $R^{6D}$, $R^{6E}$ and/or $R^{6F}$, independently of each other (in particular $R^{6B}$, $R^{6D}$ and/or $R^{6E}$, independently of each other, e.g. $R^{6D}$), is or are: a hydrogen atom (H), a fluorine, chlorine or bromine atom, methyl, ethyl, n-propyl, isopropyl, $C_4$alkyl such as t-butyl, trifluoromethyl, cyclopentyl, $-CH_2OH$, methoxy, ethoxy, n-propoxy, isopropoxy, $C_1$fluoroalkoxy (e.g. trifluoromethoxy or difluoromethoxy), nitro ($-NO_2$), OH, $C_{1-3}$alkylS(O)$_2$- such as MeS(O)$_2$-, Me-S(O)$_2$-NH-, -C(O)$NH_2$, -C(O)NHMe, -NHC(O)Me, cyano (-CN), $NMe_2$, 1-piperidinyl, 2-oxo-1-pyrrolidinyl, or N-morpholino (4-morpholinyl);
provided that $R^{6A}$, $R^{6B}$, $R^{6D}$, $R^{6E}$ and $R^{6F}$ independently cannot be a chlorine or bromine atom (and optionally not a fluorine atom) when substituted on a ring-carbon which is bonded to a ring-nitrogen.

Still more preferably, $R^{6A}$, $R^{6B}$, $R^{6D}$, $R^{6E}$ and/or $R^{6F}$, independently of each other (in particular $R^{6B}$, $R^{6D}$ and/or $R^{6E}$, independently of each other, e.g. $R^{6D}$), is or are: a hydrogen atom (H), a fluorine or chlorine atom, methyl, ethyl, n-propyl, isopropyl, t-butyl, trifluoromethyl, cyclopentyl, methoxy, ethoxy, n-propoxy, difluoromethoxy, OH, MeS(O)$_2$-, -C(O)$NH_2$, -C(O)NHMe, -NHC(O)Me, cyano (-CN), $NMe_2$, 1-piperidinyl, 2-oxo-1-pyrrolidinyl, or N-morpholino (4-morpholinyl) ;
provided that $R^{6A}$, $R^{6B}$, $R^{6D}$, $R^{6E}$ and $R^{6F}$ independently cannot be a chlorine atom (and optionally not a fluorine atom) when substituted on a ring-carbon which is bonded to a ring-nitrogen.

When (x) is sub-formula (x3), (x5) or (x7), then $R^{6D}$ can for example be H, a fluorine atom, $C_{1-4}$alkyl such as methyl or t-butyl, trifluoromethyl, cyano (-CN), or -NHC(O)Me; in particular H, methyl, t-butyl, trifluoromethyl, or cyano (-CN). When (x) is sub-formula (x12b), then $R^{6D}$ can for example be H, methyl or -NHC(O)Me.

When two adjacent groups selected from $R^{6A}$, $R^{6B}$, $R^{6D}$, $R^{6E}$ and $R^{6F}$ are taken together, then, preferably, when taken together they are: -CH=CH-CH=CH-, $-(CH_2)_n{}^{14a}$- where $n^{14a}$ is 3, 4 or 5, $-O-(CH_2)_n{}^{14c}$- or $-S(O)_2-(CH_2)_n{}^{14d}$- where $n^{14c}$ and $n^{14d}$ independently are 2 or 3, or -NH-NH-C(O)-. Suitably, in this embodiment, two adjacent groups selected from $R^{6A}$, $R^{6B}$, $R^{6D}$, $R^{6E}$ and $R^{6F}$ can be taken together and can be $-O-(CH_2)_n{}^{14c}$- or $-S(O)_2-(CH_2)_n{}^{14d}$- where

$n^{14c}$ and $n^{14d}$ independently are 2 or 3, or -NH-NH-C(O)-. In the invention, $n^{14a}$ can e.g. be 3 or 4; and/or $n^{14c}$ can e.g. be 2, and/or $n^{14d}$ can e.g. be 3.

In sub-formula (x), e.g. in sub-formula (x1), [and/or independently in sub-formulae (y)], suitably, one, two or three of $R^{6B}$, $R^{6D}$ and $R^{6E}$ are other than a hydrogen atom (H).

In sub-formula (x), e.g. in sub-formula (x1), suitably, one or both of $R^{6A}$ and $R^{6F}$ are independently a hydrogen atom (H), a fluorine atom (F), or methyl; and/or one of $R^{6A}$ and $R^{6F}$ is OH, methoxy or ethoxy or a fluorine atom (F). For example, one or both of $R^{6A}$ and $R^{6F}$ can be a hydrogen atom (H), and/or one of $R^{6A}$ and $R^{6F}$ can be OH or methoxy or a fluorine atom (F).

In sub-formula (x), e.g. in sub-formula (x1), suitably the ring or ring system is unsubstituted, monosubstituted, disubstituted or trisubstituted; or suitably the ring or ring system is unsubstituted, monosubstituted or disubstituted; for example monosubstituted or disubstituted. In sub-formula (x), e.g. in sub-formula (x1), for monosubstitution of the ring or ring system, then the one substituent selected from $R^{6A}$, $R^{6B}$, $R^{6D}$, $R^{6E}$ and $R^{6F}$ is suitably present at the 3- or 4-position with respect to the connection point (i.e., for a 4-position substituent, D is $CR^{6D}$ where $R^{6D}$ is other than H), or is a 2-methyl, 2-methoxy, 2-OH, or 2-fluoro substituent. In sub-formula (x), e.g. in sub-formula (x1), for disubstitution of the ring or ring system, then 3,4-disubstitution, 2,4-disubstitution, 2,3-disubstitution or 3,5-disubstitution is one possibility. In sub-formula (x), 2,5-disubstitution is also one possibility.

In one suitable embodiment, Ar has the sub-formula (x1) and is: phenyl, monoalkyl-phenyl-, mono(fluoroalkyl)-phenyl-, monohalo-phenyl-, monoalkoxy-phenyl-, mono(fluoroalkoxy)-phenyl-, mono(N,N-dimethylamino)-phenyl-, mono(methyl-$SO_2$-NH-)-phenyl-, mono(methyl-$SO_2$-)-phenyl-, dialkyl-phenyl-, monoalkyl-monohalo-phenyl-, mono (fluoroalkyl)-monohalo-phenyl-, dihalo-phenyl-, dihalo-monoalkyl-phenyl-, dihalo-mono(hydroxymethyl)-phenyl- (e.g. 2,3-dichloro-6-(hydroxymethyl)-phenyl-), or dialkoxy-phenyl- such as 3,4-dimethoxy-phenyl-. The substituents can preferably be further defined, as defined in preferable embodiments herein.

In one suitable embodiment, Ar is of sub-formula (x1) and is: monoalkyl-phenyl-, mono(fluoroalkyl)-phenyl-, monohalo-phenyl-, monoalkoxy-phenyl-, mono(fluoroalkoxy)-phenyl-, dialkyl-phenyl-, monoalkyl-monohalo-phenyl-, dihalo-phenyl-or dihalo-monoalkyl-phenyl-.

Suitably, in this embodiment, Ar is:

- mono$C_{1-4}$alkyl-phenyl- or mono$c_{1-3}$alkyl-phenyl- such as 4-$C_{1-4}$alkyl-phenyl- (e.g. 4-$C_{1-3}$alkyl-phenyl-) or 2-$C_{1-2}$alkyl-phenyl-;
- mono$C_1$fluoroalkyl-phenyl- such as 4-$C_1$fluoroalkyl-phenyl-;
- mono$C_{1-3}$alkoxy-phenyl- such as 4-$C_{1-3}$alkoxy-phenyl- or 3-$C_{1-3}$alkoxy-phenyl-;
- mono($C_1$fluoroalkoxy)-phenyl- such as 4-$C_1$fluoroalkoxy-phenyl-;
- di$C_{1-3}$alkyl-phenyl- or di$C_{1-2}$alkyl-phenyl- or dimethyl-phenyl- such as 3,4-dimethyl-phenyl-, 2,4-dimethyl-phenyl-, 3,5-dimethyl-phenyl-, 2,3-dimethyl-phenyl- or 2,5-dimethyl-phenyl-; for example 3,4-dimethyl-phenyl-, 2,4-dimethyl-phenyl-, 2,3-dimethyl-phenyl- or 3,5-dimethyl-phenyl-;
- mono$C_{1-3}$alkyl-monohalo-phenyl-, such as mono$C_{1-2}$alkyl-monohalo-phenyl- and/or mono$C_{1-3}$alkyl-monochloro-phenyl- or mono$C_{1-3}$alkyl-monofluoro-phenyl-, for example 4-methyl-3-chloro-phenyl-, 3-methyl-4-chloro-phenyl-, or 2-methyl-4-chloro-phenyl-;
- dihalo-phenyl- such as 2-chloro-4-fluorophenyl- or 2,4-difluoro-phenyl- or 4-bromo-2-fluorophenyl- or preferably 4-chloro-2-fluorophenyl-; for example dichloro-phenyl- such as 3,4-dichloro-phenyl- or 2,4-dichloro-phenyl- or 2,6-dichloro-phenyl- or preferably 2,3-dichloro-phenyl-; or
- dihalo-mono$C_{1-2}$alkyl-phenyl- e.g. 2,4-dichloro-6-methyl-phenyl-.

In an alternative embodiment, Ar has the sub-formula (x1) and is tri$C_{1-2}$alkyl-phenyl-such as trimethylphenyl-, e.g. 2,4,6-trimethylphenyl-.

In an alternative suitable embodiment, Ar has the sub-formula (z). (z) is connected at a ring carbon.

Preferably, in sub-formula (z), three or more (for example all) of J, L, M and Q are independently C-H, C-F, C-$C_{1-2}$alkyl (e.g. C-Me), C-$C_1$fluoroalkyl (e.g. C-$CF_3$), C-[connection point to formula (I)], or nitrogen (N).

Preferably, in sub-formula (z), no more than two (for example no more than one) of J, L, M and Q are nitrogen (N).

Suitably, either Q or M is C-[connection point to formula (I)].

Suitably, $R^9$ is a hydrogen atom (H) or methyl.

According to one optional embodiment, $R^{6J}$, $R^{6L}$, $R^{6M}$ and/or $R^{6Q}$ independently is or are: a hydrogen atom (H), a halogen atom (e.g. fluoro or chloro); $C_{1-4}$alkyl (e.g. $C_{1-2}$alkyl); $C_{1-2}$fluoroalkyl (e.g. $CF_3$); $C_{3-6}$cycloalkyl; $C_{1-4}$alkoxy (e.g. $C_{1-2}$alkoxy); $C_{1-2}$fluoroalkoxy (e.g. $CF_2HO$-); $C_{3-6}$cycloalkyloxy; OH (including any tautomer thereof); or phenyl optionally substituted by one or two (e.g. one) substituents independently being fluoro, chloro, $C_{1-2}$alkyl, $C_1$fluoroalkyl, $C_{1-2}$alkoxy or $C_1$fluoroalkoxy.

Suitably, $R^{6J}$, $R^{6L}$, R6M and/or $R^{6Q}$ independently is or are: a hydrogen atom (H); fluoro; chloro; $C_{1-2}$alkyl (e.g. methyl); $C_1$fluoroalkyl (e.g. $CF_3$); $C_{1-2}$alkoxy (e.g. methoxy); $C_1$fluoroalkoxy (e.g. $CF_2HO$-); OH (including any tautomer

thereof); or phenyl optionally substituted by one substituent being fluoro, methyl, $C_1$fluoroalkyl (e.g. $CF_3$), methoxy or $C_1$fluoroalkoxy (e.g. $CF_2HO$-).

Preferably, $R^{6J}$, $R^{6L}$, $R^{6M}$ and/or $R^{6Q}$ independently is or are: OH (including any keto tautomer thereof); or more preferably a hydrogen atom (H), $C_{1-2}$alkyl (e.g. methyl) or $C_1$fluoroalkyl (e.g. C-$F_3$).

Sub-formula (z) can suitably be one of the following sub-formulae:

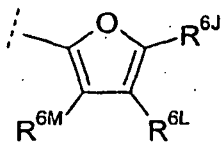 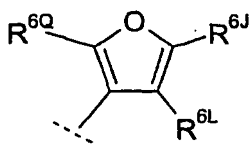 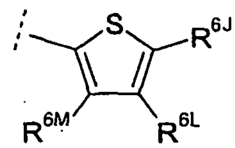 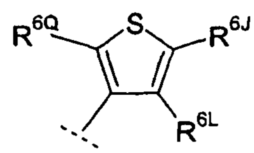

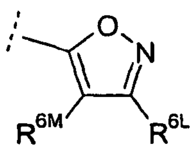 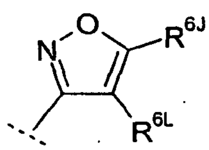 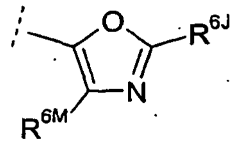 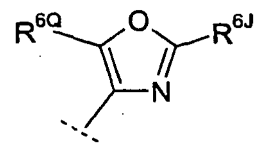

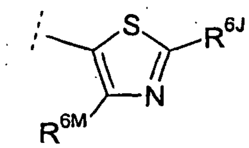 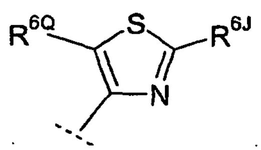 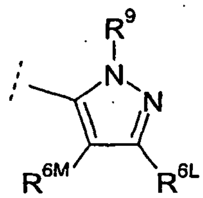 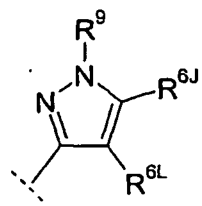

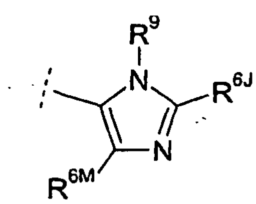 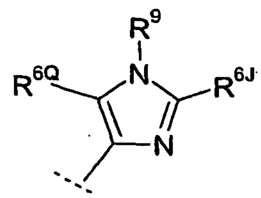

Preferably, sub-formula (z) is sub-formula (z1), (z2), (z3), (z4), (z5), (z6), (z7), (z8), (z9), (z10), (z10a), (z10b), (z11), (z12), (z13), (z14), (z15), (z16), (z17), (z18), (z19), (z20), (z21), (z22), (z23), (z24), (z25), (z26), (z27) or (z28):

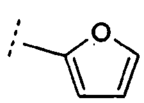
(z1)

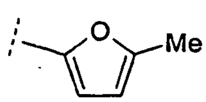
(z2)

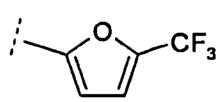
(z3)

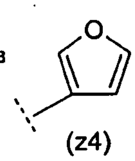
(z4)

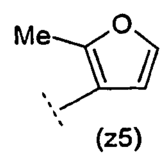
(z5)

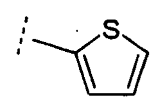
(z6)

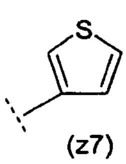
(z7)

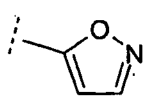
(z8)

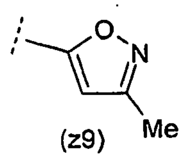
(z9)

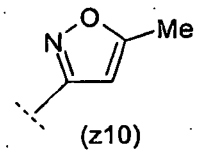
(z10)

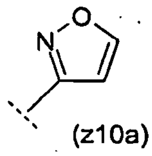
(z10a)

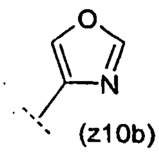
(z10b)

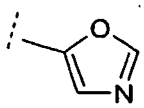 (z11) 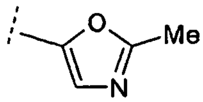 (z12) 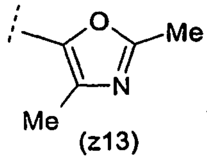 (z13) 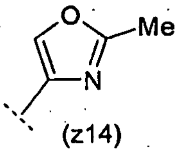 (z14) 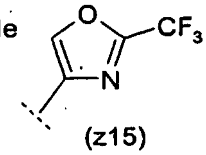 (z15) 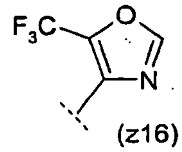 (z16)

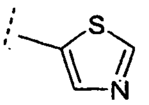 (z17) 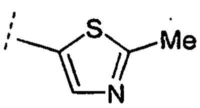 (z18) 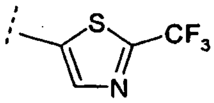 (z19) 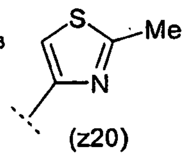 (z20) 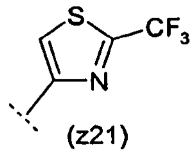 (z21) 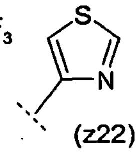 (z22)

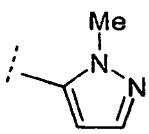 (z23) 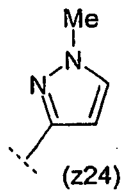 (z24) 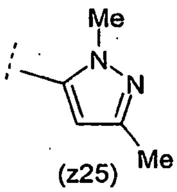 (z25) 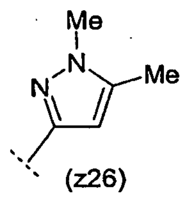 (z26) 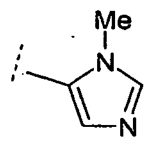 (z27) 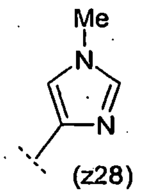 (z28)

Sub-formula (z) can suitably be sub-formula (z1), (z3), (z4), (z5), (z6), (z7), (z8), (z9), (z10), (z11), (z12), (z13), (z14), (z16), (z17), (z18), (z20), (z21), (z23), (z24) or (z25).

Preferably, Sub-formula (z) is sub-formula (z9), (z14), (z18) or (z24).

In one preferable embodiment, $NR^4R^5$ is -NH-C(O)-Ar (i.e. $R^4$ is H and $R^5$ is $-C(O)-(CH_2)_n-Ar$ wherein n is 0); Ar has the sub-formula (z); and Ar is: sub-formula (z1), (z2), (z3), (z4), (z5), (z6), (z7), (z8), (z9), (z10), (z10a), (z10b), (z11), (z12), (z13), (z14), (z15), (z16), (z17), (z18), (z19), (z20), (z21), (z22), (z23), (z24), (z25), or (z26); for example sub-formula (z1), (z3), (z4), (z5), (z6), (z7), (z8), (z9), (z10), (z11), (z12), (z13), (z14), (z16), (z17), (z18), (z20), (z21), (z23), (z24) or (z25); more preferably sub-formula (z9), (z14), (z18) or (z24).

Suitably, $R^7$ and/or $R^8$ are independently a hydrogen atom (H); $C_{1-3}$alkyl (e.g. $C_{1-2}$alkyl such as methyl); $C_{3-6}$cycloalkyl; or phenyl optionally substituted by one or two (e.g. one) substituents independently being: fluoro, chloro, $C_{1-2}$alkyl, $C_1$fluoroalkyl, $C_{1-2}$alkoxy or $C_1$fluoroalkoxy; or $R^7$ and $R^8$ together are $-(CH_2)_n{}^{6-}$, $-C(O)-(CH_2)_n{}^{7}-$ or $-(CH_2)_2-X^7-(CH_2)_2-$ wherein $X^7$ is $NR^{14}$ or suitably O.

When $R^7$ is cycloalkyl or (cycloalkyl)methyl- or optionally substituted phenyl, then preferably $R^8$ is neither cycloalkyl nor (cycloalkyl)methyl- nor optionally substituted phenyl. In this case, $R^8$ can for example be H:

Preferably, $R^7$ and/or $R^8$ independently are a hydrogen atom (H) or $C_{1-2}$alkyl (e.g. H or methyl); or $NR^7R^8$ is 1-piperidinyl, 1-pyrrolidinyl, 2-oxo-1-piperidinyl, 2-oxo-1-pyrrolidinyl, or N-morpholino (4-morpholinyl).

It is preferable that $R^8$ is a hydrogen atom (H).

Suitably, $n^6$ is 4 or 5. Suitably, $n^7$ is 3 or 4. Suitably, $n^{10}$ is 2.

Suitably, $R^{12}$ and/or $R^{13}$ independently are H; $C_{1-3}$alkyl (e.g. $C_{1-2}$alkyl such as methyl); $C_{3-6}$cycloalkyl; (cyclopropyl)methyl-; or phenyl optionally substituted by one or two (e.g. one) substituents, independently being: fluoro, chloro, $C_{1-2}$alkyl, $C_1$fluoroalkyl, $C_{1-2}$alkoxy or $C_1$fluoroalkoxy; or $R^{12}$ and $R^{13}$ together are $-(CH_2)_n{}^{6a}-$ or $-(CH_2)_2-X^{12}-(CH_2)_2-$ in which $X^{12}$ is $NR^{14}$ or suitably O.

When $R^{12}$ is cycloalkyl or (cycloalkyl)methyl- or optionally substituted phenyl, then preferably $R^{13}$ is neither cycloalkyl nor (cycloalkyl)methyl- nor optionally substituted phenyl. In this case, $R^{13}$ can for example be H.

Preferably, $R^{12}$ and/or $R^{13}$ independently are a hydrogen atom (H) or $C_{1-2}$alkyl (e.g. H or methyl); or $NR^{12}R^{12}$ is 1-piperidinyl, 1-pyrrolidinyl, or N-morpholino (4-morpholinyl).

It is preferable that $R^{13}$ is a hydrogen atom (H).

Suitably, $n^{6a}$ is 4 or 5.

In one embodiment of the invention, $NR^7R^8$ and/or $NR^{12}R^{13}$ can for example independently be:

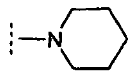, or 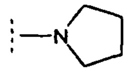, or 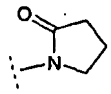

(for $NR^7R^8$ only), or

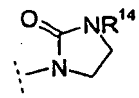

(for $NR^7R^8$ only), or

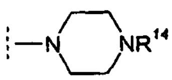

(i.e. $R^{12}$ and $R^{13}$ together or $R^7$ and $R^8$ together are $-(CH_2)_2-N(R^{14})-(CH_2)_2-$), or

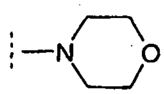

(i.e. $R^{12}$ and $R^{13}$ together or $R^7$ and $R^8$ together are $-(CH_2)_2-O-(CH_2)_2-$), or $NMe_2$.

Suitably, $R^{14}$, independent of other $R^{14}$, is: a hydrogen atom (H); $C_{1-2}$alkyl; $C_1$fluoroalkyl (e.g. $CF_3$); -C(O)Me; $-C(O)NH_2$; or-$S(O)_2$Me. More suitably, $R^{14}$, independent of other $R^{14}$, is H, $C_{1-2}$alkyl, or-C(O)Me; or for example H or $C_{1-2}$alkyl.

Suitably, $R^{7a}$ is H or $C_{1-2}$alkyl, more suitably H or methyl. Suitably, $R^{8a}$ is H.

$R^{15}$, independent of other $R^{15}$, can for example be H, $^t$Bu or $C_{1-2}$alkyl such as methyl. Suitably, $R^{15}$, independent of other $R^{15}$, is H or $C_{1-2}$alkyl (e.g. H or methyl), more preferably H.

$R^{15b}$, independent of other $R^{15b}$, can for example be H or methyl. Preferably, $R^{15b}$, independent of other $R^{15b}$, is H.

Suitably, $R^{16}$, independent of other $R^{16}$, is:

$C_{1-4}$alkyl (e.g. $C_{1-2}$alkyl);
$C_{3-6}$cycloalkyl (e.g. $C_{5-6}$cycloalkyl);
$C_{3-6}$cycloalkyl-$CH_2$- (e.g. $C_{5-6}$cycloalkyl-$CH_2$-);
pyridinyl (e.g. pyridin-2-yl) optionally substituted on a ring carbon atom by one of: a halogen atom, $C_{1-2}$alkyl, $C_1$fluoroalkyl, $C_{1-2}$alkoxy or $C_1$fluoroalkoxy;
$Ar^{16}$;
phenyl optionally substituted by one or two substituents independently being: a halogen atom, $C_{1-2}$alkyl, $C_1$fluoroalkyl, $C_{1-2}$alkoxy or $C_1$fluoroalkoxy;
benzyl optionally substituted on its ring by one or two substituents independently being: a halogen atom, $C_{1-2}$alkyl, $C_1$fluoroalkyl, $C_{1-2}$alkoxy or $C_1$fluoroalkoxy; or
a 5- or 6-membered saturated heterocyclic ring connected at a ring-carbon and containing one or two ring-heteroatoms independently selected from O, S, and N; wherein any ring-nitrogens which are present are present as $NR^{27}$ where $R^{27}$ is H, $C_{1-2}$alkyl or -C(O)Me (preferably H or $C_{1-2}$alkyl); and wherein the ring is not substituted at carbon.

Preferably, $R^{16}$, independent of other $R^{16}$, is: $C_{1-4}$alkyl (e.g. $C_{1-2}$alkyl); $C_{3-6}$cycloalkyl (e.g. $C_{5-6}$cycloalkyl); phenyl optionally substituted by one or two substituents independently being: a halogen atom, $C_{1-2}$alkyl, $C_1$fluoroalkyl, $C_{1-2}$alkoxy or $C_1$ fluoroalkoxy; or benzyl optionally substituted on its ring by one or two substituents independently being: a halogen atom, $C_{1-2}$alkyl, $C_1$fluoroalkyl, $C_{1-2}$alkoxy or $C_1$fluoroalkoxy.

Preferably, $R^{16}$, independent of other $R^{16}$, is $C_{1-4}$alkyl (e.g. $C_{1-2}$alkyl) or phenyl.

Suitably, $R^{30}$, independent of other $R^{30}$, is a hydrogen atom (H) or $C_{1-4}$alkyl, for example H, t-butyl or $C_{1-2}$alkyl.

Suitably, Het, independent of other Het, is a 5- or 6-membered saturated heterocyclic ring. Suitably, the heterocyclic ring Het contains one ring-hetero-atom selected from O, S and N. Suitably, the Het ring is not substituted at a ring carbon , or the Het ring is substituted by one oxo (=O) substituent at a ring-carbon atom which is bonded to a ring-nitrogen wherein $R^{31}$ is H or $C_{1-4}$alkyl. Het can for example be:

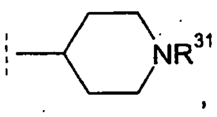 , 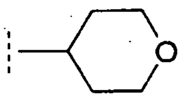 , 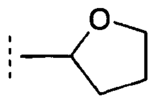 , 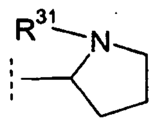 ,

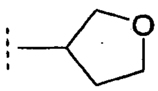 , 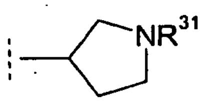 , or 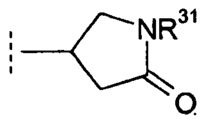 .

$R^{31}$ can for example be H, $C_{1-3}$alkyl (e.g. methyl or isopropyl), -C(O)-$C_{1-3}$alkyl (e.g. -C(O)Me), or -$S(O)_2$-$C_{1-3}$alkyl (e.g. -$S(O)_2$Me).

Suitably, $Het^1$, independent of other $Het^1$, is a 5- or 6-membered saturated heterocyclic ring. Suitably, the heterocyclic ring $Het^1$ contains one ring-hetero-atom selected from O, S and N. Suitably, the $Het^1$ ring is not substituted at a ring carbon, or the $Het^1$ ring is substituted by one oxo (=O) substituent at a ring-carbon atom which is bonded to a ring-nitrogen wherein $R^{31a}$ is H or $C_{1-2}$alkyl. $Fiet^1$ can for example be:

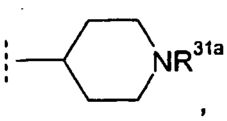 , 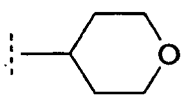 , 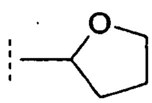 , 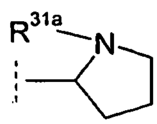 ,

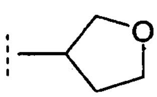 ; 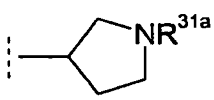 , or 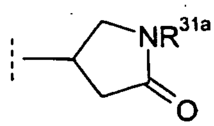 .

$R^{31a}$ can for example be H, methyl or -C(O)Me.

Suitably, $Ar^6$ and/or $Ar^{16}$ can independently be a 5-membered aromatic heterocyclic ring connected at a ring-carbon and containing one O, S or $NR^{15}$ in the 5-membered ring, wherein the 5-membered ring can optionally contain in addition one or two N atoms (e.g. one N atom), and wherein the heterocyclic ring is optionally substituted on a ring carbon atom by one of: $C_{1-2}$alkyl (e.g. methyl) or OH (including any keto tautomer thereof).

The compound of formula (I) or the salt thereof can suitably be:

1-ethyl-6-methyl-5-({4-[3-(3-pyridinyl)-1,2,4-oxad iazol-5-yl]-1-piperidinyl}methyl)-N-(tetrahydro-2H-pyran-4-yl)-1H-pyrazolo[3,4-b]pyridin-4-amine,

1-{[1-ethyl-6-methyl-4-(tetrahydro-2H-pyran-4-ylamino)-1H-pyrazolo[3,4-b]pyridin-5-yl]methyl}-4-phenyl-4-piperidinecarbonitrile,

1-ethyl-6-methyl-5-{[4-(phenylmethyl)-1-piperazinyl]methyl}-N-(tetrahydro-2H-pyran-4-yl)-1H-pyrazolo[3,4-b]pyridin-4-amine,

5-[(3,3-dimethyl-1-piperidinyl)methyl]-1-ethyl-6-methyl-N-(tetrahydro-2H-pyran-4-yl)-1H-pyrazolo[3,4-b]pyridin-4-amine,

1-ethyl-6-methyl-5-{[4-(2-pyrazinyl)-1-piperazinyl]methyl}-N-(tetrahydro-2H-pyran-4-yl)-1H-pyrazolo[3,4-b]pyridin-4-amine,

1-ethyl-6-methyl-5-{[4-(3-methylphenyl)-1-piperazinyl]methyl}-N-(tetrahydro-2H-pyran-4-yl)-1H-pyrazolo[3,4-b]pyridin-4-amine,

1-ethyl-6-methyl-5-{[4-(tetrahydro-2-furanylcarbonyl)-1-piperazinyl]methyl}-N-(tetrahydro-2H-pyran-4-yl)-1H-pyrazolo[3,4-b]pyridin-4-amine,

1-ethyl-6-methyl-5-{[4-(5-phenyl-1,3,4-oxadiazol-2-yl)-1-piperidinyl]methyl}-N-(tetrahydro-2H-pyran-4-yl)-1H-pyrazolo[3,4-b]pyridin-4-amine,

1-ethyl-6-methyl-5-{[4-(2-pyridinylcarbonyl)-1-piperazinyl]methyl}-N-(tetrahydro-2H-pyran-4-yl)-1H-pyrazolo[3,4-b]pyridin-4-amine,
1-ethyl-5-{[4-(5-phenyl-1,3,4-oxadiazol-2-yl)-1-piperidinyl]methyl}-N-(tetrahydro-2H-pyran-4-yl)-1H-pyrazolo[3,4-b]pyridin-4-amine,
1-ethyl-5-{[4-(phenylsulfonyl)-1-piperazinyl]methyl}-N-(tetrahydro-2H-pyran-4-yl)-1H-pyrazolo[3,4-b]pyridin-4-amine,
1-ethyl-5-{[4-(ethylsulfonyl)-1-piperazinyl]methyl}-N-(tetrahydro-2H-pyran-4-yl)-1H-pyrazolo[3,4-b]pyridin-4-amine,
1-ethyl-6-methyl-5-({4-[(phenylmethyl)oxy]-1-piperidinyl}methyl)-N-(tetrahydro-2H-pyran-4-yl)-1H-pyrazolo[3,4-b]pyridin-4-amine,
1-ethyl-5-{[4-(2-pyrazinyl)-1-piperazinyl]methyl}-N-(tetrahydro-2H-pyran-4-yl)-1H-pyrazolo[3,4-b]pyridin-4-amine,
1-ethyl-5-{[4-(3-methylphenyl)-1-piperazinyl]methyl}-N-(tetrahydro-2H-pyran-4-yl)-1H-pyrazolo[3,4-b]pyridin-4-amine,
5-(3,4-dihydro-2(1H)-isoquinolinyimethyl)-1-ethyl-N-(tetrahydro-2H-pyran-4-yl)-1H-pyrazolo[3,4-b]pyridin-4-amine,
1-ethyl-5-{[4-(2-pyridinylcarbonyl)-1-piperazinyl]methyl}-N-(tetrahydro-2H-pyran-4-yl)-1H-pyrazolo[3,4-b]pyridin-4-amine,
1-ethyl-5-({4-[3-(3-pyridinyl)-1,2,4-oxadiazol-5-yl]-1-piperidinyl}methyl)-N-(tetrahydro-2H-pyran-4-yl)-1H-pyrazolo[3,4-b]pyridin-4-amine,
3-(1-{[1-ethyl-4-(tetrahydro-2H-pyran-4-ylamino)-1H-pyrazolo[3,4-b]pyridin-5-yl]methyl}-3-piperidinyl)-1-(4-hydroxyphenyl)-1-propanone,
1-ethyl-6-methyl-5-{[4-(phenylsulfonyl)-1-piperazinyl]methyl}-N-(tetrahydro-2H-pyran-4-yl)-1H-pyrazolo[3,4-b]pyridin-4-amine,
1-ethyl-5-[(4-{1-[3-(methyloxy)phenyl]ethyl}-1-piperazinyl)methyl]-N-(tetrahydro-2H-pyran-4-yl)-1H-pyrazolo[3,4-b]pyridin-4-amine,
1-[4-(4-{[1-ethyl-4-(tetrahydro-2H-pyran-4-ylamino)-1H-pyrazolo[3,4-b]pyridin-5-yl]methyl}-1-piperazinyl)phenyl]ethanone,
1-{[1-ethyl-4-(tetrahydro-2H-pyran-4-ylamino)-1H-pyrazolo[3,4-b]pyridin-5-yl]methyl}-4-phenyl-4-piperidinecarbonitrile,
1-ethyl-5-{[4-(phenylmethyl)-1-piperazinyl]methyl}-N-(tetrahydro-2H-pyran-4-yl)-1H-pyrazolo[3,4-b]pyridin-4-amine,
5-[(3,3-dimethyl-1-piperidinyl)methyl]-1-ethyl-N-(tetrahydro-2H-pyran-4-yl)-1H-pyrazolo[3,4-b]pyridin-4-amine,
1-ethyl-6-methyl-5-{[4-(4-pyridinyl)-1-piperazinyl]methyl}-N-(tetrahydro-2H-pyran-4-yl)-1H-pyrazolo[3,4-b]pyridin-4-amine,
1-ethyl-5-{[4-(tetrahydro-2-furanylcarbonyl)-1-piperazinyl]methyl}-N-(tetrahydro-2H-pyran-4-yl)-1H-pyrazolo[3,4-b]pyridin-4-amine,
5-({4-[(3-chlorophenyl)oxy]-1-piperidinyl}methyl)-1-ethyl-N-(tetrahydro-2H-pyran-4-yl)-1H-pyrazolo[3,4-b]pyridin-4-amine,
1-ethyl-5-({4-[(phenylmethyl)oxy]-1-piperidinyl}methyl)-N-(tetrahydro-2H-pyran-4-yl)-1H-pyrazolo[3,4-b]pyridin-4-amine,
3-(1-{[1-ethyl-6-methyl-4-(tetrahydro-2H-pyran-4-ylamino)-1H-pyrazolo[3,4-b]pyridin-5-yl]methyl}-3-piperidinyl)-1-(4-hydroxyphenyl)-1-propanone,
1-ethyl-5-{[4-(ethylsulfonyl)-1-piperazinyl]methyl}-6-methyl-N-(tetrahydro-2H-pyran-4-yl)-1H-pyrazolo[3,4-b]pyridin-4-amine,
5-({4-[2-(1,1-dimethylethyl)-2H-tetrazol-5-yl]-1-piperidinyl}methyl)-1-ethyl-N-(tetrahydro-2H-pyran-4-yl)-1H-pyrazolo[3,4-b]pyridin-4-amine,
4-(4-{[1-ethyl-4-(tetrahydro-2H-pyran-4-ylamino)-1H-pyrazolo[3,4-b]pyridin-5-yl]methyl}-1-piperazinyl)benzonitrile,
1-ethyl-5-{[4-(4-pyridazinyl)-1-piperazinyl]methyl}-N-(tetrahydro-2H-pyran-4-yl)-1H-pyrazolo[3,4-b]pyridin-4-amine,
4-(4-{[1-ethyl-4-(tetrahydro-2H-pyran-4-ylamino)-1H-pyrazolo[3,4-b]pyridin-5-yl]methyl}-1-piperazinyl)benzamide,
5-{[4-(1,2-benzisoxazol-3-yl)-1-piperazinyl]methyl}-1-ethyl-N-(tetrahydro-2H-pyran-4-yl)-1H-pyrazolo[3,4-b]pyridin-4-amine,
5-[(4-cydopentyl-1-piperazinyl)methyl]-1-ethyl-6-methyl-N-(tetrahydro-2H-pyran-4-yl)-1H-pyrazolo[3,4-b]pyridin-4-amine,
1-[4-(4-{[1-ethyl-6-methyl-4-(tetrahydro-2H-pyran-4-ylamino)-1H-pyrazolo[3,4-b]pyridin-5-yl]methyl}-1-piperazinyl)phenyl]ethanone,
1-ethyl-6-methyl-5-{[4-(2-pyridinyl)-1-piperazinyl]methyl}-N-(tetrahydro-2H-pyran-4-yl)-1H-pyrazolo[3,4-b]pyridin-4-amine,

5-[(4-{[1-ethyl-6-methyl-4-(tetrahydro-2H-pyran-4-ylamino)-1H-pyrazolo[3,4-b]pyridin-5-yl]methyl}-1-piperazinyl) carbonyl]-2(1H)-pyridinone,
5-{[4-(cyclopropylcarbonyl)-1-piperazinyl]methyl}-1-ethyl-6-methyl-N-(tetrahydro-2H-pyran-4-yl)-1H-pyrazolo[3,4-b]pyridin-4-amine,
1-ethyl-6-methyl-5-{[4-(4-pyridazinyl)-1-piperazinyl]methyl}-N-(tetrahydro-2H-pyran-4-yl)-1H-pyrazolo[3,4-b]pyridin-4-amine,
5-{[4-(1,2-benzisoxazol-3-yl)-1-piperazinyl]methyl}-1-ethyl-6-methyl-N-(tetrahydro-2H-pyran-4-yl)-1H-pyrazolo [3,4-b]pyridin-4-amine,
1-ethyl-6-methyl-5-{[4-(2-methylphenyl)-1-piperazinyl]methyl}-N-(tetrahydro-2H-pyran-4-yl)-1H-pyrazolo[3,4-b]pyridin-4-amine,
1-ethyl-5-{[methyl(3-pyridinylmethyl)amino]methyl}-N-(tetrahydro-2H-pyran-4-yl)-1H-pyrazolo[3,4-b]pyridin-4-amine,
1-ethyl-5-{[4-(2-furanylcarbonyl)-1-piperazinyl]methyl}-N-(tetrahydro-2H-pyran-4-yl)-1H-pyrazolo[3,4-b]pyridin-4-amine,
8-{[1-ethyl-4-(tetrahydro-2H-pyran-4-ylamino)-1H-pyrazolo[3,4-b]pyridin-5-yl]methyl}-3-(phenylmethyl)-1-oxa-3,8-diazaspiro[4.5]decan-2-one,
1-ethyl-5-[(4-phenyl-1-piperidinyl)methyl]-N-(tetrahydro-2H-pyran-4-yl)-1H-pyrazolo[3,4-b]pyridin-4-amine,
(1-{[1-ethyl-4-(tetrahydro-2H-pyran-4-ylamino)-1H-pyrazolo[3,4-b]pyridin-5-yl]methyl}-4-piperidinyl)(phenyl)methanone,
1-ethyl-5-({4-[2-oxo-2-(1-pyrrolidinyl)ethyl]-1-piperazinyl}methyl)-N-(tetrahydro-2H-pyran-4-yl)-1H-pyrazolo[3,4-b] pyridin-4-amine,
1-ethyl-5-{[3-(1H-pyrrol-1-ylmethyl)-1-piperidinyl]methyl}-N-(tetrahydro-2H-pyran-4-yl)-1H-pyrazolo[3,4-b]pyridin-4-amine,
1-ethyl-6-methyl-5-{[methyl(3-pyridinylmethyl)amino]methyl}-N-(tetrahydro-2H-pyran-4-yl)-1H-pyrazolo[3,4-b]pyridin-4-amine,
1-ethyl-5-{[4-(2-furanylcarbonyl)-1-piperazinyl]methyl}-6-methyl-N-(tetrahydro-2H-pyran-4-yl)-1H-pyrazolo[3,4-b] pyridin-4-amine,
8-{[1-ethyl-6-methyl-4-(tetrahydro-2H-pyran-4-ylamino)-1H-pyrazolo[3,4-b]pyridin-5-yl]methyl}-3-(phenylmethyl)-1-oxa-3,8-diazaspiro[4.5]decan-2-one,
1-ethyl-6-methyl-5-[(4-phenyl-1-piperidinyl)methyl]-N-(tetrahydro-2H-pyran-4-yl)-1H-pyrazolo[3,4-b]pyridin-4-amine,
1-ethyl-6-methyl-5-{[4-(2-pyrimidinyl)-1-piperazinyl]methyl}-N-(tetrahydro-2H-pyran-4-yl)-1H-pyrazolo[3,4-b]pyridin-4-amine,
1-ethyl-5-({[(5-methyl-3-phenyl-4-isoxazolyl)methyl]amino}methyl)-N-(tetrahydro-2H-pyran-4-yl)-1H-pyrazolo[3,4-b]pyridin-4-amine,
5-[({[4-(dimethylamino)phenyl]methyl}amino)methyl]-1-ethyl-6-methyl-N-(tetrahydro-2H-pyran-4-yl)-1H-pyrazolo [3,4-b]pyridin-4-amine,
(1-{[1,6-diethyl-4-(tetrahydro-2H-pyran-4-ylamino)-1H-pyrazolo[3,4-b]pyridin-5-yl]methyl}-4-piperidinyl)(phenyl) methanone,
1,6-diethyl-5-{[(phenylmethyl)amino]methyl}-N-(tetrahydro-2H-pyran-4-yl)-1H-pyrazolo[3,4-b]pyridin-4-amine,
5-[({[4-(dimethylamino)phenyl]methyl}amino)methyl]-1,6-diethyl-N-(tetrahydro-2H-pyran-4-yl)-1H-pyrazolo[3,4-b] pyridin-4-amine,
5-[({[4-(dimethylamino)phenyl]methyl}amino)methyl]-1-ethyl-N-(tetrahydro-2H-pyran-4-yl)-1H-pyrazolo[3,4-b]pyridin-4-amine,
1-ethyl-5-{[(phenylmethyl)amino]methyl}-N-(tetrahydro-2H-pyran-4-yl)-1H-pyrazolo[3,4-b]pyridin-4-amine,
1-ethyl-6-methyl-5-{[(phenylmethyl)amino]methyl}-N-(tetrahydro-2H-pyran-4-yl)-1H-pyrazolo[3,4-b]pyridin-4-amine,
N-{[1-ethyl-4-(tetrahydro-2H-pyran-4-ylamino)-1H-pyrazolo[3,4-b]pyridin-5-yl]methyl}benzamide,
4-(dimethylamino)-N-{[1-ethyl-4-(tetrahydro-2H-pyran-4-ylamino)-1H-pyrazolo[3,4-b]pyridin-5-yl]methyl}benzamide,
N-{[1-ethyl-4-(tetrahydro-2H-pyran-4-ylamino)-1H-pyrazolo[3,4-b]pyridin-5-yl]methyl}benzenesulfonamide,
N-{[1-ethyl-4-(tetrahydro-2H-pyran-4-ylamino)-1H-pyrazolo[3,4-b]pyridin-5-yl]methyl}-5-isoxazolecarboxamide,
N-{[1,6-diethyl-4-(tetrahydro-2H-pyran-4-ylamino)-1H-pyrazolo[3,4-b]pyridin-5-yl]methyl}-5-isoxazolecarboxamide, '
N-{[1-ethyl-4-(tetrahydro-2H-pyran-4-ylamino)-1H-pyrazolo[3,4-b]pyridin-5-yl]methyl}-4-pyridinecarboxamide,
4-(acetylamino)-N-{[1-ethyl-6-methyl-4-(tetrahydro-2H-pyran-4-ylamino)-1H-pyrazolo[3,4-b]pyridin-5-yl]methyl} benzamide,
N-{[1-ethyl-6-methyl-4-(tetrahydro-2H-pyran-4-ylamino)-1H-pyrazolo[3,4-b]pyridin-5-yl]methyl}-5-isoxazolecarboxamide,
N-{4-[({[1-ethyl-6-methyl-4-(tetrahydro-2H-pyran-4-ylamino)-1H-pyrazolo[3,4-b]pyridin-5-yl]methyl}amino)sulfonyl]phenyl}acetamide,
N-{[1-ethyl-6-methyl-4-(tetrahydro-2H-pyran-4-ylamino)-1H-pyrazolo[3,4-b]pyridin-5-yl]methyl}ethanesulfonamide,
N-{[1-ethyl-6-methyl-4-(tetrahydro-2H-pyran-4-ylamino)-1H-pyrazolo[3,4-b]pyridin-5-yl]methyl}-2-(4-fluorophenyl)ethanesulfonamide,
N-{[1-ethyl-6-methyl-4-(tetrahydro-2H-pyran-4-ylamino)-1H-pyrazolo[3,4-b]pyridin-5-yl]methyl}-1-phenylmethanesulfonamide,
N-{[1-ethyl-6-methyl-4-(tetrahydro-2H-pyran-4-ylamino)-1H-pyrazolo[3,4-b]pyridin-5-yl]methyl}-1-butanesulfonamide,
N-{[1-ethyl-6-methyl-4-(tetrahydro-2H-pyran-4-ylamino)-1H-pyrazolo[3,4-b]pyridin-5-yl]methyl}-2-propanesulfonamide,
N-{[1-ethyl-4-(tetrahydro-2H-pyran-4-ylamino)-1H-pyrazolo[3,4-b]pyridin-5-yl]methyl}-1-(4-pyridinyl)methanesulfonamide,
N-{[1-ethyl-4-(tetrahydro-2H-pyran-4-ylamino)-1H-pyrazolo[3,4-b]pyridin-5-yl]methyl}-2-(4-fluorophenyl)ethanesulfonamide,
N-{[1-ethyl-6-methyl-4-(tetrahydro-2H-pyran-4-ylamino)-1H-pyrazolo[3,4-b]pyridin-5-yl]methyl}-1-(4-pyridinyl)methanesulfonamide,
N-{4-[({[1-ethyl-4-(tetrahydro-2H-pyran-4-ylamino)-1H-pyrazolo[3,4-b]pyridin-5-yl]methyl}amino)sulfonyl]phenyl}acetamide,
N-{[1-ethyl-4-(tetrahydro-2H-pyran-4-ylamino)-1H-pyrazolo[3,4-b]pyridin-5-yl]methyl}-1-phenylmethanesulfonamide,
N-{[1-ethyl-4-(tetrahydro-2H-pyran-4-ylamino)-1H-pyrazolo[3,4-b]pyridin-5-yl]methyl}ethanesulfonamide,
N-{[1-ethyl-4-(tetrahydro-2H-pyran-4-ylamino)-1H-pyrazolo[3,4-b]pyridin-5-yl]methyl}-2-thiophenesulfonamide,
N-{[1-ethyl-6-methyl-4-(tetrahydro-2H-pyran-4-ylamino)-1H-pyrazolo[3,4-b]pyridin-5-yl]methyl}-2-thiophenesulfonamide,
N-{[1-ethyl-4-(tetrahydro-2H-pyran-4-ylamino)-1H-pyrazolo[3,4-b]pyridin-5-yl]methyl}-5-(2-pyridinyl)-2-thiophenesulfonamide,
N-{[1-ethyl-4-(tetrahydro-2H-pyran-4-ylamino)-1H-pyrazolo[3,4-b]pyridin-5-yl]methyl}-3-pyridinecarboxamide,
N-{[1-ethyl-4-(tetrahydro-2H-pyran-4-ylamino)-1H-pyrazolo[3,4-b]pyridin-5-yl]methyl}-2-phenylacetamide,
N-{[1-ethyl-6-methyl-4-(tetrahydro-2H-pyran-4-ylamino)-1H-pyrazolo[3,4-b]pyridin-5-yl]methyl}-2-phenylacetamide,
3-(dimethylamino)-N-{[1-ethyl-6-methyl-4-(tetrahydro-2H-pyran-4-ylamino)-1H-pyrazolo[3,4-b]pyridin-5-yl]methyl}benzamide,
1-acetyl-N-{[1-ethyl-6-methyl-4-(tetrahydro-2H-pyran-4-ylamino)-1H-pyrazolo[3,4-b]pyridin-5-yl]methyl}-4-piperidinecarboxamide,
N'-{[1-ethyl-6-methyl-4-(tetrahydro-2H-pyran-4-ylamino)-1H-pyrazolo[3,4-b]pyridin-5-yl]methyl}-N,N-dimethylbutanediamide,
N-{[1-ethyl-6-methyl-4-(tetrahydro-2H-pyran-4-ylamino)-1H-pyrazolo[3,4-b]pyridin-5-yl]methyl}-4-(1-piperidinyl)benzamide,
N-{[1-ethyl-6-methyl-4-(tetrahydro-2H-pyran-4-ylamino)-1H-pyrazolo[3,4-b]pyridin-5-yl]methyl}propanamide,
4-(acetylamino)-N-{[1-ethyl-4-(tetrahydro-2H-pyran-4-ylamino)-1H-pyrazolo[3,4-b]pyridin-5-yl]methyl}benzamide,
N-{[1-ethyl-4-(tetrahydro-2H-pyran-4-ylamino)-1H-pyrazolo[3,4-b]pyridin-5-yl]methyl}-3,3-dimethylbutanamide,
3-(dimethylamino)-N-{[1-ethyl-4-(tetrahydro-2H-pyran-4-ylamino)-1H-pyrazolo[3,4-b]pyridin-5-yl]methyl}benzamide,
1-acetyl-N-{[1-ethyl-4-(tetrahydro-2H-pyran-4-ylamino)-1H-pyrazolo[3,4-b]pyridin-5-yl]methyl}-4-piperidinecarboxamide,
N'-{[1-ethyl-4-(tetrahydro-2H-pyran-4-ylamino)-1H-pyrazolo[3,4-b]pyridin-5-yl]methyl}-N,N-dimethylbutanediamide,
N-{[1-ethyl-4-(tetrahydro-2H-pyran-4-ylamino)-1H-pyrazolo[3,4-b]pyridin-5-yl]methyl}propanamide,
N-{[1-ethyl-6-methyl-4-(tetrahydro-2H-pyran-4-ylamino)-1H-pyrazolo[3,4-b]pyridin-5-yl]methyl}-4-(methylsulfonyl)benzamide,
N-{[1-ethyl-6-methyl-4-(tetrahydro-2H-pyran-4-ylamino)-1H-pyrazolo[3,4-b]pyridin-5-yl]methyl}-3-pyridinecarboxamide,
N-{[1-ethyl-6-methyl-4-(tetrahydro-2H-pyran-4-ylamino)-1H-pyrazolo[3,4-b]pyridin-5-yl]methyl}-3,3-dimethylbutanamide,
N-{[1-ethyl-4-(tetrahydro-2H-pyran-4-ylamino)-1H-pyrazolo[3,4-b]pyridin-5-yl]methyl}-4-(methylsulfonyl)benzamide,

N-{[1-ethyl-4-(tetrahydro-2H-pyran-4-ylamino)-1H-pyrazolo[3,4-b]pyridin-5-yl]methyl}-2,2,2-trifluoroacetamide,
N-{[1-ethyl-4-(tetrahydro-2H-pyran-4-ylamino)-1H-pyrazolo[3,4-b]pyridin-5-yl]methyl}-2,2,2-trifluoro-N-(phenylmethyl)acetamide,
N-{[1-ethyl-6-methyl-4-(tetrahydro-2H-pyran-4-ylamino)-1H-pyrazolo[3,4-b]pyridin-5-yl]methyl}-N-(phenylmethyl) acetamide,
1-ethyl-5-{1-[(phenylmethyl)amino]propyl}-N-(tetrahydro-2H-pyran-4-yl)-1H-pyrazolo[3,4-b]pyridin-4-amine,
5-[({[3-(dimethylamino)phenyl]methyl}amino)methyl]-1-ethyl-N-(tetrahydro-2H-pyran-4-yl)-1H-pyrazolo[3,4-b]pyridin-4-amine,
1-ethyl-6-methyl-5-{[({5-[(methyloxy)methyl]-2-furanyl}methyl)amino]methyl}-N-(tetrahydro-2H-pyran-4-yl)-1H-pyrazolo[3,4-b]pyridin-4-amine,
N-{4-[({[1-ethyl-6-methyl-4-(tetrahydro-2H-pyran-4-ylamino)-1H-pyrazolo[3,4-b]pyridin-5-yl]methyl}amino)methyl] phenyl}acetamide,
N-{3-[({[1-ethyl-6-methyl-4-(tetrahydro-2H-pyran-4-ylamino)-1H-pyrazolo[3,4-b]pyridin-5-yl]methyl}amino)methyl] phenyl}acetamide,
5-[({[1-ethyl-6-methyl-4-(tetrahydro-2H-pyran-4-ylamino)-1H-pyrazolo[3,4-b]pyridin-5-yl]methyl}amino)methyl]-N-methyl-3-pyridinecarboxamide,
1-ethyl-6-methyl-5-{[(3-pyridinylmethyl)amino]methyl}-N-(tetrahydro-2H-pyran-4-yl)-1H-pyrazolo[3,4-b]pyridin-4-amine,
5-({[(1,3-dimethyl-1H-pyrazol-5-yl)methyl]amino}methyl)-1-ethyl-6-methyl-N-(tetrahydro-2H-pyran-4-yl)-1H-pyrazolo[3,4-b]pyridin-4-amine,
N-{[1-ethyl-4-(tetrahydro-2H-pyran-4-ylamino)-1H-pyrazolo[3,4-b]pyridin-5-yl]methyl}-N-methyl-5-isoxazolecarboxamide,
N-ethyl-N-{[1-ethyl-4-(tetrahydro-2H-pyran-4-ylamino)-1H-pyrazolo[3,4-b]pyridin-5-yl]methyl}-5-isoxazolecarboxamide,
N-{[1,6-diethyl-4-(tetrahydro-2H-pyran-4-ylamino)-1H-pyrazolo[3,4-b]pyridin-5-yl]methyl}-2-pyrazinecarboxamide,
3-(acetylamino)-N-{[1,6-diethyl-4-(tetrahydro-2H-pyran-4-ylamino)-1H-pyrazolo[3,4-b]pyridin-5-yl]methyl}benzamide,
N-{[1,6-diethyl-4-(tetrahydro-2H-pyran-4-ylamino)-1H-pyrazolo[3,4-b]pyridin-5-yl]methyl}-6-(1H-pyrrol-1-yl)-3-pyridinecarboxamide,
N-{[1,6-diethyl-4-(tetrahydro-2H-pyran-4-ylamino)-1H-pyrazolo[3,4-b]pyridin-5-yl]methyl} 3-(2-oxo-1-pyrrolidinyl) benzamide,
N-{[1,6-diethyl-4-(tetrahydro-2H-pyran-4-ylamino)-1H-pyrazolo[3,4-b]pyridin-5-yl]methyl}-4-pyridazinecarboxamide,
N-{[1,6-diethyl-4-(tetrahydro-2H-pyran-4-ylamino)-1H-pyrazolo[3,4-b]pyridin-5-yl]methyl}-5-pyrimidinecarboxamide,
1-acetyl-N-{[1,6-diethyl-4-(tetrahydro-2H-pyran-4-ylamino)-1H-pyrazolo[3,4-b]pyridin-5-yl]methyl}-4-piperidinecarboxamide,
N-{[1,6-diethyl-4-(tetrahydro-2H-pyran-4-ylamino)-1H-pyrazolo[3,4-b]pyridin-5-yl]methyl}-2-furancarboxamide,
N-{[1,6-diethyl-4-(tetrahydro-2H-pyran-4-ylamino)-1H-pyrazolo[3,4-b]pyridin-5-yl]methyl}-3-furancarboxamide,
N-{[1,6-diethyl-4-(tetrahydro-2H-pyran-4-ylamino)-1H-pyrazolo[3,4-b]pyridin-5-yl]methyl}-2-methyl-5-(trifluoromethyl)-1,3-oxazole-4-carboxamide,
N-{[1,6-diethyl-4-(tetrahydro-2H-pyrin-4-ylamlino)-1H-pyrazolo[3,4-b]pyridin-5-yl]methyl}tetrahydro-2-furancarboxamide,
4-(acetylamino)-N-{[1,6-diethyl-4-(tetrahydro-2H-pyran-4-ylamino)-1H-pyrazolo[3,4-b]pyridin-5-yl]methyl}-2-(methyloxy)benzamide,
N-{[1,6-diethyl-4-(tetrahydro-2H-pyran-4-ylamino)-1H-pyrazolo[3,4-b]pyridin-5-yl]methyl}-1-methyl-1H-pyrazole-5-carboxamide,
N-{[1,6-diethyl-4-(tetrahydro-2H-pyran-4-ylamino)-1H-pyrazolo[3,4-b]pyridin-5-yl]methyl}-2-(ethyloxy)-3-pyridinecarboxamide,
N-{[1,6-diethyl-4-(tetrahydro-2H-pyran-4-ylamino)-1H-pyrazolo[3,4-b]pyridin-5-yl]methyl}-2-(ethyloxy)benzamide,
N-{[1,6-diethyl-4-(tetrahydro-2H-pyran-4-ylamino)-1H-pyrazolo[3,4-b]pyridin-5-yl]methyl}-2,3-dihydro-1-benzofuran-7-carboxamide,
N-{[1,6-diethyl-4-(tetrahydro-2H-pyran-4-ylamino)-1H-pyrazolo[3,4-b]pyridin-5-yl]methyl}-2-(3-pyridinyl)acetamide,
2-[3-(acetylamino)phenyl]-N-{[1,6-diethyl-4-(tetrahydro-2H-pyran-4-ylamino)-1H-pyrazolo[3,4-b]pyridin-5-yl]methyl}acetamide,
N-{[1,6-diethyl-4-(tetrahydro-2H-pyran-4-ylamino)-1H-pyrazolo[3,4-b]pyridin-5-yl]methyl}-2-[2-(methyloxy) phenyoacetamide,

N-{[1,6-diethyl-4-(tetrahydro-2H-pyran-4-ylamino)-1H-pyrazolo[3,4-b]pyridin-5-yl]methyl}-2-(2-furanyl)acetamide,
N-{[1-6-diethyl-4-(tetrahydro-2H-pyran-4-ylamino)-1H-pyrazolo[3,4-b]pyridin-5-yl]methyl}-2-(3-isoxazolyl)acetamide,
N-{[1,6-diethyl-4-(tetrahydro-2H-pyran-4-ylamino)-1H-pyrazolo[3,4-b]pyridin-5-yl]methyl}propanamide,
N-{4-[({[1,6-diethyl-4-(tetrahydro-2H-pyran-4-ylamino)-1H-pyrazolo[3,4-b]pyridin-5-yl]methyl}amino)sulfonyl]phenyl}acetamide,
N-{[1,6-diethyl-4-(tetrahydro-2H-pyran-4-ylamino)-1H-pyrazolo[3,4-b]pyridin-5-yl]methyl}-1-phenylmethanesulfonamide,
N-{[1,6-diethyl-4-(tetrahydro-2H-pyran-4-ylamino)-1H-pyrazolo[3,4-b]pyridin-5-yl]methyl}-5-(2-pyridinyl)-2-thiophenesulfonamide,
N-{[1,6-diethyl-4-(tetrahydro-2H-pyran-4-ylamino)-1H-pyrazolo[3,4-b]pyridin-5-yl]methyl}-1-butanesulfonamide,
N-{(1,6-diethyl-4-(tetrahydro-2H-pyran-4-ylamino)-1H-pyrazolo[3,4-b]pyridin-5-yl]methyl}-3,4-bis(methyloxy)benzenesulfonamide,
N-{[1,6-diethyl-4-(tetrahydro-2H-pyran-4-ylamino)-1H-pyrazolo[3,4-b]pyridin-5-yl]methyl}-2-propanesulfonamide,
6-chloro-N-{[1,6-diethyl-4-(tetrahydro-2H-pyran-4-ylamino)-1H-pyrazolo[3,4-b]pyridin-5-yl]methyl}-3-(rnethyloxy)-2-quinoxalinecarboxamide,
N-{[1,6-diethyl-4-(tetrahydro-2H-pyran-4-ylamino)-1H-pyrazolo[3,4-b]pyridin-5-yl]methyl}-4-(1-piperidinyl)benzamide,
N-{[1,6-diethyl-4-(tetrahydro-2H-pyran-4-ylamino)-1H-pyrazolo[3,4-b]pyridin-5-yl]methyl}-2,3-dihydro-1H-indene-5-carboxamide,
N-{[1,6-diethyl-4-(tetrahydro-2H-pyran-4-ylamino)-1H-pyrazolo[3,4-b]pyridin-5-yl]methyl}-5,6,7,8-tetrahydro-2-naphthalenecarboxamide,
N-{[1,6-diethyl-4-(tetrahydro-2H-pyran-4-ylamino)-1H-pyrazolo[3,4-b]pyridin-5-yl]methyl}-4-(2-oxo-1-pyrrolidinyl) benzamide,
N-{[1,6-diethyl-4-(tetrahydro-2H-pyran-4-ylamino)-1H-pyrazolo[3,4-b]pyridin-5-yl]methyl}-2-(methyloxy)-5-(phenylsulfonyl)benzamide,
N-{[1,6-diethyl-4-(tetrahydro-2H-pyran-4-ylamino)-1H-pyrazolo[3,4-b]pyridin-5-yl]methyl}-4-(4-morpholinyl)benzamide,
N-{[1,6-diethyl-4-(tetrahydro-2H-pyran-4-ylamino)-1H-pyrazolo[3,4-b]pyridin-5-yl]methyl}-2-[(2,2-dimethylpropanoyl)amino]-6-(methyloxy)benzamide,
N~3~-{[1,6-diethyl-4-(tetrahydro-2H-pyran-4-ylamino)-1H-pyrazolo[3,4-b]pyridin-5-yl]methyl}-N~1~-methyl-4-(methyloxy)-N~1~-phenyl-1,3-benzenedicarboxamide,
5-{[(cyclopropylmethyl)amino]sulfonyl}-N-{[1,6-diethyl-4-(tetrahydro-2H-pyran-4-ylamino)-1H-pyrazolo[3,4-b]pyridin-5-yl]methyl}-2-(methyloxy)benzamide,
4-cyclopentyl-N-{[1,6-diethyl-4-(tetrahydro-2H-pyran-4-ylamino)-1H-pyrazolo[3,4-b]pyridin-5-yl]methyl}benzamide,
5-[(diethylamino)sulfonyl]-N-{[1,6-diethyl-4-(tetrahydro-2H-pyran-4-ylamino)-1H-pyrazolo[3,4-b]pyridin-5-yl]methyl}-2-(methyloxy)benzamide,
5-[(cyclobutylamino)sulfonyl]-N-{[1,6-diethyl-4-(tetrahydro-2H-pyran-4-ylamino)-1H-pyrazolo[3,4-b]pyridin-5-yl] methyl}-2-(methyloxy)benzamide,
N-{[1,6-diethyl-4-(tetrahydro-2H-pyran-4-ylamino)-1H-pyrazolo[3,4-b]pyridin-5-yl]methyl}-4-(hexahydro-1 (2H)-azocinyl)benzamide,
5-cyano-N-{[1,6-diethyl-4-(tetrahydro-2H-pyran-4-ylamino)-1H-pyrazolo[3,4-b]pyridin-5-yl]methyl}-4-(1-methylethyl)-2-(methyloxy)benzamide,
N-{[1,6-diethyl-4-(tetrahydro-2H-pyran-4-ylamino)-1H-pyrazolo[3,4-b]pyridin-5-yl]methyl}-2,3-dihydro-1H-indene-4-carboxamide,
N-{[1,6-diethyl-4-(tetrahydro-2H-pyran-4-ylamino)-1H-pyrazolo[3,4-b]pyridin-5-yl]methyl}-3-(4-morpholinyl)benzamide,
N-{[1,6-diethyl-4-(tetrahydro-2H-pyran-4-ylamino)-1H-pyrazolo[3,4-b]pyridin-5-yl]methyl}-2-naphthalenecarboxamide,
N-{[1,6-diethyl-4-(tetrahydro-2H-pyran-4-ylamino)-1H-pyrazolo[3,4-b]pyridin-5-yl]methyl}-1-naphthalenecarboxamide,
N-{[1,6-diethyl-4-(tetrahydro-2H-pyran-4-ylamino)-1H-pyrazolo[3,4-b]pyridin-5-yl]methyl}-4-(1-methylethyl)benzamide,
4-(acetylamino)-N-{[1,6-diethyl-4-(tetrahydro-2H-pyran-4-ylamino)-1H-pyrazolo[3,4-b]pyridin-5-yl]methyl}benzamide,
N-{[1,6-diethyl-4-(tetrahydro-2H-pyran-4-ylamino)-1H-pyrazolo[3,4-b]pyridin-5-yl]methyl}-2-methyl-3-furancarboxamide,

N-{[1,6-diethyl-4-(tetrahydro-2H-pyjan-4-ylamino)-1H-pyrazolo[3,4-b]pyridin-5-yl]methyl}benzamide,
N-{[1,6-diethyl-4-(tetrahydro-2H-pyran-4-ylamino)-1H-pyrazolo[3,4-b]pyridin-5-yl]methyl}-2-methyl-3-furancarboxamide,
5-{[1,6-diethyl-4-(tetrahydro-2H-pyran-4-ylamino)-1H-pyrazolo[3,4-b]pyridin-5-yl]methyl}-5,6-dihydro-4H-furo[2,3-c]pyrrol-4-one,
2-{[1,6-diethyl-4-(tetrahydro-2H-pyran-4-ylamino)-1H-pyrazolo[3,4-b]pyridin-5-yl]methyl}-2,3-dihydro-1H-isoindol-1-one,
N-{[1,6-diethyl-4-(tetrahydro-2H-pyran-4-ylamino)-1H-pyrazolo[3,4-b]pyridin-5-yl]methyl}-N'-(phenylmethyl)urea,
N-{[1,6-diethyl-4-(tetrahydro-2H-pyran-4-ylamino)-1H-pyrazolo[3,4-b]pyridin-5-yl]methyl}-N'-(tetrahydro-2H-pyran-4-yl)urea,
N-{[1,6-diethyl-4-(tetrahydro-2H-pyran-4-ylamino)-1H-pyrazolo[3,4-b]pyridin-5-yl]methyl}-N'-(2-phenylethyl)urea,
N-{[1,6-diethyl-4-(tetrahydro-2H-pyran-4-ylamino)-1H-pyrazolo[3,4-b]pyridin-5-yl]methyl}-N'-phenylurea,
4-({1,6-diethyl-5-[({[4-(1-piperidinyl)phenyl]carbonyl}amino)methyl]-1H-pyrazolo[3,4-b]pyridin-4-yl}amino)-1-piperidinecarboxamide,
4-[(5-{[(2,3-dihydro-1-benzofuran-7-ylcarbonyl)amino]methyl}-1,6-diethyl-1H-pyrazolo[3,4-b]pyridin-4-yl)amino]-1-piperidinecarboxamide,
4-[(5-{[(2,3-dihydro-1H-inden-5-ylcarbonyl)amino]methyl}-1,6-diethyl-1H-pyrazolo[3,4-b]pyridin-4-yl)amino]-1-piperidinecarboxamide,
4-({1,6-diethyl-5-[({[4-(4-morpholinyl)phenyl]carbonyl}amino)methyl]-1H-pyrazolo[3,4-b]pyridin-4-yl}amino)-1-piperidinecarboxamide,
4-({1,6-diethyl-5-[({[4-(1-methylethyl)phenyl]carbonyl}amino)methyl]-1H-pyrazolo[3,4-b]pyridin-4-yl}amino)-1-piperidinecarboxamide,
4-({1,6-diethyl-5-[({[4-(2-oxo-1-pyrrolidinyl)phenyl]carbonyl}amino)methyl]-1H-pyrazolo[3,4-b]pyridin-4-yl}amino)-1-piperidinecarboxamide,
4-{[5-({[(4-cyclopentylphenyl)carbonyl]amino}methyl)-1,6-diethyl-1H-pyrazolo[3,4-b]pyridin-4-yl]amino}-1-piperidinecarboxamide,
4-{[1,6-diethyl-5-({[(2-hydroxyphenyl)carbonyl]amino}methyl)-1H-pyrazolo[3,4-b]pyridin-4-yl]amino}-1-piperidinecarboxamide,
4-({1,6-diethyl-5-[(1-oxo-1,3-dihydro-2H-isoindol-2-yl)methyl]-1H-pyrazolo[3,4-b]pyridin-4-yl}amino)-1-piperidinecarboxamide,
N-{[1-ethyl-6-methyl-4-(tetrahydro-2H-pyran-4-ylamino)-1H-pyrazolo[3,4-b]pyridin-5-yl]methyl}-2-methyl-5-(trifluoromethyl)-1,3-oxazole-4-carboxamide,
N-{[1-ethyl-6-methyl-4-(tetrahydro-2H-pyran-4-ylamino)-1H-pyrazolo[3,4-b]pyridin-5-yl]methyl}-6-(1H-pyrrol-1-yl)-3-pyridinecarboxamide,
N-{[1-ethyl-6-methyl-4-(tetrahydro-2H-pyran-4-ylamino)-1H-pyrazolo[3,4-b]pyridin-5-yl]methyl}-1-methyl-1H-pyrazole-5-carboxamide, N-{[1-ethyl-6-methyl-4-(tetrahydro-2H-pyran-4-ylamino)-1H-pyrazolo[3,4-b]pyridin-5-yl]methyl}-3-(2-oxo-1-pyrrolidinyl)benzamide,
N-{[1-ethyl-6-methyl-4-(tetrahydro-2H-pyran-4-ylamino)-1H-pyrazolo[3,4-b]pyridin-5-yl]methyl}-3-furancarboxamide,
N-{[1-ethyl-6-methyl-4-(tetrahydro-2H-pyran-4-ylamino)-1H-pyrazolo[3,4-b]pyridin-5-yl]methyl}-2-furancarboxamide,
N-{[1-ethyl-6-methyl-4-(tetrahydro-2H-pyran-4-ylamino)-1H-pyrazolo[3,4-b]pyridin-5-yl]methyl}-2-(ethyloxy)benzamide,
N-{[1-ethyl-6-methyl-4-(tetrahydro-2H-pyran-4-ylamino)-1H-pyrazolo[3,4-b]pyridin-5-yl]methyl}-2,3-dihydro-1-benzofuran-7-darboxamide,
N-{[1-ethyl-6-methyl-4-(tetrahydro-2H-pyran-4-ylamino)-1H-pyrazolo[3,4-b]pyridin-5-yl]methyl}-2-pyrazinecarboxamide,
4-(acetylamino)-N-{[1-ethyl-6-methyl-4-(tetrahydro-2H-pyran-4-ylamino)-1H-pyrazolo[3,4-b]pyridin-5-yl]methyl}-2-(methyloxy)benzamide,
N-{[1-ethyl-6-methyl-4-(tetrahydro-2H-pyran-4-ylamino)-1H-pyrazolo[3,4-b]pyridin-5-yl]methyl}-2-(ethyloxy)-3-pyridinecarboxamide,
N-{[1-ethyl-6-methyl-4-(tetrahydro-2H-pyran-4-ylamino)-1H-pyrazolo[3,4-b]pyridin-5-yl]methyl}-3-fluoro-4-pyridinecarboxamide,
N-{[1-ethyl-6-methyl-4-(tetrahydro-2H-pyran-4-ylamino)-1H-pyrazolo[3,4-b]pyridin-5-yl]methyl}-5-methyl-3-isoxazolecarboxamide,
N-{[1-ethyl-6-methyl-4-(tetrahydro-2H-pyran-4-ylamino)-1H-pyrazolo[3,4-b]pyridin-5-yl]methyl}-1,3-thiazole-5-carboxamide,
N-{[1-ethyl-6-methyl-4-(tetrahydro-2H-pyran-4-ylamino)-1H-pyrazolo[3,4-b]pyridin-5-yl]methyl}-3-thiophenecarboxamide,
N-[1-ethyl-6-methyl-4-(tetrahydro-2H-pyran-4-ylamino)-1H-pyrazolo[3,4-b]pyridin-5-yl]methyl}-5-methyl-2-pyrazinecarboxamide,
N-{[1-ethyl-6-methyl-4-(tetrahydro-2H-pyran-4-ylamino)-1H-pyrazolo[3,4-b]pyridin-5-yl]methyl}-1-methyl-1H-pyrazole-3-carboxamide,
N-{[1-ethyl-6-methyl-4-(tetrahydro-2H-pyran-4-ylamino)-1H-pyrazolo[3,4-b]pyridin-5-yl]methyl}-1,3-oxazole-5-carboxamide,
N-{[1,6-diethyl-4-(tetrahydro-2H-pyran-4-ylamino)-1H-pyrazolo[3,4-b]pyridin-5-yl]methyl}-3-fluoro-4-pyridinecarboxamide,
N-{[1,6-diethyl-4-(tetrahydro-2H-pyran-4-ylamino)-1H-pyrazolo[3,4-b]pyridin-5-yl]methyl}-2-fluorobenzamide,
N-{[1,6-diethyl-4-(tetrahydro-2H-pyran-4-ylamino)-1H-pyrazolo[3,4-b]pyridin-5-yl]methyl}-5-methyl-3-isoxazolecarboxamide,
N-{[1,6-diethyl-4-(tetrahydro-2H-pyran-4-ylamino)-1H-pyrazolo[3,4-b]pyridin-5-yl]methyl}-1,3-thiazole-5-carboxamide,
N-{[1,6-diethyl-4-(tetrahydro-2H-pyran-4-ylamino)-1H-pyrazolo[3,4-b]pyridin-5-yl]methyl}-3-thiophenecarboxamide,
N-{[1,6-diethyl-4-(tetrahydro-2H-pyran-4-ylamino)-1H-pyrazolo[3,4-b]pyridin-5-yl]methyl}-5-methyl-2-pyrazinecarboxamide,
N-{[1,6-diethyl4-(tetrahydro-2H-pyran-4-ylamino)-1H-pyrazolo[3,4-b]pyridin-5-yl]methyl}-3-methyl-5-isoxazolecarboxamide,
N-{[1,6-diethyl-4-(tetrahydro-2H-pyran-4-ylamino)-1H-pyrazolo[3,4-b]pyridin-5-yl]methyl}-1-methyl-1H-pyrazole-3-carboxamide,
N-{[1,6-diethyl-4-(tetrahydro-2H-pyran-4-ylamino)-1H-pyrazolo[3,4-b]pyridin-5-yl]methyl}-1,3-oxazole-5-carboxamide,
4-[(1,6-diethyl-5-{[(2-furanylcarbonyl)amino]methyl}-1H-pyrazolo[3,4-b]pyridin-4-yl)amino]-1-piperidinecarboxamide,
4-({5-[({[4-(acetylamino)-2-hydroxyphenyl]carbonyl}amino)methyl]-1,6-diethyl-1H-pyrazolo[3,4-b]pyridin-4-yl}amino)-1-piperidinecarboxamide,
4-({5-[({[4-(acetylamino)phenyl]carbonyl}amino)methyl]-1,6-diethyl-1H-pyrazolo[3,4-b]pyridin-4-yl}amino)-1-piperidinecarboxamide,
4-({5-[({[4-(1,1-dimethylethyl)phenyl]carbonyl}amino)methyl]-1,6-diethyl-1H-pyrazolo[3,4-b]pyridin-4-yl}amino)-1-piperidinecarboxamide,
4-({5-[({[3-(acetylamino)phenyl]carbonyl}amino)methyl]-1,6-diethyl-1H-pyrazolo[3,4-b]pyridin-4-yl}amino)-1-piperidinecarboxamide,
4-{[1,6-diethyl-5-({[(2-hydroxy-4-methylphenyl)carbonyl]amino}methyl)-1H-pyrazolo[3,4-b]pyridin-4-yl]amino}-1-piperidinecarboxamide,
4-{[1,6-diethyl-5-({[(2-fluorophenyl)carbonyl]amino}methyl)-1H-pyrazolo[3,4-b]pyridin-4-yl]amino}-1-piperidinecarboxamide,
4-({1,6-diethyl-5-[({[3-(2-oxo-1-pyrrolidinyl)phenyl]carbonyl}amino)methyl]-1H-pyrazolo[3,4-b]pyridin-4-yl}amino)-1-piperidinecarboxamide,
N-[(4-{[1-(aminocarbonyl)-4-piperidinyl]amino}-1,6-diethyl-1H-pyrazolo[3,4-b]pyridin-5-yl)methyl]-3-oxo-2,3-dihydro-1H-indazole-6-carboxamide,
N-{[1,6-diethyl-4-(tetrahydro-2H-pyran-4-ylamino)-1H-pyrazolo[3,4-b]pyridin-5-yl]methyl}-4-(methylsulfonyl)benzamide,
N-{[1,6-diethyl-4-(tetrahydro-2H-pyran-4-ylamino)-1H-pyrazolo[3,4-b]pyridin-5-yl]methyl}-2-hydroxybenzamide,
4-(acetylamino)-N-{[1,6-diethyl-4-(tetrahydro-2H-pyran-4-ylamino)-1H-pyrazolo[3,4-b]pyridin-5-yl]methyl}-2-hydroxybenzamide,
N-{[1,6-diethyl-4-(tetrahydro-2H-pyran-4-ylamino)-1H-pyrazolo[3,4-b]pyridin-5-yl]methyl}-4-(1,1-dimethylethyl) benzamide,
N-{[1,6-diethyl-4-(tetrahydro-2H-pyran-4-ylamino)-1H-pyrazolo[3,4-b]pyridin-5-yl]methyl}-1,4-benzenedicarboxamide,
N-{[1,6-diethyl-4-(tetrahydro-2H-pyran-4-ylamino)-1H-pyrazolo[3,4-b]pyridin-5-yl]methyl}-2-hydroxy-4-methylbenzamide,
N-{[1,6-diethyl-4-(tetrahydro-2H-pyran-4-ylamino)-1H-pyrazolo[3,4-b]pyridin-5-yl]methyl}-N'-methyl-1,4-benzenedicarboxamide,
N-{[1,6-diethyl-4-(tetrahydro-2H-pyran-4-ylamino)-1H-pyrazolo[3,4-b]pyridin-5-yl]methyl}-4-(1,1-dimethylethyl)-2-hydroxybenzamide,
N-{[1,6-diethyl-4-(tetrahydro-2H-pyran-4-ylamino)-1H-pyrazolo[3,4-b]pyridin-5-yl]methyl}-3-oxo-2,3-dihydro-1H-indazole-6-carboxamide,
N-{[1,6-diethyl-4-(tetrahydro-2H-pyran-4-ylamino)-1H-pyrazolo[3,4-b]pyridin-5-yl]methyl}-3,4-dihydro-2H-1-benzothiopyran-6-carboxamide 1,1-dioxide,
4-({1-ethyl-6-methyl-5-[({[4-(methylsulfonyl)phenyl]carbonyl}amino)methyl]-1H-pyrazolo[3,4-b]pyridin-4-yl}amino)-1-piperidinecarboxamide,
4-{[1-ethyl-5-({[(2-hydroxyphenyl)carbonyl]amino}methyl)-6-methyl-1H-pyrazolo[3,4-b]pyridin-4-yl]amino}-1-piperidinecarboxamide,
4-({5-[({[4-(acetylamino)-2-hydroxyphenyl]carbonyl}amino)methyl]-1-ethyl-6-methyl-1H-pyrazolo[3,4-b]pyridin-4-yl}amino)-1-piperidinecarboxamide,
4-({5-[({[4-(1,1-dimethylethyl)phenyl]carbonyl}amino)methyl]-1-ethyl-6-methyl-1H-pyrazolo[3,4-b]pyridin-4-yl}amino)-1-piperidinecarboxamide,
4-({1-ethyl-6-methyl-5-[({[4-(1-methylethyl)phenyl]carbonyl}amino)methyl]-1H-pyrazolo[3,4-b]pyridin-4-yl}amino)-1-piperidinecarboxamide,
4-({5-[({[3-(acetylamino)phenyl]carbonyl}amino)methyl]-1-ethyl-6-methyl-1H-pyrazolo[3,4-b]pyridin-4-yl}amino)-1-piperidinecarboxamide,
4-{[1-ethyl-5-({[(2-hydroxy-4-methylphenyl)carbonyl]amino}methyl)-6-methyl-1H-pyrazolo[3,4-b]pyndin-4-yl]amino}-1-piperidinecarboxamide,
4-{[1-ethyl-5-({[(2-fluorophenyl)carbonyl]amino}methyl)-6-methyl-1H-pyrazolo[3,4-b]pyridin-4-yl]amino}-1-piperidinecarboxamide,
4-({1-ethyl-6-methyl-5-[({[3-(2-oxo-1-pyrrolidiriyl)phenyl]carbonyl}amino)methyl]-1H-pyrazolo[3,4-b]pyridin-4-yl}amino)-1-piperidinecarboxamide,
N-[(4-{[1-(aminocarbonyl)-4-piperidinyl]amino}-1-ethyl-6-methyl-1H-pyrazolo[3,4-b]pyridin-5-yl)methyl]-N'-methyl-1,4-benzenedicarboxamide,
4-({1-ethyl-6-methyl-5-[({[4-(1-piperidinyl)phenyl]carbonyl}amino)methyl]-1H-pyrazolo[3,4-b]pyridin-4-yl}amino)-1-piperidinecarboxamide,
4-[(5-{[(2,3-dihydro-1-benzofuran-7-ylcarbonyl)amino]methyl}-1-ethyl-6-methyl-1H-pyrazolo[3,4-b]pyridin-4-yl)amino]-1-piperidinecarboxamide,
4-[(5-{[(2,3-dihydro-1H-inden-5-ylcarbonyl)amino]methyl}-1-ethyl-6-methyl-1H-pyrazolo[3,4-b]pyridin-4-yl)amino]-1-piperidinecarboxamide,
4-({5-[({[4-(1,1-dimethylethyl)-2-hydroxyphenyl]carbonyl}amino)methyl]-1-ethyl-6-methyl-1H-pyrazolo[3,4-b]pyridin-4-yl}amino)-1-piperidinecarboxamide,
4-{[5-({[(4-cyclopentylphenyl)carbonyl]amino}methyl)-1-ethyl-6-methyl-1H-pyrazolo[3,4-b]pyridin-4-y)]amino}-1-piperidinecarboxamide,
4-{[1-ethyl-6-methyl-5-({[(4-methyl-2,3-dihydro-1-benzofuran-7-yl)carbonyl]amino}methyl)-1H-pyrazolo[3,4-b]pyridin-4-yl]amino}-1-piperidinecarboxamide,
N-{[1-ethyl-6-methyl-4-(tetrahydro-2H-pyran-4-ylamino)-1H-pyrazolo[3,4-b]pyridin-5-yl]methyl}-2-hydroxybenzamide,
4-(acetylamino)-N-{[-ethyl-6-methyl-4-(tetrahydro-2H-pyran-4-ylamino)-1H-pyrazolo[3,4-b]pyridin-5-yl]methyl}-2-hydroxybenzamide,
4-(1,1-dimethylethyl)-N-{[1-ethyl-6-methyl-4-(tetrahydro-2H-pyran-4-ylamino)-1H-pyrazolo[3,4-b]pyridin-5-yl]methyl}benzamide,
N-{[1-ethyl-6-methyl-4-(tetrahydro-2H-pyran-4-ylamino)-1H-pyrazolo[3,4-b]pyridin-5-yl]methyl}-4-(1-methylethyl)benzamide,
N-{[1-ethyl-6-methyl-4-(tetrahydro-2H-pyran-4-ylamino)-1H-pyrazolo[3,4-b]pyridin-5-yl]methyl}-1,4-benzenedicarboxamide,
3-(acetylamino)-N-{[1-ethyl-6-methyl-4-(tetrahydro-2H-pyran-4-ylamino)-1H-pyrazolo[3,4-b]pyridin-5-yl]methyl}benzamide,
N-{[1-ethyl-6-methyl-4-(tetrahydro-2H-pyran-4-ylamino)-1H-pyrazolo[3,4-b]pyridin-5-yl]methyl}-2-hydroxy-4-methylbenzamide,
N-{[1-ethyl-6-methyl-4-(tetrahydro-2H-pyran-4-ylamino)-1H-pyrazolo[3,4-b]pyridin-5-yl]methyl}-2-fluorobenzamide,
N-{[1-ethyl-6-methyl-4-(tetrahydro-2H-pyran-4-ylamino)-1H-pyrazolo[3,4-b]pyridin-5-yl]methyl}-N'-methyl-1,4-benzenedicarboxamide,
N-{[1-ethyl-6-methyl-4-(tetrahydro-2H-pyran-4-ylamino)-1H-pyrazolo[3,4-b]pyridin-5-yl]methyl}-2,3-dihydro-1H-indene-5-carboxamide,
4-(1,1-dimethylethyl)-N-{[1-ethyl-6-methyl-4-(tetrahydro-2H-pyran-4-ylamino)-1H-pyrazolo[3,4-b]pyridin-5-yl]methyl}-2-hydroxybenzamide,
N-{[1-ethyl-6-methyl-4-(tetrahydro-2H-pyran-4-ylamino)-1H-pyrazolo[3,4-b]pyridin-5-yl]methyl}-3-oxo-2,3-dihydro-1H-indazole-6-carboxamide,
N-{[1-ethyl-6-methyl-4-(tetrahydro-2H-pyran-4-ylamino)-1H-pyrazolo[3,4-b]pyridin-5-yl]methyl}-3,4-dihydro-2H-1-benzothiopyran-6-carboxamide 1,1-dioxide,
N-{[1-ethyl-6-methyl-4-(tetrahydro-2H-pyran-4-ylamino)-1H-pyrazolo[3,4-b]pyridin-5-yl]methyl}-4-(2-oxo-1-pyrrolidinyl)benzamide,
N-{[1,6-diethyl-4-(tetrahydro-2H-pyran-4-ylamino)-1H-pyrazolo[3,4-b]pyridin-5-yl]methyl}-1-pyrrolidinecarboxamide,
N-{[1,6-diethyl-4-(tetrahydro-2H-pyran-4-ylamino)-1H-pyrazolo[3,4-b]pyridin-5-yl]methyl}-1-piperidinecarboxamide,
N-{[1,6-diethyl-4-(tetrahydro-2H-pyran-4-ylamino)-1H-pyrazolo[3,4-b]pyridin-5-yl]methyl}-4-morpholinecarboxamide,
N-{[1-ethyl-6-methyl-4-(tetrahydro-2H-pyran-4-ylamino)-1H-pyrazolo[3,4-b]pyridin-5-yl]methyl}-1-pyrrolidinecarboxamide,
N-{[1-ethyl-6-methyl-4-(tetrahydro-2H-pyran-4-ylamino)-1H-pyrazolo[3,4-b]pyridin-5-yl]methyl}-4-morpholinecarboxamide,
N-{[1-ethyl-6-methyl-4-(tetrahydro-2H-pyran-4-ylamino)-1H-pyrazolo[3,4-b]pyridin-5-yl]methyl}-N'-(tetrahydro-2H-pyran-4-yl)urea,
1-{[1-ethyl-4-(tetrahydro-2H-pyran-4-ylamino)-1H-pyrazolo[3,4-b]pyridin-5-yl]methyl}-3-methyl-2-imidazolidinone,
1-{[1-ethyl-6-methyl-4-(tetrahydro-2H-pyran-4-ylamino)-1H-pyrazolo[3,4-b]pyridin-5-yl]methyl}-3-methyl-2-imidazolidinone,
1-{[1,6-diethyl-4-(tetrahydro-2H-pyran-4-ylamino)-1H-pyrazolo[3,4-b]pyridin-5-yl]methyl}-2-piperidinone,
1-{[1-ethyl-6-methyl-4-(tetrahydro-2H-pyran-4-ylamino)-1H-pyrazolo[3,4-b]pyridin-5-yl]methyl}-2-pyrrolidinone,
1-{[1-ethyl-6-methyl-4-(tetrahydro-2H-pyran-4-ylamino)-1H-pyrazolo[3,4-b]pyridin-5-yl]methyl}-2-piperidinone,
2-{[1-ethyl-6-methyl-4-(tetrahydro-2H-pyran-4-ylamino)-1H-pyrazolo[3,4-b]pyridin-5-yl]methyl}-2,3-dihydro-1H-isoindol-1-one;
5-{[1-ethyl-6-methyl-4-(tetrahydro-2H-pyran-4-ylamino)-1H-pyrazolo[3,4-b]pyridin-5-yl]methyl}-5,6-dihydro-4H-furo[2,3-c]pyrrol-4-one,
4-({5-[({[4-(1,1-dimethylethyl)-2-hydroxyphenyl]carbonyl}amino)methyl]-1,6-diethyl-1H-pyrazolo[3,4-b]pyridin-4-yl}amino)-1-piperidinecarboxamide,
4-({1-ethyl-6-methyl-5-[({[4-(4-morpholinyl)phenyl]carbonyl}amino)methyl]-1H-pyrazolo[3,4-b]pyridin-4-yl}amino)-1-piperidinecarboxamide,
4-({5-[({[4-(acetylamino)-2,3-dihydro-1-benzofuran-7-yl]carbonyl}amino)methyl]-1,6-diethyl-1H-pyrazolo[3,4-b]pyridin-4-yl}amino)-1-piperidinecarboxamide,
4-({5-[({[4-(acetylamino)-2,3-dihydro-1-benzofuran-7-yl]carbonyl}amino)methyl]-1-ethyl-6-methyl-1H-pyrazolo[3,4-b]pyridin-4-yl}amino)-1-piperidinecarboxamide,
4-(acetylamino)-N-{[1,6-diethyl-4-(tetrahydro-2H-pyran-4-ylamino)-1H-pyrazolo[3,4-b]pyridin-5-yl]methyl}-2,3-dihydro-1-benzofuran-7-carboxamide,
N-{[1,6-diethyl-4-(tetrahydro-2H-pyran-4-ylamino)-1H-pyrazolo[3,4-b]pyridin-5-yl]methyl}-4-fluorobenzamide,
N-{[1,6-diethyl-4-(tetrahydro-2H-pyran-4-ylamino)-1H-pyrazolo[3,4-b]pyridin-5-yl]methyl}-2,4-difluorobenzamide,
N-{[1,6-diethyl-4-(tetrahydro-2H-pyran-4-ylamino)-1H-pyrazolo[3,4-b]pyridin-5-yl]methyl}-2-methyl-1,3-thiazole-4-carboxamide,
N-{[1,6-diethyl-4-(tetrahydro-2H-pyran-4-ylamino)-1H-pyrazolo[3,4-b]pyridin-5-yl]methyl}-2-methyl-1,3-oxazole-4-carboxamide, or
N-{[1,6-diethyl-4-(tetrahydro-2H-pyran-4-ylamino)-1N-pyrazolo[3,4-b]pyridin-5-yl]methyl}-2-methyl-1,3-thiazole-5-carboxamide;

or a salt thereof such as a pharmaceutically acceptable salt thereof.

The structures of the above-listed specific compounds, or embodiments thereof, are given in Examples 1 to 345 hereinafter.

In an alternative embodiment, the compound of formula (I) or the salt thereof is for example:

*N*-{[1,6-diethyl-4-(tetrahydro-2*H*-pyran-4-ylamino)-1*H*-pyrazolo[3,4-*b*]pyridin-5-yl]methyl}-2-(1-methylethyl)-5-pyrimidinecarboxamide,
*N*-{[1-ethyl-6-methyl-4-(tetrahydro-2*H*-pyran-4-ylamino)-1*H*-pyrazolo[3,4-b]pyridin-5-yl]methyl}-2-(1-methylethyl)-5-pyrimidinecarboxamide,
*N*-{[1-Ethyl-4-(tetrahydro-2*H*-pyran-4-ylamino)-6-(trifluoromethyl)-1*H* pyrazolo[3,4-*b*]pyridin-5-yl]methyl}-5-methyl-2-pyrazinecarboxamide, or
*N*-{[1-Ethyl-4-(tetrahydro-2*H*-pyran-4-ylamino)-6-(trifluoromethyl)-1*H*-pyrazolo[3,4-*b*]pyridin-5-yl]methyl}-2-methyl-1,3-thiazole-5-carboxamide;

or a salt thereof such as a pharmaceutically acceptable salt thereof.

The structures of the above-listed specific compounds, or embodiments thereof, are given in Examples 346 to 349 hereinafter.

In one particular embodiment of the invention, the compound of formula (I) or the salt thereof can be one of the following Examples, as a compound or a (any) salt thereof, e.g. as a compound or a pharmaceutically acceptable salt thereof:

Example 77, 88, 93, 108, 120, 124, 125, 138, 146, 150, 151, 154, 155, 156, 158, 169, 173, 177, 187, 189, 191, 192, 195, 196, 198, 200, 202, 203, 206, 214, 216, 218, 219, 225, 226, 227, 228, 229, 231, 234, 237, 238, 239, 249, 259, 266, 279, 282, 283, 284, 286, 287, 289, 290, 300, 301, 307, 308, 309, 310, 312, 313, 314, 318, 319, 322, 324, 325, 326, 331, 334, 335, 336, 337, 341, 343, or 344.

The structures of the above-listed specific compounds, or embodiments thereof, are given in the Examples section hereinafter. Some of the chemical names of these compounds are given in the Examples section hereinafter, and the names are among the list of compound or salt names disclosed herein.

Alternatively, in another particular embodiment of the invention, the compound of formula (I) or the salt thereof can be one of the following Examples, as a compound or a (any) salt thereof, e.g. as a compound or a pharmaceutically acceptable salt thereof:

Example 116, 147, 148, 149, 153, 157, 159, 160, 161, 174, 175, 176, 178, 179, 180, 182, 183, 184, 185, 186, 188, 190, 193, 194, 199, 204, 205, 207, 208, 209, 210, 211, 212, 220, 232, 233, 235, 236, 240, 241, 242, 243, 244, 245, 246, 247, 248, 250, 251, 252, 253, 254, 255, 256, 257, 258, 260, 261, 262, 263, 264, 265, 267, 268, 269, 270, 271, 272, 273, 274, 275, 276, 277, 278, 280, 281, 285, 288, 302, 303, 304, 305, 306, 311, 315, 316, 317, 323, or 339.

The structures of the above-listed specific compounds, or embodiments thereof, are given in the Examples section hereinafter. Some of the chemical names of these compounds are given in the Examples section hereinafter, and the names are among the list of compound or salt names disclosed herein.

In one preferred embodiment, the compound of formula (I) or the salt thereof is a compound of Example 311, 318 or 331, as defined by the structures and/or names described herein, or a (any) salt thereof, e.g. the compound or salt can be a compound or a pharmaceutically acceptable salt thereof. The structures and names of these Examples are described in the Examples section and/or in the compounds list herein. The compound of Example 311, 318 or 331 or the salt thereof can suitably be for oral administration e.g. to a mammal such as a human. The compound of Example 311, 318 or 331, or a pharmaceutically acceptable salt thereof, can suitable be contained / comprised in a pharmaceutical composition suitable and/or adapted for oral administration, e.g. for oral administration to a mammal such as a human, monkey or rodent (e.g. rat).

In one preferred embodiment, the compound of formula (I) or the salt thereof is a compound of Example 236, 237 or 238 (in particular a compound of Example 236 or 238), as defined by the structures and/or names described herein, or a (any) salt thereof, e.g. the compound or salt can be a compound or a pharmaceutically acceptable salt thereof. The structures and names of these Examples are described in the Examples section and/or in the compounds list herein. The compound of Example 236, 237 or 238 (for example a compound of Example 236 or 238) or the salt thereof can suitably be for external topical administration (e.g. to a mammal such as a human or pig), in particular for topical administration to the skin. The compound of Example 236, 237 or 238 (for example a compound of Example 236 or 238) or a pharmaceutically acceptable salt thereof can suitably be contained / comprised in a pharmaceutical composition suitable and/or adapted for external topical administration (e.g. to a mammal such as a human or pig), in particular in a pharmaceutical composition suitable and/or adapted for topical administration to the skin (e.g. to a mammal such as a human or pig).

Therefore, in one embodiment of particular interest, the compound of formula (I) or the salt thereof (or the compound or salt of the invention in an external-topical pharmaceutical composition), e.g. the compound of formula (I) or the pharmaceutically acceptable salt thereof, can for example comprise (e.g. be):

*N*-{[1,6-diethyl-4-(tetrahydro-2*H*-pyran-4-ylamino)-1*H*-pyrazolo[3,4-*b*]pyridin-5-yl]methyl}-5-methyl-2-pyrazinecarboxamide (e.g. see Example 236) or a salt (e.g. pharmaceutically acceptable salt) thereof,

or *N*-{[1,6-diethyl-4-(tetrahydro-2*H*-pyran-4-ylamino)-1*H*-pyrazolo[3,4-*b*]pyridin-5-y)]methyl}-1-methyl-1*H*-pyrazole-3-carboxamide (e.g. see Example 238) or a salt (e.g. pharmaceutically acceptable salt) thereof.

Therefore, one particular aspect of the invention provides *N*-{[1,6-diethyl-4-(tetrahydro-2*H*-pyran-4-ylamino)-

1*H*-pyrazolo[3,4-*b*]pyridin-5-yl]methyl}-5-methyl-2-pyrazinecarboxamide

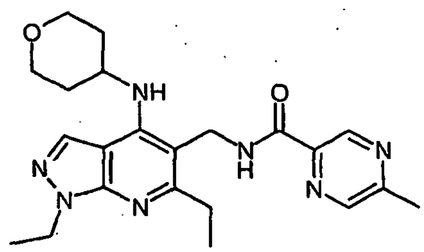

or a salt (e.g. pharmaceutically acceptable salt) thereof.

One other particular aspect of the invention provides *N*-{[1,6-diethyl-4-(tetrahydro-2*H*-pyran-4-ylamino)-1*H*-pyrazolo[3,4-*b*]pyridin-5-yl]methyl}-1-methyl-1*H*-pyrazole-3-carboxamide

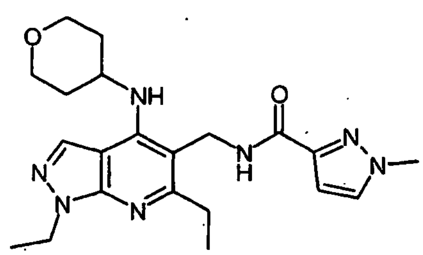

or a salt (e.g. pharmaceutically acceptable salt) thereof.

In particular, the compound of formula (I) or the salt thereof (or the compound or salt of the invention in an external-topical pharmaceutical composition), can for example comprise (e.g. be):

*N*-{[1,6-diethyl-4-(tetrahydro-2*H*-pyran-4-ylamino)-1*H*-pyrazolo[3,4-*b*]-pyridin-5-yl]methyl}-5-methyl-2-pyrazinecarboxamide (e.g. see Example 236),
or *N*-{[1,6-diethyl-4-(tetrahydro-2*H*-pyran-4-ylamino)-1*H*-pyrazolo[3,4-*b*]pyridin-5-yl]methyl}-1-methyl-1*H*-pyrazole-3-carboxamide (e.g. see Example 238), (i.e. as the compound, i.e. as the "free base" form).

In one particular embodiment, the compound of the invention or the salt thereof does not include, and/or is not, *N*-{[1,6-diethyl-4-(tetrahydro-2*H*-pyran-4-ylamino)-1*H*-pyrazolo[3,4-*b*]pyridin-5-yl]methyl}-3-methyl-5-isoxazolecarboxamide

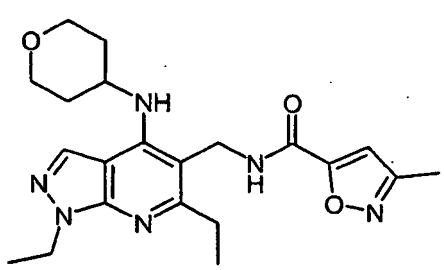

or a salt thereof.

**SALTS, SOLVATES, ISOMERS, TAUTOMERIC FORMS, MOLECULAR WEIGHTS, ETC.**

Because of their potential use in medicine, the salts of the compounds of formula (I) may be used in the form of a pharmaceutically acceptable salt thereof. Pharmaceutically acceptable salts can include acid or base addition salts.

In one embodiment, a pharmaceutically acceptable acid addition salt is optionally formed by mixing (e.g. intimately mixing) a compound of formula (I) with a suitable pharmaceutically acceptable inorganic or organic acid (such as hydrobromic, hydrochloric, sulfuric, nitric, phosphoric, succinic, maleic, formic, acetic, propionic, fumaric, citric, tartaric,

lactic, benzoic, salicylic, glutamaic, aspartic, p-toluenesulfonic, benzenesulfonic, methanesulfonic, ethanesulfonic, naphthalenesulfonic such as 2-naphthalenesulfonic, or hexanoic acid), optionally in (e.g. dissolved in) a suitable solvent, and/or optionally in (e.g. dissolved in) an organic solvent such as methanol, ethanol, isopropanol, ethyl acetate, acetonitrile and/or dichloromethane, to give the salt which is usually isolated for example by crystallisation and filtration. In one embodiment, the compound of formula (I) is for example dissolved in a suitable solvent, and/or in an organic solvent such as methanol, ethanol, isopropanol, ethyl acetate, acetonitrile and/or dichloromethane, and the pharmaceutically acceptable inorganic or organic acid (e.g. in solution or in isolated form) is then added, to prepare the pharmaceutically acceptable salt, which is usually isolated for example by crystallisation and filtration. For preparation of a hydrochloride salt, an anhydrous solution (e.g. 1-5M, e.g. 2M) of HCl in dioxane or diethyl ether, or aqueous hydrochloric acid, or HCl gas, is optionally used as the acid. For preparation of a sulfate or nitrate salt, in one embodiment an aqueous, methanolic or ethanolic solution of sulfuric acid or nitric acid respectively is optionally added to a solution of the compound of formula (I) in a water-miscible organic solvent.

A suitable pharmaceutically acceptable inorganic or organic acid can e.g. be hydrobromic, hydrochloric, sulfuric, nitric, phosphoric, p-toluenesulfonic, benzenesulfonic, methanesulfonic, ethanesulfonic, or naphthalenesulfonic such as 2-naphthalenesulfonic acid.

A pharmaceutically acceptable acid addition salt of a compound of formula (I) can comprise or be for example a hydrobromide, hydrochloride, sulfate, nitrate, phosphate, succinate, maleate, formate, acetate, propionate, fumarate, citrate, tartrate, lactate, benzoate, salicylate, glutamate, aspartate, p-toluenesulfonate, benzenesulfonate, methanesulfonate, ethanesulfonate, naphthalenesulfonate (e.g. 2-naphthalenesulfonate) or hexanoate salt of a compound of formula (I).

In one embodiment, a pharmaceutically acceptable base addition salt is optionally formed by reaction of a compound of formula (I) with a suitable inorganic or organic base (e.g. triethylamine, ethanolamine, triethanolamine, choline, arginine, lysine or histidine), optionally in a suitable solvent such as an organic solvent, to give the base addition salt which is usually isolated for example by crystallisation and filtration.

Other suitable pharmaceutically acceptable salts include pharmaceutically acceptable metal salts, for example pharmaceutically acceptable alkali-metal or alkaline-earth-metal salts such as sodium, potassium, calcium or magnesium salts; in particular pharmaceutically acceptable metal salts of one or more carboxylic acid moieties that may be present in the the compound of formula (I).

Other non-pharmaceutically acceptable salts, e.g. oxalates or trifluoroacetates, may be used, for example in the isolation of compounds of the invention, and are included within the scope of this invention.

The invention includes within its scope all possible stoichiometric and non-stoichiometric forms of the salts of the compounds of formula (I).

Also included within the scope of the invention are all solvates, hydrates and polymorphs of compounds and salts of the invention.

Certain groups, substituents, compounds or salts included in the present invention may be present as isomers, e.g. positional, geometric or optical isomers. The present invention includes within its scope all such isomers, including racemates, enantiomers and mixtures thereof.

In the compounds or salts, pharmaceutical, compositions, uses, methods of treatment/prophylaxis, methods of preparing, etc. according to the present invention, where a defined isomeric configuration e.g. stereochemical configuration is described or claimed, the invention includes a mixture comprising (a) a major component of the compound or salt which is in the described or claimed configuration, together with (b) one or more minor components of the compound or salt which is/are not in the described or claimed configuration. Preferably, in such a mixture, the major component of the compound or salt which is in the described or claimed configuration represents 70% or more, or 75% or more, more preferably 85% or more, still more preferably 90% or more, yet more preferably 95% or more, yet more preferably 98% or more, of the total amount of compound or salt present in the mixture on a molarity basis.

The percentage of one isomeric / stereochemical component in a mixture of different isomeric / stereochemical components, and if appropriate enantiomeric and/or diastereomeric excesses, can be measured using techniques known in the art. Such methods include the following:

(1) Measurement using NMR (e.g. $^1H$ NMR) spectroscopy in the presence of chiral agent. One can measure a nuclear magnetic resonance (NMR) spectrum (preferably a $^1H$ NMR spectrum, and/or a solution-phase NMR spectrum e.g. in $CDCl_3$ or D6-DMSO solvent) of the compound/salt mixture in the presence of a suitable chiral agent which "splits" the NMR peaks of a given atom in different isomers into different peak positions. The chiral agent can be: i) an optically pure reagent which reacts with the compound/salt e.g. to form a mixture of diastereomers, ii) a chiral solvent, iii) a chiral molecule which forms a transient species (e.g. diastereomeric species) with the compound/salt, or iv) a chiral shift reagent. See e.g. J. March, "Advanced Organic Chemistry", 4th edn., 1992, pages 125-126 and refs. 138-146 cited therein. A chiral shift reagent can be a chiral lanthanide shift reagent such as tris[3-trifluoroacetyl-*d*-camphorato]europium-(III) or others as described in Morrill, "Lanthanide Shift Reagents in Stereochemical

Analysis", VCH, New York, 1986. Whatever the chiral agent is that is used, usually, the relative integrals (intensities) for the NMR peaks of a given atom or group in different isomers can provide a measurement of the relative amounts of each isomer present.

(2) Measurement using chiral chromatography, especially on an analytical scale. A suitable chiral column which separates the different isomeric components can be used to effect separation, e.g. using gas or liquid chromatography such as HPLC, and/or e.g. on an analytical scale. The peaks for each isomer can be integrated (area under each peak); and a comparison or ratio of the integrals for the different isomers present can give a measurement of the percentage of each isomeric component present. See for example: "Chiral Chromatography", Separation Science Series Author: T.E. Beesley and R.P.W. Scott, John Wiley & Sons, Ltd., Chichester, UK, 1998, electronic Book ISBN: 0585352690, Book ISBN: 0471974277.

(3) Separation of pre-existing diastereomeric mixtures which are compounds/salts of the invention can be achieved (usually directly, without derivatisation) using separation techniques such as gas or liquid chromatography. Diastereomeric ratios and/or excesses can thereby be derived e.g. from the relative peak areas or relative separated masses.

(4) Conversion with a chiral / optically-active agent and subsequent separation of the resulting isomers, e.g. diastereomers. Conversion can be via derivatisation of a derivatisable group (e.g. -OH, -NHR) on the compound/salt with an optically-active derivatising group (e.g. optically active acid chloride or acid anhydride); or can be via formation of an acid or base addition salt of the compound by treatment of the compound with an optically-active acid or base, such as + or - di-para-toluoyl tartaric acid. After derivatisation, separation of the resulting isomers e.g. diastereomers, can be using gas or liquid chromatography (usually non-chiral); or (especially with isomeric salts) can be by selective crystallisation of a single isomeric e.g. diastereoisomeric salt. Determination of isomeric ratios and/or excesses can be using chromatography peak areas or measurement of mass of each separated isomer.

See e.g. J. March, "Advanced Organic Chemistry", 4th edn., 1992, pages 120-121 and 126, and refs. 105-115 and 147-149 cited therein.

(5) Measurement of optical activity [alpha] of mixture and comparison with optical activity of pure isomer $[alpha]_{max}$ if available (e.g. see J. March, "Advanced Organic Chemistry", 4th edn., 1992, page 125 and refs. 138-139 cited therein). This assumes a substantially linear relationship between [alpha] and concentration.

Certain of the groups, e.g. heteroaromatic ring systems or amide moiety(ies), included in compounds of formula (I) or their salts may exist in one or more tautomeric forms. The present invention includes within its scope all such tautomeric forms, including mixtures.

Especially when intended for oral medicinal use, the compound of formula (I) can optionally have a molecular weight of 1000 or less, for example 800 or less, in particular 650 or less or 600 or less. Molecular weight here refers to that of the unsolvated "free base" compound, that is excluding any molecular weight contributed by any addition salts, solvent (e.g. water) molecules, etc.

## SYNTHETIC PROCESS ROUTES

The following non-limiting processes can generally be used to prepare the compounds of the invention:

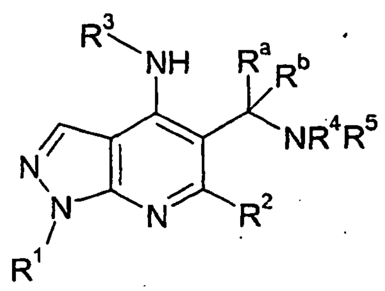

(I)

Some of the following synthetic processes may be exemplified for compounds of Formula (I) wherein $R^a$ and $R^b$ are both a hydrogen atom (H). However, at least some of these processes can be used with appropriate modification, e.g. of starting materials and reagents, for making compounds of Formula (I) wherein $R^a$ is methyl or ethyl.

**Process A**

To prepare a compound of formula (I), wherein $R^4$ is H or alkyl and $R^5$ is -C(O)-R' wherein R' is any group covered by the invention, (in particular wherein $R^5$ is $-C(O)-(CH_2)_n-Ar$, -C(O)-Het, $-C(O)-C_{1-6}alkyl$, $-C(O)-C_1fluoroalkyl$, $-C(O)-(CH_2)_2-C(O)-NR^{15b}NR^{15b}$, or $-C(O)-CH_2-C(O)-NR^{15b}NR^{15b}$), an amine of formula (II) or a salt thereof can be reacted with a compound $R'-C(O)-X^1$, wherein $X^1$ is a leaving group substitutable by the $NHR^4$ amine moiety of the compound of formula (II):

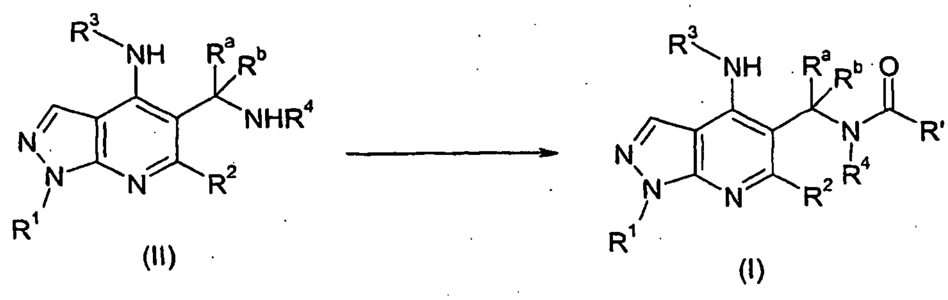

$R'-C(O)-X^1$ is typically an activated derivative of carboxylic acid R'-C(O)-OH. In the reaction of (II) to (I), a compound $R'-C(O)-X^1$ can for example be the acid chloride R'-C(O)Cl ($X^1$ = Cl). For use of R'-C(O)Cl, the reaction is typically carried out in the presence of a base such as diisopropylethylamine ($^iPr_2NEt$ = DIPEA) and/or in a suitable non-aqueous non-alcohol organic solvent such as acetonitrile (e.g. anhydrous), for example at room temperature (e.g. about 18 to about 25 °C).

The acid chloride R'-C(O)Cl can typically be formed from the carboxylic acid by reaction with thionyl chloride, either in an organic solvent such as chloroform or without solvent.

Alternatively, the compound $R'-C(O)-X^1$ can be an activated derivative of the carboxylic acid R'-C(O)-OH wherein the leaving group $X^1$ is

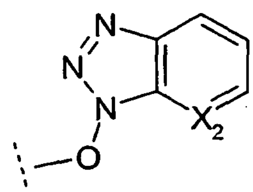

$X_2$ = CH or N

The latter activated compounds $R'-C(O)-X^1$ is typically formed from the carboxylic acid R'-C(O)-OH either:

(a) by reaction of the carboxylic acid with a carbodiimide such as EDC, which is 1-ethyl-3-(3'-dimethylaminopropyl) carbodiimide and is also 1-(3-dimethylaminopropyl)-3-ethylcarbodiimide, or a salt thereof e.g. hydrochloride salt, usually followed by reaction of the resulting product with 1-hydroxybenzotriazole (HOBT);
reaction (a) usually being carried out in the presence of a solvent (e.g. anhydrous) such as dimethyl formamide (DMF) or acetonitrile and/or usually under anhydrous conditions and/or e.g. at room temperature;
or:

(b) by reaction with 2-(1H-benzotriazole-1-yl)-1,1,3,3-tetramethyluronium tetrafluoroborate (TBTU) or O-(7-Azabenzotriazol-1-yl)-N,N,N',N'-tetramethyluronium hexafluorophosphate (HATU) or benzotriazole-1-yl-oxy-trispyrrolidiriophosphonium hexafluorophosphate (PyBOP);
reaction (b) usually being carried out in the presence of a base such as diisopropylethylamine ($^iPr_2NEt$ = DIPEA), and/or usually in the presence of a solvent such as dimethyl formamide (DMF) or acetonitrile, and/or for example under anhydrous conditions and/or for example at room temperature.

Alternatively, e.g. when R' = $CF_3$, in the compound $R'-C(O)-X^1$ the leaving group $X^1$ can sometimes be O-$C_{1-4}$alkyl such as OEt.

Amine compounds of formula (II) wherein $R^4$ is H (and in particular wherein $R^a$ and $R^b$ are H) can generally be prepared by hydrogenation of an azide compound of formula (IIIa):

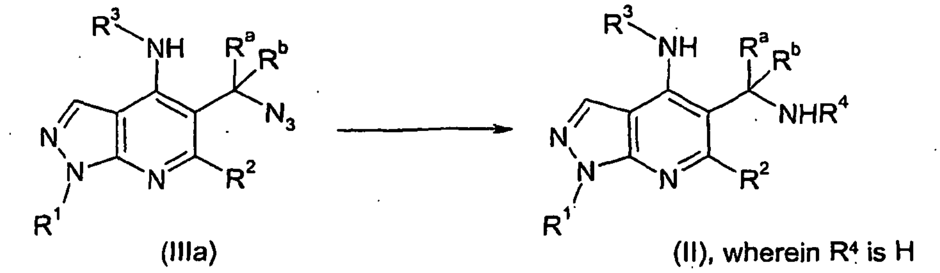

(IIIa) (II), wherein $R^4$ is H

Typical hydrogenation conditions can include $H_2$ / palladium on carbon.

Azide compounds of formula (IIIa), in particular wherein $R^a$ and $R^b$ are H, can generally or sometimes be prepared by reaction of a compound of formula (IIIb), wherein $X^{3b}$ is a leaving group displaceable by azide, with a metal azide such as lithium azide:

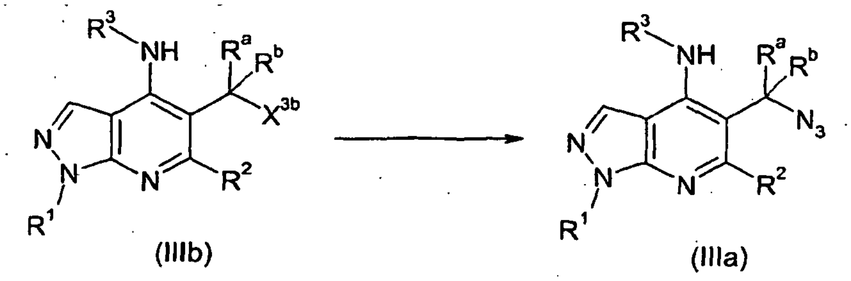

(IIIb) (IIIa)

Typical conditions for the (IIIb) to (IIIa) reaction, e.g. with lithium azide, can e.g. include DMSO solvent at room temperature. See for example Intermediates 11, 12 and 13. In one embodiment, $X^{3b}$ is a chlorine atom (Cl) or an organic sulfonate such as trifluoromethanesulfonate $CF_3SO_2$- or p-toluenesulfonate.

Compounds of formula (IIIb), in particular wherein $X^{3b}$ is Cl, and in particular wherein $R^a$ and $R^b$ are H, can generally or sometimes be prepared by conversion (e.g. chlorination) of an alcohol compound of formula (IIIc), e.g. with $SOCl_2$ for chlorination:

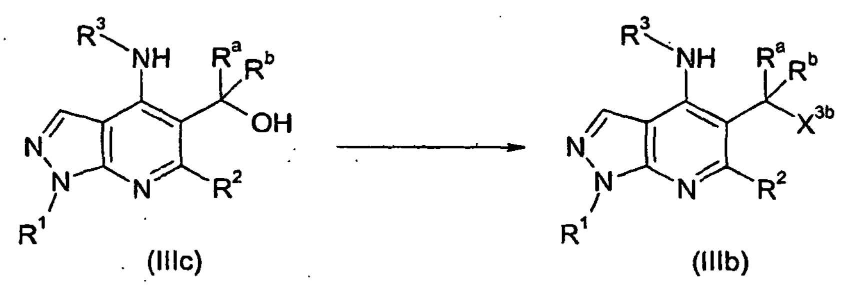

(IIIc) (IIIb)

Compounds of formula (IIIc), wherein $R^a$ and $R^b$ are H, can generally be prepared by reduction of a compound of formula (IV), an ester, for example in the presence of diisobutylaluminium hydride e.g. in dichloromethane solvent:

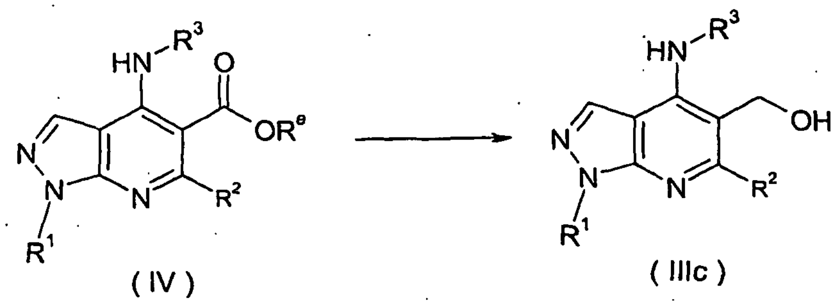

(IV) (IIIc)

Compounds of formula (IV) can generally be prepared according to a method, for example as described by Yu et. al. in J. Med Chem., 2001, 44, 1025-1027, by reaction of a compound of formula (V) with an amine of formula $R^3NH_2$. The reaction is typically carried out in the presence of a base such as triethylamine or *N*,*N*-diisopropylethylamine, and/or in an organic solvent such as ethanol, dioxane or acetonitrile. The reaction may require heating e.g. to ca. 60-100°C, for example ca. 80-90°C:

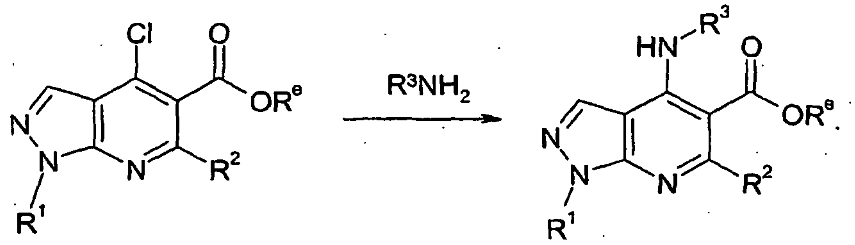


(V) (IV)

Compounds of formula (V) are also described in the above reference. They can typically be prepared by reaction of a compound of formula (VI) with $(R^2)(OEt)C{=}C(CO_2R^e)_2$ or $(R^2)(Cl)C{=}C(CO_2R^e)_2$, which can for example be diethyl (ethoxymethylene)malonate (wherein $R^2$ is H and $R^e$ is Et) or diethyl 2-(1-ethoxyethylidene)malonate (wherein $R^2$ is Me and $R^e$ is Et) or diethyl (1-chloropropylidene)propanedioate (wherein $R^2$ is Et and $R^e$ is Et), with heating, followed by reaction with phosphorous oxychloride, again with heating:

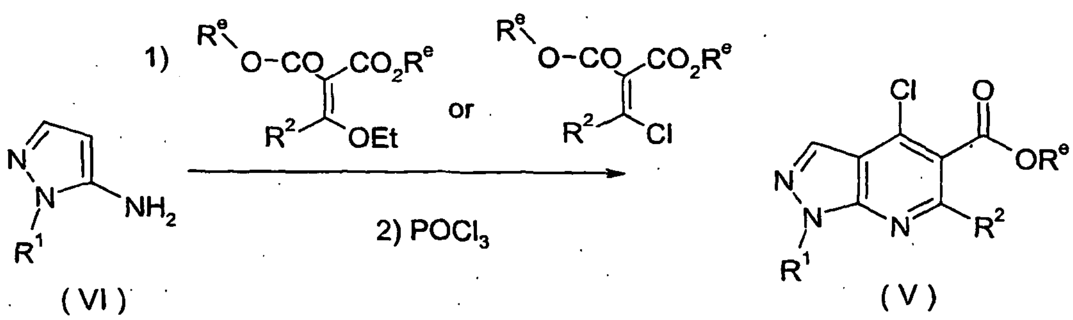


(VI) (V)

For examples of the compound (VI) to compound (V) process, see for example: (i) the Intermediate 51 synthesis and e.g. G. Yu et. al., J. Med Chem., 2001, 44, 1025-1027 hereinafter, where $R^2$ = H and $R^1$ = ethyl; and see e.g. (ii) the Intermediate 60 synthesis hereinafter where $R^2$ = methyl and $R^1$ = ethyl; and see e.g. (iii) Intermediate 182 synthesis hereinafter wherein $R^2$ = H and $R^1$ = methyl (i.e. reaction of 5-amino-1-methyl pyrazole with diethyl ethoxymethylene malonate); and see e.g. (iv) Intermediate 1 synthesis hereinafter wherein $R^2$ = ethyl and $R^1$ = ethyl.

Where the desired amino pyrazole of formula (VI) is not commercially available, preparation of the amino pyrazole (VI) can sometimes be achieved, for example, using methods described by Dorgan et. al. in J. Chem. Soc., Perkin Trans. 1, (4), 938-42; 1980, by reaction of cyanoethyl hydrazine with a suitable aldehyde of formula $R^{40}CHO$ in a solvent such as ethanol, with heating, followed by reduction, for example reduction with sodium in a solvent such as t-butanol. $R^{40}$ should be chosen so as to contain one less carbon atom than $R^1$, for example $R^{40}$ = methyl will afford $R^1$

= ethyl.

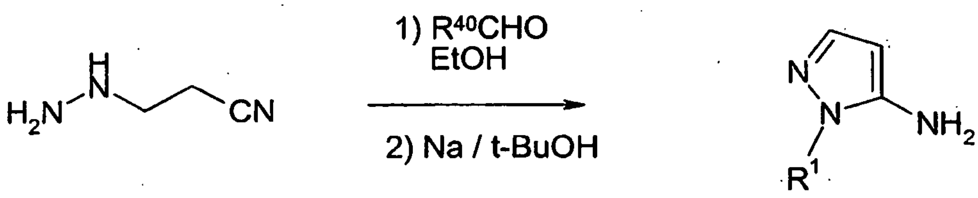


( VI )

Alternatively, e.g. where the desired amino pyrazole of Formula (VI) is not commercially available, preparation of the 4-amino 5-ester compounds of Formula (IV) can be achieved from a (different $R^1$) 4-chloro 5-ester compound of Formula (V) (e.g. Intermediate 51, wherein $R^1$ = ethyl and $R^2$ = H), using a generalised version of the reaction scheme shown in Intermediate 170 and shown below. In this method:

- the 4-chloro 5-ester pyrazolopyridine of Formula (V) (e.g. Intermediate 51) is optionally converted to the 4-alkoxy (e.g. $C_{1-4}$alkoxy such as ethoxy) pyrazolopyridine;
- the $R^1$ group is removed (e.g. using N-bromosuccinimide (NBS) and preferably base e.g. $Na_2CO_3$) (e.g. to give intermediate 51A - an alternative synthesis for which is given under "Intermediate 51A" hereinafter);
- the 4-amino $NHR^3$ group is inserted by displacing the 4-chloro or 4-alkoxy group by reaction with $R^3NH_2$;
- and the resulting pyrazolopyridine is alkylated at N-1 by reacting it with $R^1$-$X^{41}$, where $X^{41}$ is a group displaceable by the N-1 nitrogen of the pyrazolopyridine, in order to reinsert the desired $R^1$ group [i.e. to prepare the 4-amino 5-ester compound of Formula (IV)]. $X^{41}$ can for example be a halogen, e.g. Cl, Br or I; or $X^{41}$ can be -O-S(O)$_2$-$R^{41}$ where $R^{41}$ is $C_{1-4}$alkyl, $C_{1-2}$fluoroalkyl, or phenyl optionally, substituted by $C_{1-2}$alkyl.

The N-1 alkylation reation with $R^1$-$X^{41}$ is preferably carried out in the presence of base - see the (IX) to (IV) reaction hereinafter for examples of suitable bases.

The scheme below (scheme for Intermediates 170 and 171) shows a suitable exemplary route and conditions for this $R^1$ removal and re-insertion route, for insertion of $R^1$ = n-propyl and $R^3$ = tetrahydro-2H-pyran-4-yl, when $R^2$ = a hydrogen atom (H):

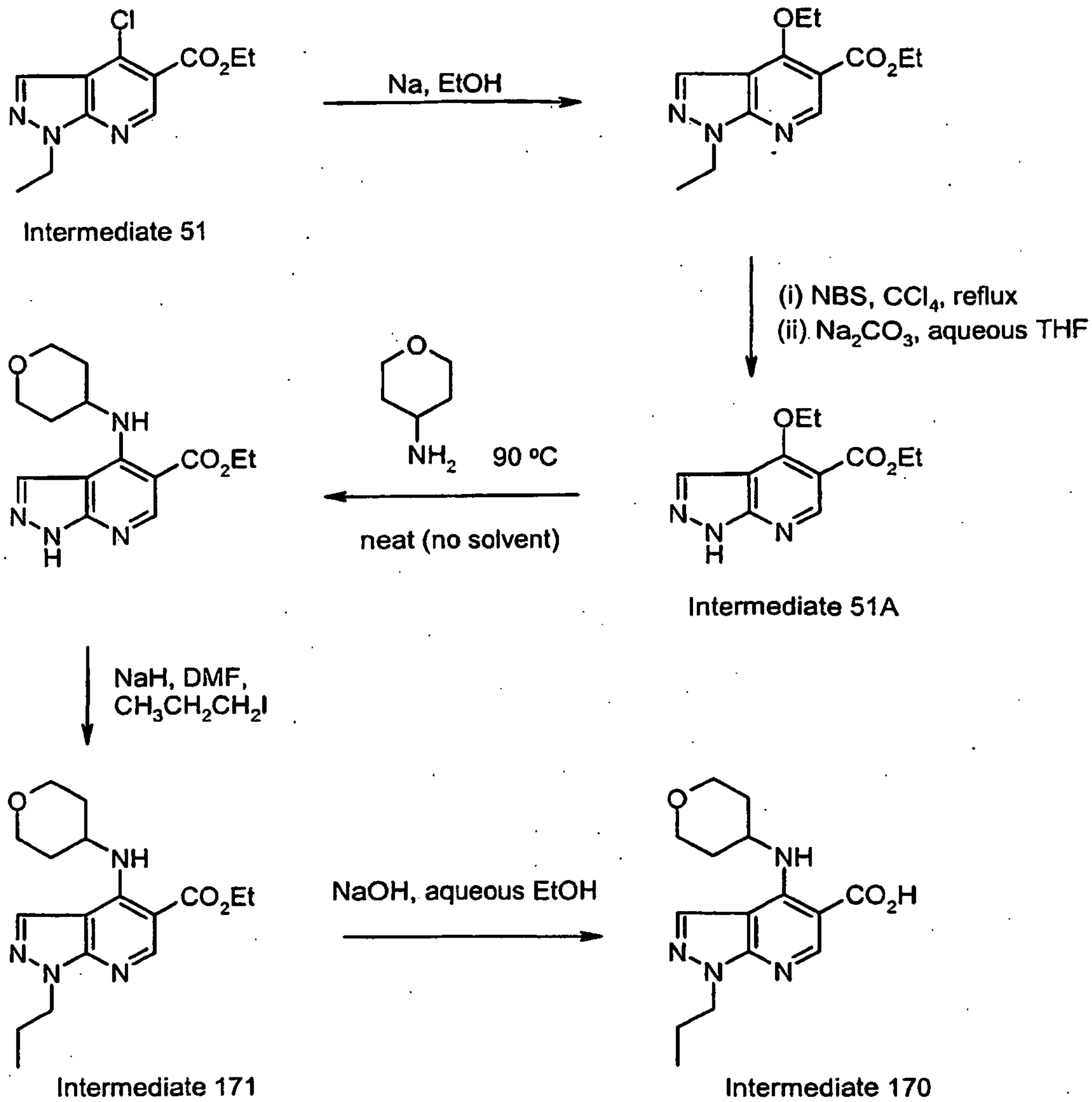


Intermediate 51

Intermediate 51A

Intermediate 171

Intermediate 170

In an alternative embodiment of Process A, the 4-chloro substituent in the compound of formula (V) can be replaced by another halogen atom, such as a bromine atom, or by another suitable leaving group which is displaceable by an amine of formula $R^3NH_2$. The leaving group displaceable by the amine can for example be $R^{LA}$, in a compound of formula (Va), wherein $R^{LA}$ is an alkoxy group $OR^{35}$ such as $OC_{1-4}$alkyl (in particular OEt) or a group $-O-S(O)_2-R^{37}$. Here, $R^{37}$ is $C_{1-8}$alkyl (e.g. $C_{1-4}$alkyl or $C_{1-2}$alkyl such as methyl), $C_{1-6}$fluoroalkyl (e.g. $C_{1-4}$fluoroalkyl or $C_{1-2}$fluoroalkyl such as $CF_3$ or $C_4F_9$), or phenyl wherein the phenyl is optionally substituted by one or two of independently $C_{1-2}$alkyl, halogen or $C_{1-2}$alkoxy (such as phenyl or 4-methyl-phenyl). The reaction of the compound of formula (Va) with the amine of formula $R^3NH_2$ may be carried out with or without solvent and may require heating:

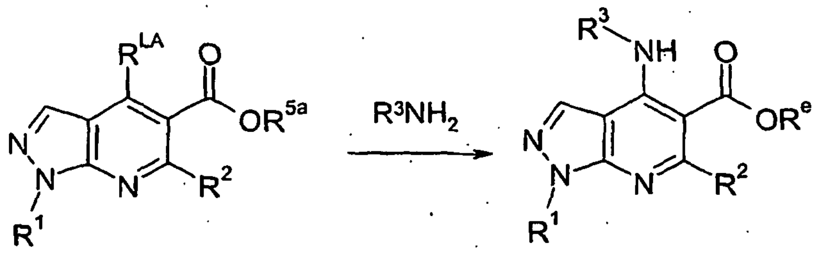


(Va) (IV)

In another alternative embodiment of Process A, the compound of formula (IV), described herein, can be prepared by reaction of a compound of formula (IX) with an alkylating agent of formula $R^1$-$X^3$, where $X^3$ is a leaving group displaceable by the 1-position pyrazolopyridine nitrogen atom of the compound of formula (IX):

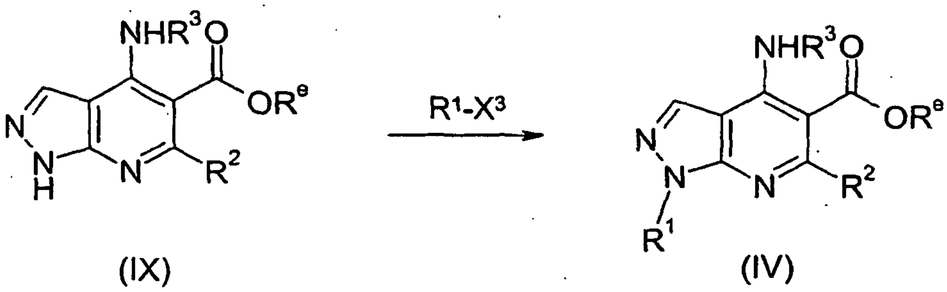


(IX) (IV)

A suitable alkylating agent of formula $R^1$-$X^3$ can be used. For example, $X^3$ can be a halogen atom such as a chlorine atom or more preferably a bromine or iodine atom, or $X^3$ can be -O-S(O)$_2$-$R^{36}$ wherein $R^{36}$ is $C_{1-8}$alkyl (e.g. $C_{1-4}$alkyl or $C_{1-2}$alkyl such as methyl), $C_{1-6}$fluoroalkyl (e.g. $C_{1-4}$fluoroalkyl or $C_{1-2}$fluoroalkyl such as $CF_3$ or $C_4F_9$), or phenyl wherein the phenyl is optionally substituted by one or two of independently $C_{1-2}$alkyl, halogen or $C_{1-2}$alkoxy (such as phenyl or 4-methyl-phenyl). The reaction is preferably carried out in the presence of a base; the base can for example comprise or be potassium carbonate, sodium carbonate, sodium hydride, potassium hydride, or a basic resin or polymer such as polymer-bound 2-tert-butylimino-2-diethylamino-1,3-dimethyl-perhydro-1,3,2-diazaphosphorine. The reaction is preferably carried out in the presence of a solvent, e.g. an organic solvent such as DMF; the solvent is preferably anhydrous.

Compounds of formula (IX) can be prepared, using a method analogous to that used for the preparation of compounds of formula (IV) from compounds of formula (V), by reaction of a compound of formula (X) (which is the same as compound of formula (V) but wherein $R^1$ = H) with an amine of formula $R^3NH_2$. The reaction is suitably carried out in the presence of a base such as triethylamine or N,N-diisopropylethylamine, and/or in an organic solvent such as ethanol, dioxane or acetonitrile. The reaction may require heating e.g. to ca. 60-100˚C, for example ca. 80-90˚C:

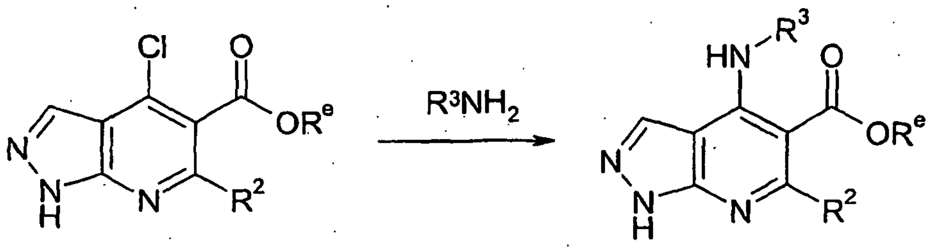


(X) (IX)

Alternatively, in formula (X), the 4-chloro can be replaced by 4-$C_{1-4}$alkoxy such as 4-ethoxy; these modified compounds, of formula (Xa), can optionally be made as described above, e.g. see the Intermediate 170 scheme shown and described above or Intermediate 51A below.

**Process B**

To prepare a compound of formula (I), wherein $R^4$ is H or alkyl and $R^5$ is $-S(O)_2-R''$ wherein R" is any group covered by the invention, (in particular wherein $R^5$ is $-S(O)_2-(CH_2)_m{}^2-Ar$, $-S(O)_2$-Het, or $-S(O)_2-C_{1-6}$alkyl), an amine of formula (II) or a salt thereof can be reacted with a compound $R''-S(O)_2-X^s$, wherein $X^s$ is a leaving group substitutable by the $NHR^4$ amine moiety of the compound of formula (II):

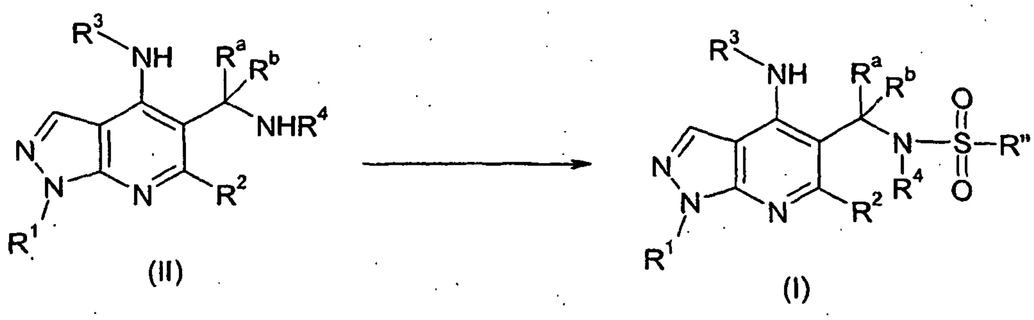

(II) (I)

$R''-S(O)_2-X^s$ is typically an activated derivative of sulfonic acid $R''-S(O)_2OH$. In the reaction of (II) to (I), a compound $R''-S(O)_2-X^s$ can for example be the sulfonyl chloride $R''-S(O)_2Cl$ ($X^s$ = Cl). For use of $R''-S(O)_2Cl$, the reaction is typically carried out in the presence of a base such as diisopropylethylamine ($^iPr_2NEt$ = DIPEA) and/or in a suitable non-aqueous non-alcohol organic solvent such as acetonitrile, for example at room temperature. See for example Examples 95 to 109 or Examples 168 to 173.

**Process C**

Process C to make compounds of formula (I), e.g. wherein $R^5$ is $-CH_2-Ar$, involves reaction of an amine of formula (II) or a salt thereof, as described herein, with R'CHO (in this case, Ar-CHO) as follows. This can be e.g. as shown below for the example wherein $NHR^3$ is of sub-formula (h), $R^1$= Et, and $R^a$ = $R^b$ = H:

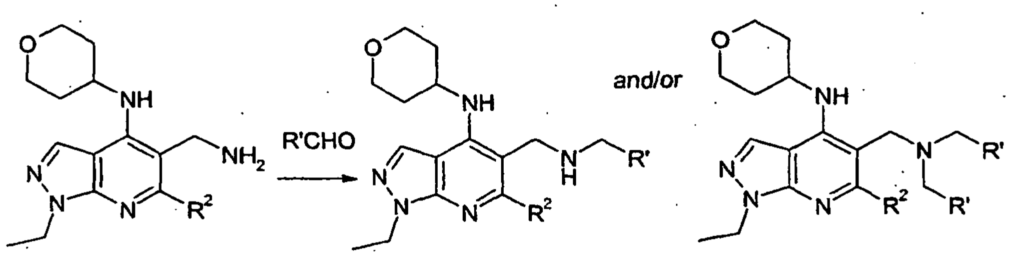


and/or

Possible conditions for Process C include $NaBH(OAc)_3$ /AcOH /THF. See e.g. Examples 135 and 137 to 143.

**Process D**

Process D to make compounds of formula (I) [e.g. wherein $R^5$ is $-CH_2-Ar$ or wherein $R^4$ and $R^5$ taken together are $-(CH_2)p^1-$ (optionally substituted), or perhaps $-(CH_2)_2-X^5-(CH_2)_2-$], involves reaction of a compound of formula (IIIb) or a salt thereof, as described herein, with $HNR^4R^5$. The leaving group $X^{3b}$ is suitably a chlorine atom (Cl) or optionally an organic sulfonate such as trifluoromethanesulfonate $CF_3SO_2-$ or p-toluenesulfonate.

Process D can be e.g. as shown below for the example wherein $NHR^3$ is of sub-formula (h), $R^1$ = Et, and $R^a$ = $R^b$ = H, and $X^{3b}$ is a chlorine atom (Cl):

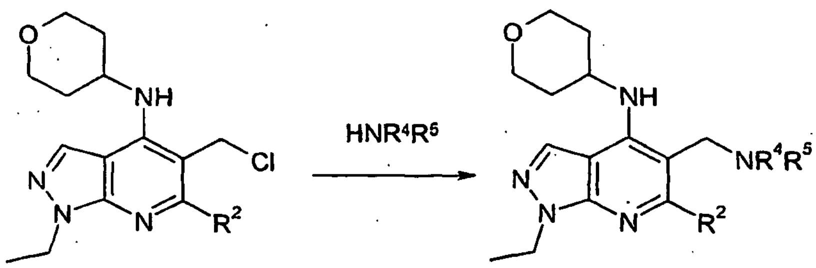


The Process D reaction is typically carried out in the presence of DIPEA and DMF, e.g. at 70°C. See e.g. Examples 1 to 83. $NR^4R^5$ may optionally contain a protecting group which may optionally be deprotected after the Process D reaction (eg $COO^tBu$ to COOH).

**Process E**

Process E comprises, optionally, reaction of an amine of formula (II) or a salt thereof (but wherein $R^5$ = H), as described herein, with a reagent suitable for adding $CF_3C(O)$- or MeC(O)- to the $NR^4$ nitrogen (e.g. $CF_3C(O)OEt$); and then alkylation of the $NR^4$ nitrogen with $R^5$ (e.g. using $R^5$-[leaving group] such as $R^5$-iodide); followed by optional removal of the $CF_3C(O)$- group from the $NR^4$ nitrogen e.g. using NaOH. Process E can e.g. be as shown below for an example wherein $NHR^3$ is of sub-formula (h), $R^1$ = Et, $R^2$ = H, and $R^a = R^b$ = H; and wherein $CF_3C(O)$- is added to the NR4 nitrogen:

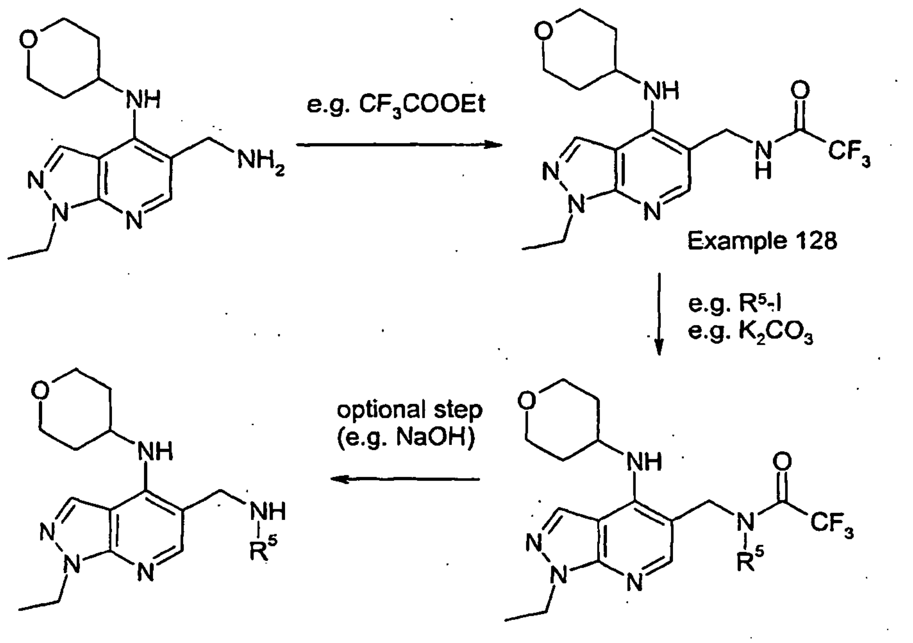


e.g. Example 128 for $R^5$ = benzyl

**Process F**

To make compounds of formula (I) wherein $R^a$ is methyl or ethyl, the $R^a$ group can be inserted by reacting the 5-imine derivative of a compound of formula (I) wherein $R^a$ is H, with reagent capable of adding $R^a$ to the imine (such as a Grignard reagent for example $R^aMgBr$, e.g. EtMgBr or MeMgBr, for example in THF solvent, e.g. at less than 0°C such as at about -10°C to about -5°C).

This reaction is illustrated in a non-limiting manner below for an example wherein $NHR^3$ is of sub-formula (h), $R^1$ = Et, $R^2$ = H, and Ra = $R^b$ = H:

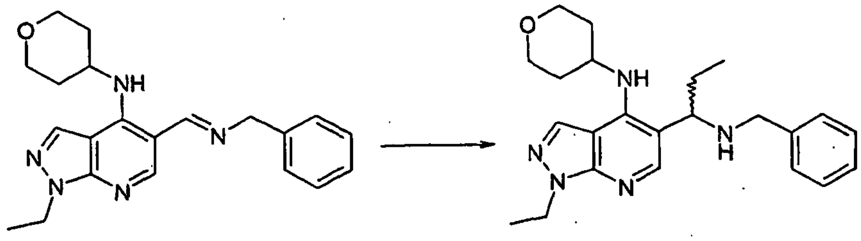

See for example Example 134. The exemplary imine illustrated above is optionally prepared as follows:

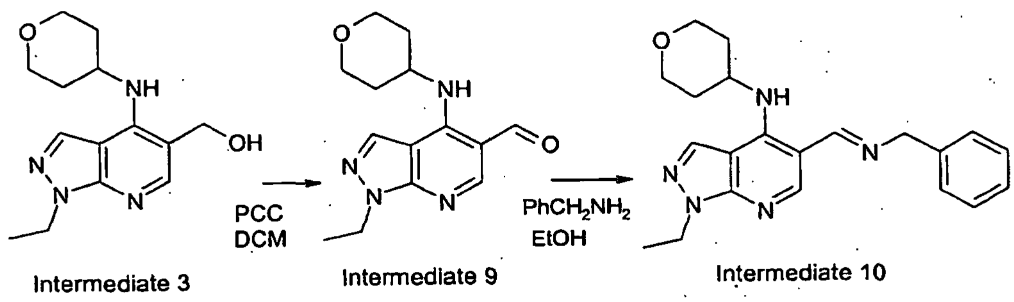


**Process G**

Possible alternative processes G1, G2, G3, G4, and/or G5 for making compounds of formula (I) wherein $R^2$ is a $C_1$fluoroalkyl (e.g. $CF_3$ or $CHF_2$, or $CH_2F$), cyano (-CN), or $-CH_2OH$ are as follows, generally illustrated for non-limiting examples wherein $NHR^3$ is of sub-formula (h), $R^1$ = Et, and $R^a$ = $R^b$ = H:

G1. To prepare compounds wherein $R^2$ is cyano (-CN), the following intermediates are for example made:

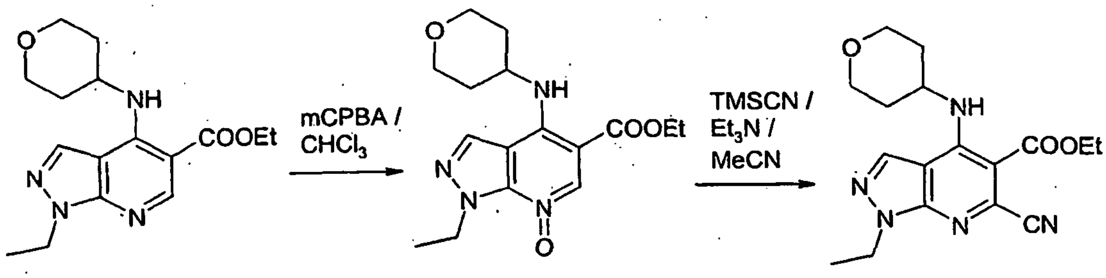


G2. To prepare compounds wherein $R^2$ is $CF_3$, the following intermediates are for example made:

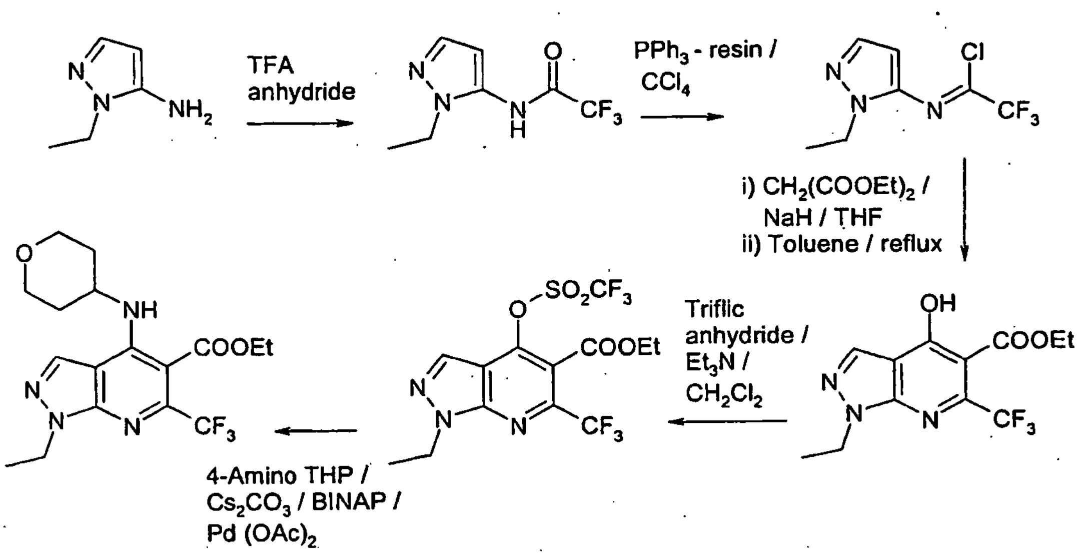


G3. To prepare compounds wherein $R^2$ is $CHF_2$ or $CH_2F$, the following intermediate, are for example made:

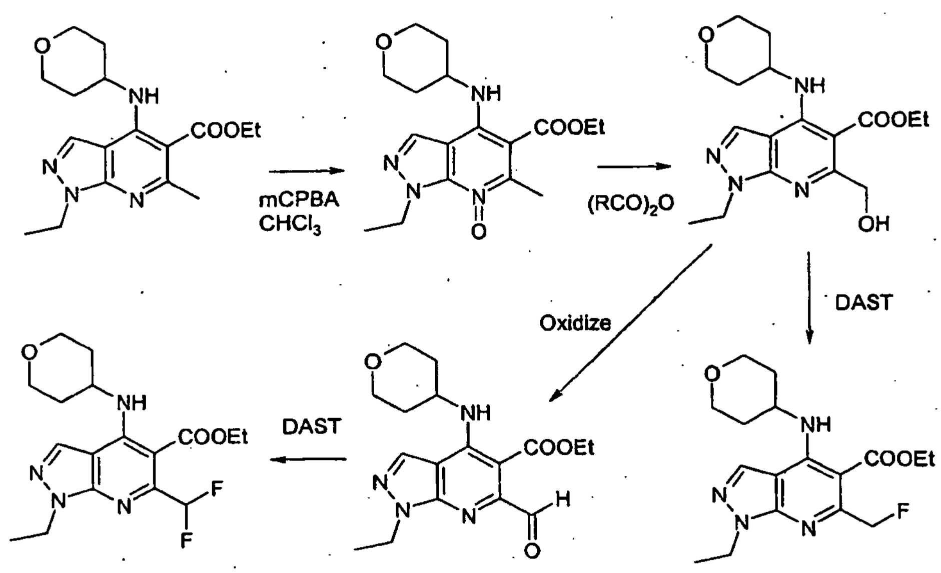


G4. To OH-protect compounds wherein $R^2$ is $CH_2OH$ the following intermediates are for example made:

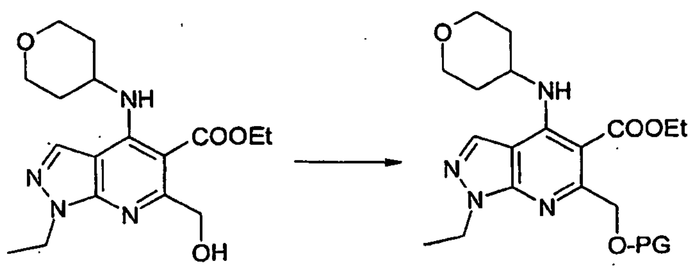

PG = OH-protecting group

After Processes G1, G2, G3 and/or G4, the following steps can be performed, generally as described before, for example to form the compounds wherein $R^5$ is -C(O)-R' such as -C(O)-Ar:

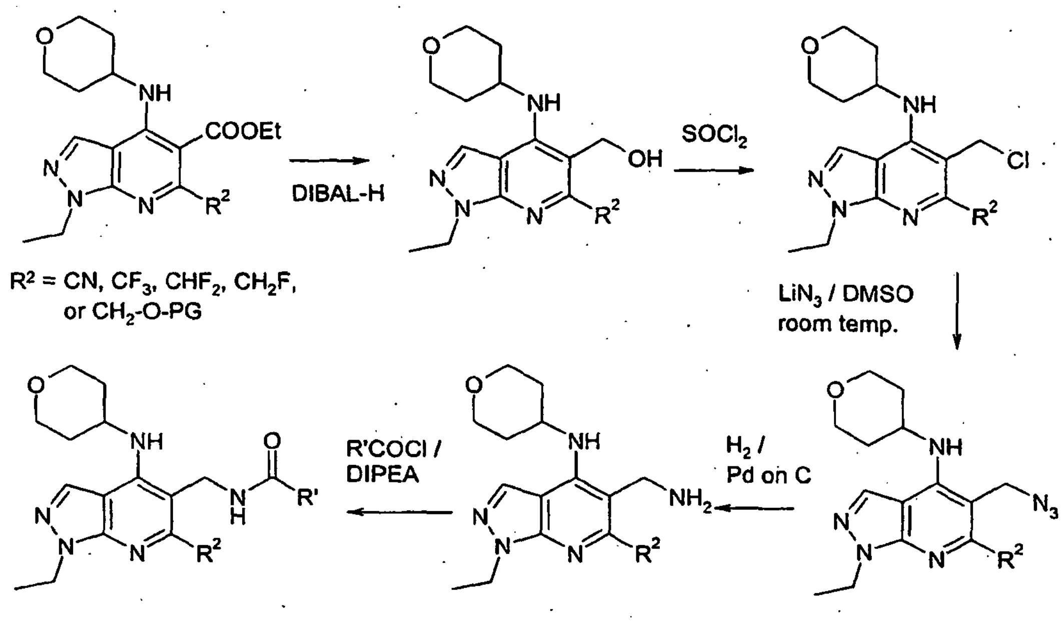


To obtain, $R^2$ is $CH_2OH$, the OH protecting group PG can then be removed to prepare the compound of formula (I) wherein $R^2$ is $CH_2OH$, and e.g. $R^5$ is -C(O)-R' such as -C(O)-Ar, e.g. as follows:

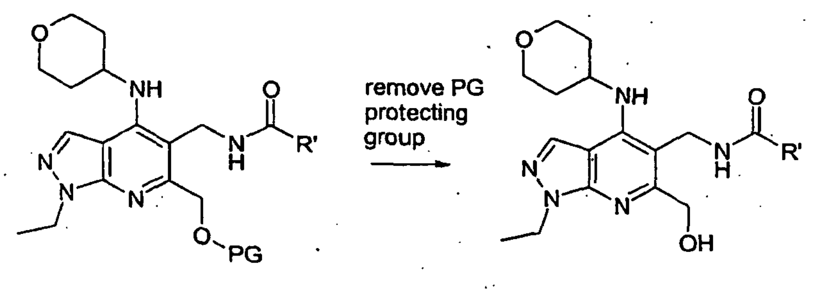


**Process H**

Where $R^4$ and $R^5$ taken together are $-C(O)-(CH_2)p^2-$, the following process is for example used, illustrated for a non-limiting example wherein $NHR^3$ is of sub-formula (h), $R^1$ = Et, and $R^a$ = $R^b$ = H:

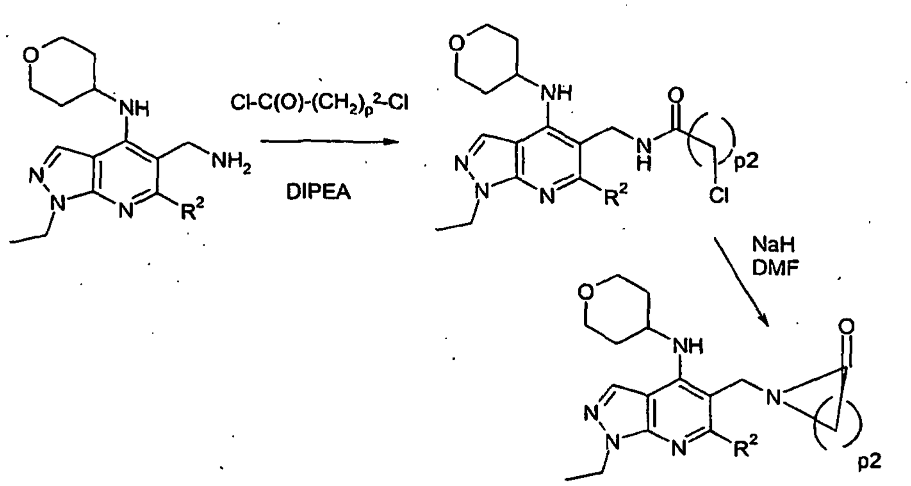

**Process I**

When $NR^4R^5$ is of sub-formula (y), (y1), (y2) or (y3), the following Process I is for example used, illustrated fora non-limiting example wherein $NHR^3$ is of sub-formula (h), $R^1$ = Et, and $R^a$ = $R^b$ = H:

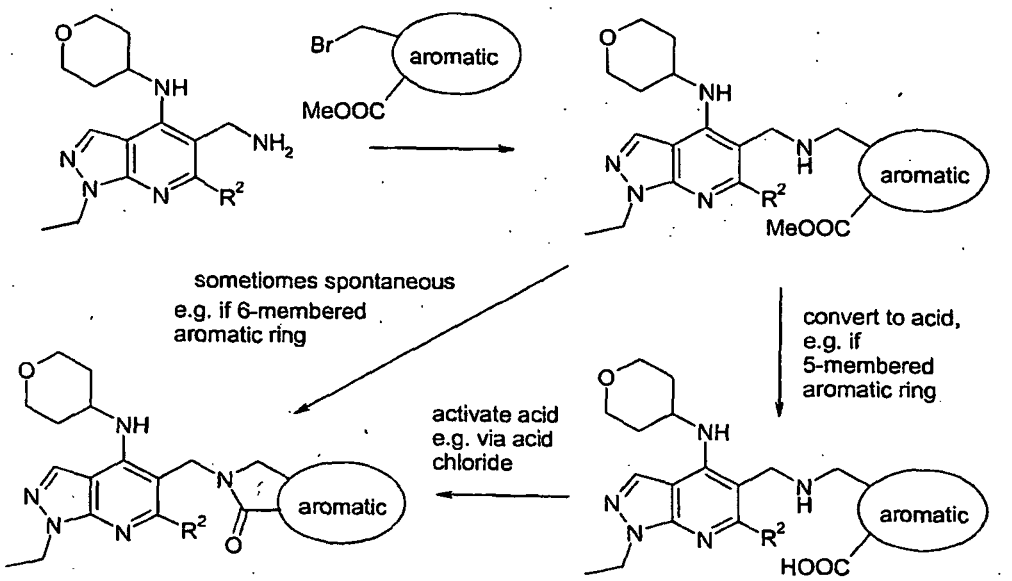


The starting material from the above reaction,

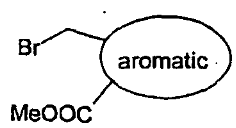

,

can be obtained by reaction of

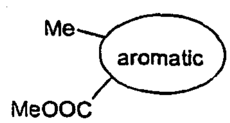

with N-bromosuccinimide (NBS).

**Process J**

Compounds of formula (I) wherein $R^5$ is -C(O)-NR'''R'''', for example $-C(O)-NR^{15b}-(CH_2)_m{}^1$-Ar, $-C(O)-NR^{15b}$-Het, $-C(O)-NR^{15b}-C_{1-6}$alkyl, or $-C(O)-NR^{5a}R^{5b}$], are for example made as follows, illustrated for a non-limiting example wherein $NHR^3$ is of sub-formula (h), $R^1$ = Et, and $R^a$ = $R^b$ = H:

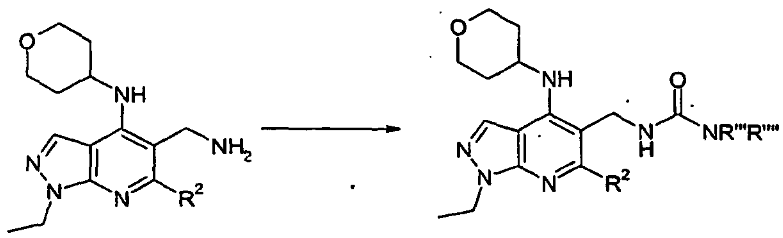

DIPEA is optionally used, and/or the starting material used is optionally, for example:

(a) 4-$NO_2$-phenyl-O-C(O)-NR'''R'''' (e.g. from 4-$NO_2$-phenyl-O-C(O)Cl and HNR'''R''''), or
(b)

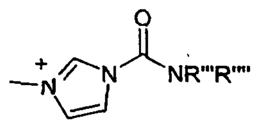 iodide (e.g. from 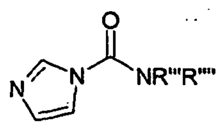 and methyl iodide).

**Process K**

Compounds of formula (I) wherein $R^4$ and $R^5$ taken together are -C(O)-N($R^{15}$)-$(CH_2)_{p}{}^3$-, are optionally prepared as follows, illustrated for a non-limiting example wherein $NHR^3$ is of sub-formula (h), $R^1$ = Et, and $R^a$ = $R^b$ = H:

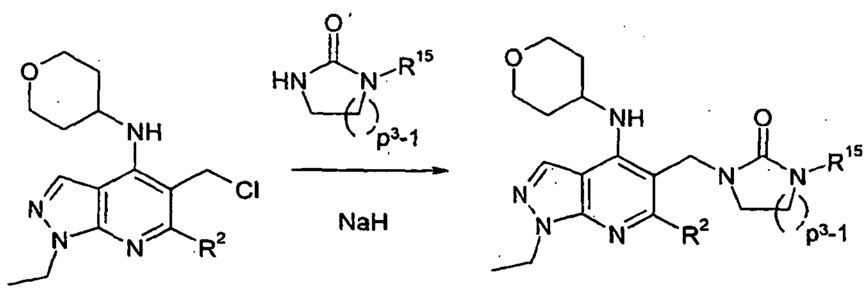


**Process L**

It is possible that compounds of formula (I) might also be preparable by reaction of a compound of formula (VII) with an amine of formula $R^3NH_2$. In the compound of formula (VII), $R^{LB}$ is a leaving group which is displaceable by the amine of formula R3$NH_2$. $R^{LB}$ can e.g. be a bromine atom (Br) or more particularly a chlorine atom (Cl), or alternatively $R^{LB}$ can be an alkoxy group $OR^{35}$ such as $OC_{1-4}$alkyl (in particular OEt) or a group -O-S$(O)_2$-$R^{37}$. Here, $R^{37}$ is $C_{1-8}$alkyl (e.g. $C_{1-4}$alkyl or $C_{1-2}$alkyl such as methyl), $C_{1-6}$fluoroalkyl (e.g. $C_{1-4}$fluoroalkyl or $C_{1-2}$fluoroalkyl such as $CF_3$ or $C_4F_9$), or phenyl wherein the phenyl is optionally substituted by one or two of independently $C_{1-2}$alkyl, halogen or $C_{1-2}$alkoxy (such as phenyl or 4-methyl-phenyl). The reaction of (VII) to (I) is optionally carried out in the presence of a base, such as triethylamine or N,N-diisopropylethylamine, and/or in an organic solvent such as ethanol, THF, dioxane or acetonitrile. The reaction may require heating, e.g. to ca. 60-100 °C or ca. 80-90 °C, for example for 8-48 or 12-24 hours:

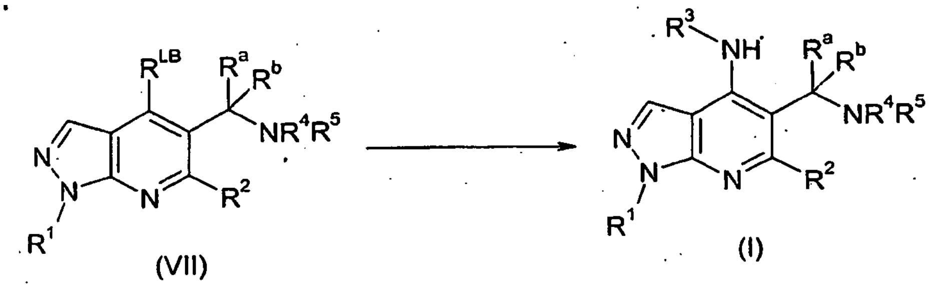

(VII) (I)

**Process M**

In optional Process M, a compounds of formula (I) is e.g. prepared by reaction of a compound of formula (IXa) with an alkylating agent of formula $R^1$-$X^3$, where $X^3$ is a leaving group displaceable by the 1-position pyrazolopyridine nitrogen atom of the compound of formula (IXa):

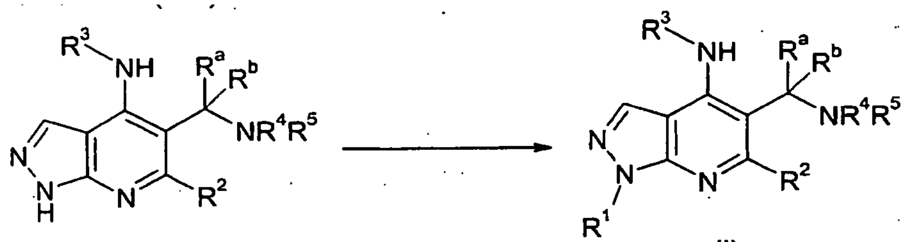

(IXa) (I)

A suitable alkylating agent of formula $R^1$-$X^3$ can be used. For example, $X^3$ is e.g. a halogen atom such as a chlorine atom or more preferably a bromine or iodine atom, or $X^3$ can be $-O-S(O)_2-R^{36}$ wherein $R^{36}$ is $C_{1-8}$alkyl (e.g. $C_{1-4}$alkyl or $C_{1-2}$alkyl such as methyl), $C_{1-6}$fluoroalkyl (e.g. $C_{1-4}$fluoroalkyl or$C_{1-2}$fluoroalkyl such as $CF_3$ or $C_4F_9$), or phenyl wherein the phenyl is optionally substituted by one or two of independently $C_{1-2}$alkyl, halogen or $C_{1-2}$alkoxy (such as phenyl or 4-methyl-phenyl). The reaction is optionally carried out in the presence of a base; the base can for example comprise or be potassium carbonate, sodium carbonate, sodium hydride, potassium hydride, or a basic resin or polymer such as polymer-bound 2-tert-butylimino-2-diethylamino-1,3-dimethyl-perhydro-1,3,2-diazaphosphorine. The reaction is e.g. carried out in the presence of a solvent, e.g. an organic solvent such as DMF; the solvent can be anhydrous.

**Process N:** *Conversion of one compound of formula (I), or a salt thereof into another compound of formula (I) or a salt thereof*

In optional Process N, one compound of formula (I) or salt thereof (or a protected version thereof, such as an N-protected version e.g. BOC-N-protected) is optionally converted into another compound of formula (I) or a salt thereof. This conversion optionally comprises or is one or more of the following processes N1 to N10:

N1. Conversion of a ketone into the corresponding oxime.

N2. An oxidation process. For example, the oxidation process can comprise or be oxidation of an alcohol to a ketone (e.g. using Jones reagent) or oxidation of an alcohol or a ketone to a carboxylic acid. The oxidation process can e.g. comprise or be conversion of a nitrogen-containing compound of formula (I) or salt thereof to the corresponding N-oxide (e.g. using *meta*-chloroperoxybenzoic acid), for example conversion of a pyridine-containing compound to the corresponding pyridine N-oxide (e.g. see Examples 210-212 of PCT/EP03/11814 (WO 2004/024728 A2), incorporated herein by reference, for suitable process details).

N3. A reduction process, for example reduction of a ketone or a carboxylic acid to an alcohol.

N4. Acylation, for example acylation of an amine (e.g. see Examples 329-349 and Example 353 of PCT/EP03/11814 (WO 2004/024728 A2), incorporated herein by reference, for suitable process details), or acylation of a hydroxy group.

N5. Alkylation, for example alkylation of an amine or of a hydroxy group.

N6. Hydrolysis, e.g. hydrolysis of an ester to the corresponding carboxylic acid or salt thereof (e.g. see Examples 351, 488, 489, 650, 651 of PCT/EP03/11814 (WO 2004/024728 A2), incorporated herein by reference, for suitable process details).

N7. Deprotection, e.g. deprotection of (e.g. deacylation of or t-butyloxycarbonyl (BOC) removal from) an amine group. BOC deprotection can be carried out under acidic conditions e.g. using hydrogen chloride in an organic solvent such as dioxan.

N8. Formation of an ester or amide, for example from the corresponding carboxylic acid.

N9. Sulfonylation, e.g. sulfonamide formation by reaction of an amine with a sulfonyl halide e.g. a sulfonyl chloride (e.g. see Examples 322-328 of PCT/EP03/11814 (WO 2004/024728 A2), incorporated herein by reference, for suitable process details).
and/or

N10. Beckmann rearrangement of one compound of formula (I) into another compound of formula (I), for example using cyanuric chloride (2,4,6-trichloro-1;3,5-triazine) together with a formamide such as DMF, e.g. at room temperature (see L.D. Luca, J. Org. Chem., 2002, 67, 6272-6274). The Beckmann rearrangement can for example comprise conversion of a compound of formula (I) wherein $NHR^3$ is of sub-formula (o2)

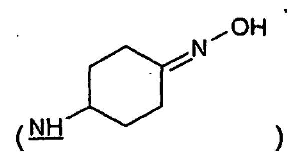

into a compound of formula (I) wherein $NHR^3$ is of sub-formula (m3)

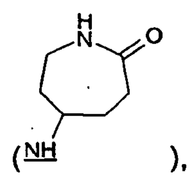

and suitable process details can be as illustrated in Examples 658 and 659 of PCT/EP03/11814 (WO 2004/024728 A2), incorporated herein by reference.

The present invention therefore also provides a method (process) of preparing a compound of formula (I) or a salt thereof:

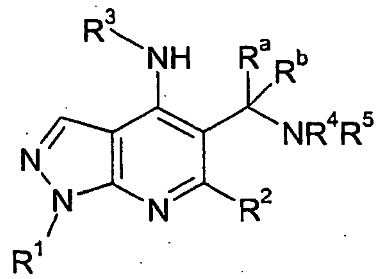

(I)

, using the final step of Process A, B, C, D, E, F, G, H, I, J, K, L, M or N, generally as described hereinabove, and optionally converting the compound of formula (I) into a salt thereof e.g. a pharmaceutically acceptable salt thereof.

Preferred, suitable or optional features of Processes A, B, C, D, E, F, G, H, I, J, K, L, M or N, independently of each other, can be as described above for Processes A, B, C, D, E, F, G, H, I, J, K, L, M or N, with all necessary changes

being made.

The present invention also provides: (e) a method (process) of preparing a pharmaceutically acceptable salt of a compound of formula (I) comprising conversion of the compound of formula (I) or a salt thereof into the desired pharmaceutically acceptable salt thereof.

The present invention also provides a compound of formula (I) or a salt thereof, prepared by a method as defined herein.

**MEDICAL USES**

The present invention also provides a compound of formula (I) or a pharmaceutically acceptable salt thereof for use as an active therapeutic substance in a mammal such as a human. The compound or salt can be for use in the treatment and/or prophylaxis of any of the diseases / conditions described herein (e.g. for use in the treatment and/or prophylaxis of an inflammatory and/or allergic disease in a mammal such as a human; or e.g. for use in the treatment and/or prophylaxis of cognitive impairment (e.g. in a neurological disorder such as Alzheimer's disease) or depression in a mammal such as a human) and/or can be for use as a phosphodiesterase 4 (PDE4) inhibitor. "Therapy" may include treatment and/or prophylaxis.

The compound or salt can for example be for use in the treatment and/or prophylaxis of an inflammatory and/or allergic skin disease, such as atopic dermatitis or psoriasis (in particular atopic dermatitis), in a mammal such as a human.

Also provided is the use of a compound of formula (I) or a pharmaceutically acceptable salt thereof in the manufacture of a medicament (e.g. pharmaceutical composition) for the treatment and/or prophylaxis of any of the diseases / conditions described herein in a mammal such as a human, e.g. for the treatment and/or prophylaxis of an inflammatory and/or allergic disease in a mammal such as a human, or e.g. for the treatment and/or prophylaxis of cognitive impairment (e.g. in a neurological disorder such as Alzheimer's disease or schizophrenia) or depression in a mammal.

Phosphodiesterase 4 inhibitors are thought to be, or may be, potentially useful in the treatment and/or prophylaxis of a variety of diseases / conditions, especially inflammatory and/or allergic diseases, in a mammal such as a human, for example: asthma, chronic obstructive pulmonary disease (COPD) (e.g. chronic bronchitis and/or emphysema), atopic dermatitis, urticaria, rhinitis (e.g. allergic rhinitis), allergic conjunctivitis, vernal conjunctivitis, eosinophilic granuloma, psoriasis, rheumatoid arthritis, septic shock, ulcerative colitis, Crohn's disease, reperfusion injury of the myocardium and brain, chronic glomerulonephritis, endotoxic shock, adult respiratory distress syndrome, multiple sclerosis, cognitive impairment (e.g. in a neurological disorder such as Alzheimer's disease or schizophrenia), depression, or pain (e.g. inflammatory pain). Ulcerative colitis and/or Crohn's disease are collectively often referred to as inflammatory bowel disease.

Phosphodiesterase 4 inhibitors, e.g. perhaps the compound or salt of the invention, may also be potentially useful in the treatment and/or prophylaxis of anxiety in a mammal such as a human.

In the treatment and/or prophylaxis, with a compound or salt of the invention, the inflammatory and/or allergic disease can for example be chronic obstructive pulmonary disease (COPD), asthma, rheumatoid arthritis, rhinitis (e.g. allergic rhinitis), psoriasis or atopic dermatitis in a mammal (e.g. human). In particular, the treatment and/or prophylaxis, with a compound or salt of the invention, can be of COPD, asthma, psoriasis or atopic dermatitis in a mammal (e.g. human).

Suitably, the treatment and/or prophylaxis is of atopic dermatitis in a mammal such as a human or pig, in particular in a human, in particular in a human aged 21 years or less, e.g. 18 years or less. For treatment and/or prophylaxis of atopic dermatitis in a mammal, external topical administration to the mammal of the compound of formula (I) or a pharmaceutically acceptable salt thereof (e.g. topical administration to the skin e,g. to skin affected by the atopic dermatitis) can in particular be used; though alternatively oral or parenteral administration can be used. For treatment and/or prophylaxis of atopic dermatitis, inhaled administration is usually not suitable.

"Atopic dermatitis" has been proposed to include two general sub-classes: (1) an "allergic (extrinsic)" type of atopic dermatitis which generally occurs in the context of sensitization to environmental allergens and/or which is generally accompanied by elevated serum IgE levels; and (2) an "non-allergic (intrinsic)" type of atopic dermatitis generally with little or no detectable sensitization and/or generally with normal or low serum IgE levels (N. Novak et al., J. Allergy Clin. Immunol., 2003, 112, 252-262; and T.C. Roos et al., Drugs, 2004, 64(23), 2639-2666, see e.g. pages 2640-2641). The compound of formula (I) or the pharmaceutically acceptable salt thereof can therefore be for the treatment and/or prophylaxis of allergic (extrinsic) atopic dermatitis and/or non-allergic (intrinsic) atopic dermatitis in a mammal (e.g. human or pig, in particular a human).

"External topical" administration means topical administration to an external body part (i.e. excluding, for example, the lung or mouth, but including the lips), in particular excluding the eye.

"External topical" administration is for example topical administration to the skin, for example to the skin of an arm, hand, leg, foot, head (e.g. face), neck and/or torso of a mammal such as a human. External topical administration can for example be to those parts of a mammal's skin affected by or susceptible to atopic dermatitis.

For the use of PDE4 inhibitors in atopic dermatitis, see for example:

- J.M. Hanifin et al., "Type 4 phosphodiesterase inhibitors have clinical and in vitro anti-inflammatory effects in atopic dermatitis", J. Invest. Dermatol., 1996, 107(1), 51-56; which reports reductions of inflammatory parameters in atopic dermatitis patients treated with PDE4 inhibitor CP80,633 (0.5% ointment, twice daily topical application);
- C.E.M. Griffiths et al., "Randomized comparison of the type 4 phosphodiesterase inhibitor cipamfylline cream, cream vehicle and hydrocortisone 17-butyrate cream for the treatment of atopic dermatitis", Br. J. Dermatol., 2002, 147 (2), 299-307, which reports that cipamfylline (0.15%) cream is significantly more effective than vehicle, but significantly less effective than hydrocortisone 17-butyrate (0.1%) cream, in the treatment of atopic dermatitis patients;
- T.C. Roos et al., "Recent advances in treatment strategies for atopic dermatitis", Drugs, 2004, 64(23), 2639-2666 (see e.g. page 2657 and refs. 201-209 therein);
- A.M.Doherty, Current Opinion Chem. Biol., 1999, 3(4), 466-473 (e.g. see p. 470); and
- H.J.Dyke et al., Expert Opinion Invest. Drugs, 2002,11(1),1-13 (e.g. see p.7 and refs. 74, 75 and 76 cited therein);

and references cited in the above references.

For the use of the PDE4 inhibitors SB 207499 (cilomilast) and AWD 12-281 in mouse models of the allergic type of dermatitis, see W. Bäumer et al., Eur. J. Pharmacol., 2002, 446, 195-200 and W. Bäumer et al., J. Pharmacy Pharmacol., 2003, 55, 1107-1114.

PDE4 inhibitors, for example cilomilast and roflumilast, are thought to be effective in the treatment of COPD. For example, see S.L. Wolda, Emerging Drugs, 2000, 5(3), 309-319; Z. Huang et al., Current Opinion in Chemical Biology, 2001, 5: 432-438; H.J.Dyke et al., Expert Opinion on Investigational Drugs, January 2002, 11 (1), 1-13; C.Burnouf et al., Current Pharmaceutical Design, 2002, 8(14), 1255-1296; A.M.Doherty, Current Opinion Chem. Biol., 1999, 3(4), 466-473; A.M. Vignola, Respiratory Medicine, 2004, 98, 495-503; D. Spina, Drugs, 2003, 63(23), 2575-2594; and references cited in the aforementioned publications; and G. Krishna et al., Expert Opinion on Investigational Drugs, 2004, 13(3), 255-267 (see especially pp. 259-261 and refs. 102-111 and 201 therein).

The PDE4 inhibitor cilomilast (Ariflo ™) at 15 mg orally twice daily appears to improve forced expiratory volume in 1 s ($FEV_1$) in COPD patients (C.H.Compton et al., The Lancet, 2001, vol.358, 265-270), and appears to have antiinflammatory effects in COPD patients (E.Gamble et al., Am. J. Respir. Crit. Care Med., 2003,168, 976-982). On cilomilast, see also R.D. Border et al., Chest, 2003, vol. 124 Suppl. 4, p.170S (abstract) and J.D. Eddleston et al., Am. J. Respir. Crit. Care Med., 2001, 163, A277 (abstract). The PDE4 inhibitor roflumilast appears to show small improvements in $FEV_1$ in COPD patients (see B.J. Lipworth, The Lancet, 2005, 365, 167-175, and refs 49-50 therein).

COPD is often characterised by the presence of airflow obstruction due to chronic bronchitis and/or emphysema (e.g., see S.L. Wolda, Emerging Drugs, 2000, 5(3), 309-319).

For treatment and/or prophylaxis of COPD or asthma in a mammal (e.g. human), oral, inhaled or parenteral administration to the mammal of the compound of formula (I) or a pharmaceutically acceptable salt thereof is optionally used, e.g. oral or inhaled administration.

PDE4 inhibitors are thought to be effective in the treatment and/or prophylaxis of asthma (e.g. see M.A.Giembycz, Drugs, Feb. 2000, 59(2),193-212; Z. Huang et al., Current Opinion in Chemical Biology, 2001, 5: 432-438; H.J. Dyke et al., Expert Opinion on Investigational Drugs, January 2002, 11(1), 1-13; C.Bumouf et al., Current Pharmaceutical Design, 2002, 8(14), 1255-1296; A.M.Doherty, Current Opinion Chem. Biol., 1999, 3(4), 466-473; P.J. Barnes, Nature Reviews - Drug Discovery, October 2004, 831-844; B.J. Lipworth, The Lancet, 2005, 365, 167-175; and references cited in the aforementioned publications).

The PDE4 inhibitor roflumilast, given orally at 500 ug once daily for 9 days, is reported to be effective in improving rhinal airflow during the treatment period (compared to placebo), in humans with histories of allergic rhinitis but asymptomatic at screening, and who were challenged with intranasal allergen provocation (pollen extracts) daily beginning the third day of treatment and each time approx. 2 hours after study drug administration (see B.M. Schmidt et al., J. Allergy & Clinical Immunology, 108(4), 2001., 530-536).

For treatment and/or prophylaxis of rhinitis (e.g. allergic rhinitis), intranasal, oral or parenteral administration of the compound of formula (I) or a pharmaceutically acceptable salt thereof is optionally used.

In one optional embodiment, the compound of formula (I) or the pharmaceutically acceptable salt thereof is for the treatment and/or prophylaxis of rhinitis, such as allergic rhinitis (e.g. seasonal allergic rhinitis or perennial allergic rhinitis) or non-allergic rhinitis (e.g. vasomotor rhinitis), in a mammal such as a human.

PDE4 inhibitors are thought to be, or may be, effective in the treatment of rheumatoid arthritis and multiple sclerosis (e.g. see H.J.Dyke et al., Expert Opinion on Investigational Drugs, January 2002, 11(1), 1-13; C.Bumouf et al., Current Pharmaceutical Design, 2002, 8(14), 1255-1296; and A.M.Doherty, Current Opinion Chem. Biol., 1999, 3 (4), 466-473; and references cited in these publications). For rheumatoid arthritis, oral or parenteral administration is optionally used.

PDE4 inhibitors have been suggested as having analgesic properties and thus being effective in the treatment

of pain (A.Kumar et al., Indian J. Exp. Biol., 2000,38(1), 26-30).

In the invention, the treatment and/or prophylaxis can be of cognitive impairment e.g. cognitive impairment in a neurological disorder (such as Alzheimer's disease or schizophrenia); and/or administration of the compound or salt can optionally be oral. For example, the treatment and/or prophylaxis can comprise cognitive enhancement e.g. in a neurological disorder. For cognition background, see for example: H.T.Zhang et al. in: Psychopharmacology, June 2000, 150(3), 311-316 and Neuropsychopharmacology, 2000, 23(2), 198-204; and T. Egawa et al., Japanese J. Pharmacol., 1997, 75(3), 275-81.

PDE4 inhibitors such as rolipram have been suggested as having antidepressant properties (e.g. J. Zhu et al., CNS Drug Reviews, 2001, 7(4), 387-398; O'Donnell, Expert Opinion on Investigational Drugs, 2000, 9(3), 621-625; H.T. Zhang et al., Neuropsychopharmacology, October 2002, 27(4), 587-595; J. M. O'Donnell and H.-T. Zhang, Trends Pharmacol. Sci., March 2004, 25(3), 158-163; and T.E.Renau, Curr. Opinion Invest. Drugs, 2004, 5(1), 34-39).

PDE4 inhibition has been suggested for the treatment of inflammatory bowel disease (e.g. ulcerative colitis and/or Crohn's disease), see K.H.Banner and M.A.Trevethick, Trends Pharmacol. Sci., August 2004, 25(8), 430-436.

## PHARMACEUTICAL COMPOSITIONS, ROUTES OF ADMINISTRATION, AND DOSAGE REGIMENS

For use in medicine, the compounds or salts of the present invention are usually administered as a pharmaceutical composition.

The present invention therefore provides in a further aspect a pharmaceutical composition comprising a compound of formula (I) or a pharmaceutically acceptable salt thereof and one or more pharmaceutically acceptable carriers and/or excipients.

The pharmaceutical composition can be for use in the treatment and/or prophylaxis of any of the conditions described herein, for example chronic obstructive pulmonary disease (COPD), asthma, rheumatoid arthritis, allergic rhinitis, psoriasis or atopic dermatitis in a mammal (e.g. human).

The invention also provides a method of preparing a pharmaceutical composition comprising a compound of formula (I), as herein defined, or a pharmaceutically acceptable salt thereof, and one or more pharmaceutically acceptable carriers and/or excipients,

the method comprising mixing the compound or salt with the one or more pharmaceutically acceptable carriers and/or excipients.

The invention also provides a pharmaceutical composition prepared by said method.

The compounds of formula (I) and/or the pharmaceutical composition may be administered, for example, by external topical (e.g. skin topical), oral, parenteral (e.g. intravenous, subcutaneous, or intramuscular), inhaled, or nasal administration. Inhaled administration involves topical administration to the lung e.g. by aerosol or dry powder composition.

Accordingly, the pharmaceutical composition can be suitable for (e.g. adapted for) external topical (e.g. skin topical), oral, parenteral (e.g. intravenous, subcutaneous, or intramuscular), inhaled, or nasal administration, e.g. to a mammal such as a human. The pharmaceutical composition can for example be suitable for external topical (e.g. skin topical) or oral administration, e.g. to a mammal such as a human.

The pharmaceutical composition can optionally be in unit dose form. The unit dose form can for example be:

(a) a tablet or capsule for oral administration e.g. for oral administration to a human;

(b) a rupturable or peel-openable sealed dose container containing a dry powder inhalable pharmaceutical composition (e.g. a plurality of which are usually disposed inside a suitable inhalation device);

(c) a vial, ampoule or filled syringe for parenteral administration e.g. comprising a solution or suspension of the compound or pharmaceutically acceptable salt in a suitable carrier such as an aqueous carrier or e.g. containing a lyophilised parenteral pharmaceutical composition (the vial or ampoule can optionally be manufactured using a blow-fill-seal process).

Alternatively, the composition can be in a form adapted for the administration of varying amounts of composition as desired by the user, such as a spreadable or sprayable external topical composition such as a cream, an ointment, a gel, or a liquid.

### *Pharmaceutical compositions suitable for external topical administration*

The pharmaceutical composition of the invention can for example be suitable for (e.g. adapted for) external topical administration (e.g. skin topical administration, i.e. topical administration to the skin), for example to a mammal such as a human. The pharmaceutical composition suitable for external topical administration can suitably be for the treatment and/or prophylaxis of atopic dermatitis in a mammal such as a human, e.g. by external topical administration.

"External topical administration" is defined above under the "medical uses" section. External topical administration can for example be to those parts of the skin affected by or susceptible to the disease or condition e.g. atopic dermatitis, in particular in a mammal (e.g. human) suffering from or susceptible to atopic dermatitis.

An external-topical pharmaceutical composition, e.g. skin topical pharmaceutical composition, can for example be an ointment, a cream (usually an oil-in-water or water-in-oil pharmaceutical composition, usually an emulsion), an aqueous gel, or a microemulsion. The pharmaceutical composition can alternatively be a DMSO-containing solution such as a DMSO/acetone solution or DMSO/water solution (DMSO = dimethyl sulfoxide); a DMSO-containing solution can be used for experimental animal tests, but is not usually desirable for use in humans.

An external-topical pharmaceutical composition, e.g. skin topical pharmaceutical composition, of particular interest is an ointment.

In the external-topical pharmaceutical composition, e.g. an ointment or an oil-in-water or water-in-oil composition, the compound of formula (I) or the pharmaceutically acceptable salt thereof can be present in 0.1% to 10%, such as 0.2% to 10%, or 0.2% to 5%, or 0.5% to 5%, in particular 1% to 10% (e.g. about 2%, about 4% or about 6%), or 1% to 5% (e.g. 1.5% to 5% or 1.5% to 5%, such as about 2% or about 4%), or 0.5% to 3% (e.g. 0.5% or about 2%), or 1% to 3% (e.g. about 2%), by weight of the composition (w/w).

In the external-topical pharmaceutical composition of the invention, the compound of formula (I) or the pharmaceutically acceptable salt thereof preferably comprises (e.g. is) Example 236; 237 or 238 (for example Example 236 or 238), as the compound or a pharmaceutically acceptable salt thereof, in particular as the compound (i.e. as the "free base" form).

Therefore, the external-topical pharmaceutical composition of the invention, the compound of formula (I) or the pharmaceutically acceptable salt thereof can for example comprise (e.g. be):

*N*-{[1,6-diethyl-4-(tetrahydro-2*H*-pyran-4-ylamino)-1*H*-pyrazolo[3,4-*b*]pyridin-5-yl]methyl}-5-methyl-2-pyrazinecarboxamide (e.g. see Example 236) or a pharmaceutically acceptable salt thereof,
or *N*-{[1,6-diethyl-4-(tetrahydro-2*H*-pyran-4-ylamino)-1*H*-pyrazolo[3,4-*b*]pyridin-5-yl]methyl}-1-methyl-1H-pyrazole-3-carboxamide (e.g. see Example 238) or a pharmaceutically acceptable salt thereof;
in particular as the compound (i.e. as the "free base" form).

In one optional embodiment, the compound of formula (I) or the pharmaceutically acceptable salt thereof can optionally be in a particle-size-reduced form, for example obtained or obtainable by micronisation. This can be, for example, for use in a pharmaceutical composition suitable for (e.g. adapted for) external topical (e.g. skin topical) administration. See the Particle size reduction sub-section herein, within the Inhalable pharmaceutical compositions section, for more details:

Aqueous solubility: A preliminary screen, which can aim to estimate roughly the aqueous solubility of a compound or salt of the invention, can include (as an approximate summary): (i) creating a ca. 10mM solution of the compound in DMSO, (ii) diluting a portion of this DMSO solution by mixing about 19 parts by volume of pH 7.4 aqueous phosphate buffered saline (PBS) buffer with 1 part by volume of the ca. 10mM DMSO solution, (iii) "filtering" the mixture with the aid of centrifugation, and then (iv) measuring the concentration of the dissolved compound in the "filtrate". Although some DMSO (about 5% by volume) is usually present in this solubility screen "filtrate", the results can be a very approximate estimate of aqueous solubility, e.g. at room temperature.

Lipophilicity: The clogP (calculated loq of the octanol/water partition coeficient (P)) of a particular compound or salt of the invention can estimate the lipophilicity of the compound or salt.

*Solubilising and/or skin-penetration-enhancing agents:* An external-topical pharmaceutical composition, e.g. an ointment or an oil-in-water cream or water-in-oil cream, can for example include an agent which acts as a skin-penetration enhancer for and/or a solubiliser of the compound of formula (I) or the salt thereof. The skin-penetration-enhancing- and/or solubilising- agent can for example be propylene glycol, diethylene glycol monoethyl ether (e.g. TRANSCUTOL™) and/or caprylocaproyl macrogolglycerides (e.g. LABRASOL™), in particular propylene glycol. The solubiliser and/or skin-penetration enhancer suitably does not comprise DMSO. The solubiliser and/or skin-penetration enhancer is in particular both a solubiliser and skin-penetration enhancer, and/or can be present in 0.5% to 50%, in particular 5% to 50%, for example 7% to 30%, such as 7% to 25%, e.g. about 10% to about 20% (e.g. about 10% or about 20%), by weight of the composition (w/w).

The skin-penetration enhancer is for delivery of the compound of formula (I) or salt thereof ("active agent" or "drug") through the skin. Solubilization of the drug also helps. The solubilising and/or skin-penetration-enhancing agents should ideally (a) be safe and/or tolerable, (b) have as low a potential for skin irritancy as possible consistent with being an effective skin penetration enhancer, and (c) be compatibile with the active pharmaceutical ingredient. Note that the agent optionally functions both as a solubilising agent and a skin-penetration-enhancing agent.

*Surfactants:* An external-topical pharmaceutical composition, e.g. an ointment or an oil-in-water cream or water-in-oil cream, can, in one embodiment, include a surfactant (e.g. as an emulsifier), for example for achieving emulsification

of compositions having two or more phases. The total surfactant content can for example be 0.3% to 20%, e.g. 0.5% to 15% or 0.5% to 12% or 0.5% to 10% or 1% to 12% or 3% to 10%, by weight of the composition (w/w). The surfactant can for example comprise one or more of the following: a polyoxyl $C_{12-22}$alkyl ether (e.g. a polyoxyl $C_{14-20}$alkyl ether such as polyoxyl cetyl ether or polyoxyl stearyl ether) (e.g. present at 0.5% to 10% w/w, e.g. 2.5% to 10% w/w such as about 5% to about 8% w/w), glycerol monostearate (e.g. Arlacel 165 ™) (e.g. present at 0.5% to 10% w/w, e.g. about 2% w/w), sorbitan monostearate (e.g. Span 60 ™) (e.g. present at 0.05% to 10% w/w, e.g. about 1% w/w), cetyl alcohol and/or stearyl alcohol (e.g. wherein the total of any cetyl alcohol and any stearyl alcohol present is 0.1 % to 15% w/w, e.g. 1% to 10% w/w such as about 2% to about 5% w/w), and sodium dodecyl sulphate (SDS) (e.g. present at 0.3% to 2% w/w such as about 1% w/w). Polyoxyl stearyl ether (steareth) can e.g. be polyoxyl 2 stearyl ether (steareth 2) or polyoxyl 21 stearyl ether (steareth 21).

*DMSO-containing solutions:* One possible external-topical pharmaceutical composition is a solution of the compound of formula (I) or the pharmaceutically acceptable salt thereof present at ca. 0.5% to ca. 2.5% w/w in a DMSO-containing solvent such as in DMSO/acetone or in DMSO/water; for example a solution of the compound or salt present at ca. 0.5% to ca. 2.5% w/w in DMSO/acetone (1:1). DMSO-containing solutions, often being capable of high skin penetration, are often good experimental pre-clinical formulations for use in animals e.g. pigs, but their likely skin irritancy generally make them less suitable for use in humans such as patients, e.g. atopic dermatitis patients.

***Ointments (and oil phase in ointments and creams):***

An external-topical pharmaceutical composition can for example be an ointment or an oil-in-water cream or water-in-oil cream. An ointment is of particular interest. The ointment or cream typically contains an oil phase (oily ointment base). The oil phase (oily ointment base) typically comprises an oil and/or a fat, for example of a consistency suitable for skin-spreadability.

The oil phase (oily ointment base) can for example comprise (e.g. be) an oil, wherein the oil comprises (e.g. is) white soft paraffin (white petrolatum) and/or a silicone oil and/or a mineral oil (such as liquid paraffin). (Mineral oil can also be used as a solubiliser and/or emollient).

In particular, the oil phase (oily ointment base) comprises (e.g. is) an oil, wherein the oil comprises (e.g. is) white soft paraffin (white petrolatum) and/or a silicone oil.

In an embodiment of particular interest, the external-topical pharmaceutical composition is an ointment, and the oil phase (oily ointment base) comprises (e.g. is) an oil, wherein the oil comprises (e.g. consists essentially of) white soft paraffin (white petrolatum) and a silicone oil.

The white soft paraffin (white petrolatum), e.g. in an ointment or cream, can be of various grades, for example (for Penreco supplier) Penreco Regent White ™ grade, Penreco Snow White ™ grade, or Penreco Ultima White ™ grade; in particular high melting point white petrolatum (high melting point white soft paraffin) (e.g. of Penreco Ultima White ™ grade). The white petrolatum can be present at 25% to 99.9% w/w or 45% to 99.5% w/w or 50% to 99.5% w/w or 45% to 99% w/w or 50% to 99% w/w or 45% to 85% w/w or 45% to 75% w/w (i.e. by weight of the composition).

The silicone oil, e.g. in an ointment or cream, in particular in an ointment, can for example be present at: 5% to 60 % w/w such as 5% to 50% w/w, in particular 10% to 50% w/w such as 15% to 40% w/w, suitably 20% to 35% w/w such as about 25% w/w (measured as the total silicone oil content, by weight of the composition).

The silicone oil can be solid or liquid. The silicone oil, e.g. in an ointment or cream, can for example comprise (e.g. be): decamethyl-cyclopentasiloxane (e.g. ST-Cyclomethicone 5-NF ™, available from Dow Coming), stearoxytrimethylsilane [$Me(CH_2)_{17}O-SiMe_3$], polydimethylsiloxane (dimethicone), hexamethyldisiloxane (e.g. ca. 0.65 cSt viscosity at 25˚C), octamethyltrisiloxane (e.g. ca. 1.0 cSt viscosity at 25˚C), decamethyltetrasiloxane, dodecamethylpentasiloxane, or hydroxy-terminated polydimethylsiloxane (e.g. ST-Dimethiconol 40 ™, Dow Coming), or mixtures of any of the foregoing. The silicone oil, e.g. in an ointment or cream, can in particular comprise (e.g. be): decamethyl-cyclopentasiloxane, stearoxytrimethylsilane [$Me(CH_2)_{17}O-SiMe_3$], or polydimethylsiloxane (dimethicone), or mixtures of any of the foregoing. Preferably, the silicone oil, e.g. in an ointment or cream, can comprise (e.g. be) decamethyl-cyclopentasiloxane.

The decamethyl-cyclopentasiloxane can be ST-Cyclomethicone 5-NF ™, available from Dow Corning, and which is described by Dow Corning as being a polydimethylcyclosiloxane having a decamethyl-cyclopentasiloxane content of >95% and having a octamethyl-cyclotetrasiloxane content of <1.0%. The decamethyl-cyclopentasiloxane can for example be present at 5% to 60 % w/w such as 5% to 50% w/w, in particular 10% to 50% w/w such as 15% to 40% w/w, suitably 20% to 35% w/w such as about 25% w/w (i.e. by weight of the composition).

Stearoxytrimethylsilane [$Me(CH_2)_{17}O-SiMe_3$] can for example be present as a mixture of stearoxytrimethylsilane and stearyl alcohol for example Silky Wax 10 ™ which is available from Dow Corning. Stearoxytrimethylsilane (and/or stearoxytrimethylsilane and stearyl alcohol mixture), e.g. in an ointment or cream e.g. ointment, can for example be present at 1% to 30% w/w or 2% to 20% w/w or 5% to 20% w/w such as about 10% w/w.

Polydimethylsiloxane (dimethicone), whose structure is given in the Merck Index 12th edition 1996 as $Me_3Si-O-1-Si(CH_3)_2-O-]_n-SiMe_3$, can for example have a viscosity at 25˚C of from about 20 to about 12500 cSt (centistokes),

such as a viscosity at 25°C of from about 20 to about 350 cSt or from about 20 to about 100 cSt. For example, polydimethylsiloxane (dimethicone) can have a viscosity at 25°C of: 20 cSt (±10%) ("dimethicone 20"), 100 cSt (±5%), 350 cSt (±5%) ("dimethicone 350"), 1000 cSt (±5%), or 12500 cSt (±5%); grades of polydimethylsiloxane having these five different viscosities are available from Dow Corning as Q7-9120 ™ Silicone Fluid. Polydimethylsiloxane (dimethicone), e.g. in an ointment, can e.g. be present at 0.1% to 15% w/w such as 0.5% to 10% w/w e.g. 0.5% to 5% w/w.

Microcrystalline wax or beeswax or beeswax substitute can alternatively or additionally be used as an oil / fat in the oil phase.

Alternatively or additionally, one or more fats like straight or branched chain mono- or dialkyl esters such as isopropyl myristate, isopropyl palmitate, diisopropyl adipate, isocetyl stearate, isostearyl isostearate, decyl oleate, butyl stearate, 2-ethylhexyl palmitate, propylene glycol diester of coconut fatty acids, or a mixed ester of 2-ethyl hexanoic acid with a blend of cetyl or stearyl alcohols (e.g. known as Crodamol CAP) may be used in the oil phase (some of these are also solubilisers and/or surfactants). These may be used singly or in combination depending on the properties required.

The oil phase (oily ointment base) can for example be present at 25% to 99.9% w/w or 25% to 99.5% w/w or 25% to 85% w/w (in particular 45% to 99.5% w/w or 45% to 99% w/w, or 50% to 99.5% w/w or 50% to 99% w/w or 50% to 80% w/w, or 70% to 99.5% w/w or 80% to 99.5% w/w) in an ointment (e.g. as an emulsion, or e.g. as a homogeneous single phase (which does not exclude the compound or salt being at least partly in suspension)).

The oil phase (oily ointment base) can for example be present at 25% to 85% w/w (e.g. 35% to 70% w/w) in an water-in-oil cream (e.g. emulsion), or at 8% to, 55% w/w (e.g. 10% to 45% w/w) in an oil-in-water cream (e.g. emulsion).

*Example ointments:*

One particular example of an external-topical pharmaceutical composition is an ointment comprising (e.g. consisting essentially of):

- a compound of formula (I) or the pharmaceutically acceptable salt thereof present at 0.5% to 10% w/w (in particular 1% to 10% w/w or 1% to 5% w/w, or 1% to 3% w/w, e.g. about 2% w/w);
- white petrolatum present at 25% to 99.9% w/w, in particular 45% to 99.5% w/w or 50% to 99.5% w/w or 45% to 99% w/w or 50% to 99% w/w or 45% to 85% w/w or 45% to 75% w/w (e.g. about 60-85% w/w, e.g. about 73-75% w/w) (i.e. by weight of the composition); and
- a silicone oil present at: 5% to 60 % w/w such as 5% to 50% w/w, in particular. 10% to 50% w/w such as 15% to 40% w/w, suitably 20% to 35% w/w such as about 25% w/w (measured as the total silicone oil content, by weight of the composition).

One preferable example an external-topical pharmaceutical composition is an ointment comprising (e.g. consisting essentially of):

- a compound of formula (I) or the pharmaceutically acceptable salt thereof present at 0.5% to 10% w/w (in particular 1% to 10% w/w or 1% to 5% w/w, or 1% to 3% w/w, e.g. about 2% w/w);
- white petrolatum present at 25% to 99.9% w/w, in particular 45% to 99.5% w/w or 50% to 99.5% w/w or 45% to 99% w/w or 50% to 99% w/w or 45% to 85% w/w or 45% to 75% w/w (e.g. about 60-85% w/w, e.g. about 73-75% w/w) (i.e. by weight of the composition); and
- decamethyl-cyclopentasiloxane (e.g. (e.g. ST-Cyclomethicone 5-NF ™) present at 5% to 60 % w/w such as 5% to 50% w/w, in particular 10% to 50% w/w such as 15% to 40% w/w, suitably 20% to 35% w/w such as about 25% w/w (i.e. by weight of the composition).

One example of an external-topical pharmaceutical composition is an ointment comprising:

- the compound of formula (I) or the pharmaceutically acceptable salt thereof present at 0.2% to 5% w/w (e.g. 0.5% to 5% w/w, or 1% to 3% w/w, e.g. about 2% w/w);
- an oil phase (oily ointment base) present at 25% to 99% w/w or 50% to 99% w/w or 25% to 85% w/w or 50% to 80% w/w (for example, the oil phase can comprise white petrolatum present at 25 to 75% w/w or 45 to 75% w/w, and optionally also comprising mineral oil (e.g. as solubiliser and emollient) present at 2.5% to 15% w/w such as 4% to 12% w/w);
- one or more surfactants (e.g. polyoxyl stearyl ether) present in total at 0.5% to 10% w/w or 3% to 10% w/w; and
- one or more agents acting as a skin-penetration enhancer (in particular acting as both a solubiliser and skin-penetration enhancer and/or in particular a hydrophilic skin-penetration enhancer such as propylene glycol) present in total at 0.5% to 50% w/w, such as 5% to 50% w/w or 7% to 30% w/w (e.g. about 20% w/w, e.g. of propylene

glycol); and

- optionally one or more antioxidants (e.g. butylated hydroxyanisole), e.g. present in total at 0.001 to 2% w/w such as 0.02 to 2% w/w; and
- optionally one or more preservatives, e.g. present in total at 0.01 to 4% w/w such as 0.05 to 1% w/w (e.g. methylparaben present at 0.05 to 2% w/w and/or propylparaben present at 0.01 to 2% w/w).

The above example composition, including the oil "phase" and the penetration enhancer, can optionally be a homogeneous single phase. However, in one embodiment of the above example ointment composition, e.g. when using propylene glycol or another hydrophilic solubiliser and penetration enhancer, the oil phase (oily ointment base) and a hydrophilic phase containing the hydrophilic solubiliser and penetration enhancer (e.g. propylene-glypol-containing phase) have been emulsified to form an ointment emulsion.

Ointment compositions having two phases can optionally be prepared using an emulsification process whereby the hydrophilic phase (e.g. propylene-glycol-containing phase) and oil phase are first prepared in separate vessels. The hydrophilic phase can optionally contain a penetration enhancer such as propylene glycol, and optionally some or all of the compound of formula (I) or salt thereof. The oil phase can optionally contain a surfactant. Temperatures of both phases are maintained at elevated temperatures, such as about 45-90˚C or about 45-80˚C or about 55-90˚C or about 55-80˚C (e.g. about 60-65˚C), or from above 70 to 90 ˚C, the oil phase temperature being sufficiently high (e.g: from above 70 to 90 ˚C) to melt the oil phase. While hot, one phase is added to another while mixing, e.g. using a high shear mixer, to effect emulsification, optionally keeping the temperature above 70˚C such as from above 70 to 90 ˚C. The resulting ointment emulsion is allowed to cool, e.g. to about 15-35˚C such as to about 17-30˚C, in particular while the agitation continues e.g. at lower speeds. The ointment emulsion can then optionally be dispensed from the manufacturing vessel and filled into primary packaging, for example tubes or sachets.

Optionally, an ointment can comprise a polyethylene glycol base, e.g. present at 25 to 98% w/w such as 50 to 95% w/w, instead of or as well as an oily ointment base.

***Creams***: An external-topical pharmaceutical composition can be a cream, e.g. a water-in-oil cream or an oil-in-water cream.

*Water-in-oil creams:* These usually have an increased aqueous content compared to ointments. In particular, the water-in-oil cream can be a water-in-oil cream emulsion. That is, in particular, in the water-in-oil cream; an oil phase and an aqueous phase can have been emulsified to form a water-in-oil cream emulsion.

One example of an external-topical pharmaceutical composition is a water-in-oil cream (e.g. cream emulsion) comprising:

- the compound of formula (I) or pharmaceutically acceptable salt thereof present at 0.2% to 10% w/w or 0.2% to 5% w/w (in particular 0.5% to 10% w/w or 1% to 10% w/w, such as 0.5% to 5% w/w or 1% to 5% w/w, or 1% to 3% w/w, e.g. about 2% w/w);
- an oil phase (oily ointment base) present at 25% to 85% w/w or 35% to 70% w/w (for example comprising white petrolatum present at 25% to 75% w/w or 30% to 65% w/w (e.g. about 40% w/w), and optionally also comprising mineral oil (e.g. as solubiliser and emollient) present at 2.5% to 15% w/w or 4% to 12% w/w, e.g. about 10% w/w);
- water present at 2% to 30% w/w, e.g. 5% to 25% or 10% to 22% w/w (e.g. about 20% w/w);
- one or more surfactants (e.g. polyoxyl stearyl ether such as polyoxyl 2 stearyl ether) present in total at 0.5% to 12% w/w, such as 3% to 10% w/w (e.g. about 8% w/w); and
- one or more agents acting as a skin-penetration enhancer (in particular acting as both a solubiliser and skin-penetration enhancer and/or in particular a hydrophilic skin-penetration enhancer such as propylene glycol) present in total at 0.5% to 50% w/w, such as 5% to 50% w/w or 7% to 30% w/w (e.g. about 20% w/w, e.g. about 20% w/w of propylene glycol).

The above water-in=oil cream can also optionally comprise:

- one or more antioxidants (e.g. butylated hydroxyanisole), e.g. present in total at 0.001 to 2% w/w such as 0.02 to 2% w/w; and/or
- one or more preservatives, e.g. present in total at 0.01 to 4% w/w such as 0.05 to 1 % w/w (e.g. methylparaben present at 0.05 to 2% w/w and/or propylparaben present at 0.01 to 2% w/w).

*Oil-in-water creams:* These usually have an increased aqueous content compared to ointments and water-in-oil creams. In particular, the oil-in-water cream can be an oil-in-water cream emulsion. That is, in particular, in the oil-in-water cream, an oil phase and an aqueous phase can have been emulsified to form an oil-in-water cream emulsion.

Oil-in-water creams can for example be high-occlusion creams, wherein, after topical administration to the skin, moisture loss from the skin and/or from the cream is reduced or limited by means of sufficiently high coverage of

the skin and/or by providing a sufficient barrier at the site of application.

An oil-in-water cream can in particular contain one or more emollients (hydrating agents), such as silicones (e.g. dimethicone, e.g. dimethicone 360 or dimethicone 20), a high-viscosity wax such as microcrystalline wax, and/or mineral oil.

In an oil-in-water cream, suitably there is a sufficiently high water content, for example wherein the water is present in 15% to 60% w/w, 20% to 50% w/w, or 25% to 40% w/w.

One example of an external-topical pharmaceutical composition is a oil-in-water cream (e.g. cream emulsion) comprising:

- the compound of formula (I) or pharmaceutically acceptable salt thereof present at 0.2% to 10% w/w or 0.2% to 5% w/w (in particular 0.5% to 10% w/w or 1% to 10% w/w, such as 0.5% to 5% w/w or 1% to 5% w/w, or 1% to 3% w/w, e.g. about 2% w/w); .
- an oil phase (oily ointment base) containing one or more ingredients capable of acting as emollients, the oil phase being present at 5% to 60% w/w or in particular 20% to 60% w/w or 30% to 60% w/w such as 30% to 55% w/w;
- water present at 12% to 75% w/w or 15% to 75% w/w or 15% to 60% w/w, in particular 15% to 50% w/w or 20% to 40% w/w;
- one or more surfactants (e.g. polyoxyl stearyl ether such as polyoxyl 2 stearyl ether) present in total at 0.5% to 12% w/w, e.g. 3% to 10% w/w; and
- one or more agents acting as a skin-penetration enhancer (in particular acting as both a solubiliser and skin-penetration enhancer and/or in particular a hydrophilic skin-penetration enhancer such as propylene glycol) present in total at 0.5% to 50% w/w, in particular 5% to 50% w/w or 7% to 25% w/w (e.g. about 20% w/w, e.g. about 20% w/w of propylene glycol).

The above oil-in-water cream can also optionally comprise:

- one or more solubilisers (e.g. isopropyl myristate), e.g. present at 0.5% to 20% w/w, e.g. 3 to 12% w/w; and/or
- one or more buffers (e.g. citric acid and/or dibasic sodium phosphate), e.g. present in total at 0.05 to 5% w/w.

In the above example oil-in-water cream composition, the oil phase can in particular comprise mineral oil (e.g. as emollient and solubiliser) present at 15% to 50% w/w or 20% to 45% w/w, and/or can in particular comprise a high-viscosity wax such as microcrystalline wax (e.g. as emollient) present at 5% to 25% w/w such as 8% to 15% w/w, and/or can in particular comprise a silicone (such as dimethicone e.g. dimethicone 360 or dimethicone 20, e.g. as emollient) present at 0.5% to 20% such as 0.5% to 10% or 1% to 5% w/w.

In the above exampla oil-in-water cream composition, the one or more surfactants can for example comprise: glycerol monostearate present at 0.5% to 10% w/w, and/or sorbitan monostearate present at 0.05% to 10% w/w, and/or [cetyl alcohol and/or stearyl alcohol] present in total at 0.1% to 15% or 1 to 10% w/w.

Cream emulsions, e.g. water-in-oil or oil-in-water cream emulsions, can be generally prepared by a process in which an aqueous phase is prepared, e.g. prepared before emulsification. The aqueous phase usually contains water and a solubiliser and/or skin-penetration enhancer such as propylene glycol, and optionally contains some or all of the compound of formula (I) or salt thereof, and/or optionally contains surfactant. The oil phase, e.g. containing white petrolatum and/or mineral oil, and/or optionally containing surfactant, can be prepared in a separate vessel. Temperatures of both phases are suitably maintained at (or heated to) elevated temperatures, such as about 45-90˚C or about 45-80˚C or about 45-75˚C, for example about 55-90˚C or about 55-80˚C or about 55-75˚C (in particular at about 60-65˚C), or e.g. from above 70 to 90 ˚C, the oil phase temperature being sufficiently high (e.g. about 45-90˚C or about 55-90˚C or from above 70 to 90 ˚C) to melt the oil phase. While hot, one phase is suitably added to another while mixing, e.g. using a high shear mixer, to effect emulsification, for example keeping the temperature 45˚C or above, or 55˚C or above such as above 70˚C, e.g. from above 70 to 90 ˚C. The resulting emulsion is typically allowed to cool, e.g. to about 15-35˚C such as to about 17-30˚C (e.g. to about 17-22˚C) or to about 18-30˚C, for example while the agitation continues e.g. at lower speeds. The cream emulsion can then optionally be dispensed from the manufacturing vessel and filled into primary packaging, for example tubes or sachets.

Typically, a pharmaceutical composition of the invention suitable for external topical administration can be administered once daily, twice daily or more than twice daily, to external body part(s), e.g. on the skin such as at a site of diseased skin, e.g. skin suffering from atopic dermatitis.

***Pharmaceutical compositions suitable for oral or parenteral administration***

A pharmaceutical composition suitable for oral administration can be liquid or solid; for example it can be a syrup, suspension or emulsion, a tablet, a capsule or a lozenge.

A liquid formulation (e.g. oral) can generally consist of a suspension or solution of the compound or pharmaceutically acceptable salt in a suitable pharmaceutically acceptable liquid carrier(s), for example an aqueous solvent such as water, ethanol or glycerine, or a non-aqueous solvent, such as polyethylene glycol or an oil. The formulation may also contain a suspending agent, preservative, flavouring and/or colouring agent.

In one embodiment, the pharmaceutical composition is in unit dose form, such as a tablet or capsule for oral administration, e.g. for oral administration to a human.

A pharmaceutical composition suitable for oral administration being a tablet can comprise one or more pharmaceutically acceptable carriers and/or excipients suitable for preparing tablet formulations. The carrier can for example be or include lactose, cellulose (for example microcrystalline cellulose), or mannitol. The tablet can also or instead contain one or more pharmaceutically acceptable excipients, for example a binding agent such as hydroxypropylmethylcellulose or povidone (polyvinylpyrrolidone), a lubricant e.g. an alkaline earth metal stearate such as magnesium stearate, and/or a tablet disintegrant such as sodium starch glycollate, croscarmellose sodium, or crospovidone (cross-linked polyvinylpyrrolidone). The pharmaceutical composition being a tablet can be prepared by a method comprising the steps of: (i) mixing the compound of formula (I), as herein defined, or a pharmaceutically acceptable salt thereof, with the one or more pharmaceutically acceptable carriers and/or excipients, (ii) compressing the resulting mixture (which is usually in powder form) into tablets, and (iii) optionally coating the tablet with a tablet film-coating material.

A pharmaceutical composition suitable for oral administration being a capsule can be prepared using encapsulation procedures. For example, pellets or powder containing the active ingredient can be prepared using a suitable pharmaceutically acceptable carrier and then filled into a hard gelatin capsule. Alternatively, a dispersion or suspension can be prepared using any suitable pharmaceutically acceptable carrier, for example an aqueous gum or an oil and the dispersion or suspension then filled into a soft gelatin capsule.

In a pharmaceutical composition for oral administration of the invention, the compound of formula (I) or the pharmaceutically acceptable salt thereof suitably comprises (e.g. is) Example 311, 318 or 331, as the compound or a pharmaceutically acceptable salt thereof, in particular as the compound (i.e. as the "free base" form).

A pharmaceutical composition suitable for (e.g. adapted for) parenteral (e.g. intravenous, subcutaneous, or intramuscular) administration can comprise a solution or suspension of the compound or pharmaceutically acceptable salt in a sterile pharmaceutically acceptable aqueous carrier (e.g. sterile water) or parenterally acceptable oil. Alternatively, the solution can be lyophilised. A lyophilised pharmaceutical composition suitable for (e.g. adapted for) parenteral administration may, in use, optionally be reconstituted with a suitable solvent, e.g. sterile water or a sterile parenterally acceptable aqueous solution, just prior to administration.

***Pharmaceutical compositions suitable for inhalable or intranasal administration, and particle-size reduction***

Compositions suitable for (e.g. adapted for) nasal or inhaled administration may conveniently be formulated as aerosols, drops, gels or dry powders.

Aerosol formulations, e.g. for inhaled administration, can comprise a solution or fine suspension of the active substance in a pharmaceutically acceptable aqueous or non-aqueous solvent. Aerosol formulations can be presented in single or multidose quantities in sterile form in a sealed container, which can take the form of a cartridge or refill for use with an atomising device or inhaler. Alternatively the sealed container may be a unitary dispensing device such as a single dose nasal inhaler or an aerosol dispenser fitted with a metering valve (metered dose inhaler) which is intended for disposal once the contents of the container have been exhausted.

Where the dosage form comprises an aerosol dispenser, it can contain a suitable propellant under pressure such as compressed air, carbon dioxide, or an organic propellant such as a chlorofluorocarbon (CFC) or hydrofluorocarbon (HFC). Suitable CFC propellants include dichlorodifluoromethane, trichlorofluoromethane and dichlorotetrafluoroethane. Suitable HFC propellants include 1,1,1,2,3,3,3-heptafluoropropane and 1,1,1,2-tetrafluoroethane. The aerosol dosage forms can also take the form of a pump-atomiser.

***Particle size reduction of compound of formula (I) or salt thereof***

In, for example, pharmaceutical compositions suitable (e.g. adapted for) inhaled administration, the compound or salt of formula (I) can be in a particle-size-reduced form. The size-reduced form can for example be obtained or obtainable by micronisation. Micronisation usually involves subjecting the compound/salt to collisional and/or abrasional forces in a fast-flowing circular or spiral/vortex-shaped airstream often including a cyclone component. The particle size of the size-reduced (e.g. micronised) compound or salt can be defined by a D50 value of about 0.5 to about 10 microns, e.g. about 1 to about 7 microns or about 1 to about 5 microns (e.g. as measured using laser diffraction). For example, the compound or salt of formula (I) can have a particle size defined by: a D10 of about 0.3 to about 3 microns (e.g. about 0.5 to about 2 microns, or about 1 micron), and/or a D50 of about 0.5 to about 10 microns or about 1 to about 7 microns or (e.g. about 1 to about 5 microns or about 2 to about 5 microns or about 2 to about 4 microns), and/or a D90 of about

1 to about 30 microns or about 2 to about 20 microns or about 2 to about 15 microns or about 3 to about 15 microns (e.g. about 5 to about 15 microns or about 5 to about 10 microns or about 2 to about 10 microns); for example as measured using laser diffraction.

In particle size measurements, D90, D50 and D10 respectively mean that 90%, 50% and 10% of the material is less than the micron size specified. D50 is the median particle size. DV90, DV50 and DV10 respectively, mean that 90%, 50% and 10% by volume of the material is less than the micron size specified. DM90, DM50 and DM10 respectively mean that 90%, 50% and 10% by weight of the material is less than the micron size specified.

Laser diffraction measurement of particle size can use a dry method (wherein a suspension of the compound/ salt in an airflow crosses the laser beam) or a wet method [wherein a suspension of the compound/salt in a liquid dispersing medium, such as isooctane or (e.g. if compound is soluble in isooctane) 0.1% Tween 80 in water, crosses the laser beam]. With laser diffraction, particle size can for example be calculated using the Fraunhofer calculation; and/or optionally a Malvern Mastersizer or Sympatec apparatus is used for measurement. For example, particle size measurement and/or analysis by laser diffraction can use any or all of (e.g: all of) the following: a Malvern Mastersizer longbed version, a dispersing medium of 0.1% Tween 80 in water, a stir rate of ca. 1500 rpm, ca. 3 mins sonification prior to final dispersion and analysis, a 300 RF (Reverse Fourier) lens, and/or the Fraunhofer calculation with Malvern software.

An illustrative non-limiting example of a small-scale micronisation process is now given:

***Micronisation Example:*** *Micronisation of a compound or salt of one of the Examples*

- Purpose: To micronise a compound or salt of one of the Examples (described hereinafter), e.g. in an amount of approximately 600-1000 mg thereof, using a Jetpharma MC1 micronizer.
- The parent (unmicronised) and micronised materials are analyzed for particle size by laser diffraction and crystallinity by PXRD.

*Micronisation Example: Equipment and material*

| Equipment/material | Description and specification |
|---|---|
| Jetpharma MC1 Micronizer | Nitrogen supply: Air tank with 275psi rate tubing |
| Analytical balance | Sartorius Analytical |
| Top loader balance | Mettler PM400 |
| Digital Caliper | VWR Electronic caliper |
| Materials to be micronised (Procedure 1) | a compound or salt of one of the Examples |
| Materials to be micronised (Procedure2) | a compound or salt of one of the Examples |

The Jetpharma MC1 Micronizer comprises a horizontal disc-shaped milling housing having: a tubular compound inlet (e.g. angled at ca. 30 degrees to the horizontal) for entry of a suspension of unmicronised compound of formula (I) or salt in a gasflow, a separate gas inlet for entry of gases, a gas outlet for exit of gases, and a collection vessel (micronizer container) for collecting micronised material. The milling housing has two chambers: (a) an outer annular chamber in gaseous connection with the gas inlet, the chamber being for receiving pressurised gas (e.g. air or nitrogen), and (b) a disc-shaped inner milling chamber within and coaxial with the outer chamber for micronising the input compound / salt, the two chambers being separated by an annular wall. The annular wall (ring R) has a plurality of narrow-bored holes connecting the inner and outer chambers and circumferentially-spaced-apart around the annular wall. The holes opening into the inner chamber are directed at an angle (directed part-way between radially and tangentially), and in use act as nozzles directing pressurised gas at high velocity from the outer chamber into the inner chamber and in an inwardly-spiral path (vortex) around the inner chamber (cyclone). The compound inlet is in gaseous communication with the inner chamber via a nozzle directed tangentially to the inner chamber, within and near to the annular wall / ring R. Upper and lower broad-diameter exit vents in the central axis of the inner milling chamber connect to (a) (lower exit) the collection vessel which has no air outlet, and (b) (upper exit) the gas outlet. Inside and coaxial with the tubular compound inlet and longitudinally-movable within it is positioned a venturi inlet (V) for entry of gases. The compound inlet also has a bifurcation connecting to an upwardly-directed material inlet port for inputting material.

In use, the narrow head of the venturi inlet (V) is suitably positioned below and slightly forward of the material

inlet port, so that when the venturi delivers pressurised gas (e.g. air or nitrogen) the feed material is sucked from the material inlet port into the gas stream through the compound inlet and is accelerated into the inner milling chamber tangentially at a subsonic speed. Inside the milling chamber the material is further accelerated to a supersonic speed by the hole/nozzle system around the ring (R) (annular wall) of the milling chamber. The nozzles are slightly angled so that the acceleration pattern of the material is in the form of an inwardly-directed vortex or cyclone. The material inside the milling chamber circulates rapidly and particle collisions occur during the process, causing larger particles to fracture into smaller ones. "Centrifugal" acceleration in the vortex causes the larger particles to remain at the periphery of the inner chamber while progressively smaller particles move closer to the centre until they exit the milling chamber, generally through the lower exit, at low pressure and low velocity. The particles that exit the milling chamber are heavier than air and settle downward thorugh the lower exit into the collection vessel (micronizer container), while the exhaust gas rises (together with a minority of small particles of micronised material) and escapes into the atmosphere at low pressure and low velocity.

*Micronisation Example: Procedure:*

The micronizer is assembled. The narrow head of the venturi inlet is positioned below and slightly forward of the material inlet port and is measured with a micro-caliper to make sure that it is inserted correctly. The ring (R) and venturi (V) pressures are adjusted according to the values specified in the experimental design (refer to experimental section below) by adjusting the valves on the pressure gauges on the micronizer. The setup is checked for leakage by observing if there is any fluctuation in the reading of the pressure gauges.

Note that the venturi (V) pressure is kept at least 2 bars greater than the ring (R) pressure to prevent regurgitation of material, e.g. outwardly from the material inlet port.

Balance performance is checked with calibration weights. Specified amount of the parent material is fed into the input container of the micronizer using a spatula. The input container plus material is weighed. The equipment pressure is monitored during the micronization process.

Upon completion of the micronising run, the nitrogen supply is shut off and the micronised material is allowed to settle into the micronizer container. The micronised powder in the micronizer container (collection vessel) and the cyclone (above the recovery vessel) are collected together into a pre-weighed and labelled collection vial. The weight of the micronised material is recorded. The input container is re-weighed in order to calculate the amount of input material by difference. The micronizer is disassembled and residual PDE4 compound on the micronizer inner surface is rinsed with 70/30 isopropyl alcohol / water and collected into a flask. The micronizer is then thoroughly cleaned in a Lancer washing machine and dried before subsequent runs are performed.

*Micronisation Example: Optional Experimental Parameters*

*Procedure 1: Optional Experimental Parameters*

This experiment, Procedure 1, can optionally be carried out generally using a procedure and an apparatus generally as described above or similar to those described, using generally the following experimental parameters:

| Procedure no. | Material input amount (g) | Venturi Pressure (V) / ring (R) Pressure (bar) |
|---|---|---|
| 1 | ca. 0.9 g | V = 5 to 7 bar<br>R = 3 to 4bar |

% yield = (Material from collection vessel + Material from cyclone)/Material input amount] x 100

The above optional parameters can be varied using the skilled person's knowledge.

*Procedure 2: Optional Experimental Parameters*

The optional experimental parameters can for example be as follows:

| Procedure no. | Material input amount (g) | Venturi Pressure (V) / ring (R) Pressure (bar) | Intended feed-rate |
|---|---|---|---|
| 2 | ca. 0.9 g | V = 8 to 10 bar<br>R = 5.5 to 6 bar | 180 to 200 mg/min |

The above optional parameters can be varied using the skilled person's knowledge.

***Dry powder inhalable compositions***

For pharmaceutical compositions suitable (e.g. adapted for) inhaled administration, the pharmaceutical composition may for example be a dry powder inhalable composition. Such a composition can comprise a powder base such as lactose or starch, the compound of formula (I) or salt thereof (suitably in particle-size-reduced form, e.g. in micronised form), and optionally a ternary agent such as L-leucine, mannitol, trehalose, magnesium stearate and/or cellobiose octaacetate (e.g. alpha-D-isomer of cellobiose octaacetate, e.g. available from Aldrich). For cellobiose octaacetate and storage stability, see WO 03/088943.

The dry powder inhalable composition can comprise a dry powder blend of lactose and the compound of formula (I) or salt thereof. The lactose can be lactose hydrate e.g. lactose monohydrate and/or can be inhalation-grade and/or fine-grade lactose. The particle size of the lactose can for example be defined by 90% or more (by weight or by volume) of the lactose particles being less than 1000 microns (micrometres) (e.g. 10-1000 microns e.g. 30-1000 microns) in diameter, and/or 50% or more of the lactose parties being less than 500 microns (e.g. 10-500 microns) in diameter. The particle size of the lactose can for example be defined by 90% or more of the lactose particles being less than 300 microns (e.g. 10-300 microns e.g. 50-300 microns) in diameter, and/or 50% or more of the lactose particles being less than 100 microns in diameter. Optionally, the particle size of the lactose can be defined by 90% or more of the lactose particles being less than 100-200 microns in diameter, and/or 50% or more of the lactose particles being less than 40-70 microns in diameter. It can be about 3 to about 30% (e.g. about 10%) (by weight or by volume) of the particles are less than 50 microns or less than 20 microns in diameter. For example, without limitation, a suitable inhalation-grade lactose is E9334 lactose (10% fines) (Borculo Domo Ingredients, Hanzeplein 25, 8017 JD Zwolle, Netherlands).

In the dry powder inhalable composition the compound of formula (I) or salt thereof can for example be present in about 0.1% to about 70% (e.g. about 1% to about 50%, e.g. about 5% to about 40%, e.g. about 20 to about 30%) by weight of the composition.

An illustrative non-limiting example of a dry powder inhalable composition follows:

*Dry Powder Formulation Example - Dry powder Lactose Blend Preparation*

Using a size-reduced e.g. micronised form of the compound of formula (I) or salt thereof (e.g. as prepared in the Micronisation Example herein), the dry powder blend is, for example, prepared by mixing the required amount of the compound/salt (e.g. 10 mg, 1% w/w) with inhalation-grade lactose containing 10% fines (e.g. 990 mg, 99% w/w) in a Teflon™ (polytetrafluoroethene) pot in a Mikro-dismembrator ball-mill (but without a ball bearing) at % speed (ca. 2000-2500 rpm) for about 4 hours at each blend concentration. The Mikro-dismembrator (available from B. Braun Biotech International, Schwarzenberger Weg 73-79, D-34212 Melsungen, Germany; www.bbraunbiotech.com) comprises a base with an upwardly-projecting and sidewardly-vibratable arm to which is attached the Teflon ™ pot. The vibration of the arm achieves blending.

Other blends can include: 10% w/w compound/salt (50 mg) + 90% w/w lactose (450 mg, inhalation-grade lactose containing 10% fines).

Serial dilution of the 1 % w/w blend can achieve e.g. 0.1% and 0.3% w/w blends.

***Dry powder inhalation devices***

Optionally, in particular for dry powder inhalable compositions, a pharmaceutical composition for inhaled administration can be incorporated into a plurality of sealed dose containers (e.g. containing the dry powder composition) mounted longitudinally in a strip or ribbon inside a suitable inhalation device. The container can be rupturable or peel-openable on demand and the dose, e.g. of the dry powder composition, can be administered by inhalation via a device such as the DISKUS ™ device, marketed by GlaxoSmithKline. The DISKUS™ inhalation device can e.g. be substantially as described in GB 2,242,134 A. In such device at least one container for the pharmaceutical composition in powder form (the at least one container in particular being a plurality of sealed dose containers mounted longitudinally in a strip or ribbon) is defined between two members peelably secured to one another; the device comprises: means defining an opening station for the said at least one container; means for peeling the members apart at the opening station to open the container; and an outlet, communicating with the opened container; through which a user can inhale thy pharmaceutical composition in powder form from the opened container.

***Dosage regimens***

In a pharmaceutical composition suitable for (e.g. adapted for) external topical administration, e.g. an ointment or an oil-in-water or water-in-oil composition, the compound of formula (I) or the pharmaceutically acceptable salt thereof can be present in 0.1% to 10%, such as 0.2% to 10% or 0.2% to 5%, or 0.5% to 10% or 0.5% to 5%, or 1% to 10% or

1% to 5%, or 0.5% to 3%, or 1% to 3% (e.g. about 0.5% or in particular about 2%), by weight of the composition (w/w). Typically, an external-topical pharmaceutical composition can be administered once daily, twice daily or more than twice daily, to external body part(s), e.g. to the skin such as at a site of diseased skin. The amount administered is usually such as substantially to cover the site(s) of diseased skin

A pharmaceutical composition of the invention can e.g. be in unit dose form such as a tablet or capsule for oral administration, e.g. for oral administration to a human.

In the pharmaceutical composition of the invention, a or each dosage unit for oral or parenteral administration can for example contain from 0.01 to 3000 mg, such as 0.5 to 1000 mg, of a compound of the formula (I) or a pharmaceutically acceptable salt thereof, calculated as the free base. A or each dosage unit for nasal or inhaled administration can for example contain from 0.001 to 50 mg, such as 0.01 to 5 mg, of a compound of the formula (I) or a pharmaceutically acceptable salt thereof, calculated as the free base.

When a parenteral or oral composition is used, a pharmaceutically acceptable compound or salt of the invention can for example be administered to a mammal (e.g. human) in a daily oral or parenteral dose of 0.001 mg to 50 mg per kg body weight per day (mg/kg/day), for example 0.01 to 20 mg/kg/day or 0.03 to 10 mg/kg/day or 0.1 to 2 mg/kg/day, of the compound of the formula (I) or a pharmaceutically acceptable salt thereof, calculated as the free base.

When a parenteral or oral composition is used, a pharmaceutically acceptable compound or salt of the invention can for example be administered to a mammal (e.g. human) in a daily nasal or inhaled dose of: 0.0001 to 5 mg/kg/day or 0.0001 to 1 mg/kg/day, e.g. 0.001 to 1 mg/kg/day or 0.001 to 0.3 mg/kg/day or 0.001 to 0.1 mg/kg/day or 0.005 to 0.3 mg/kg/day, of the compound of the formula (I) or a pharmaceutically acceptable salt thereof, calculated as the free base.

The pharmaceutically acceptable compounds or salts of the invention can for example be administered to a human in a daily dose (for an adult patient) of, for example, an oral or parenteral dose of 0.01 mg to 3000 mg per day or 0.5 to 1000 mg per day e.g. 2 to 500 mg per day, or a nasal or inhaled dose of 0.001 to 50 mg per day or 0.01 to 30 mg per day or 0.01 to 5 mg per day or 0.02 to 2 mg per day, of the compound of the formula (I) or a pharmaceutically acceptable salt thereof, calculated as the free base.

**COMBINATIONS**

The compounds, salts and/or pharmaceutical compositions according to the invention may also be used in combination with another therapeutically active agent, for example, a $\beta_2$ adrenoreceptor agonist, an anticholinergic compound (e.g. muscarinic (M) receptor antagonist), an anti-histamine, an anti-allergic, an anti-inflammatory agent, an antiinfective agent or an immunosuppressant.

The invention thus provides, in a further aspect, a combination comprising a compound of formula (I) or a pharmaceutically acceptable salt thereof together with another therapeutically active agent, for example, a $\beta_2$-adrenoreceptor agonist, an anti-histamine, an anti-allergic, an anti-inflammatory agent, an antiinfective agent or an immunosuppressant.

In one particular embodiment, the $\beta_2$-adrenoreceptor agonist is salmeterol (e.g. as racemate or a single enantiomer such as the R-enantiomer), salbutamol, formoterol, salmefamol, fenoterol or terbutaline, or a salt thereof (e.g. pharmaceutically acceptable salt thereof), for example the xinafoate salt of salmeterol, the sulphate salt or free base of salbutamol or the fumarate salt of formoterol. Long-acting $\beta_2$-adrenoreceptor agonists are optionally used, especially those having a therapeutic effect over a 12-24 hour period such as salmeterol or formoterol. In particular, the $\beta_2$-adrenoreceptor agonist is for inhaled administration, e.g. once per day and/or for simultaneous inhaled administration; and in particular the $\beta_2$-adrenoreceptor agonist can be in particle-size-reduced form e.g. as defined herein. The $\beta_2$-adrenoreceptor agonist combination can for example be for treatment and/or prophylaxis of COPD or asthma. Salmeterol or a pharmaceutically acceptable salt thereof, e.g. salmeterol xinofoate, is suitably administered to humans at an inhaled dose of 25 to 50 micrograms twice per day (measured as the free base).

Examples of long acting $\beta_2$-adrenoreceptor agonists which could be used include those described in WO 02/066422A, WO 03/024439, WO 02/070490 and WO 02/076933.

Particular examples of $\beta_2$-adrenoreceptor agonists disclosed in WO 02/066422 include: 3-(4-{[6-({(2R)-2-hydroxy-2-[4-hydroxy-3-(hydroxymethyl)-phenyl]ethyl}amino)hexyl]oxy}butyl)benzenesulfonamide and 3-(3-{[17-({(2R)-2-hydroxy-2-[4-hydroxy-3-hydroxymethyl)phenyl]ethyl}-amino)heptyl]oxy}propyl)benzenesulfonamide.

A particular example of a $\beta_2$-adrenoreceptor agonist disclosed in WO 03/024439 is:

4-{(1*R*)-2-[(6-{2-[(2,6-dichlorobenzyl)oxy]ethoxy}hexyl)amino]-1-hydroxyethyl}-2-(hydroxymethyl)phenol.

An anti-histamine usable in a combination of a compound of formula (I) or salt can for example be for oral administration (e.g. as a combined composition such as a combined tablet), and can be for treatment and/or prophylaxis of allergic rhinitis. Examples of antihistamines include methapyrilene, or H1 antagonists such as cetirizine, loratadine

(e.g. Clarityn™), desloratadine (e.g. Clarinex™) or fexofenadine (e.g. Allegra™).

The invention also provides, in a further aspect, a combination comprising a compound of formula (I) or a pharmaceutically acceptable salt thereof together with an anticholinergic compound, e.g. a muscarinic (M) receptor antagonist such as an $M_1$, $M_2$, $M_1/M_2$, or $M_3$ receptor antagonist, in particular a $M_3$ receptor antagonist, such as a $M_3$ receptor antagonist which selectively antagonises (e.g. antagonises 10 times or more strongly) the $M_3$ receptor over the $M_1$ and/or $M_2$ receptor. For combinations of anticholinergic compounds / muscarinic (M) receptor antagonist with PDE4 inhibitors, see for example WO 03/011274 A2 and WO 02/069945 A2 / US 2002/0193393 A1 and US 2002/052312 A1, and some or all of these publications give examples of : anticholinergic compounds / muscarinic (M) receptor antagonists which may be used with the compounds of formula (I) or salts, and/or suitable pharmaceutical compositions. For example, the muscarinic receptor antagonist can comprise or be an ipratropium salt (e.g. ipratropium bromide), an oxitropium salt (e.g. oxitropium bromide), or a tiotropium salt (e.g. tiotropium bromide); see e.g. EP 418 716 A1 for tiotropium.

The anticholinergic compound or muscarinic (M) receptor antagonist, e.g. $M_3$ receptor antagonist, can for example be for inhaled administration, in particular in particle-size-reduced form e.g. as defined herein. In once optional embodiment, both the muscarinic (M) receptor antagonist and the compound of formula (I) or the pharmaceutically acceptable salt thereof are for inhaled administration. For example, the anticholinergic compound or muscarinic receptor antagonist and the compound of formula (I) or salt are optionally for simultaneous administration. The muscarinic receptor antagonist combination is optionally for treatment and/or prophylaxis of COPD.

Other possible combinations include, for example, a combination comprising a compound of formula (I) or a pharmaceutically acceptable salt thereof together with another anti-inflammatory agent such as an anti-inflammatory corticosteroid; or a non-steroidal anti-inflammatory drug (NSAID) such as a leukotriene antagonist (e.g. montelukast), an iNOS inhibitor, a tryptase inhibitor, a elastase inhibitor, a beta-2 integrin antagonist, a adenosine 2a agonist, a CCR3 antagonist, or a 5-lipoxogenase inhibitor; or an antiinfective agent (e.g. an antibiotic or an antiviral). An iNOS inhibitor is can e.g. be for oral administration. Examples of iNOS inhibitors (inducible nitric oxide synthase inhibitors) include those disclosed in WO 93/13055, WO 98/30537, WO 02/50021, WO 95/34534 and WO 99/62875.

Example combinations, in particular for external topical administration (e.g. versus atopic dermatitis), include, for example, a combination comprising a compound of formula (I) or a pharmaceutically acceptable salt thereof together with an immunosuppressant, e.g. a calcineurin inhibitor such as pimecrolimus or tacrolimus. The immunosuppressant can in particular be an externally-topically administrable immunosuppressant such as pimecrolimus (e.g. pimecrolimus at ca. 1% w/w concentration in a topical composition such as a cream, and/or e.g. Elidel ™) or tacrolimus (e.g. tacrolimus at from about 0.03% to about 0.1 % w/w concentration in a topical composition such as an ointment, and/or e.g. Protopic ™). The externally-topically administrable immunosuppressant can be administered or administrable in a external-topical composition separately from the compound or salt of the invention, or it can be contained with the compound of formula (I) or pharmaceutically acceptable salt in a combined externally-topically-administrable composition.

For external topical administration, e.g. versus an inflammatory and/or allergic skin disease such as atopic dermatitis or psoriasis, in a combination of the compound or salt of the invention together with an anti-infective agent, the anti-infective agent can include (e.g. be) an externally-topically-administrable antibacterial, such as mupiricin or a salt thereof (e.g. mupiricin calcium salt) (e.g. Bactroban ™) or such as an extemally-topically-administrable pleuromutilin antibacterial (e.g. retapamulin or a salt thereof, which can be present in about 1% w/w by weight of an externally-topically-administrable pharmaceutical composition, such as an ointment). Alternatively or additionally, for external topical administration, the anti-infective agent can include an externally-topically-administrable antifungal such as clortrimazole, clotrimazole or ketoconazole.

For external topical administration, e.g. versus atopic dermatitis, a combination with an anti-itch compound may optional be used.

In a combination comprising a compound of formula (I) or a pharmaceutically acceptable salt thereof together with an anti-inflammatory corticosteroid (which can for example be for treatment and/or prophylaxis of asthma, COPD or allergic rhinitis), then optionally the anti-inflammatory corticosteroid is fluticasone propionate (e.g. see US patent 4,335,121), beclomethasone 17-propionate ester, beclomethasone 17,21-dipropionate ester, dexamethasone or an ester thereof, mometasone or an ester thereof (e.g. mometasone furoate), ciclesonide, budesonide, flunisolide, or a compound as described in WO 02/12266 A1 (e.g. as claimed in any of claims 1 to 22 therein), or a pharmaceutically acceptable salt of any of the above. If the anti-inflammatory corticosteroid is a compound as described in WO 02/12266 A1, then it can for example be Example 1 therein {which is 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester} or Example 41 therein {which is 6α, 9α-difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester}, or a pharmaceutically'acceptable salt thereof. The anti-inflammatory corticosteroid can for example be for external topical, intranasal or inhaled administration. Fluticasone propionate can be used for inhaled administration to a human; for example either (a) at a dose of 250 micrograms once per day or (b) at a dose of 50 to 250 micrograms twice per day.

Also provided is a combination comprising a compound of formula (I) or a pharmaceutically acceptable salt thereof together with $\beta_2$-adrenoreceptor agonist and an anti-inflammatory corticosteroid, for example as described in WO 03/030939 A1. This combination can for example be for treatment and/or prophylaxis of asthma, COPD or allergic rhinitis. The $\beta_2$-adrenoreceptor agonist and/or the anti-inflammatory corticosteroid can be as described above and/or as described in WO 03/030939 A1. In this "triple" combination, the $\beta_2$-adrenoreceptor agonist can e.g. be salmeterol or a pharmaceutically acceptable salt thereof (e.g. salmeterol xinafoate), and the anti-inflammatory corticosteroid can e.g. be fluticasone propionate.

The combinations referred to above may be presented for use in the form of a pharmaceutical composition and thus a pharmaceutical composition comprising a combination as defined above together with one or more pharmaceutically acceptable carriers and/or excipients represent a further aspect of the invention.

The individual compounds of such combinations may be administered either sequentially or simultaneously in separate or combined pharmaceutical composition.

In one embodiment, the combination as defined herein can be for simultaneous inhaled administration and is disposed in a combination inhalation device. Such a combination inhalation device is another aspect of the invention. Such a combination inhalation device can comprise a combined pharmaceutical composition for simultaneous inhaled administration (e.g. dry powder composition), the composition comprising all the individual compounds of the combination, and the composition being incorporated into a plurality of sealed dose containers mounted longitudinally in a strip or ribbon inside the inhalation device, the containers being rupturable or peel-openable on demand; for example such inhalation device can be substantially as described in GB 2,242,134 A (DISKUS™) and/or as described above. Alternatively, the combination inhalation device can be such that the individual compounds of the combination are administrable simultaneously but are stored separately (or wholly or partly stored separately for triple combinations), e.g. in separate pharmaceutical compositions, for example as described in PCT/EP03/00598 filed on 22 January 2003, published as WO 03/061743 (e.g. as described in the claims thereof e.g. claim 1).

The invention also provides a method of preparing a combination as defined herein, the method comprising either

(a) preparing a separate pharmaceutical composition for administration of the individual compounds of the combination either sequentially or simultaneously, or
(b) preparing a combined pharmaceutical composition for administration of the individual compounds of the combination simultaneously,

wherein the pharmaceutical composition comprises the combination together with one or more pharmaceutically acceptable carriers and/or excipients.

The invention also provides a combination as defined herein, prepared by a method as defined herein.

## BIOLOGICAL TEST METHODS

### PDE 3, PDE 4B, PDE 4D, PDE 5, PDE 6 Primary assay methods

The biological activity of the compounds or salts of the invention can be measured in the assay methods shown below.

Some of the Examples disclosed herein and encompassed within the invention are selective PDE4 inhibitors, i.e. they inhibit PDE4 (e.g. PDE4B) more strongly than they inhibit PDE3 and/or more strongly than they inhibit PDE5 and/or more strongly than they inhibit PDE6. It is to be recognised that such selectivity is not essential to the invention.

### *Possible PDE enzyme sources and literature references*

Human recombinant PDE4B, in particular the 2B splice variant thereof (HSPDE4B2B), is disclosed in WO 94/20079 and also M.M. McLaughlin et al.; "A low Km, rolipram-sensitive, cAMP-specific phosphodiesterase from human brain: cloning and expression of cDNA, biochemical characterisation of recombinant protein, and tissue distribution of mRNA", J. Biol. Chem., 1993, 268, 6470-6476. For example, in Example 1 of WO 94/20079, human recombinant PDE4B is described as being expressed in the PDE-deficient yeast *Saccharomyces cerevisiae* strain GL62, e.g. after induction by addition of 150 uM $CuSO_4$, and 100,000 x *g* supernatant fractions of yeast cell lysates are described for use in the harvesting of PDE4B enzyme.

Human recombinant PDE4D (HSPDE4D3A) is disclosed in P. A. Baecker et al., "Isolation of a cDNA encoding a human rolipram-sensitive cyclic AMP phoshodiesterase (PDE IVD)", Gene, 1994, 138, 253-256.

Human recombinant PDE5 is disclosed in K. Loughney et al., "Isolation and characterisation of cDNAs encoding PDE5A, a human cGMP-binding, cGMP-specific 3',5'-cyclic nucleotide phosphodiesterase", Gene, 1998, 216, 139-147.

PDE3 can be purified from bovine aorta as described by H. Coste and P. Grondin, "Characterisation of a novel potent and specific inhibitor of type V phosphodiesterase", Biochem. Pharmacol., 1995, 50, 1577-1585.

PDE6 can be purified from bovine retina as described by: P. Catty and P. Deterre, "Activation and solubilization of the retinal cGMP-specific phosphodiesterase by limited proteolysis", Eur. J. Biochern., 1991; 199, 263-269; A. Tar et al. "Purification of bovine retinal cGMP phosphodiesterase", Methods in Enzymology, 1994, 238, 3-12; and/or D. Srivastava et al. "Effects of magnesium on cyclic GMP hydrolysis by the bovine retinal rod cyclic GMP phosphodiesterase", Biochem. J., 1995, 308, 653-658.

***Inhibition of PDE 3, PDE 4B, PDE 4D, PDE 5 or PDE 6 activity: radioactive Scintillation Proximity Assay (SPA)***

The ability of compounds to inhibit catalytic activity at PDE4B or 4D (human recombinant), PDE3 (from bovine aorta), PDE5 (human recombinant) or PDE6 (from bovine retina) can optionally be determined by Scintillation Proximity Assay (SPA) in a 96-well format.

Test compounds (as a solution in DMSO, suitably about 2 microlitre (ul) volume of DMSO solution) are preincubated at ambient temperature (room temperature, e.g. 19-23˚C) in Wallac Isoplates (code 1450-514) with PDE enzyme in 50mM Tris-HCl buffer pH 7.5 , 8.3mM $MgCl_2$, 1.7mM EGTA, 0.05% (w/v) bovine serum albumin for 10-30 minutes (usually 30 minutes). The enzyme concentration is adjusted so that no more than 20% hydrolysis of the substrate defined below occurs in control wells without compound, during the incubation. For the PDE3, PDE4B and PDE4D assays, [5', 8-$^3$H]Adenosine 3',5'-cyclic phosphate (Amersham Pharmacia Biotech, code TRK.559; or Amersham Biosciences UK Ltd, Pollards Wood, Chalfont St Giles, Buckinghamshire HP8 4SP, UK) is added to give 0.05uCi per well and about 10nM final concentration. For the PDE5 and PDE6 assays, [8-$^3$H]Guanosine 3',5'-cyclic phosphate (Amersham Pharmacia Biotech, code TRK.392) is added to give 0.05uCi per well and about 36nM final concentration. Plates containing assay mixture, suitably approx. 100 ul volume of assay mixture, are mixed on an orbital shaker for 5 minutes and incubated at ambient temperature for 1 hour. Phosphodiesterase SPA beads (Amersham Pharmacia Biotech, code RPNQ 0150) are added (about 1 mg per well) to terminate the assay. Plates are sealed and shaken and allowed to stand at ambient temperature for 35 minutes to 1 hour (suitably 35 minutes) to allow the beads to settle. Bound radioactive product is measured using a WALLAC TRILUX 1450 Microbeta scintillation counter. For inhibition curves, 10 concentrations (e.g. 1.5nM - 30uM) of each compound are assayed. Curves are analysed using ActivityBase and XLfit (ID Business Solutions Limited, 2 Ocean Court, Surrey Research Park, Guildford, Surrey GU2 7QB, United Kingdom). Results are expressed as $pIC_{50}$ values.

In an alternative to the above radioactive SPA assay, PDE4B or PDE4D inhibition can be measured in the following Fluorescence Polarisation (FP) assay:

***Inhibition of PDE4B or PDE4D activity: Fluorescence polarisation (FP) assay***

The ability of compounds to inhibit catalytic activity at PDE4B (human recombinant) or PDE4D (human recombinant) can optionally be determined by IMAP Fluorescence Polarisation (FP) assay (IMAP Explorer kit, available from Molecular Devices Corporation, Sunnydale, CA, USA; Molecular Devices code: R8062) in 384-well format.

The IMAP FP assay is able to measure PDE activity in an homogenous, nonradioactive assay format. The FP assay uses the ability of immobilised trivalent metal cations, coated onto nanoparticles (tiny beads), to bind the phosphate group of Fl-AMP that is produced on the hydrolysis of fluorescein-labelled (Fl) cyclic adenosine monophosphate (Fl-cAMP) to the non-cyclic Fl-AMP forum. Fl-cAMP substantially does not bind. Binding of Fl-AMP product to the beads (coated with the immobilised trivalent cations) slows the rotation of the bound Fl-AMP and leads to an increase in the fluorescence polarisation ratio of parallel to perpendicular light. Inhibition of the PDE reduces/inhibits this signal increase.

Test compounds (small volume, e.g. ca. 0.5 to 1 microlitres (ul), suitably ca. 0.5 ul, of solution in DMSO) are preincubated at ambient temperature (room temperature, e.g. 19-23˚C) in black 384-well microtitre plates (supplier: NUNC, code 262260) with PDE enzyme in 10mM Tris-HCl buffer pH 7.2, 10mM $MgCl_2$, 0.1% (w/v) bovine serum albumin, and 0.05% $NaN_3$ for 10-30 minutes. The enzyme level is set by experimentation so that reaction is linear throughout the incubation. Fluorescein adenosine 3',5'-cyclic phosphate (from Molecular Devices Corporation, Molecular Devices code: R7091) is added to give about 40nM final concentration (final assay volume usually ca. 20-40 ul, suitably ca. 20 ul). Plates are mixed on an orbital shaker for 10 seconds and incubated at ambient temperature for 40 minutes. IMAP binding reagent (as described above, from Molecular Devices Corporation, Molecular Devices code: R7207) is added (60ul of a 1 in 400 dilution in binding buffer of the kit stock solution) to terminate the assay. Plates are allowed to stand at ambient temperature for 1 hour. The Fluorescence Polarisation (FP) ratio of parallel to perpendicular light is measured using an Analyst™ plate reader (from Molecular Devices Corporation). For inhibition curves, 10 concentrations (e.g. 1.5nM - 30uM) of each compound are assayed. Curves are analysed using ActivityBase and XLfit (ID Business Solutions Limited, 2 Ocean Court, Surrey Research Park, Guildford, Surrey GU2 7QB, United Kingdom). Results are expressed as $pIC_{50}$ values.

In the FP assay, reagents can be dispensed using Multidrop™ (available from Thermo Labsystems Oy, Ratastie 2, PO Box 100, Vantaa 01620, Finland).

For a given PDE4 inhibitor, the PDE4B (or PDE4D) inhibition values measured using the SPA and FP assays can differ slightly.

Biological Data obtained for some of the Examples (PDE4B inhibitory activity, either as one reading (n = 1) or as an average of 2 or more readings (n = 2 or more) are generally as follows, based on measurements only, generally using SPA and/or FP assay(s) generally as described above or generally similar or generally analogous to those described above. In each of the SPA and FP assays, absolute accuracy of measurement is not possible, and the readings given are generally thought to be accurate only up to very approximately ± 0.5 of a log unit, depending on the number of readings made and averaged:

| Example numbers | PDE4B $pIC_{50}$ (± about 0.5) |
|---|---|
| 77, 88, 93, 108, 120, 124, 125, 138, 146, 150, 151, 154, 155, 156, 158, 169, 173, 177, 187, 189, 191, 192, 195, 196, 198, 200, 202, 203, 206, 214, 216, 218, 219, 225, 226, 227, 228, 229, 231, 234, 237, 238, 239, 249, 259, 266, 279, 282, 283, 284, 286, 287, 289, 290, 300, .301, 307, 308, 309, 310, 312, 313, 314, 318, 319, 322, 324, 325, 326, 331, 334, 335, 336, 337, 341, 343, 344, 346, 347, 348 and 349 | about 9.0 to about 9.9 |
| 116, 147, 148, 149, 153, 157, 159, 160, 161, 174, 175, 176, 178, 179, 180, 182, 183, 184, 185, 186, 188, 190, 193, 194, 199, 204, 205, 207, 208, 209, 210, 211, 212, 220, 232, 233, 235, 236, 240, 241, 242, 243, 244, 245, 246, 247, 248, 250, 251, 252, 253, 254, 255, 256, 257, 258, 260, 261, 262, 263, 264, 265, 267, 268, 269, 270, 271, 272, 273, 274, 275, 276, 277, 278, 280, 281, 285, 288, 302, 303, 304, 305, 306, 311, 315, 316, 317, 323, and 339 | about 10.0 to about 11.0 |

A large majority or substantially all of the Examples (excluding Reference Examples) have been tested for PDE4B inhibition, mostly using the the FP assay generally as described above or generally similar or generally analogous to those described above.

A large majority or substantially all of the Examples (excluding Reference Examples) tested have PDE4B inhibitory activities in the range of $pIC_{50}$ = about 6.4 (± about 0.5) to about 11.0 (± about 0.5).

Only selected ones of the PDE4B-tested Examples have also been tested, on an optional basis, for one or more of: PDE3, PDE5 or PDE6 inhibition, e.g. using the above-described or other assays.

*Emesis:* Some known PDE4 inhibitors can cause emesis and/or nausea to greater or lesser extents, especially after systemic exposure e.g. after oral administration (e.g. see Z. Huang et al., Current Opinion in Chemical Biology, 2001, 5: 432-438, see especially pages 433-434 and refs cited therein). Therefore, it would be preferable, but not essential, if a PDE4 inhibitory compound or salt of the invention were to cause only limited or manageable emetic side-effects, e.g. after oral or parenteral or external-topical administration. Emetic side-effects can for example be measured by the emetogenic potential of the compound or salt when administered to ferrets; for example one can measure the time to onset, extent, frequency and/or duration of vomiting, retching and/or writhing in ferrets after oral or parenteral administration of the compound or salt. See for example In vivo Assay 4 hereinafter for one optional measurement method for anti-inflammatory effect, emetic side-effects and therapeutic index (TI) in the ferret. See also for example A. Robichaud et al., "Emesis induced by inhibitors of [PDE IV] in the ferret", Neuropharmacology, 1999, 38, 289-297, erratum Neuropharmacology, 2001, 40, 465-465. However, optionally, emetic side-effects and therapeutic index (TI) after oral administration in rats can be conveniently measured by monitoring the pica feeding behaviour of rats after administration of the compound or salt of the invention (see In Vivo Assay 2 below).

**Other optional *in vitro* assays:**

***Inhibition of TNF-α (TNF-alpha) Production in Human PBMC (peripheral blood mononuclear cell) assay (MSD technology)***

This is an optional supplementary test.

A 96-well plate (96 MicroWell™ Plates Nunclon™Δ - High Flange Design, Fisher Scientific UK, Bishop Meadow Road, Loughborough LE 11 5 RG, Leicestershire, UK) is prepared by initially adding to column 1 ca. 10mM of test compound dissolved in DMSO. For a more potent compound, a more diluted solution in DMSO may be used. The compound is further diluted with DMSO into columns 2 to 9 by 8 successive 3-fold dilutions using the Biomek® FX Laboratory Automation Workstation (Beckman Coulter, Inc., 4300 N. Harbor Boulevard; P.O. Box 3100, Fullerton, CA

92834-3100 USA). Column 10 is used as a DMSO negative control (High Signal, 0% response), whilst column 11, which contains 10 mM of the PDE4 inhibitor roflumilast, is used as a positive control (Low Signal, 100% response). About 1 μl (about 1ul) of compound is transferred to the compound plate using the Biomek® FX.

PBMC cells (peripheral blood mononuclear cells) are prepared from heparinised human blood (using 1% v/v Heparin Sodium 10001U/ml Endotoxin Free, Leo Laboratories Ltd., Cashel Road, Dublin 12 . Ireland, Cat No: PL0043/0149) from normal volunteers using the Accuspin™ System-Histopaque®-1077 essentially (Sigma-Aldrich Company Ltd., The Old Brickyard New Rd, Gillinghan Dorset SP8 4XT). About 20 ml of blood is overlaid onto 15m1 Histopaque® in Accuspin™ tubes. The tube is then centrifuged at about 800g for ca. 20 minutes. The cells are collected from the interface, washed by centrifugation (ca. 1300g, ca. 10 minutes) and resuspended in RPMI1640 medium (Low endotoxin RPMI1640 medium, Cat No: 31870-025, Invitrogen Corporation Invitrogen Ltd, 3 Fountain Drive, Inchinnan Business Park, Paisley PA4 9RF, UK) containing 10% foetal calf serum, 1% L-glutamine (Invitrogen Corporation, Cat No: 25030024) and 1% penicillin/streptomycin (Invitrogen Corporation, Cat No: 15140-122). Viable cells are counted by trypan blue staining and diluted to $1x10^6$ viable cells/ml. About 50μl (about 50ul) of diluted cells and about 75μl (about 75ul) of LPS (ca. 1 ng/ml final; Sigma Cat No: L-6386) are added to the compound plate, which is then incubated at 37˚C, 5% $CO_2$, for 20 hours.

The supernatant is removed and the concentrations of TNF-α are determined by electrochemiluminescence assay using the Meso Scale Discovery (MSD) technology (Meso Scale Discovery, 9238 Gaither Road, Gaithersburg, Maryland 20877, USA). See the "TNF-α (TNF-alpha) MSD Assay" described below for typical details.

Results can be expressed as pIC50 values for inhibition of TNF-α (TNF-alpha) production in PBMCs, and it should be appreciated that these results can be subject to variability or error.

***Inhibition of TNFα (TNF-alpha) Production in Human PBMC (peripheral blood mononuclear cell) assay (IGEN technology)***

This is an optional supplementary test.

Test compounds are prepared as a ca. 10mM stock solution in DMSO and a dilution series prepared in DMSO with 8 successive 3-fold dilutions, either directly from the 10mM stock solution or from a more dilute solution in DMSO. The compound is added to assay plates using a Biomek Fx liquid handling robot.

PBMC cells (peripheral blood mononuclear cells) are prepared from heparinised human blood from normal volunteers by centrifugation on histopaque at ca. 1000g for ca. 30 minutes. The cells are collected from the interface, washed by centrifugation (ca. 1300g, ca. 10 minutes) and resuspended in assay buffer (RPMI1640 containing 10% foetal calf serum, 1% L-glutamine and 1% penicillin / streptomycin) at $1x10^6$ cells/ml. Ca. 50μl (ca. 50ul) of cells are added to microtitre wells containing ca. 0.5 or ca/1.0μl (ul) of an appropriately diluted compound solution. Ca. 75μl (ul) of LPS (lipopolysaccharide) (ca. 1 ng/ml final) is added and the samples are incubated at 37 ˚C, 5% $CO_2$, for 20 hours. The supernatant is removed and the concentrations of TNF-α are determined by electrochemiluminescence assay using the IGEN technology or by ELISA (see below).

Results can be expressed as pIC50 values for inhibition of TNF-α (TNF-alpha) production in PBMCs, and it should be appreciated that these results can be subject to variability or error.

***Inhibition of TNFα (TNF-alpha) Production in Human Whole Blood***

This is an optional supplementary test, e.g. for potentially orally-administrable PDE4 inhibitors. Also, as the assay may measure the effect of PDE4 inhibitors after loss by protein binding, it might possibly also be relevant to externally-topically-administrable PDE4 inhibitors as protein-binding-loss of compound is possible during transport through the skin.

Test compounds are prepared as a ca. 10mM stock solution in DMSO and a dilution series prepared in DMSO with 8 successive 3-fold dilutions, either directly from the 10mM stock solution or from a more dilute solution in DMSO. The compound is added to assay plates using a Biomek Fx liquid handling robot.

Heparinised blood drawn from normal volunteers is dispensed (ca. 100μl = ca. 100ul) into microtitre plate wells containing ca. 0.5 or ca. 1.0μl (ul, microlitres) of an appropriately diluted test compound solution. After ca. 1 hr incubation at ca. 37 ˚C, 5% $CO_2$, ca. 25μl (ca. 25ul) of LPS (lipopolysaccharide) solution (S. typhosa) in RPMI 1640 (containing 1% L-glutamine and 1% Penicillin/ streptomycin) is added (ca. 50ng/ml final). The samples are incubated at ca. 37˚C, 5% $CO_2$, for ca. 20 hours, and ca. 100μl (ca. 100ul) physiological saline (0.138% NaCl) is added, and diluted plasma is collected using a Platemate or Biomek FX liquid handling robot after centrifugation at ca. 1300 g for ca. 10 min. Plasma TNFα content is determined by electrochemiluminescence assay using the MSD technology (see below), the IGEN technology (see below) or by enzyme linked immunosorbant assay (ELISA) (see bellow).

Results can be expressed as pIC50 values for inhibition of TNF-α (TNF-alpha) production in Human Whole Blood, and it should be appreciated that these results can be subject to variability or error.

***TNF-α (TNF-alpha) MSD Assay***

Using the Biomek FX, 25μl (25ul) of MSD Human Serum Cytokine Assay Diluent (Meso Scale Discovery, 9238 Gaither Road, Gaithersburg, Maryland 20877) is added to a 96-well High-Bind MSD plate pre-coated with anti-hTNF alpha capture antibody (MA6000) and then incubated for 24 hours at 4˚C to prevent non-specific binding. About 20μl (ul) of supernatant from the PBMC plate or about 40μl (ul) of supernatant from the whole blood (WB) plate are then transferred from columns 1-11 to columns 1-11 of the MSD plate using the Biomek FX. About 20 μl (ul) of TNF-α standard (Cat No. 210-TA; R&D Systems Inc., 614 McKinley Place NE, Minneapolis, MN 55413, USA) are added to column 12 of the MSD plate to generate a standard calibration curve (about 0 to 30000 pg/ml final).

For the Whole Blood assay, plates are washed after 2 hours shaking with a Skanwasher 300 version B (Skatron Instruments AS. PO Box 8, N-3401 Lier, Norway). About 40μl (ul) of diluted sulfo-TAG antibody (ca. 1 μg/ml final) is added, the plates are shaken at room temperature for 1 hours, and the plates washed again as above. About 150μl (ul) of Read Buffer T (2X) is added to the plates, which are then read on a MSD Sector 6000.

For the PBMC assay, about 20 μl (ul) of diluted sulfo-TAG antibody (ca. 1 μg/ml final) is added to each well, and the plates / wells are shaken at room temperature for 2 hours. Finally, about 90μl (ul) of MSD Read Buffer P (diluted to 2.5 times with distilled water) is added and the plates are read on a MSD Sector 6000.

*Data Analysis*

Data analysis is performed with ActivityBase/XC50 module (ID Business Solutions Ltd., 2 Occam Court, Surrey Research Park, Guildford, Surrey, GU2 7QB UK). Data are normalized and expressed as %inhibition using the formula 100*((U-C1)/(C2-C1)) where U is the unknown value, C1 is the average of the high signal (0%) control wells (column 10), and C2 is the average of the low signal (100%) control wells (column 11). Curve fitting is performed with the following equation: y = A+((B-A)/(1+(10^x/10^C)^D)), where A is the minimum response, B is the maximum response, C is the log10(IC50), and D is the Hill slope. The results for each compound are recorded as pIC50 values (-C in the above equation).

***TNF-α (TNF-alpha) IGEN Assay***

Ca. 50μl supernatant from either whole blood or PBMC assay plates is transferred to a 96 well polypropylene plate. Each plate also contains a TNF-α standard curve (ca. 0 to 30000 pg/ml: R+D Systems, 210-TA). Ca. 50μl (ul) of streptavidin/biotinylated anti-TNF-α antibody, mix, ca. 25μl ruthenium tagged anti-TNF-α monoclonal and ca. 100μl PBS containing 0.1 % bovine serum albumin are added to each well and the plates are sealed and shaken for ca. 2 hours before being read on an IGEN instrument.

***TNF-α (TNF-alpha) ELISA Assay (enzyme linked immunosorbant assay)***

Human TNF-α can be assayed using a commercial assay kit (AMS Biotechnology, 211-90-164-40) according to the manufacturers' instructions but with TNF-α calibration curves prepared using Pharmirigen TNF-α (cat No. 555212).

***In Vivo* Biological Assays**

The *in vitro* enzymatic PDE4B inhibition assay(s) described above or generally similar or generally analogous assays should be regarded as being the primary test(s) of biological activity. However, some additional *in vivo* biological tests, which are optional only, and which are not an essential measure of activity, efficacy or side-effects, and which have not necessarily been carried out, are described below.

***In Vivo Assay* A:**

***Activity of topically-applied compounds in a pig model* of *atopic dermatitis: Effect of compounds, applied by skin topical administration, on the dinitrofluorobenzene (DNFB)-induced delayed type hypersensitivity (DTH) response in pigs***

<u>*General Study Design:*</u>

The pig DTH (delayed type hypersensitivity) model of contact hypersensitivity utilizes the Th2-mediated inflammatory response in pig skin to mimic the pathology of atopic dermatitis in humans. The model measures the potential anti-inflammatory effect of compounds, topically-applied to the skin, on the acute DTH (delayed type hypersensitivity)

response in castrated male Yorkshire pigs.

In general in the assay, pigs (domestic Yorkshire pigs, 15-18 kg at time of sensitization, castrated males) are sensitized by topical application of ca. 10% (w/v) dinitrofluorobenzene (DNFB) dissolved in DMSO:acetone:olive oil (ca. 1:5:3) (ca. 40 mg DNFB, 400 microlitre solution total) to the ears (outer) and groin (inner) on day -12 and - 5. The pigs are then challenged with ca. 0.6% (w/v) DNFB applied to randomized sites on the shaved back of the pigs (ca. 90 micrograms/site; sites are identified and numbered by grid made with marking pen).

On the day of challenge, the treatments are performed at the challenge sites at about 2 hours prior to and about 6 hours after challenge (for DMSO / acetone solutions/suspensions containing the PDE4 inhibitor, to maximize exposure to drug), or at about 30 minutes after and about 6 hours after challenge (for topical ointments or creams containing the PDE4 inhibitor, representing a more clinically relevant treatment protocol).

After 24 hrs and 48 hrs post challenge, test sites are visually evaluated for intensity and extent of erythema by measuring the diameter of the reaction at its widest point and assigning scores of 0 to 4 for each of erythema intensity and erythema extent. Induration (a measure of swelling) is also scored 0 to 4. Scores for erythema intensity, erythema extent and induration are assigned according to the following criteria: *Intensity of Erythema:* 0=normal, 1=minimal, barely visible, 2=mild, 3=moderate, 4=severe. *Extent of Erythema* (not raised): 0=no edema, 1=macules of pin head size, 2=lentil sized macules, 3=confluent macules, 4=diffuse over entire site. *Induration* (palpable): 0=normal, 1 =nodules of pin head size, 2=doughy lentil sized nodules, 3=confluent firm nodules, 4=diffuse hard lesion. The summed visual score at ca. 24 and 48 hours includes the individual scores for erythema intensity, erythema extent, and induration; so the maximal summed score for each site would be 12. High summed scores can generally indicate a high inflammatory response. Visual scores are subject to some inaccuracy / error.

Differences in the summed score between adjacent control (placebo) and treatment sites on the grids are calculated (see experimental design for details). This difference value is then used to determine the percent inhibition compared to the summed score for the control (placebo) sites. The more negative the difference value, the greater the calculated inhibition. Percent inhibition of (percent inhibition compared to) the mean summed score can be calculated.

About 24 and 48 hours after challenge, treatment sites can optionally also be visually evaluated for lesion area.

*Experimental Design*

An 8 row by 3 column grid is outlined on the back of each pig. Due to potential heterogeneity in skin thickness, etc, across various regions of the back, the assignment of treatments and their controls were assigned to each row/ column in a systematic manner. Specifically, each row contained a treatment and its control (n=1 or 2 of each in the 3 columns). Row assignments were randomized across pigs to ensure that treatments were not always in the same region of the pig's backs. Column assignments were also randomized: the number of treatments per row (either 1 or 2) was randomly chosen, and treatment and control assignments were randomized across the 3 columns.

*Data Analysis*

Proper statistical analysis will incorporate features of the experimental design, which is generically known as a randomized block design (a block being a row on the grid). For each row, the average treatment and control response is calculated. The difference, d, between treatment and control is determined, and this is the response variable. The set of differences for each treatment and pig is analyzed using a 2 factor analysis of variance (ANOVA), with pig and treatment as factors. The average difference of each treatment group is tested against 0 using results from the two factor ANOVA; p-values $< 0.05$ are considered significant. (However, given the number of tests, a significant p-value cutoff of $<0.01$ might optionally (but not necessarily) be used to protect against possible false positive results.)

Statistical analysis which does not incorporate features of the randomized block design generally will not be as powerful (i.e. will tend to generate higher p-values). For example, pooling treatment and control data across all rows and columns from all pigs and conducting t-tests on treatment vs. control will be expected to have larger variance estimates because heterogeneity within and between pigs are part of the variance. In the 2 factor ANOVA on the differences, within and between effects are factored out of the variance resulting in more powerful tests. See *Statistics for Experimenters,* Box, Hunter and Hunter, 2nd edition, chapter 4 (Wiley and Sons, 2005).

Data are presented as Change (average difference in Matched pair scores) and p value (2 factor ANOVA via SAS software).

*Results: Pig DTH assay - Study #8*

A single specific ointment composition, being either Composition Example 1 or Composition Example 3 (Ointment D or D2) as disclosed hereinafter, and containing 2% w/w of the compound of the invention being Example 237, and white petrolatum and ST-Cyclomethicone 5-NF™, significantly ($p < 0.05$) inhibited inflammation scores at the 48

hour time point, but not at the 24 hour time point (placebo/control = Reference Composition Example 2 or 4 = Placebo Ointment AP or AP2, disclosed hereinafter). See the results tabulated below under the label "Modified White Petrolatum Ointment".

A single specific cream composition, being either Composition Example 5 or Composition Example 7 (Water-in-oil cream Cr-D or Cr-D2) as disclosed hereinafter, and containing *inter alia* propylene glycol and 2% w/w of the compound of the invention being Example 237, significantly ($p < 0.05$) inhibited inflammation scores at the 48 hour time point, but not at the 24 hour time point (placebo/control = Reference Composition Example 6 or 8 = Placebo Water-in-oil cream Cr-AP or Cr-AP2, disclosed hereinafter). See the results tabulated below under the label "Cream".

| 24 hr Modified White Petrolatum Ointment | | | | | 48 hr Modified White Petrolatum Ointment | | | |
|---|---|---|---|---|---|---|---|---|
| **Drug %** | **Change** | **study** | ***P value*** | | **Drug %** | **Change** | **study** | ***P value*** |
| **2** | **0.15** | **8** | ***0.766*** | | **2** | **-1.10** | **8** | ***0.031*** |
| **24 hr Cream** | | | | | **48 hr Cream** | | | |
| **Drug %** | **Change** | **study** | ***p value*** | | **Drug %** | **Change** | **study** | ***p value*** |
| **2** | **0.10** | **8** | ***0.843*** | | **2** | **-1.10** | **8** | ***0.031*** |

*Results: Pig DTH assay - Study #9*

A single specific ointment composition, being either Composition Example 3 or Composition Example 1 (Ointment D2 or D) as disclosed hereinafter, and containing 2% w/w of the compound of the invention being Example 237, and white petrolatum and ST-Cyclomethicone 5-NF ™, significantly ($p < 0.05$) inhibited inflammation scores at the 24 and 48 hour time point (placebo/control = Reference Composition Example 4 or 2 = Placebo Ointment AP2 or AP, disclosed hereinafter). See the results tabulated below under the label "Modified White Petrolatum Ointment".

A single specific cream composition, being either Composition Example 7 or Composition Example 5 (Water-in-oil cream Cr-D2 or Cr-D) as disclosed hereinafter, and containing *inter alia* propylene glycol and 2% w/w of the compound of the invention being Example 237, appeared to be somewhat inhibitory (negative Change observed), but the differences (with $p \geq 0.05$) did not reach statistical significance (placebo/control = Reference Composition Example 8 or 6 = Placebo Water-in-oil cream Cr-AP2 or Cr-AP, disclosed hereinafter). See the results tabulated below under the label "Cream".

| 24 hr Modified White Petrolatum Ointment | | | | | 48 hr Modified White Petrolatum Ointment | | | |
|---|---|---|---|---|---|---|---|---|
| **Drug %** | **Change** | **study** | ***p value*** | | **Drug %** | **Change** | **study** | **p *value*** |
| **2** | **-1.29** | **9** | ***0.028*** | | **2** | **-1.92** | **9** | ***about 0.000*** |
| **24 hr Cream** | | | | | **48 hr Cream** | | | |
| **Drug %** | **Change** | **study** | ***p value*** | | **Drug %** | **Change** | **study** | ***p value*** |
| **2** | **-1.00** | **9** | ***0.086*** | | **2** | **-0.65** | **9** | ***0.156*** |

Compounds or pharmaceutical compositions showing apparent activity in the above pig DTH model (In Vivo Assay A) may have potential utility via skin topical administration in the treatment and/or prophylaxis of atopic dermatitis, e.g. in humans. The preliminary nature of In Vivo Assay A, and the potential for error or for variation in results when the assay is repeated (see e.g. results of Study #8 of. those of Study #9) is noted.

***In Vivo Assay* 1. *LPS-induced pulmonary neutrophilia in rats: effect of orally administered PDE4 inhibitors***

Pulmonary neutrophil influx is thought to be a significant component to the family of pulmonary diseases like chronic obstructive pulmonary disease (COPD) which can involve chronic bronchitis and/or emphysema (G.F. Filley, *Chest.* 2000; 117(5); 251s-260s). The purpose of this neutrophilia model is to study the potentially anti-inflammatory effects *in vivo* of orally administered PDE4 inhibitors on neutrophilia induced by inhalation of aerosolized lipopolysaccharide (LPS), modelling the neutrophil inflammatory component(s) of COPD. See the literature section below for scientific background.

Male Lewis rats (Charles River, Raleigh, NC, USA) weighing approximately 300-400 grams are pretreated with

either (a) test compound, for example suspended in about 0.5% methylcellulose (obtainable from Sigma-Aldrich, St Louis, MO, USA) in water or (b) vehicle only, delivered orally in a dose volume of ca. 10 ml/kg. Generally, dose response curves can for example be generated using the following approx. doses of PDE4 inhibitors: 2.0, 0.4, 0.08, 0.016 and 0.0032 mg/kg (or alternatively approx. 10, 2.0, 0.4, 0.08 and 0.016 mg/kg). About thirty minutes following pretreatment, the rats are exposed to aerosolized LPS (Serotype E. Coli 026:B6 prepared by trichloroacetic acid extraction, obtainable from Sigma-Aldrich, St Louis, MO, USA), generated from a nebulizer containing a ca. 100 μg/ml LPS solution (ca. 100 ug/ml). Rats are exposed to the LPS aerosol at a rate of ca. 4 L/min for ca. 20 minutes. LPS exposure is carried out in a closed chamber with internal dimensions of roughly 45 cm length x 24 cm width x 20 cm height. The nebulizer and exposure chamber are contained in a certified fume hood. At about 4 hours-post LPS exposure the rats are euthanized by overdose with pentobarbital at ca. 90 mg/kg, administered intraperitoneally. Bronchoalveolar lavage (BAL) is performed through a 14 gauge blunt needle into the exposed trachea. Five, 5 ml washes are performed to collect a total of 25 ml of BAL fluid. Total cell counts and leukocyte differentials are performed on BAL fluid in order to calculate neutrophil influx into the lung. Percent neutrophil inhibition at each dose (cf. vehicle) is calculated and a variable slope, sigmoidal dose-response curve is generated, usually using Prism Graph-Pad. The dose-response curve is used to calculate an ED50 value (in mg per kg of body weight) for inhibition by the PDE4 inhibitor of the LPS-induced neutrophilia.

*Alternative method:* In an alternative simpler embodiment of the procedure, a singe oral dose of 10 mg/kg, or more usually 1.0 mg/kg or 0.3 mg/kg, of the PDE4 inhibitor (or vehicle) is administered to the rats, and percent neutrophil inhibition is calculated and reported for that specific dose.

*Literature:*

Filley G.F. Comparison of the structural and inflammatory features of COPD and asthma. *Chest.* 2000; 117(5) 251s-260s.

Howell RE, Jenkins LP, Fielding LE, and Grimes D. Inhibition of antigen-induced pulmonary eosinophilia and neutrophilia by selective inhibitors of phosphodiesterase types 3 and 4 in brown Norway rats. Pulmonary Pharmacology. 1995; 8: 83-89.

Spond J, Chapman R, Fine J, Jones H, Kreutner W, Kung TT, Minnicozzi M. Comparison of PDE 4 inhibitors, Rolipram and SB 207499 (Ariflo™), in a rat model of pulmonary neutrophilia. Pulmonary Pharmacology and Therapeutics. 2001; 14: 157-164.

Underwood DC, Osbom RR, Bochnowicz S, Webb EF, Rieman DJ, Lee JC, Romanic AM, Adams JL, Hay DWP, and Griswold DE. SB 239063, a p38 MAPK inhibitor, reduces neutrophilia, inflammatory cytokines, MMP-9, and fibrosis in lung. Am J Physiol Lung Cell Mol Physiol. 2000; 279: L895-L902.


***In Vivo* Assay 2. *Rat Pica Model of emesis***

*Background:* Selective PDE4 inhibitors are thought to inhibit inflammation in various *in vitro* and *in vivo* models by increasing intracellular levels of cAMP of many immune cells (e.g. lymphocytes, monocytes). However, a side effect of some PDE4 inhibitors in some species is emesis. Because many rat models of inflammation are well characterized, they can be used in procedures (see e.g. In Vivo Assay 1 above) to show beneficial anti-inflammatory effects of PDE 4 inhibitors. However rats have no emetic response (they have no vomit reflex), so that the relationship between beneficial anti-inflammatory effects of PDE 4 inhibitors and emesis is difficult to study directly in rats.

However, in 1991, Takeda *et al.* (see Literature section below) demonstrated that the pica feeding response is analogous to emesis in rats. Pica feeding is a behavioural response to illness in rats wherein rats eat non-nutritive substances such as earth or in particular clay (e.g. kaolin) which may help to absorb toxins. Pica feeding can be induced by motion and chemicals (especially chemicals which are emetic in humans), and can be inhibited pharmacologically with drugs that inhibit emesis in humans. The Rat Pica Model, In Vivo Assay 2, can determine the level of pica response of rats to PDE 4 inhibition at pharmacologically relevant doses in parallel to in vivo anti-inflammatory Assays in (a separate set of) rats (e.g. In Vivo Assay 1 above).

Anti-inflammatory and pica assays in the same species together can provide data on the "therapeutic index" (TI) in the rat of the compounds/salts of the invention. The Rat TI can for example be calculated as the ratio of a) the potentially-emetic Pica Response ED50 dose from Assay 2 to b) the rat anti-inflammatory ED50 dose (e.g. measured by rat neutrophilia-inhibition in eg In Vivo Assay 1), with larger T1 ratios possibly indicating lower emesis at many anti-inflammatory doses. This might allow a choice of a non-emetic or low-emetic pharmaceutical dose of the compounds or salts of the invention which has an anti-inflammatory effect. It is recognised however that achieving a low-emetic PDE4 inhibitory compound is not essential to the invention.

*Procedure:* On the first day of the experiment, the rats are housed individually in cages without bedding or

"enrichment". The rats are kept off of the cage floor by a wire screen. Pre-weighed food cups containing standard rat chow and clay pellets are placed in the cage. The clay pellets, obtainable from Languna Clay Co, City of Industry, CA, USA, are the same size and shape as the food pellets. The rats are acclimated to the clay for 72 hours, during which time the cups and food and clay debris from the cage are weighed daily on an electronic balance capable of measuring to the nearest 0.1 grams. By the end of the 72 hour acclimation period the rats generally show no interest in the clay pellets.

At the end of 72 hours the rats are placed in clean cages and the food cups weighed. Rats that are still consuming clay regularly are removed from the study. Immediately prior to the dark cycle (the time when the animals are active and should be eating) the animals are split into treatment groups and dosed orally with a dose of the compound or salt of the invention (different doses for different treatment groups) or with vehicle alone, at a dose volume of ca. 2 ml/kg: In this oral dosing, the compound or salt can for example be in the form of a suspension in about 0.5% methylcellulose (obtainable Sigma-Aldrich, St. Louis, MO, USA) in water. The food and clay cups and cage debris are weighed the following clay and the total clay and food consumed that night by each individual animal is calculated.

A dose response is calculated by first converting the data into quantal response, where animals are either positive or negative for the pica response. A rat is "pica positive" if it consumes greater than or equal to 0.3 grams of clay over the mean of its control group. The D50 value is usually calculated using logistic regression performed by the Statistica software statistical package. A Pica Response ED50 value in mg per kg of body weight can then be calculated.

The Pica Response ED50 value can be compared to the neutrophilia-inhibition ED50 values for the same compound administered orally to the rat (measurable by In Vivo Assay 1 above), so that a Therapeutic Index (TI) in rats can be calculated thus:

$$\text{Rat Therapeutic index (TI) (50/50)} = \frac{\text{Pica Response ED50 value}}{\text{rat neutrophilia-inhibition ED50 value}}$$

In general, the Therapeutic Index (TI) calculated this way can be substantially different to, and for example (without being bound) can be substantially higher than, the TI (D20/D50) calculated in the ferret (see In vivo Assay 4 below).

Alternatively, e.g. for a simpler test, the In Vivo Assay 2 (pica) can use only a single oral dose of the test compound (e.g. 10 mg/kg orally).

*Literature:*

Beavo JA, Contini, M., Heaslip, R.J. Multiple cyclic nucleotide phosphodiesterases. Mol Pharmacol. 1994; 46: 399-405.

Spond J, Chapman R, Fine J, Jones H, Kreutner W, Kung TT, Minnicozzi M. Comparison of PDE 4 inhibitors, Rolipram and SB 207499 (Ariflo™), in a rat model of pulmonary neutrophilia. Pulmonary Pharmacology and Therapeudtics. 2001; 14:157-164.

Takeda N, Hasegawa S, Morita M, and Matsunaga T. Pica in rats is analogous to emesis: an animal model in emesis research. Pharmacology, Biochemistry and Behavior. 1991; 45:817-821.

Takeda N, Hasegawa S, Morita M, Horii A, Uno A, Yamatodani A and Matsunaga T. Neuropharmacological mechanisms of emesis. I . Effects of antiemetic drugs on motion- and apomorphine-induced pica in rats. Meth Find Exp Clin Pharmacol. 1995; 17(9) 589-596.

Takeda N, Hasegawa S, Morita M, Horii A, Uno A, Yamatodani A and Matsunaga T. Neuropharmacological mechanisms of emesis. II. Effects of antiemetic drugs on cisplatin-induced pica in rats. Meth Find Exp Clin Pharmacol. 1995; 17(9) 647-652.

*In Vivo Assay* 3. *LPS induced pulmonary neutrophilia in rats: effect* of *intratracheally administered PDE4 inhibitors*

This assay is an animal model of inflammation in the lung - specifically neutrophilia induced by lipopolysaccharide (LPS) - and allows the study of putative inhibition of such neutrophilia (anti-inflammatory effect) by intratracheally (i.t.) administered PDE4 inhibitors. The PDE4 inhibitors are preferably in dry powder or wet suspension form. I.t. administration is one model of inhaled administration, allowing topical delivery to the lung.

*Animals:* Male CD (Sprague Dawley Derived) rats supplied by Charles River, Raleigh, NC, USA or Charles River, United Kingdom are housed in groups of 5 rats per cage, acclimatised after delivery for at least 5 days with bedding/nesting material regularly changed, fed on SDS diet R1 pelleted food given ad lib, and supplied with daily-changed pasteurised animal grade drinking water.

*Device for dry powder administration:* Disposable 3-way tap between dosing needle and syringe. The intratracheal dosing device (a 3-way sterile tap, Vycon 876.00; or Penn Century dry powder insufflator, DP-4) is weighed, the drug blend or inhalation grade lactose (vehicle control) is then added to the tap, the tap is closed to prevent loss of drug, and the tap is re-weighed to determine the weight of drug in the tap. After dosing, the tap is weighed again to determine the weight of drug that had left the tap. The needle, a Sigma Z21934-7 syringe needle 19-gauge 152 mm (6 inches) long with luer hub, is cut by engineering to approximately 132 mm (5.2 inches), a blunt end is made to prevent them damaging the rat's trachea, and the needle is weighed prior to and after drug delivery to confirm that no drug is retained in the needles after dosing.

*Device for wet suspension administration:* This is similar to the above but a blunt dosing needle, whose forward end is slightly angled to the needle axis, is used, with a flexible plastic portex canula inserted into the needle.

*Drugs and Materials:* Lipopolysaccharide (LPS) (Serotype:0127:B8) is dissolved in phosphate-buffered saline (PBS). PDE4 inhibitors are preferably used in size-reduced (e.g. micronised) form, for example according to the Micronisation Example(s) given herein.

For dry powder administration of the drug, the Dry Powder Formulation Example given herein, comprising drug and inhalation-grade lactose, can optionally be used. One suitable inhalation-grade lactose that can be used has 10% fines (10% of material under 15um (15 micron) particle size measured by Malvem particle size).

Wet suspensions of the drug (aqueous) can be prepared by adding the required volume of vehicle to the drug; the vehicle used can for example be saline alone or a mixture of saline/tween (e.g. 0.2% tween 80). The wet suspension is usually sonicated for ca. 10 minutes prior to use.

*Preparation, and dosing with PDE 4 inhibitor:* Rats are anaesthetised by placing the animals in a sealed Perspex chamber and exposing them to a gaseous mixture of isoflourane (4.5 %), nitrous oxide (3 litres.minute$^{-1}$) and oxygen (1 litre.minute$^{-1}$). Once anaesthetised, the animals are placed onto a stainless steel i.t. dosing support table. They are positioned on their back at approximately a 35° angle. A light is angled against the outside of the throat to highlight the trachea. The mouth is opened and the opening of the upper airway visualised. The procedure varies for wet suspension and dry powder administration of PDE4 inhibitors as follows:

*Dosing with a Wet suspension:* A portex cannula is introduced via a blunt metal dosing needle that has been carefully inserted into the rat trachea. The animals are intratracheally dosed with vehicle or PDE4 inhibitor via the dosing needle with a new internal canula used for each different drug group. The formulation is slowly (ca. 10 seconds) dosed into the trachea using a syringe attached to the dosing needle.

*Dosing with a Dry Powder:* The intratracheal dosing device (a three-way sterile tap device, Vycon 876.00; or Penn Century dry powder insufflator, DP-4) and needle are inserted into the rat trachea up to a pre-determined point established to be located approximately 1 cm above the primary bifurcation. Another operator holds the needle at the specified position whilst 2 x 4ml of air (using 3-way tap device) is delivered through the three-way tap by depressing the syringes (ideally coinciding with the animal inspiring), aiming to expel the entire drug quantity from the tap. (Alternatively, 2 x 3ml of air is delevered using Penn Century dry powder insufflator device.) After dosing, the needle and tap or device are removed from the airway, and the tap closed off to prevent any retained drug leaving the tap.

After dosing with either wet suspension or dry powder, the animals are then removed from the table and observed constantly until they have recovered from the effects of anaesthesia. The animals are returned to the holding cages and given free access to food and water; they are observed and any unusual behavioural changes noted.

*Exposure* to *LPS:* About 2 hours after i.t. dosing with vehicle control or the PDE4 inhibitor, the rats are placed into sealed Perspex containers and exposed to an aerosol of LPS (nebuliser concentration ca. 150 $\mu$g.ml$^{-1}$ = ca. 150 ug/ml) for ca. 15 minutes. Aerosols of LPS are generated by a nebuliser (DeVilbiss, USA) and this is directed into the Perspex exposure chamber. Following the 15-minute LPS-exposure period, the animals are returned to the holding cages and allowed free access to both food and water.

[In an alternative embodiment, the rats can be exposed to LPS less than 2 hours (e.g. about 30 minutes) after i.t. dosing. In another alternative embodiment, the rats can be exposed to LPS more than 2 hours (e.g. ca. 4 to ca. 24 hours) after i.t. dosing by vehicle or PDE4 inhibitor, to test whether or not the PDE4 inhibitor has a long duration of action (which is not essential).]

*Bronchoalveolar lavage:* About 4 hours after LPS exposure the animals are killed by overdose of sodium pentobarbitone (i.p.). The trachea is cannulated with polypropylene tubing and the lungs are lavaged (washed out) with 3 x 5 mls of heparinised (25 units.ml$^{-1}$) phosphate buffered saline (PBS).

*Neutrophil cell counts:* The Bronchoalveolar lavage (BAL) samples are centrifuged at ca. 1300 rpm for ca. 7 minutes. The supernatant is removed and the resulting cell pellet resuspended in ca. 1 ml PBS. A cell slide of the resuspension fluid is prepared by placing ca. 100$\mu$l (ca. 100ul) of resuspended BAL fluid into cytospin holders and then is spun at ca. 5000 rpm for ca. 5 minutes. The slides are allowed to air dry and then stained with Leishmans stain (ca. 20 minutes) to allow differential cell counting. The total cells are also counted from the resuspension. From these two counts, the total numbers of neutrophils in the BAL are determined. For a measure of PDE4-inhibitor-induced inhibition

of neutrophilia, a comparison of the neutrophil count in rats treated with vehicle and rats treated with PDE4 inhibitors is conducted.

By varying the dose of the PDE4 inhibitor used in the dosing step (e.g. 0.2 or 0.1 mg of PDE4 inhibitor per kg of body-weight, down to e.g. 0.01 mg/kg), a dose-response curve can be generated.

***In Vivo Assay* 4. *Evaluation of Therapeutic Index of Orally-administered PDE 4 inhibitors in the conscious ferret***

*1.1 Materials*

The following materials can be used for these studies:

PDE4 inhibitors are prepared for oral (p.o.) administration by dissolving in a fixed volume (ca. 1 ml) of acetone and then adding cremophor to ca. 20% of the final volume.
Acetone is evaporated by directing a flow of nitrogen gas onto the solution. Once the acetone is removed, the solution is made up to final volume with distilled water.
LPS is dissolved in phosphate buffered saline.

*1.2 Animals*

Male ferrets (Mustela Pulorius Furo, weighing 1 - 2 kg) are transported and allowed to acclimatise for not less than 7 days. The diet comprises SDS diet C pelleted food given *ad lib* with Whiskers™ cat food given 3 times per week. The animals are supplied with pasteurised animal grade drinking water changed daily.

*1.3 Experimental Protocol(s)*

*1.3.1 Dosing with PDE4 inhibitors*

PDE4 inhibitors are administered orally (p.o.), using a dose volume of ca. 1ml/kg. Ferrets are fasted overnight but allowed free access to water. The animals are orally dosed with vehicle or PDE 4 inhibitor using a ca. 15cm dosing needle that is passed down the back of the throat into the oesophagus. After dosing, the animals are returned to holding cages fitted with perspex doors to allow observation, and given free access to water. The animals are constantly observed and any emetic episodes (retching and vomiting) or behavioural changes are recorded. The animals are allowed access to food ca. 60 - 90 minutes after p.o. dosing.

*1.3.2 Exposure to LPS*

About thirty minutes after oral dosing with compound or vehicle control, the ferrets are placed into sealed perspex containers and exposed to an aerosol of LPS (ca. 30 μg/ml = ca. 30 ug/ml) for ca. 10 minutes. Aerosols of LPS are generated by a nebuliser (DeVilbiss, USA) and this is directed into the perspex exposure chamber. Following a 10-minute exposure period, the animals are returned to the holding cages and allowed free access to water, and at a later stage, food. General observation of the animals continues for a period of at least 2.5 hours post oral dosing. All emetic episodes and behavioural changes are recorded.

*1.3.3 Bronchoalveolar lavage and cell counts*

About six hours after LPS exposure the animals are killed by overdose of sodium pentobarbitone administered intraperitoneally. The trachea is then cannulated with polypropylene tubing and the lungs lavaged twice with ca. 20 ml heparinised (10 units/ml) phosphate buffered saline (PBS). The bronchoalveolar lavage (BAL) samples are centrifuged at ca. 1300 rpm for ca. 7 minutes. The supernatant is removed and the resulting cell pellet re-suspended in ca. 1 ml PBS. A cell smear of re-suspended fluid is prepared and stained with Leishmans stain to allow differential cell counting. A total cell count is made using the remaining re-suspended sample. From this, the total number of neutrophils in the BAL sample is determined.

*1.3.4 Pharmacodynamic readouts*

The following parameters are recorded:

a) % inhibition of LPS-induced pulmonary neutrophilia to determine the dose of PDE4 inhibitor which gives 50%

inhibition (D50).

b) Emetic episodes - the number of vomits and retches are counted to determine the dose of PDE4 inhibitor that gives a 20% incidence of emesis (D20).

c) A therapeutic index (TI), using this assay, is then calculated for each PDE4 inhibitor using the following equation:

$$\text{Ferret Therapeutic index (TI) (D20/D50)} = \frac{\text{D20 incidence of emesis in ferret}}{\text{D50 inhibition of neutrophilia in ferret}}$$

It is noted that the Ferret Therapeutic index (TI) (D20/D50) calculated using this in vivo Assay 4 can be substantially different to, and for example (without being bound) can be substantially lower than, the Rat TI (50/50) calculated using the rat oral inflammation and pica feeding Assays 1+2.

All publications, including but not limited to patents and patent applications, cited in this specification are herein incorporated by reference as if each individual publication were specifically and individually indicated to be incorporated by reference herein as though fully set forth.

**EXAMPLES**

The various aspects of the invention will now be described by reference to the following examples. These examples are merely illustrative and are not to be construed as a limitation of the scope of the present invention.

In this section, "Intermediates" can represent syntheses of intermediate compounds intended for use in the synthesis of one or more of the "Examples", and/or "Intermediates" can represent syntheses of intermediate compounds which can be used in the synthesis of compounds of formula (I) or salts thereof. "Examples" are generally exemplary compounds or salts of the invention, for example compounds of formula (I) or salts thereof.

**Abbreviations** used herein:

| | |
|---|---|
| AcOH | acetic acid |
| $Ac_2O$ | acetic anhydride |
| BEMP | 2-t-butylimino-2-diethylamino-1,3-dimethylperhydro-1,3,2- diazaphosphazine |
| $BOC_2O$ | di tert-butyl carbonate |
| DMSO | dimethyl sulfoxide |
| DCM | dichloromethane |
| DMF | dimethyl formamide |
| DIPEA | diisopropylethyl amine ($^iPr_2NEt$) |
| EDC | 1-(3-Dimethylaminopropyl)-3-ethylcarbodiimide hydrochloride |
| EtOAc | ethyl acetate |
| $Et_2O$ | diethyl ether |
| $Et_3N$ | triethylamine |
| EtOH | ethanol |
| HATU | O-(7-Azabenzotriazol-1-yl)-*N,N,N',N'*-tetramethyluronium hexafluorophosphate |
| HBTU | O-(Benzotriazol-1-yl)-*N,N,N',N*-tetramethyluronium hexafluorophosphate |
| HOBT | hydroxybenzotriazole = 1-hydroxybenzotriazole |
| Lawesson's reagent | 2,4-bis(4-methoxyphenyl)-1,3-dithia-2,4-diphosphetane-2,4- disulphide |
| MeCN | acetonitrile |
| MeOH | methanol |
| PyBOP | Benzotriazole-1-yl-oxy-trispyrrolidinophosphonium hexafluorophosphate |
| THF | Tetrahydrofuran |
| TFA | Trifluoroacetic acid |
| HPLC | high performance liquid chromatography |
| h | hours |
| min | minutes |
| LCMS | liquid chromatography / mass spectroscopy |
| NMR | nuclear magnetic resonance (in which: s = singlet, d = doublet, t = triplet, |

| | |
|---|---|
| | q = quartet, dd = doublet of doublets, m = multiplet, H = no. of protons) |
| SPE | solid phase extraction |
| TLC | thin layer chromatography |
| $T_{RET}$ | retention time (e.g. from LCMS) |
| $T_{int}$ | internal temperature of the reaction mixture |
| Room temperature (ambient temperature): | this is usually in the range of about 18 to about 25 ˚C. |

## General Experimental Details

**Machine Methods used herein:**

***LCMS (liquid chromatography/mass spectroscopy)***

Waters ZQ mass spectrometer operating in positive Ion electrospray mode, mass range 100-1000 amu.
UV wavelength: 215-330 nm
Column : 3.3cm x 4.6mm ID (internal diameter), 3μm (3 micrometres) ABZ+PLUS
Flow Rate : 3ml/min
Injection Volume : 5μl (5 microlitres)
Solvent A : 0.05% v/v solution of formic acid in a mixture of [95% acetonitrile and 5% water]
Solvent B : aqueous solution of [0.1% v/v formic acid + 10mMolar ammonium acetate] Gradient: Mixtures of Solvent A and Solvent B are used according to the following gradient profiles (expressed as % Solvent A in the mixture): 0% A/0.7min, 0-100% A/3.5min, 100% A/1.1min, 100-0% A/0.2min

It should be noted that retention times ($T_{RET}$) quoted herein are inherently variable (e.g. the variability can be about +/- 0.2 min or more.). Variability can arise e.g. when samples are run on different Waters machines, or on the same Waters machine at different times of day or under slightly different conditions, even when the same type of column and identical flow rates, injection volumes, solvents and gradients are used.

***Mass directed autoprep HPLC***

The preparative HPLC column generally used is a Supelcosil ABZplus (10cm x 2.12cm internal diameter; particle size 5μm = 5 micrometres) e.g. for eluent containing formic acid, or is a C18 column (10cm x 2.12cm internal diameter; particle size 5μm = 5 micrometres) e.g. for eluent containing trifluoroacetic acid. A mass spectrometer attached to the end of the column can detect peaks arising from eluted compounds.

UV wavelength : usually 200-320 nm
Flow : 20ml/min
Injection Volume: 0.5 to 1ml
Gradient systems: mixtures of Solvent A and Solvent B are used according to a choice of 5 generic gradient profiles (expressed as % Solvent B in the mixture), ranging from a start of 0 to 50% Solvent B, with all finishing at 100% Solvent B to ensure total elution. Generally, two alternative solvent systems have been used, Method 1 and Method 2:

*Method 1*

Solvent A : 0.1 % v/v aqueous formic acid solution
Solvent B : 0.05% v/v solution of formic acid in a mixture of [95% acetonitrile and 5% water]

It is thought that compounds isolated by this method can sometimes be isolated as formate salts.

*Method 2*

Solvent A: 0.1% v/v aqueous trifluoroacetic acid solution

Solvent B: solution of 0.1% v/v trifluoroacetic acid in acetonitrile

It is thought that compounds isolated by this method can sometimes be isolated as trifluoroacetate salts.

## Intermediates and Examples

Reagents not detailed in the text below are usually commercially available from chemicals suppliers, e.g. established suppliers such as Sigma-Aldrich. The addresses and/or contact details of the suppliers for some of the starting materials mentioned in the Intermediates and Examples below or the Assays above, or suppliers of chemicals in general, are as follows:

- AB Chem, Inc., 547 Davignon, Dollard-des-Ormeaux, Quebec, H9B 1Y4, Canada
- ABCR GmbH & CO. KG, P.O. Box 21 01 35, 76151 Karlsruhe, Germany
- Albemarle Corporation, 451 Florida Street, Baton Rouge, LA 70801, USA; or Albemarle Europe SPRL, Parc Scientific de LLN, Rue du Bosquet 9, B-1348 Louvain-la-Neuve, Belgium
- ACB Blocks Ltd; Kolokolnikov Per, 9/10 Building 2, Moscow, 103045, Russia
- Aceto Color Intermediates (catalogue name), Aceto Corporation, One Hollow Lane, Lake Success, NY, 11042-1215, USA
- Acros Organics, A Division of Fisher Scientific Company, 500 American Road, Morris Plains, NJ 07950, USA
- Apin Chemicals Ltd., 82 C Milton Park, Abingdon, Oxon OX14 4RY, United Kingdom
- Apollo Scientific Ltd., Unit 1A, Bingswood Industrial Estate, Whaley Bridge, Derbyshire SK23 7LY, United Kingdom
- Aldrich (catalogue name), Sigma-Aldrich Company Ltd., Dorset, United Kingdom, telephone: +44 1202 733114; Fax: +44 1202 715460; ukcustsv@eurnotes.sial.com; or
- Aldrich (catalogue name), Sigma-Aldrich Corp., P.O. Box 14508, St. Louis, MO 63178-9916, USA; telephone: +1-314-771-5765; fax: +1-314-771-5757; custserv@sial.com; or
- Aldrich (catalogue name), Sigma-Aldrich Chemie GmbH, Munich, Germany; telephone: +49 89 6513 0; Fax: +49 89 6513 1169; deorders@eurnotes.sial.com.
- Alfa Aesar, A Johnson Matthey Company, 30 Bond Street, Ward Hill, MA 01835-8099, USA
- Amersham Biosciences UK Ltd, Pollards Wood, Chalfont St Giles, Buckinghamshire HP8 4SP, United Kingdom
- Arch Corporation, 100 Jersey Avenue, Building D, New Brunswick, NJ08901, USA
- Array Biopharma Inc., 1885 33rd Street, Boulder, CO 80301, USA
- Art-Chem GmbH: (a) Lininsky prospect 47, Moscow 119991, Russia, or (b) Campus Berlin-Buch, Haus B55, Robert-Roessle Strasse 10, 13125 Berlin, Germany
- AstaTech, Inc., 8301 Torresdale Ave., 19C, Philadelphia, PA 19136, USA
- Austin Chemical Company, Inc., 1565 Barclay Blvd., Buffalo Grove, IL 60089, USA
- Avocado Research, Shore Road, Port of Heysham Industrial Park, Heysham, Lancashire LA3 2XY, United Kingdom
- Bayer AG, Business Group Basic and Fine Chemicals, D-51368 Leverkusen, Germany
- Berk Univar plc, Berk House, P.O.Box 56, Basing View, Basingstoke, Hants RG21 2E6, United Kingdom
- Bionet Research Ltd; Highfield Industrial Estate, Camelford, Cornwall PL32 9QZ UK
- Butt Park Ltd., Braysdown Works, Peasedown St. John, Bath BA2 8LL, United Kingdom
- Chemical Building Blocks (catalogue name), Ambinter, 46 quai Louis Bleriot, Paris, F-75016, France
- ChemBridge Europe, 4 Clark's Hill Rise, Hampton Wood, Evesham, Worcestershire WR11 6FW, United Kingdom
- ChemService Inc., P.O.Box 3108, West Chester, PA 19381, USA
- CiventiChem, PO Box 12041, Research Triangle Park, NC 27709, USA
- Combi-Blocks Inc., 7949 Silverton Avenue, Suite 915, San Diego, CA 92126, USA
- Dynamit Nobel GmbH, Germany; also available from: Seville Whittle Ltd (UK agents of Dynamit Nobel), Vickers Street, Manchester M40 8EF, United Kingdom
- E. Merck, Germany; or E. Merck (Merck Ltd), Hunter Boulevard, Magna Park, Lutterworth, Leicestershire LE17 4XN, United Kingdom
- Enamine, 23 A. Motrosova Street, Kiev 01103, Ukraine
- Esprit Chemical Company, Esprit Plaza, 7680 Matoaka Road, Sarasota, FL 34243, USA
- Exploratory Library (catalogue name), Ambinter, 46 quai Louis Bleriot, Paris, F-75016, France
- Fluka Chemie AG, Industriestrasse 25, P.O. Box 260, CH-9471 Buchs, Switzerland
- Fluorochem Ltd., Wesley Street, Old Glossop, Derbyshire SK13 7RY, United Kingdom
- Heterocyclic Compounds Catalog (Florida Center for Heterocyclic Compounds, University of Florida, PO Box 117200, Gainsville, FL 32611-7200 USA
- ICN Biomedicals, Inc., 3300 Hyland Avenue, Costa Mesa, CA 92626, USA
- Interchim Intermediates (catalogue name), Interchim, 213 Avenue Kennedy, BP 1140, Montlucon, Cedex, 03103,

France

- Key Organics Ltd., 3, Highfield Indusrial Estate, Camelford, Cornwall PL32 9QZ, United Kingdom
- Lancaster Synthesis Ltd., Newgate, White Lund, Morecambe, Lancashire LA3 3DY, United Kingdom
- Manchester Organics Ltd., Unit 2, Ashville Industrial Estate, Sutton Weaver, Runcorn, Cheshire WA7 3PF, United Kingdom
- Matrix Scientific, P.O. Box 25067, Columbia, SC 29224-5067, USA
- Maybridge Chemical Company Ltd., Trevillett, Tintagel, Cornwall PL34 OHW, United Kingdom
- Maybridge Combichem (catalogue name), Maybridge Chemical Company Ltd., Trevillett, Tintagel, Cornwall PL34 OHW, United Kingdom
- Maybridge Reactive Intermediates (catalogue name), Maybridge Chemical Company Ltd., Trevillett, Tintagel, Cornwall PL34 OHW, United Kingdom
- MicroChemistry Building Blocks (catalogue name), MicroChemistry-RadaPharma, Shosse Entusiastov 56, Moscow, 111123, Russia
- Miteni S.p.A., Via Mecenate 90, Milano, 20138, Italy
- Molecular Devices Corporation, Sunnydale, CA, USA
- N.D. Zelinsky Institute, Organic Chemistry, Leninsky prospect 47, 117913 Moscow B-334, Russia
- Oakwood Products Inc., 1741, Old Dunbar Road, West Columbia, SC, 29172, USA
- OmegaChem Inc., 8800, Boulevard de la Rive Sud, Levis, PQ, G6V 9H1, Canada
- Optimer Building Block (catalogue name), Array BioPharma, 3200 Walnut Street, Boulder, CO 80301, USA
- Peakdale Molecular Ltd., Peakdale Science Park, Sheffield Road, Chapel-en-le-Frith, High Peak SK23 OPG, United Kingdom
- Pfaltz & Bauer, Inc., 172 East Aurora Street, Waterbury, CT 06708, USA
- Rare Chemicals (catalogue name), Rare Chemicals GmbH, Schulstrasse 6, 24214 Gettorf, Germany
- SALOR (catalogue name) (Sigma Aldrich Library of Rare Chemicals), Aldrich Chemical Company Inc, 1001 West Saint Paul Avenue, Milwaukee, WI 53233, USA
- Sigma (catalogue name), Sigma-Aldrich Corp., P.O. Box 14508, St. Louis, MO 63178-9916, USA; see "Aldrich" above for other non-US addresses and other contact details
- SIGMA-RBI, One Strathmore Road, Natick, MA 01760-1312, USA
- Synchem OHG Heinrich-Plett-Strasse 40, Kassel, D-34132, Germany
- Syngene International Pvt Ltd, Hebbagodi, Hosur Road, Bangalore, India.
- TCI America, 9211 North Harborgate Street, Portland, OR 97203, USA
- TimTec Corporation (e.g. "TimTec Overseas Stock"), 100 Interchange Boulevard, Newark, DE 19711, USA
- "TimTec Building Blocks A or B", or "TimTec Stock Library", TimTec, Inc., P O Box 8941, Newark, DE 19714-8941, USA
- Trans World Chemicals, Inc., 14674 Southlawn Lane, Rockville, MD 20850, USA
- Ubichem PLC, Mayflower Close, Chandlers Ford Industrial Estate, Eastleigh, Hampshire S053 4AR, United Kingdom
- Ultrafine (UFC Ltd.), Synergy House, Guildhall Close, Manchester Science Park, Manchester M15 6SY, United Kingdom

## INTERMEDIATES

### Intermediate 1 Ethyl 4-chloro-1,6-diethyl-1*H*-pyrazolo[3,4-*b*]pyridine-5-carboxylate

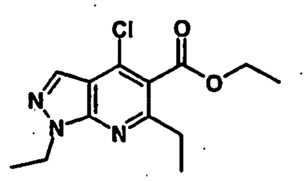

Diethyl propanoylpropanedioate which is Et-C(O)-C($CO_2$Et)$_2$ [e.g. see J. Org. Chem., (1976), 41 (24), 3857] (2.74g) was dissolved in phosphoryl chloride (30ml), tributylamine (3ml) was added cautiously, and the mixture was heated to 110˚C for 5h. The mixture was concentrated under reduced pressure to remove the phosphoryl chloride, and the residue was dissolved in diethyl ether (about 20ml) and *n*-hexane added until two layers formed. The top layer (diethyl ether) was collected and the diethyl ether/hexane extraction procedure repeated twice more. The combined

ether extracts were washed with 1 N HCl solution (2 x 50ml), 1 N sodium hydroxide solution and water, and were dried and evaporated to give diethyl (1-chloropropylidene)propanedioate (1.61g). LCMS showed $MH^+$ = 235; $T_{RET}$ = 3.21 min.

A mixture of diethyl (1-chloropropylidene)propanedioate (1.61g) and 1-ethyl-1H-pyrazol-5-amine (0.755g) [e.g. available from Aldrich Chemical Company, Inc., Albemarle Corporation, Art-Chem GmbH, Enamine and/or TimTec Corporation] in toluene (40ml) was treated with triethylamine (1.8ml) and the mixture heated at 120˚C for 16h. The solvent was evaporated under reduced pressure and the residue was dissolved in phosphoryl chloride (50ml) and heated at 110˚C overnight. The mixture was evaporated under reduced pressure to remove the phosphoryl chloride, and the residue was partitioned between ethyl acetate and saturated aqueous sodium hydrogen carbonate solution. The organic layer was separated, dried (sodium sulphate) and evaporated under reduced pressure. The residue was purified on an SPE cartridge (silica; 50g) eluting with 5% ethyl acetate in cyclohexane to give the title compound as a yellow oil (1.22g). LCMS showed $MH^+$ = 282; $T_{RET}$ = 3.40min.

**Intermediate 1 (Alternative Synthesis B) Ethyl 4-chloro-1,6-diethyl-1*H*-pyrazolo[3,4-*b*]pyridine-5-carboxylate**

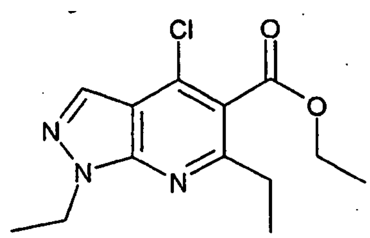

Triethylamine (230 ml) is added dropwise to a mixture of diethyl (1-chloropropylidene)propanedioate (208g) and 1-ethyl-1H-pyrazol-5-amine (101g) in toluene (2.65L). The mixture is heated under reflux for 16 hours. The reaction mixture is cooled to room temperature, and the solid removed by filtration. The filtrate is evaporated under reduced pressure. The residue is heated under reflux in phosphorus oxychloride ($POCl_3$, 2.65L) for 16hrs. Excess phosphorus oxychloride is removed under reduced pressure and the cooled mixture is poured onto a mixture of saturated aqueous $NaHCO_3$ solution (4L) and EtOAc (1.5L). The organic layer is separated and the aqueous layer further extracted with ethyl acetate (2x1L). The combined EtOAc extracts are washed with saturated aqueous $NaHCO_3$ solution (2x2L) and dried ($Na_2SO_4$). Evaporation of solvent under reduced pressure affords the crude product. The crude product is purified by chromatography (silica gel, 60-120 mesh, 3.5 kg), eluting with 3% EtOAc in hexane. Fractions containing the product are pooled and evaporated to give the title compound.

**Intermediate 2 Ethyl 1,6-diethyl-4-(tetrahydro-2*H*-pyran-4-ylamino)-1*H*-pyrazolo[3,4-*b*]pyridine-5-carboxylate**

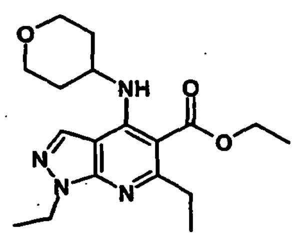

Ethyl 4-chloro-1,6-diethyl-1*H*-pyrazolo[3,4-b]pyridine-5-carboxylate (0.50g) (e.g. this can be as prepared in Intermediate 1) was dissolved in 1-methyl-2-pyrrolidinone (5ml) and treated with tetrahydro-2H-pyran-4-amine hydrochloride (0.49g) [e.g. this can be as prepared in Intermediate 52A, see below] and DIPEA (0.60ml) at 120˚C overnight. The mixture was allowed to cool and was partitioned between ethyl acetate (3 x 50ml) and water (50ml). The organic layer was separated, dried and evaporated *in vacuo.* The residue was purified on an SPE cartridge (e.g. solid phase can be ca. 20g or ca. 50g; e.g. can be silica) eluting with from 5% to 20% ethyl acetate in cyclohexane to give the title compound as pale yellow solid (0.413g). LCMS showed $MH^+$ = 347; $T_{RET}$ = 3.05min.

**Intermediate 2 (Alternative Synthesis B) Ethyl 1,6-diethyl-4-(tetrahydro-2*H*-pyran-4-ylamino)-1*H*-pyrazolo [3,4-*b*]pyridine-5-carboxylate**

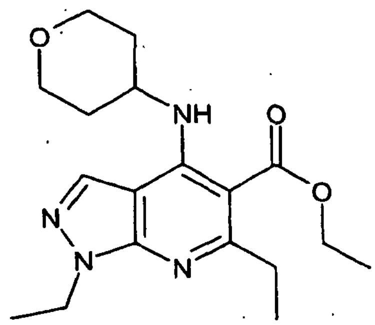

To a solution of ethyl 4-chloro-1,6-diethyl-1*H*-pyrazolo[3,4-*b*]pyridine-5-carboxylate (36g, 127.8mmol) (which can e.g. be as prepared in Intermediate 1) in 1-methyl-2-pyrrolidinone (300ml) is added DIPEA (44.5ml, 255.6mmol). Tetrahydro-2H-pyran-4-amine (e.g. available from Peakdale and/or Combi-Blocks Inc., 15.5g, 153.3mmol) is added and the reaction mixture is heated at 115°C with stirring overnight. The cooled mixture is poured into water (1200ml), which may form an oily mixture. This is extracted with EtOAc (4x250ml), and the organic extracts are combined, washed with water (50ml), 5% aqueous LiCl solution (50ml), dried ($MgSO_4$), filtered and evaporated. The residue is purified by silica gel (1kg) chromatography eluting with 2:1 cyclohexane:EtOAc (6000ml) followed by 1:1 cyclohexane:EtOAc (3000ml). The fractions containing product are pooled and evaporated to give the title compound.

**Intermediate 3 [1-ethyl-4-(tetrahydro-2*H*-pyran-4-ylamino)-1*H*-pyrazolo[3,4-*b*]pyridin-5-yl]methanol**

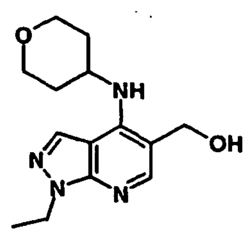

A solution of 1 M diisobutylaluminium hydride in dichloromethane (80ml) was added dropwise to a stirred solution of ethyl 1-ethyl-4-(tetrahydro-2*H*-pyran-4-ylamino)-1*H*-pyrazolo[3,4-*b*]pyridine-5-carboxylate (13.80g) [e.g. see Intermediate 32 and/or Example 3 of WO 2004/024728 A2, and/or Intermediate 4 of WO 2005/058892 A1] in dichloromethane (75ml) at 0°C under a nitrogen atmosphere. The reaction mixture was maintained below 5°C during the addition, and was then stirred at about 0°C. The reaction mixture was then quenched by addition of aqueous potassium sodium tartrate (10% solution), diluted with water (150ml) and the organic phase separated. The aqueous phase was extracted with ethyl acetate (2 x 250ml) and the combined organics were dried (magnesium sulphate) and evaporated. The residue was divided into three portions and each portion was purified by column chromatography on silica gel (100g) eluting with a gradient of from 0 to 100% ethyl acetate in cyclohexane followed by from 0 to 20% methanol in ethyl acetate, and appropriate fractions were combined and evaporated, to give the title compound as a white solid (10.29g). LCMS showed $MH^+$ = 277;$T_{RET}$= 1.81min.

**Intermediate 4 [1-ethyl-6-methyl-4-(tetrahydro-2*H*-pyran-4-ylamino)-1*H*-pyrazolo[3,4-*b*]pyridin-5-yl]methanol**

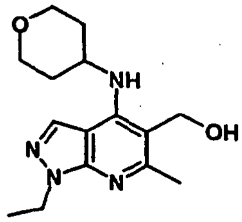

Intermediate 4 was prepared in an analogous manner to Intermediate 3 from ethyl 1-ethyl-6-methyl-4-(tetrahydro-2*H*-pyran-4-ylamino)-1*H*-pyrazolo[3,4-*b*]pyridine-5-carboxylate [e.g. see WO 2004/024728]. LCMS showed $MH^+$ 291; $T_{RET}$ = 1.76min.

**Intermediate 5 [1,6-diethyl-4-(tetrahydro-2*H*-pyran-4-ylamino)-1*H*-pyrazolo[3,4-b]pyridin-5-yl]methanol**

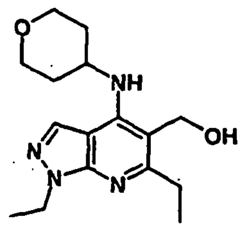

Intermediate 5 was prepared in an analogous manner to Intermediate 3 from ethyl 1,6-diethyl-4-(tetrahydro-2*H*-pyran-4-ylamino)-1*H*-pyrazolo[3,4-b]pyridine-5-carboxylate (e.g. which can be as prepared in Intermediate 2).

*One specific synthesis of Intermediate 5:*

Two reactions were done. The first reaction was as follows: To ethyl 1,6-diethyl-4-(tetrahydro-2*H*-pyran-4-ylamino)-1*H*-pyrazolo[3,4-b]pyridine-5-carboxylate (7.5g) (e.g. which can be as prepared in Intermediate 2) in dichloromethane (75ml) under nitrogen at 0 ˚C was added a solution of diisobutyaluminium hydride in toluene (1.5M, 43ml), dropwise, keeping the temperature at 0 ˚C. The addition took 9.5mins. Stirring at 0 ˚C was continued for 30mins; then the reaction was quenched with saturated aqueous sodium potassium tartrate solution (30ml). There was much effervescence. The mixture gelled which made work-up more difficult. Ethyl acetate and water were added and the layers separated. The aqueous phase was extracted with more ethyl acetate and the combined organic extracts were washed with brine, dried and evaporated to give a white solid (6.13g). The second duplicate reaction was generally the same (also using a 9.5mins addition), and gave 6.59g of product. The two batches of product from each reaction were each dissolved in chloroform and were filtered. The solutions from each duplicate reaction were combined and then evaporated to give the title compound as a white solid (12.57g). LCMS showed $MH^+$ 305; $T_{RET}$ = 1.84min.

**Intermediate 5 (Alternative. Synthesis B) [1,6-diethyl-4-(tetrahydro-2H-pyran-4-ylamino)-1*H*-pyrazolo[3,4-b]pyridin-5-yl]methanol**

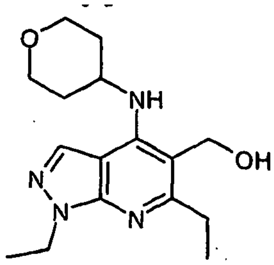

**Intermediate 5 (Alternative Synthesis B) Process Scheme**

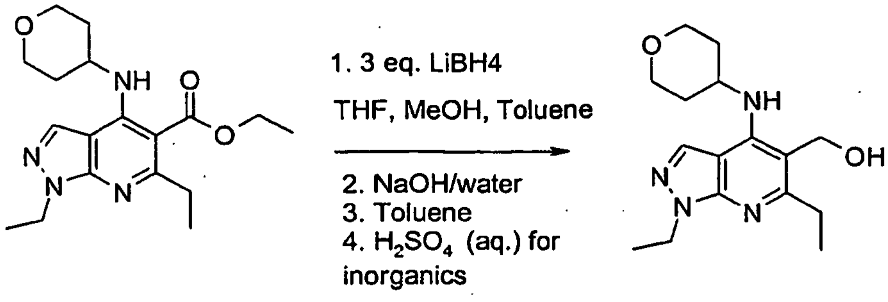


**Intermediate 5 (Alternative Synthesis B) Process Summary**

Note: All or most of this process is carried out under a nitrogen atmosphere. All weights, volumes and equivalents are relative to ethyl 1,6-diethyl-4-(tetrahydro-2*H*-pyran-4-ylamino)-1*H*-pyrazolo[3,4-b]pyridine-5-carboxylate.

To a stirred solution of lithium borohydride (3eq, 4.34vol, 2M in THF) and toluene (4 vols) at 64-68 ˚C, is added a solution of ethyl 1,6-diethyl-4-(tetrahydro-2*H*-pyran-4-ylamino)-1*H*-pyrazolo[3,4-*b*]pyridine-5-carboxylate (1wt) in THF (1vol) and methanol (9eq, 1.05vol) over 2.5 hours. The reaction is monitored by HPLC and is typically complete 60 minutes after the addition. The reaction mixture is then cooled down to 20±3˚C and water (3 vol) is added dropwise over 20mins followed by 10.8M NaOH (6.0 vol) (dropwise over 10 mins), ensuring the temperature remains below 40 ˚C. The temperature of the reaction is then adjusted to 40±3˚C and stirred vigorously for at least 90 minutes. The organic layer is separated and the aqueous layer is transferred to a Schott bottle. Water is added to the organic layer (2.5 vols) followed by NaOH (10.8M, 2 vols), the biphase is stirred vigorously at 40˚C for at least 30 minutes. The organic layer is again separated and the organic solution is concentrated *in vacuo* to 3vol, and the product precipitates as yellow solid. Toluene (3vol) is then added and the slurry is reconcentrated *in vacuo* to 3vol. The suspension is then cooled to 10±3˚C and aged for at least 30min. The solid is then filtered and washed with toluene (3vol). The product is then dried *in vacuo* at 45˚C to constant temperature.

**Intermediate 5 (Alternative Synthesis B) Procedure (can be run in 1.0L or 10.0-L equipment)**

Note: All or most of this process is carried out under a nitrogen atmosphere. All weights, volumes and equivalents are relative to ethyl 1,6-diethyl-4-(tetrahydro-2*H*-pyran-4-ylamino)-1*H*-pyrazolo[3,4-*b*]pyridine-5-carboxylate.

1. Purge vessel with nitrogen.

2. Charge toluene (4 vols) to the vessel, mark 3 volumes during addition.

3. Charge $LiBH_4$ (2M in THF, 4.34 vols) to vessel.

4. Heat contents to 64-68 ˚C.

5. Charge a Schott bottle with ethyl 1,6-diethyl-4-(tetrahydro-2*H*-pyran-4-ylamino)-1*H*-pyrazolo[3,4-*b*]pyridine-5-carboxylate (1wt), add THF (1 vol) and MeOH (1.05vols) and stir to dissolve contents.

6. Add the solution from step 5 to the $LiBH_4$ solution *via* peristaltic pump fitted with silicon tubing over 2.5 hours. Maintain $T_{int}$ at 64-68 ˚C.

7. Stir the mixture for at 50-70 minutes at 64-68 ˚C.

8. Sample the reaction and check for completeness by generic HPLC (typically complete at 60 minutes). The reaction can be held at 20˚C overnight at this point. Do not heat reaction beyond 70 minutes; start to cool at 70 minutes.

9. Cool contents of vessel to 20±3 ˚C, add water (3vols) to the vessel *via* peristaltic pump fitted with silicon tubing over at least 20 minutes. Ensure $T_{int}$ <40 ˚C.

10. Adjust contents to 20±3 ˚C and add 10.8M sodium hydroxide (6.0 vols) to the vessel *via* peristaltic pump fitted with silicon tubing over 10 minutes, stir vigorously.

11. Adjust contents to 40±3 ˚C and stir for at least 90 minutes then allow layers to separate (separation <3minutes).

12. Remove bottom aqueous layer from vessel. The organic layer can be held at this point at 20˚C overnight.

13. Add water (2.5 vols) and NaOH (10.8M, 2 vols) to the organic layer, stir biphase for at least 30 minutes at 40˚C and then allow layers to separate.

14. Remove bottom aqueous layer from vessel.

15. Concentrate the vessel contents to 3vols using vacuum distillation.

16. Add toluene to the vessel (3vols) and reconcentrate the contents to 3vol *via* vacuum distillation.

17. Cool the contents to 10±3 ˚C and stir for at least 30 minutes. The batch can be held overnight at this point.

18. Filter off the product and wash the cake with toluene (1x3vol) before sucking the cake dry (filtration times are both less than 2 minutes).

19. Transfer product to a vacuum oven and dry to constant temperature at 45-50 ˚C under vacuum (drying complete in less than 24 hours).

20. A yellow solid is obtained of [1,6-diethyl-4-(tetrahydro-2*H*-pyran-4-ylamino)-1*H*-pyrazolo[3,4-*b*]pyridin-5-yl]methanol.

21. For dispposal of aqueous layer, aqueous layer ex-step 12 and 14 can be charged to a vessel and the contents adjusted to 10±3˚C. The stirred solution is acidified using 5M $H_2SO_4$ (11 vols) which is added over approx 1 hour (caution, exothermic). Check pH <3.

**<u>Intermediate 6</u> 5-(chloromethyl)-1-ethyl-*N*-(tetrahydro-2*H*-pyran-4-yl)-1*H*-pyrazolo[3,4-b]pyridin-4-amine**

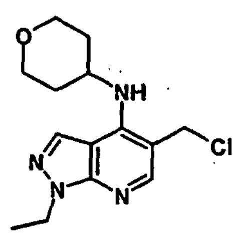

[1-ethyl-4-(tetrahydro-2*H*-pyran-4-ylamino)-1*H*-pyrazolo[3,4-*b*]pyridin-5-yl]methanol (80mg) (e.g. which can be as prepared in Reference Intermediate 3) was treated with thionyl chloride (1 ml), heated at 80˚C for 1 h and then allowed to cool. The orange solution was evaporated to dryness and the residue azeotroped with toluene (2 x 5ml) to give the title compound as a brown foam. LCMS of the compound in methanol showed $MH^+$ 291; $T_{RET}$ = 1.90min which is consistent with the 5-(chloromethyl)-pyrazolo[3,4-*b*]pyridine derivative reacting with the methanol during LCMS to give the corresponding 5-(methoxymethyl)-pyrazolo[3,4-*b*]pyridine derivative.

**<u>Intermediate 7</u> 5-(chloromethyl)-1-ethyl-6-methyl-*N*-(tetrahydro-2*H*-pyran-4-yl)-1*H*-pyrazolo[3,4-*b*]pyridin-4-amine**

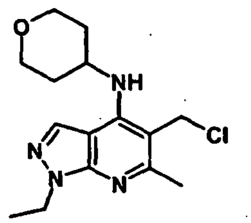

Intermediate 7 was prepared in an analogous manner to Intermediate 6 from Intermediate 4. LCMS of the compound in methanol showed $MH^+$ 305; $T_{RET}$ = 1.89min which is consistent with the 5-(chloromethyl)-pyrazolo[3,4-*b*] pyridine derivative reacting with the methanol during LCMS to give the corresponding 5-(methoxymethyl)-pyrazolo[3,4-*b*] pyridine derivative.

**Intermediate 8 5-(chloromethyl)-1,6-diethyl-*N*-(tetrahydro-2*H*-pyran-4-yl)-1*H*-pyrazolo[3,4-*b*]pyridin-4-amine**

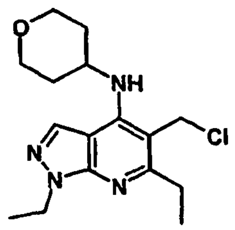

Intermediate 8 can be prepared in an analogous manner to Intermediate 6 from [1,6-diethyl-4-(tetrahydro-2*H*-pyran-4-ylamino)-1*H*-pyrazolo[3,4-*b*]pyridin-5-yl]methanol (e.g. which can be as prepared in Intermediate 5).

*One specific synthesis of Intermediate 8*:

[1,6-diethyl-4-(tetrahydro-2*H*-pyran-4-ylamino)-1*H*-pyrazolo[3,4-*b*]pyridin-5-yl]methanol (0.120g) (e.g. which can be as prepared in Intermediate 5) is treated with thionyl chloride (1.5ml), and the solution is heated at 80 ˚C for 1.5h and then allowed to cool. The reaction mixture is concentrated to dryness and then azeotroped with toluene (5ml) to leave the title compound.

**Intermediate 9 1-Ethyl-4-(tetrahydro-2*H*-pyran-4-ylamino)-1*H*-pyrazolo[3,4-*b*]pyridine-5-carbaldehyde**

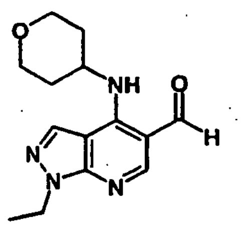

Pyridinium chlorochromate (PCC) (3.90g) and sodium acetate (0.297g) were suspended in dichloromethane (15ml) and a solution of Intermediate 3 (1.0g) in dichloromethane (15ml) was added in one portion with stirring. After 2.5h at room temperature, the reaction mixture was diluted with diethyl ether (30ml) and the supernatant was decanted from the dark gum. The insoluble gum was washed with 1:1 dichloromethane/diethyl ether (3 x 30ml). The supernatant and organic washings were combined and evaporated to give an orange oil (1.015g) which was dissolved in dichloromethane (5ml) and applied to an SPE cartridge (silica; 20g). The cartridge was eluted with a gradient of 20 -100% ethyl acetate in petroleum ether. Fractions containing the desired material were combined and concentrated *in vacuo* to give Intermediate 9 as an off-white crystalline solid (0.59g). LCMS showed $MH^+$ = 275; $T_{RET}$ = 2.38min.

**Intermediate 10 1-Ethyl-5-{[(phenylmethyl)imino]methyl}-*N*-(tetrahydro-2*H*-pyran-4-yl)-1*H*-pyrazolo[3,4-*b*]pyridin-4-amine**

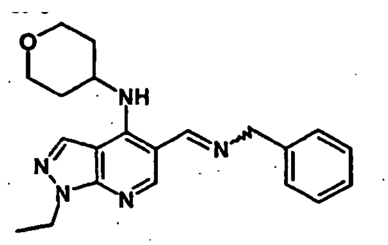

Benzylamine (0-37ml) was added to a stirred solution of Intermediate 9 (0.467g) in ethanol (20ml) and the resulting mixture was heated under reflux for 18h. The reaction mixture was then evaporated, and the residual oil dissolved in dichloromethane (10ml) and applied to an SPE cartridge (silica; 10g). The cartridge was eluted with a gradient of 33%, 50% and 100% ethyl acetate in petroleum ether. Fractions containing the desired material were combined and concentrated *in vacuo* to give Intermediate 10 as a colourless oil (0.62g). LCMS showed $MH^+$ = 364; $T_{RET}$ = 3.13min.

**Intermediate 11 5-(azidomethyl)-1-ethyl-*N*-(tetrahydro-2*H*-pyran-4-yl)-1*H*-pyrazolo[3,4-*b*]pyridin-4-amine**

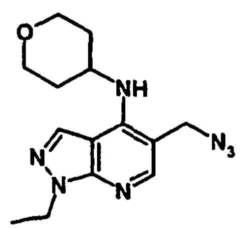

[1-ethyl-4-(tetrahydro-2*H*-pyran-4-ylamino)-1*H*-pyrazolo[3,4-*b*]pyridin-5-yl]methanol (0.552 g) (e.g. which can be as prepared in Intermediate 3) was treated with thionyl chloride (2ml, excess) and the solution was heated at 80˚C for 2h and then allowed to cool. The thionyl chloride was evaporated off and the resulting solid was azeotroped with toluene (2 x 5ml) to leave a yellow solid.

A solution of this solid, presumed to be 5-(chloromethyl)-1-ethyl-*N*-(tetrahydro-2*H*-pyran-4-yl)-1*H*-pyrazolo [3,4-*b*]pyridin-4-amine, (50mg) in anhydrous dimethylsulphoxide (0.20ml) was treated with lithium azide (9mg) and the solution stirred at room temperature for 20h. A further portion of lithium azide (15mg) was then added, and after a further day of stirring at room temperature, water (0.25ml) was added. The solution was extracted with dichloromethane (2 x 5ml) and the combined organic extracts were passed through a hydrophobic frit (6ml) then blown to dryness to leave a clear colourless gum. This was dissolved in dichloromethane (0.5ml) and applied to an SPE cartridge (silica; 1g). The cartridge was eluted with 50% ethyl acetate in cyclohexane and fractions containing the desired material were combined and blown to dryness to give the title compound as a clear colourless gum (10mg). LCMS showed $MH^+$ = 302; $T_{RET}$ = 2.06min.

**Intermediate 12 5-(azidomethyl)-1-ethyl-6-methyl-*N*-(tetrahydro-2*H*-pyran-4-yl)-1*H*-pyrazolo[3,4-b]pyridin-4-amine**

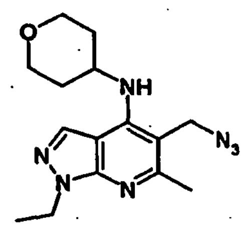

Intermediate 12 was prepared in analogous manner to Intermediate 11 from Intermediate 7. LCMS showed $MH^+ = 316$; $T_{RET} = 2.00$min.

**Intermediate 13 5-(azidomethyl)-1,6-diethyl-*N*-(tetrahydro-2*H*-pyran-4-yl)-1*H*-pyrazolo[3,4-*b*]pyridin-4-amine**

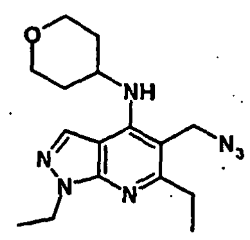

It is thought that Intermediate 13 can be prepared, in an analogous manner to Intermediate 11 from 5-(chloromethyl)-1,6-diethyl-N-(tetrahydro-2H-pyran-4-yl)-1H-pyrazolo[3,4-b]pyridin-4-amine (e.g. which can be as prepared in Intermediate 8).

*One specific synthesis of Intermediate 13:*

To [1,6-diethyl-4-(tetrahydro-2*H*-pyran-4-ylamino)-1*H*-pyrazolo[3,4-*b*]pyridin-5-yl]methanol (12.57g) (e.g. which can be as prepared in Intermediate 5) was added thionyl chloride (51ml; 83.6g) and the mixture was heated to 80 ˚C. There was effervescence during the addition, and the reaction mixture turned yellow, and then red on heating. After 5h excess thionyl chloride was removed on an evaporator and the wine-red residue was azeotroped with toluene (75ml) to leave a residue containing 5-(chloromethyl)-1,6-diethyl-*N*-(tetrahydro-2*H*-pyran4-yl)-1*H*-pyrazolo[3,4-*b*] pyridin-4-amine.

This residue was dissolved in DMF (100ml) and treated with sodium azide (4.0g) and the mixture was stirred at room temperature overnight. Water was added, followed by 2M sodium hydroxide solution to adjust from a pH of about 3.5 to a pH of about 8. Ethyl acetate was added and the layers separated. The aqueous phase was extracted with more ethyl acetate. Then the combined organics were washed with water, aqueous lithium chloride solution and brine, and were dried and evaporated to give an orange-brown oil which was subject to high vacuum. This was purified by column chromatography using 400ml of 7734 silica gel eluting with a 3 : 2 mixture of cyclohexane : ethyl acetate, and fractions containing a mixture of two products (as nmeasured by TLC) were collected and reduced to give the title compound as a golden oil which started to crystallise under high vacuum (9.74g). LCMS showed MH+ = 330; $T_{RET}$ = 2.30min.

**Intermediate 14 5-(aminomethyl)-1-ethyl-*N*-(tetrahydro-2*H*-pyran-4-yl)-1*H*-pyrazolo[3,4-b]pyridin-4-amine**

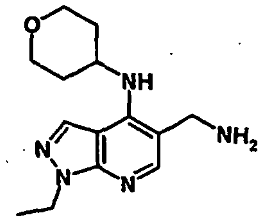

A solution of 5-(azidomethyl)-1-ethyl-*N*-(tetrahydro-2*H*-pyran-4-yl)-1*H*-pyrazolo[3,4-*b*]pyridin-4-amine (0.351g) (e.g. which can be as prepared in Reference Intermediate 11) in ethanol (30ml) was added to 5% palladium on carbon (wet) (0.050g) and the mixture was stirred at room temperature for 20 hours under an atmosphere of hydrogen. The mixture was filtered first through a glass fibre filter and then through a plug of celite, which was then washed with ethanol (about 50ml). The combined filtrate and washings were evaporated to dryness to give the title compound as a dirty green gum (0.318g). LCMS showed $MH^+$ = 276; $T_{RET}$ = 1.63min.

**Intermediate 15 5-(aminomethyl)-1-ethyl-6-methyl-*N*-(tetrahydro-2*H*-pyran-4-yl)-1*H*-pyrazolo[3,4-b]pyridin-4-amine**

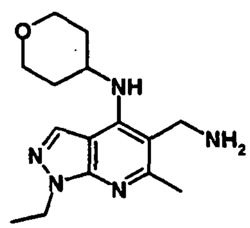

Intermediate 15 was prepared in an analogous manner to Intermediate 14 from Intermediate 12. LCMS showed $MH^+$ = 290; $T_{RET}$ = 1.52min.

**Intermediate 16 5-(aminomethyl)-1,6-diethyl-*N*-(tetrahydro-2*H*-pyran-4-yl)-1*H*-pyrazolo[3,4-*b*]pyridin-4-amine**

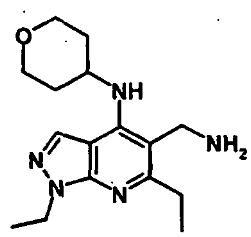

It is thought that Intermediate 16 can be prepared, in an analogous manner to Intermediate 14, from 5-(azidomethyl)-1,6-diethyl-*N*-(tetrahydro-2*H*-pyran-4-yl)-1*H*-pyrazolo[3,4-*b*]pyridin-4-amine (e.g. which can be as prepared in Intermediate 13).

*One specific synthesis of Intermediate 16:*

To 10% by weight palladium on carbon (0.4g) was added ethanol (10ml) followed by 5-(azidomethyl)-1,6-diethyl-*N*-(tetrahydro-2*H*-pyran-4-yl)-1*H*-pyrazolo[3,4-*b*]pyridin-4-amine (4g) (e.g. which can be as prepared in Intermediate 13) in ethanol (90ml) in a hydrogenation flask. The mixture was then hydrogenated at room temperature and atmospheric pressure with stirring overnight. Then, the palladium on carbon catalyst was filtered off twice to give a solution. The solvent was removed to give the title compound as a grey solid (2.59g). LCMS showed $MH^+$ = 304; $T_{RET}$ = 1.63min.

**Intermediate 16A 5-(aminomethyl)-1 ,6-diethyl-*N*-(tetrahydro-2*H*-pyran-4-yl)-1*H*-pyrazolo[3,4-*b*]pyridin-4-amine hydrochloride**

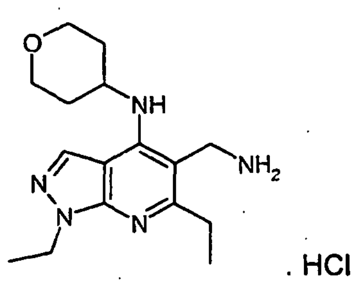

**Intermediate 16A Process Scheme:**

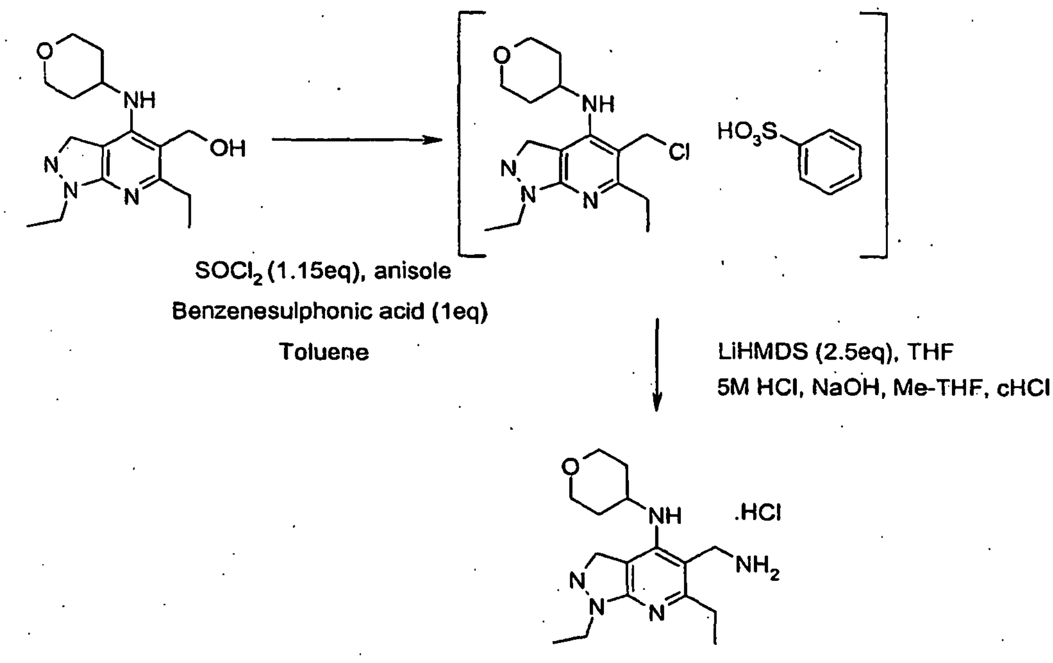


Intermediate 16A

**Intermediate 16A Process Summary**

Note: All or most of this process is carried out under a nitrogen atmosphere.

[1,6-diethyl-4-(tetrahydro-2*H*-pyran-4-ylamino)-1*H*-pyrazolo[3,4-*b*]pyridin-5-yl]methanol (e.g. which can be as prepared in Intermediate 5, e.g. Alternative Synthesis B thereof) is suspended in anisole and treated with solid benzensulfonic acid. This suspension is aged at room temperature for 30 minutes. Thionyl chloride is added at about 20 degree C over 20 minutes, and stirred for 20 minutes. The reaction is then sampled for HPLC. The resulting solution of 5-(chloromethyl)-1,6-diethyl-*N*-(tetrahydro-2*H*-pyran-4yl)-1*H*-pyrazolo[3,4-*b*]pyridin-4-amine benzenesulfonate is treated with toluene and then placed under medium vacuum (100 mbar) to remove hydrogen chloride and sulfur dioxide, then the vacuum is increased (50 mbar) to azeotrope off excess thionyl chloride and toluene.

This solution of 5-(chloromethyl)-1,6-diethyl-*N*-(tetrahydro-2*H*-pyran-4-yl)-1*H*-pyrazolo[3,4-*b*]pyridin-4-amine benzenesulfonate is added to a solution of lithium hexamethyldisilazide (LiHMDS) in tetrahydrofuran at 35-40 degrees C over about an hour and the reaction is usually complete after a further 10 minutes.

The mixture is cooled to 10 degrees C and hydrochloric acid (5M) is added. The phases are separated and the lower aqueous layer is transferred back into the vessel, and the organic phase is discarded. Methyl-THF (methyltetrahydrofuran) is added and then the biphasic mixture is adjusted to pH >13 with sodium hydroxide solution (10M). The layers are separated and the lower aqueous layer is back extracted with further methyl-THF. The combined organic phase is washed with brine.

The amount of the desired 5-(aminomethyl)-1,6-diethyl-*N*-(tetrahydro-2*H*-pyran-4-yl)-1*H* pyrazolo[3,4-*b*]pyridin-4-amine is quantified via yieldaliser, and an appropriate amount of concentrated hydrochloric acid is added at 55-60 degrees C. The suspension is held for 2 hours at about 50 degrees C, and then cooled to 10 degrees C over 3 hours and held at this temperature for at least 3 hours.

The slurry is filtered and washed with methyl-THF and dried at 60 ˚C to constant weight or batch temperature, to give 5-(aminomethyl)-1,6-diethyl-*N*-(tetrahydro-2*H*-pyran-4-yl)-1 H-pyrazolo[3,4-b]pyridin-4-amine hydrochloride (Intermediate 16A).

**Intermediate 16A Detailed Procedure: (can be run in 1.0/10 L equipment)**

Note: All or most of this process is carried out under a nitrogen atmosphere.

1. [1,6-diethyl-4-(tetrahydro-2*H*-pyran-4-ylamino)-1*H*-pyrazolo[3,4-*b*]pyridin-5-yl]methanol (1wt) is suspended in anisole (6vol) at 20-25˚C.

2. Benzenesulfonic acid (1 equivalent, 0.52wt) is added.

3. The temperature is adjusted to 20-25˚C and the reaction is aged for 30 minutes.

4. Thionyl chloride (1.15 equivalents, 0.45wt, 0.275vol) is added over about 20 minutes at 20-25˚C.

5. Anisole (0.25vol) is used to wash the line.

6. Sample after 20 minutes at 20-25˚C. Reaction is judged complete when <4% of [1,6-diethyl-4-(tetrahydro-2*H*-pyran-4-ylamino)-1*H*-pyrazolo[3,4-*b*]pyridin-5-yl]methanol remains when compared to the 5-(methoxymethyl)-pyrazolo[3,4-*b*]pyridine derivative derived from a methanol quench of 5-(chloromethyl)-1,6-diethyl-*N*-(tetrahydro-2*H*-pyran-4-yl)-1*H*-pyrazolo[3,4-*b*]pyridin-4-amine benzenesulfonate.

7. Vessel fill volume is noted, and toluene (2vol) added.

8. The mixture is heated to a jacket temperature of 40 degrees C and held under moderate vacuum (100 mbar) for 1 hour with agitation.

9. Vacuum is increased to about 50 mbar and contents are distilled to marked volume above (about 7.5vol). The Contents should not be heated above 50 degrees C. (Distillation time around 80 minutes, p = 45-50 mbar, $T_{int}$ = 20-48˚C.)

10. Reaction mixture is cooled to 20-25˚C and can be held at this point.

11. This mixture (ex-step 10) is added to lithium hexamethyldisilazide in THF (1.26M in THF, 6.51vol, 5.73wt) over 60-70 minutes at 35-40˚C.

12. Sample after a further 10 minutes at 35-40˚C. Reaction is judged complete if <4% of the 5-(methoxymethyl)-pyrazolo[3,4-b]pyridine derivative is seen relative to 5-(aminomethyl)-1,6-diethyl-*N*-(tetrahydro-2*H*-pyran-4-yl)-1*H*-pyrazolo[3,4-*b*]pyridin-4-amine (after quench into methanol) by HPLC.

13. Mixture is cooled to 10˚C and hydrochloric acid (5M, 3.6 vol) is added over 20 minutes. The phases are stirred vigorously for 10 minutes.

14. The lower aqueous layer is retained, and the upper anisole layer discarded. The batch can be held at 20-25 ˚C at this point.

15. The brown aqueous layer is treated with methyl-tetrahydrofuran (8vol).

16. Sodium hydroxide solution (ca 1.8 vol, 10M) is added to the biphasic mixture to give a resulting aqueous layer with pH >13. The temperature is raised to 30˚C and the mixture is stirred vigorously for 20 minutes (separation time 5 minutes in 10L CLR equipment).

17. The layers are separated and the lower aqueous layer (yellow) is backwashed with methyl-tetrahydrofuran (2vol). The aqueous layer is sampled and analysed for residual 5-(aminomethyl )-1,6-diethyl-*N*-(tetrahydro-2*H*-pyran-4-yl)-1*H*-pyrazolo[3,4-*b*]pyridin-4-amine prior to disposal.

18. Brine (15%w/w NaCl, 2vol) is added to the combined brown organic layers the biphase is vigorously stirred at 30 ˚C and separated. The lower aqueous layer is removed.

19. The volume of the organic layer is measured arid a sample taken. The yieldaliser system is used to determine the absolute amount of 5-(aminomethyl)-1,6-diethyl-N (tetrahydro-2*H*-pyran-4-yl)-1*H*-pyrazolo[3,4-*b*]pyridin-4-amine present.

20. The reaction mixture is heated to 55-60˚C and treated with concentrated hydrochloric acid [1.03 equivalents relative to the 5-(aminomethyl)-1,6-diethyl-N-(tetrahydro-2*H*-pyran-4-yl)-1*H*-pyrazolo[3,4-*b*]pyridin-4-amine present, about 0.24-0.30 vol].

21. The mixture is aged at 50-55˚C for 2 hours and then cooled to 5-10˚C over 3 hours.

22. The mixture is aged at 5-10˚C for at least 3 hours.

23. Mixture is filtered and washed with methyl-THF (2 x 2vol). The mother liquors are kept until a dried weight and yield of product is obtained. (Filtration times all less then 90seconds on a scale of about 700g.)

24. The off white solid cake is dried in a vacuum oven at 60 ˚C to constant temperature.

**Intermediate 17: Ethyl 4-chloro-1-ethyl-6-methyl-1H-pyrazolo[3,4-b]pyridine-5-carboxylate**

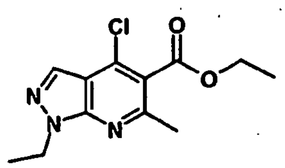

A mixture of 5-amino-1-ethylpyrazole (1.614g, 14.5mmol) and diethyl 2-(1-ethoxyethylidene)malonate [J. Am. Chem. Soc., 1931, 53, 1836] (3.68g) was heated at 150 ˚C under Dean Stark conditions for 5 hours. Phosphorous oxychloride (25ml) was carefully added to the mixture and the resulting solution was heated at 130 ˚C under reflux for 18 hours. The mixture was concentrated *in vacuo* and the residual oil was carefully added, with cooling, to water (100ml). The resulting mixture was extracted with dichloromethane (3 x 100ml) and the combined organic extracts were dried over anhydrous sodium sulphate and concentrated *in vacuo.* The residual oil was purified by Biotage chromatography (silica; 90g) eluting with 5% ethyl acetate in petroleum ether. Fractions containing the desired product were combined and concentrated *in vacuo* to give Intermediate 17 (1.15g). LCMS showed $MH^+$ = 268; $T_{RET}$ = 3.18min.

**Intermediate 18 Ethyl 4-[(1-{[(1,1-dimethylethyl)oxy]carbonyl}-4-piperidinyl)amino]-1,6-diethyl-1*H*-pyrazolo[3,4-*b*]pyridine-5-carboxylate**

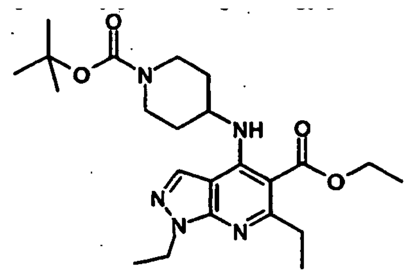

Intermediate 1 (5.0g) was dissolved in N-methyl-2-pyrrolidinone (60ml) and treated with 1,1-dimethylethyl 4-amino-1-piperidinecarboxylate *[Astatech]* (3.91g) and DIPEA (7.7ml) at 120°C for 18h. The mixture was allowed to cool and poured into 10% aqueous lithium chloride solution (400ml). The mixture was extracted with ethyl acetate (3 x 100ml) and the combined organic extracts were dried (sodium sulphate), filtered and evaporated to give a gum. This residue was purified on an SPE cartridge (silica; 50g), eluting with a gradient of 0 - 100% ethyl acetate in cyclohexane to give Intermediate 18 as pale yellow solid (8.22g). LCMS showed $MH^+$ = 446; $T_{RET}$= 3.67min.

**Intermediate 19 Ethyl 4-[(1-{[(1,1-dimethylethyl)oxy]carbonyl}-4-piperidinyl)amino]-1-ethyl-6-methyl-1*H*-pyrazolo[3,4-*b*]pyridine-5-carboxylate**

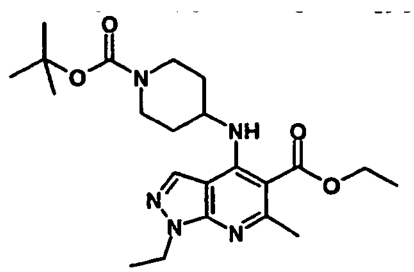

Intermediate 19 was prepared in an analogous manner to Intermediate 18 from Intermediate 17 and 1,1-dimethylethyl 4-amino-1-piperidinecarboxylate. LCMS showed $MH^+$ = 432 ; $T_{RET}$ = 3.5min.

**Intermediate 20 1,1 -dimethylethyl-4-{[1,6-diethyl-5-(hydroxymethyl)-1*H*-pyrazolo[3,4-*b*]pyridin-4-yl]amino}-1-piperidinecarboxylate**

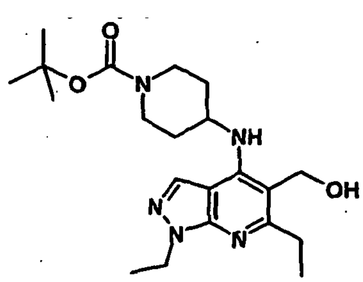

Intermediate 18 (8.2g) was dissolved in THF (60ml). The solution was treated with lithium borohydride (0.7g) and methanol (1.3ml) and then heated at reflux for 4 hours. The mixture was cooled and treated again with lithium borohydride (0.7g) and methanol (1.3ml) and heated at reflux for a further 2 hours. The mixture was cooled in ice/water and quenched with methanol (20ml) and stirred for 1 hour at room temperature, then diluted with dichloromethane (100ml) and water (100ml). The organic phase was separated and the aqueous phase extracted with dichloromethane (100ml). The combined organic extracts were passed through a hydrophobic frit and evaporated. The residue was triturated with diethyl ether to give a solid which was collected by filtration and dried to give Intermediate 20 as a white

solid (5.2g). LCMS showed $MH^+$= 404; $T_{RET}$ = 2.44min.

**Intermediate 21 1,1-dimethylethyl 4-{[1-ethyl-5-(hydroxymethyl)-6-methyl-1*H*-pyrazolo[3,4-*b*]pyridin-4-yl]amino}-1-piperidinecarboxylate**

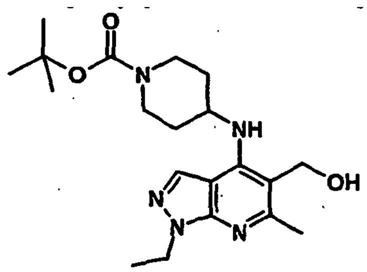

Intermediate 21 was prepared in an analogous manner to Intermediate 20 from Intermediate 19. LCMS showed $MH^+$ = 390; $T_{RET}$ = 2.34min.

**Intermediate 22 1,1-dimethylethyl 4-{[5-(azidomethyl)-1,6-diethyl-1*H*-pyrazolo[3,4-b]pyridin-4-yl]amino}-1-piperidinecarboxylate**

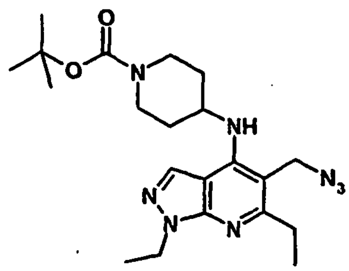

Intermediate 20 (5.2g) was dissolved in DMF (50ml). The solution was treated with sodium azide (1.7g) and carbon tetrabromide (8.6g). The resulting yellow solution was cooled in an ice/water bath and a solution of triphenylphosphine (6.9g) in DMF (50ml) was added dropwise over 15 minutes. The mixture was stirred and allowed to warm to room temperature over 2.5 hours. The solvent was removed by evaporation and the residue was treated with dichloromethane (60ml). The resulting suspension was filtered, the filtrate was evaporated and the residue purified on three SPE cartridges (silica; 50g), eluting with 0% - 50% ethyl acetate in cyclohexane to give Intermediate 22 (5.1g) as a pale yellow solid. LCMS showed $MH^+$ = 429; $T_{RET}$ = 3.00min.

**Intermediate 23 1,1-dimethylethyl 4-{[5-(azidomethyl)-1-ethyl-6-methyl-1*H*-pyrazolo[3,4-*b*]pyridin-4-yl]amino}-1-piperidinecarboxylate**

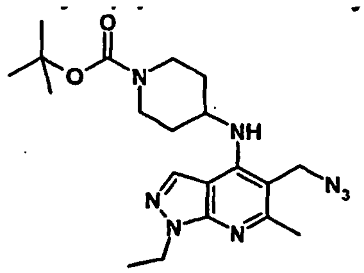

Intermediate 23 was prepared in an analogous manner to Intermediate 22 from Intermediate 21. LCMS showed $MH^+$ = 415; $T_{RET}$ = 2.84min.

**Intermediate 24 4-{[5-(azidomethyl)-1,6-diethyl-1*H*-pyrazolo[3,4-*b*]pyridin-4-yl]amino}-1-piperidinecarboxamide**

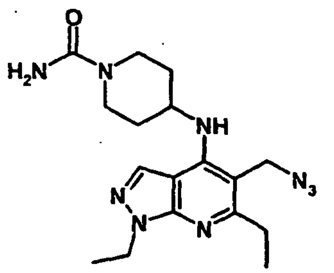

Intermediate 22 (5.14g) was treated with a 4M solution of hydrogen chloride in dioxane (50ml). The reaction mixture was left to stand for 2 hours. The solvent was evaporated and co-evaporated with dichloromethane (2 x 50ml) then diethyl ether (2 x 50ml) to give 5-(azidomethyl)-1,6-diethyl-*N*-4-piperidinyl-1*H*-pyrazolo[3,4-*b*]pyridin-4-amine, hydrochloride salt. [LCMS showed $MH^+$ = 329; $T_{RET}$ = 1.80min]. This product was dissolved in dichloromethane (100ml) and the solution was treated with DIPEA (6.3ml) and trimethylsilyl isocyanate (1.8ml) then left to stand at room temperature for 18 hours. Water (50ml) was added and the precipitated solid collected by filtration, washed with diethyl ether and dried to give Intermediate 24 (2.6g) as a white solid. LCMS showed $MH^+$ = 372; $T_{RET}$ = 2.11 min.

**Intermediate 25 4-{[5-(azidomethyl)-1-ethyl-6-methyl-1*H*-pyrazolo[3,4-*b*]pyridin-4-yl]amino}-1-piperidinecarboxamide**

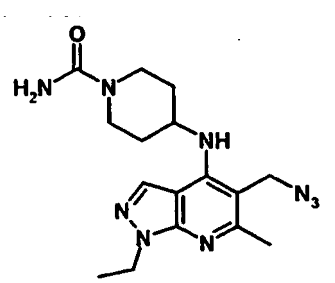

Intermediate 25 was prepared in an analogous manner to Intermediate 24 from Intermediate 23. LCMS showed $MH^+$ = 358; $T_{RET}$ = 1.93min.

**Intermediate 26 4-{[5-(aminomethyl)-1,6-diethyl-1*H*-pyrazolo[3,4-b]pyridin-4-yl]amino}-1-piperidinecarboxamide**

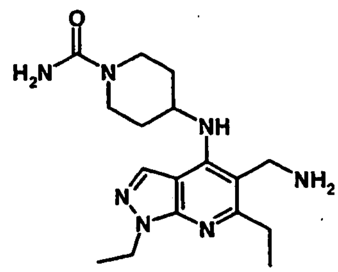

A suspension of Intermediate 24 (2.6g) in ethanol (500ml) was hydrogenated over 10% palladium on activated carbon (0.42g) at room temperature for 16 hours. The mixture was filtered through Celite and the filter bed washed with ethanol. The combined filtrate and washings were evaporated to give Intermediate 26 (2.1g) as a grey solid. LCMS showed $MH^+$ = 345; $T_{RET}$ = 1.68min.

**Intermediate 27 4-{[5-(aminomethyl)-1-ethyl-6-methyl-1*H*-pyrazolo[3,4-*b*]pyridin-4-yl]amino}-1-piperidinecarboxamide**

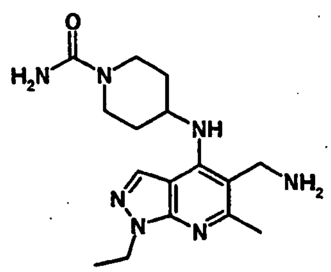

Intermediate 27 was prepared in an analogous manner to Intermediate 26 from Intermediate 25. LCMS showed $MH^+$ = 332; $T_{RET}$ = 1.54min.

**Intermediate 28 4-(acetylamino)-2,3-dihydro-1-benzofuran-7-carboxylic acid**

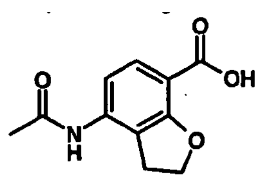

A solution containing methyl 4-(acetylamino)-2,3-dihydro-1-benzofuran-7-carboxylate [Synlett. (1993), (4), 269] (110mg) and lithium hydroxide (14mg) in 1,4-dioxane (6ml) and water (2ml) was stirred at room temperature for 2h. Solvents were evaporated *in vaccuo* and the residue suspended in 10% aqueous citric acid. The solid was collected by filtration and dried to give Intermediate 28 as a white solid (45mg). LCMS showed $MH^+$ 222; $T_{RET}$ = 1.92min.

**Intermediate 28A 1-{[1-ethyl-4-(tetrahydro-2*H*-pyran-4-ylamino)-1*H*-pyrazolo[3,4-*b*]pyridin-5-yl]methyl}-4-piperidinecarboxylic acid, HCl salt**

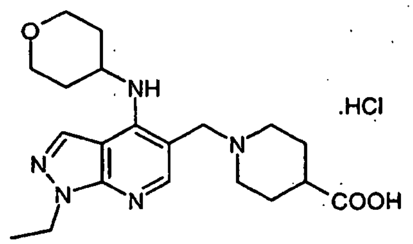

A solution of Intermediate 3 (350mg) in thionyl chloride (5ml) was stirred at 80°C for 1.5h. Solvent was evaporated under reduced pressure and the residue co-evaporated with toluene (2 x 5ml). The residue was diluted with acetonitrile (10ml) and treated with pyridine (0.42ml) at room temperature for 1h and then 1,1-dimethylethyl 4-piperidinecarboxylate [e.g. available from *Tyger Scientific Inc]* (350mg) and DIPEA (0.9ml) were added. The resulting solution was stirred at 60°C for 20h and evaporated to dryness under reduced pressure. The residue was purified by SPE cartridge (silica) eluting with 30 - 60% ethyl acetate in petroleum ether to give 1,1-dimethylethyl 1-{[1-ethyl-4-(tetrahydro-2*H*-pyran-4-ylamino)-1*H*-pyrazolo[3,4-*b*]pyridin -5-yl]methyl}-4-piperidine-carboxylate (480mg).

A mixture of 1,1-dimethylethyl 1-{[1-ethyl-4-(tetrahydro-2*H*-pyran-4-ylamino)-1*H*-pyrazolo[3,4-*b*]pyridin-5-yl] methyl}-4-piperidine-carboxylate (1.01g) and 4N HCl in 1,4-dioxane (10ml) was stirred at room temperature for 2h. Further portions of 4N HCl in 1,4-dioxane (5ml) were added after 2h and 5h and the mixture was evaporated to dryness under reduced pressure. The residue was treated with 4N HCl in 1,4-dioxane as above and the mixture evaporated under reduced pressure to give Intermediate 28A as a yellow solid (1.09g). LCMS showed $MH^+$ = 388; $T_{RET}$ = 1.89min.

**Intermediate 28B 1-{[1-ethyl-6-methyl-4-(tetrahydro-2*H*-pyran-4-ylamino)-1H-pyrazolo[3,4-b]pyridin-5-yl]methyl}-4-piperidinecarboxylic acid, HCl salt**

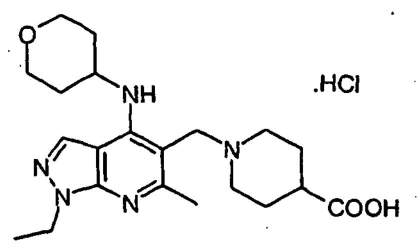

A solution of Intermediate 4 (1.4g) in thionyl chloride (8ml) was stirred at 80°C for 2h. Solvent was evaporated under reduced pressure and the residue co-evaporated with toluene (2 x 5ml). The residue was diluted with acetonitrile (20ml) and treated with 1,1-dimethylethyl 4-piperidinecarboxylate [e.g. available from *Tyger Scientific Inc]* (1.07g) and DIPEA (2.7ml). The resulting solution was stirred at room temperature for 2h and evaporated to dryness under reduced pressure. The residue was purified by SPE cartridge (silica) eluting with 50 - 100% ethyl acetate in petroleum ether to give 1,1-dimethylethyl 1-{[1-ethyl-6-methyl-4-(tetrahydro-2*H*-pyran-4-ylamino)-1*H*-pyrazolo [3,4-b]pyridine-5-yl]methyl}-4-piperidinecarboxylate (1.22g).

A mixture of 1,1-dimethylethyl 1-{[1-ethyl-6-methyl-4-(tetrahydro-2*H*-pyran-4-ylamino)-1*H*-pyrazolo[3,4-*b*]pyridin-5-yl]methyl}-4-piperidinecarboxylate (1.32g) and 4N HCl in 1,4-dioxane (5ml) was stirred at room temperature for 1 h. The mixture was evaporated *in vacuo* and the residue treated with further portions of 4N HCl in 1,4-dioxane (4 x 2ml) whilst being stirred at room temperature for 20h. The mixture was evaporated under reduced pressure to give Intermediate 28B as a yellow solid (1.54g). LCMS showed $MH^+$ = 402; $T_{RET}$ = 1.92min.

**Intermediate 29 *N*-(1-Ethyl-1*H*-pyrazol-5-yl)-2,2,2-trifluoroacetamide**

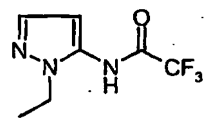

A solution of 5-amino-1-ethylpyrazole (6.0g, 54mmol, e.g. available from Aldrich) in dry dichloromethane (10ml) was added to trifloroacetic anhydride (35ml) over 20min with cooling such that the reaction temperature was maintained at 17 - 21 °C. After a further 20min, the reaction mixture was evaporated to dryness, and the residual oil was dissolved in dichloromethane (250ml) and diluted with 5%-sodium hydrogen carbonate solution (50ml). Solid sodium hydrogen carbonate was then added portionwise to the vigorously stirred two-phase mixture until the aqueous phase was pH 8 - 9. The phases were separated by passage through a hydrophobic frit, and the aqueous phase was extracted with further dichloromethane (50ml). The combined organic extracts were dried over anhydrous sodium sulphate and evaporated to give Intermediate 29 as a yellow oil which slowly crystallised to a yellow crystalline solid (11.2g). LCMS showed $MH^+$ = 208; $T_{RET}$ = 2.20min.

**Intermediate 30 (1Z)-*N*-(1-Ethyl-1*H*-pyrazol-5-yl)-2,2,2-trifluoroethanimidoyl chloride**

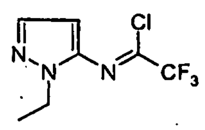

Intermediate 29 (5.58g, 26.9mmol) was stirred with carbon tetrachloride (130ml) and the resulting mixture was treated with triphenylphosphine, polymer supported (22.4g, 67.2 mmol, 3mmol/g of resin, Fluka). The resulting mixture was heated with stirring, under gentle reflux (bath temp = 87°C). After 18h at reflux, the reaction mixture was cooled to 35°C and filtered through a hydrophobic frit, using dichloromethane (150ml) to wash the filter bed. The filtrate was evaporated to give Intermediate 30 as a yellow oil (4.845g) which partially crystallized on standing. LCMS showed $MH^+$ = 226(weak); $T_{RET}$= 3.34min.

**Intermediate 31 Ethyl 1-ethyl-4-hydroxy-6-(trifluoromethyl)-1*H*-pyrazolo[3,4-*b*]pyridine-5-carboxylate**

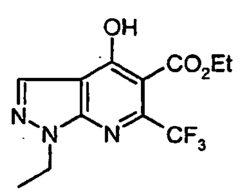

A solution of diethyl malonate (5.165g, 32.2mmol, e.g. commercially available from e.g. Acros) in dry tetrahydrofuran (50ml) was carefully added to a mixture of sodium hydride (60% dispersion in mineral oil, 1.419g, 35.5mmol) in dry tetrahydrofuran (50ml) at 17-21°C under nitrogen. A vigorous reaction occurred, and mild cooling in a cold bath was necessary.. After 40min at room temperature, a mixture of Intermediate 30 (4.84g, 21.5mmol) in dry tetrahydrofuran (50ml) was added to the reaction mixture over 10min. The resulting yellow cloudy solution was stirred at room temperature under nitrogen. After 2h, ethanol (2ml) was added, and the reaction mixture was evaporated to dryness. The residual yellow oil was treated with toluene (100ml) and heated under gentle reflux (bath temp = 130°C). After 18h at reflux, the reaction mixture was evaporated to dryness to give a brown oil (10.594g). This oil was stirred with chloroform (50ml) and the resulting cloudy solution was filtered through a bed of celite, and the filtrate was evaporated to an orange oil (9.08g). Trituration of this oil (9.08g) with petroleum ether (50ml, bp = 40 - 60°C) did not produce any solid product. The mixture was therefore evaporated to dryness and the residual oil was dissolved in ethanol (40ml) and applied in equal portions to four aminopropyl SPE cartridges (4 x 50g). The cartridges were each eluted with ethanol (5 column volumes) followed by 10% aqueous ammonia (p = 0.88) in ethanol (5 column volumes). The product-containing fractions were

combined and evaporated to give an orange oil (8.08g). Trituration of this oil (8.08g) with petroleum ether (3 x 50ml, bp = 40 - 60˚C) gave Intermediate 31 as an orange foam (5.489g, contains 0.45 molar equivalents of excess diethyl malonate). LCMS showed $MH^+$ = 304; $T_{RET}$ = 3.43min.

**Intermediate 32 Ethyl 1-ethyl-6-(trifluoromethyl)-4-{[(trifluoromethyl)sulfonyl]oxy}-1*H*-pyrasolo[3,4-*b*]pyridine-5-carboxylate**

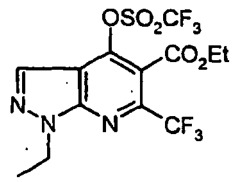

Trifluoromethanesulphonic anhydride (0.331ml, 1.96mmol) was slowly added to a stirred mixture of Intermediate 31 (0.5g, equivalent to 1.31 mmol taking into account the excess diethyl malonate) and triethylamine (0.548ml, 3.93mmol) in dry dichloromethane (10ml) at 0˚C under nitrogen. After 1.5h at 0˚C, the reaction mixture was diluted with dichloromethane (30ml) and washed with water (5ml). The phases were separated by passage through a hydrophobic frit and the organic phase was evaporated to a brown oil (0.85g). The oil (0.85g) was dissolved in dichloromethane (5ml) and applied to a silica SPE cartridge (20g). The cartridge was eluted with dichloromethane (4 column volumes). The product containing fraction was evaporated to dryness to give Intermediate 32 as a pale yellow oil (0.477g). LCMS showed $MH^+$ = 436; $T_{RET}$ = 3.90min.

**Intermediate 33 Ethyl 1-ethyl-4-(tetrahydro-2*H*-pyran-4-ylamino)-6-(trifluoromethyl)-1 H-pyrazolo[3,4-*b*]pyridine-5-carboxylate**

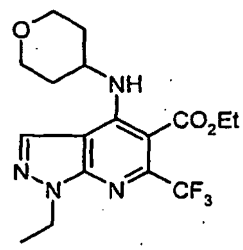

A mixture of cesium carbonate (4.744g, 14.56mmol), palladium (II) acetate (0.117g, 5mol%) and rac-2,2'-bis (diphenylphosphino)-1,1'-binaphthyl (0.486g, 7.5mol%) was placed in a 250ml round bottomed flask, and the flask was flushed with nitrogen for 5min. A solution of Intermediate 32 (4.542g, 10.4mmol) and 4-aminotetrahydropyran [e.g. available from Combi-Blocks Inc.] (2.10g, 20.76mmol) in dry dioxan (70ml) was added and the resulting mixture was stirred at room temperature under nitrogen. After 30min at room temperature, the reaction mixture was heated to 90˚C. After 1.5h at 90˚; the reaction mixture was cooled to room temperature and filtered through a Varian filter tube using dichloromethane (100ml) to wash the filter tube. The filtrate was evaporated to a brown oil (6.977g) which was dissolved in dichloromethane (30ml) and applied equally to two silica SPE cartridges (2 x 50g). The cartridges were eluted successively with ethyl acetate-cyclohexane (1/8, 2 column volumes), (1/4, 2 column volumes), (1/2, 2 column volumes), 1/1, 2 column volumes) and finally ethyl acetate (2 column volumes). The product containing fractions were combined and evaporated to give Intermediate 33 as a pale yellow crystalline solid (3.793g). LCMS showed $MH^+$ = 387; $T_{RET}$ = 3.52min.

**Intermediate 34 [1-Ethyl-4-(tetrahydro-2*H*-pyran-4-ylamino)-6-(trifluoromethyl)-1*H*-pyrazolo[3,4-b]pyridin-5-yl] methanol**

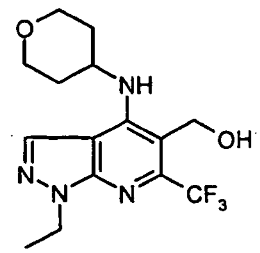

Diisobutylaluminium hydride (5.18ml; 7.77mmol, 1.5M solution in toluene) was added dropwise, over 5min, to a stirred solution of Intermediate 33 (1.or, 2.59mmol) in dry dichloromethane (10ml) at 0˚C under nitrogen. The resulting yellow solution was stirred at 0˚C for 30min. A saturated solution of potassium, sodium tartrate (7ml) was added dropwise to the reaction mixture at 0˚, with ice-bath cooling to ensure that the temperature did not rise above 3˚C. The resulting mixture was diluted with ethyl acetate (50ml) and water (5ml) and transferred to a separatory funnel. An emulsion was formed. The phases were separated and the aqueous phase was extracted with more ethyl acetate (2 x 25ml). The combined organic extracts were dried over anhydrous sodium sulphate and evaporated to give Intermediate 34 as an off-white solid (0.886g). LCMS showed $MH^+$ = 345; $T_{RET}$ = 2.76min.

**Intermediate 35 5-(Azidomethyl)-1-ethyl-*N*-(tetrahydro-2*H*-pyran-4-yl)-6-(trifluoromethyl)-1 H-pyrazolo[3,4-b] pyridin-4-amine**

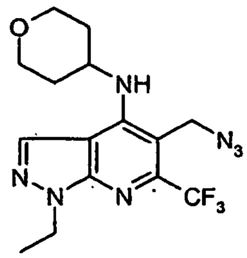

Carbon tetrabromide (5.346g, 16.12mmol) and sodium azide (1.048g, 16.12 mmol) were added to a stirred solution of Intermediate 34 (2.774g, 8.06mmol) in dry dimethylformamide (18ml) at 0-5˚C under nitrogen. The resulting mixture was stirred at 0-5˚C under nitrogen for 15min, and it was then treated dropwise, over 15min, with a solution of triphenylphosphine (4.228g, 16.12mmol) in dry dimethylformamide (18ml) whilst maintaining the reaction temperature at 0-5˚C. The resulting clear yellow solution was stirred at 0-5˚C under nitrogen for 1.5h and then allowed to warm up to room temperature overnight.

The reaction mixture was diluted with 5%-sodium hydrogen carbonate solution (75ml) and extracted with ethyl acetate (200ml). During this extraction, the insoluble triphenylphosphine oxide was removed by filtration of the two phases through a glass-fibre filter pad. The phases were then separated and the aqueous phase was extracted with more ethyl acetate (2x 200ml). The combined organic extracts were washed with saturated sodium chloride solution (75ml), then dried over anhydrous sodium sulphate and evaporated to give **Intermediate 35** as a yellow oil (9.426g) containing 2 molar equivalents of excess triphenylphospine oxide. LCMS showed $MH^+$ = 370; $T_{RET}$ = 3.29min.

**Intermediate 36 5-(Aminomethyl)-1-ethyl-*N*-(tetrahydro-2*H*-pyran-4-yl)-6-(trifluoromethyl)-1*H*-pyrazolo[3,4-*b*] pyridin-4-amine**

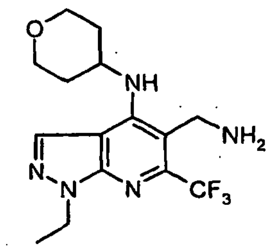

A solution of Intermediate 35 (12.56g, contains 2 molar equivalents of triphenylphoshine oxide as impurity) and 10% palladium on activated carbon (wet, 2.0g) in ethanol (200ml) was stirred under an atmosphere of hydrogen at room temperature for 40h. The reaction mixture was filtered through a bed of celite, and the filter bed was washed with ethanol (200ml). The filtrate was evaporated to dryness to give a yellow waxy solid (11.174g) which was dissolved in methanol (150ml) and applied equally to five SCX cartridges (5 x 50g). The cartridges were each eluted sequentially with methanol (4 column volumes) and 10% aqueous ammonia (p=0.88) in methanol (4 column volumes). The product-containing fractions were combined and evaporated to give Intermediate 36 as a pale yellow solid (3.108g). LCMS showed $MH^+ = 344$; $T_{RET} = 2.11$ min.

**Intermediate 37 *N*-{[1,6-diethyl-4-(tetrahydro-2*H*-pyran-4-ylamino)-1*H*-pyrazolo[3,4-*b*]pyridin-5-yl]methyl}-4-nitrobenzamide**

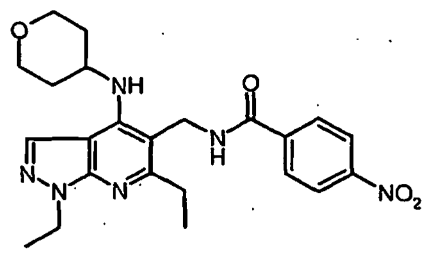

A solution of Intermediate 16 (1.0 g) in anhydrous acetonitrile (50 ml) was treated with 4-nitrobenzoyl chloride (0.62 g) and DIPEA (1 mL) and stirred at room temperature under nitrogen for 3 hours. More 4-nitrobenzoyl chloride (0.05 g) was added and stirring continued for 30 minutes. The solvent was removed by evaporation and the mixture partitioned between dichloromethane and sodium bicarbonate solution. The organics were evaporated to dryness and triturated with ether to give Intermediate 37 as a yellow solid (1.51 g). LCMS showed $MH^+ = 453$; $T_{RET} = 2.51$ min.

**Intermediate 38 *N*-{[1,6-diethyl-4-(tetrahydro-2*H*-pyran-4-ylamino)-1*H*-pyrazolo[3,4-*b*]pyridin-5-yl]methyl}-3-nitrobenzamide**

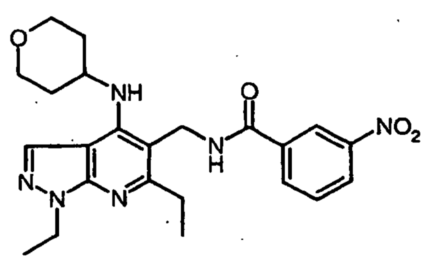

**Intermediate 38** was prepared in an analogous manner to Intermediate 37 using 3-nitrobenzoyl chloride. LCMS showed $MH^+ = 453$; $T_{RET} = 2.51$ min.

**Intermediate 52: 4-Aminotetrahydropyran**

Commercially available from: Peakdale; and/or Combi-Blocks Inc., 7949 Silverton Avenue, Suite 915, San Diego, CA 92126, USA (CAS 38041-19-9)

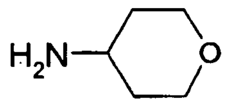

**Intermediate 52A: Tetrahydro-2H-pyran-4-amine hydrochloride = 4-Aminotetrahydropyran hydrochloride**

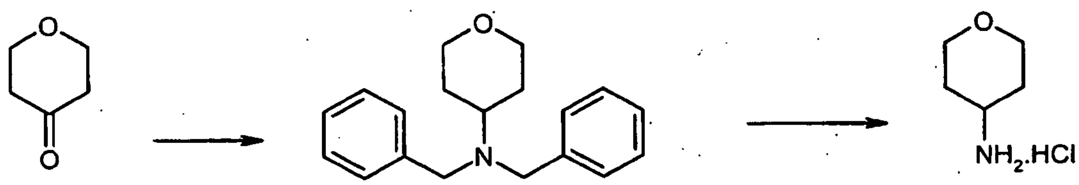

*Step1: N,N-dibenzyltetrahydro-2H-pyran-4-amine*

Dibenzylamine (34.5g) and acetic acid (6.7ml) are added to a stirred solution of tetrahydro-4H-pyran-4-one (16.4g, e.g. commercially available from Aldrich) in dichloromethane (260ml) at 0 ˚C to 5 ˚C. After 2.5h at 0 ˚C to 5 ˚C, sodium triacetoxyborohydride (38.9g) is added portionwise, and the mixture is allowed to warm to room temperature. After stirring at room temperature overnight, the reaction mixture is washed successively with 2M-sodium hydroxide (200ml and 50ml), water (2 x 50ml) and brine (50ml), then dried and evaporated. The residue (which may be an oil) is stirred with methanol (50ml) at 4 ˚C for 30min to give the product (e.g. as a white solid).

*Step 2: Tetrahydro-2H-pyran-4-amine hydrochloride*

*N,N*-dibenzyltetrahydro-2*H*-pyran-4-amine (20.5g) is dissolved in ethanol (210ml) and hydrogenated over 10% palladium on carbon catalyst (4g) at 100 psi for 72h at room temperature. The reaction mixture is filtered and the filtrate is adjusted to pH 1 with 2M-hydrogen chloride in diethyl ether. Evaporation of solvents gives a residue (which may be a solid) which is triturated with diethyl ether to give the product (which may be a solid).

**OPTIONAL INTERMEDIATES**

In this Optional Intermediates section, "Intermediates" generally represent syntheses of intermediate compounds which (in some cases) might theoretically be usable in the synthesis of compounds of formula (I) or salts thereof, but which have not necessarily been used to prepare specific compounds of formula (I) or salts thereof (and in most or all cases they have not been so used).

| Optional Intermediate Number | Structure of compound | One or two possible published reference(s) to (e.g. synthetic reference to), or one possible source of, the compound |
|---|---|---|
| 100 | 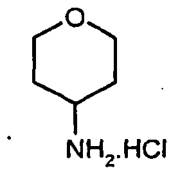 | Intermediate 8A of WO 2004/024728 A2 (Glaxo Group Limited) |
| 101 | 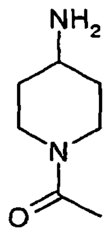 e.g. as HCl salt | Intermediate 6 of WO 2004/024728 A2 (Glaxo Group Limited), which refers to WO 00 /42011 |
| 102 | 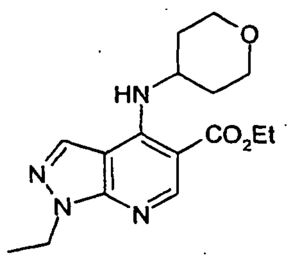 | Intermediate 32 and/or Example 3 of WO 2004/024728 A2 (Glaxo Group Limited); or Intermediate 4 of WO 2005/058892 A1 (Glaxo Group Limited) |
| 103 | 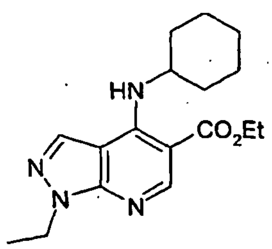 | Intermediate 5 of WO 2005/058892 A1 (Glaxo Group Limited) |
| 104 | 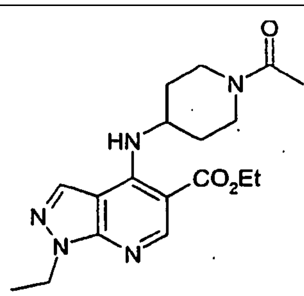 | Example 207 of WO 2004/024728 A2 (Glaxo Group Limited); or Intermediate 6 of WO 2005/058892 A1 (Glaxo Group Limited) |
| 105 | 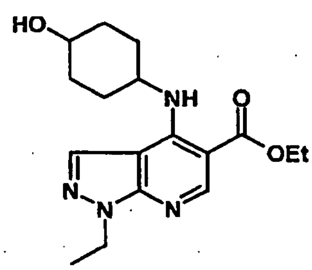 | Example 204 of WO 2004/024728 A2 (Glaxo Group Limited) |

(continued)

| Optional Intermediate Number | Structure of compound | One or two possible published reference(s) to (e.g. synthetic reference to), or one possible source of, the compound |
|---|---|---|
| 106 | 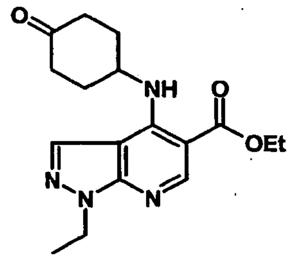 | Example 205 of WO 2004/024728 A2 (Glaxo Group Limited) |
| 107 | 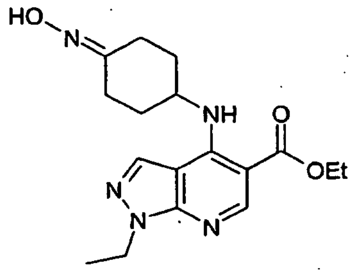 | Example 652 of WO 2004/024728 A2 (Glaxo Group Limited) |
| 108 | 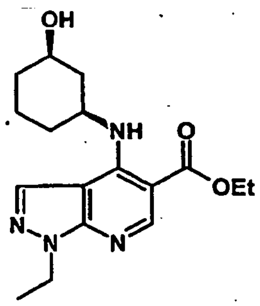 [*cis*-(3-hydroxycyclohex-1-yl)amino group, racemic] | Intermediate 12 of WO 2005/058892 A1 (Glaxo Group Limited) |
| 109 | 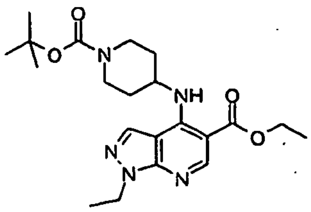 | Intermediate 102 of WO 2005/058892 A1 (Glaxo Group Limited) |
| 110 | 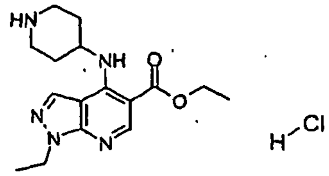 | Intermediate 103 of WO 2005/058892 A1 (Glaxo Group Limited) |

(continued)

| Optional Intermediate Number | Structure of compound | One or two possible published reference(s) to (e.g. synthetic reference to), or one possible source of, the compound |
|---|---|---|
| 111 | 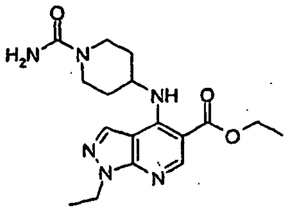 | Intermediate 104 of WO 2005/058892 A1 (Glaxo Group Limited) |
| 112 | 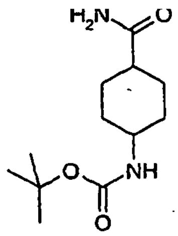 | Intermediate 112 of WO 2005/058892 A1 (Glaxo Group Limited) |
| 113 | 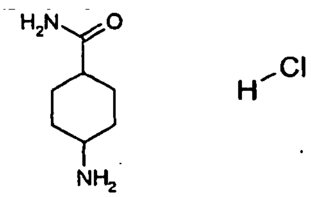 | Intermediate 113 of WO 2005/058892 A1 (Glaxo Group Limited) |
| 114 | 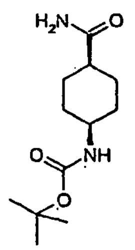 | Intermediate 114 of WO 2005/058892 A1 (Glaxo Group Limited) |
| 115 | 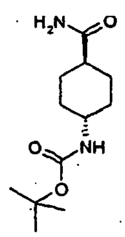 | Intermediate 115 of WO 2005/058892 A1 (Glaxo Group Limited) |
| 116 | 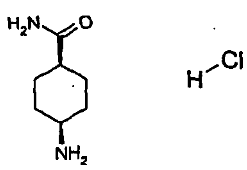 | Intermediate 116 of WO 2005/058892 A1 (Glaxo Group Limited) |

(continued)

| Optional Intermediate Number | Structure of compound | One or two possible published reference(s) to (e.g. synthetic reference to), or one possible source of, the compound |
|---|---|---|
| 117 | 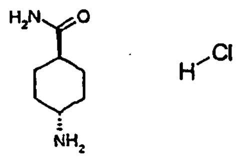 | Intermediate 117 of WO 2005/058892 A1 (Glaxo Group Limited) |
| 118 | 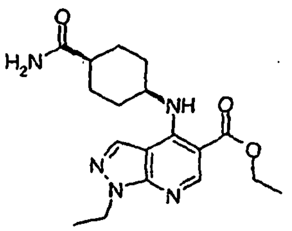 | Intermediate 118 of WO 2005/058892 A1 (Glaxo Group Limited) |
| 119 | 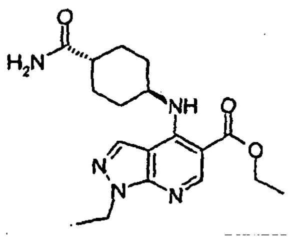 | Intermediate 119 of WO 2005/058892 A1 (Glaxo Group Limited) |
| 120 | 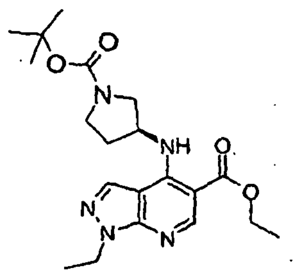 | Intermediate 146 of WO 2005/058892 A1 (Glaxo Group Limited) |
| 121 | 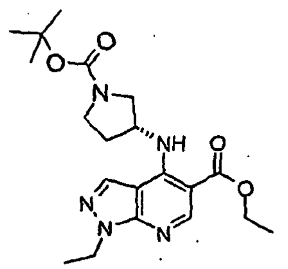 | Intermediate 147 of WO 2005/058892 A1 (Glaxo Group Limited) |
| 122 | 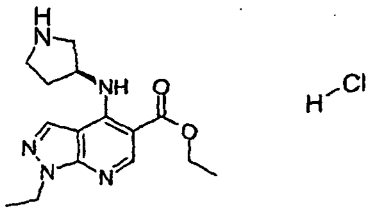 | Intermediate 148 of WO 2005/058892 A1 (Glaxo Group Limited) |

(continued)

| Optional Intermediate Number | Structure of compound | One or two possible published reference(s) to (e.g. synthetic reference to), or one possible source of, the compound |
|---|---|---|
| 123 | 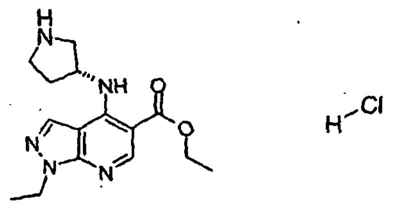 | Intermediate 149 of WO 2005/058892 A1 (Glaxo Group Limited) |
| 124 | 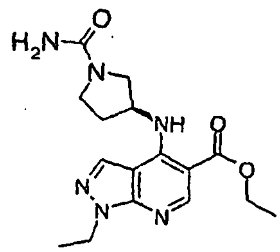 | Intermediate 150 of WO 2005/058892 A1 (Glaxo Group Limited) |
| 125 | 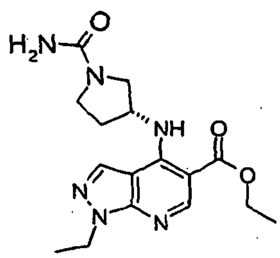 | Intermediate 151 of WO 2005/058892 A1 (Glaxo Group Limited) |
| 126 | 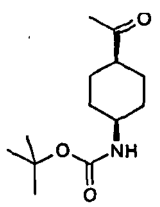 | Intermediate 155 of WO 2005/058892 A1 (Glaxo Group Limited) |
| 127 | 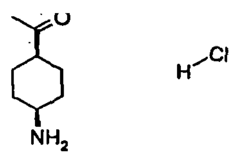 | Intermediate 156 of WO 2005/058892 A1 (Glaxo Group Limited) |
| 128 | 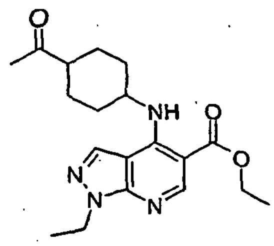 (mixture of *cis* and *trans* isomers) | Intermediate 157 of WO 2005/058892 A1 (Glaxo Group Limited) |

(continued)

| Optional Intermediate Number | Structure of compound | One or two possible published reference(s) to (e.g. synthetic reference to), or one possible source of, the compound |
|---|---|---|
| 129 | 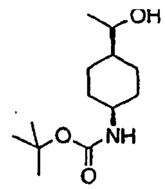 | Intermediate 159 of WO 2005/058892 A1 (Glaxo Group Limited) |
| 130 | 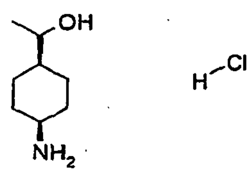 | Intermediate 160 of WO 2005/058892 A1 (Glaxo Group Limited) |
| 131 | 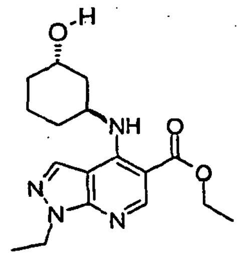 [contains *trans*-(3-hydroxycyclohex-1-yl)amino group, racemic] | Intermediate 161 of WO 2005/058892 A1 (Glaxo Group Limited) |
| 132 | 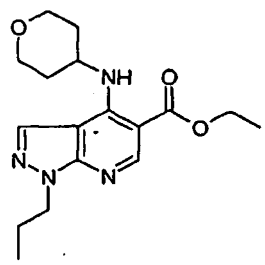 | Example 185 of WO 2004/024728 A2 (Glaxo Group Limited); or Intermediate 171 of WO 2005/058892 A1 (Glaxo Group Limited) |
| 133 | 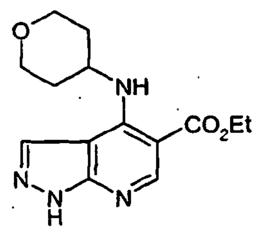 | Example 20 of WO 2004/024728 A2 (Glaxo Group Limited) |

(continued)

| Optional Intermediate Number | Structure of compound | One or two possible published reference(s) to (e.g. synthetic reference to), or one possible source of, the compound |
|---|---|---|
| 134 | 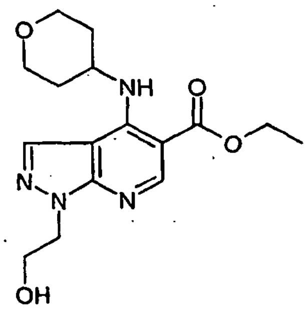 | Example 186 of WO 2004/024728 A2 (Glaxo Group Limited) |
| 135 | 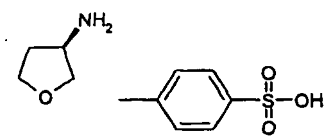 | Fluka Chemie AG, Germany (CAS 111769-27-8) |
| 136 | 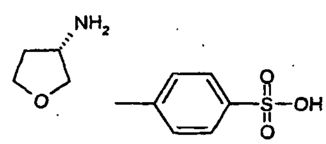 | E. Merck, Germany; or E. Merck (Merck Ltd), Hunter Boulevard, Magna Park, Lutterworth, Leicestershire LE17 4XN, United Kingdom (CAS 104530-80-5) |
| 137 | 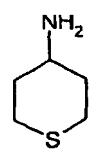 | Intermediate 11 of WO 2004/024728 A2, and optionally reference cited therein |
| 138 | 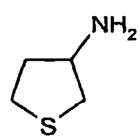 | Intermediate 12 of WO 2004/024728 A2, and optionally references cited therein |
| 139 | 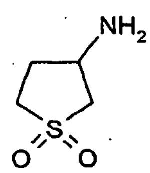 | Sigma Aldrich Library of Rare Chemicals (SALOR) (CAS-6338-70-1) |
| 140 | 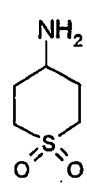 | Intermediate 14 of WO 2004/024728 A2, and optionally references cited therein |

(continued)

| Optional Intermediate Number | Structure of compound | One or two possible published reference(s) to (e.g. synthetic reference to), or one possible source of, the compound |
|---|---|---|
| 141 | 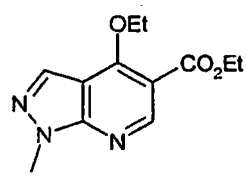 | Intermediate 3 of WO 2004/024728 A2 (Glaxo Group Limited) |
| 142 | 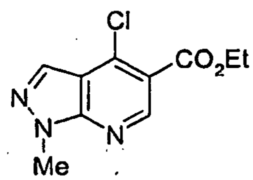 | Intermediate 25 of WO 2004/024728 A2 (Glaxo Group Limited) |
| 143 | 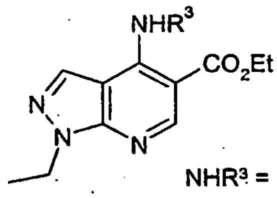 NHR³ = 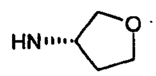 | Example 8 of WO 2004/024728 A2 (Glaxo Group Limited) |
| 144 | 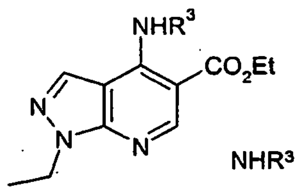 NHR³ = 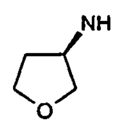 | Example 9 of WO 2004/024728 A2 (Glaxo Group Limited) |
| 145 | 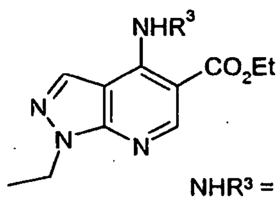 NHR³ = 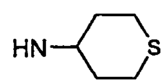 | Example 10 of WO 2004/024728 A2 (Glaxo Group Limited) |
| 146 | 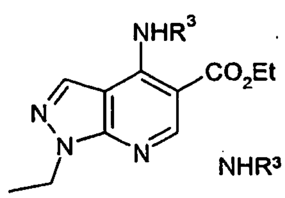 NHR³ = 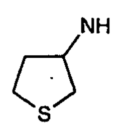 | Example 11 of WO 2004/024728 A2 (Glaxo Group Limited) |
| 147 | 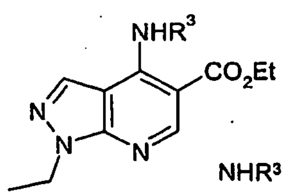 NHR³ = 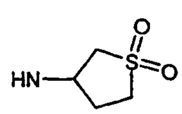 | Example 13 of WO 2004/024728 A2 (Glaxo Group Limited) |

(continued)

| Optional Intermediate Number | Structure of compound | One or two possible published reference(s) to (e.g. synthetic reference to), or one possible source of, the compound |
|---|---|---|
| 148 | 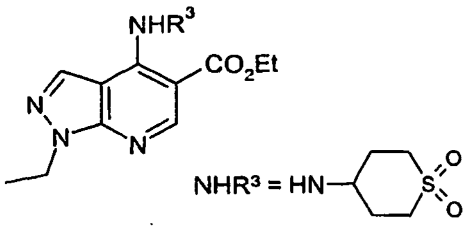 | Example 14 of WO 2004/024728 A2 (Glaxo Group Limited) |
| 149 | 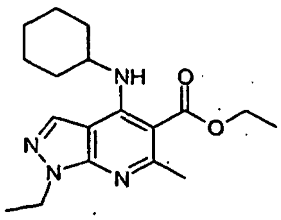 | Example 190 of WO 2004/024728 A2 (Glaxo Group Limited) |
| 150 | 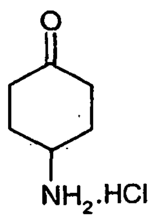 | Intermediate 54 of WO 2004/024728 A2 (Glaxo Group Limited) |
| 151 | 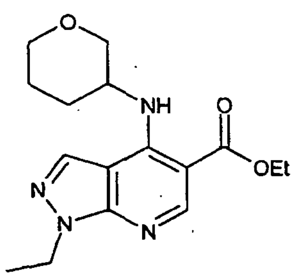 | Intermediate 58A of WO 2004/024728 A2 (Glaxo Group Limited) |
| 152 | 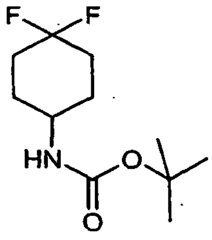 [sample containing 1,1-dimethylethyl (4-fluoro-3-cyclohexen-1-yl)carbamate as an impurityl | Intermediate 62 of WO 2004/024728 A2 (Glaxo Group Limited) |

(continued)

| Optional Intermediate Number | Structure of compound | One or two possible published reference(s) to (e.g. synthetic reference to), or one possible source of, the compound |
|---|---|---|
| 153 | 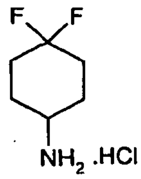 [sample containing 4-fluoro-3-cyclohexen-1-amine) as an impurity] | Intermediate 63 of WO 2004/024728 A2 (Glaxo Group Limited) |
| 154 | 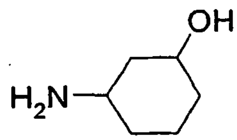 | AB Chem, Inc., Canada (mixture of *cis* and *trans*); or *J. Chem. Soc., Perkin Trans. 1,* 1994, 537 |
| 155 | as Intermediate 154, but racemic cis-somer, i.e. racemic cis-(3-hydroxy-cyclohex-1-yl)-amine | *J. Chem. Soc.*, *Perkin Trans 1,* 1994,537 (discloses a 3.3 : 1 *cis : trans* mixture) |
| 156 | 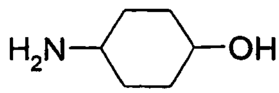 | Aldrich; or TCI-America |
| 157 | 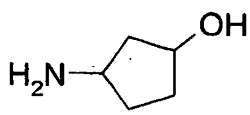 | US patent 4,219,660 |
| 158 | 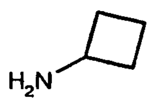 | Aldrich |
| 159 | 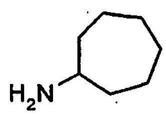 | Aldrich |
| 160 | 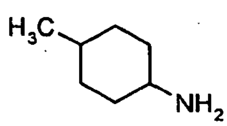 | Aldrich |
| 161 | 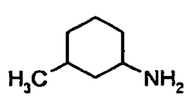 | Pfaltz-Bauer |
| 162 | 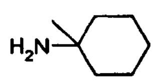 | *J. Org. Chem.,* 1985, 50(11), 1859 |
| 163 | 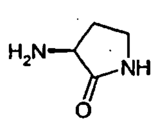 | WO 99/12933 |

(continued)

| Optional Intermediate Number | Structure of compound | One or two possible published reference(s) to (e.g. synthetic reference to), or one possible source of, the compound |
|---|---|---|
| 164 | 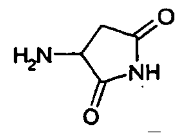 | EP 1188744 |
| 165 | 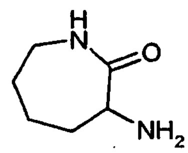 (3-Aminoazepan-2-one) | Sigma-Aldrich Company Ltd |
| 166 * | 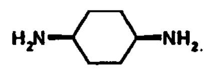 | *J. Med. Chem.*, 1994, 37(17), 2360 |
| 167 * | 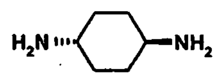 | Aldrich |
| 168 * | 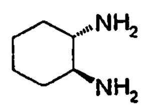 (*trans* isomer, e.g. optionally racemic) | Aldrich |
| 169 | 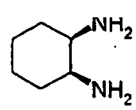 | Aldrich |
| 170 | 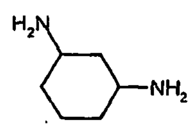 | Peakdale Molecular Ltd |
| 171 | 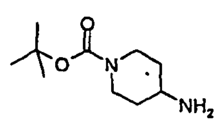 1,1-dimethylethyl 4-amino-1-piperidinecarboxylate | AstaTech |
| 172 | 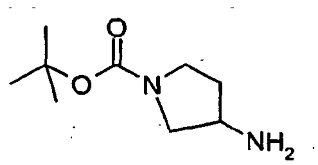 | |
| 173 | 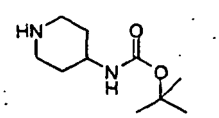 1,1-dimethylethyl 4-piperidinecarbamate | Syngene or AstaTech |

(continued)

| Optional Intermediate Number | Structure of compound | One or two possible published reference(s) to (e.g. synthetic reference to), or one possible source of, the compound |
|---|---|---|
| 174 | 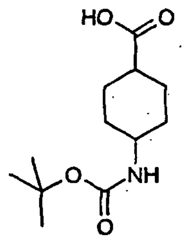<br>4-({[(1,1-dimethylethyl)oxy]carbonyl}amino) cyclohexane carboxylic acid | Fluka |
| 175 | 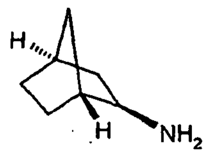 | Aldrich |
| 176 | 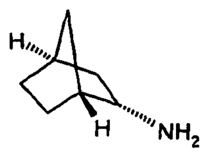 | Aldrich |

## EXAMPLES

### Examples 1 to 77

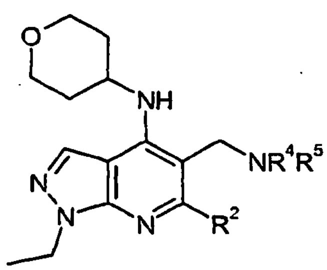

$R^2$ = H or Me

### General Procedure for Examples 1 to 46

A solution of the amine or amine hydrochloride $HNR^4R^5$ (0.15mmol) in DMF (0.5ml) was added to a mixture of Intermediate 6 or 7 (0.1 mmol) and DIPEA (0.02ml) in DMF (0.5ml). (If the amine hydrochloride was used, an additional portion of DIPEA (0.02ml) was added). After heating at 70˚C 80˚C for 18h, the reaction mixture was concentrated *in vacuo* and the residue dissolved in methanol (0.5ml) and applied to a SPE cartridge (0.5g; SCX2). The cartridge was eluted sequentially with methanol (1.5ml) and 2M-ammonia in methanol (1.5ml). Fractions containing the desired product were concentrated *in vacuo* and where necessary the residue was purified by mass directed autoprep HPLC.

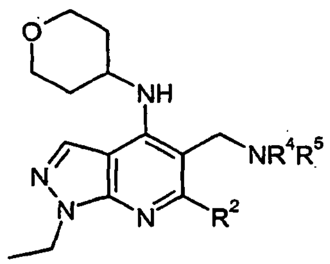

R² = H or Me

| Example Number | $R^2$ | $NHR^4R^5$ | One possible source of or reference for $NHR^4R^5$ | Starting Material | MH+ ion | LC-MS Retention Time (min) |
|---|---|---|---|---|---|---|
| 1 (TFA salt) | Me | 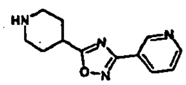 | Peakdale Molecular Ltd | Intermediate 7 | 503 | 2.35 |
| 2 (TFA salt) | Me | 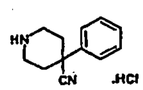 | Aldrich Chemical Company, Inc. | Intermediate 7 | 459 | 2.61 |
| 3 (TFA salt) | Me | 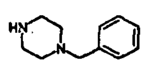 | Lancaster Synthesis Ltd. | Intermediate 7 | 449 | 1.87 |
| 4 (TFA salt) | Me | 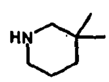 | Manchester Organics Ltd | Intermediate 7 | 386 | 2.3 |
| 5 (TFA salt) | Me | 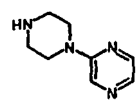 | Apollo Scientific Ltd. | Intermediate 7 | 437 | 2.19 |
| 6 (TFA salt) | Me | 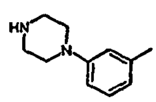 | Apollo Scientific Ltd. | Intermediate 7 | 449 | 2.58 |
| 7 (TFA salt) | Me | 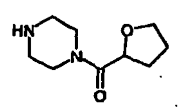 | Lancaster Synthesis Ltd. | Intermediate 7 | 457 | 2.06 |
| 8 (TFA salt) | Me | 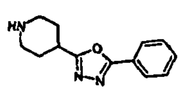 | Peakdale Molecular Ltd | Intermediate 7 | 502 | 2.43 |
| 9 (TFA salt) | Me | 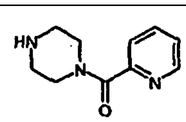 | WO 03/057676 | Intermediate 7 | 464 | 2.07 |
| 10 (TFA salt) | H | 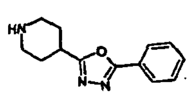 | Peakdale Molecular Ltd | Intermediate 6 | 488 | 2.28 |
| 11 (TFA salt) | H | 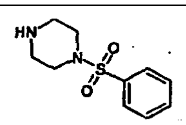 | TimTec Corporation | Intermediate 6 | 485 | 2.48 |

(continued)

| Example Number | $R^2$ | $NHR^4R^5$ | One possible source of or reference for $NHR^4R^5$ | Starting Material | MH+ ion | LC-MS Retention Time (min) |
|---|---|---|---|---|---|---|
| 12 (TFA salt) | H | 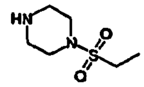 | Apollo Scientific Ltd. | Intermediate 6 | 437 | 2.12 |
| 13 (TFA salt) | Me | 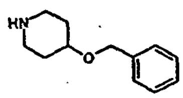 | Peakdale Molecular Ltd | Intermediate 7 | 464 | 2.39 |
| 14 (TFA salt) | H | 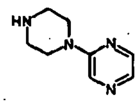 | Apollo Scientific Ltd. | Intermediate 6 | 423 | 2.13 |
| 15 (TFA salt) | H | 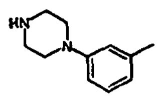 | Apollo Scientific Ltd. | Intermediate 6 | 435 | 2.5 |
| 16 (TFA salt) | H | 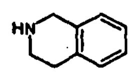 | Aldrich Chemical Company, Inc. | Intermediate 6 | 392 | 2.23 |
| 17 (TFA salt) | H | 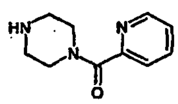 | WO 03/057676 | Intermediate 6 | 450 | 2.05 |
| 18 (TFA salt) | H | 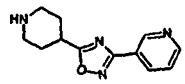 | Peakdale Molecular Ltd | Intermediate 6 | 489 | 2.19 |
| 19 (TFA salt) | H | 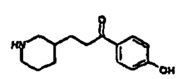 | Not known | Intermediate 6 | 492 | 2.24 |
| 20 (TFA salt) | Me | 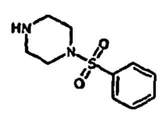 | TimTec Corporation | Intermediate 7 | 499 | 2.42 |
| 21 (TFA salt) | H | 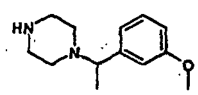 | Micro Chemistry Building blocks | Intermediate 6 | 479 | 1.99 |
| 23 (TFA satt) | H | 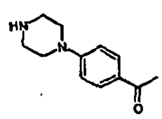 | Aldrich Chemical Company, Inc. | Intermediate 6 | 463 | 2.38 |
| 24 (TFA salt) | H | 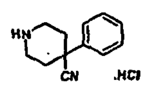 | Aldrich Chemical Company, Inc. | Intermediate 6 | 445 | 2.56 |
| 25 (TFA salt) | H | 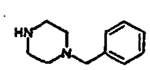 | Lancaster Synthesis Ltd. | Intermediate 6 | 435 | 1.9 |
| 26 (TFA salt) | H | 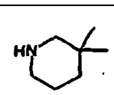 | Manchester Organics Ltd | Intermediate 6 | 372 | 2.08 |

(continued)

| Example Number | $R^2$ | $NHR^4R^5$ | One possible source of or reference for $NHR^4R^5$ | Starting Material | MH+ ion | LC-MS Retention Time (min) |
|---|---|---|---|---|---|---|
| 27 (TFA salt) | Me | 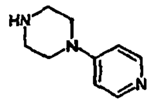 | Lancaster Synthesis Ltd. | Intermediate 7 | 436 | 1.79 |
| 28 (TFA salt) | H | 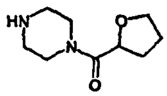 | Lancaster Synthesis Ltd. | Intermediate 6 | 443 | 2.00 |
| 29 (TFA salt) | H | 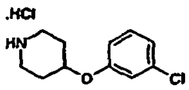 | Array Biopharma Inc. | Intermediate 6 | 470 | 2.51 |
| 31 (TFA salt) | H | 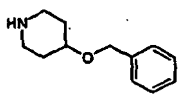 | Peakdale Molecular Ltd | Intermediate 6 | 450 | 2.28 |
| 32 (TFA salt) | Me | 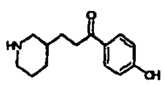 | Not known | Intermediate 7 | 506 | 2.29 |
| 33 (TFA salt) | Me | 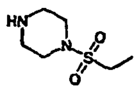 | Apollo Scientific Ltd. | Intermediate 7 | 451 | 2.15 |
| 34 (TFA salt) | H | 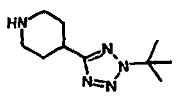 | Manchester Organics | Intermediate 6 | 468 | 2.31 |
| 35 (TFA salt) | H | 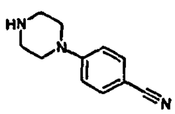 | Apin Chemicals Ltd. | Intermediate 6 | 446 | 2.58 |
| 36 (TFA salt) | H | 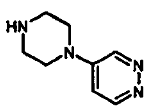 | WO 2003/104225 | Intermediate 6 | 423 | 2.33 |
| 37 (TFA salt) | H | 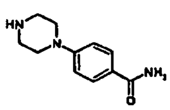 | Davos Chemical Corporation | Intermediate 6 | 464 | 2.17 |
| 38 (TFA salt) | H | 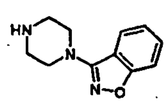 | J. Med. Chem., (1986), 29(3), 359-69 | Intermediate 6 | 462 | 2.57 |
| 39 (TFA salt) | Me | 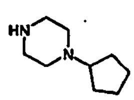 | Apollo Scientific Ltd. | Intermediate 7 | 427 | 2.26 |
| 40 (Formate) | Me | 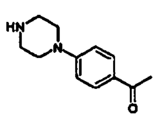 | Aldrich Chemical Company, Inc. | Intermediate 7 | 477 | 2.49 |

(continued)

| Example Number | $R^2$ | $NHR^4R^5$ | One possible source of or reference for $NHR^4R^5$ | Starting Material | MH+ ion | LC-MS Retention Time (min) |
|---|---|---|---|---|---|---|
| 41 (Formate) | Me | 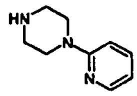 | Aldrich Chemical Company, Inc. | Intermediate 7 | 436 | 1.97 |
| 42 (Formate) | Me | 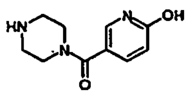 | Not known | Intermediate 7 | 480 | 2.02 |
| 43 (Formate) | Me | 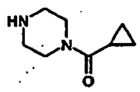 | WO 2002/032867 | Intermediate 7 | 427 | 2.12 |
| 44 (Formate) | Me | 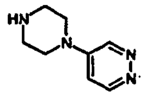 | 2003/104225 | Intermediate 7 | 437 | 2.15 |
| 45 (Formate) | Me | 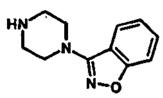 | J. Med. Chem., (1986), 29(3), 359-69 | Intermediate 7 | 476 | 2.59 |
| 46 (Formate) | Me | 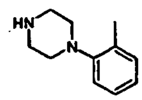 | Lancaster Synthesis Ltd. | Intermediate 7 | 449 | 2.79 |

The following **Examples 47 - 77** were prepared by an analogous procedure to that described above adding a solution of the amine $HNR^4R^5$ or amine hydrochloride (0.06mmol) in DMF (0.14ml) to a solution of Intermediate 6 or 7 (0.05mmol) and DIPEA (0.015ml) in DMF (0.5ml).

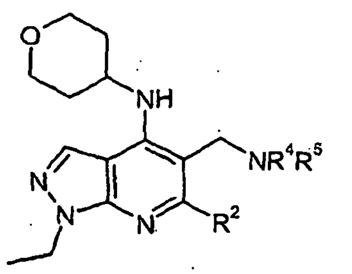

$R^2$ = H or Me

| Example Number | $R^2$ | $NHR^4R^5$ | One possible source of or reference for $NHR^4R^5$ | Starting Material | MH+ ion | LC-MS Retention time (Min) |
|---|---|---|---|---|---|---|
| 47 (TFA salt) | H | 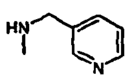 | Maybridge Chemical Company Ltd. | Intermediate 6 | 381 | 1.94 |

(continued)

| Example Number | $R^2$ | $NHR^4R^5$ | One possible source of or reference for $NHR^4R^5$ | Starting Material | MH+ ion | LC-MS Retention time (Min) |
|---|---|---|---|---|---|---|
| 48 (TFA salt) | H | 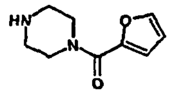 | Lancaster Synthesis Ltd. | Intermediate 6 | 439 | 2.18 |
| 50 (TFA salt) | H | 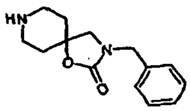 | Peakdale Molecular Ltd | Intermediate 6 | 505 | 2.0 |
| 52 (TFA salt) | H | 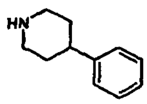 | Peakdale Molecular Ltd | Intermediate 6 | 420 | 2.33 |
| 54 (TFA salt) | H | 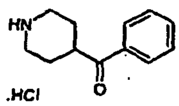 | Maybridge Chemical Company Ltd. | Intermediate 6 | 448 | 2.29 |
| 55 (TFA salt) | H | 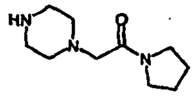 | Apollo Scientific Ltd. | Intermediate 6 | 456 | 1.83 |
| 59 (TFA salt) | H | 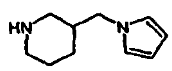 | Maybridge Chemical Co | Intermediate 6 | 423 | 2.01 |
| 60 (TFA salt) | Me | 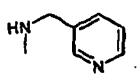 | Maybridge Chemical Company Ltd. | Intermediate 7 | 395 | 1.98 |
| 61 (TFA salt) | Me | 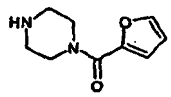 | Lancaster Synthesis Ltd. | Intermediate 7 | 453 | 2.21 |
| 63 (TFA salt) | Me | 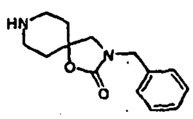 | Peakdale Molecular Ltd | Intermediate 7 | 519 | 2.48 |
| 65 (TFA salt) | Me | 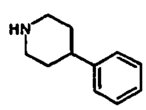 | Peakdale Ltd Molecular Ltd | Intermediate 7 | 434 | 2.45 |
| 67 (TFA salt) | Me | 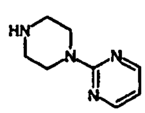 | Aldrich Chemical Company, Inc. | Intermediate 7 | 437 | 1.89 |
| 71 (TFA salt) | H | 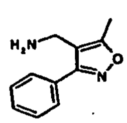 | Maybridge Chemical Co | Intermediate 6 | 447 | 1.84 |
| 77 (TFA salt) | Me | 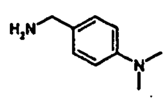 | Peakdale Molecular Ltd | Intermediate 7 | 423 | 1.61 |

**Example 78 (1-{[1,6-diethyl-4-(tetrahydro-2H-pyran-4-ylamino)-1H-pyrazolo[3,4-b]pyridin-5-yl]methyl}-4-piperidinyl)(phenyl)methanone**

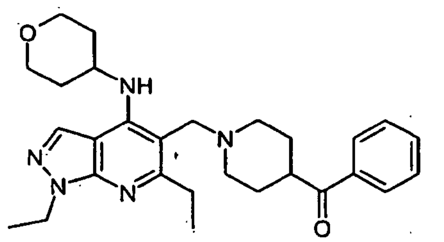

A solution containing Intermediate 5 (120mg) in thionyl chloride (1.5ml) was heated at 80°C for 1.5h and then evaporated to dryness *in vacuo.* The residue was co-evaporated with toluene (5ml) and dissolved in acetonitrile (1.5ml). A portion of this solution (0.5ml) was added dropwise to a solution of 4-benzoylpiperidine hydrochloride [e.g. available from *Maybridge Chemical Company Ltd*]. (118mg) and DIPEA (0.092ml) in acetonitrile (1.5ml) and stirred at room temperature for 0.5h. The mixture was purified by SPE cartridge (silica) eluting with 25 - 33% ethyl acetate in cyclohexane followed by a second' cartridge eluting with 0 - 1.5%. methanol in chloroform to give Example 78 as a gum (52mg). LCMS showed $MH^+$ = 476; $T_{RET}$ = 2.47min.

**Example 79 1,6-diethyl-5-{[(phenylmethyl)amino]methyl}-N-(tetrahydro-2H-pyran-4-yl)-1H-pyrazolo[3,4-b]pyridin-4-amine**

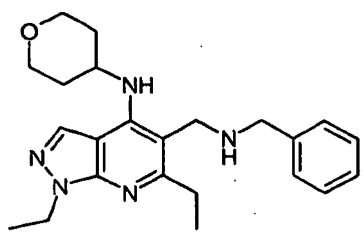

Example 79 was prepared in an analogous manner to Example 78 from Intermediate 5 and benzylamine. LCMS showed $MH^+$= 394; $T_{RET}$ = 2.02min.

**Example 80 5-[(([4-(dimethylamino)phenyl]methyl)amino)methyl]-1,6-diethyl-N-(tetrahydro-2H-pyran-4-yl)-1H-pyrazolo[3,4-b]pyridin-4-amine**

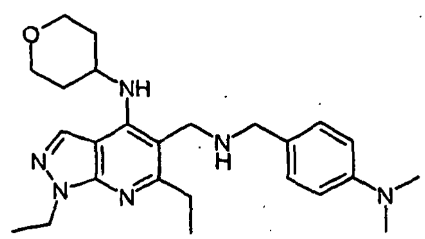

Example 80 was prepared in an analogous manner to Example 78 from Intermediate 5 using 4-dimethylaminobenzylamine, dihydrochloride [e.g. available from *Aldrich Chemical Company, Inc*.] and DIPEA. LCMS showed $MH^+$ = 437; $T_{RET}$ = 2.08min.

**Example.81 5-[({[4-(dimethylamino)phenyl]methyl}amino)methyl]-1-ethyl-N-(tetrahydro-2H-pyran-4-yl)-1H-pyrazolo[3,4-b]pyridin-4-amine**

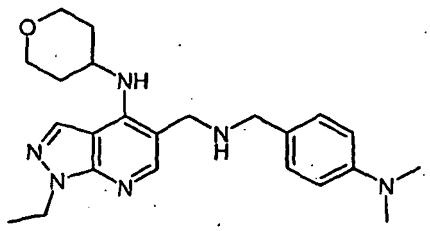

A solution containing Intermediate 6 (100mg) in acetonitrile (3.5ml) was added to a suspension containing 4-dimethylaminbenzylamine dihydrochloride (269mg) and DIPEA (0.428ml) in acetonitrile (3.5ml) and the mixture was stirred at room temperature for 2.5h. The mixture was diluted with dichloromethane (5ml) and applied to an SPE cartridge (silica; 10g) and eluted with 50 - 100% ethyl acetate in cyclohexane to give Example 81 as a gum (10mg). LCMS showed $MH^+ = 409$; $T_{RET} = 1.97$min.

**Example 82 1-ethyl-5-{[(phenylmethyl)amino]methyl}-N-(tetrahydro-2H-pyran-4-yl)-1H-pyrazolo[3,4-b]pyridin-4-amine**

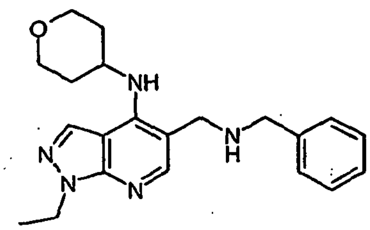

A solution containing Intermediate 6 (50mg) and benzylamine (0.06ml) in acetonitrile (0.5ml) was stirred at room temperature for 22h. The solution was blown down to dryness and the residue dissolved in dichloromethane (2ml), applied to an SPE cartridge (silica; 5g) and eluted sequentially with dichloromethane, chloroform, ether and ethyl acetate to give Example 82 as a gum (20mg). LCMS showed $MH^+ = 366$; $T_{RET} = 1.98$min.

**Example 83 1-ethyl-6-methyl-5-{[(phenylmethyl)amino]methyl}-N-(tetrahydro-2H-pyran-4-yl)-1H-pyrazolo [3,4-b]pyridin-4-arhine**

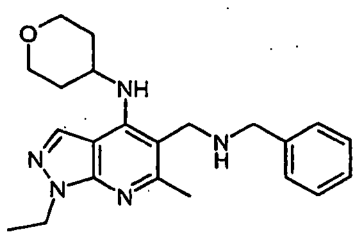

A solution containing Intermediate 4 (50mg) in thionyl chloride (0.5ml) was heated at 80˚C for 1.5h and then evaporated to dryness *in vacuo.* The residue was dissolved in acetonitrile (1.0ml) and treated with benzylamine (0.27ml) and the mixture stirred at room temperature for 20h. The mixture was purified by SPE cartridge (SCX2) eluting with methanol to remove impurities and then with 10% ammonia in methanol to give crude product. Further purification using

an SPE cartridge (silica) eluting with ethyl acetate followed by a second cartridge eluting with 50% ethyl acetate in cyclohexane gave Example 83 as a gum (21 mg). LCMS showed $MH^+$ = 380; $T_{RET}$ = 1.88min.

**Example 87 *N*-{[1-ethyl-4-(tetrahydro-2H-pyran-4-ylamino)-1H-pyrazolo[3,4-b]pyridin-5-yl]methyl}benzamide**

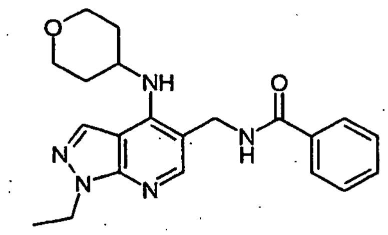

A solution of Intermediate 14 (16mg) in anhydrous acetonitrile (0.5ml) was treated with benzoyl chloride (0.01ml) and DIPEA (0.016m) and stirred at room temperature for 20 hours. The solution was blown to dryness and the residue was purified by mass directed autoprep HPLC to give Example 87 as a colourless gum (12mg). LCMS showed $MH^+$ = 380; $T_{RET}$ = 2.25min.

**Example 88 4-(dimethylamino)-N-{[1-ethyl-4-(tetrahydro-2H-pyran-4-ylamino)-1H-pyrazolo[3,4-b]pyridin-5-yl] methyl}benzamide**

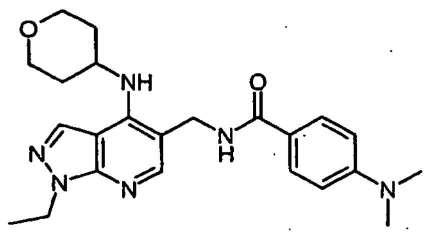

Example 88 was prepared in an analogous manner to Example 87 from Intermediate 14 and 4-(dimethylamino) benzoyl chloride [e.g. available from *Lancaster Synthesis Ltd].* LCMS showed $MH^+$ = 423; $T_{RET}$ = 2.38min.

**Example 89 N-{[1-ethyl-4-(tetrahydro-2H-pyran-4-ylamino)-1H-pyrazolo[3,4-b]pyridin-5-yl]methyl}benzenesulfonamide**

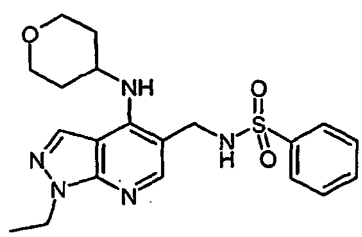

Example 89 was prepared in an analogous manner to Example 87 from Intermediate 14 and benzenesulphonyl chloride. LCMS showed $MH^+$ = 416; $T_{RET}$ = 2.27min.

**Example 90 N-{[1-ethyl-4-(tetrahydro-2H-pyran-4-ylamino)-1H-pyrazolo[3,4-b]pyridin-5-yl]methyl}-5-isoxazolecarboxamide**

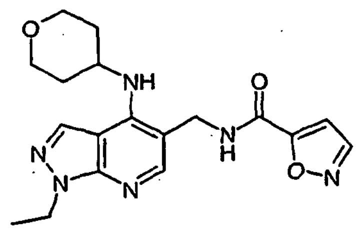

A solution of Intermediate 14 (20mg) in anhydrous acetonitrile (0.4ml) was treated with isoxazole-5-carbonyl chloride [e.g. available from *Lancaster Synthesis Ltd.]* (13mg) and DIPEA (0.016ml) and stirred at room temperature for 24 hours. The solution was blown to dryness and the residue was purified by mass directed autoprep HPLC to give Example 90 as a clear colourless gum (12mg). LCMS showed $MH^+$ = 371; $T_{RET}$ = 2.03min.

**Example 91 N-{[1,6-diethyl-4-(tetrahydro-2H-pyran-4-ylamino)-1H-pyrazolo[3,4-b]pyridin-5-yl]methyl}-5-isoxazolecarboxamide**

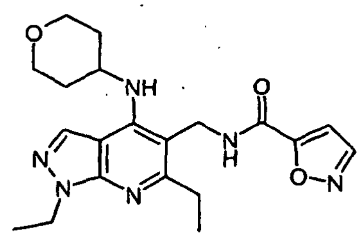

A solution of Intermediate 16 (75mg) in anhydrous acetonitrile (1.25ml) was treated with isoxazole-5-carbonyl chloride (33mg) and DIPEA (0.044ml) and stirred at room temperature for 24 hours. The solution was diluted with dichloromethane (10ml), washed with dilute aqueous sodium chloride (2 x 7ml) and evaporated *in* vacuo to give Example 91 as a yellow sold (104mg). LCMS showed $MH^+$ = 399; $T_{RET}$ = 1.98min.

**Example 92 N-{[1-ethyl-4-(tetrahydro-2H-pyran-4-ylamino)-1H-pyrazolo[3,4-b]pyridin-5-yl]methyl}-4-pyridinecarboxamide**

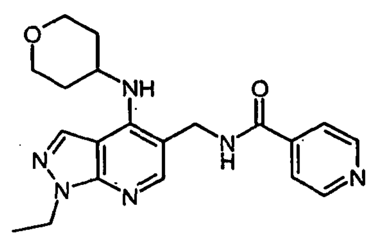

A solution of Intermediate 14 (52mg) in anhydrous acetonitrile (1.5ml) was treated with isonicotinoyl chloride, hydrochloride salt [e.g. available from *Aldrich Chemical Company, Inc*.] (37mg) and DIPEA (0.073ml) and stirred at room temperature for 3 days. The solution was blown down to dryness and the residue dissolved in dichloromethane (1ml), applied to an SPE cartridge (silica; 5g) and eluted with 0.5% methanol in ethyl acetate to give Example 92 as a white sold (64mg). LCMS showed $MH^+$ = 381; $T_{RET}$ = 2.04min.

**Example 93 4-(acetytamino)-N-{[1-ethyl-6-methyl-4-(tetrahydro-2H-pyran-4-ylamino)-1H-pyrazolo[3,4-b]pyridin-5-yl]methyl}benzamide**

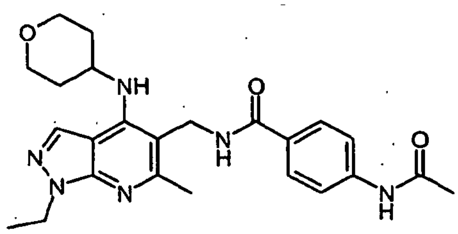

A solution of Intermediate 15 (77mg) in anhydrous acetonitrile (1.25ml) was treated with 4-acetylaminobenzoyl chloride [e.g. available from *Lancaster Synthesis Ltd*.] (47mg) and DIPEA (0.042ml) and stirred at room temperature for 24h. The solution was diluted with dichloromethane (5ml), applied to an SPE cartridge (silica; 10g) and eluted with 0 - 2% methanol in ethyl acetate to give Example 93 as a yellow sold (69mg). LCMS showed $MH^+ = 451$; $T_{RET} = 2.05$min.

**Example 94 N-{[1-ethyl-6-methyl-4-(tetrahydro-2H-pyran-4-ylamino)-1H-pyrazolo[3,4-b]pyridin-5-yl]methyl}-5-isoxazolecarboxamide**

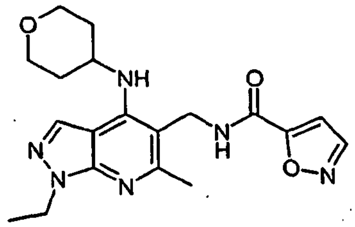

A solution of Intermediate 15 (77mg) in anhydrous acetonitrile (1.25ml) was treated with isoxazole-5-carbonyl chloride (32mg) and DIPEA (0.042ml) and stirred at room temperature for 24h. The solution was diluted with dichloromethane (5ml), applied to an SPE cartridge (silica; 10g) and eluted with 50 - 100% ethyl acetate in cyclohexane to give Example 94 as a yellow sold (63mg). LCMS showed $MH^+ = 385$; $T_{RET} = 1.92$ min.

**Examples 95 to 109**

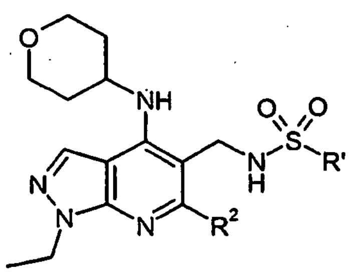

**$R^2$ = H or Me**

**General Procedure**

The sulphonyl chloride $R'SO_2Cl$ (0.12mmol) was dissolved in acetonitrile (0.5ml). [Where compounds did not fully dissolve, DIPEA (0.12mmol) was added]. The solution was treated with a solution of Intermediate 14 or 15 (0.1mmol) in acetonitrile (0.5ml) [containing a few drops of DMF to aid solubility] and DIPEA (0.15mmol). The solution was stirred

at room temperature for 18h and concentrated *in vacuo.* The residue was dissolved in chloroform (0.5ml) and applied to an SPE cartridge (0.5g; Flash $NH_2$). The cartridge was eluted sequentially with chloroform (1.5ml), ethyl acetate (1.5ml) and 20% methanol in ethyl acetate (1.5ml). Fractions containing the desired product were concentrated *in* vacuo and where necessary the residue was purified by mass directed autoprep HPLC.

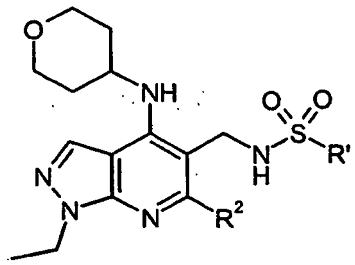

$R^2$ = H or Me

| Example Number | $R^2$ | R'$SO_2$Cl | One possible source of or reference for R'$SO_2$Cl | Starting Material | MH+ ion | LC-MS Retention Time (min) |
|---|---|---|---|---|---|---|
| 95 (Formate) | Me | 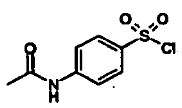 | Aldrich Chemical Company, Inc. | Intermediate 15 | 487 | 2.01 |
| 96 (Formate) | Me | 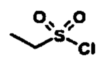 | Aldrich Chemical Company, Inc. | Intermediate 15 | 382 | 1.91 |
| 97 (Formate) | Me | 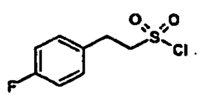 | WO 2002/068409 | Intermediate 15 | 476 | 2.34 |
| 98 (Formate) | Me | 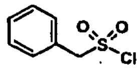 | Aldrich Chemical Company, Inc. | Intermediate 15 | 444 | 2.16 |
| 99 (Formate) | Me | 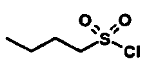 | Aldrich Chemical Company, Inc. | Intermediate 15 | 410 | 2.12 |
| 100 (Formate) | Me | 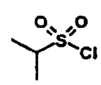 | Aldrich Chemical Company, Inc. | Intermediate 15 | 396 | 1.98 |
| 101 (Formate) | H | 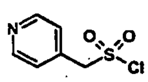 .$CF_3SO_3H$ | Interchim S.A. 14 | Intermediate | 431 | 1.76 |
| 102 (Formate) | H | 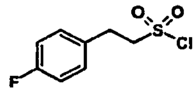 | WO 2002/068409 | Intermediate 14 | 462 | 2.42 |
| 103 (Formate) | Me | 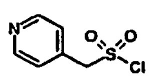 .$CF_3SO_3H$ | InterchimS.A. | Intermediate 15 | 445 | 1.76 |

(continued)

| Example Number | $R^2$ | R'$SO_2$Cl | One possible source of or reference for R'$SO_2$Cl | Starting Material | MH+ ion | LC-MS Retention Time (min) |
|---|---|---|---|---|---|---|
| 104 (Formate) | H | 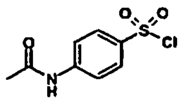 | Aldrich Chemical Company, Inc. | Intermediate 14 | 473 | 2.04 |
| 105 (Formate) | H | 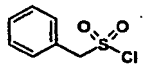 | Aldrich Chemical Company, Inc. | Intermediate 14 | 430 | 2.21 |
| 106 (Formate) | H | 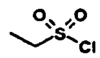 | Aldrich Chemical Company, Inc. | Intermediate 14 | 368 | 1.93 |
| 107 (Formate) | H | 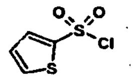 | Aldrich Chemical Company, Inc. | Intermediate 14 | 422 | 2.15 |
| 108 (Formate) | Me | 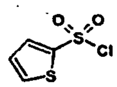 | Aldrich Chemical Company, Inc. | Intermediate 15 | 436 | 2.1 |
| 109 (Formate) | H | 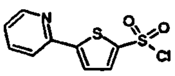 | Apollo Scientific Ltd. | Intermediate 14 | 499 | 2.31 |

**Examples 110 to 127**

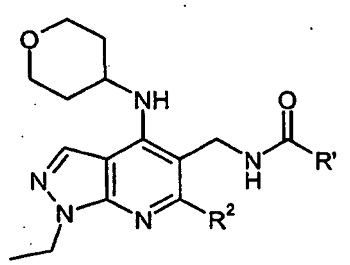

$R^2$ = H or Me

*General Procedure*

A solution of the carboxylic acid R'COOH (0.1mmol) in DMF (0.5ml) was treated with HATU (0.12mmol) and DIPEA (0.3mmol). The resulting solution was treated with a solution of Intermediate 14 or 15 (0.1mmol) in DMF (0.5ml) at room temperature for 18h and then concentrated *in vacuo.* The residue was dissolved in chloroform (0.5ml) and applied to an SPE cartridge (0.5g; Flash $NH_2$). The cartridge was eluted sequentially with chloroform (1.5ml), ethyl acetate (1.5ml) and 20% methanol in ethyl acetate (1.5ml). Fractions containing the desired product were concentrated *in vacuo* and where necessary the residue was purified by mass directed autoprep HPLC.

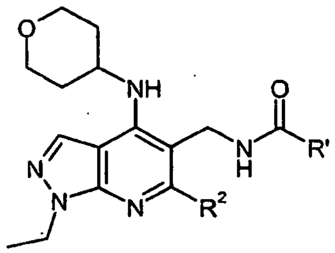

$R^2$ = H or Me

| Example Number | $R^2$ | R'COOH | One possible source of or reference for R'COOH | Starting Material | MH+. ion | LC-MS Ret. Time (min) |
|---|---|---|---|---|---|---|
| 110 | H | 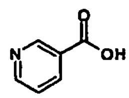 | Aldrich Chemical Company, Inc. | Intermediate 14 | 381 | 2.08 |
| 111 | H | 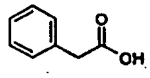 | Aldrich Chemical Company, Inc. | Intermediate 14 | 394 | 2.29 |
| 112 | Me | 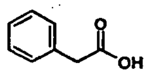 | Aldrich Chemical Company, Inc. | Intermediate 15 | 408 | 2.26 |
| 113 | Me | 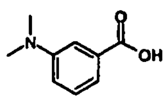 | Aldrich Chemical Company, Inc. | Intermediate 15 | 437 | 2.38 |
| 114 | Me | 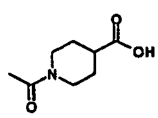 | Lancaster Synthesis Ltd. | Intermediate 15 | 443 | 2.01 |
| 115 | Me | 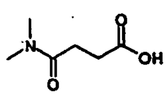 | Lancaster Synthesis Ltd. | Intermediate 15 | 417 | 1.98 |
| 116 | Me | 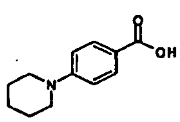 | Maybridge Chemical Co. | Intermediate 15 | 477 | 2.58 |
| 117 | Me | 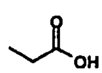 | Aldrich Chemical Company, Inc. | Intermediate 15 | 346 | 2.00 |
| 118 | H | 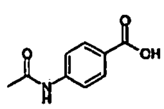 | Aldrich Chemical Company, Inc. | Intermediate 14 | 437 | 2.21 |
| 119 | H | 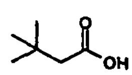 | Aldrich Chemical Company, Inc. | Intermediate 14 | 374 | 2.31 |
| 120 | H | 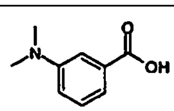 | Aldrich Chemical Company, Inc. | Intermediate 14 | 423 | 2.45 |

(continued)

| Example Number | $R^2$ | R'COOH | One possible source of or reference for R'COOH | Starting Material | MH+. ion | LC-MS Ret. Time (min) |
|---|---|---|---|---|---|---|
| 121 | H | 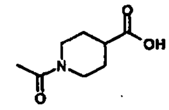 | Lancaster Synthesis Ltd. | Intermediate 14 | 429 | 2.01 |
| 122 | H | 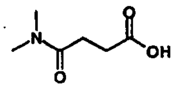 | Lancaster Synthesis Ltd. | Intermediate 14 | 403 | 1.96 |
| 123 | H | 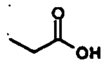 | Aldrich Chemical Company, Inc. | Intermediate 14 | 332 | 2.00 |
| 124 | Me | 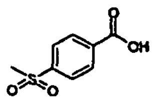 | Aldrich Chemical Company, Inc. | Intermediate 15 | 472 | 2.18 |
| 125 | Me | 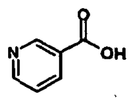 | Aldrich Chemical Company, Inc. | Intermediate 15 | 395 | 2.04 |
| 126 | Me | 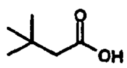 | Aldrich Chemical Company, | Intermediate 15 Inc. | 388 | 2.29 |
| 127 | H | 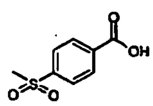 | Aldrich Chemical Company, Inc. | Intermediate 14 | 458 | 2.23 |

**Example 128** ***N*-{[1-ethyl-4-(tetrahydro-2*H*-pyran-4-ylamino)-1*H*-pyrazolo[3,4-*b*]pyridin-5-yl]methyl}-2,2,2-trifluoroacetamide**

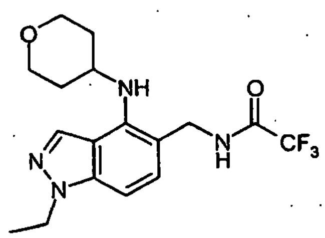

To an ice-cooled solution of Intermediate 14 (296mg) in methanol (2ml) was added ethyl trifluoroacetate (0.128ml). The solution was stirred under nitrogen for 20h during which time it was allowed to warm to room temperature and was then evaporated to dryness *in vacuo* to give Example 128 as a beige solid (384mg). LCMS showed $MH^+$ = 372; $T_{RET}$ = 2.02min.

**Example 129** ***N*-{[1-ethyl-4-(tetrahydro-2*H*-pyran-4-ylamino)-1*H*-pyrazolo[3,4-*b*]pyridin-5-yl]methyl}-2,2,2-trifluoro-*N*-(phenylmethyl)acetamide**

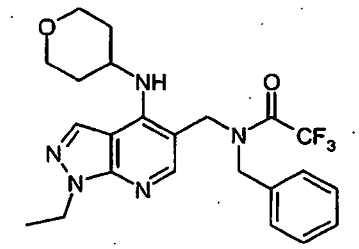

A mixture containing Example 128 (37mg), benzyl bromide (0.024ml), tetrabutylammonium bromide (3.2mg) and anhydrous potassium carbonate (41mg) in anhydrous acetonitrile (0.4ml) was stirred at 80˚C for 3h. It was then diluted with water (3ml) and extracted with dichloromethane (2 x 3ml) and purified by SPE cartridge (silica). Elution using 50% ethyl acetate in cyclohexane gave Example 129 (25mg). LCMS showed $MH^+$ = 462; $T_{RET}$ = 3.00min.

**Example 130 *N*-{[1-ethyl-6-methyl-4-(tetrahydro-2*H*-pyran-4-ylamino)-1*H* pyrazolo[3,4-*b*]pyridin-5-yl]methyl}-*N*-(phenylmethyl)acetamide**

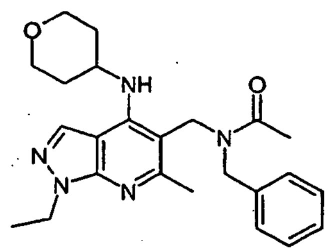

A solution of Example 83 (10mg) in acetonitrile (0.1ml) was treated with acetyl chloride (0.003ml) and DIPEA (0.007ml) for 21h at 21˚C. The mixture was diluted with dichloromethane (2ml), washed with brine (1ml) and evaporated to dryness. The residue was purified by mass directed autoprep HPLC to give Example 130 (9mg). LCMS showed $MH^+$ = 422; $T_{RET}$ = 2.11 min.

**Example 134 1-ethyl-5-{1-[(phenylmethyl)amino]propyl}-*N*-(tetrahydro-2*H*-pyran-4-yl)-1*H*-pyrazolo[3,4-*b*]pyridin-4-amine**

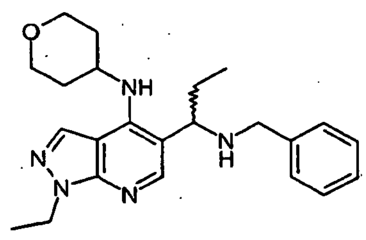

Ethyl magnesium bromide (3M solution in ether, 1.59ml) was added to a stirred solution of Intermediate 10 (124mg) in dry THF (10ml) at -10˚C under nitrogen. The reaction mixture was stirred at -10 to -5˚C for 4h, and then allowed to warm to room temperature. After 16h at room temperature, the reaction mixture was heated at 50˚C for 4h and was then quenched by the careful addition of 2M-ammonium chloride solution (2ml). Water (3ml) and dichloromethane (20ml) were added, and the aqueous layer extracted with more dichloromethane (2 x 10ml). The combined organic extracts were dried over anhydrous sodium sulphate and evaporated. The residual oil was purified by mass directed autoprep HPLC to give Example 134 as a colourless oil (19mg). LCMS showed $MH^+$ = 394; $T_{RET}$ = 2.16min.

**Example 135 5-[({[3-(dimethylamino)phenyl]methyl}amino)methyl]-1-ethyl-*N*-(tetrahydro-2*H*-pyran-4-yl)-1*H*-pyrazolo[3,4-*b*]pyridin-4-amine, formate**

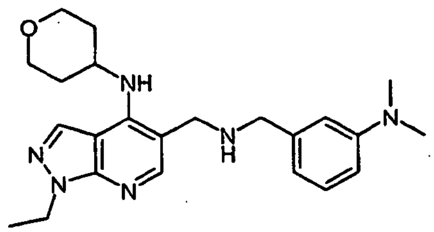

, formate

To a solution of Intermediate 14 (19mg) in THF (0.4ml) was added 3-(dimethylamino)benzaldehyde [e.g. see J. Am. Chem. Soc., (2000), 122 (40), 9723] (10mg), acetic acid (0.025ml) and sodium triacetoxyborohydride (36mg). The solution was stirred at room temperature for 21 h, quenched with water (0.5ml) and blown down to dryness. The residue was dissolved in methanol (2ml), applied to an SPE cartridge (1.0g; SCX2) and eluted sequentially with methanol and 2M-ammonia in methanol. Fractions containing the desired products were concentrated *in vacuo* and the residue was purified by mass directed autoprep HPLC to give Example 135 (8.4mg) LCMS showed $MH^+$ = 409; $T_{RET}$ = 2.12min.

**Examples 137 to 143**

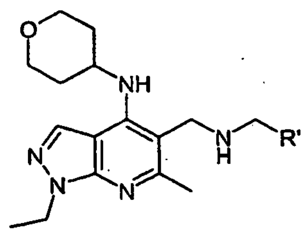

*General Procedure*

A solution of Intermediate **15** (0.1mmol) in THF (0.5ml) was treated with a solution of the aldehyde R'CHO (0.1 mmol) and as catalytic amount of acetic acid under nitrogen at room temperature for 0.5h. A solution of sodium triacetoxyborohydride (0.3mmol) in THF (0.5ml) was added and the solution stirred under nitrogen at room temperature for 18h. The reaction was treated with methanol (0.5ml) followed by chloroform (0.5ml) and washed with water (0.5ml). The organic layer was separated using a hydrophobic frit, concentrated *in vacuo* and the residue was purified by mass directed autoprep HPLC.

| Example Number | R'CHO | One possible source of or reference for R'CHO | MH+ iron | LC-MS Ret. Time (min) |
|---|---|---|---|---|
| 137 (Formate) | 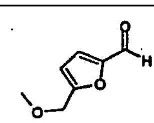 | N.D. Zelinsky Institute | 414 | 1.76 |
| 138 (Formate) | 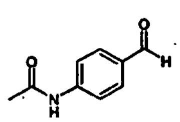 | Aldrich Chemical Company, Inc. | 437 | 1.72 |

(continued)

| Example Number | R'CHO | One possible source of or reference for R'CHO | MH+ iron | LC-MS Ret. Time (min) |
|---|---|---|---|---|
| 139 (Formate) | 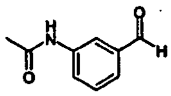 | J. Org. Chem., (1996), 61(11), 3623 | 437 | 1.74 |
| 140 (Formate) | 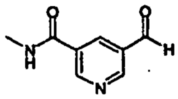 | unknown | 438 | 1.68 |
| 141 (Formate) | 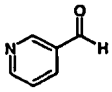 | Aldrich Chemical Company, Inc. | 381 | 1.20 |
| 143 (Formate) | 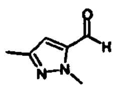 | Maybridge Chemical Company Ltd. | 398 | 1.74 |

**Example 144 *N*-{[1-ethyl-4-(tetrahydro-2*H*-pyran-4-ylamino)-1*H*-pyrazolo[3,4-*b*]pyridin-5-yl]methyl}-*N*-methyl-5-isoxazolecarboxamide**

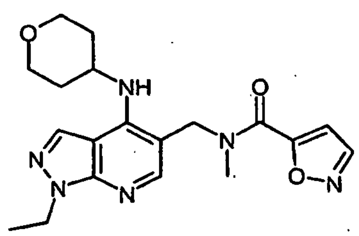

A solution of Example 128 (32mg) in anhydrous acetonitrile (0.8ml) was treated with tetrabutylammonium bromide (3mg), potassium carbonate (35mg) and methyl iodide (0.02ml) at room temperature for 24h. The mixture was treated with 2N sodium hydroxide (0.25ml) and methanol (0.25ml) for 3h, neutralised with 2N HCl (0.25ml) and then blown down to dryness. The residue was dissolved in methanol and purified using an SPE cartridge (SCX2) eluting with methanol and 10% ammonia in methanol. Appropriate fractions were blown down to dryness to give 1-ethyl-5-[(methylamino)methyl] - *N* - (tetrahydro-2*H*-pyran-4-yl) -1*H*- pyrazolo[3,4-*b*]pyridine-4-amine. This was dissolved in acetonitrile (0.4ml) and treated with isoxazole-5-carbonyl chloride (13mg) and DIPEA (0.016ml) for 20h at room temperature. The solution was blown down to dryness and the residue purified by mass directed autoprep HPLC to give Example 144. LCMS showed $MH^+ = 385$; $T_{RET} = 2.05$min.

**Example 145 *N*-ethyl-*N*-{[1-ethyl-4-(tetrahydro-2*H*-pyran-4-ylamino)-1*H*-pyrazolo[3,4-*b*]pyridin-5-yl]methyl}-5-isoxazolecarboxamide**

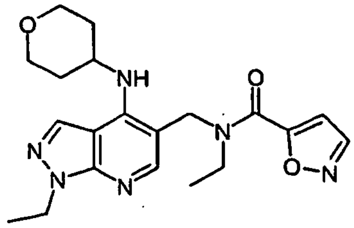

Example 145 was prepared in an analogous manner to Example 144 from Example 128, ethyl iodide and isoxazole-5-carbonyl chloride. LCMS showed $MH^+$ = 399; $T_{RET}$ = 2.17min.

**Examples 146 to 167**

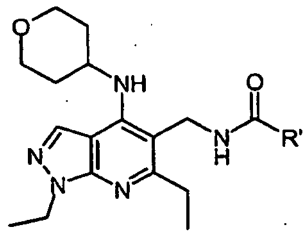

*General Procedure*

The carboxylic acid R'COOH (0.1 mmol) was treated with a solution of HATU (0.1 mmol) in DMF (0.2ml) and DIPEA (0.03ml). The solution was stirred for 5mins and treated with a solution of Intermediate 16 (0.1mmol) in DMF (0.2ml) at room temperature for 16h and then concentrated *in vacuo.* The residue was dissolved in chloroform (0.5ml) and applied to an SPE cartridge (0.5g, Flash $NH_2$). The cartridge was eluted sequentially with chloroform (1.5ml), ethyl acetate (1.5ml) and 20% methanol in ethyl acetate (1.5ml). Fractions containing the desired product were concentrated *in vacuo* and where necessary the residue was purified by mass directed autoprep HPLC. Where necessary, compounds were further purified by aminopropyl SPE cartridge (0.5g, Flash $NH_2$) eluting with methanol.

| Example Number | R'COOH | One possible source of or reference for R'COOH | MH+ ion | LC-MS Retention Time (min) |
|---|---|---|---|---|
| 146 | 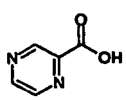 | Aldrich Chemical Company, Inc. | 410 | 2.18 |
| 147 | 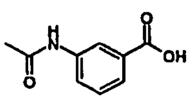 | Lancaster Synthesis Ltd. | 465 | 2.31 |
| 148 | 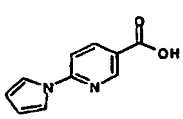 | US 2003/144283 | 474 | 2.68 |
| 149 | 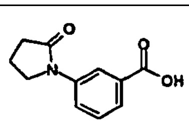 | WO 2003/032971 | 491 | 2.38 |

(continued)

| Example Number | R'COOH | One possible source of or reference for R'COOH | MH+ ion | LC-MS Retention Time (min) |
|---|---|---|---|---|
| 150 | 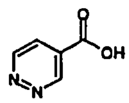 | Peakdale Molecular | 410 | 2.10 |
| 151 | 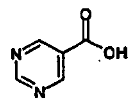 | Peakdale Molecular | 410 | 2.12 |
| 152 | 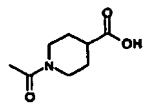 | Lancaster Synthesis Ltd. | 457 | |
| 153 | 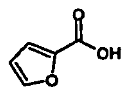 | Aldrich Chemical Company, Inc. | 398 | 2.24 |
| 154 | 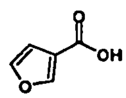 | Aldrich Chemical Company, Inc. | 398 | 2.28 |
| 155 | 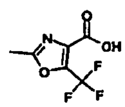 | Peakdale Molecular | 481 | 2.56 |
| 156 | 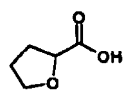 | Aldrich Chemical Company, Inc. | 402 | 2.14 |
| 157 | 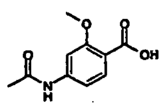 | DE 2429778 | 495 | 2.39 |
| 158 | 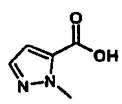 | Fluorochem Ltd. | 412 | 2.25 |
| 159 | 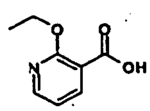 | Avocado Research | 453 | 2.52 |
| 160 | 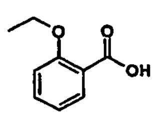 | Aldrich Chemical Company, Inc. | 452 | 2.63 |
| 161 | 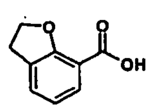 | Fluorochem Ltd. | 450 | 2.56 |
| 162 | 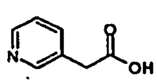 | Apollo Scientific Ltd. | 423 | 2.00 |

(continued)

| Example Number | R'COOH | One possible source of or reference for R'COOH | MH+ ion | LC-MS Retention Time (min) |
|---|---|---|---|---|
| 163 | 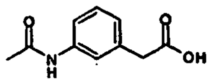 | WO 2003/106459 | 479 | 2.21 |
| 164 | 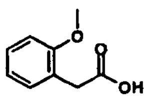 | Aldrich Chemical Company, Inc. | 452 | 2.40 |
| 165 | 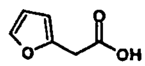 | Advanced Synthesis Technologies | 412 | 2.25 |
| 166 | 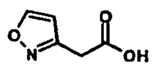 | MicroChemistry Ltd. | 413 | 2.12 |
| 167 | 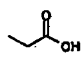 | Aldrich Chemical Company, Inc. | 360 | 2.11 |

**Examples 168 to 173**

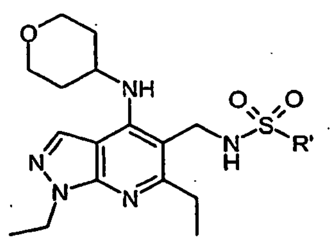

*General Procedure*

A solution of Intermediate 16 (0.1 mmol) in DMF (0.2ml) was treated with the sulphonyl chloride R'$SO_2$Cl (0.1 mmol) and DIPEA (0.03ml) at room temperature for 16h and then concentrated *in vacuo.* The residue was purified by mass directed autoprep HPLC. Where necessary, compounds were further purified by SPE cartridge (Flash $NH_2$) eluting with methanol.

| Example Number | R'$SO_2$Cl | One possible source of or reference for R'$SO_2$Cl | MH+ ion | LC-MS Retention Time (min) |
|---|---|---|---|---|
| 168 (TFA salt) | 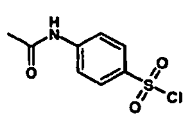 | Aldrich Chemical Company, Inc. | 501 | 2.27 |
| 169 | 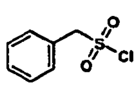 | Aldrich Chemical Company, Inc. | 458 | 2.51 |

(continued)

| Example Number | R'SO$_2$Cl | One possible source of or reference for R'SO$_2$Cl | MH+ ion | LC-MS Retention Time (min) |
|---|---|---|---|---|
| 170 (TFA salt) | 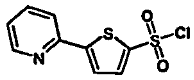 | Maybridge Chemical Company Ltd. | 527 | 2.64 |
| 171 | 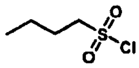 | Aldrich Chemical Company, Inc. | 424 | 2.44 |
| 172 (TFA salt) | 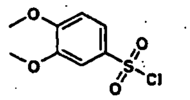 | Aldrich Chemical Company, Inc. | 504 | 2.40 |
| 173 | 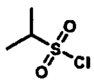 | Aldrich Chemical Company, Inc. | 410 | 2.24 |

**Examples 174 to 193**

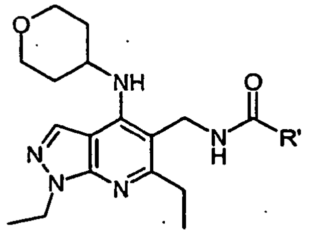

***General Procedure***

The carboxylic acid R'COOH (0.1mmol) was treated with a solution of HATU (0.1mmol) in DMF (0.2ml) and DIPEA (0.03ml). The solution was stirred for 5mins and treated with a solution of Intermediate 16 (0.1mmol) in DMF (0.2ml) at room temperature for 16h and then concentrated *in vacuo.* The residue was dissolved in chloroform (0.5ml) and applied to an SPE cartridge (0.5g, Flash $NH_2$). The cartridge was eluted sequentially with chloroform (1.5ml), ethyl acetate (1.5ml) and 20% methanol in ethyl acetate (1.5ml). Fractions containing the desired product were concentrated *in vacuo* and where necessary the residue was purified by mass directed autoprep HPLC. Where necessary, compounds were further purified by SPE cartridge (0.5g, Flash $NH_2$) eluting with methanol.

| Example Number | R'COOH | One possible source of or reference for R'COOH | MH+ ion | LC-MS Retention Time (min) |
|---|---|---|---|---|
| 174 | 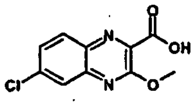 | DE74-2410852 | 524.0 | 2.4 |
| 175 | 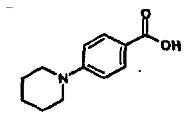 | J. Org. Chem., (1987), 52 (9), 1710 Scientific | 491.5 | 2.1 |

(continued)

| Example Number | R’COOH | One possible source of or reference for R’COOH | MH+ ion | LC-MS Retention Time (min) |
|---|---|---|---|---|
| 176 | 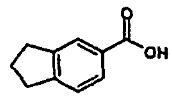 | Tyger Inc | 448.4 | 2.3 |
| 177 | 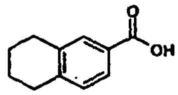 | Ubichem plc | 462.5 | 2.4 |
| 178 | 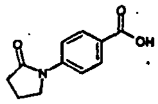 | Butt Park Ltd. WO 94/03426 | 491.5 | 2.1 |
| 179 | 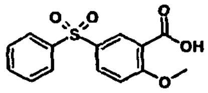 | A1 Maybridge | 578.4 | 2.3 |
| 180 | 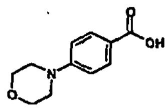 | Chemical Company Ltd. J. Org. Chem. | 493.5 | 2.1 |
| 181 | 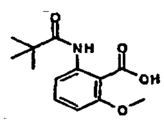 | (1985), 50(5), 718 | 537.5 | 2.2 |
| 182 | 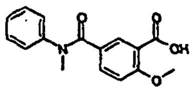 | Not known | 571.5 | 2.3 |
| 183 | 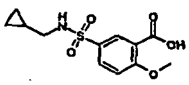 | EP 83-303097 J. Amer. Chem. | 571.5 | 2.2 |
| 184 | 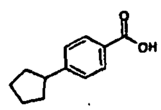 | Soc (1968), 90(13), 3404 WO | 476.5 | 2.5 |
| 185 | 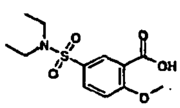 | 95/00508 A1 | 573.5 | 2.3 |
| 186 | 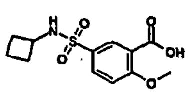 | EP 83-303097 | 571.5 | 2.2 |
| 187 | 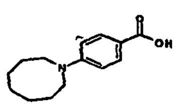 | Not known | 519.5 | 2.6 |
| 188 | 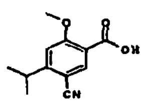 | WO 98141507 A1 J. Org. Chem. | 505.5 | 2.4 |

(continued)

| Example Number | R'COOH | One possible source of or reference for R'COOH | MH+ ion | LC-MS Retention Time (min) |
|---|---|---|---|---|
| 189 | 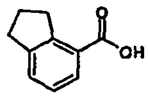 | (1981), 46(4), 783 | 448.5 | 2.3 |
| 190 | 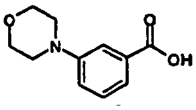 | MicroChemistry Building Blocks | 493.5 | 2.1 |
| 191 | 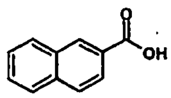 | Aldrich Chemical Company, Inc. | 458.4 | 2.4 |
| 192 | 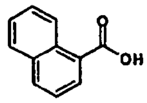 | Aldrich Chemical Company, Inc. | 458.4 | 2.3 |
| 193 | 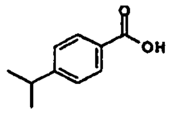 | Aldrich Chemical Company, Inc. | 450.4 | 2.4 |

**Example 194 4-(acetylamino)-N-{[1,6-diethyl-4-(tetrahydro-2H-pyran-4-ylamino)-1H-pyrazolo[3,4-b]pyridin-5-yl]methyl}benzamide**

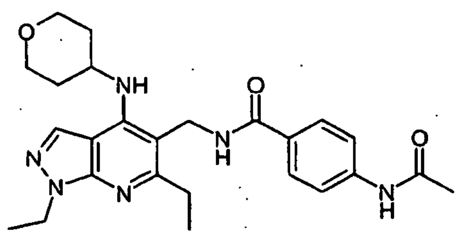

A solution of Intermediate 16 (75mg) in anhydrous acetonitrile (1.25ml) was treated with 4-acetylaminobenzoyl chloride [e.g. available from *Lancaster Synthesis Ltd]* (49mg) and DIPEA (0.044ml) at room temperature for 24h. The solution was blown down to dryness and the residue dissolved in dichloromethane (5ml) and applied to an SPE cartridge (silica; 10g). Elution using ethyl acetate gave Example 194 as a cream solid (71 mg). LCMS showed $MH^+$ = 465; $T_{RET}$ = 2.18min.

**Example 195 N-{[1,6-diethyl-4-(tetrahydro-2H-pyran-4-ylamino)-1H-pyrazolo[3,4-b]pyridin-5-yl]methyl}-2-methyl-3-furancarboxamide**

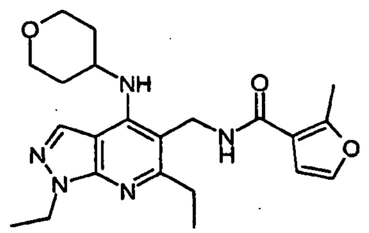

A suspension of 2-methyfuran-3-carboxylic acid [e.g. available from *Lancaster Synthesis Ltd]* (31.5mg) in dichloromethane (0.5ml) was treated with oxalyl chloride (0.022ml) and DMF (one drop) at room temperature under nitrogen for 0.5h. The solution was then added dropwise to a stirred solution of Intermediate 16 (69mg) and DIPEA (0.044ml) in acetonitrile (1.25ml) and the mixture stirred for 90h at room temperature. It was then diluted with dichloromethane (7ml), washed with dilute aqueous sodium chloride (2 x 7ml) and the organic extract applied to an SPE cartridge (Flash $NH_2$). Elution with methanol gave Example 195 as a beige solid (85mg). LCMS showed $MH^+$ = 412, $T_{RET}$ = 2.31 min.

**Example 196 N-([1,6-diethyl-4-(tetrahydro-2H-pyran-4-ylamino)-1H-pyrazolo[3,4-b]pyridin-5-yl]methyl}benzamide**

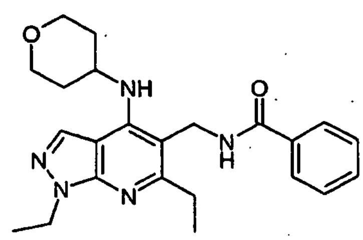

A solution of Intermediate 16 (60mg) in anhydrous acetonitrile (1.0ml) was treated with benzoyl chloride (0.023ml) and DIPEA (0.035ml) at room temperature for 2h. The solution was blown down to dryness and the residue purified by mass directed autoprep HPLC to give Example 196 as a white solid (67mg). LCMS showed $MH^+$= 408; $T_{RET}$ = 2.31 min.

**Example 197 1-{[1,6-diethyl-4-(tetrahydro-2*H*-pyran-4-ylamino)-1*H*-pyrazolo[3,4-*b*]pyridin-5-yl]methyl}-2-pyrrolidinone**

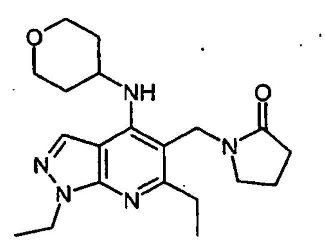

A solution of Intermediate 16 (75mg) in anhydrous acetonitrile (1.25ml) was treated with 4-chlorobutyryl chloride (0.03ml) and DIPEA (0.044ml) and stirred at room temperature for 4 days. The mixture was diluted with dichloromethane (10ml) and washed with dilute aqueous sodium chloride (2 x 7ml). The organic phase was separated using a hydrophobic frit (12ml) and blown to dryness. The residual gum was dissolved in anhydrous DMF (3ml) and then added to sodium hydride (60% dispersion in oil, 10.9mg). The mixture was stirred at room temperature for 20h then treated with water (8ml). The solution was extracted with dichloromethane (2 x 7ml) and the combined organics were separated using a hydrophobic frit (12ml) and loaded directly onto an SPE cartridge (2g, Flash $NH_2$) which had been pre-washed with methanol. Two column volumes of methanol were eluted, combined and blown to dryness to afford Example 197 as a

beige foam (0.091g). LCMS showed $MH^+$ = 372; $T_{RET}$ = 1.98min.

**Example 198 5-{[1,6-diethyl-4-(tetrahydro-2*H*-pyran-4-ylamino)-1*H*-pyrazolo[3,4-*b*)pyridin-5-yl]methyl}-5,6-dihydro-4*H*-furo[2,3-*c*]pyrrol-4-one**

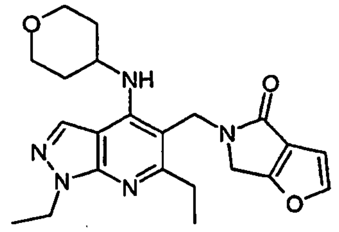

Ethyl 2-(chloromethyl)-3-furancarboxylate [e.g. see *Bulletin de la Societe Chimique de France (1974), (3-4, Pt.* 2), *519]* (62mg) was added to a solution of Intermediate 16 (91mg) in anhydrous acetonitrile (1ml). DIPEA (0.053ml) was added and the solution left to stand at room temperature for 18h, followed by stirring and heating at 80˚C for 2h. The solution was allowed to cool and was then loaded directly onto an SPE cartridge (2g; SCX) which had been prewashed with methanol. Three column volumes of methanol were eluted and discarded, then 3 column volumes of 10% 0.880 ammonia solution/methanol were collected and evaporated to dryness to give a brown gum which was purified on a 2g silica cartridge eluting with 50% ethyl acetate in cyclohexane to give ethyl 2-[({[1,6-diethyl-4 - (tetrahydro - 2*H*-pyran -4- ylamino) - 1*H* - pyrazolo[3,4-b]pyridine - 5 -yl]methyl}amino)methyl] - 3 - furancarboxylate as a brown gum (62mg). This was dissolved in ethanol (0.4ml), treated with aqueous sodium hydroxide (2M, 0.27ml) and the solution stirred at room temperature for 42h then left to stand for 1 day. The solution was diluted with water (1ml), neutralised with hydrochloric acid, evaporated to dryness *in vacuo* and the residue treated with thionyl chloride (0.028ml). After stirring at room temperature for 23h, DIPEA (0.049ml) and dichloromethane (1 ml) were added and after a further 24 hours at room temperature, the reaction was blown to dryness. The residue was purified by mass directed autoprep HPLC to give Example 198 as a yellow glass (15mg). LCMS showed $MH^+$ = 410; $T_{RET}$ = 2.17min.

**Example 199 2-{[1,6-diethyl-4-(tetrahydro-2*H*-pyran-4-ylamino)-1*H*-pyrazolo[3,4-*b*]pyridin-5-yl]methyl)-2,3-dihydro-1*H*-isoindol-1-one**

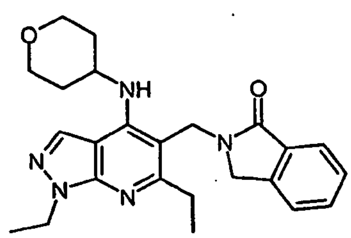

A solution of Intermediate 16 (200mg) in anhydrous acetonitrile (1ml) was treated with a solution of methyl 2-(bromomethyl)benzoate [e.g. available from *Apin Chemicals Ltd.]* (166mg) in anhydrous acetonitrile (1ml) followed by DIPEA (0.116ml) and heated to 80˚C for 1.75h then allowed to cool. The solution was diluted with methanol (~10ml) and purified on a 10g SCX cartridge which had been pre-washed with methanol. Three column volumes of methanol were eluted and discarded, then 3 column volumes of 10% 0.880 ammonia solution/methanol were collected and evaporated to dryness. The residual gum was dissolved in dichloromethane and purified on an SPE cartridge (20g; silica) eluted with a gradient of 0 - 100% ethyl acetate in cyclohexane to give Example 199 as an off-white solid (137mg). LCMS showed $MH^+$ = 420; $T_{RET}$ = 2.32min.

**Example200*N*-{[1,6-diethyl-4-(tetrahydro-2*H*-pyran-4-ylamino)-1*H*-pyrazolo[3.4-*b*]pyridin-5-yl]methyl}-*N*'-(phenylmethyl)urea**

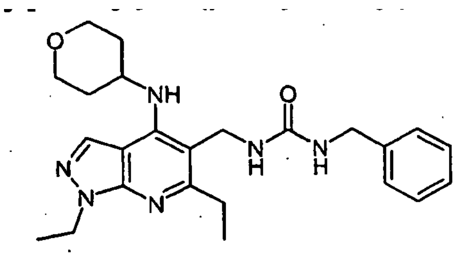

Intermediate 16 (98mg) was added to a solution of 4-nitrophenyl(phenylmethyl)-carbamate [e.g. available from *Asta Tech]* (80mg) and DIPEA (0.061ml) in dichloromethane (2ml) and the mixture stirred at room temperature under nitrogen for 18h. The solution was applied to an SPE cartridge (20g; silica) and eluted with a gradient of 50 - 85% ethyl acetate in cyclohexane. Appropriate fractions were collected and evaporated to dryness to give Example 200 as an off-white solid (65mg). LCMS showed $MH^+$ = 437; $T_{RET}$ = 2.36min.

**Example 201 *N*-{[1,6-diethyl-4-(tetrahydro-2H-pyran-4-ylamino)-1*H*-pyrazolo[3,4-*b*]pyridin-5-yl]methyl}-*N*'-(tetrahydro-2*H*-pyran-4-yl)urea**

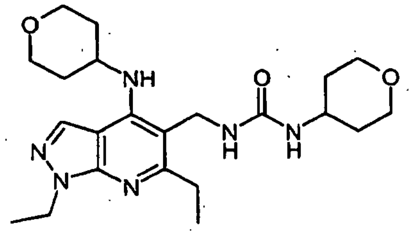

Example 201 was prepared in an analogous manner to Example 200 from Intermediate 16 and 4-nitrophenyl tetrahydro-2*H*-pyran-4-ylcarbamate. LCMS showed $MH^+$ = 431; $T_{RET}$ = 2.01min.

**Example 202 *N*-{[1,6-diethyl-4-(tetrahydro-2*H*-pyran-4-ylamino)-1*H*-pyrazolo[3,4-*b*]pyridin-5-yl]methyl}-*N*'-(2-phenylethyl)urea**

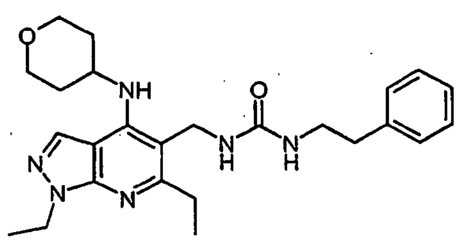

Example 202 was prepared in an analogous manner to Example 200 from Intermediate 16 and 4-nitrophenyl (2-phenylethyl)carbamate [e.g. see *J. Org Chem., 1998*, *63*, (*23*), 8515]. LCMS showed $MH^+$ = 451; $T_{RET}$ = 2.38min.

**Example 203 *N*-{[1,6-diethyl-4-(tetrahydro-2*H*-pyran-4-ylamino)-1*H*-pyrazolo[3,4-*b*]pyridin-5-yl]methyl}-*N*'-phenylurea**

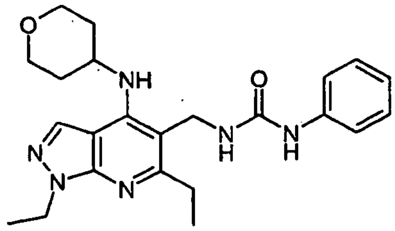

Example 203 was prepared in an analogous manner to Example 200 from Intermediate 16 and 4-nitrophenyl phenylcarbamate [e.g. see *Synthesis,* 1989, (*6*) 423]. LCMS showed $MH^+$ = 423; $T_{RET}$ = 2.42min.

**Examples 204 to 211**

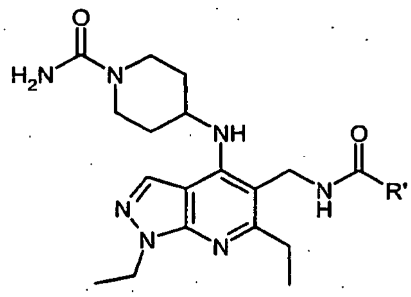

**General Procedure:**

A solution of the carboxylic acid R'COOH (0.07mmol), HATU (0.07mmol) and DIPEA (0.18mmol) in acetonitrile (1ml) was stirred at room temperature for 10 mins and then treated with Intermediate 26 (0.06mmol) in DMF (1ml). The reaction mixture was left to stand at room temperature for 18h. The solvent was evaporated and the residue was dissolved in chloroform (0.5ml) and applied to an SPE cartridge (0.5g; Flash $NH_2$). The cartridge was eluted sequentially with chloroform (1.5ml), ethyl acetate (1.5ml) and 20% methanol in ethyl acetate (1.5ml). Fractions containing the desired product were concentrated *in vacuo* and purified by mass directed autoprep HPLC.

| Example | R'$CO_2$H | One possible source of or reference for acid R'$CO_2$H | [MH]+ | LC/MS Retention Time (mins) |
|---|---|---|---|---|
| 204 (formate) | 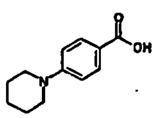 | Maybridge plc | 533 | 2.52 |
| 205 (formate) | 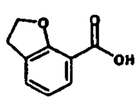 | Fluorochem Ltd. | 492 | 2.34 |
| 206 (formate) | 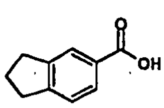 | Tyger Scientific Inc | 490 | 2.47 |
| 207 (formate) | 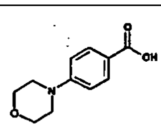 | Maybridge plc | 535 | 2..26 |

(continued)

| Example | R'$CO_2$H | One possible source of or reference for acid R'$CO_2$H | [MH]+ | LC/MS Retention Time (mins) |
|---|---|---|---|---|
| 208 (formate) | 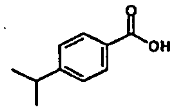 | Aldrich Chemical Company, Inc. | 492 | 2.89 |
| 209 (formate) | 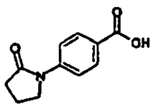 | Butt Park Ltd. | 533 | 2.19 |
| 210 (formate) | 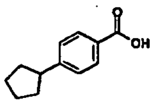 | J. Am. Chem. Soc(1968) 90(13), 3404 | 518 | 2.71 |
| 211 (formate) | 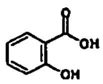 | Aldrich Chemical Company, Inc. | 466 | 2.30 |

**<u>Example 212</u> [4-({1,6-diethyl-5-[(1-oxo-1,3-dihydro-2*H*-isoindol-2-yl)methyl]-1*H*-pyrazolo[3,4-*b*]pyridin-4-yl) amino)-1-piperidinecarboxamide], formate**

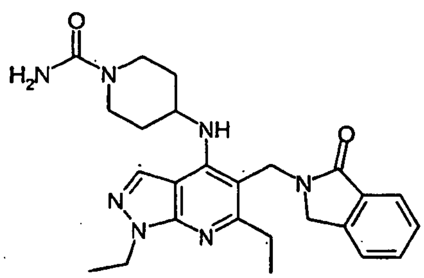

**, formate**

Intermediate 26 (25mg) was dissolved in acetonitrile (1 ml). The solution was heated with methyl 2-(bromomethyl)benzoate [e.g. available from Apin Chemicals Ltd] (18mg) at 80˚C for 2h. The mixture was cooled and the solvent removed by evaporation. The residue was purified by mass directed autoprep HPLC to give Example 212 as a white solid. LCMS showed $MH^+$ = 462; $T_{RET}$ = 2.25min.

**<u>Examples 213 to 239</u>**

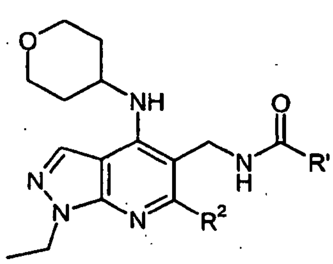

**$R^2$ = Me or Et**

*General Procedure*

The carboxylic acid R'COOH (0.12mmol) is treated with a solution of HATU (0.12mmol) in DMF (0.25ml) and DIPEA (0.052ml, ca. 0.3mmol) is added. The solution is shaken for 10mins, and is treated with a solution of Intermediate 15 or 16 (0.1 mmol) in DMF (0.2ml). The resulting solution is shaken for 10mins and left to stand for 18h (e.g. at room temperature), and then the DMF is removed in a Genevac vacuum centrifuge. The residue is dissolved in chloroform (0.3ml), is applied to an SPE cartridge (1g, aminopropyl) which has been pre-washed with chloroform (6ml), and is eluted sequentially with chloroform (3ml) and 10% methanol in ethyl acetate (3ml). Fractions containing the desired product are concentrated *in vacuo* in a Genevac vacuum centrifuge, and where necessary the residue is purified by mass directed autoprep HPLC (e.g. acetonitrile/water). Where necessary, the compound(s) is/are dissolved in chloroform, and is/are further purified by loading onto a SPE cartridge (0.5g, aminopropyl) which has been prewashed with chloroform, eluting with 10% methanol in ethyl acetate.

| Example | $R^2$ | $R'CO_2H$ | One possible source of or reference for acid $R'CO_2H$ | Starting Material |
|---|---|---|---|---|
| 213 | Me | 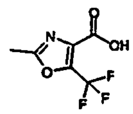 | Peakdale Molecular | Intermediate 15 |
| 214 | Me | 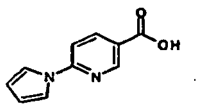 | US 2003/144283 | Intermediate 15 |
| 215 | Me | 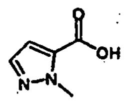 | Fluorochem Ltd. | Intermediate 15 |
| 216 | Me | 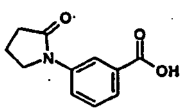 | WO 2003/032971 | Intermediate 15 |
| 217 | Me | 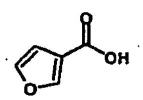 | Aldrich Chemical Company, Inc. | Intermediate 15 |
| 218 | Me | 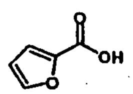 | Aldrich Chemical Company, Inc. | Intermediate 15 |
| 219 | Me | 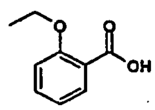 | Aldrich Chemical Company, Inc. | Intermediate 15 |
| 220 | Me | 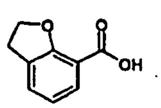 | Fluorochem Ltd. | Intermediate 15 |
| 221 | Me | 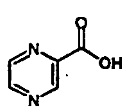 | Aldrich Chemical Company, Inc. | Intermediate 15 |
| 222 | Me | 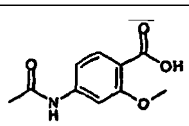 | DE 2335439 | Intermediate 15 |

(continued)

| Example | $R^2$ | R'$CO_2H$ | One possible source of or reference for acid R'$CO_2H$ | Starting Material |
|---|---|---|---|---|
| 223 | Me | 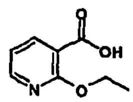 | Avocado Research | Intermediate 15 |
| 224 | Me | 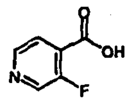 | Fluorochem Ltd. | Intermediate 15 |
| 225 | Me | 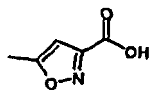 | Lancaster Synthesis Ltd. | Intermediate 15 |
| 226 | Me | 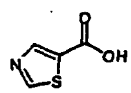 | Apin Chemicals Ltd. | Intermediate 15 |
| 227 | Me | 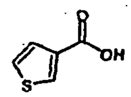 | Aldrich Chemical Company, Inc. | Intermediate 15 |
| 228 | Me | 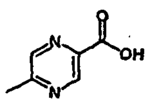 | Aldrich Chemical Company, Inc. | Intermediate |
| 229 | Me | 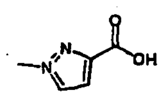 | Fluorochem Ltd. | Intermediate 15 |
| 230 | Me | 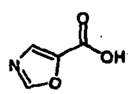 | US 4785012 | Intermediate 15 |
| 231 | Et | 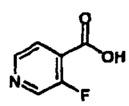 | Fluorochem Ltd. | Intermediate 16 |
| 232 | Et | 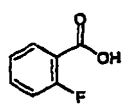 | Aldrich Chemical Company, Inc. | Intermediate 16 |
| 233 | Et | 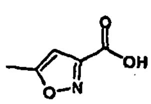 | Aldrich Chemical Company, Inc., or perhaps Lancaster Synthesis Ltd. | Intermediate 16 |
| 234 | Et | 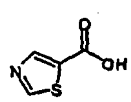 | Apin Chemicals Ltd. | Intermediate 16 |
| 235 | Et | 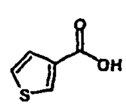 | Aldrich Chemical Company, Inc. | Intermediate 16 |

(continued)

| Example | $R^2$ | R'$CO_2H$ | One possible source of or reference for acid R'$CO_2H$ | Starting Material |
|---|---|---|---|---|
| 236 | Et | 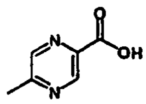 | Aldrich Chemical Company, Inc. | Intermediate 16 |
| 237 | Et | 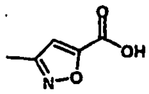 | eg Fluorochem, Oakwood, Enamine, TimTec, and/or MicroChemistry Building Blocks | Intermediate 16 |
| 238 | Et | 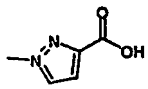 | Fluorochem Ltd. | Intermediate 16 |
| 239 | Et | 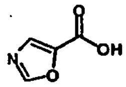 | US 4785012 | Intermediate 16 |

**Example 236 (Synthesis B) *N*-{[1,6-diethyl-4-(tetrahydro-2*H*-pyran-4-ylamino)-1*H* pyrazolo[3,4-*b*]pyridin-5-yl] methyl}-5-methyl-2-pyrazinecarboxamide**

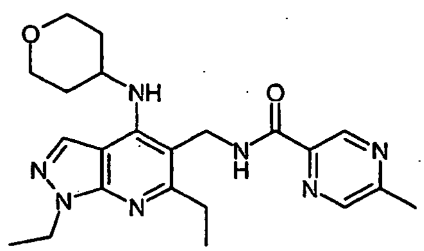

An example of a specific synthesis (Synthesis B) of Example 236 is as follows:

5-Methyl-2-pyrazinecarboxylic acid (0.4mmol) was dissolved in DMF (0.5ml) and a solution of HATU (152mg) in DMF (0.5ml) was added. DIPEA (0.172ml) was added and the solution left to stand for 10mins. A solution of 5-(aminomethyl)-1,6-diethyl-*N*-(tetrahydro-2*H*-pyran-4-yl)-1*H*-pyrazolo[3,4-*b*]pyridin-4-amine (e.g. which can be as prepared in Intermediate 16) (100mg) in DMF (0.5ml) was added and the solution left to stand for 18h. DMF was removed in a Genevac vacuum centrifuge and the residue dissolved in chloroform (1ml) and applied to an SPE cartridge (2g, aminopropyl) that had been pre-washed with chloroform (10ml). The cartridge was eluted with chloroform (2 x 10ml) and 10% methanol in ethyl acetate and fractions containing the product collected and blown down to dryness. The residue was purified by mass directed autoprep HPLC to give the title compound *N*-{[1,6-diethyl-4-(tetrahydro-2H-pyran-4-ylamino)-1H-pyrazolo[3,4-b]pyridin-5-yl]methyl}-5-methyl-2-pyrazinecarboxamide as a solid. (81.8mg). LCMS showed $MH^+$ = 424; $T_{RET}$ = 2.18min.

**Example 236 (Synthesis C) *N*-{[1,6-diethyl-4-(tetrahydro-2*H*-pyran-4-ylamino)-1*H*-pyrazolo[3,4-*b*]pyridin-5-yl] methyl}-5-methyl-2-pyrazinecarboxamide**

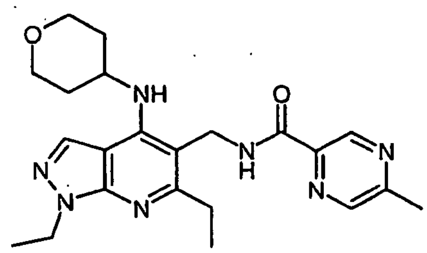

5-Methyl-2-pyrazinecarboxylic acid (6.8g, ca. 1.0 eq.), HBTU (19.3g or 19.5g, ca. 1.08 eq.) and DMF (300ml, 20 vols) were stirred under nitrogen, followed by the addition of diisopropylethylamine (18ml, 2.0 eq.) to form a black solution. The mixture was left to stir for 10 minutes and 5-(aminomethyl)-1,6-diethyl-*N*-(tetrahydro-2*H*-pyran-4-yl)-1*H*-pyrazolo[3,4-*b*]pyridin-4-amine (15g, 1.0 eq.) (e.g. which can be as prepared in Intermediate 16) was added together with DMF (10 ml) to wash in the solids. The reaction was left to stir for 3h, and then left to stand overnight prior to work-up. Work-up proceded by pouring the mixture into saturated sodium hydrogen carbonate solution (3 L) and extracting with DCM (2 x 800 mL). The combined organic layers were backwashed with water (2 x 500 mL), dried over $MgSO_4$ (50 g), filtered, reduced *in vacuo* to give a brown oil.

This crude material was purified approximately as follows:

Crude material was purified using gradient silica (500g) column chromatography (2-5% MeOH in DCM); after evaporation, the resulting solid was dissolved in DCM (300 mL), washed with water (2 x 200 mL), to remove DMF, dried ($MgSO_4$), filtered and reduced in vacuo. Recrystallisation from EtOAc / hexane (100 ml, 1:1) and drying in a vacuum oven at 40°C gave the title compound as a solid (10.578 g).

**Example 237 (Synthesis B) *N*-{[1,6-diethyl-4-(tetrahydro-2*H*-pyran-4-ylamino)-1*H*-pyrazolo[3,4-*b*]pyridin-5-yl]methyl}-3-methyl-5-isoxazolecarboxamide**

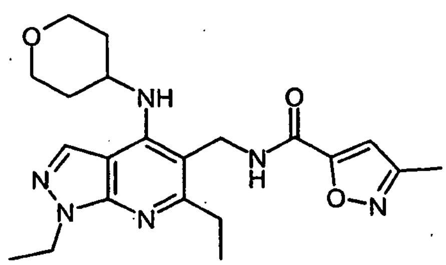

An example of a specific synthesis (Synthesis B) of Example 237 is as follows:

3-Methyl-5-isoxazolecarboxylic acid (92mg) was suspended in dry dichloromethane (2ml) and treated at 20°C with oxalyl chloride (0.064ml) and diethyl formamide (1 drop). Effervescence occurred over ca. 10mins and after 40mins stirring at room temperature the solution was added dropwise to a solution of 5-(aminomethyl)-1,6-diethyl-*N*-(tetrahydro-2*H*-pyran-4-yl)-1*H*-pyrazolo[3,4-*b*]pyridin-4-amine (198mg) (e.g. which can be as prepared in Intermediate 16) in anhydrous acetonitrile (4ml). The mixture was treated with DIPEA (0.128ml) and stirred at room temperature under nitrogen for 15h. Dichloromethane (50ml) was added to the reaction mixture and the solution was washed with dilute aqueous sodium chloride solution (2 x 50ml). The organic phase was separated using a hydrophobic frit (70ml) and loaded directly onto an SPE cartridge (10g, aminopropyl) which had been pre-washed with methanol. The cartridge was eluted with methanol (x2) and the fractions collected and blown down / evaporated to dryness. The residual gum was dissolved in dichloromethane and purified by SPE cartridge (10g, silica) on a Flashmaster 2 eluting with a gradient of 0 - 100% ethyl acetate in cyclohexane over 40 mins. Two fractions (from two chromatographic peaks) were collected and evaporated separately, and each was purified by mass directed autoprep HPLC. Relevant fractions from each were evaporated to dryness. The two residual gums (125 mg and 48mg respectively) were combined and purified by SPE cartridge (2g, aminopropyl) which had been pre-washed with methanol. Elution with

methanol (x2), collection of methanol, and evaporation to dryness gave the title compound *N*-{[1,6-diethyl-4-(tetrahydro-2H-pyran-4-ylamino)-1H-pyrazolo[3,4-b]pyridin-5-yl]methyl}-3-methyl-5-isoxazolecarboxamide as a white foam (173mg). LCMS showed $MH^+$ = 413; $T_{RET}$ = 2.20min.

Note: 3-Methyl-5-isoxazolecarboxylic acid is thought to be commercially available from one or more of the following suppliers: Chemical Block Stock Library, Chemical Block Building Blocks, Fluorochem, Scientific Exchange, Aurora Screening Library, Oakwood Products Catalog, Ambinter Stock Screening Collection, TimTec Building Blocks and Reagents, TimTec Overseas Stock, Enamine Building Blocks, Enamine Screening Library, Interchim Intermediates, AslnEx Express Gold Collection, and/or MicroChemistry Building Blocks. See near the start of the "Intermediates and Examples" section hereinabove, for the addresses of some of these suppliers.

**Example 237 (Synthesis C)**

***N*-{[1,6-diethyl-4-(tetrahydro-2*H*-pyran-4-ylamino)-1*H*-pyrazolo[3,4-*b*]pyridin-5-yl]methyl}-3-methyl-5-isoxazolecarboxamide**

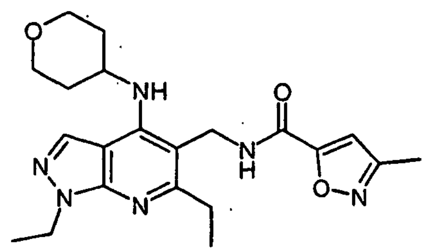

3-Methyl-5-isoxazolecarboxylic acid (6.59 g, 0.052 mol, 1.0 equivalent), O-(benzotriazol-1-yl)-*N,N,N',N'*-tetramethyluronium hexafluorophosphate (HBTU) (20.88g, 1.08 equivalents) and diisopropylethylamine (18 ml, 2 equivalents) in dimethyl formamide (DMF, 314ml, 20 volumes) were stirred under nitrogen for 10 minutes. 5-(aminomethyl)-1,6-diethyl-*N*-(tetrahydro-2H-pyran-4-yl)-1*H*-pyrazolo[3,4-*b*]pyridin-4-amine (15.7 g, 0.052 mol, 1.0 equivalent) (e.g. which can be as prepared in Intermediate 16) was added to the mixture with extra DMF (20ml) to wash it into the flask. The reaction mixture was left to stir for three (3) hours, and then left under nitrogen overnight. The reaction mixture was partitioned between saturated sodium hydrogen carbonate solution (3 L) and dicloromethane (800 mL). The aqueous layer was extracted again with dicloromethane (800 mL). The combined organic layers were backwashed with water (2 x 500 mL), dried over $MgSO_4$ (50 g), filtered, reduced *in vacuo,* and cooled to give a crude brown oil (about 30g) which appeared to include DMF.

This crude brown oil was slurried in 1 : 0.98 : 0.2 DCM : EtOAc : MeOH, whereupon the crude product precipitated, was filtered and the filtered solid was washed with diethyl ether (100 ml) to give a damp mass (12 g). The reduced liquors (the reduced slurry liquids remaining after filtration and the diethyl ether washings) were purified by column chromatography (silica, 800 ml), using 1 : 0.98 : 0.2 DCM : EtOAc : MeOH (4L) as eluent, to give a further batch of product (4 g).

Both batches of product were combined (appeared impure by 1H NMR), and were purified by column chromatography (silica, 800 ml), using 3 to 4 % MeOH in DCM (3L) as eluent, to give 13 g of material which still appeared impure (by TLC).

This resulting impure solid (13 g) was slurried in 1 : 0.96 : 0.4 of DCM : EtOAc : MeOH and the solid remaining was separated by filtration and dried, to give the title compound *N*-{[1,6-diethyl-4-(tetrahydro-2*H*-pyran-4-ylamino)-1*H*-pyrazolo[3,4-*b*]pyridin-5-yl]methyl}-3-methyl-5-isoxazolecarboxamide as an off-white solid (9 g).

The reduced liquors (the reduced slurry liquids) were purified by column chromatography (silica, 400 ml) using 1 : 0.96 : 0.4 DCM : EtOAc : MeOH (2L) as eluent, giving, after reduction *in vacuo,* a further batch of product.

The two batches of product were combined to give 10.714 g in total of the title compound *N*-{[1,6-diethyl-4-(tetrahydro-2*H*-pyran-4-ylamino)-1*H*-pyrazolo[3,4-*b*]pyridin-5-yl]methyl}-3-methyl-5-isoxazolecarboxamide as an off-white solid (more than about 95% purity as measured by NMR in $CDCl_3$ solvent (not the NMR given below)).

$^1H$ NMR (500MHz in $d_6$-DMSO, δ (delta) ppm): 9.38 (br t, 1H), 8.04 (s, 1H), 7.00 (s, 1H), 6.64 (d, 1H), 4.52 (d, 2H), 4.33 (q, 2H), 4.12 (br s, 1H), 3.88 (d, 2H), 3.57 (t, 2H), 2.95 (q, 2H), 2.29 (s, 3H), 1.93 (d, 2H), 1.54 (q, 2H), 1.35 (t, 3H), 1.24 (t, 3H). Plus trace other peaks: possibly solvent.

**Example 238 (Synthesis B)** ***N*-{[1,6-diethyl-4-(tetrahydro-2*H*-pyran-4-ylamino)-1*H*-pyrazolo[3,4-*b*]pyridin-5-yl] methyl}-1-methyl-1H-pyrazole-3-carboxamide**

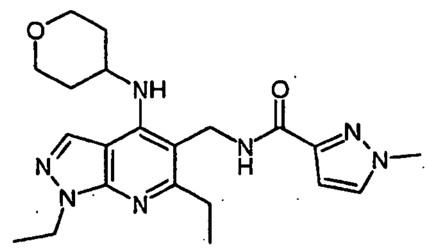

An example of a specific synthesis (Synthesis B) of Example 238 is as follows:

HATU (1.095g) was dissolved in DMF (6ml) and an aliquot (0.25ml) of this solution added to 1-methyl-1*H*-pyrazole-3-carboxylic acid (0.12mmol). DIPEA (0.052ml) was added and the solution shaken for 10mins. Meanwhile Intermediate 16 (364mg) was dissolved in DMF (2.4ml) and an aliquot (0.2ml) of this solution added. The solution was shaken for 10min and left to stand for 18h. DMF was removed in a Genevac vacuum centrifuge and the residue dissolved in chloroform (0.3ml) and applied to an SPE cartridge (1g, aminopropyl) pre-washed with chloroform (6ml). The cartridge was eluted with chloroform (3ml) and the solvent removed in a Genevac. The residue was dissolved in 50% DMSO in methanol (0.5ml) and purified by mass directed autoprep HPLC followed by SPE cartridge (0.5g, Flash $NH_2$) eluting with 10% methanol in ethyl acetate to give Example 238 (20.7mg). LCMS showed $MH^+$ = 462; $T_{RET}$ = 1.87min.

**Example 238 (Synthesis C)** ***N*-{[1,6-diethyl-4-(tetrahydro-2*H*-pyran-4-ylamino)-1*H*-pyrazolo[3,4-*b*]pyridin-5-yl] methyl}-1-methyl-1H-pyrazole-3-carboxamide**

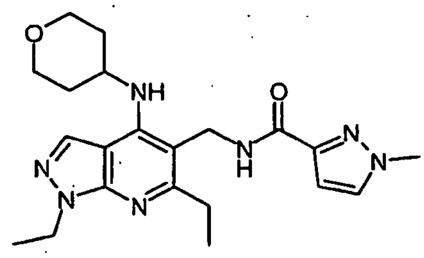

1-methyl-1*H*-pyrazole-3-carboxylic acid (6.24g, ca. 1.0 eq.), HBTU (19.3g or 19.5g, ca. 1.08 eq.) and DMF (300ml, 20 vols) were stirred under nitrogen, followed by the addition of diisopropylethylamine (18ml, 2.0 eq.). The mixture was left to stir for 10 minutes and 5-(aminomethyl)-1,6-diethyl-*N*-(tetrahydro-2*H*-pyran-4-yl)-1*H*-pyrazolo[3,4-*b*] pyridin-4-amine (15g, 1.0 eq.) (e.g. which can be as prepared in Intermediate 16) was added and washed in with DMF (10 ml). The reaction was left to stir for 3h, and then left to stand overnight prior to work-up. Work-up proceded by pouring the mixture into saturated sodium hydrogen carbonate solution (3 L) and extracting with DCM (2 x 800 mL). The combined organic layers were backwashed with water (2 x 500 mL), dried over $MgSO_4$ (50 g), filtered, reduced *in vacuo* to give a brown oil.

This crude material was purified approximately as follows:

Crude material was purified using gradient silica (500g) column chromatography (2-5% MeOH in DCM); after evaporation, the resulting solid was dissolved in DCM (300 mL), washed with water (2 x 200 mL), to remove DMF, dried ($MgSO_4$), filtered and reduced in vacuo. Recrystallisation from EtOAc / hexane (100 ml, 1: 1) and drying in a vacuum oven at 40˚C gave the title compound as an off-white solid (15.17 g).

## Examples 240 to 288

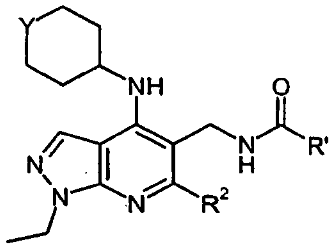

$R^2$ = Me or Et

Y = O or $NCONH_2$

*General Procedure*

A solution of the carboxylic acid R'COOH (0.125mmol) in DMF (0.1 ml) was treated with a solution of Intermediate 15, 16, 26 or 27 (0.069mmol) in DMF (0.2ml). DIPEA (0.21 mmol) was added followed by a solution of PyBOP (0.104mmol) in DMF (0.2ml). The mixture was shaken briefly and left to stand at room temperature for 18h and then concentrated *in vacuo.* The residue was purified by mass directed autoprep HPLC. Where necessary, compounds were further purified by SPE cartridges (Flash $NH_2$; 0.5g) eluting with 10% methanol in ethyl acetate and ammonia in methanol, and SCX2; 0.5g) eluting with ammonia in methanol.

| Example | Y | $R^2$ | $R'CO_2H$ | One possible source of or reference for acid $R'CO_2H$ | Starting Material | $[MH]^+$ | LC/M S Retention Time (mins ) |
|---|---|---|---|---|---|---|---|
| 240 | $NCONH_2$ | Et | 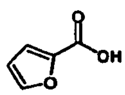 | Aldrich Chemical Company, Inc. | Intermediate 26 | 440 | 2.12 |
| 241 | $NCONH_2$ | Et | 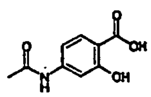 | Apin Chemicals Ltd. | Intermediate 26 | 523 | 2.28 |
| 242 | $NCONH_2$ | Et | 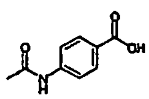 | Aldrich Chemical Company, Inc. | Intermediate 26 | 507 | 2.18 |
| 243 | $NCONH_2$ | Et | 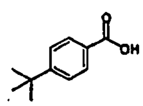 | Aldrich Chemical Company, Inc. | Intermediate 26 | 506 | 2.64 |
| 244 | $NCONH_2$ | Et | 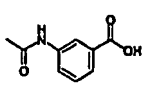 | Lancaster Synthesis Ltd. | Intermediate 26 | 507 | 2.19 |
| 245 | $NCONH_2$ | Et | 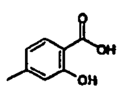 | Aldrich Chemical Company, Inc. | Intermediate 26 | 480 | 2.44 |
| 246 | $NCONH_2$ | Et | 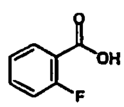 | Aldrich Chemical Company, Inc. | Intermediate 26 | 468 | 2.23 |
| 247 | $NCONH_2$ | Et | 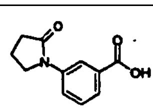 | WO 2003/032971 | Intermediate 26 | 533 | 2.25 |

(continued)

| Example | Y | $R^2$ | R'$CO_2$H | One possible source of or reference for acid R'$CO_2$H | Starting Material | $[MH]^+$ | LC/M S Retention Time (mins ) |
|---|---|---|---|---|---|---|---|
| 248 | $NCONH_2$ | Et | 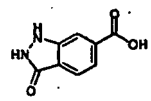 | Not known | Intermediate 26 | 506 | 2.12 |
| 249 | O | Et | 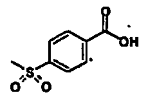 | Aldrich Chemical Company, Inc. | Intermediate 16 | 486 | 2.31 |
| 250 | O | Et | 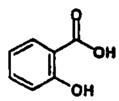 | Aldrich Chemical Company, Inc. | Intermediate 16 | 424 | 2.54 |
| 251 | O | Et | 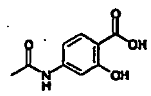 | Apin Chemicals Ltd. | Intermediate 16 | 481 | 2.42 |
| 252 | O | Et | 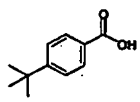 | Aldrich Chemical Company, Inc. | Intermediate 16 | 464 | 2.88 |
| 253 | O | Et | 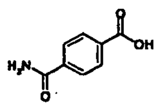 | ChemPacific Corporation | Intermediate 16 | 451 | 2.19 |
| 254 | O | Et | 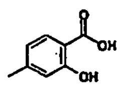 | Aldrich Chemical Company, Inc. | Intermediate 16 | 438 | 2.68 |
| 255 | O | Et | 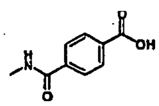 | WO 96/30329 | Intermediate 16 | 465 | 2.24 |
| 256 | O | Et | 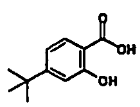 | GB 1132542 | Intermediate 16 | 480 | 3.01 |
| 257 | O | Et | 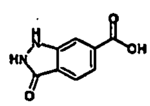 | Not known | Intermediate 16 | 464 | 2.22 |
| 258 | O | Et | 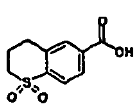 | Not known Not known | Intermediate 16 | 512 | 2.36 |
| 259 | $NCONH_2$ | Me | 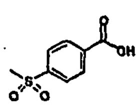 | Aldrich Chemical Company, Inc. | Intermediate 27 | 514 | 2.09 |
| 260 | $NCONH_2$ | Me | 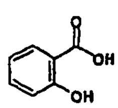 | Aldrich Chemical Company, Inc. | Intermediate 27 | 452 | 2.25 |

(continued)

| Example | Y | $R^2$ | R'$CO_2$H | One possible source of or reference for acid R'$CO_2$H | Starting Material | $[MH]^+$ | LC/M S Retention Time (mins ) |
|---|---|---|---|---|---|---|---|
| 261 | $NCONH_2$ | Me | 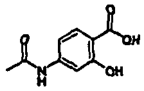 | Apin Chemicals Ltd. | Intermediate 27 | 509 | 2.20 |
| 262 | $NCONH_2$ | Me | 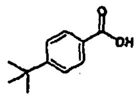 | Aldrich Chemical Company, Inc. | Intermediate 27 | 492 | 2.58 |
| 263 | $NCONH_2$ | Me | 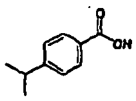 | Aldrich Chemical Company, Inc. | Intermediate 27 | 478 | 2.48 |
| 264 | $NCONH_2$ | Me | 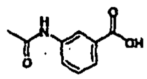 | Lancaster Synthesis Ltd. | Intermediate 27 | 493 | 2.05 |
| 265 | $NCONH_2$ | Me | 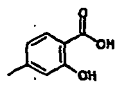 | Aldrich Chemical Company, Inc. | Intermediate 27 | 466 | 2.35 |
| 266 | $NCONH_2$ | Me | 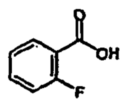 | Aldrich Chemical Company, Inc. | Intermediate 27 | 454 | 2.12 |
| 267 | $NCONH_2$ | Me | 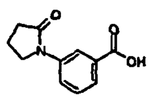 | WO 2003/032971 | Intermediate 27 | 519 | 2.18 |
| 268 | $NCONH_2$ | Me | 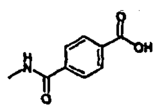 | WO 96/30329 | Intermediate 27 | 493 | 2.06 |
| 269 | $NCONH_2$ | Me | 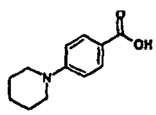 | Maybridge plc | Intermediate 27 | 519 | 2.44 |
| 270 | $NCONH_2$ | Me | 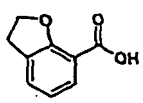 | Fluorochem Ltd. | Intermediate 27 | 478 | 2.27 |
| 271 | $NCONH_2$ | Me | 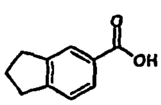 | Tyger Scientific Inc | Intermediate 27 | 476 | 2.41 |
| 272 | $NCONH_2$ | Me | 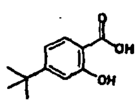 | GB 1132542 | Intermediate 27 | 508 | 2.64 |
| 273 | $NCONH_2$ | Me | 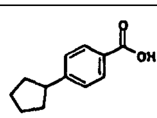 | J. Am. Chem. Soc (1968) 90 (13), 3404 | Intermediate 27 | 504 | 2.65 |

(continued)

| Example | Y | $R^2$ | R'$CO_2$H | One possible source of or reference for acid R'$CO_2$H | Starting Material | $[MH]^+$ | LC/M S Retention Time (mins ) |
|---|---|---|---|---|---|---|---|
| 274 | $NCONH_2$ | Me | 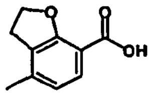 | Not known | Intermediate 27 | 492 | 2.37 |
| 275 | O | Me | 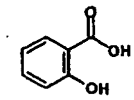 | Aldrich Chemical Company, Inc. | Intermediate 15 | 410 | 2.46 |
| 276 | O | Me | 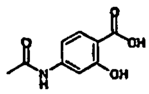 | Apin Chemicals Ltd. | Intermediate 15 | 467 | 2.34 |
| 277 | O | Me | 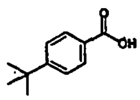 | Aldrich Chemical Company, Inc. | Intermediate 15 | 450 | 2.81 |
| 278 | O | Me | 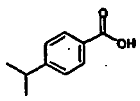 | Aldrich Chemical Company, Inc. | Intermediate 15 | 436 | 2.72 |
| 279 | O | Me | 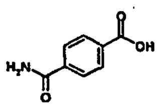 | ChemPacific Corporation | Intermediate 15 | 437 | 2.11 |
| 280 | O | Me | 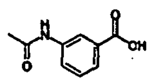 | Lancaster Synthesis Ltd. | Intermediate 15 | 451 | 2.23 |
| 281 | O | Me | 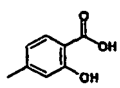 | Aldrich Chemical Company, Inc. | Intermediate 15 | 424 | 2.59 |
| 282 | O | Me | 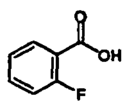 | Aldrich Chemical Company, Inc. | Intermediate 15 | 412 | 2.30 |
| 283 | O | Me | 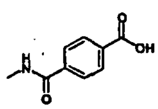 | WO 96/30329 | Intermediate 15 | 451 | 2.15 |
| 284 | O | Me | 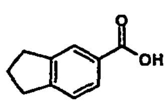 | Tyger Scientific Inc | Intermediate 15 | 434 | 2.64 |
| 285 | O | Me | 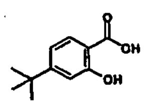 | GB 1132542 | Intermediate 15 | 466 | 2.93 |
| 286 | O | Me | 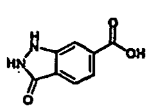 | Not known | Intermediate 15 | 450 | 2.14 |

(continued)

| Example | Y | $R^2$ | R'$CO_2$H | One possible source of or reference for acid R'$CO_2$H | Starting Material | $[MH]^+$ | LC/M S Retention Time (mins ) |
|---|---|---|---|---|---|---|---|
| 287 | O | Me | 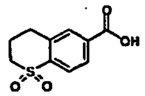 | Not known | Intermediate 15 | 498 | 2.28 |
| 288 | O | Me | 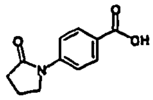 | Butt Park Ltd. | Intermediate 15 | 477 | 2.34 |

**Example 289 N-{[1,6-diethyl-4-(tetrahydro-2H-pyran-4-ylamino)-1H-pyrazolo[3,4-b]pyridin-5-yl]methyl}-1-pyrrolidinecarboxamide**

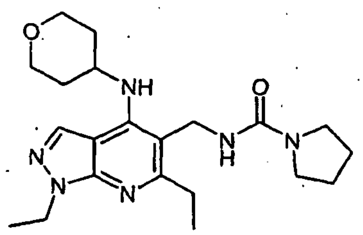

Intermediate 16 (92mg) was added to a mixture containing 3-methyl-1-(1-pyrrolidinylcarbonyl)-1*H*-imidazol-3-ium iodide [e.g. see *Organic Letters, 2002, 4* (9), 1411] (50mg) and DIPEA (0.057ml) in dichloromethane (0.2ml). The mixture was stirred at room temperature for 20h and then concentrated *in vacuo.* The residue was purified by SPE cartridge (silica) followed by mass directed autoprep to give Example 289 as a gum (50mg). LCMS showed $MH^+$ = 401; $T_{RET}$ = 2.2min.

**Example 290 N-{[1,6-diethyl-4-(tetrahydro-2H-pyran-4-ylamino)-1H-pyrazolo[3,4-b]pyridin-5-yl]methyl}-1-piperidinecarboxamide**

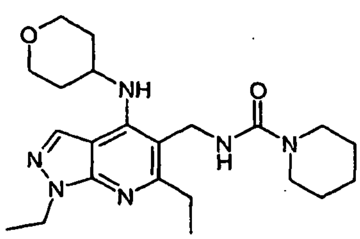

Example 290 was prepared in an analogous manner to Example 289 from Intermediate 16 (430mg), 3-methyl-1-(1-piperidinylcarbonyl)-1*H*-imidazol-3-ium iodide [e.g. see WO 03/051841] (250mg) and DIPEA (0.27ml) in dichloromethane (1.4ml). LCMS showed MH' = 415; $T_{RET}$ = 2.3min.

**Example 291 N-{[1,6-diethyl-4-(tetrahydro-2H-pyran-4-ylamino)-1H-pyrazolo[3,4-b]pyridin-5-yl]methyl}-4-morpholinecarboxamide**

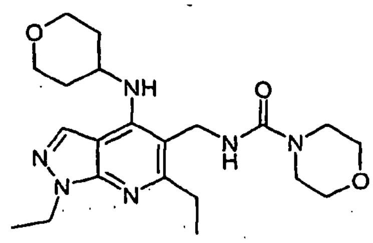

Example 291 was prepared in an analogous manner to Example 289 from Intermediate 16 (425mg), 3-methyl-1-(4-morpholinylcarbonyl)-1*H*-imidazol-3-ium iodide [e.g. see

*Combinatorial Chemistry and High Throughput Screening (2002), 5(3), 219*] (250mg) and DIPEA (0.27ml) in dichloromethane (0.7ml). LCMS showed $MH^+$ = 417; $T_{RET}$ = 2.14min.

**Example 292 N-{[1-ethyl-6-methyl-4-(tetrahydro-2H-pyran-4-ylamino)-1H-pyrazolo[3,4-b]pyridin-5-yl]methyl}-1-pyrrolidinecarboxamide**

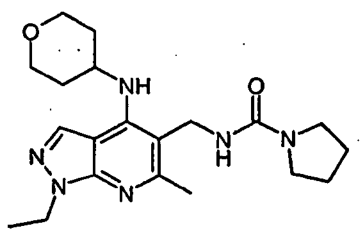

Example 292 was prepared in an analogous manner to Example 289 from Intermediate 15 (420mg), 3-methyl-1-(1-pyrrolidinylcarbonyl)-1*H*-imidazol-3-ium iodide (240mg) and DIPEA (0.28ml) in dichloromethane (3ml). LCMS showed $MH^+$ = 387; $T_{RET}$ = 2.13min.

**Example 293 *N*-{[1-ethyl-6-methyl-4(tetrahydro-2*H*-pyran-4-ylamino)-1*H*-pyrazolo[3,4-*b*]pyridin-5-yl]methyl}-4-morpholinecarboxamide**

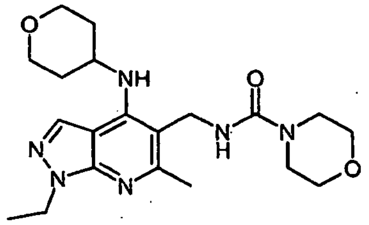

Example 293 was prepared in an analogous manner to Example 289 from Intermediate 15 (370mg), 3-methyl-1-(4-morpholinylcarbonyl)-1*H*-imidazol-3-ium iodide (231 mg) and DIPEA (0.25ml) in dichloromethane (3ml). LCMS showed $MH^+$ = 403; $T_{RET}$ = 2.04min.

**Example 294 *N*-{[1-ethyl-6-methyl-4-(tetrahydro-2*H*-pyran-4-ylamino)-1*H*-pyrazolo[3,4-*b*]pyridin-5-yl]methyl}-N'-(tetrahydro-2*H*-pyran-4-yl)urea**

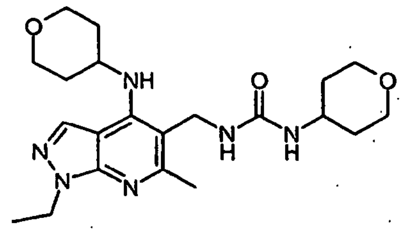

A solution of tetrahydro-2*H*-pyran-4-amine (150mg) and triethylamine (0.2ml) in dichloromethane (3ml) was added to an ice-cooled solution of 4-nitrophenyl-chloroformate (299mg) in dichloromethane (2ml). The mixture was stirred for 18h during which time it was allowed to warm to room temperature. It was then diluted with dichloromethane (5ml), washed with water (2ml) and purified on an SPE cartridge (silica) eluting with a gradient of 0-100% ethyl acetate in cyclohexane to give 4-nitrophenyl tetrahydro-2*H*-pyran-4-ylcarbamate (350mg). This was dissolved in dichloromethane (5ml) and then DIPEA (0.27ml) and Intermediate 15 (418mg) were added. The mixture was stirred at room temperature for 18h and then evaporated *in vacuo.* The residual gum was purified by SPE cartridge (silica) eluting with a gradient of 0-100% ethyl acetate in cyclohexane to give Example 294 (425mg). LCMS showed $MH^+$ = 417; $T_{RET}$ = 2.00min.

**Example 295 1-{[1-ethyl-4-(tetrahydro-2H-pyran-4-ylamino)-1H-pyrazolo[3,4-b]pyridin-5-yl]methyl}-3-methyl-2-imidazolidinone**

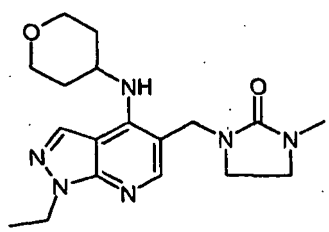

1-methyl-2-imidazolidinone [e.g. available from *Acros Organics USA*] (51mg) was added dropwise to a stirred ice-cooled mixture of sodium hydride (24.4mg) in DMF (2ml) under nitrogen. The mixture was thus stirred for 30mins and was then treated dropwise with a solution of Intermediate 6 (150mg) in DMF (2ml) at 0°C under nitrogen. The mixture was stirred for 3h, quenched with methanol and evaporated to dryness *in vacuo.* The residual solid was partitioned between dichloromethane (5ml) and water (2ml) and the organic extract purified on an SPE cartridge (silica) eluding with a gradient of 1 - 7.5% methanol in dichloromethane to give Example 295 as a white solid (55mg). LCMS showed $MH^+$ = 359; $T_{RET}$ = 2.00min.

**Example 296 1-{[1-ethyl-6-methyl-4-(tetrahydro-2*H*-pyran-4-ylamino)-1*H*-pyrazolo[3,4-*b*]pyridin-5-yl]methyl}-3-methyl-2-imidazolidinone**

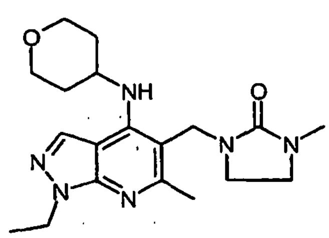

Example 296 was prepared in an analogous manner to Example 295 from Intermediate 7 (150mg) and 1-methyl-2-imidazolidinone (48.6mg) LCMS showed $MH^+$ = 373; $T_{RET}$ = 2.04min.

**Example 297 1-{[1,6-diethyl-4-(tetrahydro-2*H*-pyran-4-ylamino)-1*H*-pyrazolo[3,4-b]pyridin-5-yl]methyl}-2-piperidinone**

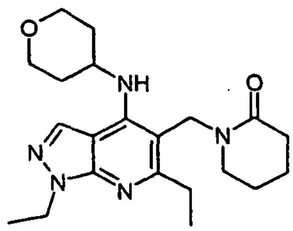

A mixture containing Intermediate 16 (64mg), 5-chlorovalerylchloride (0.026ml) and DIPEA (0.037ml) in acetonitrile (1.25ml) was stirred at room temperature for 24h. The mixture was diluted with dichloromethane (10ml) and washed with dilute aqueous sodium chloride solution (2 x 7ml) then evaporated *in vacuo* to give 5-chloro-*N*-{[1,6-diethyl-4-(tetrahydro-2*H*-pyran-4-ylamino)-1*H*-pyrazolo[3,4-*b*]pyridin-5-yl]methyl}pentanamide (90mg). This was dissolved in DMF (2.5ml) and added to sodium hydride (10mg) and the resulting mixture stirred at room temperature for 20h. It was then quenched with water (8ml) and extracted with dichloromethane (2 x 7ml). The combined organic extracts were blown down to dryness and the residue purified by SPE cartridge (Flash $NH_2$) eluting with methanol to give Example 297 as a beige gum (91 mg). LCMS showed $MH^+$ = 386; $T_{RET}$ = 2.06min.

**Example 298 1-{[1-ethyl-6-methyl-4-(tetrahydro-2H-pyran-4-ylamino)-1H-pyrazolo[3,4-b]pyridin-5-yl]methyl}-2-pyrrolidinone**

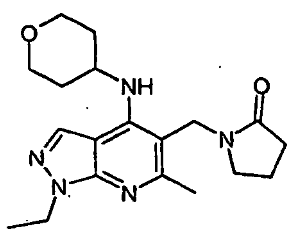

Example 298 was prepared in an analogous manner to Example 297 from Intermediate 15 (58mg) and 4-chlorobutyryl chloride (0.024ml). LCMS showed $MH^+$ = 358; $T_{RET}$ = 1.95min.

**Example 299 1-{[1-ethyl-6-methyl-4-(tetrahydro-2*H*-pyran-4-ylamino)-1*H*-pyrazolo[3,4-*b*]pyridin-5-yl]methyl}-2-piperidinone**

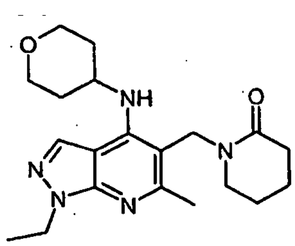

Example 299 was prepared in an analogous manner to Example 297 from Intermediate 15 (58mg) and 5-chlorovalerylchloride (0.025ml). LCMS showed $MH^+$ = 372; $T_{RET}$ = 2.02min.

**Example 300 2-{[1-ethyl-6-methyl-4-(tetrahydro-2*H*-pyran-4-ylamino)-1*H*-pyrazolo[3,4-*b*]pyridin-5-yl]methyl}-2,3-dihydro-1*H*-isoindol-1-one**

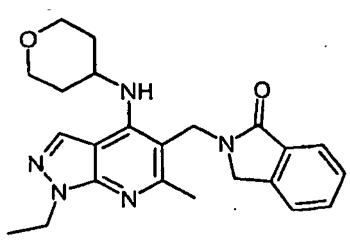

A solution of methyl 2-(bromomethyl)benzoate [e.g. available from *Apin Chemicals Ltd.]* (83mg) in acetonitrile (0.5ml) was added to a solution of Intermediate 15 (95mg) in acetontrile (0.5ml). DIPEA (0.058ml) was added and the solution stirred at room temperature for 18h and then blown down to dryness. The residue was dissolved in dichloromethane (5ml), washed with dilute aqueous sodium chloride, solution (5ml) and purified on an SPE cartridge (silica). Elution with 0-100% ethyl acetate in cyclohexane gave Example 300 as a cream solid (88mg). LCMS showed $MH^+$ = 406; $T_{RET}$ = 2.30min.

**Example 301 5-{[1-ethyl-6-methyl-4-(tetrahydro-2*H*-pyran-4-ylamino)-1*H*-pyrazolo[3,4-*b*]pyridin-5-yl]methyl}-5,6-dihydro-4*H*-furo[2,3-c]pyrrol-4-one**

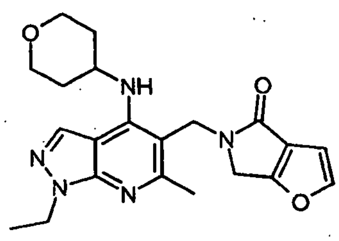

Ethyl 2-(chloromethyl)-3-furancarboxylate (93mg) was added to a solution of Intermediate 15 (130mg) in anhydrous acetonitrile (2ml). DIPEA (0.08ml) was added and the solution stirred under nitrogen at room temperature for 3 days. It was then diluted with methanol (5ml) and purified by SPE cartridge (SCX2) eluting with methanol followed by SPE cartridge (silica) eluting with 0 - 100% ethyl acetate in cyclohexane to give ethyl 2-[({[1-ethyl-6-methyl-4-(tetrahydro-2*H*-pyran-4-ylamino)-1*H*-pyrazolo[3,4-*b*]pyridin-5-yl]methyl}amino)methyl]-3-furancarboxylate as a brown gum (80mg). This was dissolved in ethanol (0.53ml), treated with aqueous sodium hydroxide (2M, 0.36ml) and the solution stirred at room temperature for 18h. The solution was neutralised with hydrochloric acid (2M, 0.23ml) and blown down to dryness. The residual solid was diluted with dichloromethane (2ml) and DMF (one drop) and treated with thionyl chloride (0.074ml). After stirring at room temperature for 1h, DIPEA (0.063ml) was added and after a further 24 hours at room temperature a further portion of thionyl chloride (0.074ml) was added and stirring continued for a further 24h. The mixture was diluted with dichloromethane (10ml), washed with aqueous sodium bicarbonate (10ml) and blown down to dryness. The residue was purified by mass directed autoprep HPLC followed' by SPE eluting with ethyl acetate to give Example 301 (7mg). LCMS showed $MH^+$ = 396; $T_{RET}$ = 2.14min.

**Example 302 4-({5-[({[4-(1,1-dimethylethyl)-2-hydroxyphenyl]carbonyl}amino)-methyl]-1,6-diethyl-1H-pyrazolo[3,4-b]pyridin-4-yl}amino)-1-piperidine-carboxamide formate**

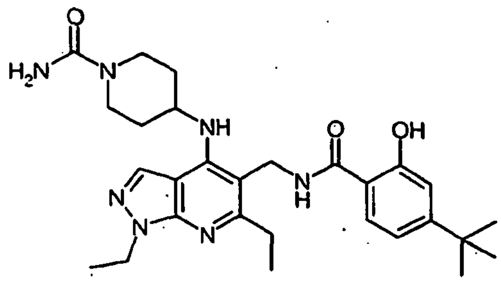

, formate

A solution of Intermediate 26 (20mg) in DMF (1ml) was added to 4-(1,1-dimethylethyl)-2-hydroxybenzoic acid (13.6mg) followed by a solution of PyBOP (36mg) in DMF (0.3ml) and DIPEA (0.025ml). The mixture was allowed to stand at room temperature for 18h and was then evaporated to dryness *in vacuo.* The residue was taken up in dichloromethane, washed with water and purified by mass directed autoprep HPLC to give Example 302 as a solid (11.8mg). LCMS showed $MH^+ = 522$; $T_{RET} = 2.71$ min.

**Example 303 4-({1-ethyl-6-methyl-5-[({[4-(4-morpholinyl)phenyl]-carbonyl}amino)methyl]-1*H*-pyrazolo[3,4-*b*] pyridin-4-yl}amino)-1-piperidine-carboxamide, formate salt**

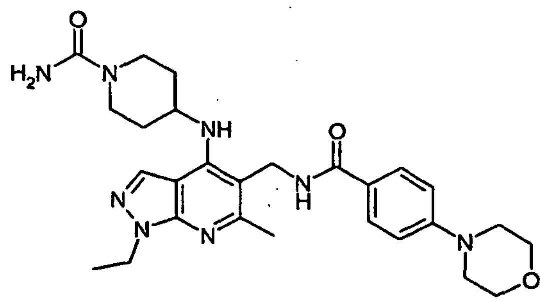

To a solution of 4-(4-morpholinyl)benzoic acid [e.g. available from Maybridge Chemical Company Ltd,] (13.8mg) in DMF (0.5ml) was added a solution of HATU (27mg) in DMF (0.5ml) followed by DIPEA (0.026ml). The solution was left to stand for 15mins and then Intermediate 27 (20mg) was added. The solution was allowed to stand at room temperature for 18h and was then evaporated to dryness *in vacuo*. The residue was purified by SPE cartridge (Flash $NH_2$) eluting sequentially with chloroform and 20% methanol in ethyl acetate followed by mass directed autoprep HPLC to give Example 303 as a solid (14.8mg). LCMS showed $MH^+ = 522$; $T_{RET} = 2.71$ min.

**Examples 304 to 306**

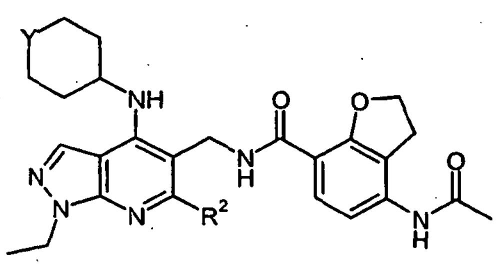

$R^2$ = Me or Et

Y = O or $NCONH_2$

*General Procedure*

A solution of Intermediate 28 (0.06mmol), DIPEA (0.18mmol) and HATU (0.06mmol) in DMF (0.5ml) was treated

with a solution of the appropriate amine Intermediate as stated below (0.06mmol) in DMF (0.2ml). The mixture was stirred at room temperature for 16h and then blown down to dryness and the residue purified by mass directed autoprep HPLC.

| Example | Y | $R^2$ | Amine starting material | $[MH]^+$ | LC/MS Retention Time (mins) |
|---|---|---|---|---|---|
| 304 | $NCONH_2$ | Et | Intermediate 26 | 549 | 2.15 |
| 305 | $NCONH_2$ | Me | intermediate 27 | 535 | 2.09 |
| 306 | O | Et | Intermediate 16 | 507 | 2.32 |

**Example 307 *N*-{[1,6-diethyl-4-(tetrahydro-2*H*-pyran-4-ylamino)-1*H*-pyrazolo[3,4-*b*]pyridin-5-yl]methyl}-4-fluorobenzamide**

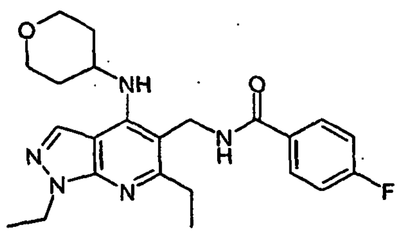

4-Flurobenzoyl chloride (0.048ml) was added to a solution of Intermediate 16 (108mg) and DIPEA (0.075ml) in acetonitrile (2ml). The solution was stirred at room temperature under an atmosphere of nitrogen for 2h. It was then diluted with dichloromethane (3ml) and washed with brine (3ml). The organic extract was applied to an SPE cartridge (5g, aminopropyl) and eluted with dichloromethane and with methanol. Relevant fractions were evaporated in vacuo and the residue purified by SPE cartridge (20g, silica) eluting with a gradient of 50 -100% ethyl acetate in cyclohexane to give Example 307 as a white solid (106mg). LCMS showed $MH^+ = 426$; $T_{RET} = 2.49$min.

**Example 308 N-{[1,6-diethyl-4-(tetrahydro-2H-pyran-4-ylamino)-1H-pyrazolo[3,4-b]pyridin-5-yl]methyl}-2,4-difluorobenzamide**

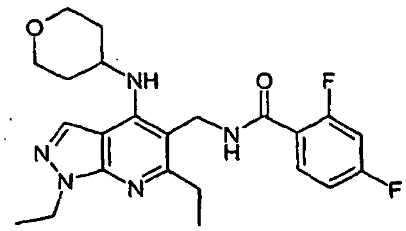

2,4-Diflurobenzoyl chloride (0.050ml) was added to a solution of Intermediate 16 (116mg) and DIPEA (0.075ml) in acetonitrile (2ml). The solution was stirred at room temperature under an atmosphere of nitrogen for 2h. It was then diluted with brine (2ml) and dichloromethane (2ml). The organic extract separated using a hydrophobic frit and blown down to dryness. The residual gum was purified by SPE cartridge (5g, aminopropyl) eluting with methanol. The eluent was blown down to dryness and the residual gum triturated with ether to give Example 308 as a white solid (149mg). LCMS showed $MH^+ = 444$; $T_{RET} = 2.47$min.

**Example 309 *N*-{[1,6-diethyl-4-(tetrahydro-2*H*-pyran-4-ylamino)-1*H*-pyrazolo[3,4-*b*]pyridin-5-yl]methyl}-2-methyl-1,3-thiazole-4-carboxamide**

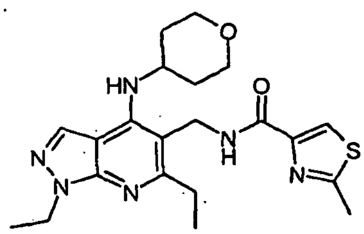

2-Methyl-1,3-thiazole-4-carboxylic acid [e.g. available from *Acros Organics*] (125mg) was dried overnight under vacuum over phosphorus pentoxide and was then suspended in dry dichloromethane (2ml) and treated at 20°C with oxalyl chloride (0.077ml) and DMF (1 drop). The mixture was stirred at room temperature for 25mins and was then added dropwise to a solution of Intermediate 16 (240mg) in anhydrous acetonitrile (5ml). DIPEA (0.155ml) was added and the solution stirred at room temperature for 3h. The solution was blown down to dryness and purified by mass directed autoprep HPLC to give a pale orange gum. The gum was further purified using an SPE cartridge (5g, aminopropyl) which had been pre-washed with methanol. Elution with methanol gave Example 309 as a pale orange gum (287mg). LCMS showed $MH^+ = 429$; $T_{RET} = 2.37$min.

**Example 310 N-{[1,6-diethyl-4-(tetrahydro-2H-pyran-4-ylamino)-1H-pyrazolo[3,4-b]pyridin-5-yl]methyl}-2-methyl-1,3-oxazole-4-carboxamide**

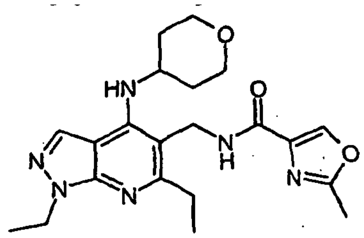

Methyl 2-methyl-1,3-oxazole-4-carboxylate [e.g. available from *AstaTech, Inc*] (50mg) in ethanol (1ml) was treated with aqueous sodium hydroxide (2M, 0.706ml). The clear solution was stirred at room temperature for 18h and then treated with aqueous hydrochloric acid (2M, 0.541 ml). The solution was blown down to dryness and the residual solid dried under vacuum over phosphorus pentoxide for 4 days. The solid was then suspended in dry dichloromethane (1ml) and treated at 20°C with oxalyl chloride (0.032ml) and DMF (1 drop). The mixture was stirred at room temperature for 30mins and was then added dropwise to a solution of Intermediate 16 (98mg) in anhydrous acetonitrile (2ml) and stirred at room temperature for 22h. The mixture was diluted with dichloromethane (15ml), washed with dilute aqueous sodium chloride (2 x 15ml) and blown down to dryness. The residue was purified by mass directed autoprep HPLC to give a gum which was further purified using an SPE cartridge (1g, aminopropyl) which had been pre-washed with methanol. Elution with methanol gave Example 310 as a clear colourless film (56mg). LCMS showed $MH^+ = 413$; $T_{RET}=$ 2.14min.

**Example 311 N-{[1,6-diethyl-4-(tetrahydro-2H-pyran-4-ylamino)-1H-pyrazolo[3,4-b]pyridin-5-yl]methyl}-2-methyl-1,3-thiazole-5-carboxamide**

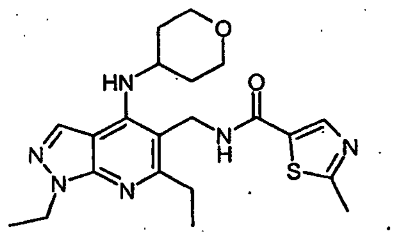

Ethyl 2-methyl-1,3-thiazole-5-carboxylate [e.g. available from *Interchim S.A.*] (61mg) in ethanol (1ml) was treated with aqueous sodium hydroxide (2M, 0.706ml). The clear solution was stirred at room temperature for 18h and then treated with aqueous hydrochloric acid (2M, 0.54ml). The solution was blown down to dryness and the residual solid dried under vacuum over phosphorus pentoxide. The solid was then suspended in dry dichloromethane (1ml) and treated at 20°C with oxalyl chloride (0.032ml) and DMF (1 drop). The mixture was stirred at room temperature for 30mins and was then added dropwise to a solution of Intermediate 16 (98mg) in anhydrous acetonitrile (2ml). DIPEA (0.064ml) was added and the solution stirred at room temperature for 20h. The mixture was diluted with dichloromethane (15ml), washed with dilute aqueous sodium chloride (2 x 15ml) and blown down to dryness. The residue was purified by mass directed autoprep HPLC to give a film which was further purified using an SPE cartridge (2g, aminopropyl) which had been pre-washed with methanol. Elution with methanol gave Example 311 as a white solid (87mg). LCMS showed $MH^+$ = 429; $T_{RET}$ = 1.94min.

**Example 312 *N*-{[1-ethyl-6-methyl-4-(tetrahydro-2*H*-pyran-4-ylamino)-1*H*-pyrazolo[3,4-*b*]pyridin-5-yl]methyl}-2,4-difluorobenzamide**

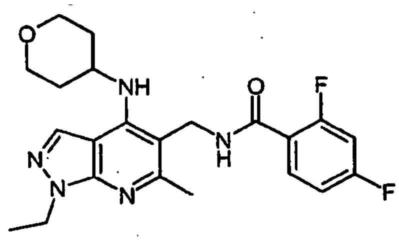

2,4-Diflurobenzoyl chloride [e.g. available from *Aldrich Chemical Company*] (0.050ml) was added to a solution of Intermediate 15 (116mg) and DIPEA (0.075ml) in acetonitrile (2ml). The solution was stirred at room temperature under an atmosphere of nitrogen for 2h. It was then diluted with brine (2ml) and dichloromethane (2ml). The organic extract was separated using a hydrophobic frit and blown down to dryness and the residual gum purified by SPE cartridge (5g, aminopropyl) eluting with methanol. The eluent was blown down to dryness and the residual gum triturated with ether to give Example 312 as a white solid (136mg). LCMS showed $MH^+$ = 430; $T_{RET}$ = 2.32min.

**Example 313 N-{[1-ethyl-6-methyl-4-(tetrahydro-2H-pyran-4-ylamino)-1H-pyrazolo[3,4-b]pyridin-5-yl]methyl}-4-fluorobenzamide**

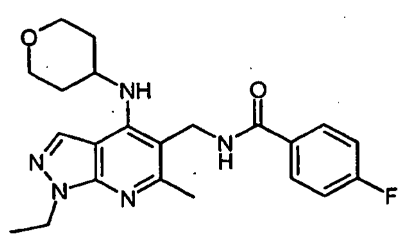

4-Flurobenzoyl chloride [e.g. available from *Aldrich Chemical Company*] (0.04ml) was added to a solution of

Intermediate 15 (100mg) and DIPEA (0.07ml) in acetonitrile (2ml). The solution was stirred at room temperature under an atmosphere of nitrogen for 16h. It was then diluted with brine (2ml) and dichloromethane (2ml). The organic extract separated using a hydrophobic frit and evaporated to dryness. The residual gum was purified by SPE cartridge (5g, aminopropyl) eluting with methanol. The eluent was blown down to dryness to give Example 313 as a white solid (104mg). LCMS showed $MH^+ = 412$; $T_{RET} = 2.50$min.

**Example 314 2-{[1-ethyl-6-methyl-4-(tetrahydro-2*H*-pyran-4-ylamino)-1*H*-pyrazolo[3,4-*b*]pyridin-5-yl]methyl}-6-fluoro-2,3-dihydro-1*H*-isoindol-1-one**

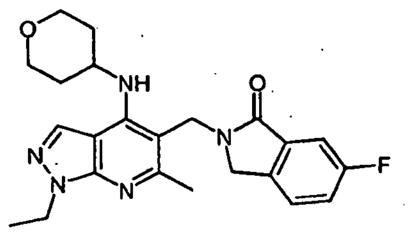

A solution containing Intermediate 15 (117mg), methyl 2-(bromomethyl)-5-fluorobenzoate [e.g. see J. Med. Chem., (1992), 35 (7), 1200] (106mg) and DIPEA (0.1ml) in anhydrous acetonitrile (2ml) was stirred at room temperature under an atmosphere of nitrogen for 18h. It was then diluted with brine (5ml) and dichloromethane (5ml). The organic extract separated using a hydrophobic frit and evaporated to dryness. The residue was purified on an SPE cartridge (5g, aminopropyl) eluting with methanol followed by SPE cartridge (10g; silica) eluting with ethyl acetate to give Example 314 as a white solid (139mg). LCMS showed $MH^+ = 424$; $T_{RET} = 2.49$min.

**Example 315 2-{[1,6-diethyl-4-(tetrahydro-2*H*-pyran-4-ylamino)-1*H*-pyrazolo[3,4-*b*]pyridin-5-yl]methyl}-6-fluoro-2,3-dihydro-1*H*-isoindol-1-one**

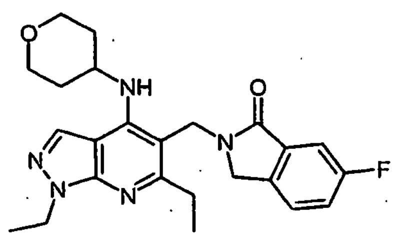

Example 315 was prepared in an analogous manner to Example 314 from Intermediate 16 (88mg) and methyl-2-(bromomethyl)-5-fluorobenzoate (71 mg). LCMS showed $MH^+ = 438$; $T_{RET} = 2.57$min.

**Example 316 2-{[1-ethyl-6-methyl-4-(tetrahydro-2*H*-pyran-4-ylamino)-1*H*-pyrazolo[3,4-*b*]pyridin-5-yl]methyl}-6-(trifluoromethyl)-2,3-dihydro-1*H*-isoindol-1-one**

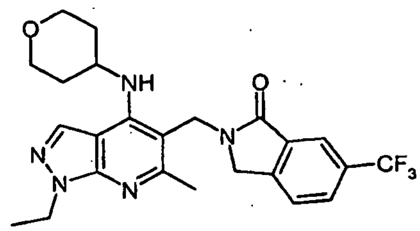

Example 316 was prepared in an analogous manner to Example 314 from Intermediate 15 (87mg) and methyl 2-(bromomethyl)-5-(trifluoromethyl)benzoate (95mg). LCMS showed $MH^+ = 474$; $T_{RET} = 2.72$min.

**Example 317 2-{[1,6-diethyl-4-(tetrahydro-2*H*-pyran-4-ylamino)-1*H*-pyrazolo[3,4-*b*]pyridin-5-yl]methyl}-6-(trifluoromethyl)-2,3-dihydro-1*H*-isoindol-1-one**

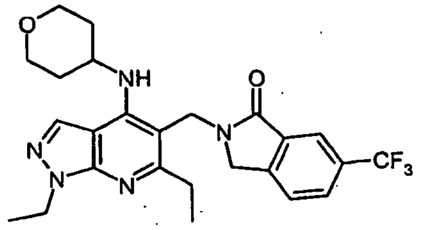

Example 317 was prepared in an analogous manner to Example 314 from Intermediates 16 (91 mg) and methyl 2-(bromomethyl)-5-(trifluoromethyl)benzoate (95mg). LCMS. showed $MH^+ = 488$; $T_{RET} = 2.81$ min.

**Example 318 *N*-{[1-ethyl-6-methyl-4-(tetrahydro-2*H*-pyran-4-ylamino)-1*H*-pyrazolo[3,4-b]pyridin-5-yl]methyl}-2-methyl-1,3-oxazole-4-carboxamide**

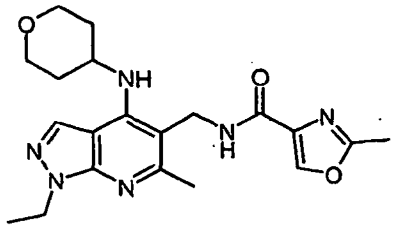

2-Methyl-1,3-oxazole-4-carboxylic acid [e.g. available from *ChemPacific Corporation*] (70mg) was dried under vacuum over phosphorous pentoxide and was then suspended in dry dichloromethane (1.5ml) and treated at room temperature with oxalyl chloride (0.049ml) and DMF (1 drop). The mixture was stirred at room temperature for 30mins and the resulting solution containing 2-methyl-1,3-oxazole-4-carbonyl chloride added dropwise to a solution of Intermediate 15 (145mg) in acetonitrile (3ml). The flask containing the acid chloride was rinsed with acetonitrile (1ml) and this was added to the reaction. DIPEA (0.098ml) was added and the solution was stirred at room temperature under an atmosphere of nitrogen for 1.7h. The solution was blown down to dryness and the residue purified by mass directed autoprep HPLC followed by SPE cartridge (5g, aminopropyl) eluting with methanol. The eluent was blown down to dryness to give Example 318 as a white solid (129mg). LCMS showed $MH^+ = 399$; $T_{RET} = 2.11$ min.

**Example 319 *N*-{[1-ethyl-6-methyl-4-(tetrahydro-2*H*-pyran-4-ylamino)-1*H*-pyrazolo[3,4-*b*]pyridin-5-yl]methyl}-2-(trifluoromethyl)-1,3-thiazole-4-carboxamide**

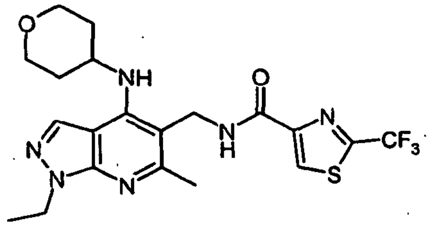

Example 319 was prepared in an analogous manner to Example 318 from Intermediate 15 (145mg) and 2-(trifluoromethyl)-1,3-thiazole-4-carboxylic acid (108mg). The solution of acid chloride and amine was stirred for 1.8h. LCMS showed $MH^+$ = 469; $T_{RET}$ = 2.45min.

**Example 320 *N*-{[1-ethyl-6-methyl-4-(tetrahydro-2H-pyran-4-ylamino)-1H-pyrazolo[3,4-b]pyridin-5-yl]methyl}-2,4-dimethyl-1,3-oxazote-5-carboxamide**

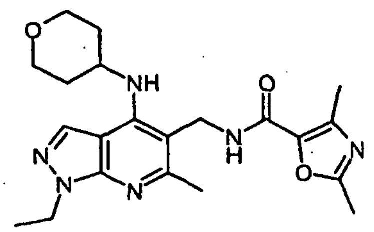

Example 320 was prepared in an analogous manner to Example 318 from Intermediate 15 (142mg) and 2,4-dimethyl-1,3-oxazole-5-carboxylic acid [e.g. available from *Matrix Scientific*] (75mg). The solution of acid chloride and amine was stirred for 1.0h. LCMS showed $MH^+$ = 413; $T_{RET}$ = 2.21 min.

**Example 321 *N*-{[1-ethyl-6-methyl-4-(tetrahydro-2*H*-pyran-4-ylamino)-1*H*-pyrazolo[3,4-*b*]pyridin-5-yl]methyl}-2-methyl-1,3-thiazole-4-carboxamide**

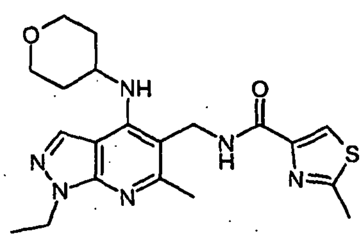

Example 321 was prepared in an analogous manner to Example 318 from Intermediate 15 (95mg) and 2-methyl-1,3-thiazole-4-carboxylic acid [e.g. available from *Acros Organics*] (52mg). The solution of acid chloride and amine was stirred for 3h. LCMS showed $MH^+$ = 415; $T_{RET}$ = 2.28min.

**Example 322 *N*-{[1-ethyl-6-methyl-4-(tetrahydro-2H-pyran-4-ylamino)-1H-pyrazolo[3,4-b]pyridin-5-yl]methyl}-2-methyl-1,3-thiazole-5-carboxamide**

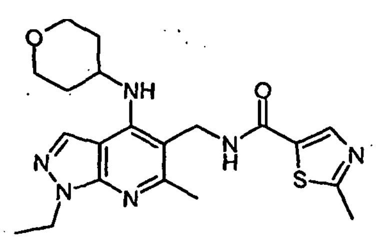

Example 322 was prepared in an analogous manner to Example 318 from Intermediate 15 (145mg) and 2-methyl-1,3-thiazole-5-carboxylic acid [e.g. available from *MicroChemistry Building Blocks*] (79mg). The solution of acid chloride and amine was stirred for 1.8h. LCMS showed $MH^+$ = 415; $T_{RET}$= 2.17min.

**Example 323 *N*-{[1,6-diethyl-4-(tetrahydro-2*H*-pyran-4-ylamino)-1*H* pyrazolo[3,4-*b*]pyridin-5-yl]methyl}-5-(trifluoromethyl)-2-furancarboxamide**

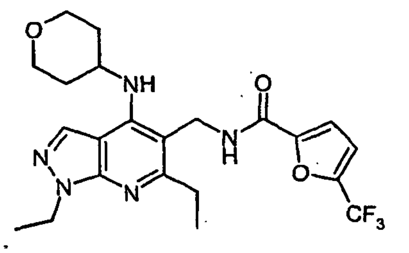

Example 323 was prepared in an analogous manner to Example 318 from Intermediate 16 (143mg) and 5-(trifluoromethyl)-2-furancarboxylic acid [e.g. available from *Acros Organics*] (92mg). The solution of acid chloride and amine was stirred for 18h. LCMS showed $MH^+$ = 466; $T_{RET}$ = 2.64min.

**Example 324 *N*-{[1-ethyl-6-methyl-4-(tetrahydro-2H-pyran-4-ylamino)-1H-pyrazolo[3,4-b]pyridin-5-yl]methyl}-5-(trifluoromethyl)-2-furancarboxamide**

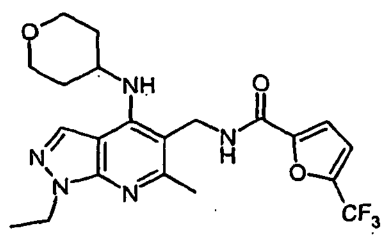

Example 324 was prepared in an analogous manner to Example 318 from Intermediate 15 (137mg) and 5-(trifluoromethyl)-2-furancarboxylic acid (92mg). The solution of acid chloride and amine was stirred for 18h. LCMS showed $MH^+$ = 452; $T_{RET}$ = 2.50min.

**Example 325 6-cyano-*N*-{[1,6-diethyl-4-(tetrahydro-2*H*-pyran-4-ylamino)-1*H*-pyrazolo[3,4-*b*]pyridin-5-yl]methyl}-3-pyridinecarboxamide**

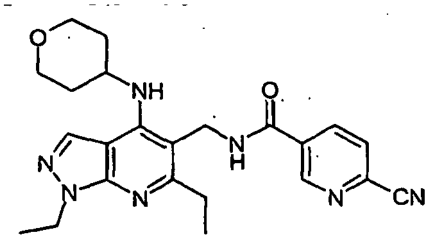

Example 325 was prepared in an analogous manner to Example 318 from Intermediate 16 (91mg) and 6-cyano-3-pyridinecarboxylic acid [e.g. available from *Lancaster Synthesis*] (49mg). The solution of acid chloride and amine was stirred for 65h. LCMS showed $MH^+$ = 434; $T_{RET}$ = 2.46min.

**Example 326 *N*-{[1,6-diethyl-4-(tetrahydro-2*H*-pyran-4-ylamino)-1*H*-pyrazolo[3,4-*b*]pyridin-5-yl]methyl}-6-(trifluoromethyl)-3-pyridinecarboxamide**

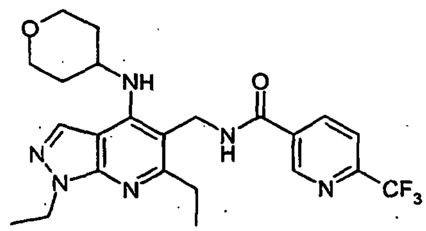

Example 326 was prepared in an analogous manner to Example 318 from Intermediate 16 (91 mg) and 6-trifluromethyl-3-pyridinecarboxylic acid [e.g. available from *Aldrich Chemical Company*] (63mg). The solution of acid chloride and amine was stirred for 65h. LCMS showed $MH^+$ = 477; $T_{RET}$ = 2.66min.

**Example 327 *N*-{[1,6-diethyl-4-(tetrahydro-2H-pyran-4-ylamino)-1H-pyrazolo[3,4-b]pyridin-5-yl]methyl}-2-methyl-6-(trifluoromethyl)-3-pyridinecarboxamide**

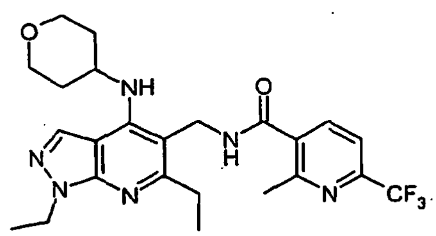

Example 327 was prepared in an analogous manner to Example 318 from Intermediate 16 (91 mg) and 2-methyl-6-trifluromethyl-3-pyridinecarboxylic acid [e.g. available from *Apollo Scientific Ltd.*] (68mg). The solution of acid chloride and amine was stirred for 65h. LCMS showed $MH^+$ = 491; $T_{RET}$ = 2.49min.

**Example 328 6-cyano-*N*-{[1-ethyl-6-methyl-4-(tetrahydro-2*H*-pyran-4-ylamino)-1*H*-pyrazolo[3,4-*b*]pyridin-5-yl]methyl}-3-pyridinecarboxamide**

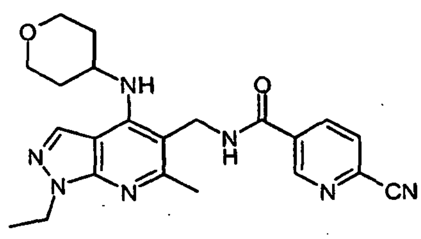

Example 328 was prepared in an analogous manner to Example 318 from Intermediate 15 (87mg) and 6-cyano-3-pyridinecarboxylic acid (49mg). The solution of acid chloride and amine was stirred for 65h. LCMS showed $MH^+$ = 420; $T_{RET}$ = 2.22min.

**Example 329 *N*-{[1-ethyl-6-methyl-4-(tetrahydro-2H-pyran-4-ylamino)-1H-pyrazolo[3,4-b]pyridin-5-yl]methyl}-6-(trifluoromethyl)-3-pyridinecarboxamide**

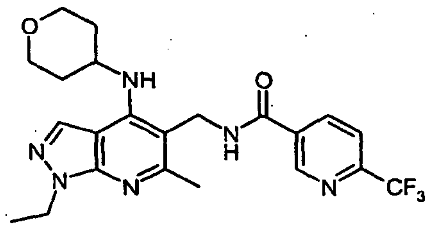

Example 329 was prepared in an analogous manner to Example 318 from Intermediate 15 (87mg) and 6-trifluromethyl-3-pyridinecarboxylic acid (63mg). The solution of acid chloride and amine was stirred for 65h. LCMS showed $MH^+$ = 463; $T_{RET}$ = 2.56mih.

**Example 330 *N*-{[1-ethyl-6-methyl-4-(tetrahydro-2*H*-pyran-4-ylamino)-1*H*-pyrazolo[3,4-*b*]pyridin-5-yl]methyl}-2-methyl-6-(trifluoromethyl)-3-pyridinecarboxamide**

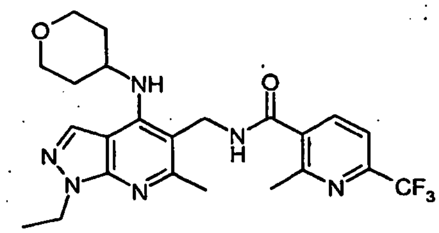

Example 330 was prepared in an analogous manner to Example 318 from Intermediate 15 (87mg) and 2-methyl-6-trifluromethyl-3-pyridinecarboxylic acid (68mg). The solution of acid chloride and amine was stirred for 65h. LCMS showed $MH^+$ = 477; $T_{RET}$ = 2.55min.

**Example 331 *N*-{[1,6-diethyl-4-(tetrahydro-2H-pyran-4-ylamino)-1H-pyrazolo[3,4-b]pyridin-5-yl]methyl}-2-methyl-5-pyrimidinecarboxamide**

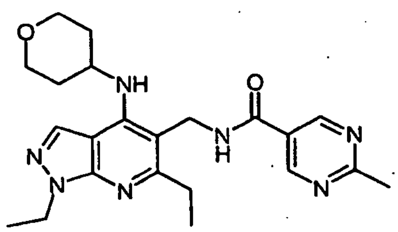

2-Methyl-5-pyrimidinecarboxylic acid [e.g. available from *Chemstep*] (72mg) was dried under vacuum over phosphorous pentoxide for 3 days and was then suspended in dry dichloromethane (1.5ml) and treated at 20˚C with oxalyl chloride (0.046ml) and DMF (1 drop). Rapid effervescence occurred and the mixture was stirred at room temperature for 30mins and then added dropwise to a solution of Intermediate 16 (143mg) in acetonitrile (3ml). DIPEA (0.093ml) was added and the mixture was stirred at room temperature for 1.75h. The mixture was blown down to dryness and the residue purified by mass directed autoprep HPLC. Relevant fractions were collected and evaporated to dryness. The residue was further purified by SPE cartridge (5g, aminopropyl) eluting with methanol. Relevant fractions were collected

and evaporated to dryness. The residue was further purified by preparative TLC on a silica plate (20cm x 20cm x 1 mm) eluting with 5% methanol in ethyl acetate. The major band was collected, extracted with 20% methanol in chloroform and filtered and the filtrate evaporated to give Example 331 (70mg) as a yellow solid. LCMS showed $MH^+$ = 424; $T_{RET}$ = 2.21 min.

**Example 332 *N*-{[1-ethyl-6-methyl-4-(tetrahydro-2H-pyran-4-ylamino)-1H-pyrazolo[3,4-b]pyridin-5-yl]methyl}-2-methyl-5-pyrimidinecarboxamide**

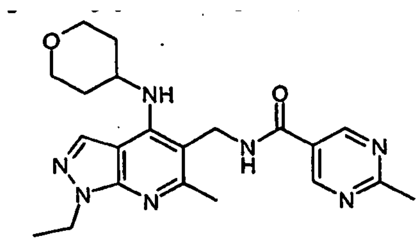

Example 332 was prepared in an analogous manner to Example 318 from Intermediate 15 (137mg) and 2-methyl-5-pyrimidinecarboxylic acid (72mg). The solution of acid chloride and amine was stirred for 2h. The product was further purified using an SPE cartridge (5g, silica) eluting with a gradient of 0-10% methanol in ethyl acetate. LCMS showed $MH^+$ = 410; $T_{RET}$ = 2.07min.

**Example 333 *N*-{[1,6-diethyl-4-(tetrahydro-2H-pyran-4-ylamino)-1H-pyrazolo[3,4-b]pyridin-5-yl]methyl}-2,4-dimethyl-5-pyrimidinecarboxamide**

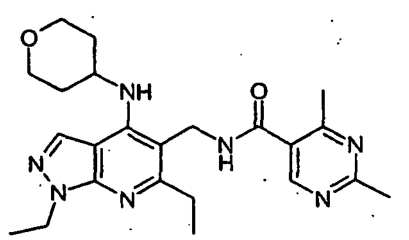

Example 333 was prepared in an analogous manner to Example 318 from Intermediate 16 (143mg) and 2,4-dimethyl-5-pyrimidinecarboxylic acid [e.g. available from *Chemstep*] (79mg). The mixture of acid chloride and amine was stirred for 2h and then a further portion of DIPEA (0.093ml) was added and the mixture stirred for a further 18h. LCMS showed $MH^+$ = 438; $T_{RET}$ = 2.20min.

**Example 334 *N*-{[1,6-diethyl-4-(tetrahydro-2H-pyran-4-ylamino)-1H-pyrazolo[3,4-b]pyridin-5-yl]methyl}-6-methyl-3-pyridinecarboxamide**

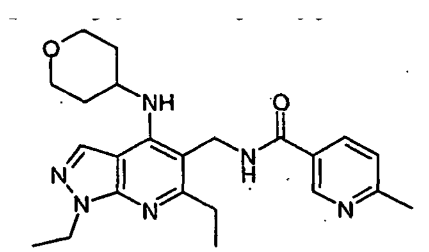

Example 334 was prepared in an analogous manner to Example 318 from Intermediate 16 (143mg) and 6-methyl-3-pyridinecarboxylic acid [*Aldrich Chemical Company*] (72mg). The mixture of acid chloride and amine was stirred for 2h and then allowed to stand for a further 17h. LCMS showed $MH^+$ = 423; $T_{RET}$ = 2.20min.

**Example 335 *N*-{[1,6-diethyl-4-(tetrahydro-2*H*-pyran-4-ylamino)-1*H*-pyrazolo[3,4-*b*]pyridin-5-yl]methyl}-2,4-dimethyl-1,3-oxazole-5-carboxamide formate**

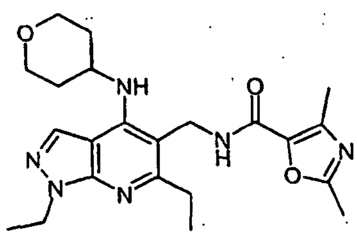

, formate

2,4-Dimethyl-1,3-oxazole-5-carboxylic acid [e.g. available from *Matrix Scientific*] (25mg) was suspended in dry dichloromethane (1.5ml) and treated at room temperature with oxalyl chloride (0.016ml) and DMF (1 drop). The mixture was stirred at room temperature for 25mins and then added dropwise to a solution of Intermediate 16 (50mg) in acetonitrile (1ml) and the mixture stirred at room temperature for 2.5h. The mixture was diluted with dichloromethane (7ml) and washed with brine (2 x 7ml) and the organic extract applied to an SPE cartridge (2g, aminopropyl) which had been pre-washed with methanol. Elution with methanol gave the crude product which was further purified using SPE cartridge (2g, silica) eluting with ethyl acetate followed by mass directed autoprep HPLC to give Example 335 as a clear film (37mg). LCMS showed $MH^+$ = 427; $T_{RET}$ = 2.26min.

**Example 336 2-(1,1-dimethylethyl)-*N*-{[1-ethyl-6-methyl-4-(tetrahydro-2*H*-pyran-4-ylamino)-1*H*-pyrazolo[3,4-*b*]pyridin-5-yl]methyl}-5-pyrimidinecarboxamide**

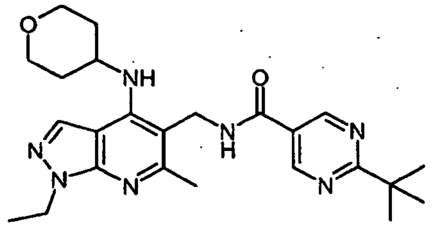

2-(1,1-dimethylethyl)-5-pyrimidinecarboxylic acid [e.g. available from *Interchim*] (95mg) was dried under vacuum over phosphorous pentoxide and was then suspended in dry dichloromethane (1.5ml) and treated at room temperature with oxalyl chloride (0.047ml) and DMF (1 drop). The mixture was stirred at room temperature for 30mins and then added dropwise to a solution of Intermediate 15 (139mg) in acetonitrile (3ml). DIPEA (0.094ml) was added and the solution was stirred at room temperature under an atmosphere of nitrogen for 2h. The solution was blown down to dryness and the residue purified by SPE cartridge (5g, SCX) which had been pre-washed with methanol. The cartridge was eluted with methanol and 10% .880 ammonia in methanol and the ammoniacal eluents evaporated to dryness and azeotroped with methanol to give Example 336 as a white solid (133mg). LCMS showed $MH^+$ = 452; $T_{RET}$ = 2.54min.

**Example 337 *N*-{[1-ethyl-6-methyl-4-(tetrahydro-2*H*-pyran-4-ylamino)-1*H*-pyrazolo[3,4-*b*]pyridin-5-yl]methyl}-6-methyl-3-pyridinecarboxamide**

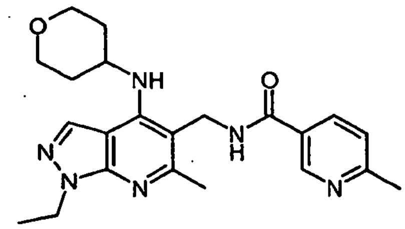

6-Methyl-3-pyridinecarboxylic acid [e.g. available from *Aldrich Chemical Company*] (72mg) was suspended in dry dichloromethane (1.5ml) and treated at room temperature with oxalyl chloride (0.05ml) and DMF (1 drop). The mixture was stirred at room temperature for 30mins and then added dropwise to a solution of Intermediate 15 (137mg) in acetonitrile (3ml). DIPEA (0.101ml) was added and the mixture was stirred at room temperature for 2h and allowed to stand for 17h. The solution was blown down to dryness and the residue treated with 50% DMSO in methanol. Undissolved material was collected by filtration and then dissolved in 50% dichloromethane in methanol (20ml) and added directly to an SPE cartridge (5g, SCX) which had been pre-washed with methanol. The cartridge was eluted with methanol and 10% .880 ammonia in methanol and the ammoniacal eluents evaporated to dryness and azeotroped with methanol to give Example 337 as an off-white solid (151 mg). LCMS showed $MH^+$ = 409; $T_{RET}$ = 2.15min.

**Example 338 *N*-{[1-ethyl-6-methyl-4-(tetrahydro-2*H*-pyran-4-ylamino)-1*H*-pyrazolo[3,4-*b*]pyridin-5-yl]methyl}-2,4-dimethyl-5-pyrimidinecarboxamide**

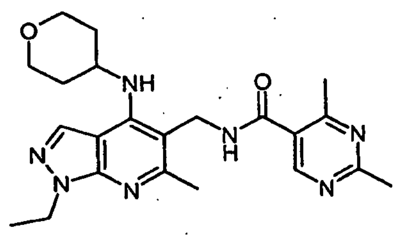

2,4-Dimethyl-5-pyrimidinecarboxylic acid [e.g. available from *Chemstep*] (79mg) was dried under vacuum over phosphorous pentoxide and was then suspended in dry dichloromethane (1.5ml) and treated at room temperature with oxalyl chloride (0.046ml) and DMF (1 drop). The mixture was stirred at room temperature for 30mins and then added dropwise to a solution of Intermediate 15 (137mg) in acetonitrile (3ml). DIPEA (0.093ml) was added and the mixture was stirred at room temperature for 2h after which time a second portion of DIPEA (0.093ml) was added and the mixture was stirred at room temperature for a further 18h. A further portion of 2,4-dimethyl-5-pyrimidine carboxylic acid (20mg) was converted to the acid chloride as above and added to the reaction and stirring was continued for a further 2h. The solution was blown down to dryness and the residue purified by mass directed autoprep HPLC followed by SPE cartridge (5g, aminopropyl) eluting with methanol. The eluent was blown down to dryness to give Example 338 as a white solid (75mg). LCMS showed $MH^+$ = 424; $T_{RET}$ = 2.13min.

**Example 339 *N*-{[1,6-diethyl-4-(tetrahydro-2*H*-pyran-4-ylamino)-1*H*-pyrazolo[3,4-*b*]pyridin-5-yl]methyl}-2-(trifluoromethyl)-1,3 thiazole-4-carboxamide**

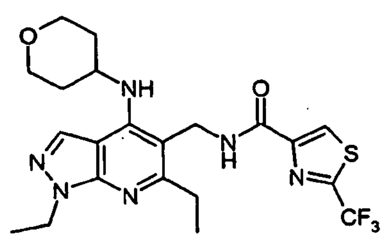

Ethyl 2-(trifluoromethyl)-1,3-thiazole-4-carboxylate (80mg) in ethanol (1ml) was treated with 2N sodium hydroxide (0.706ml) and the solution stirred at room temperature for 18h. 2M Hydrochloric acid (0.54ml) was added and the mixture blown down to dryness. The residue was dried under vacuum over phosphorous pentoxide and was then suspended in dry dichloromethane (1 ml) and treated at room temperature with oxalyl chloride (0.032ml) and DMF (1 drop). The mixture was stirred at room temperature for 30mins and then added dropwise to a solution of Intermediate 16 (98mg) in acetonitrile (2ml) and the mixture was stirred at room temperature for 22h. The mixture was diluted with dichloromethane (15ml), washed with brine (2 x 15ml) and blown down to dryness. The residue was purified by mass directed autoprep HPLC. followed by SPE cartridge (5g, aminopropyl) eluting with methanol. The eluent was blown down to dryness to give Example 339 as a brown gum (96mg). LCMS showed $MH^+$ = 483; $T_{RET}$ = 2.57min.

**Example 340 *N*-{[1-ethyl-6-methyl-4-(tetrahydro-2H-pyran-4-ylamino)-1H-pyrazolo[3,4-b]pyridin-5-yl]methyl}-2-(trifluoromethyl)-5-pyrimidine carboxamide**

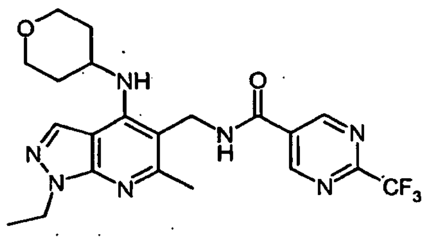

Ethyl 2-(trifluoromethyl)-5-pyrimidinecarboxylate [e.g. available from J. Med. Chem., (2000), 43 (21), 3995] (49mg) in ethanol (0.63ml) was treated with 2N sodium hydroxide (0.443ml) and the solution stirred at room temperature for 24h. 2M Hydrochloric acid (0.31ml) was added and the mixture blown down to dryness. The residue was suspended in dry dichloromethane (0.5ml) and treated at room temperature with oxalyl chloride (0.019ml) and DMF (1 drop). The mixture was stirred at room temperature for 30mins and then added dropwise to a solution of Intermediate 15 (53mg) in acetonitrile (1ml). DIPEA (0.039ml) was added and the mixture was stirred at room temperature for 18h. The mixture was blown down to dryness and the residue was purified by mass directed autoprep HPLC followed by SPE cartridge (1g, aminopropyl) eluting with methanol. The eluent was blown down to dryness to give Example 340 as a beige solid (9mg). LCMS showed $MH^+$ = 464; $T_{RET}$ = 2.42min.

**Example 341 *N*-{[1,6-diethyl-4-(tetrahydro-2H-pyran-4-ylamino)-1H-pyrazolo[3,4-b]pyridin-5-yl]methyl}-1-(methylsulfonyl)-4-piperidinecarboxamide**

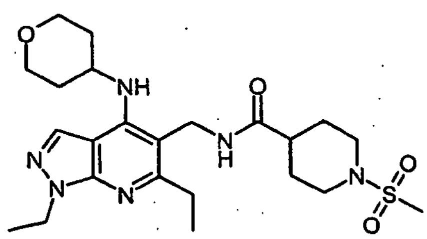

1-(Methylsulfonyl)-4-piperidinecarboxylic acid [e.g. available from *Matrix Scientific*] (150mg) was dissolved in dry dichloromethane (3ml) and treated at room temperature with oxalyl chloride (0.062ml) and DMF (3 drops). The mixture was stirred at room temperature for 30mins and then half was added dropwise to a solution of Intermediate 16 (100mg) and DIPEA (0.063ml) in acetonitrile (3ml). The mixture was stirred at room temperature for 18h and was diluted with dichloromethane (15ml). The solution was washed with brine (8ml) and the organic extract added directly to an SPE cartridge (10g, aminomethyl) which had been pre-washed with methanol. The cartridge was eluted with methanol to give crude product which was purified by SPE cartridge (5g, silica) eluting sequentially with 50% cyclohexane in ethyl acetate, ethyl acetate, 5% methanol in dichloromethane and 10% methanol in dichloromethane to give Example 341 as a white solid (108mg). LCMS showed $MH^+$ = 493; $T_{RET}$ = 2.18min.

**Example 342 *N*-{[1-ethyl-6-methyl-4-(tetrahydro-2H-pyran-4-ylamino)-1H-pyrazolo[3,4-b]pyridin-5-yl]methyl}-1-(methylsulfonyl)-4-piperidinecarboxamide**

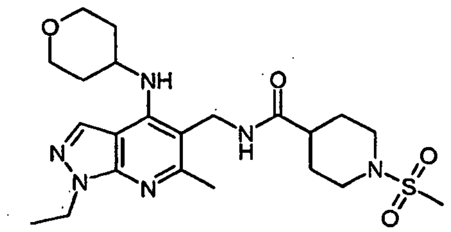

1-(Methylsulfonyl)-4-piperidinecarboxylic acid (150mg) was dissolved in dry dichloromethane (3ml) and treated at room temperature with oxalyl chloride (0.062ml) and DMF (3 drops). The mixture was stirred at room temperature for 30mins and then half was added dropwise to a solution of Intermediate 15 (95mg) and DIPEA (0.063ml) in acetonitrile (3ml). The mixture was stirred at room temperature for 18h and was diluted with dichloromethane (15ml). The solution was washed with brine (8ml) and the organic extract added directly to an SPE cartridge (10g, aminomethyl) which had been pre-washed with methanol. The cartridge was eluted with methanol to give crude product which was purified by SPE cartridge (5g, silica) eluting sequentially with 50% cyclohexane in ethyl acetate, ethyl acetate, 5% methanol in dichloromethane and 10% methanol in dichloromethane followed by mass directed autoprep HPLC to give Example 342 as a white solid (62mg). LCMS showed $MH^+$ = 479; $T_{RET}$ = 2.10min.

**Example 343 1-{[1,6-diethyl-4-(tetrahydro-2H-pyran-4-ylamino)-1H-pyrazolo[3,4-b]pyridin-5-yl]methyl}-3-methyl-2-imidazolidinone**

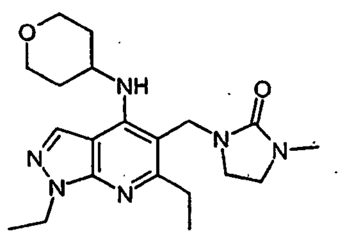

A solution of Intermediate 5 (150mg) in thionyl chloride (2ml) was heated at 75°C for 2h. Solvent was evaporated in vacuo and the residue azeotroped with toluene to give Intermediate 8. Meanwhile, a solution of 1-methyl-2-imidazolidinone [e.g. available from *Acros Organics*] (50mg) in DMF (1 ml) was added dropwise to a mixture of sodium hydride (24mg) in DMF (1 ml) at 0°C. The mixture was stirred at 0°C for 30mins and then treated dropwise with a solution of the above Intermediate 8 in DMF (1 ml). The mixture was stirred at room temperature for 3h and then quenched with methanol. Solvent was evaporated in vacuo and the residue purified by SPE cartridge (20g, silica) eluting with a gradient of 5-75% [ethyl acetate (50), ethanol (50), ammonia (1)] in cyclohexane followed by mass directed autoprep HPLC and finally SPE cartridge (1g, aminopropyl) eluting with methanol to give Example 343 as a white solid (42mg). LCMS showed $MH^+$ = 387; $T_{RET}$ = 2.11 min.

**Example 344 5-{[1,6-diethyl-4-(tetrahydro-2*H*-pyran-4-ylamino)-1*H*-pyrazolo[3,4-*b*]pyridin-5-yl]methyl}-4,5-dihydro-6*H*-pyrrolo[3,4-*d*][1,2,3]thiadiazol-6-one**

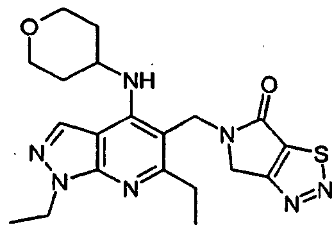

A solution of ethyl 4-(bromomethyl)-1,2,3-thiadiazole-5-carboxylate [e.g. see WO 96/29871] (594mg) in, acetonitrile (3ml) was added dropwise to a stirred mixture of Intermediate 16 (250mg) and DIPEA (0.159ml) in acetonitrile (3ml) under an atmosphere of nitrogen. The mixture was stirred at room temperature for 18h and then evaporated to dryness in vacuo. The residue was purified by SPE cartridge (50g, silica) eluting with a gradient of 0-100% ethyl acetate in cyclohexane and then 0-50% methanol in ethyl acetate to give ethyl 4-[({[1,6-diethyl-4-(tetrahydro-2*H*-pyran-4-ylamino)-1*H*-pyrazolo[3,4-*b*]pyridin-5-yl]methyl}amino)methyl]-1,2,3-thiadiazole-5-carboxylate as a yellow oil (194mg).

Aqueous sodium hydroxide (2N, 0.195ml) was added to a mixture of ethyl 4-[({[1,6-diethyl-4-(tetrahydro-2*H*-pyran-4-ylamino)-1*H*-pyrazolo[3,4-*b*]pyridin-5-yl]methyl} amino)methyl]-1,2,3-thiadiazole-5-carboxylate (187mg) in ethanol (1.5ml) and water (0.195ml) and the mixture stirred at room temperature for 4h. Solvents were evaporated in vacuo and the residue dissolved in hydrochloric acid (2N, 1ml) and then evaporated in vacuo. The residual solid was dried under vacuum over phosphorous pentoxide to give a crude sample of 4-[({[1,6-diethyl-4-(tetrahydro-2*H*-pyran-4-ylamino)-1*H*-pyrazolo[3,4-*b*]pyridin-5-yl]methyl}amino)methyl]-1,2,3-thiadiazole-5-carboxylic acid, hydrochloride salt (210mg). This was dissolved in DMF (7ml) under an atmosphere of nitrogen and N-hydroxysuccinimide (49mg) was added. The solution was cooled to 0-5°C and 1-[3-(dimethylamino)propyl]-3-ethylcarbodiimide, hydrochloride (90mg) was added portionwise. The mixture was then stirred at room temperature for 18h and then triethylamine (0.163ml) was added portionwise over 2h and the mixture was stirred at room temperature for a further 18h. Solvents were evaporated and the residue dissolved in dichloromethane (30ml) and washed with water (5ml). The organic extract was evaporated in vacuo and the residue purified by SPE cartridge (10g, silica) eluting sequentially with 20%, 33%, 50% and 100% ethyl acetate in cyclohexane followed by mass directed autoprep HPLC and finally SPE cartridge (2g, aminopropyl) eluting with methanol to give Example 344 as a pale yellow solid (91 mg). LCMS showed $MH^+ = 428$; $T_{RET} = 2.20$min.

**Example 345 5-{[1-ethyl-6-methyl-4-(tetrahydro-2*H*-pyran-4-ylamino)-1*H*-pyrazolo[3,4-*b*]pyridin-5-yl]methyl}-4,5-dihydro-6*H*-pyrrolo[3,4-*d*][1,2,3]thiadiazol-6-one**

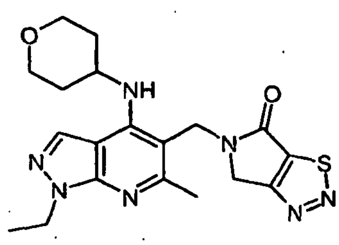

Example 345 was prepared in an analogous manner to Example 344 from ethyl 4-(bromomethyl)-1,2,3-thiadiazole-5-carboxylate and Intermediate 15. LCMS showed $MH^+ = 414$; $T_{RET} = 2.06$min.

**Example 346 N-{[1,6-diethyl-4-(tetrahydro-2*H*-pyran-4-ylamino)-1*H*-pyrazolo[3,4-*b*]pyridin-5-yl]methyl}-2-(1-methylethyl)-5-pyrimidinecarboxamide**

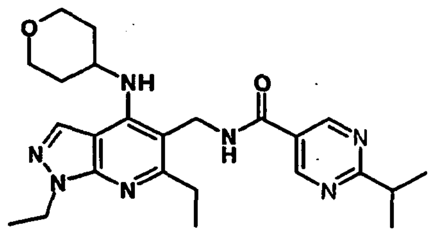

Methyl 2-(1-methylethyl)-5-pyrimidinecarboxylate (72mg) [e.g. see *Synthesis,* 2002, 6, 720] in ethanol (1.2ml) was treated with 2N sodium hydroxide (0.792ml). The solution was stirred at room temperature for 2.5h, then treated with 2N HCl (0.7ml) and blown to dryness overnight to leave an off-white solid which was further dried under vacuum over phosphorous pentoxide. Half of this solid was then suspended in dry dichloromethane (0.5ml) and treated at room temperature with oxalyl chloride (0.018ml) and diethylformamide (1 drop). The mixture was stirred at room temperature for 30mins and the resulting solution containing 2-(1-methylethyl)-5-pyrimidine-carbonyl chloride was added dropwise to a solution of Intermediate 16 (55mg) in acetonitrile (1ml). DIPEA (0.035ml) was added and the solution was stirred at room temperature for 2.5h. The solution was blown down to dryness and the residue purified by mass directed autoprep HPLC followed by SPE cartridge (2g, aminopropyl) eluting with methanol. The eluent was blown down to dryness to give Example 346 as a clear colourless gum (33mg). LCMS showed $MH^+$ = 452; $T_{RET}$ = 2.36min.

**Example 347 N-{[1-ethyl-6-methyl-4-(tetrahydro-2*H*-pyran-4-ylamino)-1*H*-Pyrazolo[3,4-*b*]pyridin-5-yl]methyl}-2-(1-methylethyl)-5-pyrimidinecarboxamide**

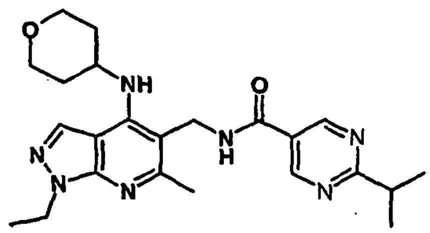

Examples 347 was prepared in an analogous manner to Example 346 from Intermediate 15 and methyl 2-(1-methylethyl)-5-pyrimidinecarboxylate [e.g. see *Synthesis,* 2002, 6, 720]. LCMS showed $MH^+$ = 438; $T_{RET}$ = 2.28min.

**Example 348 *N*-{[1-Ethyl-4-(tetrahydro-2*H*-pyran-4-ylamino)-6-(trifluoromethyl)-1*H*-pyrazolo[3,4-*b*]pyridin-5-yl]methyl}-5-methyl-2-pyrazinecarboxamide**

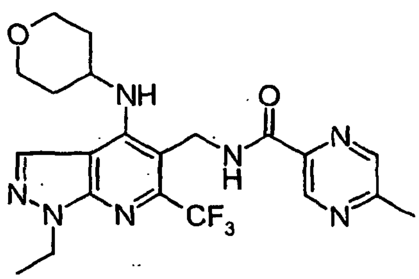

5-Methyl-2-pyrazinecarboxylic acid (152mg, 1.1mmol, commercially available from e.g. Avocado) was suspended in dichloromethane (3ml) and treated at 20˚C under nitrogen with oxalyl chloride (0.288ml, 3.3mmol) and diethylformamide (1drop). A vigorous reaction occurred. After 30min the reaction mixture was evaporated to dryness to

give a dark yellow oil which was presumed to be 5-methyl-2-pyrazinecarbonyl chloride.

A solution of 5-methyl-2-pyrazinecarbonyl chloride (1.1 mmol) in dichloromethane (2ml) was added under nitrogen at 20˚C to a stirred mixture of Intermediate 36 (0.343g, 1.0mmol) and diisopropylethylamine (0.77ml, 4.4mmol) in chloroform (7ml). The resulting solution was stirred at 20˚C under nitrogen for 1.5h, The reaction mixture was evaporated to dryness and the residual oil was dissolved methanol (5ml) and applied to an aminopropyl SPE cartridge (20g). The cartridge was eluted with methanol (3 column volumes). The product-containing fraction was evaporated to give the crude product as a brown foam (0.403g). This foam was dissolved in dichloromethane (10ml) and applied to a silica SPE cartridge (20g). The cartridge was eluted using initially a gradient of 0 to 100% ethyl acetate in cyclohexane followed by a gradient of 0 to 20% methanol in ethyl acetate/dichloromethane. The product-containing fraction was evaporated to give Example 348 as a fluorescent yellow solid (0.215g). LCMS showed $MH^+$ = 464; $T_{RET}$ = 3.23min.

**Examples 349 *N*-{[1-Ethyl-4-(tetrahydro-2*H*-pyran-4-ylamino)-6-(trifluoromethyl)-1*H*-pyrazolo[3,4-*b*]pyridin-5-yl]methyl}-2-methyl-1,3-thiazole-5-carboxamide**

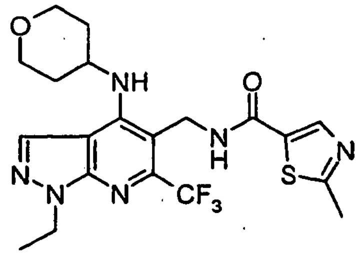

2-Methyl-1,3-thiazole-5-carboxylic acid (0.133g, 0.93mmol) (e.g. which can optionally be prepared as in Example 311) was suspended in dichloromethane (3ml) and treated at 20˚C under nitrogen with oxalyl chloride (0.24ml, 2.75mmol) and diethylformamide (1 drop). A vigorous reaction occurred. After 15min, the reaction mixture was evaporated to dryness to give a dark yellow oil which was presumed to be 2-methyl-1,3-thiazole-5-carbonyl chloride.

A solution of the 2-methyl-1,3-thiazole-5-carbonyl chloride (0.93mmol) in dichloromethane (2ml) was added, under nitrogen, at 20˚C to a stirred mixture of Intermediate 36 (0.29g, 0.84mmol) and diisopropylethylamine (0.59ml, 3.39mmol) in chloroform (7ml). The resulting solution was stirred at 20˚C under nitrogen for 20min. The reaction mixture was diluted with chloroform (25ml) and washed with 5%-sodium hydrogen carbonate solution (5ml). The phases were separated by passage through a hydrophobic frit, and the organic phase was evaporated to a brown foam (0.478g). This foam (0.478g) was dissolved in dichloromethane (5ml) and applied to a silica SPE cartridge (20g). The cartridge was eluted using initially a gradient of 0 to 100% ethyl acetate in cyclohexane followed by a gradient of 0 to 20% methanol in ethyl acetate/dichloromethane. The product-containing fractions were combined and evaporated to give the impure product as a yellow foam (0.2g). This foam was dissolved in dichloromethane (5ml) and applied to a silica SPE cartridge (20g). The cartridge was eluted using a gradient of 0 to 100% ethyl acetate in cyclohexane. The pure product-containing fractions were combined and evaporated to give Example 349 as a cream solid (0.078g). LCMS showed $MH^+$ = 469; $T_{RET}$ = 3.07min.

The impure product-containing fractions were combined and evaporated to give a yellow solid (0.107g) which was dissolved in dimethylsulphoxide-methanol-chloroform (2.5ml) and purified by mass directed autoprep HPLC to give a further quantity of Example 349 as a white solid (0.078g). LCMS showed $MH^+$ = 469; $T_{RET}$ = 3.08min.

**Example 350 4-amino-*N*-{[1,6-diethyl-4-(tetrahydro-2*H*-pyran-4-ylamino)-1*H*-pyrazolo[3,4-*b*]pyridin-5-yl]methyl}benzamide**

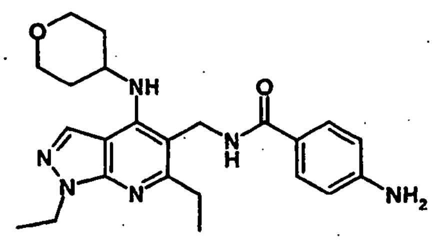

Intermediate 37 (1.5 g) was combined with 10% Palladium on Carbon (500 mg) and stirred in ethanol (200mL) under an atmosphere of hydrogen gas (1 atmosphere) for 2 hours. The mixture was filtered through celite and the catalyst washed with 1:1 methanol/chloroform (100ml) to give Example 350 (1.21g). LCMS showed $MH^+ = 423$; $T_{RET} = 2.14$min.

**Example 351 3-amino-*N*-[1,6-diethyl-4-(tetrahydro-2*H*-pyran-4-ylamino)-1*H*-pyrazolo[3,4-*b*]pyridin-5-yl]methyl}benzamide**

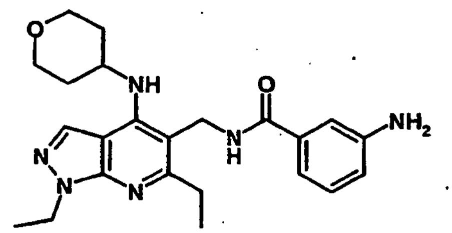

Example 351 was prepared in an analogous manner to Example 350 from Intermediate 38. LCMS showed $MH^+ = 423$; $T_{RET} = 2.11$ min.

**PHARMACEUTICAL COMPOSITION EXAMPLES**

The following are examples of pharmaceutical compositions (formulations) suitable for external topical administration (e.g. topical administration to skin).

**Composition Example 1 (Ointment D)**

The following pharmaceutical composition (formulation) is an ointment suitable for external topical administration (e.g. topical administration to skin), and comprises 2% w/w of *N*-{[1,6-diethyl-4-(tetrahydro-2*H*-pyran-4-ylamino)-1*H*-pyrazolo[3,4-*b*]pyridin-5-yl]methyl}-3-methyl-5-isoxazolecarboxamide (i.e. the "free base" form) (e.g. this can be as prepared in Example 237):

| Product: Ointment D<br>Batch Size: 20 g | | | |
|---|---|---|---|
| *Ingredient* | *Concentration in composition (% w/w)* | *Theoretical Amount (g)* | *Actual Amount (g)* |
| white petrolatum | 73 | 14.6 | 14.69 |
| decamethyl-cyclopentasiloxane (ST-Cyclomethicone 5-NF™) | 25 | 5 | 5.01 |
| Example 237 (in "free base" form) | 2 | 0.4 | 0.4006 |
| TOTAL | | 20 | 20.1006 |

*Preparation Procedure:*

Approximately the following procedure is used to prepare Ointment D:

The white petrolatum (a solid at room temperature, and e.g. which can optionally be high melting point white petrolatum such as Penreco Ultima White™ grade white petrolatum) and the decamethyl-cyclopentasiloxane (a liquid at room temperature) are heated together, in a beaker in a hot water bath, to a temperature of approximately 60-65 ˚C, to melt the white petrolatum.

The drug substance (*N*-{[1,6-diethyl-4-(tetrahydro-2*H*-pyran-4-ylamino)-1*H*-pyrazolo[3,4-b]pyridin-5-yl]methyl}-3-methyl-5-isoxazolecarboxamide, which, for example, can be as prepared in Example 237, Synthesis C) is added slowly into the melted oil phase and is stirred with a spatula and dispersed completely. The mixture is homogenised under high shear conditions (using setting #5, a high setting, on an Ultra-turrax T25 homogenizer), using the small homogenizer shaft, for 10 minutes.

The formulation is allowed to cool to room temperature (e.g. about 17 to about 22 ˚C) while stirring. The ointment formulation is filled into a 30mL transparent container.

**Reference Composition Example 2 (Placebo Ointment AP)**

The following pharmaceutical composition (formulation) is an ointment suitable for external topical administration (e.g. topical administration to skin), but it does not include any compound of the invention. Thus it can be used as a comparator placebo ointment, when testing (e.g. in "In Vivo Assay A" herein) a corresponding ointment containing the compound of the invention such as Composition Example 1 (Ointment D).

| Product: Placebo Ointment AP<br>Batch Size: 100 g | | | |
|---|---|---|---|
| *Ingredient* | *Concentration in composition (% w/w)* | *Theoretical Amount (g)* | *Actual Amount (g)* |
| white petrolatum | 75 | 75 | 75.61 |
| decamethyl-cyclopentasiloxane (ST-Cyclomethicone 5-NF ™) | 25 | 25 | 25.00 |
| TOTAL | | 100 | 100.61 |

*Preparation Procedure:*

Approximately the following procedure is used to prepare Placebo Ointment AP:

The white petrolatum (which can optionally be high melting point white petrolatum such as Penreco Ultima White™ grade white petrolatum) and the decamethyl-cyclopentasiloxane are heated together, in a small beaker in a hot water bath, to a temperature of approximately 60-65 ˚C.

The mixture is homogenised under high shear conditions (using setting #5, a high setting, on an Ultra-turrax T25 homogenizer), for approximately 10 minutes.

The formulation is allowed to cool and is stirred until room temperature (e.g. about 17 to about 22˚C) is reached. The ointment formulation is packed into a 125mL transparent plastic container.

**Composition Example 3 (Ointment D2)**

The following pharmaceutical composition (formulation) is an ointment suitable for external topical administration (e.g. topical administration to skin), and comprises 2% w/w of *N*-{[1,6-diethyl-4-(tetrahydro-2*H*-pyran-4-ylamino)-1*H*-pyrazolo[3,4-*b*]pyridin-5-yl]methyl}-3-methyl-5-isoxazolecarboxamide (i.e. the "free base" form) (e.g. this can be as prepared in Example 237):

| Product: Ointment D2<br>Batch Size: 20 g | | | |
|---|---|---|---|
| *Ingredient* | *Concentration in composition (% w/w)* | *Theoretical Amount (g)* | *Actual Amount (g)* |
| white petrolatum | 73 | 14.6 | 14.66 |
| decamethyl-cyclopentasiloxane (ST-Cyclomethicone 5-NF ™) | 25 | 5 | 5.09. |
| Example 237 (in "free base" form) | 2 | 0.4 | 0.4012 |
| TOTAL | | 20 | 20.1512 |

*Preparation Procedure:*

Substantially the same preparation procedure as in Composition Example 1 (Ointment D) is used to prepare Ointment D2.

**Reference Composition Example 4 (Placebo Ointment AP2)**

The following pharmaceutical composition (formulation) is an ointment suitable for external topical administration (e.g. topical administration to skin), but it does not include, any compound of the invention. Thus it can be used as a comparator placebo ointment, when testing (e.g. in "In Vivo Assay A" herein) a corresponding ointment containing the compound of the invention such as Composition Example 3 (Ointment D2).

| Product: Placebo Ointment AP2<br>Batch Size: 100 g | | | |
|---|---|---|---|
| *Ingredient* | *Concentration in composition (% w/w)* | *Theoretical Amount (g)* | *Actual Amount (g)* |
| white petrolatum | 75 | 75 | 75.52 |
| decamethyl-cyclopentasiloxane (ST-Cyclomethicone 5-NF ™) | 25 | 25 | 25.03 |
| TOTAL | | 100 | 100.55 |

*Preparation Procedure:*

Substantially the same preparation procedure as in Reference Composition Example 2 (Placebo Ointment AP) is used to prepare Placebo Ointment AP2.

**Composition Example 5 (Water-in-oil cream Cr-D)**

The following pharmaceutical composition (formulation) is believed to be a water-in-oil cream emulsion, for external topical administration (e.g. topical administration to skin), and comprises propylene glycol and 2% w/w of *N*-{[1,6-diethyl-4-(tetrahydro-2*H*-pyran-4-ylamino)-1*H*-.pyrazolo[3,4-*b*]pyridin-5-yl]methyl}-3-methyl-5-isoxazolecarboxamide (i.e. the "free base" form) (e.g. this can be as prepared in Example 237):

| Product: Water-in-oil cream Cr-D<br>Batch Size: 20 g | | | |
|---|---|---|---|
| *Ingredient* | *Concentration in composition (% w/w)* | *Theoretical Amount (g)* | *Actual Amount (g)* |
| white petrolatum | 40 | 8 | 8.05 |
| mineral oil | 10 | 2 | 2.00 |
| steareth-2 = polyoxyl 2 stearyl ether (Volpo s-2 ™) (surfactant) | 8 | 1.6 | 1.62 |
| Aqueous phase | | | |
| propylene glycol | 20 | 4 | 4.01 |
| purified water | 20 | 4 | 4.01 |
| Example 237 (in "free base" form) | 2 | 0.4 | 0.4001 |
| TOTAL | | 20 | 20:0901 |

*Preparation Procedure:*

Approximately the following procedure is used to prepare Water-in-oil cream Cr-D:

The white petrolatum (which can optionally be high melting point white petrolatum such as Penreco Ultima White™ grade white petrolatum), the mineral oil and the steareth-2 (Volpo s-2 ™) are heated together, via a hot water bath, to a temperature of approximately 60-65 ˚C to form an oily phase.

In a separate container, the drug substance (*N*-{[1,6-diethyl-4-(tetrahydro-2*H*-pyran-4-ylamino)-1*H*-pyrazolo [3,4-*b*]pyridin-5-yl]methyl}-3-methyl-5-isoxazolecarboxamide, which, for example, can be as prepared in Example 237, Synthesis C) is dispersed in water and sonicated for approximately 10 minutes. Propylene glycol is added to this aqueous phase and the mixture is sonicated for another 10 minutes. The drug substance is at least partly in suspension.

The aqueous phase is heated to approximately the same temperature as the oily phase (e.g. is heated to approximately 60-65 ˚C), and then the aqueous phase is added slowly to the oily phase while homogenizing the mixture under high shear conditions (using setting #5, a high setting, e.g. on an Ultra-turrax T25 homogenizer), for approximately 10 minutes.

After homogenization, the formulation is allowed to cool to room temperature (e.g. about 17 to about 22 ˚C) with constant mixing with a spatula. The cream formulation is packed into a 20mL scintillation vial.

**Reference Composition Example 6 (Placebo Water-in-oil cream Cr-AP)**

The following pharmaceutical composition (formulation) is believed to be a water-in-oil cream emulsion, for external topical administration, but it does not include any compound of the invention. Thus it can be used as a comparator placebo cream, when testing (e.g. in "In Vivo Assay A" herein) a corresponding cream containing the compound of the invention such as Composition Example 5 (Water-in-oil cream Cr-D).

| Product: Placebo Water-in-oil cream Cr-AP<br>Batch Size: 100g | | | |
|---|---|---|---|
| *Ingredient* | *Concentration in composition (% w/w)* | *Theoretical Amount (g)* | *Actual Amount (g)* |
| white petrolatum | 42 | 42 | 42.98 |
| mineral oil | 10 | 10 | 10.06 |
| steareth-2 = polyoxyl 2 stearyl ether (Volpo s-2 ™) (surfactant) | 8 | 8 | 8.04 |
| Aqueous phase | | | |

(continued)

| Product: Placebo Water-in-oil cream Cr-AP<br>Batch Size: 100g | | | |
|---|---|---|---|
| *Ingredient* | *Concentration in composition (% w/w)* | *Theoretical Amount (g)* | *Actual Amount (g)* |
| propylene glycol | 20 | 20 | 20.06 |
| purified water | 20 | 20 | 20.03 |
| TOTAL | | 100 | 101.17 |

*Preparation Procedure:*

Approximately the following procedure is used to prepare Placebo Water-in-oil cream Cr-AP:

The white petrolatum (which can optionally be high melting point white petrolatum such as Penreco Ultima White™ grade white petrolatum), the mineral oil and the steareth-2 (Volpo s-2 ™) are heated together, via a hot water bath, to a temperature of approximately 60-65 ˚C to form an oily phase. In another beaker, the aqueous phase (propylene glycol and water) is heated to a temperature of approximately 60-65 ˚C.

The aqueous phase is added slowly to the oily phase while homogenizing the mixture at low speed, and then the speed is increased to high shear conditions (using setting #5, a high setting, e.g. on an Ultra-turrax T25 homogenizer). The mixture is homogenized, while being kept hot via a hot water bath, for approximately 10 minutes.

The formulation is then allowed to cool and stirred until room temperature (e.g. about 17 to about 22 ˚C) is reached. The cream formulation is packed into a 125mL transparent plastic container.

**Composition Example 7 (Water-in-oil cream Cr-D2)**

The following pharmaceutical composition (formulation) is believed to be a water-in-oil cream emulsion, for external topical administration (e.g. topical administration to skin), and comprises propylene glycol and 2% w/w of *N*-{[1,6-diethyl-4-(tetrahydro-2*H*-pyran-4-ylamino)-1*H*-pyrazolo[3,4-*b*]pyridin-5-yl]methyl}-3-methyl-5-isoxazolecarboxamide (i.e. the "free base" form) (e.g. this can be as prepared in Example 237):

| Product: Water-in-oil cream Cr-D2<br>Batch Size: 20 g | | | |
|---|---|---|---|
| *Ingredient* | *Concentration in composition (% w/w)* | *Theoretical Amount (g)* | *Actual Amount (g)* |
| white petrolatum | 40 | 8 | 8.01 |
| mineral oil | 10 | 2 | 2.02 |
| steareth-2 = polyoxyl 2 stearyl ether (Volpo s-2 ™) (surfactant) | 8 | 1.6 | 1.64 |
| <u>Aqueous phase</u> | | | |
| propylene glycol | 20 | 4 | 4.03 |
| purified water | 20 | 4 | 4.07 |
| Example 237 (in "free base" form) | 2 | 0.4 | 0.4004 |
| TOTAL | | 20 | 20.1704 |

*Preparation Procedure:*

Substantially the same preparation procedure as in Composition Example 5 (Water-in-oil cream Cr-D) is used to prepare Water-in-oil cream Cr-D2, except that the cream formulation is packed into a 30mL transparent container.

**Reference Composition Example 8 (Placebo Water-in-oil cream Cr-AP2)**

The following pharmaceutical composition (formulation) is believed to be a water-in-oil cream emulsion, for external topical administration, but it does not include any compound of the invention. Thus it can be used as a comparator placebo cream, when testing (e.g. in "In Vivo Assay A" herein) a corresponding cream containing the compound of the invention such as Composition Example 7 (Water-in-oil cream Cr-D2).

| Product: Placebo Water-in-oil cream Cr-AP2<br>Batch Size: 100g | | | |
|---|---|---|---|
| *Ingredient* | *Concentration in composition (% w/w)* | *Theoretical Amount (g)* | *Actual Amount (g)* |
| white petrolatum | 42 | 42 | 42.71 |
| mineral oil | 10 | 10 | 10.15 |
| steareth-2 = polyoxyl 2 stearyl ether (Volpo s-2™) (surfactant) | 8 | 8 | 8.01 |
| Aqueous phase | | | |
| propylene glycol | 20 | 20 | 20.00 |
| purified water | 20 | 20 | 20.02 |
| TOTAL | | 100 | 101.89 |

*Preparation Procedure:*

Substantially the same preparation procedure as in Reference Composition Example 6 (Placebo Water-in-oil cream Cr-AP) is used to prepare Placebo Water-in-oil cream Cr-AP2.

**Composition Example 9 (Ointment C)**

The following pharmaceutical composition (formulation) is an ointment suitable for external topical administration (e.g. topical administration to skin), and comprises 2% w/w of *N*-{[1,6-diethyl-4-(tetrahydro-2*H*-pyran-4-ylamino)-1*H*-pyrazolo[3,4-*b*]pyridin-5-yl]methyl}-5-methyl-2-pyrazinecarboxamide (i.e. the "free base" form) (e.g: this can be as prepared in Example 236):

| Product: Ointment C<br>Batch Size: 20 g | | | |
|---|---|---|---|
| *Ingredient* | *Concentration in composition (% w/w)* | *Theoretical Amount (g)* | *Actual Amount (g)* |
| white petrolatum | 73 | 14.6 | 14.69 |
| decamethyl-cyclopentasiloxane (ST-Cyclomethicone 5-NF ™) | 25 | 5 | 5.00 |
| Example 236 (in "free base" form) | 2 | 0.4 | 0.4014 |
| TOTAL | | 20 | 20.0914 |

*Preparation Procedure:*

Approximately the following procedure is used to prepare Ointment C:
The white petrolatum (a solid at room temperature, and e.g. which can optionally be high melting point white petrolatum such as Penreco Ultima White™ grade white petrolatum) and the decamethyl-cyclopentasiloxane (a liquid at room temperature) are heated together, in a beaker in a hot water bath, to a temperature of approximately 60-65 ˚C, to melt the white petrolatum.
The drug substance (*N*-{[1,6-diethyl-4-(tetrahydro-2*H*-pyran-4-ylamino)-1*H*-pyrazolo[3,4-*b*]pyridin-5-yl]methyl}-5-methyl-2-pyrazinecarboxamide, which, for example, can be as prepared in Example 236, Synthesis C) is added slowly into the melted oil phase and is stirred with a spatula arid dispersed completely. The mixture is homogenised under high shear conditions (using setting #5, a high setting, on an Ultra-turrax T25 homogenizer), using the small homogenizer shaft, for 10 minutes.
The ointment formulation is allowed to cool to room temperature (e.g. about 17 to about 22˚C) while stirring.

**Composition Example 10 (Ointment E)**

The following pharmaceutical composition (formulation) is an ointment suitable for external topical administration (e.g. topical administration to skin), and comprises 2% w/w of *N*-{[1,6-diethyl-4-(tetrahydro-2*H*-pyran-4-ylamino)-1*H*-pyrazolo[3,4-*b*]pyridin-5-yl]methyl}-1-methyl-1*H*-pyrazole-3-carboxamide (i.e. the "free base" form) (e.g. this can be as prepared in Example 238):

Product: Ointment E
Batch Size: 20 g

| *Ingredient* | *Concentration in composition (% w/w)* | *Theoretical Amount (g)* | *Actual Amount (g)* |
|---|---|---|---|
| white petrolatum | 73 | 14.6 | 14.61 |
| decamethyl-cyclopentasiloxane (ST-Cyclomethicone 5-NF™) | 25 | 5 | 5.01 |
| Example 238 (in "free base" form) | 2 | 0.4 | 0.4009 |
| TOTAL | | 20 | 20.0209 |

*Preparation Procedure:*

Approximately the following procedure is used to prepare Ointment E:
The white petrolatum (a solid at room temperature, and e.g. which can optionally be high melting point white petrolatum such as Penreco Ultima White™ grade white petrolatum) and the decamethyl-cyclopentasiloxane (a liquid at room temperature) are heated together, in a beaker in a hot water bath, to a temperature of approximately 60-65 ˚C, to melt the white petrolatum.
The drug substance (*N*-{[1,6-diethyl-4-(tetrahydro-2*H*-pyran-4-ylamino)-1*H*-pyrazolo[3,4-*b*]pyridin-5-yl]methyl}-1-methyl-1*H*-pyrazole-3-carboxamide, which, for example, can be as prepared in Example 238, Synthesis C) is added slowly into the melted oil phase and is stirred with a spatula and dispersed completely. The mixture is homogenised under high shear conditions (using setting #5, a high setting, on an Ultra-turrax T25 homogenizer), using the small homogenizer shaft, for 10 minutes.
The ointment formulation is allowed to cool to room temperature (e.g. about 17 to about 22˚C) while stirring.

**Composition Example 11 (Water-in-oil cream Cr-C)**

The following pharmaceutical composition (formulation) is believed to be a water-in-oil cream emulsion, for external topical administration (e.g. topical administration to skin), and comprises propylene glycol and 2% w/w of *N*-{[1,6-diethyl-4-(tetrahydro-2*H*-pyran-4-ylamino)-1*H*-pyrazolo[3,4-*b*]pyridin-5-yl]methyl}-5-methyl-2-pyrazinecarboxamide (i.e. the "free base" form) (e.g. this can be as prepared in Example 236):

| Product: Water-in-oil cream Cr-C | | | |
|---|---|---|---|
| Batch Size: 20 g | | | |
| *Ingredient (%* | *Concentration in composition (%w/w)* | *Theoretical Amount (g)* | *Actual Amount (g)* |
| white petrolatum | 40 | 8 | 8.09 |
| mineral oil | 10 | 2 | 2.02 |
| steareth-2 = polyoxyl 2 stearyl ether (Volpo s-2 ™) (surfactant) | 8 | 1.6 | 1.61 |
| Aqueous phase | | | |
| propylene glycol | 20 | 4 | 4.01 |
| purified water | 20 | 4 | 4.03 |
| Example 236 (in "free base" form) | 2 | 0.4 | 0.4004 |
| TOTAL | | 20 | 20.1604 |

*Preparation Procedure:*

Approximately the following procedure is used to prepare Water-in-oil cream Cr-C:

The white petrolatum (which can optionally be high melting point white petrolatum such as Penreco Ultima White™ grade white petrolatum), the mineral oil and the steareth-2 (Volpo s-2 ™) are heated together, via a hot water bath, to a temperature of approximately 60-65 ˚C to form an oily phase.

In a separate container, the drug substance (*N*-{[1,6-diethyl-4-(tetrahydro-2*H*-pyran-4-ylamino)-1*H*-pyrazolo [3,4-*b*]pyridin-5-yl]methyl}-5-methyl-2-pyrazinecarboxamide, which, for example, can be as prepared in Example 236, Synthesis C) is dispersed in water and sonicated for approximately 10 minutes. Propylene glycol is added to this aqueous phase and the mixture is sonicated for another 10 minutes. The drug substance is at least partly in suspension.

The aqueous phase is heated to approximately the same temperature as the oily phase (e.g. is heated to approximately 60-65 ˚C), and then the aqueous phase is added slowly to the oily phase while homogenizing the mixture under high shear conditions (using setting #5, a high setting, e.g. on an Ultra-turrax T25 homogenizer), for approximately 10 minutes.

After homogenization, the formulation is allowed to cool to room temperature (e.g. about 17 to about 22 ˚C) with constant mixing with a spatula. The cream formulation is packed into a 20mL scintillation vial.

**Composition Example 12 (Water-in-oil cream Cr-E)**

The following pharmaceutical composition (formulation) is believed to be a water-in-oil cream emulsion, for external topical administration (e.g. topical administration to skin), and comprises propylene glycol and 2% w/w of *N*-{[1,6-diethyl-4-(tetrahydro-2*H*-pyran-4-ylamino)-1*H*-pyrazolo[3,4-*b*]pyridin-5-yl]methyl}-1-methyl-1*H*-pyrazole-3-carboxamide (i.e. the "free base" form) (e.g. this can be as prepared in Example 238):

| Product: Water-in-oil cream Cr-E | | | |
|---|---|---|---|
| Batch Size: 20 g | | | |
| *Ingredient* | *Concentration in composition (% w/w)* | *Theoretical Amount (g)* | *Actual Amount (g)* |
| white petrolatum | 40 | 8 | 8.07 |
| mineral oil | 10 | 2 | 2.01 |
| steareth-2 = polyoxyl 2 stearyl ether (Volpo s-2™) (surfactant) | 8 | 1.6 | 1.61 |
| Aqueous phase | | | |
| propylene glycol | 20 | 4 | 4.06 |

(continued)

| Product: Water-in-oil cream Cr-E | | | |
|---|---|---|---|
| Batch Size: 20 g | | | |
| *Ingredient* | *Concentration in composition (% w/w)* | *Theoretical Amount (g)* | *Actual Amount (g)* |
| purified water | 20 | 4 | 4.02 |
| Example 238 (in "free base" form) | 2 | 0.4 | 0.4000 |
| TOTAL | | 20 | 20.17 |

*Preparation Procedure:*

Approximately the following procedure is used to prepare Water-in-oil cream Cr-E:

The white petrolatum (which can optionally be high melting point white petrolatum such as Penreco Ultima White ™ grade white petrolatum), the mineral oil and the steareth-2 (Volpo s-2 ™) are heated together, via a hot water bath, to a temperature of approximately 60-65 °C to form an oily phase.

In a separate container, the drug substance (*N*-{[1,6-diethyl-4-(tetrahydro-2*H*-pyran-4-ylamino)-1*H*-pyrazolo [3,4-*b*]pyridin-5-yl]methyl}-1-methyl-1*H*-pyrazole-3-carboxamide, which, for example, can be as prepared in Example 238, Synthesis C) is dispersed in water and sonicated for approximately 10 minutes. Propylene glycol is added to this aqueous phase and the mixture is sonicated for another 10 minutes. The drug substance is at least partly in suspension.

The aqueous phase is heated to approximately the same temperature as the oily phase (e.g. is heated to approximately 60-65 °C), and then the aqueous phase is added slowly to the oily phase while homogenizing the mixture under high shear conditions (using setting #5, a high setting, e.g. on an Ultra-turrax T25 homogenizer), for approximately 10 minutes.

After homogenization, the formulation is allowed to cool to room temperature (e.g. about 17 to about 22 °C) with constant mixing with a spatula. The cream formulation is packed into a 20mL scintillation vial.

## Claims

**1.** A compound of formula (I) or a salt thereof (in particular, a pharmaceutically acceptable salt thereof):

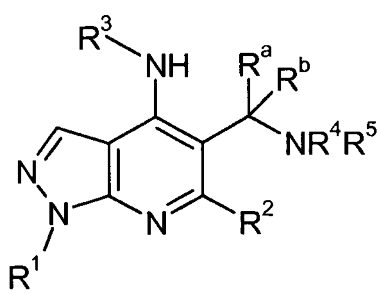

(I)

wherein:

$R^1$ is $C_{1-3}$a[kyl, $C_{1-3}$fluoroalkyl, or-$CH_2CH_2OH$;

$R^2$ is a hydrogen atom (H), methyl, ethyl, n-propyl, isopropyl, n-butyl, $C_{1-2}$fluoroalkyl, cyclopropyl, cyclobutyl, (cyclopropyl)methyl-, cyano (-CN), or -$CH_2OH$;

$R^3$ is optionally substituted $C_{4-7}$cycloalkyl or optionally substituted mono-unsaturated-$C_{5-7}$cycloalkenyl or an optionally substituted heterocyclic group of sub-formula (aa), (bb) or (cc);

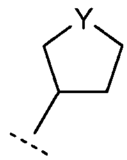 or 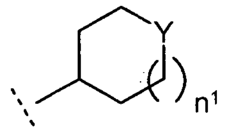 or 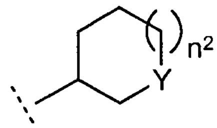

(aa) (bb) (cc)

in which $n^1$ and $n^2$ independently are 1 or 2; and in which Y is O, S, $SO_2$, or $NR^{10}$; where $R^{10}$ is a hydrogen atom (H), methyl, $C(O)NH_2$, C(O)-methyl, or C(O)-$C_1$fluoroalkyl;
or $R^3$ is a bicyclic group of sub-formula (ee):

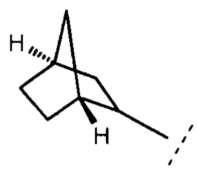

(ee) ;

and wherein, when $R^3$ is optionally substituted $C_{4-7}$cycloalkyl, then $R^3$ is $C_{4-7}$cycloalkyl optionally substituted on a ring carbon with one or two substituents independently being: oxo (=O); OH; methoxy; $C_1$fluoroalkoxy; $NH_2$; $C_{1-2}$alkyl; $C_1$fluoroalkyl; $-CH_2OH$; -CH(Me)OH; $-CH_2CH_2OH$; $-CH_2NH_2$; -C(O)OH; $-C(O)NHR^{24}$ wherein $R^{24}$ is H or methyl; $-C(O)R^{25}$ wherein $R^{25}$ is methyl; fluoro; hydroxyimino (=N-OH); or ($C_{1-2}$alkoxy)imino (=N-$OR^{26}$ where $R^{26}$ is $C_{1-2}$alkyl); and wherein any OH, methoxy, fluoroalkoxy or $NH_2$ substituent is not substituted at the $R^3$ ring carbon attached (bonded) to the -NH- group of formula (I);
and wherein, when $R^3$ is the optionally substituted heterocyclic group of sub-formula (aa), (bb) or (cc), then $R^3$ is the heterocyclic group of sub-formula (aa), (bb) or (cc) optionally substituted on a ring carbon with one or two substituents independently being oxo (=O), OH or methyl; and wherein any OH substituent is not substituted at the $R^3$ ring carbon attached (bonded) to the -NH- group of formula (I) and is not substituted at either $R^3$ ring carbon bonded to the Y group of the heterocyclic group (aa), (bb) or (cc);
and wherein, when $R^3$ is optionally substituted mono-unsaturated-$C_{5-7}$cycloalkenyl, then the cycloalkenyl is optionally substituted on a ring carbon with one substituent being fluoro or methyl , and the $R^3$ ring carbon bonded to the -NH- group of formula (I) does not partake in the cycloalkenyl double bond;
provided that:

when $R^3$ is the heterocyclic group of sub-formula (aa) and Y is $NR^{10}$, then $R^{10}$ is not C(O)-methyl, or C (O)-$C_1$fluoroalkyl; and
when $R^3$ is the heterocyclic group of sub-formula (bb), and Y is $NR^{10}$, then $R^{10}$ is not methyl; and
when $R^3$ is the heterocyclic group of sub-formula (cc), then Y is O, S, $SO_2$ or $NR^{10}$ wherein $R^{10}$ is H or methyl;

and wherein:

when $R^3$ is optionally substituted $C_{4-7}$cycloalkyl, then any $-C(O)NHR^{24}$ or $-C(O)R^{25}$ substituent on a ring carbon is: at the 3-position of a $R^3$ cyclobutyl ring; or at the 3- or 4- position of a $R^3$ cyclopentyl ring; or at the 4-position of a $R^3$ cyclohexyl ring; or at the 3-, 4-, 5- or 6- position of a $R^3$ cycloheptyl ring (wherein, in this connection, the 1-position of the $R^3$ cycloalkyl ring is deemed to be the connection point to the -NH- in formula (I), that is the ring atom connecting to the -NH- in formula (I));

and wherein:

when $R^3$ is optionally substituted $C_{4-7}$cycloalkyl, then any OH, methoxy, fluoroalkoxy, $-CH_2OH$, -CH(Me) OH, $-CH_2CH_2OH$, $-CH_2NH_2$, or-C(O)OH substituent on a ring carbon is: at the 3-position of a $R^3$ cyclobutyl ring; or at the 3- or 4- position of a $R^3$ cyclopentyl ring; or at the 3-, 4- or 5- position of a $R^3$ cyclohexyl ring;

or at the 3-, 4-, 5- or 6- position of a $R^3$ cycloheptyl ring; and

and wherein:

when $R^3$ is the heterocyclic group of sub-formula (aa), (bb) or (cc), then any OH substituent on a ring carbon is: at the 5-position of a six-membered $R^3$ heterocyclic group of sub-formula (cc) wherein $n^2$ is 1; or at the 5- or 6- position of a seven-membered $R^3$ heterocyclic group of sub-formula (cc) wherein $n^2$ is 2; or at the 6-position of a seven-membered $R^3$ heterocyclic group of sub-formula (bb) wherein $n^1$ is 2 (wherein, in this connection, the 1-position of the $R^3$ heterocyclic ring is deemed to be the connection point to the -NH- in formula (I), that is the ring atom connecting to the -NH- in formula (I), and the remaining positions of the ring are then numbered so that the ring heteroatom takes the lowest possible number);

and wherein:

$R^a$ is a hydrogen atom (H), methyl or ethyl;
$R^b$ is a hydrogen atom (H) or methyl;
wherein, when $R^b$ is methyl, then $R^a$ is methyl and $R^2$ is a hydrogen atom (H);

$R^4$ is a hydrogen atom (H), methyl, ethyl, n-propyl, -C(O)-Me, or -C(O)-$C_1$fluoroalkyl;
provided that when $R^4$ is -C(O)-Me or -C(O)-$C_1$fluoroalkyl, then $R^5$ is -$CH_2$-Ar;
and
$R^5$ is:

- C(O)-$(CH_2)_n$-Ar, -C(O)-Het, -C(O)-$C_{1-6}$alkyl, -C(O)-$C_1$fluoroalkyl,
- C(O)-$(CH_2)_2$-C(O)-$NR^{15b}NR^{15b}$, -C(O)-$CH_2$-C(O)-$NR^{15b}NR^{15b}$,
- C(O)-$NR^{15b}$-$(CH_2)_{m}{}^1$-Ar, -C(O)-$NR^{15b}$-Het, -C(O)-$NR^{15b}$-$C_{1-6}$alkyl,
- C(O)-$NR^{5a}R^{5b}$, -$S(O)_2$-$(CH_2)_{m}{}^2$-Ar, -$S(O)_2$-Het, -$S(O)_2$-$C_{1-6}$alkyl,

or -$CH_2$-Ar;
wherein n and $m^1$ and $m^2$ independently are 0, 1 or 2; and
Ar, independent of other Ar, has the sub-formula (x) or (z), wherein (z) is connected at a ring carbon:

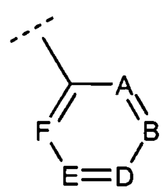
(x)

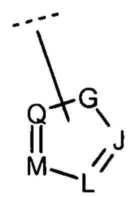
(z)

or $R^4$ and $R^5$ taken together are -$(CH_2)_{p}{}^1$- (optionally substituted), or -$(CH_2)_2$-$X^5$-$(CH_2)_2$-, or -C(O)-$(CH_2)_{p}{}^2$-, or -C(O)-N($R^{15}$)-$(CH_2)_{p}{}^3$-, in which: $X^5$ is $NR^{17}$ and $p^1$ is 4, 5 or 6, and $p^2$ is 3, 4 or 5, and $p^3$ is 2 or 3;
or $NR^4R^5$ is of sub-formula (y), (y1), (y2) or (y3) wherein $p^4$ is 1 or 2:

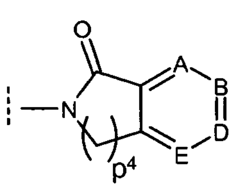
(y)

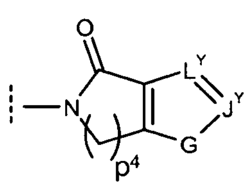
(y1)

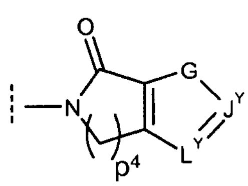
(y2)

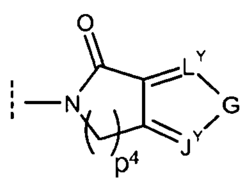
(y3)

and wherein, when $R^4$ and $R^5$ taken together is $-(CH_2)_p{}^1-$, then the $NR^4R^5$ ring is optionally substituted on the 3-position ring-carbon atom and/or on the 4-position ring-carbon atom (wherein the ring-nitrogen is the 1-position) by one or two substituents independently being:
phenyl; phenyl substituted by one or two (e.g. one) substituents independently being methyl or $CF_3$ or fluoro or chloro or methoxy or difluoromethoxy;
phenyl-C(O)-; phenyl-C(O)- whose phenyl ring is substituted by one or two (e.g. one) substituents independently being methyl or fluoro or chloro or methoxy;
benzyloxy; phenyloxy; phenyloxy whose phenyl ring is substituted by one or two (e.g. one) substituents independently being methyl or $CF_3$ or fluoro or chloro or methoxy or difluoromethoxy;
a 5-membered heteroaromatic ring being oxazolyl, isoxazolyl, thiazolyl, 1,2,4-oxadiazolyl, 1,3,4-oxadiazolyl, furanyl, thienyl, pyrrolyl, pyrazolyl, imidazolyl, triazolyl or tetrazolyl, wherein the 5-membered heteroaromatic ring is optionally substituted on a ring carbon by one substituent being methyl or ethyl or isopropyl or t-butyl or phenyl or pyridinyl and/or is optionally substituted on a ring nitrogen by one substituent being methyl or ethyl or isopropyl or t-butyl or phenyl; $-CH_2$-(pyrrol-1-yl);
a 6-membered heteroaromatic ring being pyridinyl, pyrimidinyl, pyrazinyl or pyridazinyl, wherein the 6-membered heteroaromatic ring is optionally substituted on a ring carbon either by one methyl substituent or by one OH substituent which is substituted on a ring carbon bonded to a ring nitrogen (including the keto tautomer thereof);
$C_{1-2}$alkyl (e.g. methyl);
cyano (-CN);
or (4-hydroxyphenyl)-C(O)-$CH_2$-$CH_2$-;
or alternatively when $R^4$ and $R^5$ taken together is $-(CH_2)_p{}^1-$ and $p^1$ is 5, then the $NR^4R^5$ ring is optionally substituted on the 4-position ring-carbon atom by two substituents which when taken together are -O-C(O)-N(benzyl)-$CH_2$-;
or alternatively when $R^4$ and $R^5$ taken together is $-(CH_2)_p{}^1-$ and $p^1$ is 4 or 5, then the $NR^4R^5$ ring is optionally substituted on the 3-position and 4-position ring-carbon atoms by two substituents which when taken together are =CH-CH=CH-CH=;
and wherein, in sub-formula (x), and independently in sub-formula (y):

A is C-$R^{6A}$ or nitrogen (N),
B is C-$R^{6B}$ or nitrogen (N),
D is C-$R^{6D}$ or nitrogen (N),
E is C-$R^{6E}$ or nitrogen (N),
F is C-$R^{6F}$ or nitrogen (N),

wherein, $R^{6A}$, $R^{6B}$, $R^{6D}$, $R^{6E}$ and $R^{6F}$ independently are: a hydrogen atom (H), a halogen atom; $C_{1-6}$alkyl (e.g. $C_{1-4}$alkyl or $C_{1-2}$alkyl); $C_{1-4}$fluoroalkyl (e.g. $C_{1-2}$fluoroalkyl); $C_{3-6}$cycloalkyl; $C_{1-4}$alkoxy (e.g. $C_{1-2}$alkoxy); $C_{1-2}$fluoroalkoxy; $C_{3-6}$cycloalkyloxy; -C(O)$R^{16}$; -C(O)O$R^{30}$; $-S(O)_2-R^{16}$ (e.g. $C_{1-3}$alkyl-$S(O)_2$-); $R^{16}$-$S(O)_2$-$NR^{15}$- (e.g. $C_{1-3}$alkyl-$S(O)_2$-NH-); $R^{12}R^{13}N$-$S(O)_2$-; $C_{1-2}$alkyl-C(O)-$R^{15}N$-$S(O)_2$-; $C_{1-4}$alkyl-S(O)-, Ph-S(O)-, $R^{12}R^{13}N$-C(O)-; -$NR^{15}$-C(O)$R^{16}$; $R^7R^8N$; nitro ($-NO_2$); OH (including any tautomer thereof); $C_{1-4}$alkoxymethyl; $C_{1-4}$alkoxyethyl; $C_{1-2}$alkyl-$S(O)_2$-$CH_2$-; $R^{12}R^{13}N$-$S(O)_2$-$CH_2$-; $C_{1-2}$alkyl-$S(O)_2$-$NR^{15}$-$CH_2$-; -$CH_2$-OH; -$CH_2CH_2$-OH; -$CH_2$-$NR^7R^8$; -$CH_2$-$CH_2$-$NR^7R^8$; -$CH_2$-C(O)O$R^{30}$; -$CH_2$-C(O)-$NR^{12}R^{13}$; -$CH_2$-$NR^{15}$-C(O)-$C_{l-3}$alkyl; $-(CH_2)_n{}^{14}$-$Het^1$ where $n^{14}$ is 0 or 1; cyano (-CN); $Ar^6$; 1-pyrrolyl; or phenyl, pyridinyl or pyrimidinyl wherein the phenyl, pyridinyl or pyrimidinyl independently are optionally substituted by one or two substituents independently being fluoro, chloro, $C_{1-2}$alkyl, $C_1$fluoroalkyl, $C_{1-2}$alkoxy or $C_1$fluoroalkoxy;
provided that $R^{6A}$, $R^{6B}$, $R^{6D}$, $R^{6E}$ and $R^{6F}$ independently cannot be a chlorine, bromine or iodine atom (and optionally not a fluorine atom) when substituted on a ring-carbon which is bonded to a ring-nitrogen;
and/or two adjacent groups selected from $R^{6A}$, $R^{6B}$, $R^{6D}$, $R^{6E}$ and $R^{6F}$ are taken together and are: -CH=CH-CH=CH-, $-(CH_2)_n{}^{14a}-$ where $n^{14a}$ is 3, 4 or 5 (e.g. 3 or 4),-O-$(CH_2)_n{}^{14b}$-O- where $n^{14b}$ is 1 or 2; -O-$(CH_2)_n{}^{14c}$- or- $S(O)_2$-$(CH_2)_n{}^{14c}$- where $n^{14c}$ is 2 or 3 (e.g. 2); -CH=CH-$NR^{15b}$-; -N-CH-$NR^{15b}$-; -CH=N-$NR^{15b}$-; -N=N-$NR^{15b}$-; -NH-NH-C(O)-; -CH=CH-O-; -N=CH-O-; or -CH=CH-S-;
provided that:

two or more of A, B, D, E and F are independently C-H (carbon-hydrogen), C-F (carbon-fluorine), or nitrogen (N);
and no more than two of A, B, D, E and F are nitrogen (N),

and wherein, in sub-formula (z), and independently in sub-formulae (y1), (y2) and (y3):

G is O or S or $NR^9$ wherein $R^9$ is a hydrogen atom (H), $C_{1-4}$alkyl, or $C_{1-2}$fluoroalkyl;
J is C-$R^{6J}$, C-[connection point to formula (I)], or nitrogen (N),
L is C-$R^{6L}$, C-[connection point to formula (I)], or nitrogen (N),
M is C-$R^{6M}$, C-[connection point to formula (I)], or nitrogen (N),
Q is C-$R^{6Q}$, C-[connection point to formula (I)], or nitrogen (N),
$J^Y$ is C-$R^{6J}$ or nitrogen (N), and
$L^Y$ is C-$R^{6L}$ or nitrogen (N),

wherein, $R^{6J}$, $R^{6L}$, $R^{6M}$ and $R^{6Q}$ independently are: a hydrogen atom (H), a halogen atom; $C_{1-4}$alkyl (e.g. $C_{1-2}$alkyl); $C_{1-3}$fluoroalkyl (e.g. $C_{1-2}$fluoroalkyl); $C_{3-6}$cycloalkyl; $C_{1-4}$alkoxy (e.g. $C_{1-2}$alkoxy); $C_{1-2}$fluoroalkoxy; $C_{3-6}$cycloalkyloxy; -$CH_2$-OH; -$CH_2$-OMe; OH (including any tautomer thereof); 2-pyridinyl; 3-pyridinyl; 4-pyridinyl; or phenyl optionally substituted by one or two substituents independently being fluoro, chloro, $C_{1-2}$alkyl, $C_1$fluoroalkyl, $C_{1-2}$alkoxy or $C_1$fluoroalkoxy;
provided that:

two or more of J, L, M and Q are independently C-H, C-F, C-$C_{1-2}$alkyl (e.g. C-Me), C-$C_1$fluoroalkyl (e.g. C-$CF_3$), C-[connection point to formula (I)], or nitrogen (N);
and no more than three of J, L, M and Q are nitrogen (N);
and one or both of $J^Y$ and $L^Y$ are independently C-H, C-F, C-$C_{1-2}$alkyl (e.g. C-Me), C-$C_1$fluoroalkyl (e.g. C-$CF_3$), or nitrogen (N);

and wherein:

$NR^{5a}R^{5b}$ is a 1-pyrrolidinyl, 1-piperidinyl, or N-morpholino (4-morpholinyl) group;
$R^7$ and $R^8$ are independently a hydrogen atom (H); $C_{1-4}$alkyl (e.g. $C_{1-2}$alkyl such as methyl); $C_{3-6}$cycloalkyl; ($C_{3-6}$cycloalkyl)methyl-; or phenyl optionally substituted by one or two substituents independently being: fluoro, chloro, $C_{1-2}$alkyl, $C_1$fluoroalkyl, $C_{1-2}$alkoxy or $C_1$fluoroalkoxy;
or $R^7$ and $R^8$ together are -$(CH_2)_n{}^6$- or -C(O)-$(CH_2)_n{}^7$- or -C(O)-$(CH_2)_n{}^{10}$-C(O)- or -$(CH_2)_2$-$X^7$-$(CH_2)_2$-or-C(O)-$X^7$-$(CH_2)_n{}^{10}$- in which: $n_6$ is 3, 4, 5, 6 or 7, $n^7$ is 3, 4 or 5, $n^{10}$ is 2 or 3, and $X^7$ is O or $NR^{14}$;
$R^{12}$ and $R^{13}$ independently are H; $C_{1-4}$alkyl (e.g. $C_{1-2}$alkyl); $C_{3-6}$cycloalkyl; ($C_{3-6}$cycloalkyl)methyl-; or phenyl optionally substituted by one or two substituents independently being: fluoro, chloro, $C_{1-2}$alkyl, $C_1$fluoroalkyl, $C_{1-2}$alkoxy or $C_1$fluoroalkoxy;
or $R^{12}$ and $R^{13}$ together are -$(CH_2)_n{}^{6a}$- or or -$(CH_2)_2$-$X^{12}$-$(CH_2)_2$- in which: $n^{6a}$ is 3, 4, 5, 6 or 7, and $X^{12}$ is O or $NR^{14}$;
$R^{14}$, independent of other $R^{14}$, is: a hydrogen atom (H); $C_{1-4}$alkyl (e.g. $C_{1-2}$alkyl); $C_{1-2}$fluoroalkyl (e.g. $CF_3$); cyclopropyl; -C(O)-$C_{1-4}$alkyl (e.g. -C(O)Me); -C(O)$NR^{7a}R^{8a}$ (e.g. -C(O)$NH_2$); or-$S(O)_2$-$C_{1-4}$alkyl (e.g. -$S(O)_2$Me);

$R^{7a}$ is a hydrogen atom (H) or $C_{1-4}$alkyl;
$R^{8a}$ is a hydrogen atom (H) or methyl;
$R^{15}$, independent of other $R^{15}$, is a hydrogen atom (H) or $C_{1-4}$alkyl;
$R^{15b}$, independent of other $R^{15b}$, is H or $C_{1-2}$alkyl;
$R^{16}$, independent of other $R^{16}$, is:

$C_{1-6}$alkyl (e.g. $C_{1-4}$alkyl or $C_{1-2}$alkyl);
$C_{3-6}$cycloalkyl (e.g. $C_{5-6}$cycloalkyl);
$C_{3-6}$cycloalkyl-$CH_2$- (e.g. $C_{5-6}$cycloalkyl-$CH_2$-);
pyridinyl (e.g. pyridin-2-yl) optionally substituted on a ring carbon atom by one of: a halogen atom, $C_{1-2}$alkyl, $C_1$fluoroalkyl, $C_{1-2}$alkoxy or $C_1$fluoroalkoxy;
$Ar^{16}$;
phenyl optionally substituted by one or two substituents independently being: a halogen atom, $C_{1-2}$alkyl, $C_1$fluoroalkyl, $C_{1-2}$alkoxy or $C_1$fluoroalkoxy;
benzyl optionally substituted on its ring by one or two substituents independently being: a halogen atom, $C_{1-2}$alkyl, $C_1$fluoroalkyl, $C_{1-2}$alkoxy or $C_1$fluoroalkoxy; or
a 4-, 5-, 6- or 7-membered saturated heterocyclic ring connected at a ring-carbon and containing one or two ring-hetero-atoms independently selected from O, S, and N; wherein any ring-nitrogens which are present are present as $NR^{27}$ where $R^{27}$ is H, $C_{1-2}$alkyl or -C(O)Me; and wherein the ring is optionally substituted at carbon by one $C_{1-2}$alkyl or oxo (=O) substituent, provided that any oxo (=O) substituent is

substituted at a ring-carbon atom bonded to a ring-nitrogen;

$R^{17}$, independent of other $R^{17}$, is:

$C_{5-6}$cycloalkyl; -C(O)-$C_{1-4}$alkyl (e.g. -C(O)-$C_{3-4}$alkyl); -C(O)-$C_{3-6}$cycloalkyl (e.g. -C(O)-cyclopropyl); -S(O)$_2$-$C_{1-4}$alkyl (e.g. -S(O)$_2$Me or-S(O)$_2$Et);
-C(O)-$Ar^{176}$; -C(O)-$Ar^{175}$; -C(O)-$Het^1$; - $CH_2$-C(O)-$Het^1$; - $CH_2$-C(O)-(pyrrolidin-1-yl); -S(O)$_2$-$Ar^{176}$; -S(O)$_2$-$Ar^{175}$; $Ar^{176}$; $Ar^{175}$;
-C(O)-phenyl or -S(O)$_2$-phenyl, wherein, independently, the phenyl ring is unsubstituted or substituted by one or two (e.g. one) substituents independently being methyl or ethyl or $CF_3$ or fluoro or chloro or methoxy or difluoromethoxy or cyano (-CN) or -C(O)$NH_2$ or -C(O)-Me;
phenyl; phenyl substituted by one or two (e.g. one) substituents independently being methyl or ethyl or $CF_3$ or fluoro or chloro or methoxy or difluoromethoxy or cyano (-CN) or -C(O)$NR^{7a}R^{8a}$ (e.g. -C(O)$NH_2$) or -C(O)-Me;
or benzyl or -CH(Me)-phenyl, wherein, independently, the phenyl ring is unsubstituted or substituted by one or two (e.g. one) substituents independently being methyl or fluoro or chloro or methoxy;

wherein $Ar^{175}$ is a 5-membered heteroaromatic ring being oxazolyl, isoxazolyl, thiazolyl, 1,2,4-oxadiazolyl, 1,3,4-oxadiazolyl, furanyl, thienyl, pyrrolyl, pyrazolyl, imidazolyl, triazolyl or tetrazolyl,
wherein the 5-membered heteroaromatic ring $Ar^{175}$ is optionally substituted on a ring carbon by one substituent being methyl or ethyl or isopropyl or t-butyl or phenyl or pyridinyl and/or is optionally substituted on a ring nitrogen by one substituent being methyl or ethyl or isopropyl or t-butyl or phenyl;
or wherein the 5-membered heteroaromatic ring $Ar^{175}$ is optionally fused to a phenyl ring wherein the connection point to the remainder of the molecule is within the 5-membered ring;
and wherein $Ar^{176}$ is a 6-membered heteroaromatic ring being pyridinyl, pyrimidinyl, pyrazinyl or pyridazinyl, wherein the 6-membered heteroaromatic ring is optionally substituted on a ring carbon either by one methyl substituent or by one OH substituent which is substituted on a ring carbon bonded to a ring nitrogen (including the keto tautomer thereof);
$R^{30}$, independent of other $R^{30}$, is a hydrogen atom (H), $C_{1-4}$alkyl or $C_{3-6}$cycloalkyl;
$Ar^6$ and $Ar^{16}$ independently is/are a 5-membered aromatic heterocyclic ring connected at a ring-carbon and containing one O, S or $NR^{15}$ in the 5-membered ring, wherein the 5-membered ring can optionally contain in addition one or two N atoms, and wherein the heterocyclic ring is optionally substituted on a ring carbon atom by one of: a fluorine atom, a chlorine atom, $C_{1-2}$alkyl, $C_1$fluoroalkyl, -$CH_2$OH, -$CH_2$-OMe, or OH (including any keto tautomer thereof);
Het, independent of other Het, is a 4-, 5-, 6- or 7-membered saturated heterocyclic ring connected at a ring-carbon and containing one or two ring-hetero-atoms independently selected from O, S, and N; wherein any ring-nitrogens which are present are present as $NR^{31}$ where $R^{31}$ is H, $C_{1-4}$alkyl, -C(O)-$C_{1-4}$alkyl or -S(O)$_2$-$C_{1-4}$alkyl; and wherein the Het ring is optionally substituted at a ring carbon by one methyl or oxo (=O) substituent, provided that any oxo (=O) substituent is substituted at a ring-carbon atom bonded to a ring-nitrogen wherein $R^{31}$ is H or $C_{1-4}$alkyl; and
$Het^1$, independent of other $Het^1$, is a 4-, 5-, 6- or 7-membered saturated heterocyclic ring connected at a ring-carbon and containing one or two ring-hetero-atoms independently selected from O, S, and N; wherein any ring-nitrogens which are present are present as $NR^{31a}$ where $R^{31a}$ is H, $C_{1-2}$alkyl, -C(O)Me or-S(O)$_2$Me; and wherein the $Het^1$ ring is optionally substituted at a ring carbon by one methyl or oxo (=O) substituent, provided that any oxo (=O) substituent is substituted at a ring-carbon atom bonded to a ring-nitrogen wherein $R^{31a}$ is H or $C_{1-2}$alkyl.

**2.** A compound or salt as claimed in claim 1, wherein $R^1$ is ethyl.

**3.** A compound or salt as claimed in claim 1 or 2, wherein $R^2$ is methyl or ethyl.

**4.** A compound or salt as claimed in any preceding claim, wherein $R^3$ is the optionally substituted $C_{4-7}$cycloalkyl or the optionally substituted heterocyclic group of sub-formula (aa), (bb) or (cc).

**5.** A compound or salt as claimed in any preceding claim, wherein, when $R^3$ is the heterocyclic group of sub-formula (aa), (bb) or (cc), then Y is O or $NR^{10}$.

**6.** A compound or salt as claimed in any preceding claim, wherein, when $R^3$ is the heterocyclic group of sub-formula

(aa), (bb) or (cc), then $R^3$ is the heterocyclic group of sub-formula (bb) and $n^1$ is 1.

**7.** A compound or salt as claimed in any preceding claim, wherein, in $R^3$, the heterocyclic group of sub-formula (aa), (bb) or (cc) is not substituted on a ring carbon.

**8.** A compound or salt as claimed in any preceding claim, wherein $R^3$ is tetrahydro-2H-pyran-4-yl or 1-(aminocarbonyl)-4-piperidinyl; that is $NHR^3$ is of sub-formula (h) or (k2).

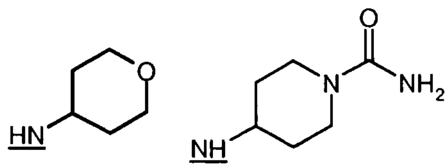

(h) (k2)

**9.** A compound or a salt thereof as claimed in any preceding claim, wherein $R^a$ is a hydrogen atom (H) and $R^b$ is a hydrogen atom (H).

**10.** A compound or a salt thereof as claimed in any preceding claim, wherein $R^4$ is a hydrogen atom (H).

**11.** A compound or a salt thereof as claimed in any preceding claim, wherein $R^5$ is:

$-C(O)-(CH_2)_n-Ar$, $-C(O)-Het$, $-C(O)-NR^{15b}-(CH_2)_{m^1}-Ar$, or $-C(O)-NR^{5a}R^{5b}$;
and $R^4$ is a hydrogen atom (H);
or $R^4$ and $R^5$ taken together are $-C(O)-N(R^{15})-(CH_2)_{p^3}-$;
or $NR^4R^5$ is of sub-formula (y), (y1), (y2) or (y3).

**12.** *N*-((1-ethyl-6-methyl-4-((tetrahydro-2*H*-pyran-4-yl)amino)-1*H*-pyrazolo[3,4-*b*]pyridin-5-yl)methyl)-5-methylpyrazine-2-carboxamide

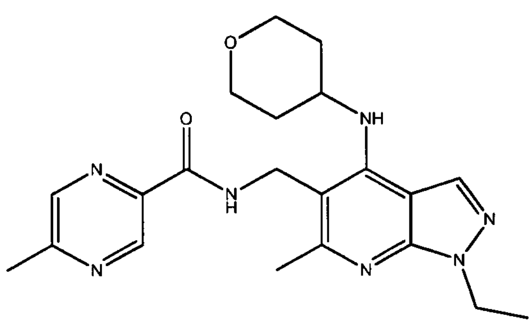

or a salt thereof.

**13.** A compound or salt as claimed in any preceding claim, for use as an active therapeutic substance in a mammal.

**14.** A pharmaceutical composition comprising a compound of formula (I), as defined in any of claims 1 to 12, or a pharmaceutically acceptable salt thereof, and one or more pharmaceutically acceptable carriers and/or excipients.

**15.** A pharmaceutical composition as claimed in claim 14, for use in the treatment and/or prophylaxis of an inflammatory and/or allergic disease, cognitive impairment or depression in a mammal.

**16.** A pharmaceutical composition as claimed in claim 15 wherein the cognitive impairment is in a neurological disorder.

**17.** A pharmaceutical composition as claimed in claim 16 wherein the neurological disorder is selected from Alzheimer's disease or schizophrenia.

**18.** The use of a compound of formula (I), as defined in any of claims 1 to 12, or a pharmaceutically acceptable salt thereof, in the manufacture of a medicament for the treatment and/or prophylaxis of asthma, chronic obstructive pulmonary disease (COPD), atopic dermatitis, urticaria, allergic rhinitis, allergic conjunctivitis, vernal conjunctivitis, eosinophilic granuloma, psoriasis, rheumatoid arthritis, septic shock, ulcerative colitis, Crohn's disease, reperfusion injury of the myocardium and brain, chronic glomerulonephritis, endotoxic shock, adult respiratory distress syndrome, multiple sclerosis, cognitive impairment, depression, or inflammatory pain, in a mammal.

**19.** The use of a compound as claimed in claim 18 wherein the cognitive impairment is in a neurological disorder.

**20.** The use of a compound as claimed in claim 19 wherein the neurological disorder is selected from Alzheimer's disease or schizophrenia.

**21.** A compound as claimed in any one of claims 1 to 12 for use in the treatment of and/or prophylaxis of asthma, chronic obstructive pulmonary disease (COPD), atopic dermatitis, urticaria, allergic rhinitis, allergic conjunctivitis, vernal conjunctivitis, eosinophilic granuloma, psoriasis, rheumatoid arthritis, septic shock, ulcerative colitis, Crohn's disease, reperfusion injury of the myocardium and brain, chronic glomerulonephritis, endotoxic shock, adult respiratory distress syndrome, multiple sclerosis, cognitive impairment, depression, or inflammatory pain, in a mammal.

**22.** A compound as claimed in claim 21 wherein the cognitive impairment is in a neurological disorder.

**23.** A compound as claimed in claim 21 wherein the neurological disorder is selected from Alzheimer's disease or schizophrenia.

## Patentansprüche

**1.** Eine Verbindung der Formel (I) oder ein Salz davon (insbesondere ein pharmazeutisch verträgliches Salz davon):

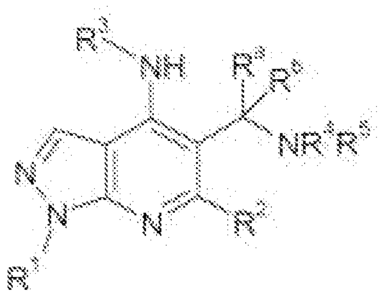

(I)

wobei:

$R^1$ $C_{1-3}$-Alkyl, $C_{1-3}$-Fluoralkyl oder $-CH_2CH_2OH$ ist;
$R^2$ ein Wasserstoffatom (H), Methyl, Ethyl, n-Propyl, Isopropyl, n-Butyl, $C_{1-2}$-Fluoralkyl, Cyclopropyl, Cyclobutyl, (Cyclopropyl)methyl-, Cyano (-CN) oder $-CH_2OH$ ist;
$R^3$ gegebenenfalls substituiertes $C_{4-7}$-Cycloalkyl oder gegebenenfalls substituiertes einfach ungesättigtes $C_{5-7}$-Cycloalkenyl oder ein gegebenenfalls substituierter heterocyclischer Rest der Unterformel (aa), (bb) oder (cc) ist;

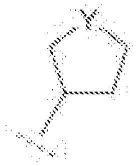

oder

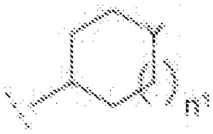

oder

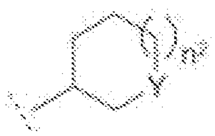

(aa) (bb) (cc)

wobei $n^1$ und $n^2$ unabhängig 1 oder 2 sind; und wobei Y gleich O, S, $SO_2$ oder $NR^{10}$ ist;
wobei $R^{10}$ ein Wasserstoffatom (H), Methyl, $C(O)NH_2$, C(O)-Methyl oder C(O)-$C_1$-Fluoralkyl ist;
oder $R^3$ ein bicyclischer Rest der Unterformel (ee) ist:

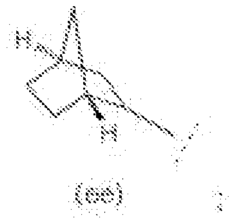

und wobei, wenn $R^3$ gegebenenfalls substituiertes $C_{4-7}$-Cycloalkyl ist, dann $R^3$ $C_{4-7}$-Cycloalkyl ist, das gegebenenfalls an einem Ring-Kohlenstoff mit einem oder zwei Substituenten substituiert ist, die unabhängig sind: Oxo (=O); OH; Methoxy; $C_1$-Fluoralkoxy; $NH_2$, $C_{1-2}$-Alkyl; $C_1$-Fluoralkyl; $-CH_2OH$; -CH(Me)OH; $-CH_2CH_2OH$; $-CH_2NH_2$, -C(O)OH; -C(O)$NHR^{24}$, wobei $R^{24}$ H oder Methyl ist; -C(O)$R^{25}$, wobei $R^{25}$ Methyl ist; Fluor; Hydroxyimino (=N-OH); oder ($C_{1-2}$-Alkoxy)imino (=N-$OR^{26}$, wobei $R^{26}$ $C_{1-2}$-Alkyl ist); und wobei jeder OH-, Methoxy-, Fluoralkoxy- oder $NH_2$-Substituent nicht an dem $R^3$-Ring-Kohlenstoff substituiert ist, der an den NH-Rest der Formel (I) angeknüpft (gebunden) ist;
und wobei, wenn $R^3$ der gegebenenfalls substituierte heterocyclische Rest der Unterformel (aa), (bb) oder (cc) ist, dann $R^3$ der heterocyclische Rest der Unterformel (aa), (bb) oder (cc) ist, der gegebenenfalls an einem Ring-Kohlenstoff mit einem oder zwei Substituenten substituiert ist, die unabhängig Oxo (=O), OH oder Methyl sind; und wobei jeder OH-Substituent nicht an dem $R^3$-Ring-Kohlenstoff substituiert ist, der an den NH-Rest der Formel (I) angeknüpft (gebunden) ist, und an keinem $R^3$-Ring-Kohlenstoff substituiert ist, der an den Y-Rest des heterocyclischen Rests (aa), (bb) oder (cc) gebunden ist;
und wobei, wenn $R^3$ gegebenenfalls substituiertes einfach ungesättigtes $C_{5-7}$-Cycloalkenyl ist, dann das Cycloalkenyl gegebenenfalls an einem Ring-Kohlenstoff mit einem Substituenten substituiert ist, bei dem es sich um Fluor oder Methyl handelt, und der $R^3$-Ring-Kohlenstoff, der an den -NH-Rest der Formel (I) gebunden ist, nicht an der Cycloalkenyl-Doppelbindung beteiligt ist;
mit der Maßgabe, dass:

wenn $R^3$ der heterocyclische Rest der Unterformel (aa) ist und Y gleich $NR^{10}$ ist, dann $R^{10}$ nicht C(O)-Methyl oder C(O)-$C_1$-Fluoralkyl ist; und
wenn $R^3$ der heterocyclische Rest der Unterformel (bb) ist und Y gleich $NR^{10}$ ist, dann $R^{10}$ nicht Methyl ist; und
wenn $R^3$ der heterocyclische Rest der Unterformel (cc) ist, dann Y gleich O, S, $SO_2$ oder $NR^{10}$ ist, wobei $R^{10}$ H oder Methyl ist;

und wobei:

wenn $R^3$ gegebenenfalls substituiertes $C_{4-7}$-Cycloalkyl ist, dann jeder -C(O)$NHR^{24}$- oder -C(O)$R^{25}$-Substituent an einem Ring-Kohlenstoff: an der 3-Position eines $R^3$-Cyclobutylrings; oder an der 3- oder 4-Position eines $R^3$-Cyclopentylrings; oder an der 4-Position eines $R^3$-Cyclohexylrings; oder an der 3-, 4-, 5- oder 6-Position eines $R^3$-Cycloheptylrings ist (wobei in diesem Zusammenhang die 1-Position des $R^3$-Cycloalkylrings der Verbindungspunkt zu dem -NH- in Formel (I) sein soll, d.h. das Ringatom, das an das -NH- in Formel (I) anknüpft);

und wobei:

wenn $R^3$ gegebenenfalls substituiertes $C_{4-7}$-Cycloalkyl ist, dann jeder OH-, Methoxy-, Fluoralkoxy-, $-CH_2OH$-, -CH(Me)OH-, $-CH_2CH_2OH$-, $-CH_2NH_2$- oder -C(O)OH-Substituent an einem Ring-Kohlenstoff: an der 3-Position eines $R^3$-Cyclobutylrings; oder an der 3- oder 4-Position eines $R^3$-Cyclopentylrings; oder an der 3-, 4- oder 5-Position eines $R^3$-Cyclohexylrings; oder an der 3-, 4-, 5- oder 6-Position eines $R^3$-Cycloheptylrings ist; und

und wobei:

wenn $R^3$ der heterocyclische Rest der Unterformel (aa), (bb) oder (cc) ist, dann jeder OH-Substituent an einem Ring-Kohlenstoff: an der 5-Position eines sechsgliedrigen $R^3$-heterocyclischen Rests der Unterformel (cc), wobei $n^2$ 1 ist; oder an der 5- oder 6-Position eines siebengliedrigen $R^3$-heterocyclischen Rests der Unterformel (cc); wobei $n^2$ 2 ist; oder an der 6-Position eines siebengliedrigen $R^3$-heterocyclischen Rests der Unterformel (bb), wobei $n^1$ 2 ist, ist (wobei in diesem Zusammenhang die 1-Position des $R^3$-heterocyclischen Rings der Verbindungspunkt zu dem -NH- in Formel (I) sein soll, d.h. das Ringatom, das an das -NH- in Formel (I) anknüpft, und die übrigen Positionen des Rings dann so nummeriert werden, dass das Ring- Heteroatom die niedrigstmögliche Zahl erhält);

und wobei:

$R^a$ ein Wasserstoffatom (H), Methyl oder Ethyl ist;
$R^b$ ein Wasserstoffatom (H) oder Methyl ist;
wobei, wenn $R^b$ Methyl ist, dann $R^a$ Methyl ist und $R^2$ ein Wasserstoffatom (H) ist;

$R^4$ ein Wasserstoffatom (H), Methyl, Ethyl, n-Propyl, -C(O)-Me oder -C(O)-$C_1$-Fluoralkyl ist;
mit der Maßgabe, dass wenn $R^4$ -C(O)-Me oder -C(O)-$C_1$-Fluoralkyl ist, dann $R^5$ gleich -$CH_2$-Ar ist;
und
$R^5$ folgendes ist:

- C(O)-$(CH_2)_n$-Ar, -C(O)-Het, -C(O)-$C_{1-6}$-Alkyl, -C(O)-$C_1$-Fluoralkyl, -C(O)-$(CH_2)_2$-C(O)-$NR^{15b}NR^{15b}$, -C(O)-$CH_2$-C(O)-$NR^{15b}NR^{15b}$, -C(O)-$NR^{15b}$-$(CH_2)_m{}^1$-Ar, -C(O)-$NR^{15b}$-Het, -C(O)-$NR^{15b}$-$C_{1-6}$-Alkyl, -C(O)-$NR^{5a}R^{5b}$, -$S(O)_2$-$(CH_2)_m{}^2$-Ar, -$S(O)_2$-Het, -$S(O)_2$-$C_{1-6}$-Alkyl oder -$CH_2$-Ar;

wobei n und $m^1$ und $m^2$ unabhängig 0, 1 oder 2 sind; und
Ar, unabhängig von anderen Ar, die Unterformel (x) oder (z) hat, wobei (z) an einen Ring-Kohlenstoff gebunden ist:

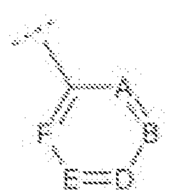
(x)

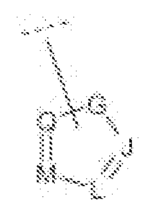
(z)

**oder** $R^4$ und $R^5$ zusammengenommen -$(CH_2)_p{}^1$- (gegebenenfalls substituiert) oder -$(CH_2)_2$-$X^5$-$(CH_2)_2$- oder -C(O)-$(CH_2)_p{}^2$- oder -C(O)-N($R^{15}$)-$(CH_2)_p{}^3$- sind, wobei: $X^5$ gleich $NR^{17}$ ist und $p^1$ 4, 5 oder 6 ist und $p^2$ 3, 4 oder 5 ist und $p^3$ 2 oder 3 ist;
**oder** $NR^4R^5$ die Unterformel (y), (y1), (y2) oder (y3) hat, wobei $p^4$ 1 oder 2 ist;

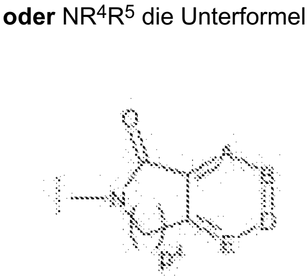
(y)

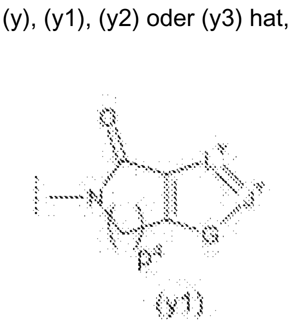
(y1)

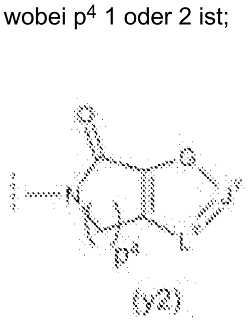
(y2)

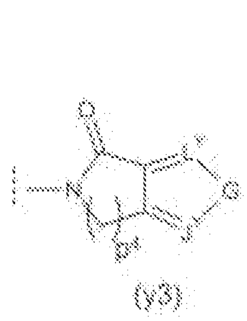
(y3)

und wobei, wenn $R^4$ und $R^5$ zusammengenommen -$(CH_2)_p{}^1$- ist, dann der $NR^4R^5$-Ring gegebenenfalls an dem Ring-Kohlenstoffatom der 3-Position und/oder an dem Ring-Kohlenstoffatom der 4-Position (wobei der Ring-Stickstoff die 1-Position ist) mit einem oder zwei Substituenten substituiert ist, die unabhängig sind:
Phenyl; Phenyl, substituiert mit einem oder zwei (z.B. einem) Substituenten, der/die unabhängig Methyl oder $CF_3$ oder Fluor oder Chlor oder Methoxy oder Difluormethoxy ist/sind;
Phenyl-C(O)-; Phenyl-C(O)-, dessen Phenylring mit einem oder zwei (z.B. einem) Substituenten substituiert ist, der/die unabhängig Methyl oder Fluor oder Chlor oder Methoxy ist/sind;
Benzyloxy; Phenyloxy; Phenyloxy, dessen Phenylring mit einem oder zwei (z.B. einem) Substituenten substituiert ist, der/die unabhängig Methyl oder $CF_3$ oder Fluor oder Chlor oder Methoxy oder Difluormethoxy ist/sind;
ein 5-gliedriger heteroaromatischer Ring, bei dem es sich um Oxazolyl, Isoxazolyl, Thiazolyl, 1,2,4-Oxadiazolyl, 1,3,4-Oxadiazolyl, Furanyl, Thienyl, Pyrrolyl, Pyrazolyl, Imidazolyl, Triazolyl oder Tetrazolyl handelt, wobei der 5-gliedrige heteroaromatische Ring gegebenenfalls an einem Ring-Kohlenstoff mit einem Substituenten substituiert ist, bei dem es sich um Methyl oder Ethyl oder Isopropyl oder t-Butyl oder Phenyl oder Pyridinyl handelt, und/oder gegebenenfalls an einem Ring-Stickstoff mit einem Substituenten substituiert ist, bei dem es sich um Methyl oder Ethyl oder Isopropyl oder t-Butyl oder Phenyl handelt;
$-CH_2$-(Pyrrol-1-yl);
ein 6-gliedriger heteroaromatischer Ring, bei dem es sich um Pyridinyl, Pyrimidinyl, Pyrazinyl oder Pyridazinyl handelt, wobei der 6-gliedrige heteroaromatische Ring gegebenenfalls an einem Ring-Kohlenstoff entweder mit einem Methyl-Substituenten oder mit einem OH-Substituenten, der an einem Ring-Kohlenstoff substituiert ist, der an einen Ring-Stickstoff (einschließlich dessen Keto-Tautomer) gebunden ist, substituiert ist;
$C_{1-2}$-Alkyl (z.B. Methyl);
Cyano (-CN);
oder (4-Hydroxyphenyl)-C(O)-$CH_2$-$CH_2$-;
oder alternativ, wenn $R^4$ und $R^5$ zusammengenommen $-(CH_2)_{p}{}^{1}$- ist und $p^1$ 5 ist, dann der $NR^4R^5$-Ring gegebenenfalls an dem Ring-Kohlenstoffatom der 4-Position mit zwei Substituenten substituiert ist, die zusammengenommen -O-C(O)-N(Benzyl)-$CH_2$- sind; oder alternativ, wenn $R^4$ und $R^5$ zusammengenommen $-(CH_2)_{p}{}^{1}$- ist und $p^1$ 4 oder 5 ist, dann der $NR^4R^5$-Ring gegebenenfalls an den Ring-Kohlenstoffatomen der 3-Position und 4-Position mit zwei Substituenten substituiert ist, die zusammengenommen =CH-CH=CH-CH= sind;
und wobei in Unterformel (x) und unabhängig in Unterformel (y):

A gleich C-$R^{6A}$ oder Stickstoff (N) ist;
B gleich C-$R^{6B}$ oder Stickstoff (N) ist;
D gleich C-$R^{6D}$ oder Stickstoff (N) ist;
E gleich C-$R^{6E}$ oder Stickstoff (N) ist;
F gleich C-$R^{6F}$ oder Stickstoff (N) ist;

wobei $R^{6A}$, $R^{6B}$, $R^{6D}$, $R^{6E}$ und $R^{6F}$ unabhängig folgendes sind: ein Wasserstoffatom (H), ein Halogenatom; $C_{1-6}$-Alkyl (z.B. $C_{1-4}$-Alkyl oder $C_{1-2}$-Alkyl); $C_{1-4}$-Fluoralkyl (z.B. $C_{1-2}$-Fluoralkyl); $C_{3-6}$-Cycloalkyl; $C_{1-4}$-Alkoxy (z.B. $C_{1-2}$-Alkoxy); $C_{1-2}$-Fluoralkoxy; $C_{3-6}$-Cycloalkyloxy; -C(O)$R^{16}$; -C(O)O$R^{30}$; $-S(O)_2-R^{16}$ (z.B. $C_{1-3}$-Alkyl-S$(O)_2$-); $R^{16}$-$S(O)_2$-$NR^{15}$- (z.B. $C_{1-3}$-Alkyl-$S(O)_2$-NH-); $R^{12}R^{13}N$-$S(O)_2$-; $C_{1-2}$-Alkyl-C(O)-$R^{15}$N-$S(O)_2$-; $C_{1-4}$-Alkyl-S(O) -, Ph-S(O)-, $R^{12}R^{13}$N-C(O)-; -$NR^{15}$-C(O)$R^{16}$; $R^7R^8$N; Nitro (-$NO_2$); OH (einschließlich all seiner Tautomere); $C_{1-4}$-Alkoxymethyl; $C_{1-4}$-Alkoxyethyl; $C_{1-2}$-Alkyl-$S(O)_2$-$CH_2$-; $R^{12}R^{13}$N-$S(O)_2$-$CH_2$-; $C_{1-2}$-Alkyl-S$(O)_2$-$NR^{15}$-$CH_2$-; -$CH_2$-OH; -$CH_2CH_2$-OH; -$CH_2$-$NR^7R^8$; -$CH_2$-$CH_2$-$NR^7R^8$; -$CH_2$-C(O)O$R^{30}$; -$CH_2$-C(O)-$NR^{12}R^{13}$; -$CH_2$-$NR^{15}$-C(O)-$C_{1-3}$-Alkyl; $-(CH_2)_{n}{}^{14}$-$Het^1$, wobei $n^{14}$ 0 oder 1 ist; Cyano (-CN); $Ar^6$; 1-Pyrrolyl; oder Phenyl, Pyridinyl oder Pyrimidinyl, wobei das Phenyl, Pyridinyl oder Pyrimidinyl unabhängig gegebenenfalls mit einem oder zwei Substituenten substituiert sind, die unabhängig Fluor, Chlor, $C_{1-2}$-Alkyl, $C_1$-Fluoralkyl, $C_{1-2}$-Alkoxy oder $C_1$-Fluoralkoxy sind;
mit der Maßgabe, dass $R^{6A}$, $R^{6B}$, $R^{6D}$, $R^{6E}$ und $R^{6F}$ unabhängig nicht ein Chlor-, Brom-oder Iodatom (und gegebenenfalls kein Fluoratom) sein können, wenn sie an einem Ring-Kohlenstoff substituiert sind, der an einen Ring-Stickstoff gebunden ist;
und/oder zwei benachbarte Reste, ausgewählt aus $R^{6A}$, $R^{6B}$, $R^{6D}$; $R^{6E}$ und $R^{6F}$, zusammengenommen werden und folgendes sind: -CH=CH-CH=CH-, $-(CH_2)_{n}{}^{14a}$-, wobei $n^{14a}$ 3, 4 oder 5 (z.B. 3 oder 4) ist, -O-$(CH_2)_{n}{}^{14b}$-O-, wobei $n^{14b}$ 1 oder 2 ist; -O-$(CH_2)_{n}{}^{14c}$- oder -$S(O)_2$-$(CH_2)_{n}{}^{14c}$-, wobei $n^{14c}$ 2 oder 3 (z.B. 2) ist; -CH=CH-$NR^{15b}$-; -N=CH-$NR^{15b}$-; -CH=N-$NR^{15b}$-; -N=N-$NR^{15b}$-; NH-NH-C(O)-; -CH=CH-O-; -N=CH-O-; oder -CH=CH-S-;
mit der Maßgabe, dass:
zwei oder mehr von A, B, D, E und F unabhängig C-H (Kohlenstoff Wasserstoff), C-F (Kohlenstoff-Fluor) oder Stickstoff (N) sind;
und nicht mehr als zwei von A, B, D, E und F Stickstoff (N) sind,
und wobei in Unterformel (z) und unabhängig in den Unterformeln (y1), (y2) und (y3):

G gleich O oder S oder $NR^9$ ist, wobei $R^9$ ein Wasserstoffatom (H), $C_{1-4}$-Alkyl oder $C_{1-2}$-Fluoralkyl ist;
J gleich C-$R^{6J}$, C-[Verbindungspunkt zu Formel (I)] oder Stickstoff (N) ist,
L gleich C-$R^{6L}$, C-[Verbindungspunkt zu Formel (I)] oder Stickstoff (N) ist,
M gleich C-$R^{6M}$, C-[Verbindungspunkt zu Formel (I)] oder Stickstoff (N) ist,
Q gleich C-$R^{6Q}$, C-[Verbindungspunkt zu Formel (I)] oder Stickstoff (N) ist,
$J^Y$ gleich C-$R^{6J}$ oder Stickstoff (N) ist und
$L^Y$ gleich C-$R^{6L}$ oder Stickstoff (N) ist,

wobei $R^{6J}$, $R^{6L}$, $R^{6M}$ und $R^{6Q}$ unabhängig folgendes sind: ein Wasserstoffatom (H), ein Halogenatom; $C_{1-4}$-Alkyl (z.B. $C_{1-2}$-Alkyl); $C_{1-3}$-Fluoralkyl (z.B. $C_{1-2}$-Fluoralkyl); $C_{3-6}$-Cycloalkyl; $C_{1-4}$-Alkoxy (z.B. $C_{1-2}$-Alkoxy); $C_{1-2}$-Fluoralkoxy; $C_{3-6}$-Cycloalkyloxy; -$CH_2$-OH; -$CH_2$-OMe; OH (einschließlich all seiner Tautomere); 2-Pyridinyl; 3-Pyridinyl; 4-Pyridinyl; oder Phenyl, gegebenenfalls substituiert mit einem oder zwei Substituenten, die unabhängig Fluor, Chlor, $C_{1-2}$-Alkyl, $C_1$-Fluoralkyl, $C_{1-2}$-Alkoxy oder $C_1$-Fluoralkoxy sind;
mit der Maßgabe, dass:

zwei oder mehr von J, L, M und Q unabhängig C-H, C-F, C-$C_{1-2}$-Alkyl (z.B. C-Me), C-$C_1$-Fluoralkyl (z.B. C-$CF_3$), C-[Verbindungspunkt zu Formel (I)] oder Stickstoff (N) sind;
und nicht mehr als drei von J, L, M und Q Stickstoff (N) sind;
und einer oder beide von $J^Y$ und $L^Y$ unabhängig C-H, C-F, C-$C_{1-2}$-Alkyl (z.B. C-Me), C-$C_1$-Fluoralkyl (z.B. C- $CF_3$) oder Stickstoff (N) sind;

und wobei:

$NR^{5a}R^{5b}$ ein 1-Pyrrolidinyl-, 1-Piperidinyl- oder N-Morpholino- (4-Morpholinyl-)Rest ist;

$R^7$ und $R^8$ unabhängig ein Wasserstoffatom (H); $C_{1-4}$-Alkyl (z.B. $C_{1-2}$-Alkyl, wie z.B. Methyl); $C_{3-6}$-Cycloalkyl; ($C_{3-6}$-Cycloalkyl)methyl-; oder Phenyl, gegebenenfalls substituiert mit einem oder zwei Substituenten, bei denen es sich unabhängig um: Fluor, Chlor, $C_{1-2}$-Alkyl, $C_1$-Fluoralkyl, $C_{1-2}$-Alkoxy oder $C_1$-Fluoralkoxy handelt, sind;
oder $R^7$ und $R^8$ zusammen -$(CH_2)_n{}^6$- oder -C(O)-$(CH_2)_n{}^7$- oder -C(O)-$(CH_2)_n{}^{10}$-C(O)-oder -$(CH_2)_2$-$X^7$-$(CH_2)_2$- oder -C(O)-$X^7$-$(CH_2)_n{}^{10}$- sind, wobei: $n^6$ 3, 4, 5, 6 oder 7 ist, $n^7$ 3, 4 oder 5 ist, $n^{10}$ 2 oder 3 ist und $X^7$ gleich O oder $NR^{14}$ ist;
$R^{12}$ und $R^{13}$ unabhängig H; $C_{1-4}$-Alkyl (z.B. $C_{1-2}$-Alkyl); $C_{3-6}$-Cycloalkyl; ($C_{3-6}$-Cycloalkyl)methyl-; oder Phenyl, gegebenenfalls substituiert mit einem oder zwei Substituenten, die unabhängig folgende sind: Fluor, Chlor, $C_{1-2}$-Alkyl, $C_1$-Fluoralkyl, $C_{1-2}$-Alkoxy oder $C_1$-Fluoralkoxy, sind;
oder $R^{12}$ und $R^{13}$ zusammen -$(CH_2)_n{}^{6a}$- oder oder -$(CH_2)_2$-$X^{12}$-$(CH_2)_2$- sind, wobei: $n^{6a}$ 3, 4, 5, 6 oder 7 ist und $X^{12}$ gleich O oder $NR^{14}$ ist;
$R^{14}$, unabhängig von anderen $R^{14}$, folgendes ist: ein Wasserstoffatom (H); $C_{1-4}$-Alkyl (z.B. $C_{1-2}$-Alkyl); $C_{1-2}$-Fluoralkyl (z.B. $CF_3$); Cyclopropyl; -C(O)-$C_{1-4}$-Alkyl (z.B. -C(O)Me); -C(O)$NR^{7a}R^{8a}$ (z.B. -C(O)$NH_2$); oder -$S(O)_2$-$C_{1-4}$-Alkyl (z.B. -$S(O)_2$Me);
$R^{7a}$ ein Wasserstoffatom (H) oder $C_{1-4}$-Alkyl ist;
$R^{8a}$ ein Wasserstoffatom (H) oder Methyl ist;
$R^{15}$, unabhängig von anderen $R^{15}$, ein Wasserstoffatom (H) oder $C_{1-4}$-Alkyl ist;
$R^{15b}$, unabhängig von anderen $R^{15b}$, H oder $C_{1-2}$-Alkyl ist;
$R^{16}$, unabhängig von anderen $R^{16}$, folgendes ist:

$C_{1-6}$-Alkyl (z.B. $C_{1-4}$-Alkyl oder $C_{1-2}$-Alkyl);
$C_{3-6}$-Cycloalkyl (z.B. $C_{5-6}$-Cycloalkyl);
$C_{3-6}$-Cycloalkyl-$CH_2$- (z.B. $C_{5-6}$-Cycloalkyl-$CH_2$-);
Pyridinyl (z.B. Pyridin-2-yl), gegebenenfalls substituiert an einem Ring-Kohlenstoffatom mit einem von: einem Halogenatom, $C_{1-2}$-Alkyl, $C_1$-Fluoralkyl, $C_{1-2}$-Alkoxy oder $C_1$-Fluoralkoxy;
$Ar^{16}$;
Phenyl, gegebenenfalls substituiert mit einem oder zwei Substituenten, die unabhängig folgende sind: ein Halogenatom; $C_{1-2}$-Alkyl, $C_1$-Fluoralkyl, $C_{1-2}$-Alkoxy oder $C_1$-Fluoralkoxy;
Benzyl, gegebenenfalls substituiert an seinem Ring mit einem oder zwei Substituenten, die unabhängig folgende sind: ein Halogenatom, $C_{1-2}$-Alkyl, $C_1$-Fluoralkyl, $C_{1-2}$-Alkoxy oder $C_1$-Fluoralkoxy; oder
ein 4-, 5-, 6- oder 7-gliedriger gesättigter heterocyclischer Ring, der an einem Ring-Kohlenstoff verknüpft ist und ein oder zwei Ring-Heteroatome, unabhängig ausgewählt aus O, S und N, enthält; wobei alle Ring-Stickstoffe, die vorhanden sind, als $NR^{27}$ vorliegen, wobei $R^{27}$ H, $C_{1-2}$-Alkyl oder -C(O)Me ist; und wobei

der Ring gegebenenfalls am Kohlenstoff mit einem $C_{1-2}$-Alkyl- oder Oxo- (=O)-Substituenten substituiert ist, mit der Maßgabe, dass jeder Oxo- (=O)-Substituent an einem Ring-Kohlenstoffatom, das an einen Ring-Stickstoff gebunden ist, substituiert ist;

$R^{17}$, unabhängig von anderen $R^{17}$, folgendes ist:

$C_{5-6}$-Cycloalkyl; -C(O)-$C_{1-4}$-Alkyl (z.B. -C(O)-$C_{3-4}$-Alkyl); -C(O)-$C_{3-6}$-Cycloalkyl (z.B. -C(O)-Cyclopropyl); -$S(O)_2$-$C_{1-4}$-Alkyl (z.B. -$S(O)_2$Me oder -$S(O)_2$Et); -C(O)-$Ar^{176}$; -C(O)-$Ar^{175}$; -C(O)-$Het^1$; -$CH_2$-C(O)-$Het^1$; -$CH_2$-C(O)-(Pyrrolidin-1-yl); -$S(O)_2$-$Ar^{176}$; -$S(O)_2$-$Ar^{175}$; $Ar^{176}$; $Ar^{175}$;
-C(O)-Phenyl oder -$S(O)_2$-Phenyl, wobei, unabhängig, der Phenylring unsubstituiert oder mit einem oder zwei (z.B. einem) Substituenten substituiert ist, die unabhängig Methyl oder Ethyl oder $CF_3$ oder Fluor oder Chlor oder Methoxy oder Difluormethoxy oder Cyano (-CN) oder -C(O)$NH_2$ oder -C(O)-Me sind;
Phenyl; Phenyl, substituiert mit einem oder zwei (z.B. einem) Substituenten, die unabhängig Methyl oder Ethyl oder $CF_3$ oder Fluor oder Chlor oder Methoxy oder Difluormethoxy oder Cyano (-CN) oder -C(O) $NR^{7a}R^{8a}$ (z.B. -C(O)$NH_2$) oder -C(O)-Me sind;
oder Benzyl oder -CH(Me)-Phenyl, wobei, unabhängig, der Phenylring unsubstituiert oder mit einem oder zwei (z.B. einem) Substituenten, die unabhängig Methyl oder Fluor oder Chlor oder Methoxy sind, substituiert ist;

wobei $Ar^{175}$ ein 5-gliedriger heteroaromatischer Ring ist, bei dem es sich um Oxazolyl, Isoxazolyl, Thiazolyl, 1,2,4-Oxadiazolyl, 1,3,4-Oxadiazolyl, Furanyl, Thienyl, Pyrrolyl, Pyrazolyl, Imidazolyl, Triazolyl oder Tetrazolyl handelt,
wobei der 5-gliedrige heteroaromatische Ring $Ar^{175}$ gegebenenfalls an einem Ring-Kohlenstoff mit einem Substituenten substituiert ist, bei dem es sich um Methyl oder Ethyl oder Isopropyl oder t-Butyl oder Phenyl oder Pyridinyl handelt, und/oder gegebenenfalls an einem Ring-Stickstoff mit einem Substituenten substituiert ist, bei dem es sich um Methyl oder Ethyl oder Isopropyl oder t-Butyl oder Phenyl handelt;
oder wobei der 5-gliedrige heteroaromatische Ring $Ar^{175}$ gegebenenfalls an einen Phenylring kondensiert ist, wobei der Verbindungspunkt zu dem Rest des Moleküls innerhalb des 5-gliedrigen Rings liegt;
und wobei $Ar^{176}$ ein 6-gliedriger heteroaromatischer Ring ist, bei dem es sich um Pyridinyl, Pyrimidinyl, Pyrazinyl oder Pyridazinyl handelt, wobei der 6-gliedrige heteroaromatische Ring gegebenenfalls an einem Ring-Kohlenstoff entweder mit einem Methyl-Substituenten oder mit einem OH-Substituenten, der an einem Ring-Kohlenstoff substituiert ist, der an einen Ring-Stickstoff (einschließlich dessen Keto-Tautomer) gebunden ist, substituiert ist;
$R^{30}$, unabhängig von anderen $R^{30}$, ein Wasserstoffatom (H), $C_{1-4}$-Alkyl oder $C_{3-6}$-Cycloalkyl ist,
$Ar^6$ und $Ar^{16}$ unabhängig ein 5-gliedriger aromatischer heterocyclischer Ring ist/sind, der an einem Ring-Kohlenstoff verknüpft ist und ein O, S oder $NR^{15}$ in dem 5-gliedrigen Ring enthält, wobei der 5-gliedrige Ring gegebenenfalls zusätzlich ein oder zwei N-Atome enthalten kann und wobei der heterocyclische Ring gegebenenfalls an einem Ring-Kohlenstoffatom substituiert ist mit einem aus: einem Fluoratom, einem Chloratom, $C_{1-2}$-Alkyl, $C_1$-Fluoralkyl, -$CH_2$OH, -$CH_2$-OMe oder OH (einschließlich all seiner Keto-Tautomere);
Het, unabhängig von anderen Het, ein 4-, 5-, 6- oder 7-gliedriger gesättigter heterocyclischer Ring ist, der an einem Ring-Kohlenstoff verknüpft ist und ein oder zwei Ring-Heteroatome enthält, die unabhängig aus O, S und N ausgewählt sind; wobei alle Ring-Stickstoffe, die vorhanden sind, als $NR^{31}$ vorliegen, wobei $R^{31}$ H, $C_{1-4}$-Alkyl, -C(O)-$C_{1-4}$-Alkyl oder -$S(O)_2$-$C_{1-4}$-Alkyl ist; und wobei der Het-Ring gegebenenfalls an einem Ring-Kohlenstoff mit einem Methyl- oder Oxo- (=O)-Substituenten substituiert ist, mit der Maßgabe, dass jeder Oxo- (=O)-Substituent an einem Ring-Kohlenstoffatom, das an einen Ring-Stickstoff gebunden ist, substituiert ist, wobei $R^{31}$ H oder $C_{1-4}$-Alkyl ist; und
$Het^1$ unabhängig von anderen $Het^1$, ein 4-, 5-, 6- oder 7-gliedriger gesättigter heterocyclischer Ring ist, der an einem Ring-Kohlenstoff verknüpft ist und ein oder zwei Ring-Heteroatome enthält, die unabhängig aus O, S und N ausgewählt sind; wobei alle Ring-Stickstoffe, die vorhanden sind, als $NR^{31a}$ vorliegen, wobei $R^{31a}$ H, $C_{1-2}$-Alkyl, -C(O)Me oder -$S(O)_2$Me ist; und wobei der $Het^1$-Ring gegebenenfalls an einem Ring-Kohlenstoff mit einem Methyl- oder Oxo- (=O)-Substituenten substituiert ist, mit der Maßgabe, dass jeder Oxo- (=O)-Substituent an einem Ring-Kohlenstoffatom, das an einen Ring-Stickstoff gebunden ist, substituiert ist, wobei $R^{31a}$ gleich H oder $C_{1-2}$-Alkyl ist.

**2.** Eine Verbindung oder ein Salz wie in Anspruch 1 beansprucht, wobei $R^1$ Ethyl ist.

**3.** Eine Verbindung oder ein Salz wie in Anspruch 1 oder 2 beansprucht, wobei $R^2$ Methyl oder Ethyl ist.

4. Eine Verbindung oder ein Salz wie in einem vorhergehenden Anspruch beansprucht, wobei $R^3$ das gegebenenfalls substituierte $C_{4-7}$-Cycloalkyl oder der gegebenenfalls substituierte heterocyclische Rest der Unterformel (aa), (bb) oder (cc) ist.

5. Eine Verbindung oder ein Salz wie in einem vorhergehenden Anspruch beansprucht, wobei, wenn $R^3$ der heterocyclische Rest der Unterformel (aa), (bb) oder (cc) ist, dann Y gleich O oder $NR^{10}$ ist.

6. Eine Verbindung oder ein Salz wie in einem vorhergehenden Anspruch beansprucht, wobei, wenn $R^3$ der heterocyclische Rest der Unterformel (aa), (bb) oder (cc) ist, dann $R^3$ der heterocyclische Rest der Unterformel (bb) ist und $n^1$ gleich 1 ist.

7. Eine Verbindung oder ein Salz wie in einem vorhergehenden Anspruch beansprucht, wobei in $R^3$ der heterocyclische Rest der Unterformel (aa), (bb) oder (cc) nicht an einem Ring-Kohlenstoff substituiert ist.

8. Eine Verbindung oder ein Salz wie in einem vorhergehenden Anspruch beansprucht, wobei $R^3$ Tetrahydro-2H-pyran-4-yl oder 1-(Aminocarbonyl)-4-piperidinyl ist; d.h. $NHR^3$ die Unterformel (h) oder (k2) hat

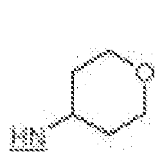
(h)

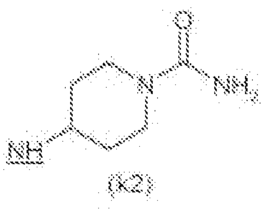
(k2)

.

9. Eine Verbindung oder ein Salz davon wie in einem vorhergehenden Anspruch beansprucht, wobei $R^a$ ein Wasserstoffatom (H) ist und $R^b$ ein Wasserstoffatom (H) ist.

10. Eine Verbindung oder ein Salz davon wie in einem vorhergehenden Anspruch beansprucht, wobei $R^4$ ein Wasserstoffatom (H) ist.

11. Eine Verbindung oder ein Salz davon wie in einem vorhergehenden Anspruch beansprucht, wobei $R^5$ folgendes ist:

$-C(O)-(CH_2)n-Ar$, -C(O)-Het, $-C(O)-NR^{15b}-(CH_2)_m{}^1-Ar$ oder $-C(O)-NR^{5a}R^{5b}$;
und $R^4$ ein Wasserstoffatom (H) ist
**oder** $R^4$ und $R^5$ zusammengenommen $-C(O)-N(R^{15})-(CH_2)_p{}^3-$ sind;
**oder** $NR^4R^5$ die Unterformel (y), (y1), (y2) oder (y3) hat.

12. N-((1-Ethyl-6-methyl-4-((tetrahydro-2H-pyran-4-yl)amino)-1H-pyrazolo[3,4-b]pyridin-5-yl)methyl)-5-methylpyrazin-2-carboxamid

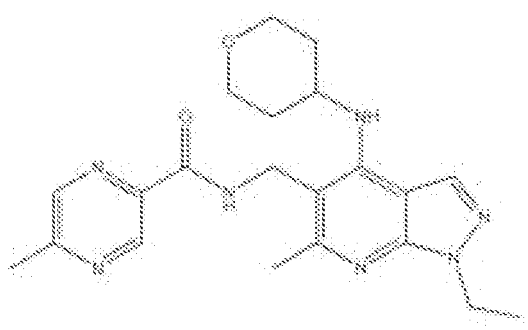

oder ein Salz davon.

13. Eine Verbindung oder ein Salz wie in einem vorhergehenden Anspruch beansprucht zur Verwendung als ein therapeutischer Wirkstoff bei einem Säuger.

**14.** Ein Arzneimittel, umfassend eine Verbindung der Formel (I) wie in einem der Ansprüche 1 bis 12 definiert oder ein pharmazeutisch verträgliches Salz davon und einen oder mehrere pharmazeutisch verträgliche Träger und/oder Exzipienten.

**15.** Ein Arzneimittel wie in Anspruch 14 beansprucht zur Verwendung bei der Behandlung und/oder Prophylaxe einer entzündlichen und/oder allergischen Erkrankung, kognitiven Beeinträchtigung oder Depression bei einem Säuger.

**16.** Ein Arzneimittel wie in Anspruch 15 beansprucht, wobei die kognitive Beeinträchtigung in einer neurologischen Störung vorliegt.

**17.** Ein Arzneimittel wie in Anspruch 16 beansprucht, wobei die neurologische Störung aus Alzheimer-Krankheit oder Schizophrenie ausgewählt ist.

**18.** Die Verwendung einer Verbindung der Formel (I) wie in einem der Ansprüche 1 bis 12 definiert oder eines pharmazeutisch verträglichen Salzes davon bei der Herstellung eines Medikaments zur Behandlung und/oder Prophylaxe von Asthma, chronisch obstruktiver Lungenerkrankung (COPD), atopischer Dermatitis, Urtikaria, allergischer Rhinitis, allergischer Konjunktivitis, Frühlingskonjunktivitis, eosinophilem Granulom, Psoriasis, rheumatoider Arthritis, septischem Schock, Colitis ulcerosa, Morbus Crohn, Reperfusionsverletzung des Myokards und Gehirns, chronischer Glomerulonephritis, endotoxischem Schock, Schocklunge, Multipler Sklerose, kognitiver Beeinträchtigung, Depression oder Entzündungschmerz bei einem Säuger.

**19.** Die Verwendung einer Verbindung wie in Anspruch 18 beansprucht, wobei die kognitive Beeinträchtigung in einer neurologischen Störung vorliegt.

**20.** Die Verwendung einer Verbindung wie in Anspruch 19 beansprucht, wobei die neurologische Störung aus Alzheimer-Krankheit oder Schizophrenie ausgewählt ist.

**21.** Eine Verbindung wie in einem der Ansprüche 1 bis 12 beansprucht zur Verwendung bei der Behandlung und/oder Prophylaxe von Asthma, chronisch obstruktiver Lungenerkrankung (COPD), atopischer Dermatitis, Urtikaria, allergischer Rhinitis, allergischer Konjunktivitis, Frühlingskonjunktivitis, eosinophilem Granulom, Psoriasis, rheumatoider Arthritis, septischem Schock, Colitis ulcerosa, Morbus Crohn, Reperfusionsverletzung des Myokards und Gehirns, chronischer Glomerulonephritis, endotoxischem Schock, Schocklunge, Multipler Sklerose, kognitiver Beeinträchtigung, Depression oder Entzündungsschmerz bei einem Säuger.

**22.** Eine Verbindung wie in Anspruch 21 beansprucht, wobei die kognitive Beeinträchtigung in einer neurologischen Störung vorliegt.

**23.** Eine Verbindung wie in Anspruch 21 beansprucht, wobei die neurologische Störung aus Alzheimer-Krankheit oder Schizophrenie ausgewählt ist.

## Revendications

**1.** Composé de formule (I) ou un de ses sels (en particulier, un de ses sels pharmaceutiquement acceptables) :

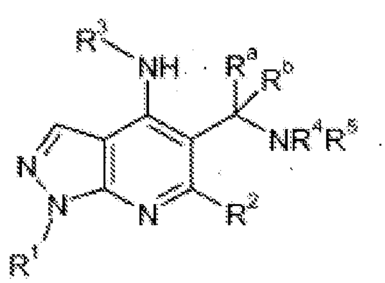

(I)

formule dans laquelle :

$R^1$ représente un groupe alkyle en $C_1$ à $C_3$, fluoralkyle en $C_1$ à $C_3$ ou $-CH_2CH_2OH$ ;

$R^2$ représente un atome d'hydrogène (H), un groupe méthyle, éthyle, n-propyle, isopropyle, n-butyle, fluoralkyle en $C_1$ ou $C_2$, cyclopropyle, cyclobutyle, (cyclopropyl)méthyle-, cyano(-CN), ou $-CH_2OH$ ;
$R^3$ représente un groupe cycloalkyle en $C_4$ à $C_7$ facultativement substitué ou cycloalcényle en $C_5$ à $C_7$ mono-insaturé facultativement substitué ou bien un groupe hétérocyclique, facultativement substitué, de sous-formule (aa), (bb) ou (cc) :

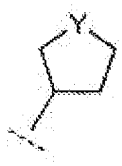
ou
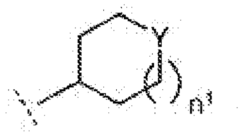
ou
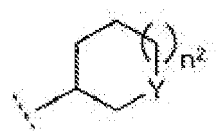

(aa) (bb) (cc)

dans lesquelles $n^1$ et $n^2$ sont égaux indépendamment à 1 ou 2 ; et dans lesquelles Y représente 0, S, un groupe $SO_2$ ou $NR^{10}$ ; dans lequel $R^{10}$ représente un atome d'hydrogène (H), un groupe méthyle, C (O) $NH_2$, C (O)-méthyle ou C (O) - (fluoralkyle en $C_1$) ;
ou bien $R^3$ représente un groupe bicyclique de sous-formule (ee) :

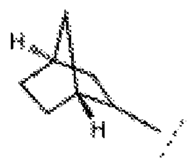

(ee) ;

et dans laquelle, lorsque $R^3$ représente un groupe cycloalkyle en $C_4$ à $C_7$ facultativement substitué, alors $R^3$ représente un groupe cycloalkyle en $C_4$ à $C_7$ facultativement substitué sur un atome du carbone du noyau avec un ou deux substituants consistant indépendamment en des substituants : oxo (=O) ; OH ; méthoxy ; fluoralkoxy en $C_1$ ; $NH_2$ ; alkyle en $C_1$ ou $C_2$ ; fluoralkyle en $C_1$, $-CH_2$-OH ; -CH (Me) OH ; $-CH_2CH_2OH$ ; $-CH_2NH_2$ ; -C (O) OH ; $-C(O)NHR^{24}$ dans lequel $R^{24}$ représente H ou un groupe méthyle, $-C(O)R^{25}$ dans lequel $R^{25}$ représente un groupe méthyle ; fluoro ; hydroxyimino (=N-OH) ; ou (alkoxy en $C_1$ ou $C_2$) imino ($=N-OR^{26}$ dans lequel $R^{26}$ représente un groupe alkyle en $C_1$ ou $C_2$) ; et dans lesquels n'importe quel substituant OH, méthoxy, fluoralkoxy ou $NH_2$ n'est pas substitué à l'atome de carbone de noyau $R^3$ fixé (lié) au groupe -NH- de la formule (I) ;
et dans laquelle, lorsque $R^3$ représente le groupe hétérocyclique, facultativement substitué, de sous-formule (aa), (bb) ou (cc), alors $R^3$ représente le groupe hétérocyclique de sous-forme (aa), (bb) ou (cc) facultativement substitué sur un atome de carbone de noyau avec un ou deux substituants consistant indépendamment en des substituants oxo (=O), OH ou méthyle ; et dans laquelle n'importe quel substituant OH n'est pas substitué au niveau de l'atome de carbone de noyau $R^3$ fixé (lié) au groupe -NH- de la formule (I) est n'est pas substitué à l'un ou l'autre atome de carbone de noyau $R^3$ lié au groupe Y du groupe hétérocyclique (aa), (bb) ou (cc) ;
et dans laquelle, lorsque $R^3$ représente un groupe cycloalcényle en $C_5$ à $C_7$ mono-insaturé facultativement substitué, alors le groupe cycloalcényle est facultativement substitué sur un atome de carbone de noyau avec un substituant consistant en un substituant fluoro ou méthyle, et l'atome de carbone de noyau $R^3$ lié au groupe -NH- de la formule (I) ne participe à la double liaison du groupe cycloalcényle ;
sous réserve que :

lorsque $R^3$ représente le groupe hétérocyclique de sous-formule (aa) et Y représente un groupe $NR^{10}$, alors $R^{10}$ ne représente pas un groupe C(O)méthyle ou C(O)-(fluoralkyle en $C_1$) ; et
lorsque $R^3$ représente le groupe hétérocyclique de sous-formule (bb) et Y représente un groupe $NR^{10}$, alors $R^{10}$ ne représente pas un groupe méthyle ; et
lorsque $R^3$ représente le groupe hétérocyclique de sous-formule (cc) et Y représente O, S, un groupe $SO_2$ ou $NR^{10}$ dans lequel $R^{10}$ représente H ou un groupe méthyle ;

et dans laquelle :

lorsque $R^3$ représente un groupe cycloalkyle en $C_4$ à $C_7$ facultativement substitué, alors n'importe quel substituant -C(O)NH$R^{24}$ ou C(O)$R^{25}$ sur un atome de carbone de noyau est : en position 3 d'un noyau cyclobutyle $R^3$ ; ou en position 3 ou 4 d'un noyau cyclopentyle $R^3$ ; ou en position 4 d'un noyau cyclohexyle $R^3$ ; ou en position 3, 4,5 ou 6 d'un noyau cycloheptyle $R^3$ (où, à cet égard, la position 1 du noyau cycloalkyle $R^3$ est considérée comme étant le point de connexion au groupe -NH- dans la formule (I), c'est-à-dire l'atome de noyau de connexion au groupe -NH- dans la formule (I)) ;

et dans laquelle :

lorsque $R^3$ représente un groupe cycloalkyle en $C_4$ à $C_7$ facultativement substitué, alors n'importe quel substituant OH, méthoxy, fluoralkoxy, -$CH_2OH$, -CH (Me) OH, -$CH_2CH_2OH$, -$CH_2NH_2$ ou -C(O)OH sur un atome de carbone du noyau est : en position 3 d'un noyau cyclobutyle $R^3$ ; ou en position 3 ou 4 d'un noyau cyclopentyle $R^3$; ou en position 3, 4 ou 5 d'un noyau cyclohexyle $R^3$ ; ou en position 3, 4, 5 ou 6 d'un noyau cycloheptyle $R^3$ ;

et dans laquelle :

lorsque $R^3$ représente le groupe hétérocyclique de sous-formule (aa), (bb) ou (cc), alors n'importe quel substituant OH sur un atome de carbone de noyau est : en position 5 d'un groupe hétérocyclique $R^3$ hexagonal de sous-formule (cc) dans laquelle $n^2$ est égal à 1 ; ou en position 5 ou 6 d'un groupe hétérocyclique $R^3$ heptagonal de sous-formule (cc) dans laquelle $n^2$ est égal à 2 ; ou en position 6 d'un groupe hétérocyclique $R^3$ heptagonal de sous-formule (bb) dans laquelle $n^1$ est égal à 2 (où, à cet égard, la position 1 du noyau hétérocyclique $R^3$ est considérée comme étant le point de connexion au groupe -NH- dans la formule (I), c'est-à-dire l'atome de carbone de noyau de connexion au groupe -NH-dans la formule (I), et les positions restantes du noyau sont alors numérotées de telle sorte que l'hétéroatome du noyau ait le plus petit numéro possible) ;

et dans laquelle :

$R^a$ représente un atome d'hydrogène (H), un groupe m éthyle ou éthyle ;
$R^b$ représente un atome d'hydrogène (H) ou un groupe méthyle ;
où, lorsque $R^b$ représente un groupe méthyle, alors $R^a$ représente un groupe méthyle et $R^2$ représente un atome d'hydrogène (H) ;

$R^4$ représente un atome d'hydrogène (H), un groupe méthyle, éthyle, n-propyle, -C(O)-Me, ou -C(O)-(fluoralkyle en $C_1$) ;
sous réserve que, lorsque $R^4$ représente un groupe -C(O)-Me ou -C(O)-(fluoralkyle en $C_1$), alors $R^5$ représente un groupe -$CH_2$-Ar ;
et
$R^5$ représente un groupe :

-C(O)- $(CH_2)_n$-Ar, -C(O)-Het, -C(O)- (alkyle en $C_1$ à $C_6$), -C(O)-(fluoralkyle en $C_1$), -C(O)-$(CH_2)_2$-C (O)-N$R^{15b}$N$R^{15b}$, -C (O) - $CH_2$-C(O) -N$R^{15b}$N$R^{15b}$, -C(O)-N$R^{15b}$- $(CH_2)_{m^1}$-Ar, -C(O)-N$R^{15b}$-Het,- C (O)-N$R^{15b}$-(alkyle en $C_1$ à $C_6$), -C(O)-N$R^{5a}R^{5b}$, -$S(O)_2$-$(CH_2)$m$^2$-Ar, -$S(O)_2$-Het, -$S(O)_2$-(alkyle en $C_1$ à $C_6$) ou -$CH_2$-Ar ;
où n et $m^1$ et $m^2$ sont égaux indépendamment à 0, 1 ou 2 ;
et
Ar, indépendant d'un autre groupe Ar, répond à la sous-formule (x) ou (z), où (z) est connecté au niveau d'un atome de carbone de noyau :

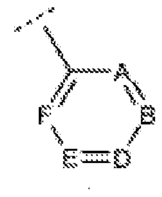

(x)

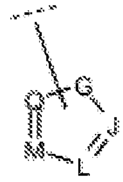

(z)

ou $R^4$ et $R^5$, pris conjointement, représentent un groupe - $(CH_2)_p{}^1$- (facultativement substitué), ou -$(CH_2)_2$-$X^5$-$(CH_2)_2$-, ou -C(O)-$(CH_2)_p{}^2$-, ou -C(O)-N($R^{15}$)-$(CH_2)_p{}^3$-, dans lesquels : $X^5$ représente un groupe $NR^{17}$ et $p^1$ est égal à 4, 5 ou 6 et $p^2$ est égal à 3, 4 ou 5 et $p^3$ est égal à 2 ou 3 ;
ou $NR^4R^5$ répond à la sous-formule (y), (y1), (y2) ou (y3) dans laquelle $p^4$ est égal 1 ou 2 ;

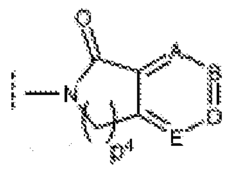

(y)

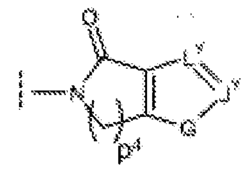

(y1)

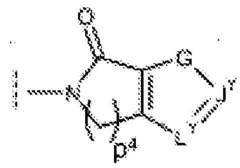

(y2)

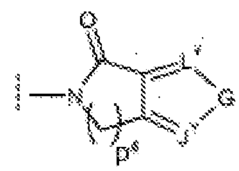

(y3)

et dans laquelle, lorsque $R^4$ et $R^5$, pris conjointement, représentent un groupe -$(CH_2)_p{}^1$-, alors le noyau $NR^4R^5$ est facultativement substitué sur l'atome de carbone de noyau en position 3 et/ou sur l'atome de carbone de noyau en position 4 (où l'atome d'azote du noyau représente la position 1) avec un ou deux substituants consistant indépendamment en des substituants :
phényle ; phényle substitué avec un ou deux (par exemple un) substituants consistant indépendamment en des substituants méthyle ou $CF_3$ ou fluoro ou chloro ou méthoxy ou difluorométhoxy ;
phényl-C(O)- ; phényl-C(O), dont le noyau phényle est substitué avec un ou deux (par exemple un) substituants consistant indépendamment en des substituants méthyle ou fluoro ou chloro ou méthoxy ;
benzyloxy ; phényloxy ; phényloxy dont le noyau phényle est substitué avec un ou deux (par exemple un) substituants consistant indépendamment en des substituants méthyle ou $CF_3$ ou fluoro ou chloro ou méthoxy ou difluorométhoxy ;
un noyau hétéroaromatique pentagonal consistant en un noyau oxazolyle, isoxazolyle, thiazolyle, 1,2,4-oxadiazolyle, 1,3,4-oxadiazolyle, furannyle, thiényle, pyrrolyle pyrazolyle, imidazolyle, triazolyle ou tétrazolyle, ledit noyau hétéroaromatique pentagonal étant facultativement substitué sur un atome de carbone de noyau avec un substituant consistant en un substituant méthyle, éthyle, isopropyle, tertiobutyle, phényle, pyridinyle et/ou étant facultativement substitué sur un atome d'azote de noyau avec un substituant consistant en un substituant méthyle, éthyle, isopropyle, tertiobutyle ou phényle ;
-$CH_2$- (pyrrol-1-yle) ;
un noyau hétéroaromatique hexagonal consistant en un noyau pyridinyle, pyrimidinyle, pyrazinyle ou pyridazinyle, ledit noyau hétéroaromatique hexagonal étant facultativement substitué sur un atome de carbone du noyau, soit avec un substituant méthyle, soit avec un substituant OH qui est substitué sur un atome de carbone de noyau lié à un atome d'azote de noyau (y compris sa forme tautomère céto) ;
alkyle en $C_1$ ou $C_2$ (par exemple méthyle) ;
cyano (-CN) ;
ou (4-hydroxyphényl)-C(O)-$CH_2$-$CH_2$- ;
ou, en variante, lorsque $R^4$ et $R^5$, pris conjointement, représentent un groupe - $(CH_2)$ $p^1$- et $p^1$ est égal à 5, alors le noyau $NR^4R^5$ est facultativement substitué sur l'atome de carbone de noyau en position 4 avec deux substituants qui, lorsqu'ils sont pris conjointement, représentent un groupe -O-C(O)-N(benzyl)-$CH_2$- ;
ou, en variante, lorsque $R^4$ et $R^5$, pris conjointement, représentent un groupe -$(CH_2)p^1$- et $p^1$ est égal à 4 ou 5, alors le noyau $NR^4R^5$ est facultativement substitué sur les atomes de carbone de noyau en position 3 et en position 4 avec deux substituants qui, lorsqu'ils sont pris conjointement, représentent un groupe =CH-CH=CH-CH= ;

et dans laquelle, dans la sous-formule (x), et indépendamment dans la sous-formule (y) :

A représente un groupe C-$R^{6A}$ ou un atome d'azote (N),
B représente un groupe C-$R^{6B}$ ou un atome d'azote (N),
D représente un groupe C-$R^{6D}$ ou un atome d'azote (N),
E représente un groupe C-$R^{6E}$ ou un atome d'azote (N),
F représente un groupe C-$R^{6F}$ ou un atome d'azote (N),
où $R^{6A}$, $R^{6B}$, $R^{6D}$, $R^{6E}$ et $R^{6F}$, indépendamment, représentent : un atome d'hydrogène (H), un atome d'halogène ; un groupe alkyle en $C_1$ à $C_6$ (par exemple alkyle en $C_1$ à $C_4$ ou alkyle en $C_1$ ou $C_2$) ; fluoralkyle en $C_1$ à $C_4$ (par exemple fluoralkyle en $C_1$ ou $C_2$) ; cycloalkyle en $C_3$ à $C_6$ ; alkoxy en $C_1$ à $C_4$ (par exemple alkoxy en $C_1$ ou $C_2$) ; fluoralkoxy en $C_1$ ou $C_2$ ; cycloalkyloxy en $C_3$ à $C_6$ ; -C(O)$R^{16}$ ; -C(O)$OR^{30}$ ; -S$(O)_2$-$R^{16}$ (par exemple (alkyle en $C_1$ à $C_3$)-S$(O)_2$-) ; $R^{16}$-S$(O)_2$-$NR^{15}$-(par exemple (alkyle en $C_1$ à $C_3$)-S$(O)_2$-NH) ; $R^{12}R^{13}$N-S$(O)_2$- ; (alkyle en $C_1$ ou $C_2$)-C(O)-$R^{15}$N-S$(O)_2$ ; (alkyle en $C_1$ à $C_4$) - S(O) -, Ph-S(O)-, $R^{12}R1^{3}$N-C(O)- ; -$NR^{15}$-C(O)$R^{16}$ ; $R^7R^8$N ; nitro (-$NO_2$) ; OH (y compris n'importe quelle forme tautomère de celui-ci) ; (alkoxy en $C_1$ à $C_4$)méthyle ; (alkoxy en $C_1$ à $C_4$)-éthyle ; (alkyle en $C_1$ ou $C_2$)-S(O) $_2$-$CH_2$- ; $R^{12}R^{14}$N-S $(O)_2$-$CH_2$- ; (alkyle en $C_1$ ou $C_2$)-S$(O)_2$-$NR^{15}$-$CH_2$- ; -$CH_2$-OH ; -$CH_2CH_2$-OH ; -$CH_2$-$NR^7R^8$ ; -$CH_2$-$CH_2$-$NR^7R^8$ ; -$CH_2$-C (O) $OR^{30}$ ; -$CH_2$-C (O) -$NR^{12}R^{13}$ ; -$CH_2$-$NR^{15}$-C(O)- (alkyle en $C_1$ à $C_3$) ; -$(CH_2)_n{}^{14}$-$Het^1$ dans lequel $n^{14}$ est égal à 0 ou 1 ; cyano (-CN) ; $Ar^6$ ; 1-pyrrolyle ; ou phényle, pyridinyle ou pyrimidinyle, ledit groupe phényle, pyridinyle ou pyrimidinyle étant facultativement substitué indépendamment avec un ou deux substituants consistant indépendamment en des substituants fluoro, chloro, alkyle en $C_1$ ou $C_2$, fluoralkyle en $C_1$, alkoxy en $C_1$ ou $C_2$ ou fluoralkoxy en $C_1$ ;

sous réserve que $R^{6A}$, $R^{6B}$, $R^{6D}$, $R^{6E}$ et $R^{6F}$, indépendamment, ne puissent représenter un atome de chlore, de brome ou d'iode (et facultativement ne puissent représenter un atome de fluor) lorsqu'ils sont substitués sur un atome de carbone de noyau qui est lié à un atome d'azote de noyau ;
et/ou deux groupes adjacentes choisis entre $R^{6A}$, $R^{6B}$, $R^{6D}$, $R^{6E}$ et $R^{6F}$ sont pris conjointement et représentent un groupe : -CH=CH-CH=CH-, -$(CH_2)$ $_n{}^{14a}$ dans lequel $n^{14a}$ est égal à 3, 4 ou 5 (par exemple 3 ou 4), -O-$(CH_2)_n{}^{14b}$-O- dans lequel $n^{14b}$ est égal à 1 ou 2 ; -O-$(CH_2)_n{}^{14c}$- ou -S$(O)_2$-$(CH_2)n^{14c}$-, dans lequel $n^{14c}$ est égal à 2 ou 3 (par exemple 2) ; -CH=CH-$NR^{15b}$- ; -N=CH-$NR^{15b}$- ; CH=N-$NR^{15b}$- ; -N=N-$NR^{15b}$- ; -NH-NH-C(O) - ; -CH=CH-O- ; -N=CH-O- ; ou -CH=CH-S- ;
sous réserve que :

deux ou plus de deux de A, B, D, E et F représentent indépendamment un groupe C-H (carbone-hydrogène), C-F (carbone-fluor) ou un atome d'azote (N) ;
et pas plus de deux de A, B, D, E et F ne représentent des atomes d'azote (N),

et dans laquelle, dans la sous-formule (z), et indépendamment dans les sous-formules (y1), (y2) et (y3) :

G représente 0 ou S un groupe $NR^9$ dans lequel $R^9$ représente un atome d'hydrogène (H), un groupe alkyle en $C_1$ à $C_4$ ou fluoralkyle en $C_1$ ou $C_2$ ;
J représente un groupe -$R^{6J}$, C-[point de connexion à la formule (I)] ou un atome d'azote (N),
L représente un groupe -$R^{6L}$, C-[point de connexion à la formule (I)] ou un atome d'azote (N),
M représente un groupe -$R^{6M}$, C-[point de connexion à la formule (I)] ou un atome d'azote (N),
Q représente un groupe -$R^{6Q}$, C-[point de connexion à la formule (I)] ou un atome d'azote (N),
$J^Y$ représente un groupe C-$R^{6J}$ ou un atome d'azote (N), et
$L^Y$ représente un groupe C-$R^{6L}$ ou un atome d'azote (N),

où $R^{6J}$, $R^{6L}$, $R^{6M}$ et $R^{6Q}$, indépendamment, représentent : un atome d'hydrogène (H), un atome d'halogène ; un groupe alkyle en $C_1$ à $C_4$ (par exemple alkyle en $C_1$ ou $C_2$) ; fluoralkyle en $C_1$ à $C_3$) (par exemple fluoralkyle en $C_1$ ou $C_2$) ; cycloalkyle en $C_3$ à $C_6$ ; alkoxy en $C_1$ à $C_4$ (par exemple alkoxy en $C_1$ ou $C_2$), fluoralkoxy en $C_1$ ou $C_2$, cycloalkyloxy en $C_3$ à $C_6$ ; -$CH_2$-OH ; -$CH_2$-OMe ; OH (y compris n'importe quelle forme tautomère de celui-ci) ; 2-pyridinyle ; 3-pyridinyle ; 4-pyridinyle ; ou phényle facultativement substitué avec un ou deux substituants consistant indépendamment en des substituants fluoro, chloro, alkyle en $C_1$ ou $C_2$, fluoralkyle en $C_1$, alkoxy en $C_1$ ou $C_2$ ou fluoralkoxy en $C_1$ ;
sous réserve que :

deux ou plus de deux de J, L, M et Q représentent indépendamment un groupe C-H, C-F, C-(alkyle en $C_1$ ou $C_2$) (par exemple C-Me), C- (fluoralkyle en $C_1$) (par exemple C-$CF_3$), C-[point de connexion à la formule (I)] ou un atome d'azote (N) ;
et pas plus de trois de J, L, M et Q ne représentent des atomes d'azote (N) ;

et l'un des ou les deux groupes $J^Y$ et $L^Y$ représentent indépendamment un groupe C-H, C-F, C-(alkyle en $C_1$ ou $C_2$) (par exemple C-Me), C-(fluoralkyle en $C_1$) (par exemple C-$CF_3$), ou un atome d'azote (N) ;

et dans laquelle :
$NR^{5a}R^{5b}$ représente un groupe 1-pyrrolidinyle, 1-pipéridinyle ou N-morpholino (4-morpholinyle) ;
$R^7$ et $R^8$ représentent indépendamment un atome d'hydrogène (H) ; un groupe alkyle en $C_1$ à $C_4$ (par exemple alkyle en $C_1$ à $C_2$ tel qu'un groupe méthyle) ; cycloalkyle en $C_3$ à $C_6$ ; (cycloalkyle en $C_3$ à $C_6$) méthyle- ; ou phényle facultativement substitué avec un ou deux substituants consistant indépendamment en des substituants : fluoro, chloro, alkyle en $C_1$ ou $C_2$, fluoralkyle en $C_1$, alkoxy en $C_1$ ou $C_2$ ou fluoralkoxy en $C_1$ ;
ou bien $R^7$ et $R^8$, conjointement, représentent un groupe $-(CH_2)_n{}^6$- ou $-C(O)-(CH_2)_n{}^7$ ou $-C(O)-(CH_2)_n{}^{10}-C(O)-$ ou- $(CH_2)_2-X^7-(CH_2)_2-$ ou $-C(O)-X^7-(CH_2)_n{}^{10}-$ dans lequel : $n^6$ est égal à 3, 4, 5, 6 ou 7, $n^7$ est égal à 3, 4 ou 5, $n^{10}$ est égal à 2 ou 3, et $X^7$ représente 0 ou un groupe $NR^{14}$ ;
$R^{12}$ et $R^{13}$ représentent indépendamment H ; un groupe alkyle en $C_1$ à $C_4$ (par exemple alkyle en $C_1$ ou $C_2$) ; cycloalkyle en $C_3$ à $C_6$ ; (cycloalkyle en $C_3$ à $C_6$)méthyle- ; ou phényle facultativement substitué avec un ou deux substituants consistant indépendamment en des substituants : fluoro, chloro, alkyle en $C_1$ ou $C_2$, fluoralkyle en $C_1$, alkoxy en $C_1$ ou $C_2$ ou fluoralkoxy en $C_1$ ;
ou bien $R^{12}$ et $R^{13}$, conjointement, représentent un groupe $-(CH_2)_n{}^{6a}$- ou $-(CH_2)_2-X^{12}-(CH_2)_2-$ dans lequel : $n^{6a}$ est égal à 3, 4, 5, 6 ou 7, et $X^{12}$ représente 0 ou un groupe $NR^{14}$ ;
$R^{14}$, indépendant d'un autre groupe $R^{14}$, représente : un atome d'hydrogène (H) ; un groupe alkyle en $C_1$ à $C_4$ (par exemple alkyle en $C_1$ ou $C_2$) ; fluoralkyle en $C_1$ ou $C_2$ (par exemple $CF_3$) ; cyclopropyle ; -C(O)-(alkyle en $C_1$ à $C_4$) (par exemple -C(O)Me) ; $-C(O)NR^{7a}R^{8a}$ (par exemple $-C(O)NH_2$) ; ou $-S(O)_2$-(alkyle en $C_1$ à $C_4$) (par exemple $-S(O)_2Me$) ;
$R^{7a}$ représente un atome d'hydrogène (H) ou un groupe alkyle en $C_1$ à $C_4$ ;
$R^{8a}$ représente un atome d'hydrogène (H) ou un groupe méthyle ;
$R^{15}$, indépendant d'un autre groupe $R^{15}$, représente un atome d'hydrogène (H) ou un groupe alkyle en $C_1$ à $C_4$ ;
$R^{15b}$, indépendant d'un autre groupe $R^{15b}$, représente H ou un groupe alkyle en $C_1$ ou $C_2$ ;
$R^{16}$, indépendant d'un autre groupe $R^{16}$, représente un groupe :

alkyle en $C_1$ à $C_6$ (par exemple alkyle en $C_1$ à $C_4$ ou alkyle en $C_1$ ou $C_2$) ;
cycloalkyle en $C_3$ à $C_6$ (par exemple cycloalkyle en $C_5$ ou $C_6$) ;
(cycloalkyle en $C_3$ à $C_6$) $-CH_2-$ (par exemple (cycloalkyle en $C_5$ ou $C_6$) $-CH_2-$) ;
pyridinyle (par exemple pyridine-2-yle) facultativement substitué sur un atome de carbone de noyau avec un des substituants suivants : un atome d'halogène ; un groupe alkyle en $C_1$ ou $C_2$, fluoralkyle en $C_1$, alkoxy en $C_1$ ou $C_2$ ou fluoralkoxy en $C_1$ ;
$AR^{16}$ ;
phényle facultativement substitué avec un ou deux substituants consistant indépendamment en des substituants : un atome d'halogène, un groupe alkyle en $C_1$ ou $C_2$, fluoralkyle en $C_1$, alkoxy en $C_1$ ou $C_2$ ou fluoralkoxy en $C_1$ ;
benzyle facultativement substitué sur son noyau avec un ou deux substituants consistant indépendamment en : un atome d'halogène, un groupe alkyle en $C_1$ ou $C_2$, fluoralkyle en $C_1$, alkoxy en $C_1$ ou $C_2$ ou fluoralkoxy en $C_1$ ; ou
un noyau hétérocyclique saturé tétra-, penta-, hexa-ou heptagonal connecté au niveau d'un atome de carbone de noyau et contenant un ou deux hétéroatomes de noyau choisis indépendamment entre 0, S et N ; où n'importe quels atomes d'azote du noyau qui sont présents sont présents sous forme d'un groupe $NR^{27}$ dans lequel $R^{27}$ représente H, un groupe alkyle en $C_1$ ou $C_2$ ou -C(O)Me ; et où le noyau est facultativement substitué au niveau d'un atome de carbone avec un substituant alkyle en $C_1$ ou $C_2$ ou oxo (=O), sous réserve que n'importe quel substituant oxo (=O) soit substitué au niveau d'un atome de carbone de noyau lié à un atome d'azote de noyau ;

$R^{17}$, indépendant d'un autre groupe $R^{17}$, représente un groupe :

cycloalkyle en $C_5$ ou $C_6$ ; -C(O)-(alkyle en $C_1$ à $C_4$) (par exemple -C(O)-(alkyle en $C_3$ ou $C_4$)) ; -C(O) (cycloalkyle en $C_3$ à $C_6$) (par exemple -C(O)-cyclopropyle), $-S(O)_2$-(alkyle en $C_1$ à $C_4$) (par exemple $-S(O)_2Me$ ou $-S(O)_2Et$) ;
$-C(O)-Ar^{176}$ ; $-C(O)Ar^{175}$; $-C(O)-Het^1$ ; $-CH_2-C(O)-Het^1$ ; $-CH_2$-C (O) - (pyrrolidine-1-yle) ; $-S(O)_2-Ar^{176}$ ; $-S(O)_2-Ar^{175}$ ; $Ar^{176}$ ; $Ar^{175}$ ;
-(O)-phényle ou $-S(O)_2$-phényle, dans lequel, indépendamment, le noyau phényle est non substitué ou substitué avec un ou deux (par exemple un) substituants consistant indépendamment en des substituants

méthyle ou éthyle ou $CF_3$ ou fluoro ou chloro ou méthoxy ou difluorométhoxy ou cyano (-CN) ou $-C(O)NH_2$ ou -C(O)Me ;
phényle ; phényle substitué avec un ou deux (par exemple un) substituants consistant indépendamment en des substituants méthyle, éthyle, $CF_3$, fluoro, chloro, méthoxy, difluorométhoxy, cyano (-CN) ou $-C(O)NR^{7a}R^{8a}$ (par exemple $-C(O)NH_2$) ou -C(O)-Me ;
ou benzyle ou -CH(Me)-phényle, dans lequel, indépendamment, le noyau phényle est non substitué ou substitué avec un ou deux (par exemple un) substituants consistant indépendamment en des substituants méthyle, fluoro, chloro ou méthoxy ;
où $Ar^{175}$ représente un noyau hétéroaromatique pentagonal consistant en un noyau oxazolyle, isoxazolyle, thiazolyle, 1,2,4-oxadiazolyle, 1,3,4-oxadiazolyle, furannyle, thiényle, pyrrolyle, pyrazolyle, imidazolyle, triazolyle ou tétrazolyle ;
où le noyau hétéroaromatique pentagonal $Ar^{175}$ est facultativement substitué sur un atome de carbone de noyau avec substituant consistant en un substituant méthyle, éthyle, isopropyle, tertiobutyle ou phényle et/ou est facultativement substitué sur un atome d'azote de noyau avec un substituant consistant en un substituant méthyle, éthyle, isopropyle, tertiobutyle ou phényle ;
ou dans laquelle le noyau hétéroaromatique pentagonal $Ar^{175}$ est facultativement condensé avec un noyau phényle, où le point de connexion au reste de la molécule est situé dans le noyau pentagonal ;

et dans laquelle $Ar^{176}$ représente un noyau hétéroaromatique hexagonal consistant en un noyau pyridinyle, pyrimidinyle, pyrazinyle ou pyridazinyle, ledit noyau hétéroaromatique hexagonal étant facultativement substitué sur un atome de carbone de noyau, soit avec un substituant méthyle, soit avec un substituant OH qui est substitué sur un atome de carbone de noyau lié à un atome d'azote de noyau (y compris sa forme tautomère céto) ;
$R^{30}$, indépendant d'un autre groupe $R^{30}$, représente un atome d'hydrogène (H), un groupe alkyle en $C_1$ à $C_4$ ou cycloalkyle en $C_3$ à $C_6$ ;
$Ar^6$ et $Ar^{16}$, indépendamment, représentent un noyau hétérocyclique aromatique pentagonal connecté au niveau d'un atome de carbone de noyau et contenant un atome de 0 ou S ou un groupe un groupe $NR^{15}$ dans le noyau pentagonal, où le noyau pentagonal peut contenir facultativement en outre un ou deux atomes de N, et où le noyau hétérocyclique est facultativement substitué sur un atome de carbone de noyau avec un des substituants suivants : un atome de fluor, un atome de chlore, un groupe alkyle en $C_1$ ou $C_2$, fluoralkyle en $C_1$, $-CH_2OH$, $-CH_2$-OMe ou OH (y compris n'importe laquelle de ses formes tautomère céto) ;
Het, indépendant d'un autre groupe Het, représente un noyau hétérocyclique saturé tétra-, penta-, hexa- ou heptagonal connecté au niveau d'un atome de carbone de noyau et contenant un ou deux hétéroatomes de noyau, choisis indépendamment entre 0, S et N ; où n'importe quels atomes d'azote du noyau qui sont présents sont présents sous forme d'un groupe $NR^{31}$, dans lequel $R^{31}$ représente H, un groupe alkyle en $C_1$ à $C_4$, -C(O)-(alkyle en $C_1$ à $C_4$) ou $-S(O)_2$-(alkyle en $C_1$ à $C_4$) ; et où le noyau Het est facultativement substitué au niveau d'un atome de carbone de noyau avec un substituant méthyle ou oxo (=O), sous réserve que n'importe quel substituant oxo (=O) soit substitué au niveau d'un atome de carbone de noyau lié à un atome d'azote de noyau dans lequel $R^{31}$ représente H ou un groupe alkyle en $C_1$ à $C_4$ ; et
$Het^1$, indépendant d'un autre groupe $Het^1$, représente un noyau hétérocyclique saturé tétra-, penta-, hexa- ou heptagonal connecté au niveau d'un atome de carbone de noyau et contenant un ou deux hétéroatomes de noyau choisis indépendamment entre 0, S et N ; où n'importe quels atomes d'azote du noyau qui sont présents sont présents sous forme d'un groupe $NR^{31a}$ dans lequel $R^{31a}$ représente H, un groupe alkyle en $C_1$ ou $C_2$, -C(O)Me ou $-S(O)_2Me$ ; et où le noyau $Het^1$ est facultativement substitué au niveau d'un atome de carbone de noyau avec un substituant méthyle ou oxo (=O), sous réserve que n'importe quel substituant oxo (=O) soit substitué au niveau d'un atome de carbone de noyau lié à un atome d'azote de noyau, dans lequel $R^{31a}$ représente H ou un groupe alkyle en $C_1$ ou $C_2$.

2. Composé ou sel suivant la revendication 1, dans lequel $R^1$ représente un groupe éthyle.

3. Composé ou sel suivant la revendication 1 ou 2, dans lequel $R^2$ représente un groupe méthyle ou éthyle.

4. Composé ou sel suivant l'une quelconque des revendications précédentes, dans lequel $R^3$ représente le groupe cycloalkyle en $C_4$ à $C_7$ facultativement substitué ou le groupe hétérocyclique, facultativement substitué, de sous-formule (aa), (bb) ou (cc).

5. Composé ou sel suivant l'une quelconque des revendications précédentes, dans lequel, lorsque $R^3$ représente le groupe hétérocyclique de sous-formule (aa), (bb) ou (cc), alors Y représente 0 ou un groupe $NR^{10}$.

**6.** Composé ou sel suivant l'une quelconque des revendications précédentes, dans lequel, lorsque $R^3$ représente le groupe hétérocyclique de sous-formule (aa), (bb) ou (cc), alors $R^3$ représente le groupe hétérocyclique de sous-formule (bb) et $n^1$ est égal à 1.

**7.** Composé ou sel suivant l'une quelconque des revendications précédentes, dans lequel, dans $R^3$, le groupe hétérocyclique de sous-formule (aa), (bb) ou (cc) n'est pas substitué sur un atome de carbone de noyau.

**8.** Composé ou sel suivant l'une quelconque des revendications précédentes, dans lequel $R^3$ représente un groupe tétrahydro-2H-pyranne-4-yle ou 1-(aminocarbonyl)-4-pipéridinyle ; c'est-à-dire que $NHR^3$ répond à la sous-formule (h) ou (k2).

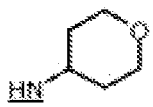
(h)

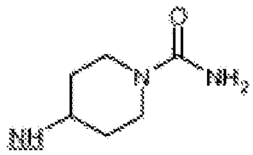
(k2)

**9.** Composé ou sel suivant l'une quelconque des revendications précédentes, dans lequel $R^a$ représente un atome d'hydrogène (H) et $R^b$ représente un atome d'hydrogène (H).

**10.** Composé ou sel suivant l'une quelconque des revendications précédentes, dans lequel $R^4$ représente un atome d'hydrogène (H).

**11.** Composé ou sel suivant l'une quelconque des revendications précédentes, dans lequel $R^5$ représente un groupe :

$-C(O)-(CH_2)_n-Ar$, $-C(O)-Het$, $-C(O)-NR^{15b}-(CH_2)_m{}^1-Ar$ ou $-C(O)-NR^{5a}R^{5b}$ ;
et $R^4$ représente un atome d'hydrogène (H) ;
ou bien $R^4$ et $R^5$, pris conjointement, représentent un groupe $-C(O)-N(R^{15})-(CH_2)_p{}^3-$ ;
ou $NR^4R^5$ répond à la sous-formule (y), (y1), (y2) ou (y3).

**12.** *N*-((1-éthyl-6-méthyl-4-((tétrahydro-2*H*-pyranne-4-yl)amino)-1*H*-pyrazolo[3,4-*b*]pyridine-5-yl)méthyl)-5-méthyl-pyrazine-2-carboxamide

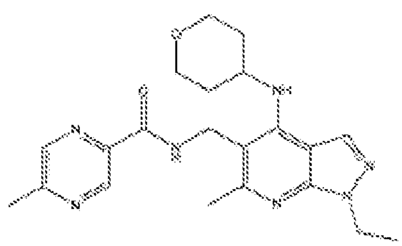

ou un de ses sels.

**13.** Composé ou sel suivant l'une quelconque des revendications précédentes, destiné à être utilisé comme substance thérapeutique active chez un mammifère.

**14.** Composition pharmaceutique comprenant un composé de formule (I), tel que défini dans l'une quelconque des revendications 1 à 12, ou un de ses sels pharmaceutiquement acceptables, et un ou plusieurs supports et/ou excipients pharmaceutiquement acceptables.

**15.** Composition pharmaceutique suivant la revendication 14, destinée à être utilisée dans le traitement et/ou la prophylaxie d'une maladie inflammatoire et/ou allergique, d'une altération cognitive ou de la dépression chez un mammifère.

**16.** Composition pharmaceutique suivant la revendication 15, l'altération cognitive étant présente dans un trouble neurologique.

**17.** Composition pharmaceutique suivant la revendication 16, le trouble neurologique étant choisi entre la maladie d'Alzheimer et la schizophrénie.

**18.** Utilisation d'un composé de formule (I) tel que défini dans l'une quelconque des revendications 1 à 12, ou d'un de ses sels pharmaceutiquement acceptables, dans la production d'un médicament destiné au traitement et/ou à la prophylaxie de l'asthme, de la maladie pulmonaire obstructive chronique (COPD), de la dermatite atopique, de l'urticaire, de la rhinite allergique, de la conjonctivite allergique, de la conjonctivite printanière, du granulome éosinophile, du psoriasis, de la polyarthrite rhumatoïde, d'un choc septique, de la colite ulcérative, de la maladie de Crohn, d'une lésion de reperfusion du myocarde et du cerveau, de la glomérulonéphrite chronique, d'un choc endotoxique, du syndrome de détresse respiratoire de l'adulte, de la sclérose en plaques, d'une altération cognitive, de la dépression ou de la douleur inflammatoire chez un mammifère.

**19.** Utilisation d'un composé suivant la revendication 18, dans laquelle l'altération cognitive est présente dans un trouble neurologique.

**20.** Utilisation d'un composé suivant la revendication 19, dans laquelle le trouble neurologique est choisi entre la maladie d'Alzheimer et la schizophrénie.

**21.** Composé suivant l'une quelconque des revendications 1 à 12, destiné à être utilisé dans le traitement et/ou la prophylaxie de l'asthme, de la maladie pulmonaire obstructive chronique (COPD), de la dermatite atopique, de l'urticaire, de la rhinite allergique, de la conjonctivite allergique, de la conjonctivite printanière, du granulome éosinophile, du psoriasis, de la polyarthrite rhumatoïde, d'un choc septique, de la colite ulcérative, de la maladie de Crohn, d'une lésion de reperfusion du myocarde et du cerveau, de la glomérulonéphrite chronique, d'un choc endotoxique, du syndrome de détresse respiratoire de l'adulte, de la sclérose en plaques, d'une altération cognitive, de la dépression ou de la douleur inflammatoire chez un mammifère.

**22.** Composé suivant la revendication 21, l'altération cognitive étant présente dans un trouble neurologique.

**23.** Composé suivant la revendication 21, le trouble neurologique étant choisi entre la maladie d'Alzheimer et la schizophrénie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004024728 A2
- EP 2003011814 W
- WO 2004056823 A1
- EP 2003014867 W
- WO 2004024728 A
- WO 2004056823 A
- WO 2005058892 A1
- EP 2004014490 W
- WO 2005090348 A1
- GB 2005000983 W
- WO 2005090354 A1
- GB 2005000987 W
- WO 2005090352 A1
- EP 2005003038 W
- WO 2005090353 A1
- GB 2005000976 W
- EP 0311814 W
- WO 03088943 A
- GB 2242134 A
- WO 02066422 A
- WO 03024439 A
- WO 02070490 A
- WO 02076933 A
- WO 03011274 A2
- WO 02069945 A2
- US 20020193393 A1
- US 2002052312 A1
- EP 418716 A1
- WO 9313055 A
- WO 9830537 A
- WO 0250021 A
- WO 9534534 A
- WO 9962875 A
- US 4335121 A
- WO 0212266 A1
- WO 03030939 A1
- EP 0300598 W
- WO 03061743 A
- WO 9420079 A
- CA 928343100
- PL 00430149
- DE 197148941
- WO 0042011 A
- US 4219660 A
- WO 9912933 A
- EP 1188744 A
- WO 03057676 A
- WO 2003104225 A
- WO 2002032867 A
- WO 2002068409 A
- US 2003144283 A
- WO 2003032971 A
- DE 2429778
- WO 2003106459 A
- DE 742410852
- WO 9403426 A
- EP 83303097 A
- DE 2335439
- US 4785012 A
- WO 9630329 A
- GB 1132542 A
- WO 03051841 A
- WO 9629871 A


### Non-patent literature cited in the description


- *Expert Opin. Ther. Patents,* 2005, vol. 15 (1), 111-114
- **Yu.** *J. Med Chem.,* 2001, vol. 44, 1025-1027
- **G. Yu.** *J. Med Chem.,* 2001, vol. 44, 1025-1027
- **Dorgan.** *J. Chem. Soc., Perkin Trans,* 1980, vol. 1 (4), 938-42
- **L.D. Luca.** *J. Org. Chem.,* 2002, vol. 67, 6272-6274
- **N. Novak et al.** *J. Allergy Clin. Immunol.,* 2003, vol. 112, 252-262
- **T.C. Roos et al.** *Drugs,* 2004, vol. 64 (23), 2639-2666
- **J.M. Hanifin et al.** Type 4 phosphodiesterase inhibitors have clinical and in vitro anti-inflammatory effects in atopic dermatitis. *J. Invest. Dermatol.,* 1996, vol. 107 (1), 51-56
- **C.E.M. Griffiths et al.** Randomized comparison of the type 4 phosphodiesterase inhibitor cipamfylline cream, cream vehicle and hydrocortisone 17-butyrate cream for the treatment of atopic dermatitis. *Br. J. Dermatol.,* 2002, vol. 147 (2), 299-307
- **T.C. Roos et al.** Recent advances in treatment strategies for atopic dermatitis. *Drugs,* 2004, vol. 64 (23), 2639-2666

- **A.M.Doherty.** *Current Opinion Chem. Biol.,* 1999, vol. 3 (4), 466-473
- **H.J.Dyke et al.** *Expert Opinion Invest. Drugs,* 2002, vol. 11 (1), 1-13
- **W. Bäumer et al.** *Eur. J. Pharmacol.,* 2002, vol. 446, 195-200
- **W. Bäumer et al.** *J. Pharmacy Pharmacol.,* 2003, vol. 55, 1107-1114
- **S.L. Wolda.** *Emerging Drugs,* 2000, vol. 5 (3), 309-319
- **Z. Huang et al.** *Current Opinion in Chemical Biology,* 2001, vol. 5, 432-438
- **H.J.Dyke et al.** *Expert Opinion on Investigational Drugs,* 2002, vol. 11 (1), 1-13
- **C.Burnouf et al.** *Current Pharmaceutical Design,* 2002, vol. 8 (14), 1255-1296
- **A.M. Vignola.** *Respiratory Medicine,* 2004, vol. 98, 495-503
- **D. Spina.** *Drugs,* 2003, vol. 63 (23), 2575-2594
- **G. Krishna et al.** *Expert Opinion on Investigational Drugs,* 2004, vol. 13 (3), 255-267
- **C.H.Compton et al.** *The Lancet,* 2001, vol. 358, 265-270
- **E.Gamble et al.** *Am. J. Respir. Crit. Care Med.,* 2003, vol. 168, 976-982
- **R.D. Border et al.** *Chest,* 2003, vol. 124, 170S
- **J.D. Eddleston et al.** *Am. J. Respir. Crit. Care Med.,* 2001, vol. 163, A277
- **B.J. Lipworth.** *The Lancet,* 2005, vol. 365, 167-175
- **M.A.Giembycz.** *Drugs,* 2000, vol. 59 (2), 193-212
- **H.J. Dyke et al.** *Expert Opinion on Investigational Drugs,* 2002, vol. 11 (1), 1-13
- **C.Bumouf et al.** *Current Pharmaceutical Design,* 2002, vol. 8 (14), 1255-1296
- **P.J. Barnes.** *Nature Reviews - Drug Discovery,* October 2004, 831-844
- **B.M. Schmidt et al.** *J. Allergy & Clinical Immunology,* 2001, vol. 108 (4), 530-536
- **C.Bumouf et al.** *Current Pharmaceutical Design,* 2002, vol. 8 (14), 1255-1296
- **A.Kumar et al.** *Indian J. Exp. Biol.,* 2000, vol. 38 (1), 26-30
- **H.T.Zhang et al.** *Psychopharmacology,* 2000, vol. 150 (3), 311-316
- *Neuropsychopharmacology,* 2000, vol. 23 (2), 198-204
- **T. Egawa et al.** *Japanese J. Pharmacol.,* 1997, vol. 75 (3), 275-81
- **J. Zhu et al.** *CNS Drug Reviews,* 2001, vol. 7 (4), 387-398
- **O'Donnell.** *Expert Opinion on Investigational Drugs,* 2000, vol. 9 (3), 621-625
- **H.T. Zhang et al.** *Neuropsychopharmacology,* 2002, vol. 27 (4), 587-595
- **J. M. O'Donnell ; H.-T. Zhang.** *Trends Pharmacol. Sci.,* 2004, vol. 25 (3), 158-163
- **T.E.Renau.** *Curr. Opinion Invest. Drugs,* 2004, vol. 5 (1), 34-39
- **K.H.Banner ; M.A.Trevethick.** *Trends Pharmacol. Sci.,* 2004, vol. 25 (8), 430-436
- **M.M. McLaughlin et al.** A low Km, rolipram-sensitive, cAMP-specific phosphodiesterase from human brain: cloning and expression of cDNA, biochemical characterisation of recombinant protein, and tissue distribution of mRNA. *J. Biol. Chem.,* 1993, vol. 268, 6470-6476
- **P. A. Baecker et al.** Isolation of a cDNA encoding a human rolipram-sensitive cyclic AMP phoshodiesterase (PDE IVD). *Gene,* 1994, vol. 138, 253-256
- **K. Loughney et al.** Isolation and characterisation of cDNAs encoding PDE5A, a human cGMP-binding, cGMP-specific 3',5'-cyclic nucleotide phosphodiesterase. *Gene,* 1998, vol. 216, 139-147
- **H. Coste ; P. Grondin.** Characterisation of a novel potent and specific inhibitor of type V phosphodiesterase. *Biochem. Pharmacol.,* 1995, vol. 50, 1577-1585
- **P. Catty ; P. Deterre.** Activation and solubilization of the retinal cGMP-specific phosphodiesterase by limited proteolysis. *Eur. J. Biochern.,* 1991, vol. 199, 263-269
- **A. Tar et al.** Purification of bovine retinal cGMP phosphodiesterase. *Methods in Enzymology,* 1994, vol. 238, 3-12
- **D. Srivastava et al.** Effects of magnesium on cyclic GMP hydrolysis by the bovine retinal rod cyclic GMP phosphodiesterase. *Biochem. J.,* 1995, vol. 308, 653-658
- **A. Robichaud et al.** Emesis induced by inhibitors of [PDE IV] in the ferret. *Neuropharmacology,* 1999, vol. 38, 289-297
- *Neuropharmacology,* 2001, vol. 40, 465-465
- *Pulmonary Pharmacology.,* 1995, vol. 8, 83-89
- **Spond J ; Chapman R ; Fine J ; Jones H ; Kreutner W ; Kung TT ; Minnicozzi M.** Comparison of PDE 4 inhibitors, Rolipram and SB 207499 (Ariflo™), in a rat model of pulmonary neutrophilia. *Pulmonary Pharmacology and Therapeutics.,* 2001, vol. 14, 157-164
- **Underwood DC ; Osbom RR ; Bochnowicz S ; Webb EF ; Rieman DJ ; Lee JC ; Romanic AM ; Adams JL ; Hay DWP ; Griswold DE.** SB 239063, a p38 MAPK inhibitor, reduces neutrophilia, inflammatory cytokines, MMP-9, and fibrosis in lung. *Am J Physiol Lung Cell Mol Physiol.,* 2000, vol. 279, L895-L902
- **Beavo JA ; Contini, M ; Heaslip, R.J.** Multiple cyclic nucleotide phosphodiesterases. *Mol Pharmacol.,* 1994, vol. 46, 399-405

- **Spond J ; Chapman R ; Fine J ; Jones H ; Kreutner W ; Kung TT ; Minnicozzi M.** Comparison of PDE 4 inhibitors, Rolipram and SB 207499 (Ariflo™), in a rat model of pulmonary neutrophilia. *Pulmonary Pharmacology and Therapeudtics.,* 2001, vol. 14, 157-164
- **Takeda N ; Hasegawa S ; Morita M ; Matsunaga T.** Pica in rats is analogous to emesis: an animal model in emesis research. *Pharmacology, Biochemistry and Behavior.,* 1991, vol. 45, 817-821
- **Takeda N ; Hasegawa S ; Morita M ; Horii A ; Uno A ; Yamatodani A ; Matsunaga T.** Neuropharmacological mechanisms of emesis. I . Effects of antiemetic drugs on motion- and apomorphine-induced pica in rats. *Meth Find Exp Clin Pharmacol.,* 1995, vol. 17 (9), 589-596
- **Takeda N ; Hasegawa S ; Morita M ; Horii A ; Uno A ; Yamatodani A ; Matsunaga T.** Neuropharmacological mechanisms of emesis. II. Effects of antiemetic drugs on cisplatin-induced pica in rats. *Meth Find Exp Clin Pharmacol.,* 1995, vol. 17 (9), 647-652
- *J. Org. Chem.,* 1976, vol. 41 (24), 3857
- *J. Am. Chem. Soc.,* 1931, vol. 53, 1836
- *J. Am. Chem. Soc.,* 2000, vol. 122 (40), 9723
- *J. Med. Chem.,* 1992, vol. 35 (7), 1200
- *J. Med. Chem.,* 2000, vol. 43 (21), 3995